(19) Europäisches Patentamt
European Patent Office
Office européen des brevets

(11) EP 4 104 861 A1

(12) **EUROPEAN PATENT APPLICATION**
published in accordance with Art. 153(4) EPC

(43) Date of publication:
**21.12.2022 Bulletin 2022/51**

(21) Application number: **21738338.9**

(22) Date of filing: **06.01.2021**

(51) International Patent Classification (IPC):
***A61K 45/00*** *(2006.01)* ***A61P 21/02*** *(2006.01)*
***A61P 21/04*** *(2006.01)* ***A61P 25/16*** *(2006.01)*
***A61P 25/28*** *(2006.01)* ***A61P 43/00*** *(2006.01)*
***C07D 237/14*** *(2006.01)* ***C07D 237/22*** *(2006.01)*
***C07D 401/04*** *(2006.01)* ***C07D 401/06*** *(2006.01)*
***C07D 401/12*** *(2006.01)* ***C07D 401/14*** *(2006.01)*
***C07D 403/04*** *(2006.01)*

(52) Cooperative Patent Classification (CPC):
**A61K 31/437; A61K 31/4375; A61K 31/496; A61K 31/4985; A61K 31/50; A61K 31/501; A61K 31/5025; A61K 31/506; A61K 31/517; A61K 31/519; A61K 31/5377; A61K 31/538; A61K 31/5386; A61K 31/55; A61K 31/551;** (Cont.)

(86) International application number:
**PCT/JP2021/000184**

(87) International publication number:
**WO 2021/141041 (15.07.2021 Gazette 2021/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.01.2020 JP 2020001010**
**29.05.2020 JP 2020094656**

(71) Applicant: **Sumitomo Pharma Co., Ltd.**
**Osaka 541-0045 (JP)**

(72) Inventors:
- **SAMPEI, Kazuaki**
  **Osaka-shi, Osaka 554-0022 (JP)**
- **ISHIYAMA, Takeo**
  **Osaka-shi, Osaka 554-0022 (JP)**
- **IKEDA, Atsushi**
  **Kobe-shi, Hyogo 650-0047 (JP)**
- **ISHIKAWA, Taizo**
  **Kobe-shi, Hyogo 650-0047 (JP)**
- **HIGUCHI, Makoto**
  **Chiba-shi, Chiba 263-8555 (JP)**
- **TAKUWA, Hiroyuki**
  **Chiba-shi, Chiba 263-8555 (JP)**
- **TAKADO, Yuhei**
  **Chiba-shi, Chiba 263-8555 (JP)**

(74) Representative: **Vossius & Partner**
**Patentanwälte Rechtsanwälte mbB**
**Siebertstraße 3**
**81675 München (DE)**

(54) **THERAPEUTIC AGENT FOR TAUOPATHIES**

(57) The present invention is to provide a medicament for treating and/or preventing tauopathy by activating the voltage-gated sodium channel (Nav). The present invention relates to a medicament for treating and/or preventing tauopathy, comprising a Nav activator as an active ingredient.



Processed by Luminess, 75001 PARIS (FR)

(Cont. next page)

Fig. 3

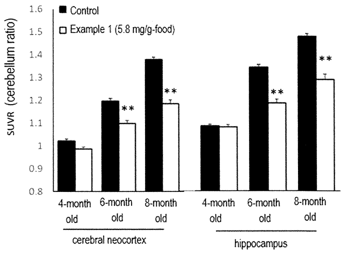


** p<0.01 vs. control; Sidak's multiple comparison test (n=6-11)

(52) Cooperative Patent Classification (CPC): (Cont.)
**A61K 45/00; A61P 21/02; A61P 21/04; A61P 25/16; A61P 25/28; A61P 43/00;**
C07D 237/14; C07D 237/22; C07D 401/04; C07D 401/06; C07D 401/12; C07D 401/14; C07D 403/04; C07D 403/06; C07D 403/12; C07D 403/14; C07D 405/04; C07D 405/06; C07D 405/12; C07D 405/14; C07D 409/14; C07D 413/06; C07D 413/12; C07D 413/14; C07D 417/12; C07D 417/14; C07D 471/04; C07D 471/08; C07D 487/04; C07D 487/10; C07D 491/048; C07D 491/08; C07D 491/107; C07D 513/04; C07D 519/00

## Description

TECHNICAL FIELD

The present invention relates to a medicament for treating and/or preventing tauopathy, comprising a Nav activator as an active ingredient.

BACKGROUND OF THE INVENTION

Nav1.1 is one of voltage-gated sodium channels (VGSC; Nav), which is expressed in, for example, parvalbumin-positive GABA neurons (PV-GABA neurons). It is known that Nav1.1 is important for the neuronal firing function in the neurons (Non-patent literature 1).

It has been suggested that patients suffering from a central nervous system disease such as schizophrenia, autism spectrum disorder (ASD), and attention deficit hyperactivity disorder (ADHD) have dysfunctions in GABAergic neurons which express Nav1.1 (Non-patent literatures 2 and 3).

It has also been reported that heterozygous loss-of-function mutation in SCN1A (Nav1.1) gene leads to epileptic syndromes such as Dravet syndrome (severe myoclonic epilepsy of infancy) and generalized epilepsy with febrile seizure plus (GEFS+) (Non-patent literature 2).

Tauopathy is a general term for a disease whose important pathogenic mechanism is thought to be as follows, tau which is one of microtubule-associated proteins is phosphorylated to be insoluble, and the phosphorylated tau is abnormally accumulated in cells. The typical tauopathy includes Alzheimer's disease (AD) and frontotemporal lobar degeneration (for example, Pick disease, progressive supranuclear palsy and corticobasal degeneration, etc.). In these diseases, the abnormal accumulated of the phosphorylated tau is thought to cause neuronopathy. The mutation of tau gene may cause familial frontotemporal dementia parkinsonism linked to chromosome 17 which is familial tauopathy (Non-patent literature 4), and thus the abnormality of tau is a sufficient condition for neurodegeneration, but the detailed mechanism how the abnormal tau accumulation causes neurodegeneration has not been particularly known.

The decrease of Nav1.1 expression may occur in AD patients or AD model mice. And, AD patients and AD model mice may present with epileptiform hyperexcitation, but the defect of tau may release the hyperexcitation in AD model mice or Nav1.1 heterozygous epilepsy model mice. Thus, it is thought that tau plays a main role in the disorder of interneuron which may cause the Nav1.1 decrease. In addition, it has been reported that the operation of increasing the expression of Nav1.1 in an AD model mouse can improve the cognitive function and can improve the mortality caused by convulsive seizure (Non-patent literature 5), but it has never been reported that the activation of the voltage-gated sodium channel such as Nav1.1 can decrease the aggregation/accumulation of phosphorylated tau or can inhibit the cerebral atrophy.

PRIOR ART

(Non-patent literature)

[Non-patent literature 1] J. Neurosci., 2007, 27, 5903.
[Non-patent literature 2] Trends in Pharmacological Sciences 2014, 35, 113.
[Non-patent literature 3] Curr. Med. Chem. 2015, 22, 1850.
[Non-patent literature 4] Annu. Rev. Pathol. Mech. Dis. 2019, 14, 239.
[Non-patent literature 5] Cell 2012, 149, 708.
[Non-patent literature 6] Journal of Alzheimer's Disease, vol. 71, no. 4, pp. 1163-1174, 2019.

SUMMARY OF INVENTION

(Technical Problem)

One of the purposes of the present invention is to provide a medicament for treating and/or preventing tauopathy by activating the voltage-gated sodium channel (Nav).

(Solution to Problem)

The present inventors have extensively studied to reach the above purpose, and then have found that a Nav

activator may be useful for treating and/or preventing tauopathy. Based upon the findings, the present invention has been completed.

Accordingly, the present invention is described as follows:

(Item 1) A medicament for treating and/or preventing tauopathy, comprising a Nav activator.
(Item 2) The medicament of Item 1, wherein the Nav activator is an activator for at least one Nav subtype selected from the group consisting of Nav1.1, Nav1.2, Navl.3, and Nav1.6.
(Item 3) The medicament of Item 1, wherein the Nav activator is Nav1.1 activator.
(Item 4) The medicament of Item 3, wherein the Nav1.1 activator is a small molecule compound.
(Item 5) The medicament of Item 3, wherein the Nav1.1 activator is a compound of formula (1):

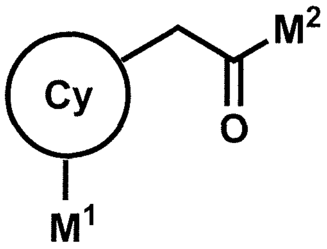

(1)

or a pharmaceutically acceptable salt thereof

wherein

$M^1$ is

(1-1) saturated or partially-unsaturated $C_{4-12}$ carbocyclyl which may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy,
(d) $C_{1-6}$ alkoxy which may be optionally substituted with the same or different 1 to 3 substituents selected from the group consisting of halogen atom, hydroxy, and $C_{1-6}$ alkoxy,
(e) amino which may be optionally substituted with the same or different 1 to 2 $C_{1-6}$ alkyl which may be optionally substituted with the same or different 1 to 3 halogen atoms, and
(f) amino-carbonyl wherein the amino may be optionally substituted with the same or different 1 to 2 $C_{1-6}$ alkyl which may be optionally substituted with the same or different 1 to 3 halogen atoms,

(1-2) 4- to 12-membered saturated or partially-unsaturated heterocyclyl which may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy,
(d) $C_{1-6}$ alkoxy which may be optionally substituted with the same or different 1 to 3 substituents selected from the group consisting of halogen atom, hydroxy, and $C_{1-6}$ alkoxy,
(e) $C_{3-6}$ cycloalkyl which may be optionally substituted with the same or different 1 to 3 halogen atoms,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-3) $C_{6-10}$ aryl which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-4) 5- to 10-membered heteroaryl which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-5) $C_{1-10}$ alkoxy which may be optionally substituted with the same or different 1 to 2 substituents selected from the group consisting of

(a) halogen atom,
(b) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy,

(1-6) $C_{6-10}$ aryloxy which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) amino which may be optionally substituted with the same or different 1 to 2 $C_{1-6}$ alkyl which may be optionally substituted with the same or different 1 to 3 halogen atoms, and
(f) amino-carbonyl wherein the amino may be optionally substituted with the same or different 1 to 2 $C_{1-6}$ alkyl which may be optionally substituted with the same or different 1 to 3 halogen atoms,

(1-7) 5- to 10-membered heteroaryloxy which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-8) $C_{1-10}$ alkyl which may be optionally substituted with the same or different 1 to 4 substituents selected from the group consisting of

(a) halogen atom,
(b) $C_{3-6}$ cycloalkyl which may be optionally substituted with the same or different 1 to 3 halogen atoms,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy,

(1-9) $C_{2-10}$ alkenyl which may be optionally substituted with the same or different 1 to 4 substituents selected from the group consisting of

(a) halogen atom,
(b) $C_{3-6}$ cycloalkyl which may be optionally substituted with the same or different 1 to 3 halogen atoms,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy,

(1-10) $C_{2-10}$ alkynyl which may be optionally substituted with the same or different 1 to 4 substituents selected from the group consisting of

(a) halogen atom,
(b) $C_{3-6}$ cycloalkyl which may be optionally substituted with the same or different 1 to 3 halogen atoms,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy, or

(1-11) $-NR^eR^f$, wherein $R^e$ and $R^f$ are independently

(a) hydrogen atom,
(b) $C_{1-6}$ alkyl which may be optionally substituted with the same or different 1 to 3 substituents selected from the group consisting of halogen atom and $C_{2-10}$ alkynyl,
(c) $C_{3-10}$ cycloalkyl which may be optionally substituted with the same or different 1 to 3 substituents selected from the group consisting of halogen atom, $C_{1-6}$ alkyl, $C_{1-6}$ alkoxy-$C_{1-6}$ alkyl, and $C_{3-6}$ cycloalkyl,
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,

(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy;

$M^2$ is

(2-1) a group of the following formula (2a) or (2b):

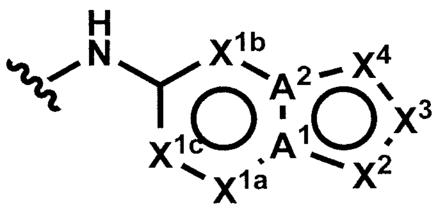
(2a)

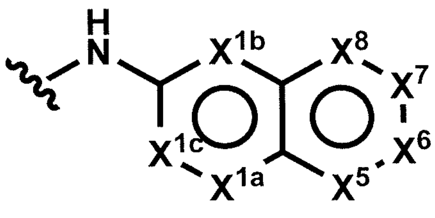
(2b)

wherein

$X^{1a}$, $X^{1b}$, $X^{1c}$, $X^5$, $X^6$, $X^7$, and $X^8$ are independently N or $CR^3$,
$X^2$, $X^3$, and $X^4$ are independently $CR^3$, O, S, N, or $NR^4$,
$A^1$ and $A^2$ are independently N or C,

wherein $X^{1a}$, $X^{1b}$, $X^{1c}$, $X^2$, $X^3$, $X^4$, $X^5$, $X^6$, $X^7$, $X^8$, $A^1$, and $A^2$ are selected so that the ring comprising them can be a 9- or 10-membered bicyclic heteroaromatic ring;

$R^3$ is

(a) hydrogen atom,
(b) halogen atom,
(c) cyano,
(d) hydroxy,
(e) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, $C_{1-6}$ alkoxy, 4- to 7-membered saturated heterocyclyl which may be optionally substituted with $C_{1-6}$ alkoxy, and amino which may be optionally substituted with the same or different 1 to 2 $C_{1-6}$ alkyl,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, $C_{1-6}$ alkoxy, and amino which may be optionally substituted with the same or different 1 to 2 $C_{1-6}$ alkyl,
(g) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl,
(h) amino which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(i) 5- or 6-membered heteroaryl which may be optionally substituted with the same or different 1 to 4 substituents selected from the group consisting of halogen atom, cyano, and $C_{1-6}$ alkyl,
(j) 4- to 7-membered saturated or partially-unsaturated heterocyclyl which may be optionally substituted with the same or different 1 to 4 substituents selected from the group

consisting of halogen atom, hydroxy, $C_{1-6}$ alkyl, and $C_{1-6}$ alkoxy, or
(k) -C(O)NR$^x$R$^y$, wherein R$^x$ and R$^y$ are independently hydrogen atom, $C_{1-6}$ alkyl, or saturated or partially-unsaturated $C_{3-7}$ carbocyclyl; or R$^x$ and R$^y$ may be taken together with the nitrogen atom to which they are attached to form 4- to 7-membered saturated heterocyclic ring,

$R^4$ is

(a) hydrogen atom,
(b) $C_{1-6}$ alkyl which may be optionally substituted with the same or different 1 to 3 substituents selected from the group consisting of halogen atom, hydroxy, and $C_{1-6}$ alkoxy, or
(c) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,

provided that when there are plural $R^3$ or $R^4$, each $R^3$ or each $R^4$ may be the same or different,

(2-2) a group of the following formula (2c):

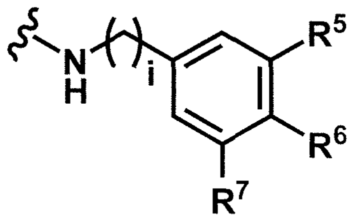

(2c)

wherein

$R^5$, $R^6$, and $R^7$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy,
(e) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,
(f) $C_{1-6}$ alkoxy which may be optionally substituted with the same or different 1 to 3 substituents selected from the group consisting of halogen atom, hydroxy, and $C_{1-6}$ alkoxy,
(g) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,
(h) 5- or 6-membered heteroaryl which may be optionally substituted with the same or different 1 to 4 substituents selected from the group consisting of halogen atom, cyano, and $C_{1-6}$ alkyl,
(i) 4- to 7-membered saturated or partially-unsaturated heterocyclyl which may be optionally substituted with the same or different 1 to 4 substituents selected from the group consisting of halogen atom, hydroxy, $C_{1-6}$ alkyl, and $C_{1-6}$ alkoxy,
(j) -C(O) NR$^x$R$^y$, wherein R$^x$ and R$^y$ are independently hydrogen atom, $C_{1-6}$ alkyl, or saturated or partially-unsaturated $C_{3-7}$ carbocyclyl; or R$^x$ and R$^y$ may be taken together with the nitrogen atom to which they are attached to form 4- to 7-membered saturated heterocyclic ring,
(k) $C_{2-7}$ alkylcarbonyl which may be optionally substituted with the same or different 1 to 3 halogen atoms, or

(l) $C_{2-7}$ alkoxycarbonyl which may be optionally substituted with the same or different 1 to 3 halogen atom,

wherein $R^5$ and $R^6$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom, oxo, $C_{1-6}$ alkyl, $C_{1-6}$ alkoxy, and $C_{2-7}$ alkoxycarbonyl,
i is 0, 1, or 2, and
the substitutable carbon atom on the ring of formula (2c) may have one fluorine atom as a substituent,

(2-3) a group of the following formula (2d), (2e), (2f), (2g), (2h), (2i), or (2j):

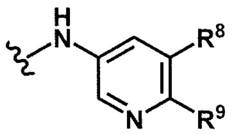
(2d)

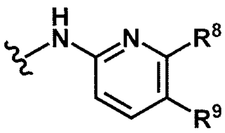
(2e)

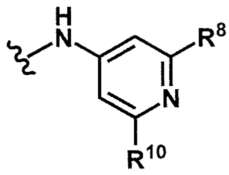
(2f)

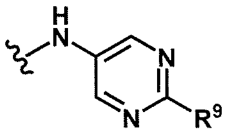
(2g)

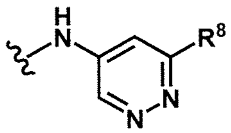
(2h)

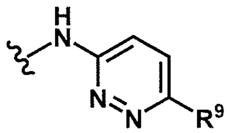
(2i)

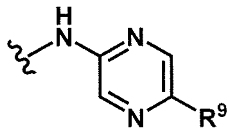
(2j)

wherein

$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; saturated or partially-unsaturated $C_{3-7}$ carbocyclyl; $C_{1-6}$ alkoxy which may be optionally substituted with hydroxy or $C_{1-6}$ alkoxy; 4- to 7-membered saturated or partially-unsaturated heterocyclyl which may be optionally substituted with $C_{1-6}$ alkoxy or $C_{1-6}$ alkyl; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),
(e) $C_{3-6}$ cycloalkyl which may be optionally substituted with the same or different 1 to 3 halogen atoms,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, $C_{1-6}$ alkoxy, and amino which may be optionally substituted with the same or different 1 to 2 $C_{1-6}$ alkyl,
(g) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl,
(h) amino which may be optionally substituted with the same or different 1 to 2 substituents

selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(i) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,
(j) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,
(k) 4- to 7-membered saturated or partially-unsaturated heterocyclyloxy which may be optionally substituted with the same or different 1 to 4 $C_{1-6}$ alkyl,
(l) -C(O)$NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,
(m) -C(O)$OR^Z$, wherein $R^Z$ is $C_{1-6}$ alkyl, or
(n) ethenyl which may be substituted with one 6-membered saturated heterocyclyl,

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl, and
the substitutable carbon atom on the ring of formula (2d), (2e), (2f), (2g), (2h), (2i), or (2j) may have one fluorine atom as a substituent,

(2-4) a group of the following formula (2k):

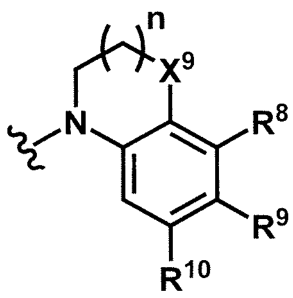

(2k)

wherein

$R^8$, $R^9$, and $R^{10}$ are as defined in the above (2-3),
n is 0, 1, or 2,
$X^9$ is $CH_2$ or O, and
the substitutable carbon atom on the ring of formula (2k) may have one fluorine atom as a substituent,

(2-5) a group of the following formula (21), (2m), or (2n) :

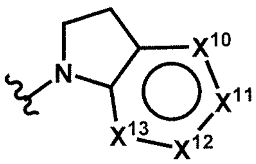 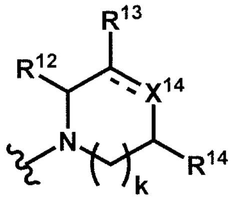 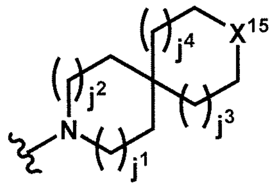

(2l) (2m) (2n)

wherein

$X^{10}$, $X^{11}$, $X^{12}$, and $X^{13}$ are independently N or $CR^{11}$, wherein $X^{10}$, $X^{11}$, $X^{12}$, and $X^{13}$ are selected so that the 6-membered ring comprising them can be a heteroaromatic ring,
$X^{14}$ is $CR^{15}$, $CHR^{15}$, $NR^{16}$, or O,
provided that when $X^{14}$ is $CR^{15}$, the bond having a broken line in formula (2m) is a double bond, or when $X^{14}$ is $CHR^{15}$, $NR^{16}$, or O, the bond having a broken line in formula (2m) is a single bond,
$X^{15}$ is $NR^{17}$ or O,
$R^{11}$ is independently

(a) hydrogen atom,
(b) halogen atom,
(c) 5- or 6-membered heteroaryl which may be optionally substituted with the same or different 1 to 4 substituents selected from the group consisting of halogen atom, cyano, $C_{1-6}$ alkyl, and $C_{1-6}$ alkoxy,
(d) 5- or 6-membered heteroaryl-methyl which may be optionally substituted with the same or different 1 to 4 substituents selected from the group consisting of halogen atom, cyano, $C_{1-6}$ alkyl, and $C_{1-6}$ alkoxy, or
(e) 4- to 7-membered saturated or partially-unsaturated heterocyclyl which may be optionally substituted with the same or different 1 to 4 substituents selected from the group consisting of $C_{1-6}$ alkyl, $C_{1-6}$ alkoxy, $C_{2-7}$ alkylcarbonyl, and $C_{2-7}$ alkoxycarbonyl,

provided that there are plural $R^{11}$, each $R^{11}$ may be the same or different,
$R^{12}$, $R^{13}$, and $R^{14}$ are independently

(a) hydrogen atom, or
(b) $C_{1-6}$ alkyl,

wherein $R^{12}$ and $R^{14}$, or $R^{13}$ and $R^{14}$ may be taken together with the carbon atoms to which they attach to form a bridged structure,
$R^{15}$ is

(a) phenyl which may be optionally substituted with the same or different 1 to 2 substituents selected from the group consisting of halogen atom, cyano, $C_{1-6}$ alkyl, and $C_{1-6}$ alkoxy,
(b) benzyl which may be optionally substituted with the same or different 1 to 2 substituents selected from the group consisting of halogen atom, cyano, $C_{1-6}$ alkyl, and $C_{1-6}$ alkoxy,
(c) 5- to 10-membered heteroaryl which may be optionally substituted with the same or different 1 to 2 substituents selected from the group consisting of halogen atom, cyano, $C_{1-6}$ alkyl, and $C_{1-6}$ alkoxy,
(d) hydroxy,
(e) phenyloxy which may be optionally substituted with the same or different 1 to 2 substituents selected from the group consisting of halogen atom, cyano, $C_{1-6}$ alkyl, and $C_{1-6}$ alkoxy, or
(f) phenylamino which may be optionally substituted with the same or different 1 to 2 substituents selected from the group consisting of halogen atom, cyano, $C_{1-6}$ alkyl, and $C_{1-6}$ alkoxy,

$R^{16}$ is

(a) phenyl which may be optionally substituted with the same or different 1 to 2 substituents

selected from the group consisting of halogen atom, cyano, $C_{1-6}$ alkyl, and $C_{1-6}$ alkoxy,
(b) 5- or 6-membered heteroaryl which may be optionally substituted with the same or different 1 to 2 substituents selected from the group consisting of halogen atom, cyano, $C_{1-6}$ alkyl which may be optionally substituted with 1 to 3 fluorine atoms, and $C_{1-6}$ alkoxy which may be optionally substituted with 1 to 3 fluorine atoms,
(c) 5- or 6-membered heteroarylmethyl which may be optionally substituted with the same or different 1 to 2 substituents selected from the group consisting of halogen atom, cyano, $C_{1-6}$ alkyl, and $C_{1-6}$ alkoxy,
(d) 5- or 6-membered saturated or partially-unsaturated carbocyclyl which may be optionally substituted with the same or different 1 to 3 halogen atoms, or
(e) 5- or 6-membered saturated heterocyclyl which may be optionally substituted with the same or different 1 to 3 halogen atoms,

$R^{17}$ is

(a) pyridyl which may be optionally substituted with the same or different 1 to 2 substituents selected from the group consisting of halogen atom, cyano, $C_{1-6}$ alkyl, and $C_{1-6}$ alkoxy, or
(b) 5- or 6-membered saturated heterocyclyl which may be optionally substituted with the same or different 1 to 3 halogen atoms,

k is 0, 1, or 2, and
$j^1$, $j^2$, $j^3$, and $j^4$ are independently 0 or 1,

(2-6) a group of the following formula (2o):

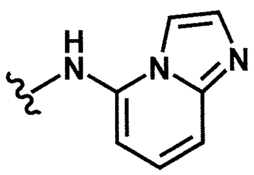

(2o) ,

or
(2-7) a group of the following formula (2p) or (2q):

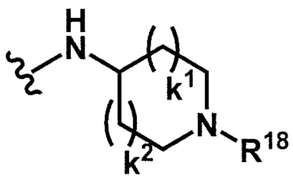

(2p)

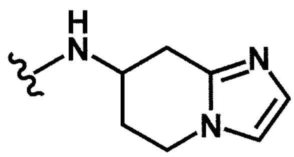

(2q)

wherein

$R^{18}$ is

(a) phenyl which may be optionally substituted with the same or different 1 to 2 substituents selected from the group consisting of halogen atom, cyano, $C_{1-6}$ alkyl, and $C_{1-6}$ alkoxy, or
(b) benzyl which may be optionally substituted with the same or different 1 to 2 substituents selected from the group consisting of halogen atom, cyano, $C_{1-6}$ alkyl, and $C_{1-6}$ alkoxy,

$k^1$ and $k^2$ are independently 0 or 1,
wherein the nitrogen-containing saturated ring in formula (2p) may be optionally substituted with oxo; and Ring Cy is an optionally-substituted 5- to 10-membered heteroarylene.

(Item 6) The medicament of Item 5, wherein
Ring Cy is a group of formula (a):

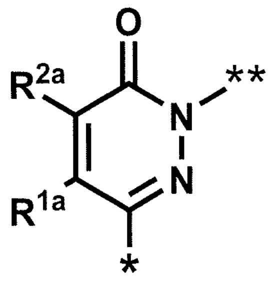

(a)

wherein

* is binding point to $M^1$, and ** is binding point to $CH_2C(=O)M^2$,

$R^{1a}$ and $R^{2a}$ are independently

(3-1) hydrogen atom,
(3-2) halogen atom,
(3-3) cyano,
(3-4) $C_{1-6}$ alkyl which may be optionally substituted with the same or different 1 to 3 substituents selected from the group consisting of

(a) halogen atom,
(b) hydroxy,
(c) saturated or partially-unsaturated $C_{3-7}$ carbocyclyl,
(d) $C_{1-6}$ alkoxy, and
(e) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,

(3-5) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,
(3-6) $C_{2-6}$ alkenyl which may be optionally substituted with the same or different 1 to 4 halogen atoms,
(3-7) $C_{1-6}$ alkoxy which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy, or
(3-8) amino which may be optionally substituted with the same or different 1 to 2 substituents selected from the group consisting of $C_{1-6}$ alkyl which may be optionally substituted with the same or different 1 to 3 halogen atoms, saturated or partially-unsaturated $C_{3-7}$ carbocyclyl, and $C_{2-7}$ alkylcarbonyl; or

$R^{1a}$ and $R^{2a}$ may be taken together with the carbon atoms to which they are attached to form

(4-1) 5- to 7-membered saturated or partially-unsaturated carbon ring which may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy, or

(4-2) 5 - to 7-membered saturated or partially-unsaturated hetero ring which may be optionally substituted with the same or different 1 to 4 substituents selected from the group consisting of the above (a) - (d) in (4-1), or a group of formula (b):

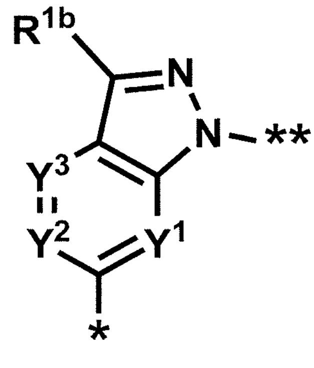

(b)

wherein

* is binding point to $M^1$, and ** is binding point to $CH_2C(=O)M^2$,

$Y^1$, $Y^2$, and $Y^3$ are independently N or $CR^{2b}$;
$R^{1b}$ is hydrogen atom, halogen atom, cyano, $C_{1-6}$ alkyl which may be optionally substituted with the same or different 1 to 3 halogen atoms, $C_{3-6}$ cycloalkyl which may be optionally substituted with the same or different 1 to 3 halogen atoms, $C_{1-6}$ alkoxy which may be optionally substituted with the same or different 1 to 3 halogen atoms, or amino which may be optionally substituted with the same or different 1 to 2 $C_{1-6}$ alkyl;
$R^{2b}$ is, independently if there are plural $R^{2b}$, hydrogen atom, halogen atom, cyano, $C_{1-6}$ alkyl which may be optionally substituted with the same or different 1 to 3 halogen atoms, $C_{3-6}$ cycloalkyl which may be optionally substituted with the same or different 1 to 3 halogen atoms, $C_{1-6}$ alkoxy which may be optionally substituted with the same or different 1 to 3 halogen atoms, or amino which may be optionally substituted with the same or different 1 to 2 $C_{1-6}$ alkyl, provided that when there are plural $R^{2b}$, each $R^{2b}$ may be the same or different.

(Item 7) The medicament of Item 5 or 6, wherein

$M^2$ is

(1) a group of any one of the following formulae (2a-1) - (2a-23) and (2b-1) - (2b-11):

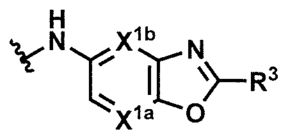
(2a-1)

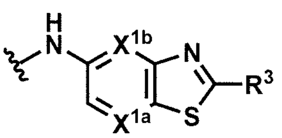
(2a-2)

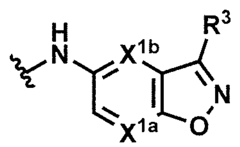
(2a-3)

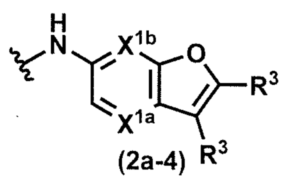
(2a-4)

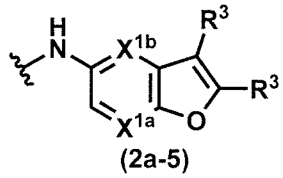
(2a-5)

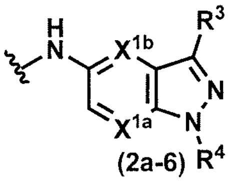
(2a-6)

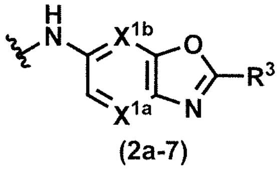
(2a-7)

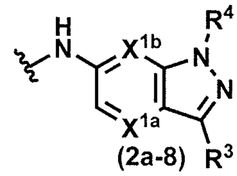
(2a-8)

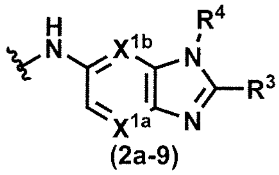
(2a-9)

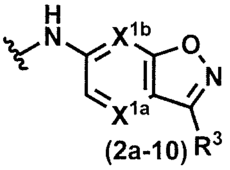
(2a-10)

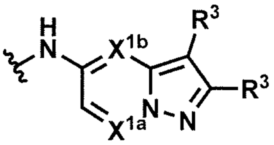
(2a-11)

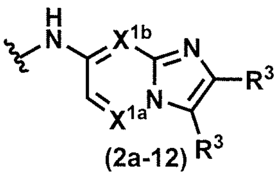
(2a-12)

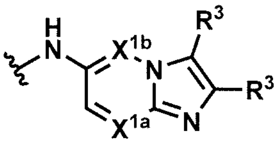
(2a-13)

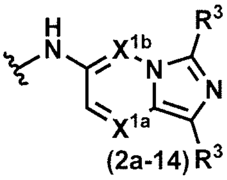
(2a-14)

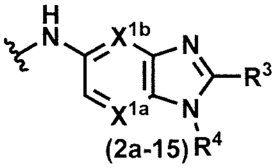
(2a-15)

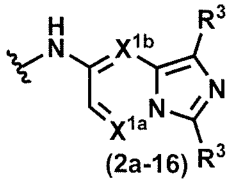
(2a-16)

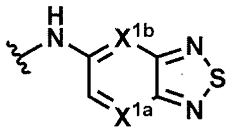
(2a-17)

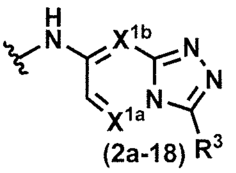
(2a-18)

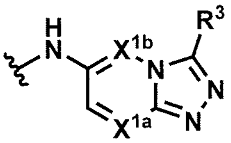
(2a-19)

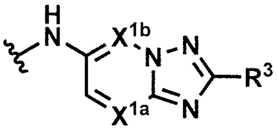
(2a-20)

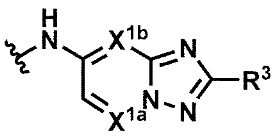
(2a-21)

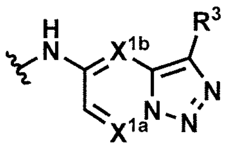
(2a-22)

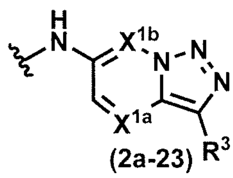
(2a-23)

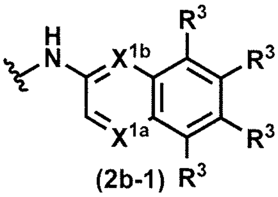
(2b-1)

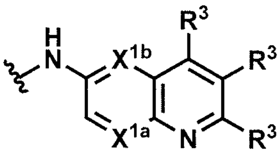
(2b-2)

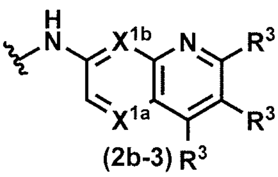
(2b-3)

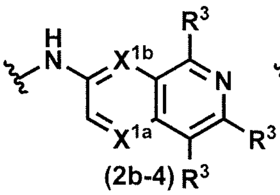
(2b-4)

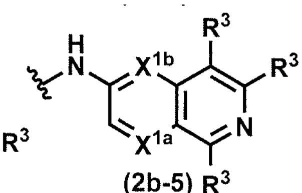
(2b-5)

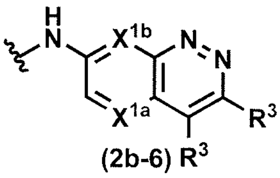
(2b-6)

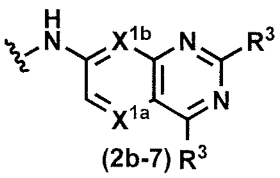
(2b-7)

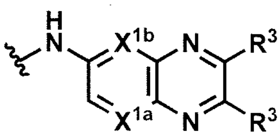
(2b-8)

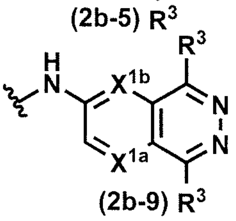
(2b-9)

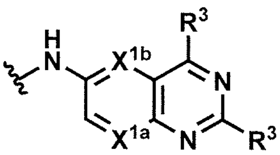
(2b-10)

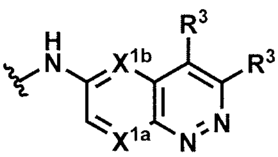
(2b-11)

wherein $X^{1a}$, $X^{1b}$, $R^3$, and $R^4$ are as defined in the above Item 5,
(2) a group of the following (2c'):

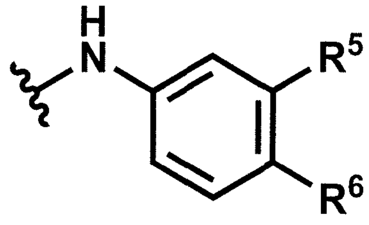

(2c')

wherein

$R^5$ and $R^6$ are independently

(a) hydrogen atom,
(b) halogen atom,
(c) cyano,
(d) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,
(e) 5- or 6-membered heteroaryl which may be optionally substituted with the same or different 1 to 4 substituents selected from the group consisting of halogen atom, cyano, and $C_{1-6}$ alkyl,
(f) 4- to 7-membered saturated or partially-unsaturated heterocyclyl which may be optionally substituted with the same or different 1 to 4 substituents selected from the group consisting of halogen atom, hydroxy, $C_{1-6}$ alkyl, and $C_{1-6}$ alkoxy, or
(g) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring, or

$R^5$ and $R^6$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom, oxo, $C_{1-6}$ alkyl, $C_{1-6}$ alkoxy, and $C_{2-7}$ alkoxycarbonyl,
the substitutable carbon atom on the ring of formula (2c') may have one fluorine atom as a substituent,

(3) a group of the following formula (2d), (2f), (2g), or (2h):

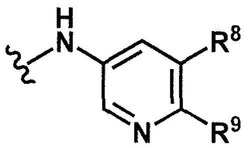

(2d)

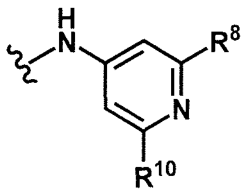

(2f)

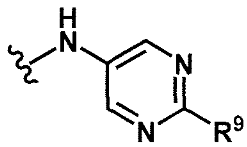

(2g)

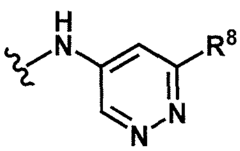

(2h)

wherein

$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy or $C_{1-6}$ alkoxy; 4-to 7-membered saturated or partially-unsaturated heterocyclyl which may be optionally substituted with $C_{1-6}$ alkyl or $C_{1-6}$ alkoxy; 5- or 6-membered

heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),

(e) $C_{1-6}$ alkoxy which may be optionally substituted with the same or different 1 to 3 substituents selected from the group consisting of halogen atom, hydroxy, and $C_{1-6}$ alkoxy,

(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,

(g) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,

(h) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,

(i) 4- to 7-membered saturated or partially-unsaturated heterocyclyloxy which may be optionally substituted with the same or different 1 to 4 $C_{1-6}$ alkyl,

(j) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,

(k) -C(O) $OR^Z$, wherein $R^Z$ is $C_{1-6}$ alkyl, or

(l) ethenyl which may be substituted with one 6-membered saturated heterocyclyl,

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl, and

the substitutable carbon atom on the ring of formula (2d), (2f), (2g), or (2h) may have one fluorine atom as a substituent, or

(4) a group of formula (2k'):

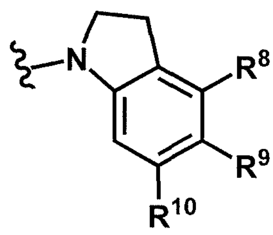

(2k')

wherein $R^8$, $R^9$, and $R^{10}$ are as defined in the above (3) .

(Item 8) The medicament of Item 5 or 6, wherein
$M^2$ is a group of

(1) a group of the following formulae (2a-1), (2a-2), (2a-12), (2a-20), (2a-21), (2b-3), (2b-4), (2b-7), or (2b-10) :

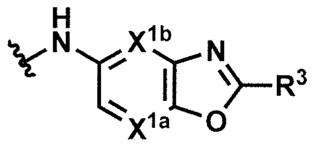
(2a-1)

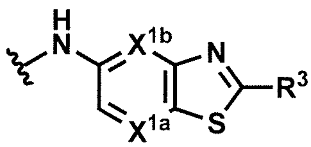
(2a-2)

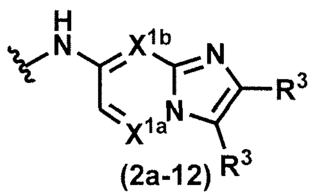
(2a-12)

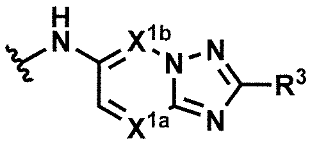
(2a-20)

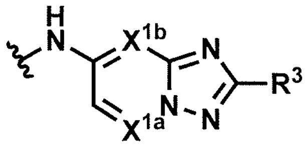
(2a-21)

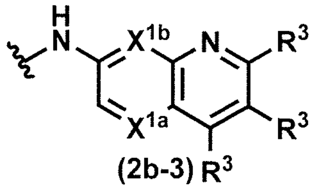
(2b-3)

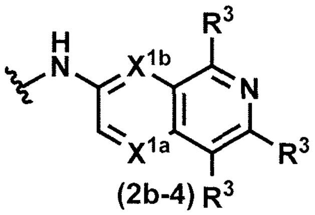
(2b-4)

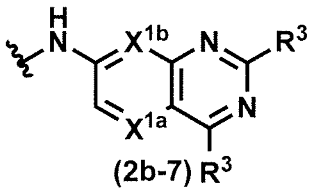
(2b-7)

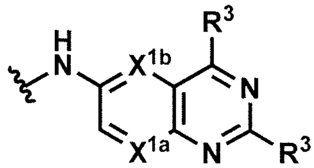
(2b-10)

wherein $X^{1a}$, $X^{1b}$, and $R^3$ are as defined in the above Item 5,
(2) a group of the following formula (2c'):

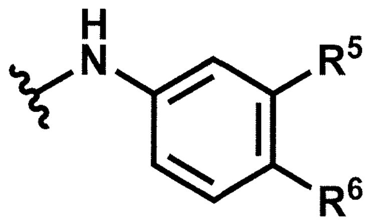

(2c')

wherein

$R^5$ and $R^6$ are independently

(a) hydrogen atom,
(b) halogen atom,
(c) cyano, or
(d) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,

the substitutable carbon atom on the ring of formula (2c') may have one fluorine atom as a substituent,

(3) a group of the following formula (2d) or (2f):

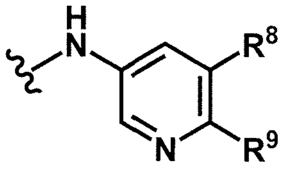

(2d)

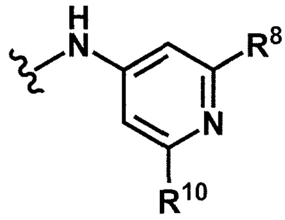

(2f)

wherein $R^8$, $R^9$, and $R^{10}$ are as defined in the above Item 7,
the substitutable carbon atom on the ring of formula (2d) or (2f) may have one fluorine atom as a substituent, or
(4) a group of the following formula (2k"):

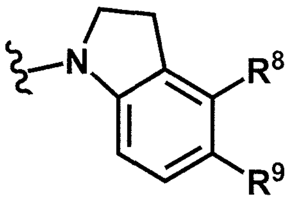

(2k")

wherein $R^8$ and $R^9$ are as defined in the above Item 7.

(Item 9) The medicament of any one of Items 5 to 8, wherein
$M^1$ is

(1) saturated or partially-unsaturated $C_{4-12}$ carbocyclyl which may be optionally substituted with the same or different 1 to 4 substituents selected from the group consisting of halogen atom; and $C_{1-6}$ alkyl which may be optionally substituted with the same or different 1 to 3 substituents selected from the group consisting of halogen atom, hydroxy, and $C_{1-6}$ alkoxy,
(2) 4- to 12-membered saturated or partially-unsaturated heterocyclyl which may be optionally substituted with the same or different 1 to 4 substituents selected from the group consisting of halogen atom; and $C_{1-6}$ alkyl which may be optionally substituted with the same or different 1 to 3 substituents selected from the group consisting of halogen atom, hydroxy, and $C_{1-6}$ alkoxy, or
(3) $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(d) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy.

(Item 10) The medicament of any one of Items 5 to 9, wherein
$M^2$ is a group of the following formula (2a-20), (2a-21), (2b-3), (2b-4), or (2b-7):

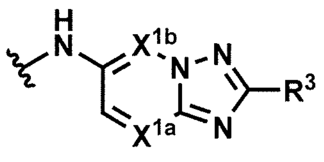
(2a-20)

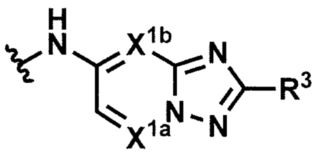
(2a-21)

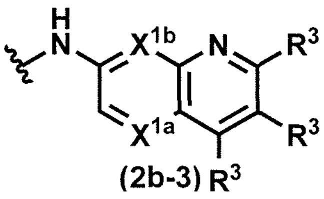
(2b-3)

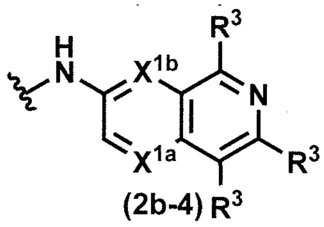
(2b-4)

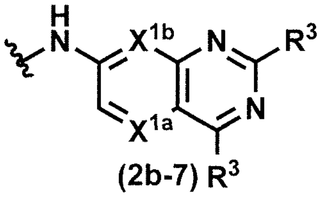
(2b-7)

wherein

$X^{1a}$ and $X^{1b}$ are independently N or $CR^3$;
$R^3$ is independently

(a) hydrogen atom,
(b) halogen atom,
(c) cyano,
(d) hydroxy,
(e) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, $C_{1-6}$ alkoxy, 4- to 7-membered saturated heterocyclyl which may be optionally substituted with $C_{1-6}$ alkoxy, and amino which may be optionally substituted with the same or different 1 to 2 $C_{1-6}$ alkyl,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, $C_{1-6}$ alkoxy, and amino which may be optionally substituted with the same or different 1 to 2 $C_{1-6}$ alkyl,
(g) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl, or
(h) amino which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,

provided that when there are plural $R^3$, each $R^3$ may be the same or different.

(Item 11) The medicament of any one of Items 5 to 9, wherein
$M^2$ is a group of the following formula (2a'-20), (2a'-21), (2b'-3), (2b'-4), or (2b'-7):

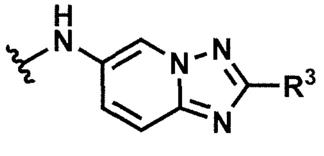
(2a'-20)

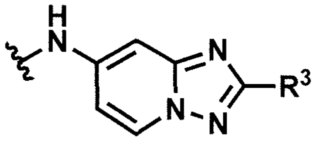
(2a'-21)

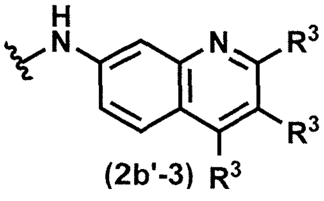
(2b'-3)

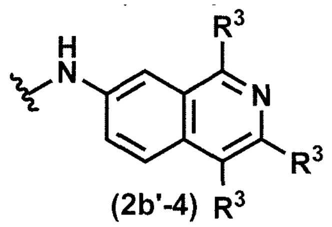

(2b'-4)

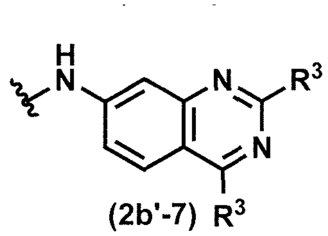

(2b'-7)

wherein

$R^3$ is independently

(a) hydrogen atom,
(b) halogen atom,
(c) cyano,
(d) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, $C_{1-6}$ alkoxy, 4- to 7-membered saturated heterocyclyl which may be optionally substituted with $C_{1-6}$ alkoxy, and amino which may be optionally substituted with the same or different 1 to 2 $C_{1-6}$ alkyl,
(e) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl, or
(f) amino which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,

provided that when there are plural $R^3$, each $R^3$ may be the same or different.

(Item 12) The medicament of any one of Items 5 to 9, wherein
$M^2$ is a group of the following formula (2d) or (2f):

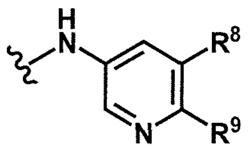

(2d)

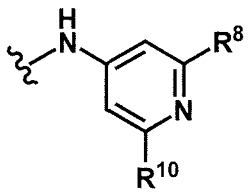

(2f)

wherein
$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy or $C_{1-6}$ alkoxy; 4-to 7-membered saturated or partially-unsaturated heterocyclyl which may be optionally substituted with $C_{1-6}$ alkyl or $C_{1-6}$ alkoxy; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with

$C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(g) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,
(h) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,
(i) 4- to 7-membered saturated or partially-unsaturated heterocyclyloxy which may be optionally substituted with the same or different 1 to 4 $C_{1-6}$ alkyl,
(j) -C(O)$NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,
(k) -C(O)$OR^Z$, wherein $R^Z$ is $C_{1-6}$ alkyl, or
(l) ethenyl which may be substituted with one 6-membered saturated heterocyclyl,

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl.
(Item 13) The medicament of Item 12, wherein
$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy or $C_{1-6}$ alkoxy; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy, or
(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy.

(Item 14) The medicament of any one of Items 5 to 13, wherein
$M^1$ is a group of the following formula (3):

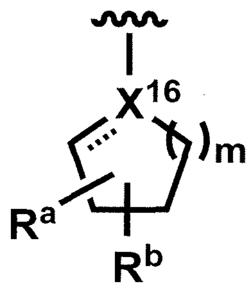

(3)

wherein

$X^{16}$ is N, C, or CH,
the bond having a broken line is a single bond or a double bond,
m is 0, 1, 2, or 3,
$R^a$ and $R^b$ are independently

(1-1) hydrogen atom,
(1-2) halogen atom, or
(1-3) $C_{1-6}$ alkyl which may be optionally substituted with the same or different 1 to 3 substituents selected from the group consisting of halogen atom, hydroxy, and $C_{1-6}$ alkoxy, or

$R^a$ and $R^b$ may be taken together with the carbon atom(s) to which they are attached to form 3- to 6-membered saturated carbocyclic ring, wherein the ring may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy.

(Item 15) The medicament of Item 14, wherein $X^{16}$ is C, and the bond having a broken line is a double bond.
(Item 16) The medicament of Item 14, wherein $X^{16}$ is CH, and the bond having a broken line is a single bond.
(Item 17) The medicament of Item 14, wherein $X^{16}$ is N, and the bond having a broken line is a single bond.
(Item 18) The medicament of any one of Items 5 to 13, wherein
$M^1$ is $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,
(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy.

(Item 19) The medicament of any one of Items 5 to 13, wherein
$M^1$ is $-NR^eR^f$, wherein $R^e$ and $R^f$ are independently

(a) hydrogen atom,
(b) $C_{1-6}$ alkyl which may be optionally substituted with the same or different 1 to 3 halogen atoms,

(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or

(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl.

(Item 20) The medicament of any one of Items 5 to 13, wherein
$M^1$ is $-NR^eR^f$, wherein

$R^e$ is methyl, and
$R^f$ is

(a) $C_{1-6}$ alkyl which may be optionally substituted with the same or different 1 to 3 halogen atoms,

(b) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or

(c) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl.

(Item 21) The medicament of any one of Items 5 to 13, wherein
$M^1$ is a group of any one of the following formulae (3a-1) - (3a-4):

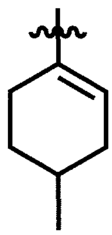
(3a-1)
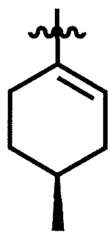
(3a-2)
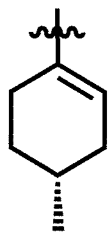
(3a-3)
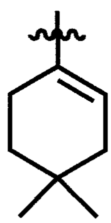
(3a-4)
.

(Item 22) The medicament of any one of Items 5 to 13, wherein
$M^1$ is a group of any one of the following formulae (3c-1) - (3c-6):

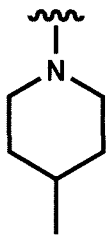
(3c-1)
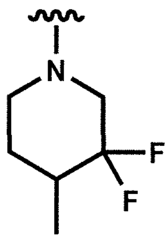
(3c-2)
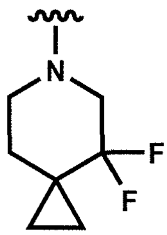
(3c-3)
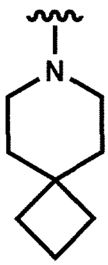
(3c-4)
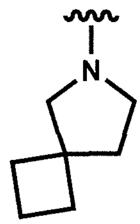
(3c-5)
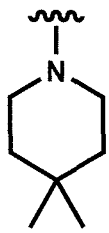
(3c-6)
.

(Item 23) The medicament of any one of Items 5 to 13, wherein
M is a group of any one of the following formulae (3d-1) - (3d-12):

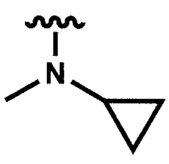
(3d-1)
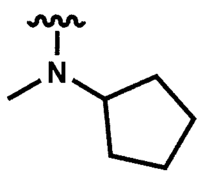
(3d-2)
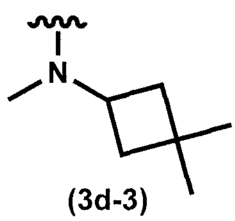
(3d-3)
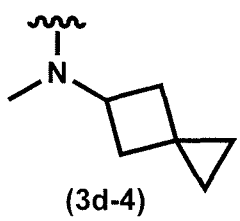
(3d-4)

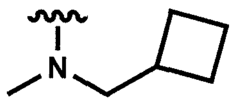
(3d-5)

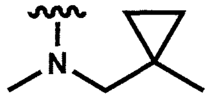
(3d-6)

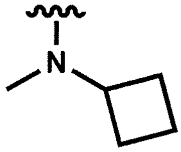
(3d-7)

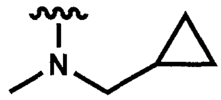
(3d-8)

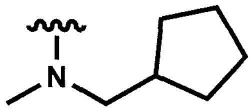
(3d-9)

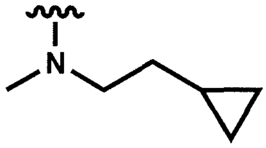
(3d-10)

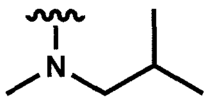
(3d-11)

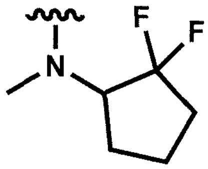
(3d-12)

.

(Item 24) The medicament of any one of Items 6 to 23, wherein Ring Cy is formula (a).
(Item 25) The medicament of Item 24, wherein $R^{1a}$ and $R^{2a}$ are independently hydrogen atom, halogen atom, cyano, methyl, or methoxy.
(Item 26) The medicament of Item 24, wherein $R^{1a}$ and $R^{2a}$ are hydrogen atom.
(Item 27) The medicament of any one of Items 6 to 23, wherein Ring Cy is formula (b).
(Item 28) The medicament of Item 27, wherein $Y^1$ is N, and $Y^2$ and $Y^3$ are independently $CR^{2b}$.
(Item 29) The medicament of Item 27, wherein $Y^1$ and $Y^2$ are N, and $Y^3$ is $CR^{2b}$.
(Item 30) The medicament of Item 27, wherein $Y^1$ and $Y^3$ are N, and $Y^2$ is $CR^{2b}$.
(Item 31) The medicament of any one of Items 27 to 30, wherein
$R^{1b}$ and $R^{2b}$ are independently hydrogen atom, halogen atom, cyano, $C_{1-6}$ alkyl, $C_{3-6}$ cycloalkyl, or $C_{1-6}$ alkoxy, provided that when there are plural $R^{2b}$, each $R^{2b}$ may be the same or different.
(Item 32) The medicament of any one of Items 27 to 30, wherein $R^{1b}$ and $R^{2b}$ are hydrogen atom.
(Item 33) The medicament of Item 5, wherein the Nav1.1 activator is any one compound selected from the group consisting of the following compounds:

N-(4-cyanophenyl)-2-[3-(4-methylpiperidin-1-yl)-6-oxopyridazin-1(6H)-yl]acetamide (Example 1),
N-(1,3-benzoxazol-5-yl)-2-[3-(4-methylpiperidin-1-yl)-6-oxopyridazin-1(6H)-yl]acetamide (Example 50),
2-[3-(6-azaspiro[3.4]octan-6-yl)-6-oxopyridazin-1(6H)-yl]-N-(quinazolin-7-yl)acetamide (Example 236),
N-[2-(dimethylamino)-1,3-benzoxazol-5-yl]-2-[3-(4-methylcyclohex-1-ene)-6-oxopyridazin-1(6H)-yl]acetamide (Example 244),
N-(2,6-dimethylpyridin-4-yl)-2-[3-(4-methylcyclohex-1-en-1-yl)-6-oxopyridazin-1(6H)-yl]acetamide (Example 364),
6-(4-methylcyclohex-1-en-1-yl)-2-{2-[4-(2-methylpyridin-3-yl) piperazin-1-yl]-2-oxoethyl}pyridazin-3(2H)-one (Example 410),
2-[3-(4,4-dimethylcyclohex-1-en-1-yl)-6-oxopyridazin-1(6H)-yl]-N-([1,2,4]triazolo[1,5-a]pyridin-6-yl)acetamide (Example 428),
2-[3-(4,4-dimethylcyclohex-1-en-1-yl)-6-oxopyridazin-1(6H)-yl]-N-([1,2,4]triazolo[1,5-a]pyridin-7-yl)acetamide (Example 429),
N-(1,3-benzoxazol-5-yl)-2-[3-(4,4-dimethylcyclohex-1-en-1-yl)-6-oxopyridazin-1(6H)-yl]acetamide (Example 445),
2-[6-oxo-3-(spiro[2.5]oct-5-en-6-yl)pyridazin-1(6H)-yl]-N-([1,2,4]triazolo[1,5-a]pyridin-7-yl)acetamide (Example 512),
2-{2-oxo-2- [4-(pyridazin-4-yl)-2,3-dihydro-1H-indol-1-yl]ethyl}-6-(spiro[2.5]oct-5-en-6-yl) pyridazin-3(2H)-one (Example 526),
N-(1,3-benzoxazol-5-yl) -2-[3-(4-methylcyclohex-1-en-1-yl) -6-oxopyridazin-1(6H)-yl]acetamide (Example 531),
2-{3-[(4S)-4-methylcyclohex-1-en-1-yl] -6-oxopyridazin-1(6H)-yl}-N-([1,2,4]triazolo[1,5-a]pyridin-7-yl)acetamide (Example 537),
2-{3-[(4R)-4-methylcyclohex-1-en-1-yl] -6-oxopyridazin-1(6H)-yl}-N-([1,2,4]triazolo[1,5-a]pyridin-7-yl)acetamide (Example 538),
2-{3- [(4S)-4-methylcyclohex-1-en-1-yl]-6-oxopyridazin-1(6H)-yl}-N-([1,2,4]triazolo[1,5-a]pyridin-6-yl)acetamide (Example 539),

2-{3-[(4R)-4-methylcyclohex-1-en-1-yl]-6-oxopyridazin-1(6H)-yl}-N-([1,2,4]triazolo[1,5-a]pyridin-6-yl)acetamide (Example 540),
N-(6-cyanopyridin-3-yl)-2-[3-(4,4-difluoro-6-azaspiro[2.5]octan-6-yl)-6-oxopyridazin-1(6H)-yl]acetamide (Example 560), and
6-[cyclopentyl(methyl)amino]-2-{2-[4-(morpholin-4-yl)-2,3-dihydro-1H-indol-1-yl] -2-oxoethyl}pyridazin-3(2H)-one (Example 565).

(Item 34) The medicament of Item 5, wherein the Nav1.1 activator is any one compound selected from the group consisting of the following compounds:

2-{6-[cyclopentyl(methyl)amino]-1H-pyrazolo[3,4-b]pyrazin-1-yl}-N-([1,2,4]triazolo[1,5-a]pyridin-7-yl)acetamide (Example 593),
2-[6-(4-methylcyclohex-1-en-1-yl)-1H-pyrazolo[3,4-b]pyrazin-1-yl]-N-([1,2,4]triazolo[1,5-a]pyridin-7-yl)acetamide (Example 577),
2-[6-(4,4-difluoro-6-azaspiro[2.5]octan-6-yl)-1H-pyrazolo[3,4-b]pyrazin-1-yl]-N-([1,2,4]triazolo[1,5-a]pyridin-7-yl)acetamide (Example 609),
2-{6-[methyl(spiro[2.3]hexan-5-yl)amino]-1H-pyrazolo[3,4-b]pyrazin-1-yl}-N-([1,2,4]triazolo[1,5-a]pyridin-7-yl)acetamide (Example 630),
2-{6-[(3,3-dimethylcyclobutyl)(methyl)amino] -1H-pyrazolo[3,4-d]pyrazin-1-yl}-N-([1,2,4]triazolo[1,5-a]pyridin-7-yl)acetamide (Example 632),
2-{6-[(3,3-dimethylcyclobutyl) (methyl)amino] -1H-pyrazolo[3,4-d]pyrazin-1-yl}-N-([1,2,4]triazolo[1,5-a]pyridin-6-yl)acetamide (Example 668),
N-(6-cyanopyridin-3-yl)-2-[6-(4,4-difluoro-6-azaspiro[2.5]octan-6-yl)-1H-pyrazolo[3,4-b]pyrazin-1-yl]acetamide (Example 746),
N-(6-cyanopyridin-3-yl)-2-{6-[methyl(spiro[2.3]hexan-5-yl)amino] -1H-pyrazolo[3,4-b]pyrazin-1-yl}acetamide (Example 753),
2-{6-[cyclopentyl(methyl)amino]-1H-pyrazolo[3,4-d]pyrimidin-1-yl}-N-([1,2,4]triazolo[1,5-a]pyridin-7-yl)acetamide (Example 795),
2-{6-[(cyclobutylmethyl)(methyl)amino]-1H-pyrazolo[3,4-d]pyrimidin-1-yl}-N-([1,2,4]triazolo[1,5-a]pyridin-6-yl)acetamide (Example 819),
2-{6-[(3,3-dimethylcyclobutyl)(methyl)amino]-1H-pyrazolo[3,4-d]pyrimidin-1-yl}-N-([1,2,4]triazolo[1,5-a]pyridin-6-yl)acetamide (Example 821), and
an optically active form of 2-[6-(4-methylcyclohex-1-en-1-yl)-1H-pyrazolo[3,4-b]pyrazin-1-yl]-N-([1,2,4]triazolo[1,5-a]pyridin-7-yl)acetamide (Example 895 or 896).

(Item 35) The medicament of any one of Items 1 to 34, wherein
the tauopathy is Alzheimer's disease, Alzheimer-type dementia, diffuse neurofibrillary tangles with calcification, amyotrophic lateral sclerosis/parkinsonism-dementia complex of Guam island, amyotrophic lateral sclerosis/parkinsonism-dementia complex of the Kii peninsula, frontotemporal lobar degeneration (including Pick's disease, progressive supranuclear palsy, corticobasal degeneration, argyrophilic grain dementia, globular glial tauopathy, and frontotemporal dementia and parkinsonism linked to chromosome 17), senile dementia of the neurofibrillary tangle type, Down syndrome, chronic traumatic encephalopathy, myotonic dystrophy, Niemann-Pick disease type C, static encephalopathy of childhood with neurodegeneration in adulthood, PLA2G6-associated neurodegeneration, Gerstmann-Straussler-Scheinker disease, familial British dementia, familial Danish dementia, post-encephalitic Parkinsonism, subacute sclerosing panencephalitis, SLC9A6-related mental retardation, or a combination of any two or more of these diseases.
(Item 36) The medicament of any one of Items 1 to 34, wherein
the tauopathy is diffuse neurofibrillary tangles with calcification, amyotrophic lateral sclerosis/parkinsonism-dementia complex of Guam island, amyotrophic lateral sclerosis/parkinsonism-dementia complex of the Kii peninsula, frontotemporal lobar degeneration (including Pick's disease, progressive supranuclear palsy, corticobasal degeneration, argyrophilic grain dementia, globular glial tauopathy, and frontotemporal dementia and parkinsonism linked to chromosome 17), senile dementia of the neurofibrillary tangle type, Down syndrome, chronic traumatic encephalopathy, myotonic dystrophy, Niemann-Pick disease type C, static encephalopathy of childhood with neurodegeneration in adulthood, PLA2G6-associated neurodegeneration, Gerstmann-Straussler-Scheinker disease, familial British dementia, familial Danish dementia, post-encephalitic Parkinsonism, subacute sclerosing panencephalitis, SLC9A6-related mental retardation, or a combination of any two or more of these diseases.
(Item 37) The medicament of any one of Items 1 to 36, which can decrease phosphorylated tau aggregation and/or inhibit cerebral atrophy.

(Item 38) A method for decreasing phosphorylated tau aggregation and/or inhibiting cerebral atrophy, comprising administering a Nav activator.

(Item 39) A method for treating and/or preventing tauopathy, comprising administering a therapeutically effective amount of a Nav activator to a patient in need thereof.

(Item 40) Use of a Nav activator in the manufacture of a medicament for treating and/or preventing tauopathy.

(Item 41) A Nav activator in use for treating and/or preventing tauopathy.

(Item 42) A combination of the medicament of any one of Items 1 to 37, and at least one medicament selected from another medicament for treating tauopathy, a medicament for treating Alzheimer-type dementia, antiepileptic agent, antipsychotic agent, antidepressive agent, anti-anxiety agent, Chinese herbal drug, sleep-inducing drug, antiparkinsonian agent, antihypertensive drug, anti-inflammatory agent, antidiabetic drug, antihyperlipidemic drug, anti-obesity agent, antiemetic drug, a medicament for treating dysphagia, a medicament for treating dysuria, and cathartic drug.

(Item 43) The medicament of any one of Items 1 to 37, which is used in combination with at least one medicament selected from another medicament for treating tauopathy, a medicament for treating Alzheimer-type dementia, antiepileptic agent, antipsychotic agent, antidepressive agent, anti-anxiety agent, Chinese herbal drug, sleep-inducing drug, antiparkinsonian agent, antihypertensive drug, anti-inflammatory agent, antidiabetic drug, antihyperlipidemic drug, anti-obesity agent, antiemetic drug, a medicament for treating dysphagia, a medicament for treating dysuria, and cathartic drug.

(Item 44) A method for treating and/or preventing tauopathy, comprising administering a therapeutically effective amount of the medicament of any one of Items 1 to 37 in combination with at least one medicament selected from another medicament for treating tauopathy, a medicament for treating Alzheimer-type dementia, antiepileptic agent, antipsychotic agent, antidepressive agent, anti-anxiety agent, Chinese herbal drug, sleep-inducing drug, antiparkinsonian agent, antihypertensive drug, anti-inflammatory agent, antidiabetic drug, antihyperlipidemic drug, anti-obesity agent, antiemetic drug, a medicament for treating dysphagia, a medicament for treating dysuria, and cathartic drug, to a patient in need thereof.

(Effect of the Invention)

The present invention can decrease the phosphorylated tau aggregation and/or inhibit the cerebral atrophy, by activating the voltage-gated sodium channel. Thereby, the present invention can provide a medicament for treating and/or preventing tauopathy accompanied with various symptoms including cognitive dysfunction, comprising a Nav activator as an active ingredient.

BRIEF DESCRIPTION OF THE DRAWINGS

Fig. 1 shows the evaluation results of tau PET in rTg4510 mice when the compound of Example 1 was administered in Test 5, and shows SUVR average images and typical T2-weighted images of a corresponding positions.

Fig. 2 shows the evaluation results of the volume change in each brain region when the compound of Example 1 was administered in Test 5.

Fig. 3 shows the evaluation results of SUVR in each brain region when the compound of Example 1 was administered in Test 5.

Fig. 4 shows the evaluation results of tau PET in rTg4510 mice when the compound of Example 795 was administered in Test 5, and shows SUVR average images at each evaluation time point corresponding to the brain region shown by the T2-weighted image.

Fig. 5 shows the evaluation results of SUVR in each brain region when the compound of Example 795 was administered in Test 5.

Fig. 6 shows the evaluation results of tau PET in rTg4510 mice when the compound of Example 560 was administered in Test 5, and shows SUVR average images at each evaluation time point corresponding to the brain region shown by the T2-weighted image.

Fig. 7 shows the evaluation results of SUVR in each brain region when the compound of Example 560 was administered in Test 5.

DESCRIPTION OF EMBODIMENTS

Hereinafter, the present invention is explained in detail.

The "Nav activator" used herein is a medicament for activating the function of voltage-gated sodium channels,

which means a medicament that acts on voltage-gated sodium channels and increases the amount of sodium current resulting from them. The "Nav activator" may be an agent that can activate any of Nav1.1 - Nav1.9 which are nine subtypes of voltage-gated sodium channels. In an embodiment, the "Nav activator" is a substance that can activate Nav expressed in the brain. It includes, for example, Nav1.1 activator, Nav1.2 activator, Navl.3 activator, and Nav1.6 activator. And, the "Nav activator" also includes selective Nav activators that selectively activate one Nav subtype (e.g., selective Nav1.1 activators, selective Nav1.2 activators and selective Navl.6 activators, etc.), dual activators that activate two Nav subtypes (e.g., Nav1.1/Nav1.2 activators and Nav1.1/Nav1.6 activators, etc.), triple activators that activate three Nav subtypes (e.g., Nav1.1/Nav1.2/Nav1.6 activators, etc.), and multiple activators that activate four or more Nav subtypes. Examples of the "Nav activator" include medicaments that increase the amount of sodium current resulting from any subtype of Nav by 50%, preferably at 10 μM, and more preferably at 1 μM. The amount of sodium current resulting from each subtype can be measured according to the method described in Example 1.

The "Nav1.1 activator" means a medicament for activating Nav1.1, which includes a medicament for selectively activating Nav1.1 and a medicament for activating both of Nav1.1 and other Nav subtypes. When activating both Nav1.1 and other Nav subtypes, it is not necessary to activate Nav1.1 the most strongly, and medicaments that activate other Nav subtypes more strongly are also included in the "Nav1.1 activator". The "Nav1.1 activator" includes, for example, selective Nav1.1 activators, Nav1.1/Nav1.2 dual activators, Nav1.1/Navl.3 dual activators, Nav1.1/Nav1.6 dual activators, and Nav1.1/Nav1.2/Nav1.6 triple activators, but should not be limited thereto. The "Nav1.1 activator" can activate the function of Nav1.1 by acting on Nav1.1, and increase the sodium current resulting from Nav1.1. As a result, the nerve firing ability of cells expressing Nav1.1 is increased, and the function of GABAergic neurons is enhanced. Examples of the "Nav1.1 activator" include medicaments that increase the amount of sodium current resulting from Nav1.1 by 50%, preferably at 10 μM, and more preferably at 1 μM. The amount of sodium current resulting from Nav1.1 can be measured according to the method described in Example 1.

The "Nav activator" or "Nav1.1 activator" includes modality such as small molecule compounds, peptides and antibodies. The "Nav activator" or "Nav1.1 activator" includes preferably small molecule compounds and peptides, more preferably small molecule compounds.

The "small molecule compound" used herein means an organic compound whose molecular weight is 1000 or less. Examples of the atoms constituting the "small molecule compound" include carbon atom, hydrogen atom, oxygen atom, nitrogen atom, sulfur atom and halogen atom, but are not limited thereto. It is hopeful that the "small molecule compound" has one or more aromatic hetero ring comprising one of more functional group and/or atom, wherein said functional group is represented by alcohol, ether, ketone, carboxylic acid, ester, amine, amide, imide, carbamate, urea, thiol, sulfide, sulfoxide, sulfonic acid, sulfone, sulfone amide and the like, and said atom is oxygen atom, nitrogen atom, or sulfur atom. The "small molecule compound" includes, preferably an organic compound having one or more functional group selected from the group consisting of alcohol, ether, ester, amine, amide, imide, carbamate, urea, sulfone, and sulfonamide, and more preferably an organic compound having one or more aromatic hetero ring comprising one of more functional group selected from the group consisting of alcohol, ether, ester, amine, amide, imide, carbamate, urea, sulfone, and sulfonamide; and one or more nitrogen atom. And, the "small molecule compound" includes preferably an organic compound having a molecular weight of 100 to 800, more preferably 150 to 600, still more preferably 150 to 500, and particularly preferably 200 to 500.

The "small molecule compound" can be identified by various physical characteristic parameters, besides the above-mentioned parameters. In an embodiment, the "small molecule compound" is identified by the lipophilicity (LogP) expressed by the partition coefficient of octanol/water. For example, the LogP of the "small molecule compound" includes preferably -10 to 10, more preferably -8 to 8, even more preferably -5 to 5, and particularly preferably -2 to 5. In another embodiment, the "small molecule compound" is also identified by the number of hydrogen bond acceptor (HBA) or hydrogen bond donor (HBD) contained within the molecule. For example, the number of HBA contained within the molecule in the "small molecule compound" includes preferably 20 or less, more preferably 15 or less, and even more preferably 10 or less; and the number of HBD contained within the molecule in the "small molecule compound" includes preferably 10 or less, more preferably 8 or less, and even more preferably 5 or less. In further another embodiment, the "small molecule compound" is also identified by the acid dissociation constant (pKa) which represents the acid strength of the compound. The pKa of the "small molecule compound" includes preferably -5 to 10, more preferably -2 to 8, and even more preferably 0 to 5. In further another embodiment, the "small molecule compound" is also identified by the number of rotatable bond (RB) contained within the molecule. The number of RB of the "small molecule compound" includes preferably 30 or less, more preferably 20 or less, even more preferably 10 or less. In a preferred embodiment, any two or more of these physical characteristic parameters may be combined. The "small molecule compound" having one or more of these physical characteristic parameters has physical characteristics suitable for development as a pharmaceutical, and the high druggability thereof can be expected.

The "Peptide" used herein means a compound in which two or more amino acids are linked via an amide bond, which should not be limited by the number of amino acids. The "peptide" includes preferably a peptide consisting of 2 to 200 amino acids, more preferably a peptide consisting of 5 to 100 amino acids, and further preferably a peptide

consisting of 5 to 50 amino acids. The amino acid may be a natural amino acid or an unnatural amino acid. And, the "peptide" also includes derivatives obtained by chemically modifying a "peptide".

The "antibody" used herein is not limited as long as it is an antibody that specifically binds to a voltage-gated sodium channel, and it may be a human antibody, a mouse antibody, or a chimeric antibody. And, the "antibody" also includes a small molecule antibody such as one consisting of a Fab region and one consisting of a Fab region and a hinge portion.

The present specification may denote the number of carbon atoms in definitions of "substituents" in formula (1) as, for example, "$C_{1-6}$". Specifically, the term "$C_{1-6}$ alkyl" is synonymous with alkyl having 1 to 6 carbon atoms.

The "halogen atom" includes, for example, fluorine atom, chlorine atom, bromine atom, and iodine atom.

The "$C_{1-6}$ alkyl" means a straight- or branched-chain saturated hydrocarbon group having 1 to 6 carbon atoms. It is preferably "$C_{1-4}$ alkyl". The "$C_{1-6}$ alkyl" includes, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

The "$C_{1-10}$ alkyl" means a straight- or branched-chain saturated hydrocarbon group having 1 to 10 carbon atoms. It is preferably "$C_{1-6}$ alkyl". The "$C_{1-10}$ alkyl" includes, for example, heptyl, 2-methyl-3,3-dimethylbutyl, octyl, 2,4-dimethylhexyl, nonyl, decyl, and 4-ethyl-2-methylheptyl, besides the examples listed in the said "$C_{1-6}$ alkyl".

The "$C_{2-7}$ alkylcarbonyl" means a carbonyl group substituted with the above "$C_{1-6}$ alkyl". It is preferably "$C_{2-4}$ alkylcarbonyl". The "$C_{2-7}$ alkylcarbonyl" includes, for example, methylcarbonyl, ethylcarbonyl, normal-propylcarbonyl, and isopropylcarbonyl.

The "$C_{1-6}$ alkoxy" means oxy group substituted with the above-mentioned "$C_{1-6}$ alkyl", and the "$C_{1-6}$ alkyl" part in "$C_{1-6}$ alkoxy" is as defined in the above-mentioned "$C_{1-6}$ alkyl". It is preferably "$C_{1-4}$ alkoxy". The "$C_{1-6}$ alkoxy" includes, for example, methoxy, ethoxy, propoxy, isopropoxy, butoxy, isobutoxy, sec-butoxy, and tert-butoxy.

The "$C_{1-10}$ alkoxy" means oxy group substituted with the above-mentioned "$C_{1-10}$ alkyl", and the "$C_{1-10}$ alkyl" part in "$C_{1-10}$ alkoxy" is as defined in the above-mentioned "$C_{1-10}$ alkyl". It is preferably "$C_{1-6}$ alkoxy". The "$C_{1-10}$ alkoxy" includes, for example, heptyloxy, 2-methyl-3,3-dimethylbutoxy, octyloxy, 2,4-dimethylhexyloxy, nonyloxy, decyloxy, and 4-ethyl-2-methylheptyloxy, besides the examples listed in the said "$C_{1-6}$ alkoxy".

The "$C_{2-7}$ alkoxycarbonyl" means carbonyl group substituted with the above-mentioned "$C_{1-6}$ alkoxy". It is preferably "$C_{2-5}$ alkoxycarbonyl". The "$C_{2-7}$ alkoxycarbonyl" includes, for example, methoxycarbonyl, ethoxycarbonyl, propoxycarbonyl, isopropoxycarbonyl, butoxycarbonyl, isobutoxycarbonyl, sec-butoxycarbonyl, and tert-butoxycarbonyl.

The "$C_{2-10}$ alkenyl" means a straight- or branched-chain unsaturated hydrocarbon group having 1 to 3 carbon-carbon double bonds and 2 to 10 carbon atoms. It is preferably "$C_{2-6}$ alkenyl". The "$C_{2-6}$ alkenyl" includes, for example, ethenyl, propenyl, butenyl, pentenyl, and hexenyl.

The "$C_{2-10}$ alkynyl" means a straight- or branched-chain unsaturated hydrocarbon group having 1 to 3 carbon-carbon triple bonds and 2 to 10 carbon atoms. It is preferably "$C_{2-6}$ alkynyl". The "$C_{2-10}$ alkynyl" includes, for example, ethynyl, propynyl, butynyl, pentynyl, and hexynyl.

The "$C_{3-10}$ cycloalkyl" means cyclic saturated or partially-unsaturated hydrocarbon group having 3 to 10 carbon atoms, which may have a bridged structure or a spiro structure. The "$C_{3-10}$ cycloalkyl" includes preferably "$C_{3-6}$ cycloalkyl". The "$C_{3-6}$ cycloalkyl" includes, for example, cyclopropyl, cyclobutyl, cyclopentyl, and cyclohexyl. The "$C_{3-10}$ cycloalkyl" includes, for example, cycloheptyl, cyclooctyl, cyclononyl, cyclodecyl, and adamanthyl, besides the examples listed in the said "$C_{3-6}$ cycloalkyl".

The "saturated or partially-unsaturated $C_{3-7}$ carbocyclyl" means a 3- to 7-membered monocyclic or polycyclic saturated or partially-unsaturated hydrocarbon group. It is preferably "saturated or partially-unsaturated $C_{5-7}$ carbocyclyl". The "saturated or partially-unsaturated $C_{3-7}$ carbocyclyl" includes, for example, cyclopropyl, cyclobutyl, cyclopentyl, cyclohexyl, cyclopentenyl, and cyclohexenyl.

The "saturated or partially-unsaturated $C_{4-12}$ carbocyclyl" means a 4- to 12-membered monocyclic or polycyclic saturated or partially-unsaturated hydrocarbon group. It is preferably "saturated or partially-unsaturated $C_{4-6}$ carbocyclyl". The "saturated or partially-unsaturated $C_{4-12}$ carbocyclyl" includes, for example, cyclooctyl, cyclodecyl, and cyclododecyl, besides those listed as examples of the above "saturated or partially-unsaturated $C_{3-7}$ carbocyclyl".

The above "saturated or partially-unsaturated $C_{4-12}$ carbocyclyl" also includes fused or bridged, saturated or partially-unsaturated bicyclic groups and saturated or partially-unsaturated spiro groups. Examples thereof include groups of the following formulae:

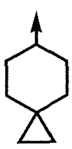 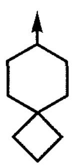 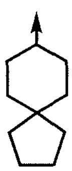 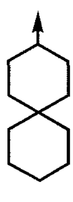 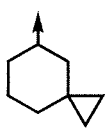 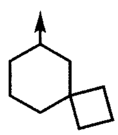 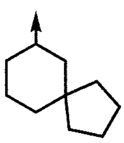 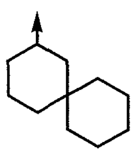 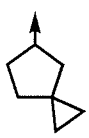 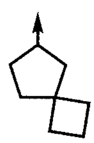

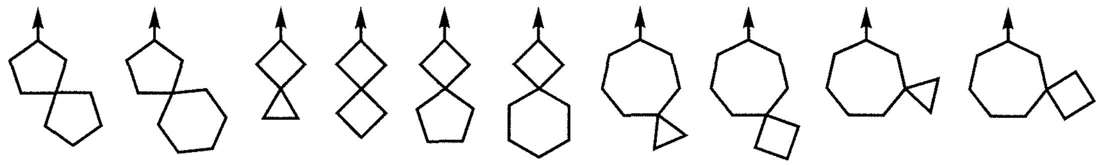

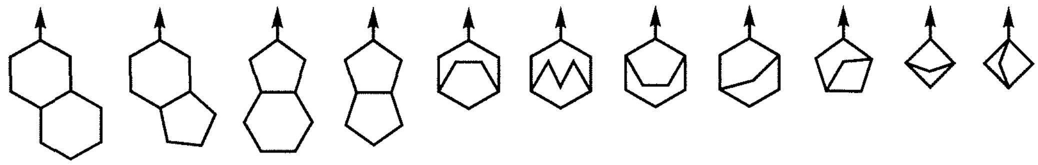

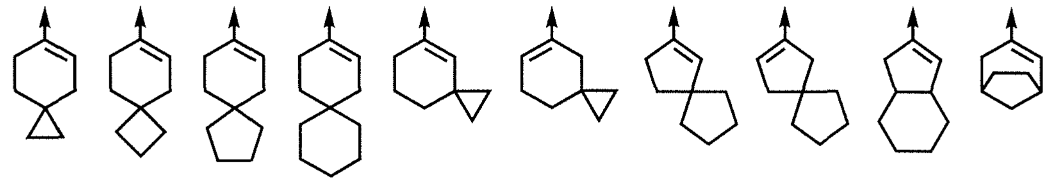

The term "5- or 6-membered saturated or partially-unsaturated carbocyclyl" means a 5- or 6-membered monocyclic saturated or partially-unsaturated hydrocarbon group. The "5- or 6-membered saturated or partially-unsaturated carbocyclyl" includes, for example, cyclopentyl, cyclohexyl, cyclopentenyl, and cyclohexenyl.

The "5- to 7-membered saturated or partially-unsaturated carbocycle" means a monocyclic or bicyclic saturated or partially-unsaturated hydrocarbon group having 5 to 7 carbon atoms, and includes structures having partially-unsaturated bond(s), structures having fused ring(s), structures having bridged structure(s), and structures forming spiro ring(s). The "5- to 7-membered saturated or partially-unsaturated carbocycle" includes, for example, cyclopentane, cyclohexane, cycloheptane, cyclopentene, cyclohexene, cycloheptene, cyclohexadiene, and cycloheptadiene.

The term "3- to 6-membered saturated carbocyclic ring" means a saturated hydrocarbon ring having 3 to 6 carbon atoms, and also includes structures forming spiro ring(s). The "3-to 6-membered saturated carbocyclic ring" includes, for example, cyclopropane, cyclobutane, cyclopentane, and cyclohexane.

The "5- or 6-membered heteroaryl" means a 5- or 6-membered aromatic heterocyclyl which comprises the same or different one or more (for example, 1 to 4) heteroatoms selected from nitrogen atom, sulfur atom, and oxygen atom, and which may be optionally substituted with oxo. The "5- or 6-membered heteroaryl" include groups of the following formulae:

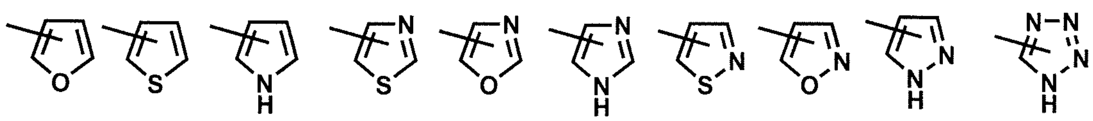

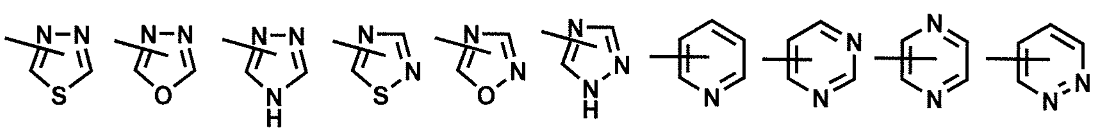

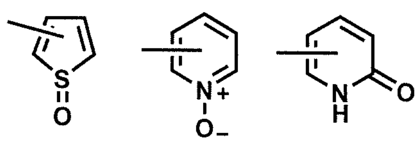

It is preferably imidazolyl, oxazolyl, thiazolyl, pyrazolyl, isoxazolyl, isothiazolyl, triazolyl, oxadiazolyl, pyridyl, pyrimidyl, pyrazinyl, or pyridazinyl; and it is more preferably imidazolyl, oxazolyl, thiazolyl, pyrazolyl, triazolyl, oxadiazolyl, pyridyl, pyrimidyl, pyrazinyl, or pyridazinyl.

The "$C_{6-10}$ aryl" means aromatic hydrocarboncyclyl having 6 to 10 carbon atoms. The "$C_{6-10}$ aryl" includes, for example, phenyl, 1-naphthyl, and 2-naphthyl. It includes preferably phenyl.

The "$C_{6-10}$ aryl" also encompasses bicyclic compounds, *i.e.*, $C_{6-10}$ aryl fused with $C_{4-6}$ cycloalkyl or 5- or 6-membered saturated heterocyclyl. The bicyclic "$C_{6-10}$ aryl" includes, for example, the following groups:

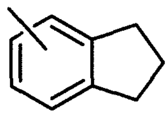 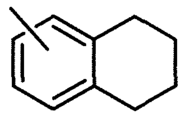 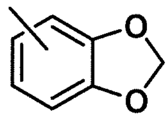 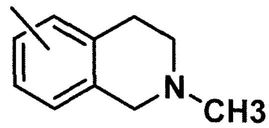

The "$C_{6-10}$ aryloxy" means oxy group substituted with the above-mentioned "$C_{6-10}$ aryl". The "$C_{6-10}$ aryl" includes, for example, phenyloxy and naphthyloxy, and preferably phenyloxy.

The term "5- to 10-membered heteroaryl" includes, for example, a 5- to 10-membered monocyclic or 9- or 10-membered bicyclic aromatic heterocyclyl. The "5- to 10-membered heteroaryl" group comprises the same or different one or more (for example, 1 to 4) heteroatoms selected from nitrogen atom, sulfur atom, and oxygen atom, and may be optionally substituted with oxo. The bicyclic heteroaryl group also includes fused structures of the above monocyclic heteroaryl with an aromatic ring (such as benzene and pyridine) or a non-aromatic ring (such as cyclohexane and piperidine). The "5- to 10-membered heteroaryl" includes, for example, groups of the following formulae:

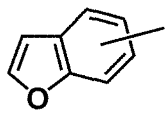 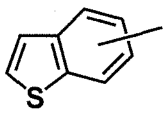 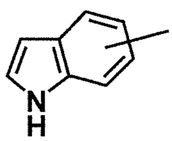 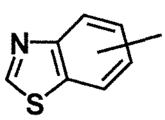 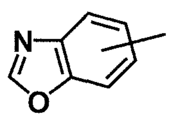 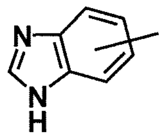

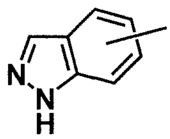 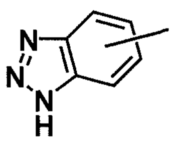 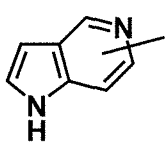 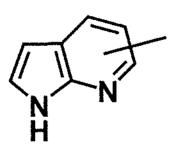 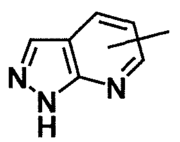 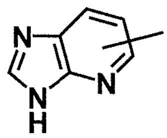

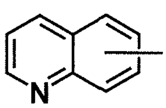 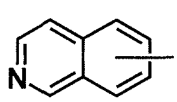 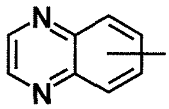 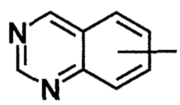 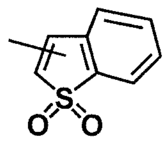 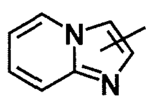

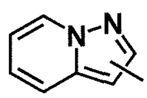 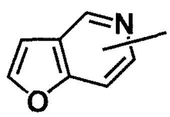

The "5- to 10-membered heteroarylene" means a divalent group of the "5- to 10-membered heteroaryl", which has the same or different one or more (for example, 1 to 4) heteroatom and may be optionally substituted with oxo. In case of 9- or 10-membered bicyclic aromatic heterocyclyl, the bond may be attached to either ring if it is substitutable. The "5- to 10-membered heteroarylene" includes, for example, groups of the following formulae:

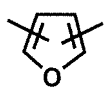 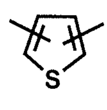 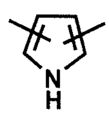 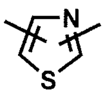 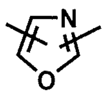 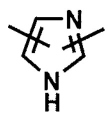 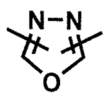 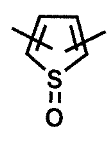

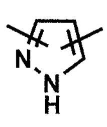 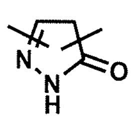 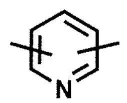 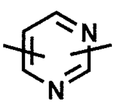 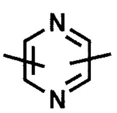 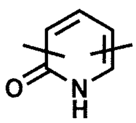 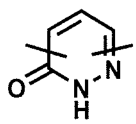 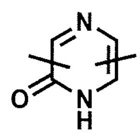

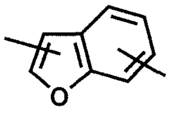 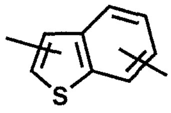 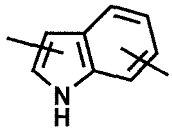 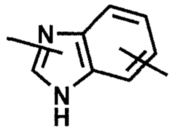 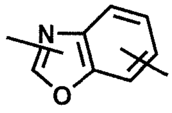 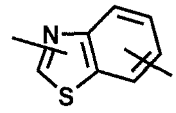

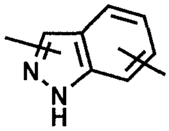 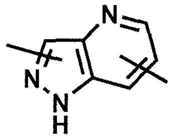 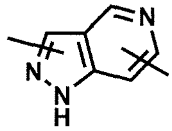 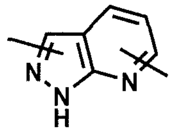 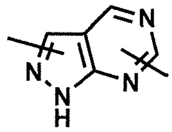 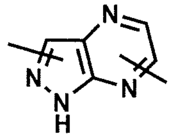

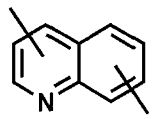 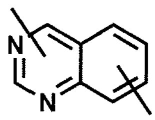 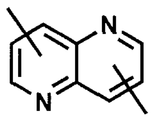 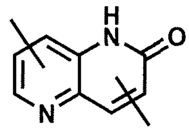 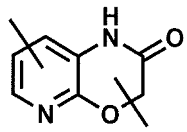

The "5- to 10-membered heteroaryloxy" means an oxy group substituted with the above "5- to 10-membered heteroaryl". The "5- to 10-membered heteroaryloxy" includes, for example, pyridyloxy, imidazolyloxy, and furyloxy, and preferably pyridyloxy.

In the present specification, a bond across a ring means that a "group" having the bond is attached at a substitutable position of the ring. For example, a heteroaryl group of the following formula:

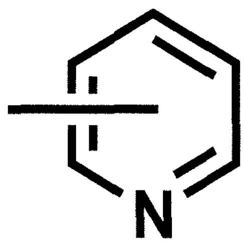

denotes 2-pyridyl, 3-pyridyl, or 4-pyridyl.

When a bond crossing a ring is drawn from a fused ring in the present description, the bond may be attached to at any substitutable site of either ring. For example, the heteroaryl of the following formula:

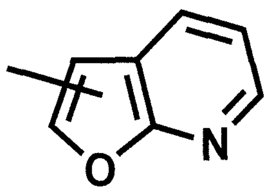

means any one group of the following groups:

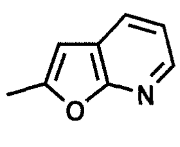 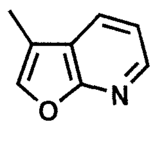 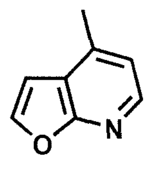 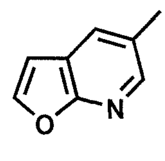 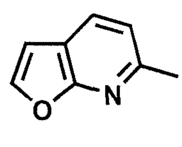 .

The term "4- to 7-membered saturated or partially-unsaturated heterocyclyl" includes, for example, a 4- to 7-membered monocyclic or polycyclic saturated or partially-unsaturated heterocyclyl comprising the same or different 1 to 2 atoms selected from nitrogen atom, oxygen atom, and sulfur atom. It is preferably "5- to 7-membered saturated or partially-unsaturated heterocyclyl". The "5- to 7-membered saturated or partially-unsaturated heterocyclyl" includes, for example, pyranyl, dihydropyranyl, tetrahydropyranyl, tetrahydrofuryl, dihydropyrrolyl, dihydrofuranyl, pyrrolidinyl, imidazolidinyl, piperidinyl, piperazinyl, dioxanyl, azepanyl, morpholinyl, and thiomorpholinyl. The "4- to 7-membered saturated

or partially-unsaturated heterocyclyl" includes, for example, azetidinyl and oxetanyl, besides those listed as examples of the above "5- to 7-membered saturated or partially-unsaturated heterocyclyl". Among them, the "4- to 7-membered saturated heterocyclyl" includes, for example, azetidinyl, oxetanyl, tetrahydropyranyl, tetrahydrofuryl, pyrrolidinyl, imidazolidinyl, piperazinyl, dioxanyl, azepanyl, morpholinyl, and thiomorpholinyl. Each group may be attached to a group via any of carbon atom(s) and nitrogen atom(s) that constitute a ring.

The term "4- to 12-membered saturated or partially-unsaturated heterocyclyl" includes, for example, a 4- to 12-membered monocyclic or polycyclic saturated or partially-unsaturated heterocyclyl comprising the same or different 1 to 3 atoms selected from nitrogen atom, oxygen atom, and sulfur atom. It is preferably a 4- to 10-membered saturated or partially-unsaturated heterocyclyl. The "4- to 12-membered saturated or partially-unsaturated heterocyclyl" includes, for example, azocanyl, 1,4-oxazocanyl, 1,5-oxazocanyl, 1,4-diazocanyl, 1,5-diazocanyl, besides those listed as examples of the above "4- to 7-membered saturated or partially-unsaturated heterocyclyl". Each group may be attached to a group via any of carbon atom(s) and nitrogen atom(s) that constitute a ring.

The term "4- to 7-membered saturated or partially-unsaturated heterocyclyl" or "4- to 12-membered saturated or partially-unsaturated heterocyclyl" includes a fused or bridged, saturated or partially-unsaturated bicyclic group and a saturated or partially-unsaturated spiro group. The "4- to 7-membered saturated or partially-unsaturated heterocyclyl" includes, for example, groups of the following formulae:

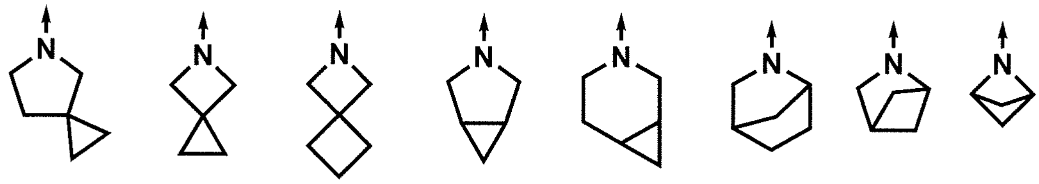

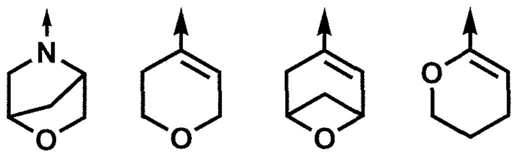

The "4- to 12-membered saturated or partially-unsaturated heterocyclyl" includes, for example, groups of the following formulae:

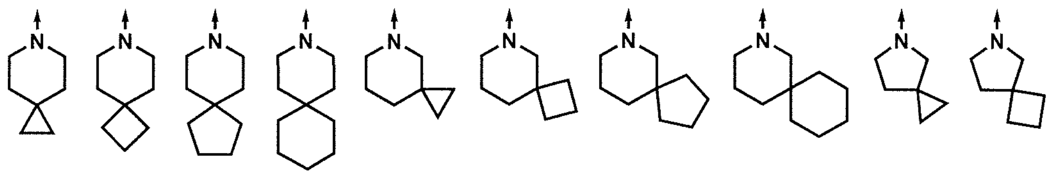

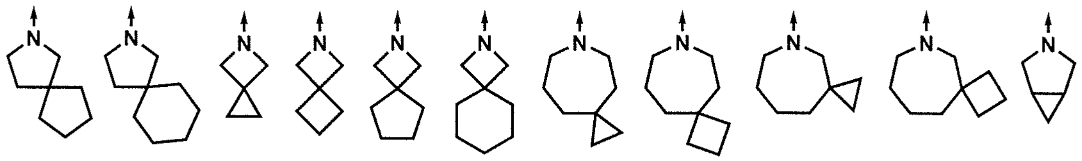

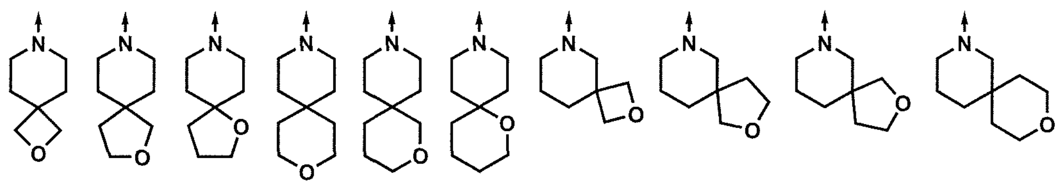

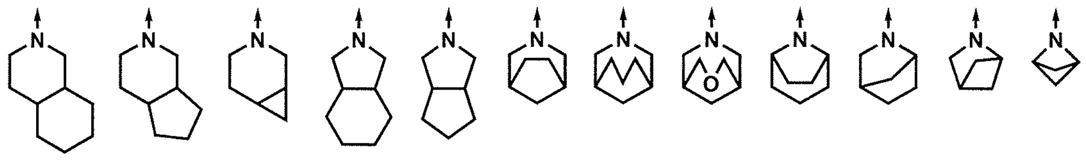

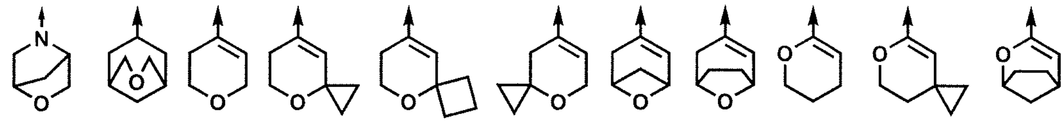

The term "nitrogen-containing saturated ring" means a saturated heterocyclic ring comprising one or more nitrogen atoms as ring components. The "nitrogen-containing saturated ring" includes, for example, azetidine, pyrrolidine, and piperidine.

The term "9- or 10-membered bicyclic heteroaromatic ring" means a bicyclic aromatic heterocyclic ring which consists of 9 or 10 atoms and comprises the same or different 1 to 3 heteroatoms selected from the group consisting of oxygen atom, nitrogen atom, and sulfur atom, and which may be optionally substituted with oxo. The oxygen atom (=O) and sulfur atom (=S) of carbonyl, sulfinyl, sulfonyl, and thiocarbonyl which compose the bicyclic heteroaromatic ring is not counted as ring members (i.e., the ring size) of the 9- or 10-membered ring nor as heteroatom(s) which compose the ring. The "9- or 10-membered bicyclic heteroaromatic ring" includes, for example, quinoline, isoquinoline, naphthyridine, quinazoline, quinoxaline, benzofuran, benzothiophene, indole, benzooxazole, benzoisooxazole, benzoimidazole, benzooxadiazole, benzothiadiazole, indolizine, benzofuran, indazole, pyrazolopyridine, imidazopyridine, triazolopyridine, imidazopyrimidine, imidazopyridazine, thiazolopyridine, pyrazolopyrimidine, triazolopyridazine, and furopyridine.

The term "3- to 6-membered saturated heterocyclic ring" means a monocyclic or bicyclic saturated heterocyclic ring which consists of 3 to 6 atoms and comprises the same or different 1 or 2 heteroatoms selected from the group consisting of oxygen atom, nitrogen atom, and sulfur atom. The saturated heterocyclic ring may be optionally substituted with oxo, and may comprise 1 or 2 carbonyl, thiocarbonyl, sulfinyl, or sulfonyl groups. The oxygen atom (=O) of carbonyl, sulfinyl, and sulfonyl is not counted as ring members (i.e., the ring size) of the 3- to 6-membered ring or as heteroatom(s) which compose the ring, and the sulfur atom (=S) of thiocarbonyl is not counted as ring members (i.e., the ring size) of the 3- to 6-membered ring or as heteroatom(s) which compose the ring. Preferably, the "3- to 6-membered saturated heterocyclic ring" includes "5- or 6-membered saturated heterocyclic ring". Examples of the "5- or 6-membered saturated heterocyclic ring" include, but not limited to, pyrrolidine, piperidine, piperazine, morpholine, tetrahydrofuran, and tetrahydropyran. The "3-to 6-membered saturated heterocyclic ring" includes, for example, aziridine and azetidine, besides those listed as examples of the above "5- or 6-membered saturated heterocyclic ring". Among them, the "6-membered saturated heterocyclic ring" includes, for example, piperidine, morpholine, and tetrahydropyran.

The "4- to 7-membered saturated or partially-unsaturated heterocyclyloxy" means an oxy group substituted with the above "4- to 7-membered saturated or partially-unsaturated heterocyclyl".

Examples of the group of formula (2c) comprising a 5-to 7-membered saturated or partially-unsaturated carbocyclic ring or heterocyclic ring which is formed by taking $R^5$ and $R^6$ together with the carbon atoms to which they attach, include groups of the following formulae:

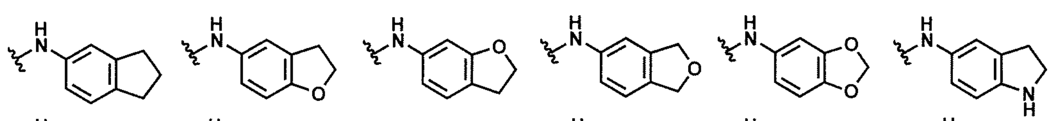

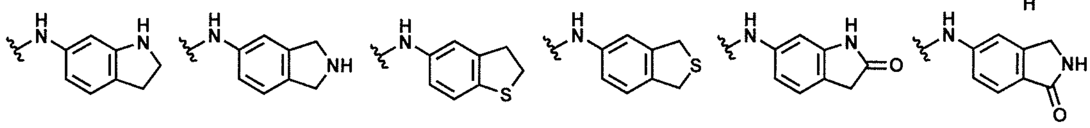

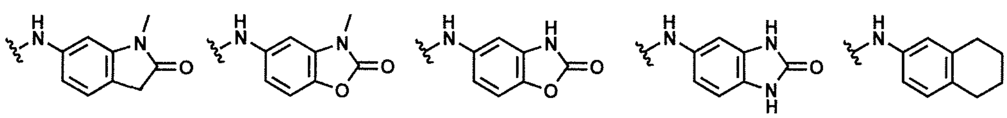

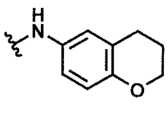 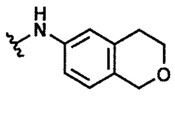 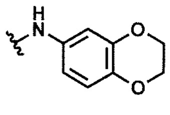 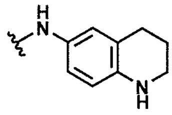 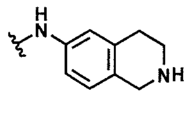

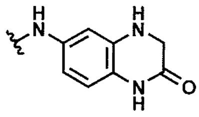 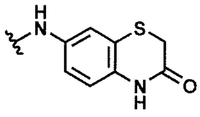 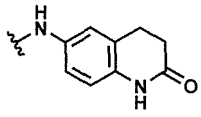 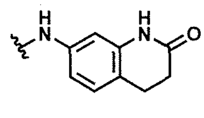 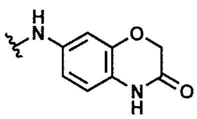

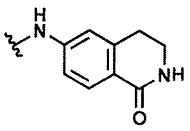 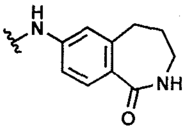 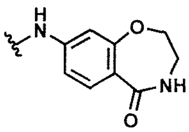 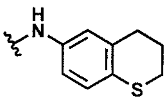 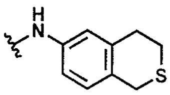

Examples of the group of formula (3) comprising a 3- to 6-membered saturated carbocyclic ring or 3- to 6-membered saturated heterocyclic ring which is formed by taking $R^a$ and $R^b$ together with the carbon atoms to which they attach, include groups of the following formulae:

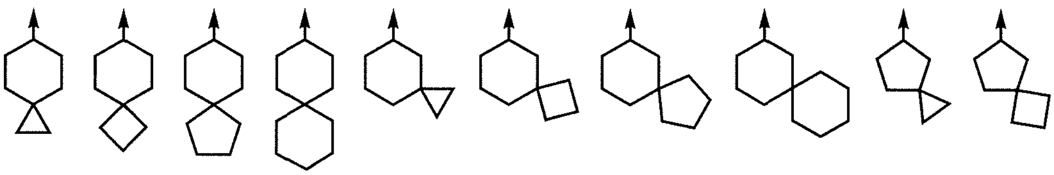

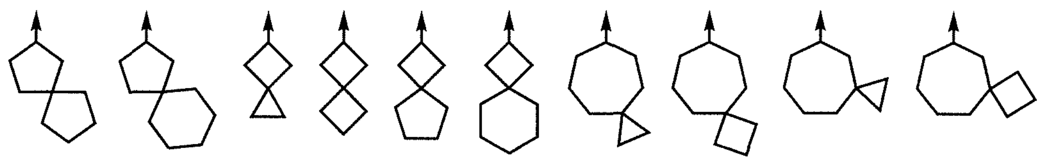

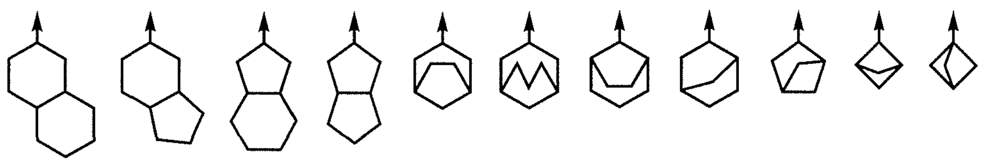

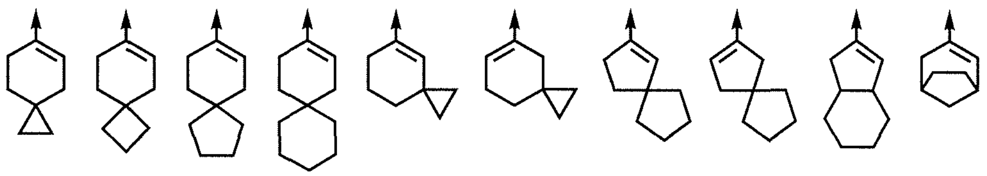

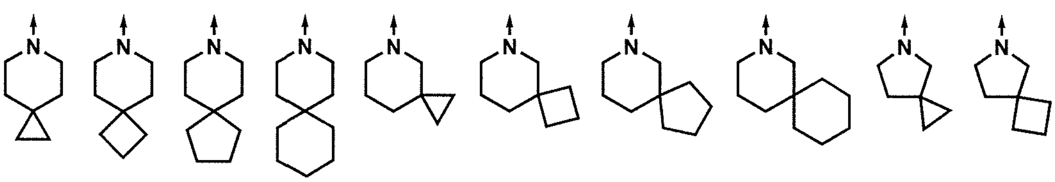

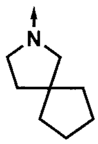 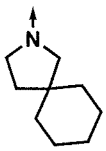 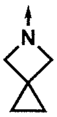 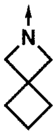 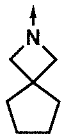 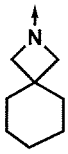 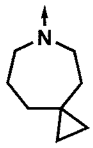 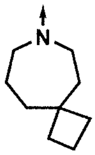 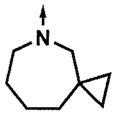 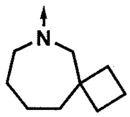 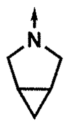

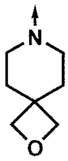 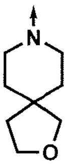 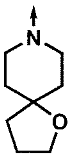 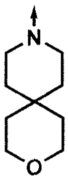 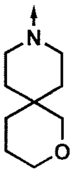 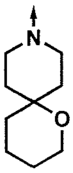 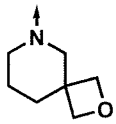 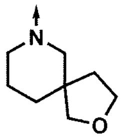 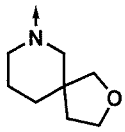 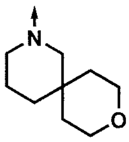 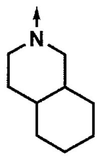

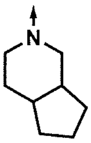 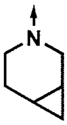 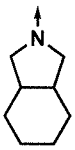 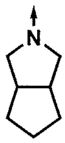 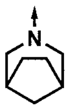 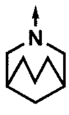 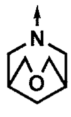 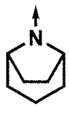 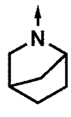 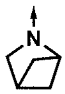 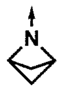 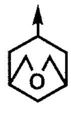

Examples of the group of formula (2a) or (2b) comprising a 9- or 10-membered bicyclic heteroaromatic ring include groups of the following formulae:

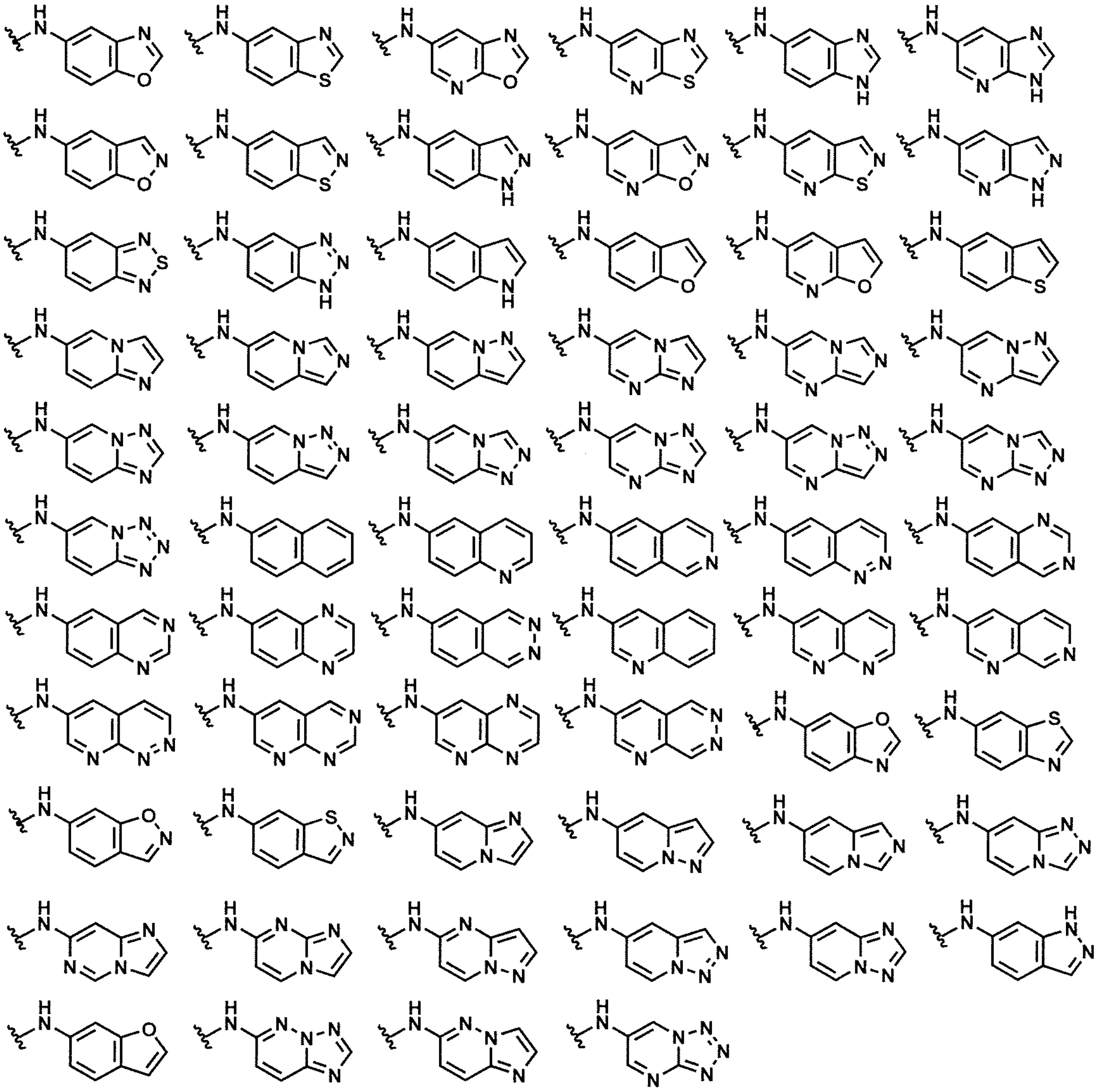

Among the present compounds of formula (1), specific embodiments of Ring Cy, $R^{1a}$, $R^{2a}$, $Y^1$, $Y^2$, $Y^3$, $R^{1b}$, $R^{2b}$, $M^1$, and $M^2$ are shown below, but the technical scope of the present invention shall not be limited to the scope of the following exemplary embodiments. The specific embodiments shown below may be optionally combined with each other as long as they do not contradict.

Ring Cy includes preferably 5- or 6-membered heteroarylene and 9- or 10-membered heteroarylene, more preferably a group of formula (a) or formula (b). An embodiment of Ring Cy includes 5- or 6-membered heteroarylene, and another embodiment thereof includes 9- or 10-membered heteroarylene. And, another embodiment of Ring Cy includes a group of formula (a), and further another embodiment includes a group of formula (b).

$R^{1a}$ and $R^{2a}$ preferably include, independently,

(1) hydrogen atom,
(2) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy,
(3) $C_{1-6}$ alkoxy which may be optionally substituted with the same or different 1 to 3 substituents selected from the group consisting of halogen atom, hydroxy, and $C_{1-6}$ alkoxy, or
(4) amino which may be optionally substituted with the same or different 1 to 2 substituents selected from the group consisting of $C_{1-6}$ alkyl which may be optionally substituted with the same or different 1 to 3 halogen atoms, saturated or partially-unsaturated $C_{3-7}$ carbocyclyl, and $C_{2-7}$ alkylcarbonyl.

Another preferred embodiment of $R^{1a}$ and $R^{2a}$ includes the case when they are taken together with the carbon atoms to which they are attached to form 5- to 7-membered saturated or partially-unsaturated carbon ring.

Preferably, $Y^1$, $Y^2$, and $Y^3$ include, independently N and $CR^{2b}$. More preferably, $Y^1$ is N, and $Y^2$ and $Y^3$ are independently N or $CR^{2b}$. In another embodiment, $Y^1$ is N, and $Y^2$ and $Y^3$ are $CR^{2b}$. In further another embodiment, $Y^1$ and $Y^2$ are N, and $Y^3$ is $CR^{2b}$. In another embodiment, $Y^1$ and $Y^3$ is N, and $Y^2$ is $CR^{2b}$. In further another embodiment, $Y^1$, $Y^2$, and $Y^3$ are $CR^{2b}$. In further another embodiment, $Y^1$, $Y^2$, and $Y^3$ are N.

Preferably, $R^{1b}$ includes hydrogen atom, halogen atom, cyano, $C_{1-6}$ alkyl which may be optionally substituted with the same or different 1 to 3 halogen atoms, and $C_{3-6}$ cycloalkyl which may be optionally substituted with the same or different 1 to 3 halogen atoms, more preferably hydrogen atom, halogen atom, cyano, $C_{1-6}$ alkyl, and $C_{3-6}$ cycloalkyl. More preferably, $R^{1b}$ includes hydrogen atom, halogen atom, and $C_{1-3}$ alkyl, especially preferably hydrogen atom and halogen atom, the most preferably hydrogen atom.

Preferably, $R^{2b}$ includes hydrogen atom, halogen atom, cyano, $C_{1-6}$ alkyl which may be optionally substituted with the same or different 1 to 3 halogen atoms, and $C_{3-6}$ cycloalkyl which may be optionally substituted with the same or different 1 to 3 halogen atoms, more preferably hydrogen atom, halogen atom, cyano, $C_{1-6}$ alkyl, and $C_{3-6}$ cycloalkyl. More preferably, $R^{2b}$ includes hydrogen atom, halogen atom, and $C_{1-3}$ alkyl, especially preferably hydrogen atom and halogen atom, the most preferably hydrogen atom.

Preferably, $M^1$ includes

(1) saturated or partially-unsaturated $C_{4-12}$ carbocyclyl which may be optionally substituted with the same or different 1 to 4 substituents selected from the group consisting of halogen atom and $C_{1-6}$ alkyl which may be optionally substituted with the same or different 1 to 3 substituents selected from the group consisting of halogen atom, hydroxy, and $C_{1-6}$ alkoxy,
(2) 4- to 12-membered saturated or partially-unsaturated heterocyclyl which may be optionally substituted with the same or different 1 to 4 substituents selected from the group consisting of halogen atom and $C_{1-6}$ alkyl which may be optionally substituted with the same or different 1 to 3 substituents selected from the group consisting of halogen atom, hydroxy, and $C_{1-6}$ alkoxy, and
(3) $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,
(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy.

More preferably, $M^1$ includes

(1) saturated or partially-unsaturated $C_{4-12}$ carbocyclyl which may be optionally substituted with the same or different 1 to 4 substituents selected from the group consisting of halogen atom and $C_{1-6}$ alkyl which may be optionally substituted with the same or different 1 to 3 halogen atoms,
(2) 4- to 12-membered saturated or partially-unsaturated heterocyclyl which may be optionally substituted with the same or different 1 to 4 substituents selected from the group consisting of halogen atom and $C_{1-6}$ alkyl which may be optionally substituted with the same or different 1 to 3 halogen atoms, and
(3) $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl.

An embodiment of $M^1$ includes a group of the following formula (3'):

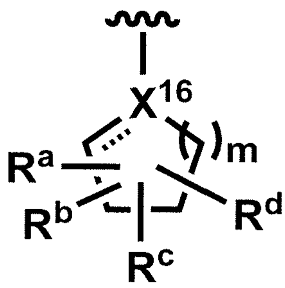

(3')

wherein

$X^{16}$ is N, C, or CH,
the bond having a broken line is a single bond or a double bond,
m is 0, 1, 2, or 3,
$R^a$, $R^b$, $R^c$, and $R^d$ are independently

(1-1) hydrogen atom,
(1-2) halogen atom,
(1-3) hydroxy,
(1-4) $C_{1-6}$ alkyl which may be optionally substituted with the same or different 1 to 3 substituents selected from the group consisting of halogen atom, hydroxy, and $C_{1-6}$ alkoxy,
(1-5) $C_{1-6}$ alkoxy which may be optionally substituted with the same or different 1 to 3 substituents selected from the group consisting of halogen atom, hydroxy, and $C_{1-6}$ alkoxy, or
(1-6) amino-carbonyl which may be substituted with $C_{1-6}$ alkyl which may be optionally substituted with the same or different 1 to 3 halogen atoms,

wherein $R^a$ and $R^b$ may be taken together with the carbon atom(s) to which they are attached to form

(2-1) 3- to 6-membered saturated carbocyclyl, wherein the ring may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy, or

(2-2) 3- to 6-membered saturated heterocyclyl which may be optionally substituted with the same or different 1 to 4 substituents selected from the group consisting of the above (a) - (d) in (2-1).

Another embodiment of $M^1$ includes a group of formula (3') wherein $X^{16}$ is C or N; m is 1 or 2; $R^a$ and $R^b$ are independently hydrogen atom, halogen atom, or $C_{1-6}$ alkyl which may be optionally substituted with the same or different 1 to 3 halogen atoms; and $R^c$ and $R^d$ are hydrogen atom.

Another embodiment of $M^1$ includes a group of following formula (3a):

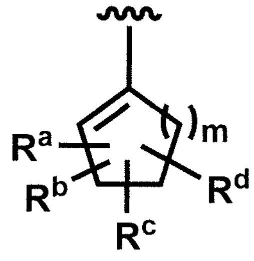

**(3a)**

wherein

m is 0, 1, 2, or 3,
$R^a$, $R^b$, $R^c$, and $R^d$ are independently

(1-1) hydrogen atom,
(1-2) halogen atom,
(1-3) hydroxy,
(1-4) $C_{1-6}$ alkyl which may be optionally substituted with the same or different 1 to 3 substituents selected from the group consisting of halogen atom, hydroxy, and $C_{1-6}$ alkoxy,
(1-5) $C_{1-6}$ alkoxy which may be optionally substituted with the same or different 1 to 3 substituents selected from the group consisting of halogen atom, hydroxy, and $C_{1-6}$ alkoxy, or
(1-6) amino-carbonyl which may be substituted with $C_{1-6}$ alkyl which may be optionally substituted with the same or different 1 to 3 halogen atoms,

wherein $R^a$ and $R^b$ may be taken together with the carbon atom(s) to which they are attached to form

(2-1) 3- to 6-membered saturated carbocyclyl, wherein the ring may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy, or

(2-2) 3- to 6-membered saturated heterocyclyl which may be optionally substituted with the same or different 1 to 4 substituents selected from the group consisting of the above (a) - (d) in (2-1).

Another embodiment of $M^1$ includes a group of formula (3b) :

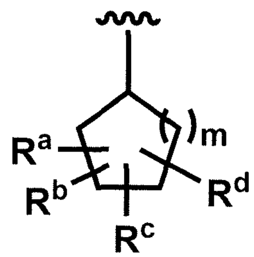

**(3b)**

wherein

m is 0, 1, 2, or 3,
$R^a$, $R^b$, $R^c$, and $R^d$ are independently

(1-1) hydrogen atom,
(1-2) halogen atom,
(1-3) hydroxy,
(1-4) $C_{1-6}$ alkyl which may be optionally substituted with the same or different 1 to 3 substituents selected from the group consisting of halogen atom, hydroxy, and $C_{1-6}$ alkoxy,
(1-5) $C_{1-6}$ alkoxy which may be optionally substituted with the same or different 1 to 3 substituents selected from the group consisting of halogen atom, hydroxy, and $C_{1-6}$ alkoxy, or
(1-6) amino-carbonyl which may be substituted with $C_{1-6}$ alkyl which may be optionally substituted with the same or different 1 to 3 halogen atoms,

wherein $R^a$ and $R^b$ may be taken together with the carbon atom(s) to which they are attached to form

(2-1) 3- to 6-membered saturated carbocyclyl, wherein the ring may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy, or

(2-2) 3- to 6-membered saturated heterocyclyl which may be optionally substituted with the same or different 1 to 4 substituents selected from the group consisting of the above (a) - (d) in (2-1).

Another embodiment of $M^1$ includes a group of formula (3c) :

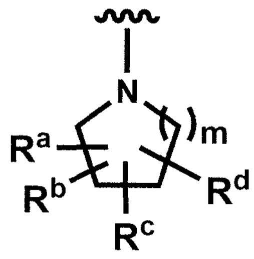

(3c)

wherein

m is 0, 1, 2, or 3,
$R^a$, $R^b$, $R^c$, and $R^d$ are independently

(1-1) hydrogen atom,
(1-2) halogen atom,
(1-3) hydroxy,
(1-4) $C_{1-6}$ alkyl which may be optionally substituted with the same or different 1 to 3 substituents selected from the group consisting of halogen atom, hydroxy, and $C_{1-6}$ alkoxy,
(1-5) $C_{1-6}$ alkoxy which may be optionally substituted with the same or different 1 to 3 substituents selected from the group consisting of halogen atom, hydroxy, and $C_{1-6}$ alkoxy, or
(1-6) amino-carbonyl which may be substituted with $C_{1-6}$ alkyl which may be optionally substituted with the same or different 1 to 3 halogen atoms,

wherein $R^a$ and $R^b$ may be taken together with the carbon atom(s) to which they are attached to form

(2-1) 3- to 6-membered saturated carbocyclyl, wherein the ring may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy, or

(2-2) 3- to 6-membered saturated heterocyclyl which may be optionally substituted with the same or different 1 to 4 substituents selected from the group consisting of the above (a) - (d) in (2-1).

Further embodiment of $M^1$ includes $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,
(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy.

Another preferred embodiment of $M^1$ includes a group of the following formula (3a-1), (3a-2), (3a-3), (3a-4), (3a-5), (3a-6), (3b-1), (3b-2), (3b-3), (3b-4), (3c-1), (3c-2), (3c-3), (3c-4), (3c-5), (3c-6), (3c-7), (3d-1), (3d-2), (3d-3), (3d-4), (3d-5), (3d-6), (3d-7), (3d-8), (3d-9), (3d-10), (3d-11), (3d-12), (3d-13), or (3d-14):

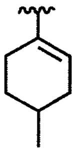
(3a-1)
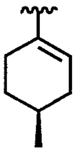
(3a-2)
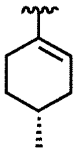
(3a-3)
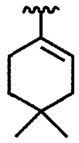
(3a-4)
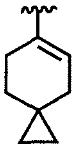
(3a-5)
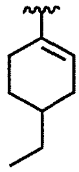
(3a-6)
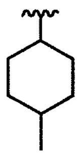
(3b-1)
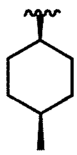
(3b-2)
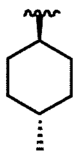
(3b-3)
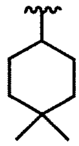
(3b-4)

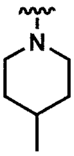
(3c-1)
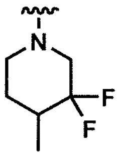
(3c-2)
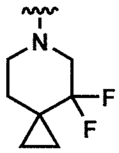
(3c-3)
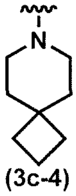
(3c-4)
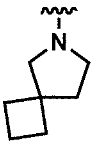
(3c-5)
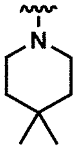
(3c-6)
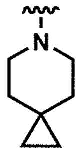
(3c-7)
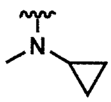
(3d-1)
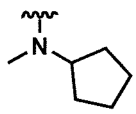
(3d-2)

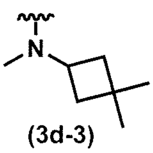
(3d-3)
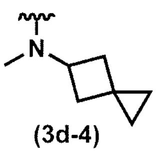
(3d-4)
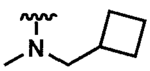
(3d-5)
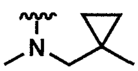
(3d-6)
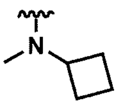
(3d-7)
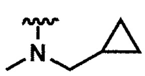
(3d-8)

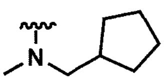
(3d-9)
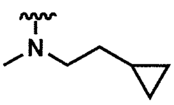
(3d-10)
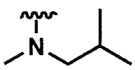
(3d-11)
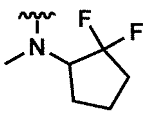
(3d-12)
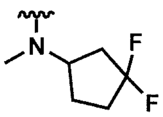
(3d-13)
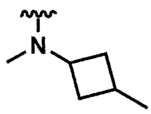
(3d-14)
.

Further preferably, it includes a group of formula (3a-1), (3a-2), (3a-3), (3a-4), (3c-2), (3c-3), (3c-4), (3d-1), (3d-2), (3d-3), (3d-4), (3d-5), (3d-6), or (3d-7).

Preferably, $M^2$ includes any group of

(1) the following formulae (2a-1) - (2a-23) and (2b-1) - (2b-11) :

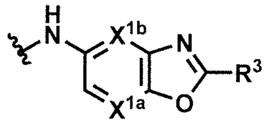
(2a-1)

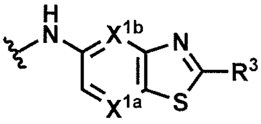
(2a-2)

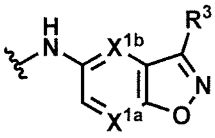
(2a-3)

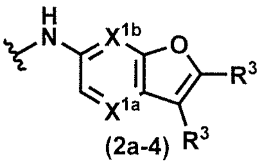
(2a-4)

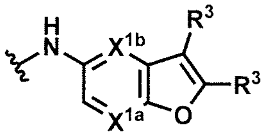
(2a-5)

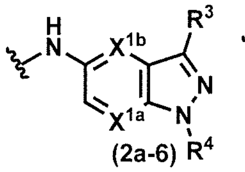
(2a-6)

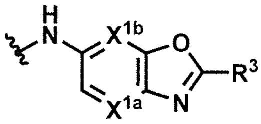
(2a-7)

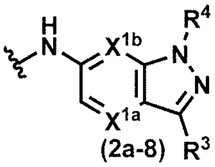
(2a-8)

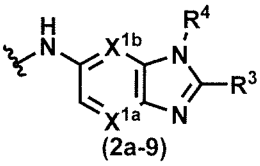
(2a-9)

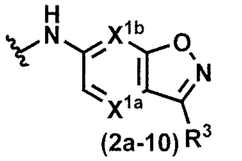
(2a-10)

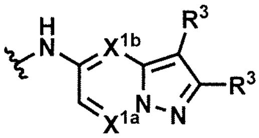
(2a-11)

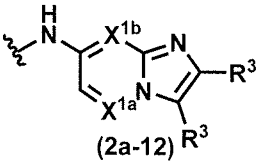
(2a-12)

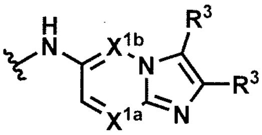
(2a-13)

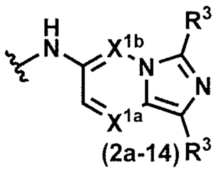
(2a-14)

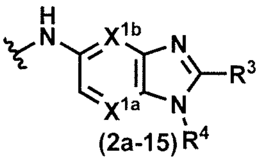
(2a-15)

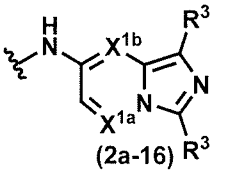
(2a-16)

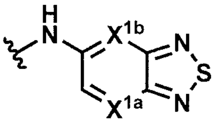
(2a-17)

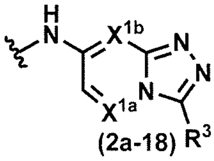
(2a-18)

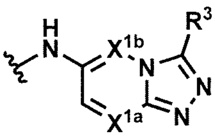
(2a-19)

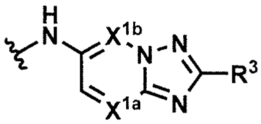
(2a-20)

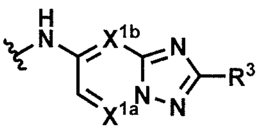
(2a-21)

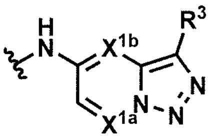
(2a-22)

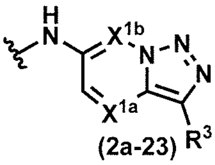
(2a-23)

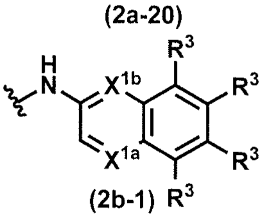
(2b-1)

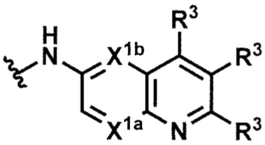
(2b-2)

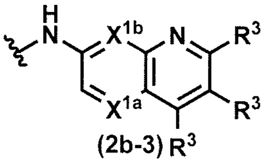
(2b-3)

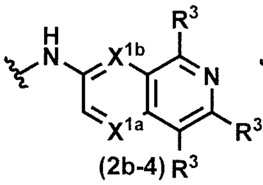
(2b-4)

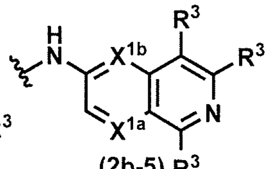
(2b-5)

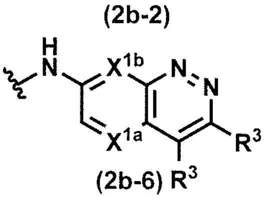
(2b-6)

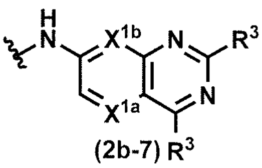
(2b-7)

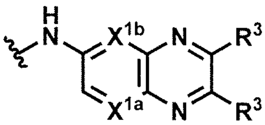
(2b-8)

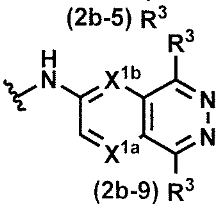
(2b-9)

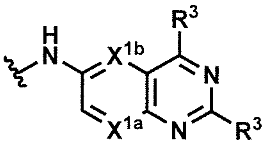
(2b-10)

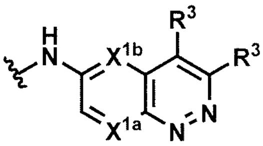
(2b-11)

wherein

$X^{1a}$ and $X^{1b}$ are independently N or $CR^3$,
$R^3$ is

(a) hydrogen atom,
(b) halogen atom,
(c) cyano,
(d) hydroxy,
(e) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,
(g) $C_{1-6}$ alkoxy which may be optionally substituted with the same or different 1 to 3 substituents selected from the group consisting of halogen atom, hydroxy, and $C_{1-6}$ alkoxy,
(h) amino which may be optionally substituted with the same or different 1 to 2 substituents selected from the group consisting of $C_{1-6}$ alkyl which may be optionally substituted with the same or different 1 to 3 halogen atoms, saturated or partially-unsaturated $C_{3-7}$ carbocyclyl, and $C_{2-7}$ alkylcarbonyl,
(i) 5- or 6-membered heteroaryl which may be optionally substituted with the same or different 1 to 4 substituents selected from the group consisting of halogen atom, cyano, and $C_{1-6}$ alkyl,
(j) saturated 5- or 6-membered heterocyclyl which may be optionally substituted with the same or different 1 to 4 substituents selected from the group consisting of halogen atom, hydroxy, $C_{1-6}$ alkyl, and $C_{1-6}$ alkoxy, or
(k) -C(O) $NR^xR^y$, wherein $R^x$ and RY are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring, provided that when there are plural $R^3$, each $R^3$ may be the same or different,

$R^4$ is

(a) hydrogen atom,
(b) $C_{1-6}$ alkyl which may be optionally substituted with the same or different 1 to 3 substituents selected from the group consisting of halogen atom, hydroxy, and $C_{1-6}$ alkoxy, or
(c) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,

(2) the following formula (2c'):

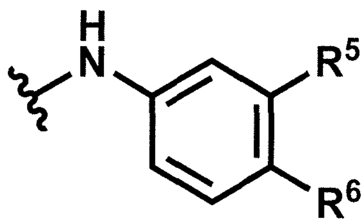

(2c')

wherein

$R^5$ and $R^6$ are independently

(a) hydrogen atom,
(b) halogen atom,
(c) cyano,
(d) amino which may be optionally substituted with the same or different 1 to 2 substituents selected from the group consisting of $C_{1-6}$ alkyl which may be optionally substituted with the same or different 1 to 3

halogen atoms; saturated or partially-unsaturated $C_{3-7}$ carbocyclyl; and $C_{2-7}$ alkylcarbonyl,
(e) 5- or 6-membered heteroaryl which may be optionally substituted with the same or different 1 to 4 substituents selected from the group consisting of halogen atom, cyano, and $C_{1-6}$ alkyl,
(f) 4- to 7-membered saturated or partially-unsaturated heterocyclyl which may be optionally substituted with the same or different 1 to 4 substituents selected from the group consisting of halogen atom, hydroxy, $C_{1-6}$ alkyl, and $C_{1-6}$ alkoxy, or
(g) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring, or

$R^5$ and $R^6$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom, oxo, $C_{1-6}$ alkyl, $C_{1-6}$ alkoxy, and $C_{2-7}$ alkoxycarbonyl, and
the substitutable carbon atom on the ring of formula (2c') may have one fluorine atom as a substituent, or

(3) the following formula (2d), (2f), (2g), or (2h):

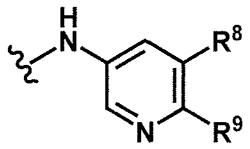
(2d)
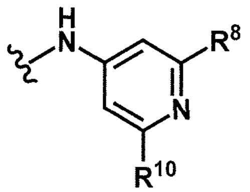
(2f)
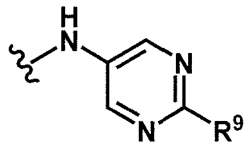
(2g)
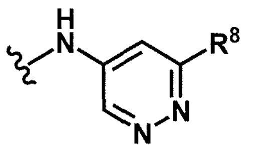
(2h)

wherein

$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy or $C_{1-6}$ alkoxy; 4-to 7-membered saturated or partially-unsaturated heterocyclyl which may be optionally substituted with $C_{1-6}$ alkyl or $C_{1-6}$ alkoxy; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(g) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,
(h) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of

halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,

(i) 4- to 7-membered saturated or partially-unsaturated heterocyclyloxy which may be optionally substituted with the same or different 1 to 4 $C_{1-6}$ alkyl,

(j) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,

(k) -C(O) $OR^Z$, wherein $R^Z$ is $C_{1-6}$ alkyl, or

(l) ethenyl which may be substituted with one 6-membered saturated heterocyclyl,

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl, and

the substitutable carbon atom on the ring of formula (2d), (2f), (2g), or (2h) may have one fluorine atom as a substituent.

An embodiment of $M^2$ includes a group of the following formula (2a') or (2b'):

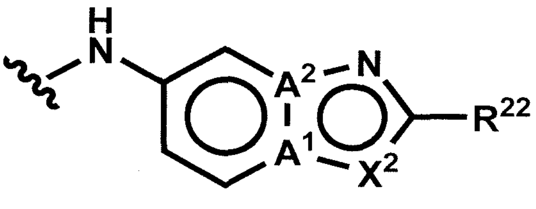
(2a')

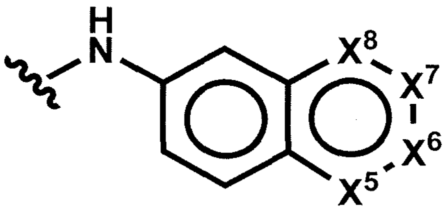
(2b')

wherein

$X^2$, $X^5$, $X^6$, $X^7$, and $X^8$ are independently N, $CR^{21}$, or O,
$A^1$ and $A^2$ are independently N or C,

wherein $X^2$, $X^5$, $X^6$, $X^7$, $X^8$, $A^1$, and $A^2$ are chosen so that the ring composed thereof can be 9- or 10-membered bicyclic heteroaromatic ring, and
$R^{21}$ and $R^{22}$ are independently

(1) hydrogen atom,
(2) halogen atom,
(3) cyano,
(4) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy,
(5) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,
(6) $C_{1-6}$ alkoxy which may be optionally substituted with the same or different 1 to 3 substituents selected from the group consisting of halogen atom, hydroxy, and $C_{1-6}$ alkoxy, or
(7) amino which may be optionally substituted with the same or different 1 to 2 substituents selected from the group consisting of $C_{1-6}$ alkyl which may be optionally substituted with the same or different 1 to 3 halogen atoms, saturated or partially-unsaturated $C_{3-7}$ carbocyclyl, and $C_{2-7}$ alkylcarbonyl.

Another embodiment of $M^2$ includes a group of any one of the following formulae (2a-1) - (2a-23) and (2b-1) - (2b-11) :

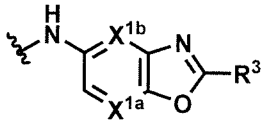
(2a-1)
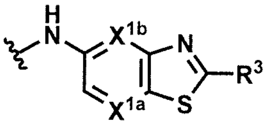
(2a-2)
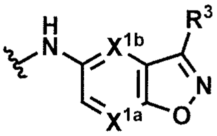
(2a-3)
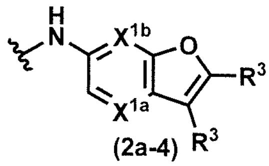
(2a-4)
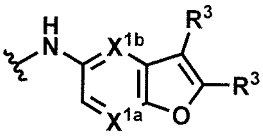
(2a-5)
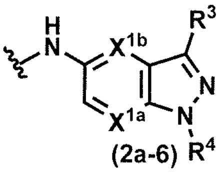
(2a-6)
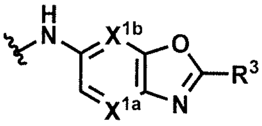
(2a-7)
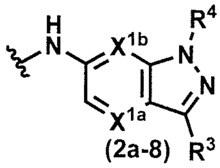
(2a-8)
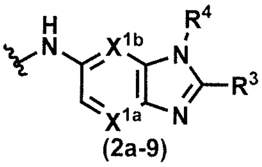
(2a-9)
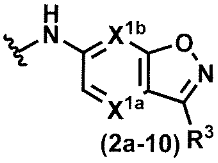
(2a-10)
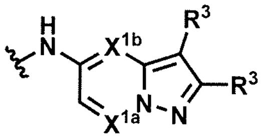
(2a-11)
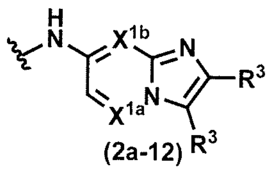
(2a-12)
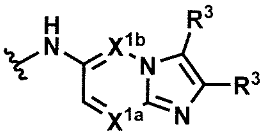
(2a-13)
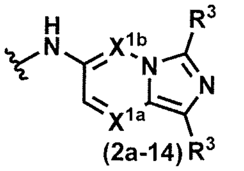
(2a-14)
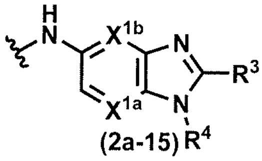
(2a-15)
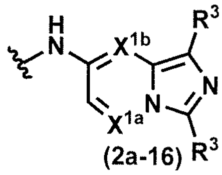
(2a-16)
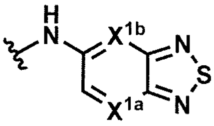
(2a-17)
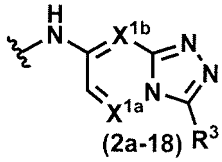
(2a-18)
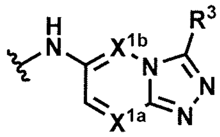
(2a-19)
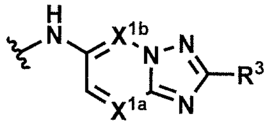
(2a-20)
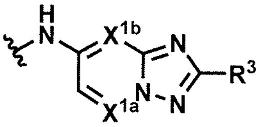
(2a-21)
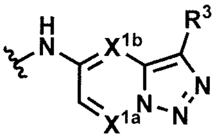
(2a-22)
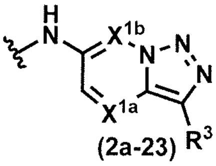
(2a-23)
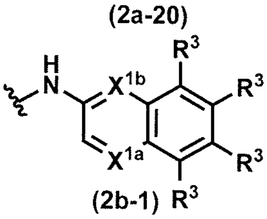
(2b-1)
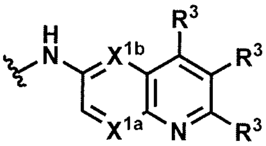
(2b-2)
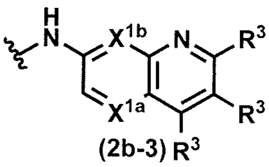
(2b-3)
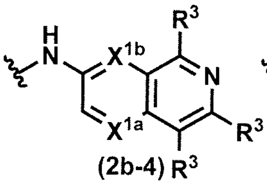
(2b-4)
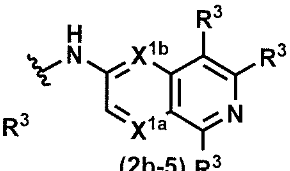
(2b-5)
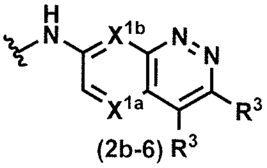
(2b-6)
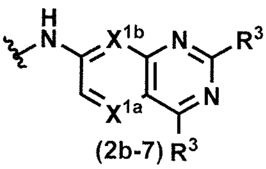
(2b-7)
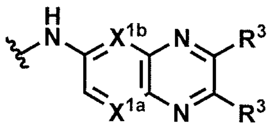
(2b-8)
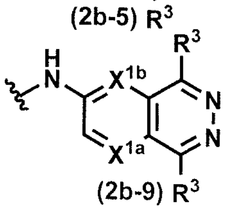
(2b-9)
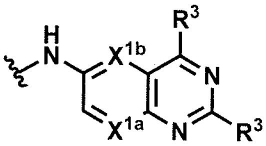
(2b-10)
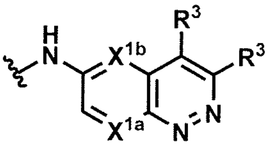
(2b-11)

wherein

$X^{1a}$ and $X^{1b}$ are independently N or $CR^3$,
$R^3$ is

(a) hydrogen atom,
(b) halogen atom,
(c) cyano,
(d) hydroxy,
(e) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,
(g) $C_{1-6}$ alkoxy which may be optionally substituted with the same or different 1 to 3 substituents selected from the group consisting of halogen atom, hydroxy, and $C_{1-6}$ alkoxy,
(h) amino which may be optionally substituted with the same or different 1 to 2 substituents selected from the group consisting of $C_{1-6}$ alkyl which may be optionally substituted with the same or different 1 to 3 halogen atoms, saturated or partially-unsaturated $C_{3-7}$ carbocyclyl, and $C_{2-7}$ alkylcarbonyl,
(i) 5- or 6-membered heteroaryl which may be optionally substituted with the same or different 1 to 4 substituents selected from the group consisting of halogen atom, cyano, and $C_{1-6}$ alkyl,
(j) 5- or 6-membered saturated heterocyclyl which may be optionally substituted with the same or different 1 to 4 substituents selected from the group consisting of halogen atom, hydroxy, $C_{1-6}$ alkyl, and $C_{1-6}$ alkoxy, or
(k) $-C(O)NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring, provided that when there are plural $R^3$, each $R^3$ may be the same or different, and

$R^4$ is

(a) hydrogen atom,
(b) $C_{1-6}$ alkyl which may be optionally substituted with the same or different 1 to 3 substituents selected from the group consisting of halogen atom, hydroxy, and $C_{1-6}$ alkoxy, or
(c) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy.

Another embodiment of $M^2$ includes a group of the following formula (2c'):

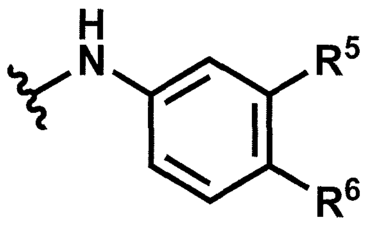

(2c')

wherein

$R^5$ and $R^6$ are independently

(a) hydrogen atom,
(b) halogen atom,
(c) cyano,
(d) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,
(e) 5- or 6-membered heteroaryl which may be optionally substituted with the same or different 1 to 4 substituents selected from the group consisting of halogen atom, cyano, and $C_{1-6}$ alkyl,

(f) 4- to 7-membered saturated or partially-unsaturated heterocyclyl which may be optionally substituted with the same or different 1 to 4 substituents selected from the group consisting of halogen atom, hydroxy, $C_{1-6}$ alkyl, and $C_{1-6}$ alkoxy, or

(g) -C(O)$NR^xR^y$, wherein $R^x$ and RY are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring, or

$R^5$ and $R^6$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom, oxo, $C_{1-6}$ alkyl, $C_{1-6}$ alkoxy, and $C_{2-7}$ alkoxycarbonyl, and
the substitutable carbon atom on the ring of formula (2c') may have one fluorine atom as a substituent.

Another embodiment of $M^2$ includes a group of the following formula (2d), (2f), (2g), or (2h):

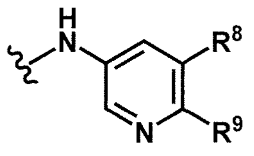
(2d)

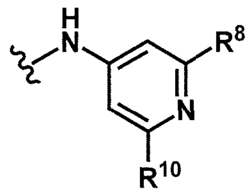
(2f)

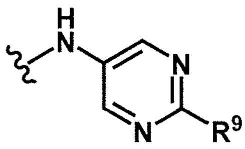
(2g)

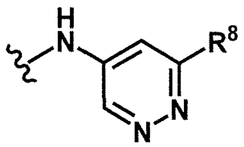
(2h)

wherein

$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy or $C_{1-6}$ alkoxy; 4-to 7-membered saturated or partially-unsaturated heterocyclyl which may be optionally substituted with $C_{1-6}$ alkyl or $C_{1-6}$ alkoxy; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(g) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,
(h) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,
(i) 4- to 7-membered saturated or partially-unsaturated heterocyclyloxy which may be optionally substituted with the same or different 1 to 4 $C_{1-6}$ alkyl,

(j) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,
(k) -C(O) $OR^Z$, wherein $R^Z$ is $C_{1-6}$ alkyl, or
(l) ethenyl which may be substituted with one 6-membered saturated heterocyclyl,
wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl, and

the substitutable carbon atom on the ring of formula (2d), (2f), (2g), or (2h) may have one fluorine atom as a substituent.

Further embodiment of $M^2$ includes a group of the following formula (2k'):

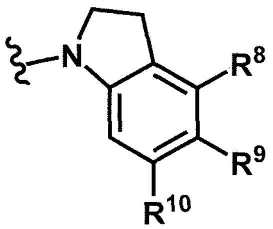

**(2k')**

wherein
$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy or $C_{1-6}$ alkoxy; 4-to 7-membered saturated or partially-unsaturated heterocyclyl which may be optionally substituted with $C_{1-6}$ alkyl or $C_{1-6}$ alkoxy; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 substituents selected from the group consisting of $C_{1-6}$ alkyl which may be optionally substituted with halogen atom; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(g) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,
(h) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,
(i) 4- to 7-membered saturated or partially-unsaturated heterocyclyloxy which may be optionally substituted with the same or different 1 to 4 $C_{1-6}$ alkyl,
(j) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4-

to 7-membered saturated heterocyclic ring,
(k) -C(O) $OR^Z$, wherein $R^Z$ is $C_{1-6}$ alkyl, or
(l) ethenyl which may be substituted with one 6-membered saturated heterocyclyl, or

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl.

In an embodiment, the present compound of formula (1) includes the following (A).

(A) A compound of formula (1) or pharmaceutically acceptable salt thereof, wherein

Ring Cy is a group of formula (a),
$R^{1a}$ and $R^{2a}$ are independently hydrogen atom, halogen atom, cyano, methyl, or methoxy,
$M^1$ is saturated or partially-unsaturated $C_{4-12}$ carbocyclyl which may be optionally substituted with the same or different 1 to 4 substituents selected from the group consisting of halogen atom and $C_{1-6}$ alkyl,
$M^2$ is a group of formula (2a-1), (2a-2), (2a-12), (2a-20), (2a-21), (2b-3), (2b-4), (2b-7), or (2b-10),
$X^{1a}$ and $X^{1b}$ are independently N or $CR^3$, and
$R^3$ is independently hydrogen atom, halogen atom, cyano, hydroxy, $C_{1-6}$ alkyl, or $C_{1-6}$ alkoxy, provided that when there are plural $R^3$, each $R^3$ may be the same or different.

Another embodiment of the present compound of formula (1) includes the following (B):
(B) A compound of formula (1) or pharmaceutically acceptable salt thereof, wherein

Ring Cy is a group of formula (a),
$R^{1a}$ and $R^{2a}$ are independently hydrogen atom, halogen atom, cyano, methyl, or methoxy,
$M^1$ is 4- to 12-membered saturated or partially-unsaturated heterocyclyl which may be optionally substituted with the same or different 1 to 4 substituents selected from the group consisting of halogen atom and $C_{1-6}$ alkyl,
$M^2$ is a group of formula (2a-1), (2a-2), (2a-12), (2a-20), (2a-21), (2b-3), (2b-4), (2b-7), or (2b-10),
$X^{1a}$ and $X^{1b}$ are independently N or $CR^3$, and
$R^3$ is independently hydrogen atom, halogen atom, cyano, hydroxy, $C_{1-6}$ alkyl, or $C_{1-6}$ alkoxy, provided that when there are plural $R^3$, each $R^3$ may be the same or different.

Another embodiment of the present compound of formula (1) includes the following (C):
(C) A compound of formula (1) or pharmaceutically acceptable salt thereof, wherein

Ring Cy is a group of formula (a),
$R^{1a}$ and $R^{2a}$ are independently hydrogen atom, halogen atom, cyano, methyl, or methoxy,
$M^1$ is $-NR^eR^f$,
$R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,

$M^2$ is a group of formula (2a-1), (2a-2), (2a-12), (2a-20), (2a-21), (2b-3), (2b-4), (2b-7), or (2b-10),
$X^{1a}$ and $X^{1b}$ are independently N or $CR^3$, and
$R^3$ is independently hydrogen atom, halogen atom, cyano, hydroxy, $C_{1-6}$ alkyl, or $C_{1-6}$ alkoxy, provided that when there are plural $R^3$, each $R^3$ may be the same or different.

Another embodiment of the present compound of formula (1) includes the following (D):
(D) A compound of formula (1) or pharmaceutically acceptable salt thereof, wherein

Ring Cy is a group of formula (a),
$R^{1a}$ and $R^{2a}$ are independently hydrogen atom, halogen atom, cyano, methyl, or methoxy,
$M^1$ is saturated or partially-unsaturated $C_{4-12}$ carbocyclyl which may be optionally substituted with the same or

different 1 to 4 substituents selected from the group consisting of halogen atom and $C_{1-6}$ alkyl,
$M^2$ is a group of formula (2d) or (2f), and
$R^8$, $R^9$, and $R^{10}$ are independently hydrogen atom, halogen atom, cyano, $C_{1-6}$ alkyl, or $C_{1-6}$ alkoxy.

Another embodiment of the present compound of formula (1) includes the following (E):
(E) A compound of formula (1) or pharmaceutically acceptable salt thereof, wherein

Ring Cy is a group of formula (a),
$R^{1a}$ and $R^{2a}$ are independently hydrogen atom, halogen atom, cyano, methyl, or methoxy,
$M^1$ is 4- to 12-membered saturated or partially-unsaturated heterocyclyl which may be optionally substituted with the same or different 1 to 4 substituents selected from the group consisting of halogen atom and $C_{1-6}$ alkyl,
$M^2$ is a group of formula (2d) or (2f), and
$R^8$, $R^9$, and $R^{10}$ are independently hydrogen atom, halogen atom, cyano, $C_{1-6}$ alkyl, or $C_{1-6}$ alkoxy.

Another embodiment of the present compound of formula (1) includes the following (F):
(F) A compound of formula (1) or pharmaceutically acceptable salt thereof, wherein

Ring Cy is a group of formula (a),
$R^{1a}$ and $R^{2a}$ are independently hydrogen atom, halogen atom, cyano, methyl, or methoxy,
$M^1$ is $-NR^eR^f$,
$R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,

$M^2$ is a group of formula (2d) or (2f), and
$R^8$, $R^9$, and $R^{10}$ are independently hydrogen atom, halogen atom, cyano, $C_{1-6}$ alkyl, or $C_{1-6}$ alkoxy.

Another embodiment of the present compound of formula (1) includes the following (G):
(G) A compound of formula (1) or pharmaceutically acceptable salt thereof, wherein

Ring Cy is a group of formula (b),
$Y^2$ and $Y^3$ are independently N or $CR^2$,
$R^{1b}$ is hydrogen atom, halogen atom, cyano, $C_{1-6}$ alkyl, or $C_{3-6}$ cycloalkyl,
$R^{2b}$ is independently hydrogen atom, halogen atom, cyano, $C_{1-6}$ alkyl, or $C_{3-6}$ cycloalkyl, provided that when there are plural $R^{2b}$, each $R^{2b}$ may be the same or different,
$M^1$ is saturated or partially-unsaturated $C_{4-12}$ carbocyclyl which may be optionally substituted with the same or different 1 to 4 substituents selected from the group consisting of halogen atom and $C_{1-6}$ alkyl,
$M^2$ is a group of formula (2a-1), (2a-2), (2a-12), (2a-20), (2a-21), (2b-3), (2b-4), (2b-7), or (2b-10),
$X^{1a}$ and $X^{1b}$ are independently N or $CR^3$, and
$R^3$ is independently hydrogen atom, halogen atom, cyano, hydroxy, $C_{1-6}$ alkyl, or $C_{1-6}$ alkoxy, provided that when there are plural $R^3$, each $R^3$ may be the same or different.

Another embodiment of the present compound of formula (1) includes the following (H):
(H) A compound of formula (1) or pharmaceutically acceptable salt thereof, wherein

Ring Cy is a group of formula (b),
$Y^2$ and $Y^3$ are independently N or $CR^2$,
$R^{1b}$ is hydrogen atom, halogen atom, cyano, $C_{1-6}$ alkyl, or $C_{3-6}$ cycloalkyl,
$R^{2b}$ is independently hydrogen atom, halogen atom, cyano, $C_{1-6}$ alkyl, or $C_{3-6}$ cycloalkyl, provided that when there are plural $R^{2b}$, each $R^{2b}$ may be the same or different,
$M^1$ is 4- to 12-membered saturated or partially-unsaturated heterocyclyl which may be optionally substituted with the same or different 1 to 4 substituents selected from the group consisting of halogen atom and $C_{1-6}$ alkyl,
$M^2$ is a group of formula (2a-1), (2a-2), (2a-12), (2a-20), (2a-21), (2b-3), (2b-4), (2b-7), or (2b-10),

$X^{1a}$ and $X^{1b}$ are independently N or $CR^3$, and
$R^3$ is independently hydrogen atom, halogen atom, cyano, hydroxy, $C_{1-6}$ alkyl, or $C_{1-6}$ alkoxy, provided that when there are plural $R^3$, each $R^3$ may be the same or different.

Another embodiment of the present compound of formula (1) includes the following (I):
(I) A compound of formula (1) or pharmaceutically acceptable salt thereof, wherein

Ring Cy is a group of formula (b),
$Y^2$ and $Y^3$ are independently N or $CR^2$,
$R^{1b}$ is hydrogen atom, halogen atom, cyano, $C_{1-6}$ alkyl, or $C_{3-6}$ cycloalkyl,
$R^{2b}$ is independently hydrogen atom, halogen atom, cyano, $C_{1-6}$ alkyl, or $C_{3-6}$ cycloalkyl, provided that when there are plural $R^{2b}$, each $R^{2b}$ may be the same or different,
$M^1$ is $-NR^eR^f$,
$R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,

$M^2$ is a group of formula (2a-1), (2a-2), (2a-12), (2a-20), (2a-21), (2b-3), (2b-4), (2b-7), or (2b-10),
$X^{1a}$ and $X^{1b}$ are independently N or $CR^3$, and
$R^3$ is independently hydrogen atom, halogen atom, cyano, hydroxy, $C_{1-6}$ alkyl, or $C_{1-6}$ alkoxy, provided that when there are plural $R^3$, each $R^3$ may be the same or different.

Another embodiment of the present compound of formula (1) includes the following (J):
(J) A compound of formula (1) or pharmaceutically acceptable salt thereof, wherein

Ring Cy is a group of formula (b),
$Y^2$ and $Y^3$ are independently N or $CR^2$,
$R^{1b}$ is hydrogen atom, halogen atom, cyano, $C_{1-6}$ alkyl, or $C_{3-6}$ cycloalkyl,
$R^{2b}$ is independently hydrogen atom, halogen atom, cyano, $C_{1-6}$ alkyl, or $C_{3-6}$ cycloalkyl, provided that when there are plural $R^{2b}$, each $R^{2b}$ may be the same or different,
$M^1$ is saturated or partially-unsaturated $C_{4-12}$ carbocyclyl which may be optionally substituted with the same or different 1 to 4 substituents selected from the group consisting of halogen atom and $C_{1-6}$ alkyl,
$M^2$ is a group of formula (2d) or (2f), and
$R^8$, $R^9$, and $R^{10}$ are independently hydrogen atom, halogen atom, cyano, $C_{1-6}$ alkyl, or $C_{1-6}$ alkoxy.

Another embodiment of the present compound of formula (1) includes the following (K):
(K) A compound of formula (1) or pharmaceutically acceptable salt thereof, wherein

Ring Cy is a group of formula (b),
$Y^2$ and $Y^3$ are independently N or $CR^2$,
$R^{1b}$ is hydrogen atom, halogen atom, cyano, $C_{1-6}$ alkyl, or $C_{3-6}$ cycloalkyl,
$R^{2b}$ is independently hydrogen atom, halogen atom, cyano, $C_{1-6}$ alkyl, or $C_{3-6}$ cycloalkyl, provided that when there are plural $R^{2b}$, each $R^{2b}$ may be the same or different,
$M^1$ is 4- to 12-membered saturated or partially-unsaturated heterocyclyl which may be optionally substituted with the same or different 1 to 4 substituents selected from the group consisting of halogen atom and $C_{1-6}$ alkyl,
$M^2$ is a group of formula (2d) or (2f), and
$R^8$, $R^9$, and $R^{10}$ are independently hydrogen atom, halogen atom, cyano, $C_{1-6}$ alkyl, or $C_{1-6}$ alkoxy.

Another embodiment of the present compound of formula (1) includes the following (L):
(L) A compound of formula (1) or pharmaceutically acceptable salt thereof, wherein

Ring Cy is a group of formula (b),
$Y^2$ and $Y^3$ are independently N or $CR^2$,

$R^{1b}$ is hydrogen atom, halogen atom, cyano, $C_{1-6}$ alkyl, or $C_{3-6}$ cycloalkyl,
$R^{2b}$ is independently hydrogen atom, halogen atom, cyano, $C_{1-6}$ alkyl, or $C_{3-6}$ cycloalkyl, provided that when there are plural $R^{2b}$, each $R^{2b}$ may be the same or different,
$M^1$ is $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,

$M^2$ is a group of formula (2d) or (2f), and
$R^8$, $R^9$, and $R^{10}$ are independently hydrogen atom, halogen atom, cyano, $C_{1-6}$ alkyl, or $C_{1-6}$ alkoxy.

The "pharmaceutically acceptable salt" includes acid addition salts, base addition salts, and amino acid salts. For example, the acid addition salt includes inorganic acid salts such as hydrochloride, hydrobromide, sulfate, hydroiodide, nitrate, and phosphate; or organic acid salts such as citrate, oxalate, phthalate, fumarate, maleate, succinate, malate, acetate, formate, propionate, benzoate, trifluoroacetate, methanesulfonate, benzenesulfonate, p-toluenesulfonate, and camphorsulfonate. The base addition salt includes inorganic base salts such as sodium salts, potassium salts, calcium salts, magnesium salts, barium salts, and aluminum salts; and organic base salts such as trimethylamine, triethylamine, pyridine, picoline, 2,6-lutidine, ethanolamine, diethanolamine, triethanolamine, tromethamine[tris(hydroxymethyl)methylamine], tert-butylamine, cyclohexylamine, dicyclohexylamine, and N,N-dibenzylethylamine. The amino acid salt includes amino acid salts of basic or acidic amino acids such as arginine, lysine, ornithine, aspartate, and glutamate.
In addition, the present compound (1) encompasses any crystalline forms thereof.
A compound of Formula (1) may have at least one asymmetric carbon atom. Thus, the present compound encompasses racemates of a compound of Formula (1), as well as optical isomers thereof. A compound of Formula (1) encompasses deuterated compounds in which any one or more $^1H$ in the compound are replaced with $^2H$ (D).
Some compounds of formula (1) may have isomers including tautomers such as keto-enol forms, regioisomers, geometric isomers, or optical isomers. All possible isomers including them, and mixtures of such isomers in any ratio, are also encompassed in the present invention. And, the compound of formula (1) may exist in a form of hydrate or solvate (e.g., ethanolate) with various types of solvents such as water and ethanol, and such hydrates and solvents are also encompassed in the present invention.
Hereinafter, methods for preparing a compound of Formula (1) in the present invention are exemplified, but the present invention is not limited to such examples.

Preparation

The present compound (1) may be prepared by the following processes and methods which are combined with common synthetic methods.
Compounds in the reaction schemes include ones in the salt form, and such salts include, for example, what are described in the above "pharmaceutically acceptable salt". It should be noted that these reactions are merely illustrative, and other methods may be optionally applied for preparing the present compound based on the knowledge of a person skilled in synthetic organic chemistry.
In each of the preparation process described below, when there is a functional group that needs protection, the functional group may be protected as necessary and deprotected after the completion of a reaction or a series of reactions to afford a targeted product, even if the use of the protective group is not specifically indicated.
The protective groups used herein include common protective groups, which include, for example, the protective groups described in the literatures (T. W. Greene and P. G. M. Wuts, "Protective Groups in Organic Synthesis", 3rd Ed., John Wiley and Sons, inc., New York (1999) etc.). More specifically, protective groups for amino group include, for example, tert-butoxycarbonyl, benzyloxycarbonyl, p-toluenesulfonyl, o-nitrobenzenesulfonyl, and tetrahydropyranyl. Protective groups for hydroxy group include, for example, trialkylsilyl, acetyl, benzyl, tetrahydropyranyl, and methoxymethyl. Protective groups for aldehyde group include, for example, dialkylacetal and cyclic alkylacetal. Protective groups for carboxyl group include, for example, tert-butyl ester, orthoester, and amide.
The introduction and deprotection of protective groups can be done by methods commonly used in organic synthetic chemistry (for example, the methods described in T. W. Greene and P. G. M. Wuts, "Protective Groups in Organic Synthesis", 3rd Ed., John Wiley and Sons, inc., New York (1999) etc.) or corresponding methods thereof.

A compound of Formula (1) is prepared by forming bonds at the positions of a, b, and c:

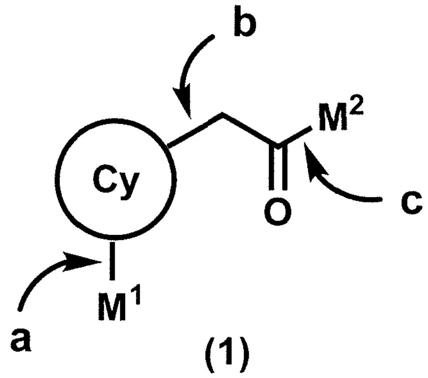

(1)

wherein ring Cy, $M^1$, and $M^2$ are as defined in the above (Item 5) .

Processes for forming bonds at the positions a, b, and c are illustrated in the following Preparations, but the sequence of forming bonds may be optionally modified.

The present compound wherein Ring Cy is a group of formula (a) may be prepared in the manners of Preparations 1a - 5a.

Preparation 1a

Among compounds of Formula (1), a compound of formula (1a) is prepared, for example, by the following process:

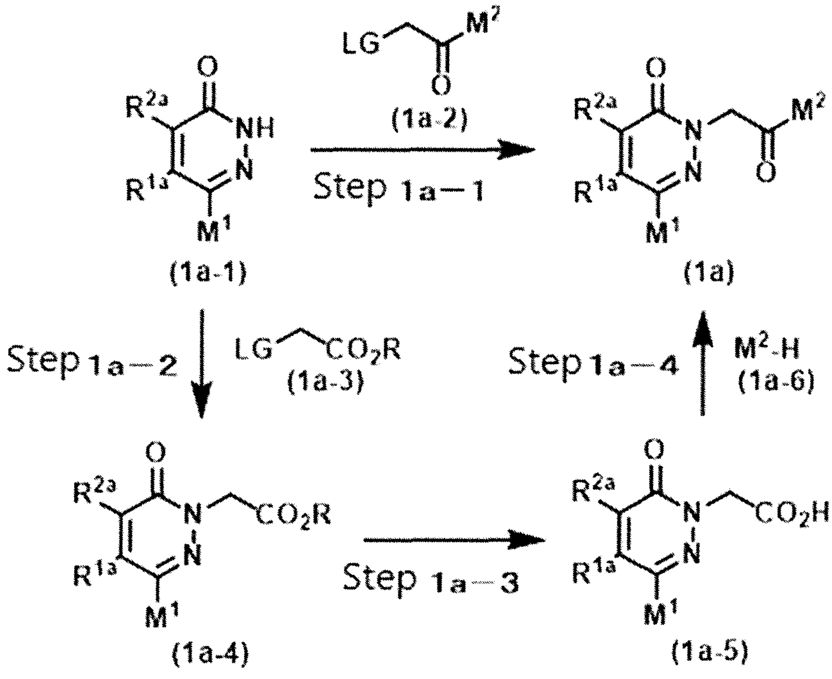


wherein $R^{1a}$ and $R^{2a}$ are the same as those defined in the above (Item 6); $M^1$ and $M^2$ are the same as those defined in the above (Item 5); R is $C_{1-6}$ alkyl; and LG is a leaving group (for example, iodine atom, bromine atom, chlorine atom, and substituted sulfonyl (such as methanesulfonyl and p-toluenesulfonyl)).

Step 1a-1: Preparation step of Compound (1a)

A compound of Formula (1a) is prepared by reacting Compound (1a-1) with Compound (la-2) in the presence of a base in an appropriate inert solvent. As Compound (1a-1), a product synthesized in Preparation 4a or 5a described

below, or a commercial product may be used. As Compound (1a-2), a commercial product or a product synthesized by common methods or corresponding methods thereof may be used.

Examples of the base used herein include inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; metal alkoxides such as sodium methoxide and potassium tert-butoxide; and organic bases such as triethylamine, diisopropylethylamine, and pyridine.

Examples of the inert solvent used herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; and mixed solvents thereof.

The reaction temperature is selected from, but not limited to, usually the range of -10°C to 200°C, preferably the range of 0°C to 40°C. The reaction time is usually 10 minutes to 48 hours, but it depends on conditions including reaction temperature, materials, and solvents which are used.

Step 1a-2: Preparation step of Compound (1a-4)

Compound (la-4) is prepared by reacting Compound (1a-1) with Compound (1a-3) according to the method described in Step 1a-1. As Compound (1a-3), a commercial product or a product synthesized by common methods or corresponding methods thereof may be used.

Step 1a-3: Preparation step of Compound (1a-5)

Compound (la-5) is prepared by hydrolyzing Compound (1a-4) by common methods (for example, Protective Groups in Organic Synthesis 3rd Edition (John Wiley & Sons, Inc.), Comprehensive Organic Transformation, R. C. Laroque et al, VCH publisher Inc., 1989 etc.) or corresponding methods thereof.

Step 1a-4: Preparation step of Compound (1a)

A compound of formula (1a) is also prepared by reacting Compound (1a-5) with Compound (1a-6) in the presence or absence of a base in an appropriate inert solvent using a condensing agent. As Compound (1-6), a commercial product or a product synthesized by common methods or corresponding methods thereof may be used.

Examples of the condensing agent include dicyclohexylcarbodiimide (DCC), diisopropylcarbodiimide (DIPC), 1-ethyl-3-(3-dimethylaminopropyl)-carbodiimide (WSC), benzotriazol-1-yl-tris(dimethylamino)phosphonium hexafluorophosphate (BOP), diphenylphosphoryl azide (DPPA), N,N-carbonyldiimidazole (CDI), benzotriazol-1-yl-N,N,N',N'-tetramethyluronium hexafluorophosphate (HBTU), and 7-azabenzotriazol-1-yl-N,N,N',N'-tetramethyluronium hexafluorophosphate (HATU). If necessary, additives such as N-hydroxysuccinimide (HOSu), 1-hydroxybenzotriazole (HOBt), and 3-hydroxy-4-oxo-3,4-dihydro-1,2,3-benzotriazine (HOOBt) may be added to the reaction.

Examples of the base used herein include organic bases such as triethylamine, diisopropylethylamine, and pyridine; inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; and metal alkoxides such as sodium methoxide and potassium tert-butoxide.

Examples of the inert solvent used herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; and mixed solvents thereof.

The reaction temperature is selected from, but not limited to, usually the range from -10°C to 200°C, preferably the range from 0°C to 40°C. Reaction time is usually 10 minutes to 48 hours, but it depends on conditions including reaction temperature, materials, and solvents which are used.

The present step can also be proceeded, for example, by activating a carbonyl group with an acid anhydride, a mixed acid anhydride, or an acid halide, and then reacting with Compound (1a-6).

Preparation 2a

Among compounds of Formula (1), a compound of formula (2a-4) is prepared, for example, by the following process:

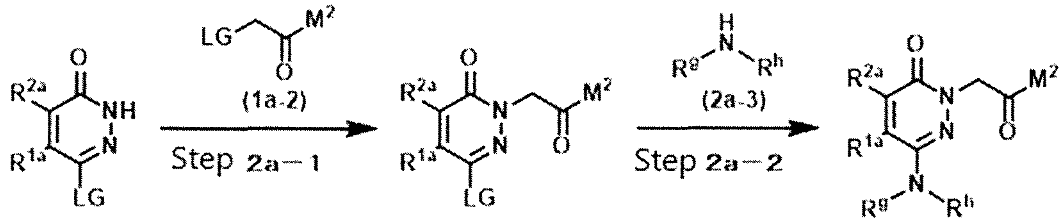


(2a-1) (2a-2) (2a-4)

wherein $R^{1a}$ and $R^{2a}$ are the same as those defined in the above (Item 6); and $M^2$ is the same as those defined in the above (Item 5); LG is a leaving group (for example, iodine atom, bromine atom, chlorine atom, and substituted sulfonyl (such as methanesulfonyl and p-toluenesulfonyl)); $R^g$ and $R^h$ are each independently the same as the definition of $R^e$ or $R^f$ defined in the above (Item 5); or alternatively, $R^g$ and $R^h$ may be taken together with the nitrogen atom to which they attach to form an optionally-substituted 4- to 12-membered saturated heterocyclic ring.

Step 2a-1: Preparation step of Compound (2a-2)

Compound (2a-2) is prepared from Compound (2a-1) and Compound (1a-2) according to the method described in Step 1a-1. For Compound (2a-1), a product synthesized by common methods (for example, those described in Tetrahedron, 2015, 71, 4859, Bioorganic & Medicinal Chemistry Letters, 2015, 25, 1030, etc.) or corresponding methods thereof, or a commercial product may be used.

Step 2a-2: Preparation step of Compound (2a-4)

Compound (2a-4) can be prepared by reacting Compound (2a-2) with Compound (2a-3) in the presence of a base in an appropriate inert solvent. As Compound (2a-3), a commercial product or a product synthesized by common methods or corresponding methods thereof may be used.

Examples of the base used herein include organic bases such as triethylamine, diisopropylethylamine, and pyridine; inorganic bases such as potassium carbonate, sodium carbonate, cesium carbonate, potassium bicarbonate, sodium bicarbonate, dipotassium hydrogen phosphate, potassium phosphate, disodium hydrogen phosphate, sodium phosphate, potassium hydroxide, sodium hydroxide, and sodium hydride; and metal alkoxides such as sodium methoxide and potassium tert-butoxide.

Examples of the inert solvent used herein include aprotic polar solvents such as dimethylformamide, N-methyl-2-pyrrolidinone, dimethylsulfoxide, acetonitrile, acetone, and methyl ethyl ketone; halogenated hydrocarbon solvents such as chloroform and dichloromethane; aromatic hydrocarbon solvents such as benzene and toluene; and mixed solvents thereof.

The reaction temperature is selected from, but not limited to, usually the range of 20°C to 200°C, preferably the range of 50°C to 170°C. The present step may be conducted under microwave irradiation, if necessary. The reaction time is usually 10 minutes to 48 hours, but it depends on conditions including reaction temperature, materials, and solvents which are used.

Preparation 3a

Among compounds of Formula (1), compounds of formulae (3a-2) and (3a-3) are prepared, for example, by the following process:

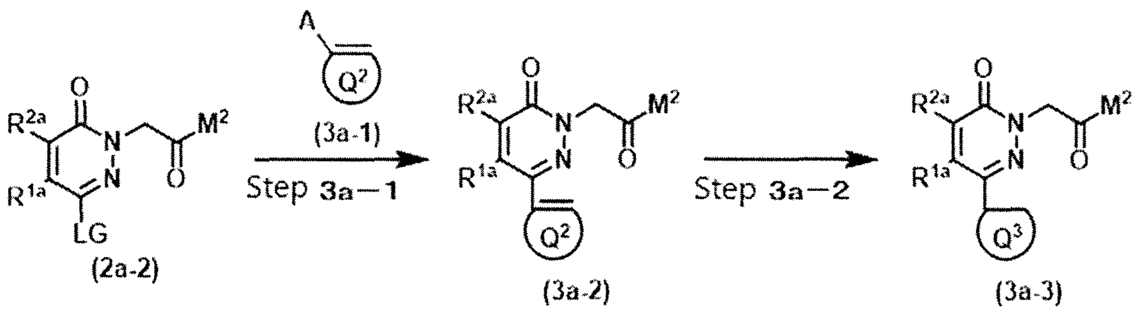


(2a-2) (3a-2) (3a-3)

wherein $R^{1a}$ and $R^{2a}$ are the same as those described in the above (Item 6); $M^2$ is the same as those described in the above (Item 5); LG is a leaving group (for example, iodine atom, bromine atom, chlorine atom, and substituted sulfonyl (such as methanesulfonyl and p-toluenesulfonyl)); A is boronic acid, boronate, $BF_3K$, or $BF_3Na$; $Q^2$ is optionally-substituted 4- to 12-membered partially-unsaturated heterocyclyl or saturated or partially-unsaturated $C_{4-12}$ carbocyclyl; and $Q^3$ is optionally-substituted saturated or partially-unsaturated $C_{4-12}$ carbocyclyl, or optionally-substituted 4- to 12-membered saturated heterocyclyl.

Step 3a-1: Preparation step of Compound (3a-2)

Compound (3a-2) is prepared by reacting Compound (2a-2) with Compound (3a-1) in the presence of a palladium catalyst, a phosphine ligand, and a base in an appropriate inert solvent. As Compound (3a-1), a commercial product, or a product synthesized by common methods or corresponding methods may be used.

Examples of the palladium catalyst herein include tetrakis(triphenylphosphine)palladium(0), bis(dibenzylideneacetone)palladium(0), tris(dibenzylideneacetone)dipalladium(0), bis(tri-tert-butylphosphine)palladium(0), palladium(0) acetate, [1,1-bis(diphenylphosphino)ferrocene]palladium(II) dichloride, and bis(di-tert-butyl(4-dimethylaminophenyl)phosphine)dichloropalladium(II).

Phosphine ligands include, for example, o-tolylphosphine, 2-dicyclohexylphosphino-2',6'-dimethoxybiphenyl (S-Phos), 2-(dicyclohexylphosphino)-2',4',6'-triisopropylbiphenyl (X-Phos), 1,1'-bis(diphenylphosphino)ferrocene (DPPF), 1,2-bis(diphenylphosphino)ethane (DPPE), 1,3-bis(diphenylphosphino)propane (DPPP), 1,4-bis(diphenylphosphino)butane (DPPB), 2,2'-bis(diphenylphosphino)-1,1'-binaphthyl (BINAP), 4,5-bis(diphenylphosphino)-9,9-dimethylxanthene (XANT-Phos), and bis(2-(diphenylphosphino)phenyl) ether (DPE-Phos).

Examples of the base used herein include sodium carbonate, potassium carbonate, cesium carbonate, potassium phosphate, sodium hydroxide, and potassium hydroxide.

Examples of the inert solvent used herein include 1,4-dioxane, THF, 1,2-dimethoxyethane, water, and mixed solvents thereof.

The reaction temperature is selected from, but not limited to, usually the range of 50°C to 200°C, preferably the range of 80°C to 150°C. The present step can be conducted under microwave irradiation, if necessary. Reaction time is usually 30 minutes to 48 hours.

Step 3a-2: Preparation step of Compound (3a-3)

Compound (3a-3) is prepared by catalytic reduction of Compound (3a-2) with a metal catalyst in an appropriate inert solvent under hydrogen atmosphere.

Examples of the metal catalyst used herein include palladium/carbon, palladium hydroxide/carbon, Raney nickel, platinum oxide/carbon, and rhodium/carbon. The amount of a metal catalyst is usually 0.1 % to 1000 % by weight to Compound (3a-2), and preferably 1 % to 100 % by weight.

Examples of the inert solvent used herein include ethers such as tetrahydrofuran; and esters such as ethyl acetate.

The hydrogen pressure is usually 1 to 100 atm, and preferably 1 to 5 atm.

The reaction temperature is selected from, but not limited to, usually the range of 0°C to 120°C, preferably the range of 20°C to 80°C. Reaction time is usually 30 minutes to 72 hours.

Preparation 4a

Among compounds of formula (1a-1), a compound of formula (4a-3) is prepared, for example, by the following process:

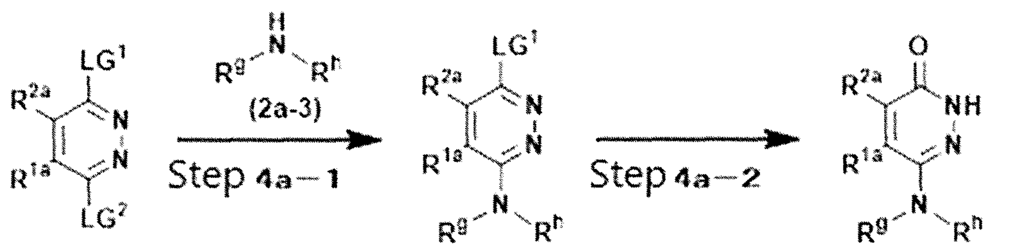

(4a-1) (4a-2) (4a-3)

wherein $R^{1a}$ and $R^{2a}$ are the same as those defined in the above (Item 6); $LG^1$ and $LG^2$ are each independently a leaving

group (for example, iodine atom, bromine atom, chlorine atom, and substituted sulfonyl (such as methanesulfonyl and p-toluenesulfonyl)); $R^g$ and $R^h$ are each independently the same as the definition of $R^e$ or $R^f$ defined in the above (Item 5); or alternatively, $R^g$ and $R^h$ may be taken together with the nitrogen atom to which they attach to form an optionally-substituted 4- to 12-membered saturated heterocyclic ring.

Step 4a-1: Preparation step of Compound (4a-2)

Compound (4a-2) is prepared from Compound (4a-1) and Compound (2a-3) according to the method described in Step 2a-2. As Compound (4a-1) and Compound (2a-3), a commercial product or a product synthesized by common methods (for example, WO 2004/006922, ACS Medicinal Chemistry Letters, 2012, 3, 903. etc.) or corresponding methods thereof may be used. The amount of Compound (2a-3) used herein is usually 1.0 equivalent to 1.5 equivalent, and preferably 1.05 equivalent to 1.2 equivalent, to the amount of Compound (4a-2) .

Step 4a-2: Preparation step of Compound (4a-3)

Compound (4a-3) is prepared from Compound (4a-2) according to common methods (for example, Bioorganic & Medicinal Chemistry Letters, 2013, 23, 2007., WO 2012/114268, etc.) or corresponding methods thereof.

Preparation 5a

Among compounds of Formula (1a-1), a compound of formula (5a-4) is prepared, for example, by the following process:

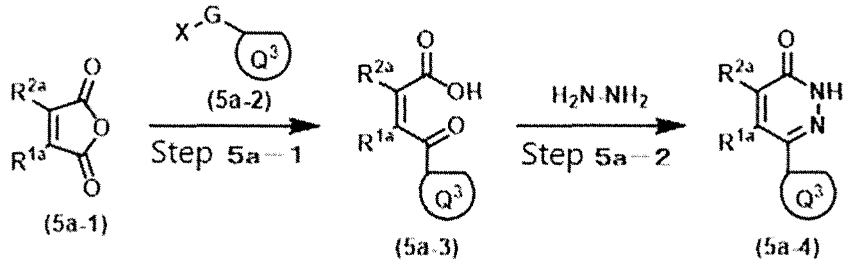


(5a-1) (5a-3) (5a-4)

wherein $R^{1a}$ and $R^{2a}$ are the same as those defined in the above (Item 6); $Q^3$ is optionally-substituted saturated or partially-unsaturated $C_{4-12}$ carbocyclyl or optionally-substituted 4- to 12-membered saturated heterocyclyl; G is a metallic species such as magnesium and zinc; and X is halogen atom.

Step 5a-1: Preparation step of Compound (5a-3)

Compound (5a-3) is prepared by reacting Compound (5a-1) with a organometallic compound (5a-2) such as Grignard reagent according to a known method (for example, Organic Letters, 2015, 17, 5517., Organic & Biomolecular Chemistry,2014, 12, 2049. etc.). As Compound (5a-1) and Compound (5a-2), a commercial product or a product synthesized by common methods (for example, Organic Letters,2008, 10, 4815., Journal of Organic Chemistry, 2015, 80, 12182., etc.) or corresponding methods thereof may be used.

Step 5a-2: Preparation step of Compound (5a-4)

Compound (5a-4) is prepared by reacting Compound (5a-3) with hydrazine according to a known method (for example, Journal of Medicinal Chemistry, 1993, 36, 4052., WO 2007/020343).

The present compound wherein Ring Cy is a group of formula (b) may be prepared in the manners of Preparations 1b - 6b.

Preparation 1b

Among compounds of Formula (1), a compound of formula (1b) is prepared, for example, by the following process:

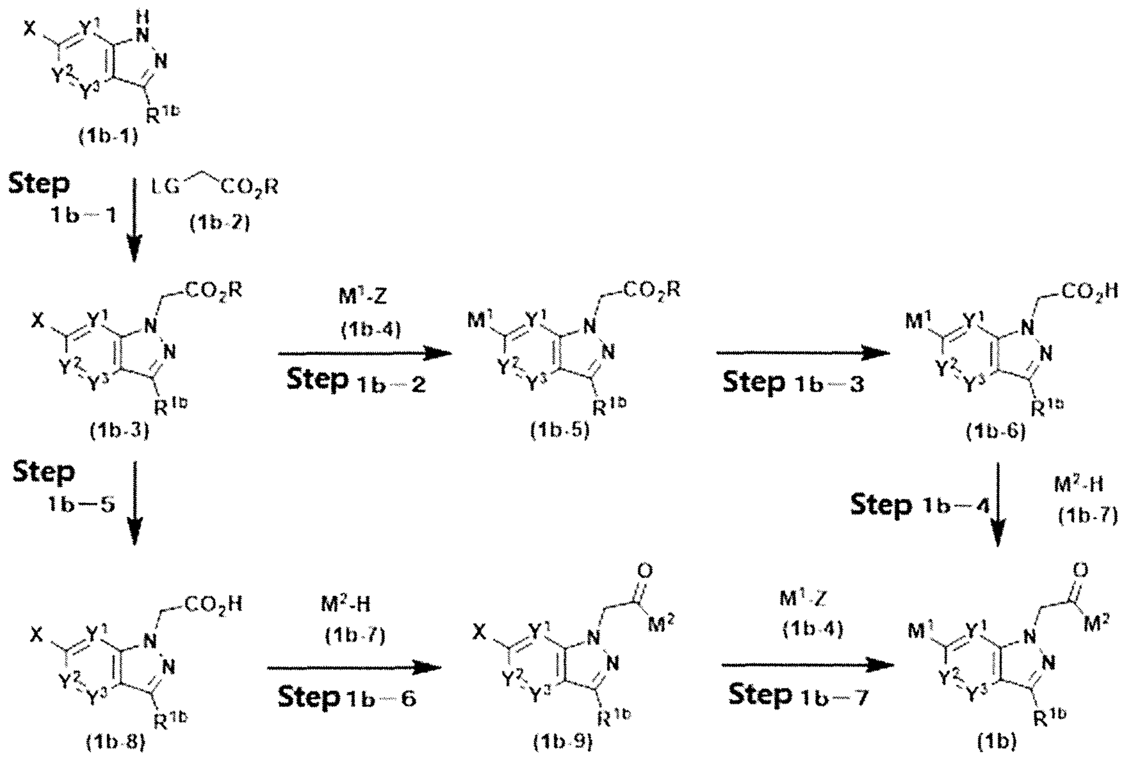


wherein $M^1$ and $M^2$ are the same as those defined in the above (Item 5); $R^{1b}$, $Y^1$, $Y^2$, and $Y^3$ are the same as those defined in the above (Item 6); R is $C_{1-6}$ alkyl; X is halogen atom; LG is a leaving group (for example, iodine atom, bromine atom, chlorine atom, and substituted sulfonyloxy (such as methanesulfonyloxy and p-toluenesulfonyloxy)); and Z is boronic acid, boronate, $BF_3K$, $BF_3Na$, trialkyltin, zinc halide, or hydrogen atom.

Step 1b-1: Preparation step of Compound (lb-3)

Compound (1b-3) can be prepared by reacting Compound (1b-1) with Compound (1b-2) in the presence of a base in an appropriate inert solvent. As Compound (1b-1), a product synthesized by common methods (for example, US 2005/0277655 A, WO 2018/081091, WO 2017/009798 A) or corresponding methods thereof, or a commercial product may be used. As Compound (1b-2), a commercial product or a product synthesized by common methods or corresponding methods thereof may be used.

Examples of the base used herein include inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; metal alkoxides such as sodium methoxide and potassium tert-butoxide; and organic bases such as triethylamine, diisopropylethylamine, and pyridine.

Examples of the inert solvent used herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; and mixed solvents thereof.

The reaction temperature is selected from, but not limited to, usually the range of -10°C to 200°C, preferably the range of 0°C to 40°C. The reaction time is usually 10 minutes to 48 hours, but it depends on conditions including reaction temperature, materials, and solvents which are used.

Step 1b-2: Preparation step of Compound (1b-5)

Among Compound (1b-5), the compound wherein $M^1$ is optionally-substituted partially-unsaturated $C_{4-12}$ carbocyclyl, optionally-substituted 4- to 12-membered partially-unsaturated heterocyclyl, optionally-substituted $C_{6-10}$ aryl, optionally-substituted 5- to 10-membered heteroaryl, or optionally-substituted $C_{2-10}$ alkenyl can be prepared by reacting Compound (1b-3) with Compound (1b-4) wherein Z is boronic acid, boronate, $BF_3K$, $BF_3Na$, trialkyltin, or zinc halide, in a suitable inert solvent, in the presence of a palladium catalyst and a phosphine ligand, optionally in the presence of a base. As Compound (1b-4), a commercial product or a product synthesized by common methods or corresponding

methods thereof may be used.

Examples of the palladium catalyst used herein include tetrakis(triphenylphosphine)palladium(0), bis(dibenzylideneacetone)palladium(0), tris(dibenzylideneacetone)dipalladium(0), bis(tri-tert-butylphosphine)palladium(0), palladium(0) acetate, [1,1-bis(diphenylphosphino)ferrocene]palladium(II) dichloride, and bis(di-tert-butyl(4-dimethylaminophenyl)phosphine)dichloropalladium(II).

Phosphine ligands include, for example, o-tolylphosphine, 2-dicyclohexylphosphino-2',6'-dimethoxybiphenyl (S-Phos), 2-dicyclohexylphosphino-2',4',6'-triisopropylbiphenyl (X-Phos), 1,1'-bis(diphenylphosphino)ferrocene (DPPF), 1,2-bis(diphenylphosphino)ethane (DPPE), 1,3-bis(diphenylphosphino)propane (DPPP), 1,4-bis(diphenylphosphino)butane (DPPB), 2,2'-bis(diphenylphosphino)-1,1'-binaphthyl (BINAP), 4,5-bis(diphenylphosphino)-9,9-dimethylxanthene (XANT-Phos), and bis(2-(diphenylphosphino)phenyl) ether (DPE-Phos).

Examples of the base used herein include sodium carbonate, potassium carbonate, cesium carbonate, potassium phosphate, sodium hydroxide, and potassium hydroxide.

Examples of the inert solvent include 1,4-dioxane, THF, 1,2-dimethoxyethane, acetonitrile, water, and mixed solvents thereof.

The reaction temperature is selected from, but not limited to, usually the range of 50°C to 200°C, preferably the range of 80°C to 150°C. The present step can be conducted under microwave irradiation, if necessary. Reaction time is usually 30 minutes to 48 hours.

Among Compound (1b-5), the compound wherein $M^1$ is optionally-substituted $C_{1-10}$ alkoxy, optionally-substituted $C_{6-10}$ aryloxy, optionally-substituted 5- to 10-membered heteroaryloxy, or $-NR^eR^f$ wherein $R^e$ and $R^f$ are the same as those defined in the above (Item 5) can be prepared by reacting Compound (lb-3) with Compound (1b-4) wherein Z is hydrogen atom, in a suitable inert solvent. Among Compound (1b-5), the compound wherein $M^1$ is $-NR^eR^f$ wherein $R^e$ and $R^f$ are the same as those defined in the above (Item 5) can be also prepared by reacting Compound (1b-3) with Compound (1b-4) wherein Z is hydrogen atom, in a suitable inert solvent, in the presence of a palladium catalyst, a phosphine ligand, and a base. As Compound (lb-4), a commercial product or a product synthesized by common methods or corresponding methods thereof may be used.

Examples of the base used herein include organic bases such as triethylamine, diisopropylethylamine, and pyridine; inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; and metal alkoxides such as sodium methoxide and potassium tert-butoxide.

Examples of the inert solvent used herein include aprotic polar solvents such as dimethylformamide, N-methyl-2-pyrrolidinone, dimethylsulfoxide, acetonitrile, acetone, and methyl ethyl ketone; halogenated hydrocarbon solvents such as chloroform and dichloromethane; aromatic hydrocarbon solvents such as benzene and toluene; and mixed solvents thereof.

Examples of the palladium catalyst herein include tetrakis(triphenylphosphine) palladium(1), bis(dibenzylideneacetone)palladium(0), tris(dibenzylideneacetone)dipalladium(0), bis(tri-tert-butylphosphine)palladium(0), palladium(0) acetate, [1,1-bis(diphenylphosphino)ferrocene]palladium(II) dichloride, and bis(di-tert-butyl(4-dimethylaminophenyl)phosphine)dichloropalladium(II).

Phosphine ligands include, for example, o-tolylphosphine, 2-dicyclohexylphosphino-2',6'-dimethoxybiphenyl (S-Phos), 2-dicyclohexylphosphino-2',4',6'-triisopropylbiphenyl (X-Phos), 1,1'-bis(diphenylphosphino)ferrocene (DPPF), 1,2-bis(diphenylphosphino)ethane (DPPE), 1,3-bis(diphenylphosphino)propane (DPPP), 1,4-bis(diphenylphosphino)butane (DPPB), 2,2'-bis(diphenylphosphino)-1,1'-binaphthyl (BINAP), 4,5-bis(diphenylphosphino)-9,9-dimethylxanthene (XANT-Phos), and bis(2-(diphenylphosphino)phenyl) ether (DPE-Phos).

The reaction temperature is selected from, but not limited to, usually the range of 20°C to 200°C, preferably the range of 50°C to 170°C. The present step can be conducted under microwave irradiation, if necessary. The reaction time is usually 10 minutes to 48 hours, but it depends on conditions including reaction temperature, materials, and solvents which are used.

In addition, the present step may be done by replacing X with another leaving group such as alkyl sulfonyl group, followed by reacting with Compound (1b-4), according to known methods.

Step 1b-3: Preparation step of Compound (1b-6)

Compound (1b-6) is prepared by hydrolyzing or hydrogenating Compound (1b-5) according to a known method (for example, Protective Groups in Organic Synthesis 3rd Edition (John Wiley & Sons, Inc.), Comprehensive Organic Transformation, R. C. Laroque et al, VCH publisher Inc., 1989, etc.).

Step 1b-4: Preparation step of Compound (1b)

The compound of formula (1b) is prepared by reacting Compound (1b-6) with Compound (1b-7) in the presence or absence of a base in an appropriate inert solvent using a condensing agent. As Compound (1b-7), a commercial product or a product synthesized by common methods or corresponding methods thereof may be used.

Examples of the condensing agent include dicyclohexylcarbodiimide (DCC), diisopropylcarbodiimide (DIPC), 1-ethyl-3-(3-dimethylaminopropyl)-carbodiimide (WSC), benzotriazol-1-yl-tris(dimethylamino)phosphonium hexafluorophosphate (BOP), diphenylphosphoryl azide (DPPA), N,N-carbonyldiimidazole (CDI), benzotriazol-1-yl-N,N,N',N'-tetramethyluronium hexafluorophosphate (HBTU), 7-azabenzotriazol-1-yl-N,N,N',N'-tetramethyluronium hexafluorophosphate (HATU). If necessary, additives such as N-hydroxysuccinimide (HOSu), 1-hydroxybenzotriazole (HOBt), and 3-hydroxy-4-oxo-3,4-dihydro-1,2,3-benzotriazine (HOOBt) may be added to the reaction.

Examples of the base used herein include organic bases such as triethylamine, diisopropylethylamine, pyridine, and 4-(dimethylamino)pyridine; inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; and metal alkoxides such as sodium methoxide and potassium tert-butoxide.

Examples of the inert solvent herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; and mixed solvents thereof.

The reaction temperature is selected from, but not limited to, usually the range of -10°C to 200°C, preferably the range of 0°C to 40°C. The reaction time is usually 10 minutes to 48 hours, but it depends on conditions including reaction temperature, materials, and solvents which are used.

In addition, the present step may be done by activating the carboxyl group in Compound (lb-6) to transform an acid anhydride, mixed acid anhydride, or acid halide thereof, followed by reacting with Compound (1b-7), according to known methods.

Step 1b-5: Preparation step of Compound (1b-8)

Compound (1b-8) is prepared from Compound (1b-3) according to the method described in Step 1b-3.

Step 1b-6: Preparation step of Compound (1b-9)

Compound (1b-9) is prepared from Compound (1b-8) and Compound (1b-7) according to the method described in Step 1b-4.

Step 1b-7: Preparation step of Compound (1b)

Compound (1b) is prepared from Compound (1b-9) and Compound (1b-4) according to the method described in Step 1b-2.

<u>Preparation 2b</u>

A compound of formula (1b-3) is prepared, for example, by the following process:

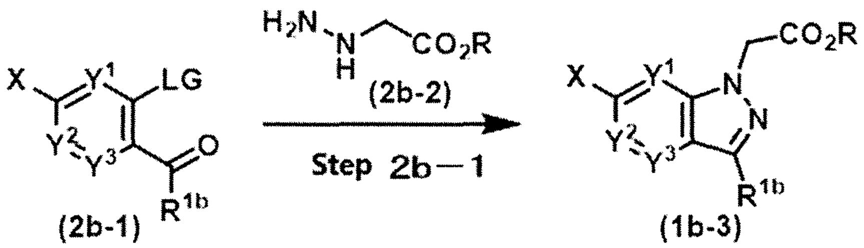


wherein $R^{1b}$, $Y^1$, $Y^2$, and $Y^3$ are the same as those defined in the above (Item 6); R is $C_{1-6}$ alkyl; X is halogen atom; LG is a leaving group (for example, iodine atom, bromine atom, chlorine atom, and substituted sulfonyloxy (such as methanesulfonyloxy and p-toluenesulfonyloxy)).

Step 2b-1: Preparation step of Compound (1b-3)

Compound (1b-3) is prepared by reacting Compound (2b-1) with Compound (2b-2) in a suitable inert solvent.

Examples of the inert solvent herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; aromatic hydrocarbon solvents such as benzene and toluene; alcohol solvents such as methanol, ethanol, 2-propanol, tert-butanol, and 1-butanol, and mixed solvents thereof.

The reaction temperature is selected from, but not limited to, usually the range of -10°C to 200°C, preferably the range of 20°C to 150°C. The reaction time herein is usually 10 minutes to 48 hours, but it depends on conditions including reaction temperature, materials, and solvents which are used.

Preparation 3b

A compound of formula (1b-3) is prepared, for example, by the following process:

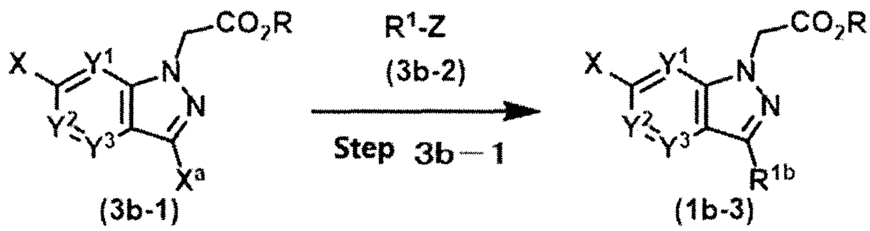


wherein $Y^1$, $Y^2$, and $Y^3$ are the same as those defined in the above (Item 6); $R^{1b}$ is cyano, optionally-substituted $C_{1-6}$ alkyl, or optionally-substituted $C_{3-6}$ cycloalkyl; R is $C_{1-6}$ alkyl; X is halogen atom; $X^a$ is iodine atom or bromine atom; Z is boronic acid, boronate, $BF_3K$, $BF_3Na$, or zinc cyanide.

Step 3b-1: Preparation step of Compound (1b-3)

Compound (1b-3) is prepared by reacting Compound (3b-1) with Compound (3b-2) in the presence of a palladium catalyst and a phosphine ligand, optionally in the presence of a base in a suitable inert solvent. Compound (3b-1) is prepared according to the method described in Step 1b-1. As Compound (3b-2), a commercial product or a product synthesized by common methods or corresponding methods thereof may be used.

Examples of the palladium catalyst herein include tetrakis(triphenylphosphine)palladium(0), bis(dibenzylideneacetone)palladium(0), tris(dibenzylideneacetone)dipalladium(0), bis(tri-tert-butylphosphine)palladium(0), palladium(0) acetate, [1,1-bis(diphenylphosphino)ferrocene]palladium(II) dichloride, and bis(di-tert-butyl(4-dimethylaminophenyl)phosphine)dichloropalladium(II).

Phosphine ligands include, for example, o-tolylphosphine, 2-dicyclohexylphosphino-2',6'-dimethoxybiphenyl (S-Phos), 2-dicyclohexylphosphino-2',4',6'-triisopropylbiphenyl (X-Phos), 1,1'-bis(diphenylphosphino)ferrocene (DPPF), 1,2-bis(diphenylphosphino)ethane (DPPE), 1,3-bis(diphenylphosphino)propane (DPPP), 1,4-bis(diphenylphosphino)butane (DPPB), 2,2'-bis(diphenylphosphino)-1,1'-binaphthyl (BINAP), 4,5-bis(diphenylphosphino)-9,9-dimethylxanthene (XANT-Phos), and bis(2-(diphenylphosphino)phenyl) ether (DPE-Phos).

Examples of the base used herein include sodium carbonate, potassium carbonate, cesium carbonate, potassium phosphate, sodium hydroxide, and potassium hydroxide.

Examples of the inert solvent used herein include 1,4-dioxane, THF, 1,2-dimethoxyethane, acetonitrile, dimethylformamide, water, and mixed solvents thereof.

The reaction temperature is selected from, but not limited to, usually the range of 50°C to 200°C, preferably the range of 80°C to 150°C. The present step can be conducted under microwave irradiation, if necessary. Reaction time is usually 30 minutes to 48 hours.

Preparation 4b

Among compounds of Formula (1), a compound of formula (4b-2) is prepared, for example, by the following process:

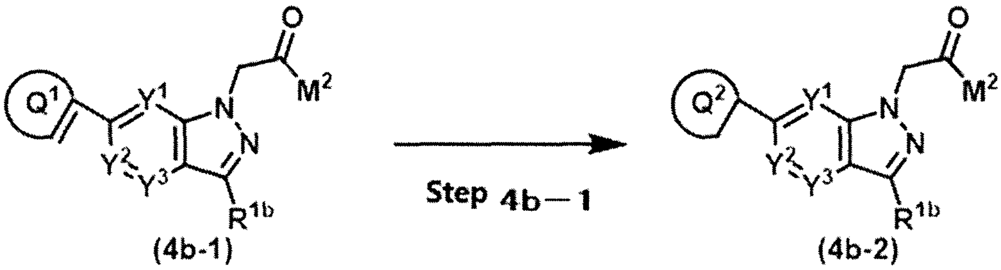

(4b-1) (4b-2)

wherein $M^2$ is the same as those defined in the above (Item 5); $R^{1b}$, $Y^1$, $Y^2$, and $Y^3$ are the same as those defined in the above (Item 6); $Q^1$ is optionally-substituted 4- to 12-membered partially-unsaturated heterocyclyl, or partially-unsaturated $C_{4-12}$ carbocyclyl; $Q^2$ is optionally-substituted 4- to 12-membered saturated heterocyclyl, or saturated $C_{4-12}$ carbocyclyl.

Step 4b-1: Preparation step of Compound (4b-2)

Compound (4b-2) is prepared by catalytic reduction of Compound (4b-1) with a metal catalyst in a suitable inert solvent under hydrogen atmosphere. Compound (4b-1) is prepared, for example, according to the method described in Preparation 1b.

Examples of the metal catalyst used herein include palladium/carbon, palladium hydroxide/carbon, Raney nickel, platinum oxide/carbon, and rhodium/carbon. The amount of a metal catalyst is usually 0.1 % to 1000 % by weight to Compound (4-1), and preferably 1 % to 100 % by weight.

Examples of the inert solvent used herein include ethers such as tetrahydrofuran; esters such as ethyl acetate; and alcohols such as methanol.

The hydrogen pressure is usually 1 to 100 atm, and preferably 1 to 5 atm. Or, the present process can be done using ammonium formate or other reagents, instead of hydrogen atmosphere.

The reaction temperature is selected from, but not limited to, usually the range of 0°C to 120°C, preferably the range of 20°C to 80°C. Reaction time is usually 30 minutes to 72 hours.

Preparation 5b

A compound of formula (1b-9) is prepared, for example, by the following process:

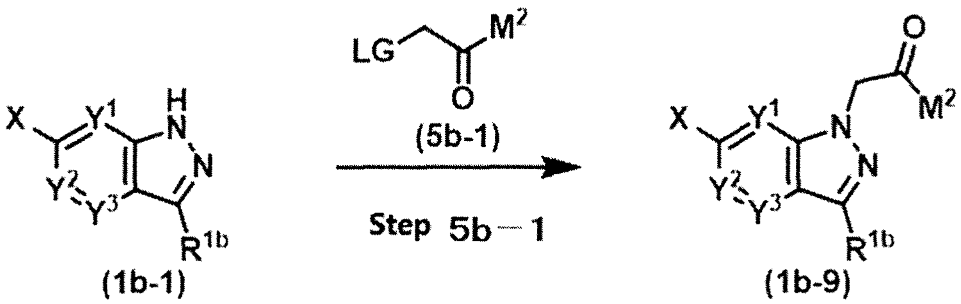

(1b-1) (1b-9)

wherein $M^2$ is the same as those defined in the above (Item 5); $R^{1b}$, $Y^1$, $Y^2$, and $Y^3$ are the same as those defined in the above (Item 6); X is halogen atom; LG is a leaving group (for example, iodine atom, bromine atom, chlorine atom, and substituted sulfonyloxy (such as methanesulfonyloxy and p-toluenesulfonyloxy)).

Step 5b-1: Preparation step of Compound (1b-9)

Compound (1b-9) is prepared from Compound (1b-1) and Compound (5b-1) according to the method described in Step 1b-1. As Compound (5b-1), a commercial product or a product synthesized by common methods or corresponding methods thereof may be used.

Preparation 6b

Among compounds of Formula (1), a compound of formula (6b-4) wherein $M^1$ is $-NR^eR^f$ is also prepared, for

example, by the following process:

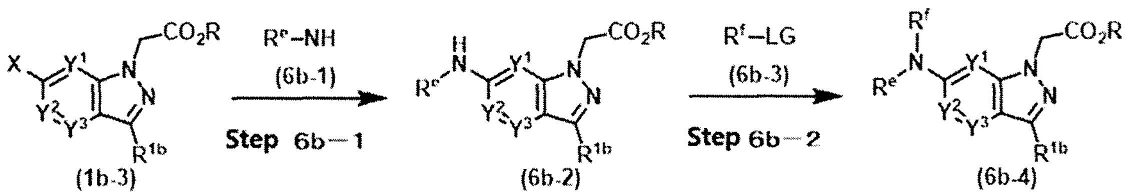


wherein $R^{1b}$, $Y^1$, $Y^2$, and $Y^3$ are the same as those defined in the above (Item 6); $R^e$ and $R^f$ are the same as those defined in the above (Item 5); X is halogen atom; R is $C_{1-6}$ alkyl; LG is a leaving group (for example, iodine atom, bromine atom, chlorine atom, and substituted sulfonyloxy (such as methanesulfonyloxy and p-toluenesulfonyloxy)).

Step 6b-1: Preparation step of Compound (6b-2)

Compound (6b-2) is also prepared from Compound (1b-3) and Compound (6b-1) according to the method described in Step 1b-2. As Compound (6b-1), a commercial product or a product synthesized by common methods or corresponding methods thereof may be used.

Step 6b-2: Preparation step of Compound (6b-4)

Compound (6b-4) is prepared by reacting Compound (6b-2) with Compound (6b-3) in the presence of a base in a suitable inert solvent.

Examples of the base used herein include inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; metal alkoxides such as sodium methoxide and potassium tert-butoxide; and organic bases such as triethylamine, diisopropylethylamine, and pyridine.

Examples of the inert solvent used herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; aromatic hydrocarbon solvents such as benzene and toluene; and mixed solvents thereof.

The reaction temperature is selected from, but not limited to, usually the range of -78°C to 200°C, preferably the range of 0°C to 180°C. The present step can be conducted under microwave irradiation, if necessary. The reaction time is usually 10 minutes to 48 hours, but it depends on conditions including reaction temperature, materials, and solvents which are used.

Among groups of $M^2$, when a corresponding reagent is not commercially available, the amine can be prepared, for example, according to the method of Preparation 7 or 8.

Preparation 7

A compound of formula (7-4) is prepared, for example, by the following process:

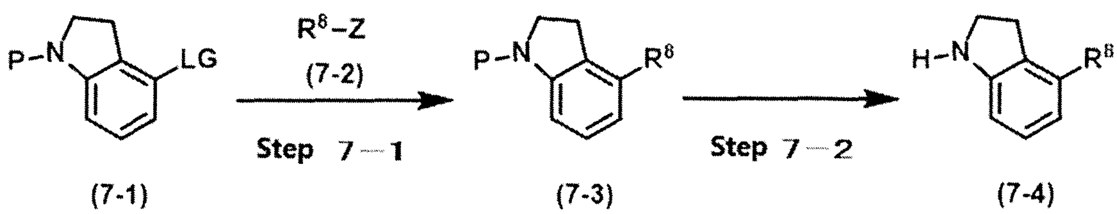


wherein P is a protective group for amino group; LG is a leaving group (for example, iodine atom, bromine atom, chlorine atom, and substituted sulfonyloxy (such as methanesulfonyloxy and p-toluenesulfonyloxy)); $R^8$ is optionally-substituted partially-unsaturated $C_{3-7}$ carbocyclyl, optionally-substituted amino, optionally-substituted 5- or 6-membered heteroaryl, or optionally-substituted 4- to 7-membered partially-unsaturated heterocyclic ring; and Z is boronic acid, boronate, $BF_3K$, $BF_3Na$, or hydrogen atom.

Step 7-1: Preparation step of Compound (7-3)

Compound (7-3) is prepared from Compound (7-1) and Compound (7-2) according to the method described in Step 1b-2. As Compound (7-1) and Compound (7-2), a commercial product or a product synthesized by common methods or corresponding methods thereof may be used.

Step 7-2: Preparation step of Compound (7-4)

Compound (7-4) is prepared from Compound (7-3) by deprotection according to a known method (for example, Protective Groups in Organic Synthesis 3rd Edition (John Wiley & Sons, Inc.), Comprehensive Organic Transformation, R. C. Laroque et al, VCH publisher Inc., 1989, etc.).

Preparation 8

A compound of formula (8-7) is prepared, for example, by the following process:

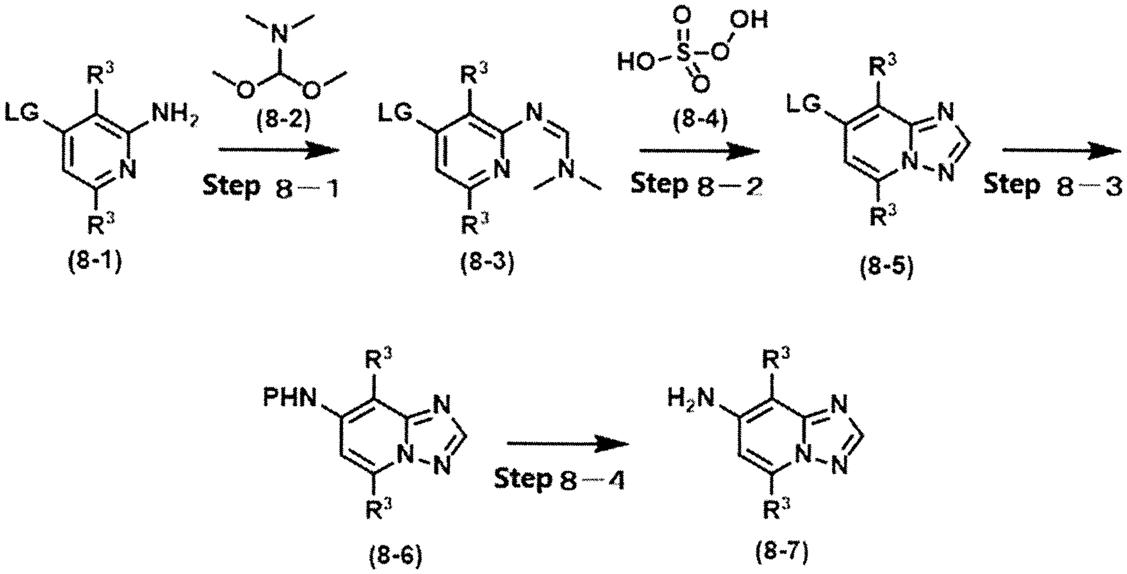


(8-1) (8-2) (8-3) (8-4) (8-5) (8-6) (8-7)

wherein $R^3$ is the same as those defined in the above (Item 5); LG is a leaving group (for example, iodine atom, bromine atom, chlorine atom, and substituted sulfonyloxy (such as methanesulfonyloxy and p-toluenesulfonyloxy)); and P is a protective group for amino group.

Step 8-1: Preparation step of Compound (8-3)

Compound (8-3) is prepared by reacting Compound (8-1) with Compound (8-2) in an inert solvent. As Compound (8-1) and compound (8-2), a commercial product or a product synthesized by common methods or corresponding methods thereof may be used.

Examples of the inert solvent include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; and mixed solvents thereof. Or, the reaction may be done without a solvent.

The reaction temperature is selected from, but not limited to, usually the range of 0°C to 200°C, preferably the range of 40°C to 150°C. The reaction time is usually 10 minutes to 48 hours, but it depends on conditions including reaction temperature, materials, and solvents which are used.

Step 8-2: Preparation step of Compound (8-5)

Compound (8-5) is prepared by reacting Compound (8-3) with Compound (8-4) in the presence of a base in a suitable inert solvent. As Compound (8-4), a commercial product or a product synthesized by common methods or corresponding methods thereof may be used.

Examples of the base used herein include organic bases such as triethylamine, diisopropylethylamine, pyridine, and 4-(dimethylamino)pyridine; inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; and metal alkoxides such as sodium methoxide and potassium tert-butoxide;.

Examples of the inert solvent used herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; alcohol solvents such as methanol, ethanol, 2-propanol, and tert-butanol; and mixed solvents thereof.

The reaction temperature is selected from, but not limited to, usually the range of -10°C to 200°C, preferably the range of 0°C to 40°C. The reaction time is usually 10 minutes to 48 hours, but it depends on conditions including reaction temperature, materials, and solvents which are used.

And, the present Step may also be done according to a known method (for example, European Journal of Organic Chemistry 2005, 3761.).

Step 8-3: Preparation step of Compound (8-6)

Compound (8-6) is prepared from Compound (8-5) according to the method described in Step 1b-2.

Step 8-4: Preparation step of Compound (8-7)

Compound (8-7) is prepared from Compound (8-6) by deprotection according to a known method (for example, Protective Groups in Organic Synthesis 3rd Edition (John Wiley & Sons, Inc.), Comprehensive Organic Transformation, R. C. Laroque et al, VCH publisher Inc., 1989, etc.).

The compound of formula (1) having desired substituent(s) at desired positon(s) can be prepared by suitably combining the above-mentioned Preparations.

The intermediates and the desired compounds in the preparations described above can be isolated and purified by a method commonly-used in organic synthetic chemistry, for example, filtration, extraction, washing, drying, enrichment, crystallization, various chromatography, and the like, or a combination thereof. In addition, intermediates may be used in next reaction without further purification.

Starting compounds or intermediates described in the above-mentioned Preparations may be in salt form such as hydrochloride, depending on each reaction condition. In such case, they may be used in salt form itself or in free form. If starting compounds or intermediates are in salt form, and the salt form should be transformed to free form, it can be transformed to free form by dissolving or suspending it in an appropriate solvent and then neutralizing it with a base such as aqueous sodium bicarbonate.

The optical isomers can also be separated by common separation processes such as methods using an optically active column or fractional crystallization at an appropriate step of the above preparation processes. An optically active material can be used as a starting material.

In the case where a salt of a compound of formula (1) is needed, when the compound of formula (1) is obtained in a salt form, the salt can be obtained by purification of the obtained salt, and when the compound of formula (1) is obtained in a free form, the salt can be formed by dissolving or suspending the compound of formula (1) in an appropriate solvent, followed by addition of an acid or base.

The "tauopathy" used herein is a general term for a sporadic or familial pathology that tau protein is abnormally phosphorylated to be insoluble, and the phosphorylated tau is abnormally accumulated in cells. The "tauopathy" includes, for example, Alzheimer's disease (AD), Alzheimer-type dementia (ATD), dementia of Alzheimer's type (DAT), dementia in Alzheimer disease, major neurocognitive disorder due to Alzheimer's disease, diffuse neurofibrillary tangles with calcification (DNTC), amyotrophic lateral sclerosis/parkinsonism-dementia complex of Guam island (Guam ALS/PDC), amyotrophic lateral sclerosis/parkinsonism-dementia complex of the Kii peninsula (Kii ALS/PDC), frontotemporal lobar degeneration (FTLD; such as Pick's disease (PiD), progressive supranuclear palsy (PSP), corticobasal degeneration (CBD), argyrophilic grain dementia (AGD), globular glial tauopathy (GGT) and frontotemporal dementia and parkinsonism linked to chromosome 17 (FTDP-17)), senile dementia of the neurofibrillary tangle type (SD-NFT), Down syndrome (DS), chronic traumatic encephalopathy (CTE), myotonic dystrophy (DM), Niemann-Pick disease type C (NPC), static encephalopathy of childhood with neurodegeneration in adulthood (SENDA), PLA2G6-associated neurodegeneration (PLAN), Gerstmann-Straussler-Scheinker disease (GSS), familial British dementia (FBD), familial Danish dementia (FDD), post-encephalitic Parkinsonism (PEP), subacute sclerosing panencephalitis (SSPE), and SLC9A6-related mental retardation, but should not be limited thereto. For example, when phosphorylated tau is abnormally accumulated in brain, the pathology may be within "tauopathy". It may be determined that any two or more of these diseases are applicable at the same

time, depending on the differences of Patient's condition and diagnostic method. And, a pathological condition in which two or more of these diseases coexist, that is, a combination of any two or more diseases of the above-mentioned diseases is also included in "tauopathy". Characteristic inclusion bodies may be formed for each disease, and there are differences in the types of cells that accumulate, the isoforms of tau that accumulate, and the phosphorylation sites of tau. In neurodegenerative diseases associated with "tauopathy", the accumulation of abnormally phosphorylated tau protein is thought to cause cerebral atrophy, cerebral dysfunction, etc.

The action of a compound on cognitive dysfunction of tauopathy can be evaluated by the method described in Test 3 described later. And, the effect of a compound on the accumulation of tau in tauopathy can be evaluated by the method described in Test 4 described later, and the effect of a compound on the accumulation of tau in tauopathy and cerebral atrophy can be evaluated by the method described in Test 5. A compound that is effective in any of Tests 3 to 5 can be expected to have a therapeutic and/or preventive effect on tauopathy.

The "Nav activator" and "Nav1.1 activator" can be used in combination with other drugs (hereinafter, optionally referred to as "concomitant drug") prescribed for the purpose of enhancing the effect and/or reducing side effects and for various symptoms of various diseases. The concomitant drug includes, for example, another medicament for treating tauopathy, a medicament for treating Alzheimer-type dementia, antiepileptic agent, antipsychotic agent, antidepressive agent, anti-anxiety agent, Chinese herbal drug, sleep-inducing drug, antiparkinsonian agent, antihypertensive drug, anti-inflammatory agent, antidiabetic drug, antihyperlipidemic drug, anti-obesity agent, antiemetic drug, a medicament for treating dysphagia, and a medicament for treating dysuria, cathartic drug. More preferably, it includes another medicament for treating tauopathy, a medicament for treating Alzheimer-type dementia, antiepileptic agent, antipsychotic agent, antidepressive agent, anti-anxiety agent, Chinese herbal drug, sleep-inducing drug, and antiparkinsonian agent. The "another medicament for treating tauopathy" means any tauopathy therapeutic agent that is different from the Nav activator used.

The concomitant drug includes, for example, acetylcholinesterase inhibitors such as donepezil, NMDA inhibitors such as memantine, GABA signal enhancers such as valproic acid, SV2A ligands such as levetiracetam, and medicaments for treating partial seizure such as carbamazepine. And, the concomitant drug may include one or more medicament selected from the group consisting of atypical antipsychotic agent for behavioral and psychological symptoms of dementia (BPSD) such as quetiapine, risperidone, clozapine, and aripiprazole; selective serotonin reuptake inhibitor (SSRI) such as paroxetine, sertraline, and citalopram; serotonin antagonist reuptake inhibitor (SARI) such as trazodone; noradrenergic and specific serotonergic antidepressant (NaSSA) such as mirtazapine; serotonin 1A receptor agonist such as tandospirone; Chinese herbal drug such as yokukansan; and Parkinson's disease drug such as levodopa.

In an embodiment, the Nav activator may exhibit selective pharmacological action on some Nav subtypes, for example, one or more Nav subtypes selected from the group consisting of Nav1.1, Nav1.2, Nav1.3 and Nav1.6, and it may have a weak effect on other Nav subtypes such as Nav1.5. It can be expected that the Nav activator having a reduced action on Nav1.5 is highly safe with less concern about cardiotoxicity.

The "preventing" used herein means the act of administering a drug or a medicament to a healthy person who has not developed a disease, and is intended, for example, to prevent the onset or development of a disease. The "treating" used herein means the act of administering a drug or a medicament to a person, *i.e.*, a patient who has been diagnosed by a doctor as being affected with a disease to improve the symptom, release the symptom, or inhibit the progress of disease.

The "therapeutically effective amount" used herein means the amount of a drug or medicament that elicits a biological or pharmaceutical response required by a researcher or physician in a tissue, system, animal or human.

A Nav activator may be administered directly via an appropriate route of administration, or administered in an appropriate dosage form after formulation.

As a route of administration, it is preferable to use the most effective route for treatment, which may vary depending on the embodiment of a Nav activator such as a small molecule compound, a peptide, and an antibody. The route of administration includes, for example, oral; and parenteral administration such as intravenous administration, application, inhalation, and eye drop. The route of administration is preferably oral administration.

The dosage form used herein may suitably vary depending on the embodiment of a Nav activator, which includes, for example, a tablet, a capsule, a powder, a granule, a liquid, a suspension, an injection, a patch, and a poultice. The dosage form is preferably a tablet.

The formulation into a dosage form or a pharmaceutical composition can be carried out according to common methods using pharmaceutically acceptable additives.

As a pharmaceutically acceptable additive, an excipient, a disintegrant, a binder, a fluidizer, a lubricant, a coating, a solubilizer, a solubilizing adjuvant, a thickener, a dispersant, a stabilizing agent, a sweetening agent, a flavor, and the like can be used, depending on a purpose. Specifically, examples of the pharmaceutically acceptable additive herein include lactose, mannitol, crystalline cellulose, low-substituted hydroxypropylcellulose, corn starch, partially-pregelatinized starch, carmellose calcium, croscarmellose sodium, hydroxypropylcellulose, hydroxypropylmethylcellulose, polyvinyl alcohol, magnesium stearate, sodium stearyl fumarate, polyethylene glycol, propylene glycol, titanium

oxide, and talc.

The amount and the frequency of administration of the dosage form or pharmaceutical composition comprising a Nav activator can be optionally determined depending on an embodiment of a Nav activator , a mode of administration, a disease of a patient or symptoms thereof, the age or weight of a patient, and the like. When the Nav activator is a small molecule compound, the amount of the active ingredient (herein, also referred to as "therapeutically effective amount") per day can be usually administered to an adult in several portions in a day, preferably in one to three portions in a day, wherein the amount ranges from about 0.0001 to about 5000 mg, more preferably from about 0.001 to about 1000 mg, further preferably from about 0.1 to about 500 mg, especially preferably from about 1 to about 300 mg.

The timing to administer a Nav activator and a concomitant drug is not limited, and they may be administered to a subject needed in treatment concurrently or with a time lag. A Nav activator may be formulated as a combination medicament with a concomitant drug. The dose or mixing ratio of the concomitant drug can be optionally selected depending on an embodiment of a Nav activator, a subject to be administered, a route of administration, a targeted disease, symptoms, and combination thereof, on the basis of the doses in the clinical use. For example, when the subject for administration is a human and the Nav activator is a small molecule compound, the concomitant drug for combination use may be used in 0.01 to 100 parts by weight to 1 part by weight of the Nav activator.

EXAMPLES

The present invention is explained in more detail in the following by referring to Reference examples, Examples, and Tests; however, the present invention is not limited thereto. The names of compounds in the following Reference examples and Examples do not necessarily conform to the IUPAC nomenclature.

In the present specification, the abbreviations shown below may be used.

$CDCl_3$: deuterated chloroform
DMSO-$d_6$: deuterated dimethylsulfoxide
Rt: retention time
min: minute(s)
HATU: O-(7-aza-1H-benzotriazol-1-yl)-N,N,N',N'-tetramethyluronium hexafluorophosphate
THF: tetrahydrofuran
TFA: trifluoroacetic acid
DMF: N,N-dimethylformamide
Boc: tert-butoxycarbonyl
Tf: trifluoromethanesulfonyl
Cbz: benzyloxycarbonyl
Ph: phenyl
WSC: 1-(3-dimethylaminopropyl)-3-ethylcarbodiimide

Physicochemical data of each compound in Examples and Reference examples were obtained with the following apparatus:
$^1$H-NMR: JEOL JNM-AL400; JEOL JNM-ECS400; Brucker AVANCE 400 Spectrometer

Symbols used in NMR are defined as follows: s for singlet, d for doublet, dd for doublet of doublets, t for triplet, td for triplet of doublets, dt for doublet of triplets, q for quartet, m for multiplet, br for broad singlet or multiplet, and J for coupling constant.

LC/MS data of each compound in Examples and Reference examples were obtained with any one of the following apparatuses:

Method A

Detection apparatus: ACQUITY™ SQ deteceter (Waters Corporation)
HPLC: ACQUITY™ UPLC SYSTEM
Column: Waters ACQUITY™ UPLC BEH C18 (1.7 pm, 2.1 mm x 30 mm)

Method B

Detection apparatus: Shimadzu LCMS-2020
Column: Phenomenex Kinetex (C18, 1.7 pm, 2.1 mm x 50 mm)

Method C

Detection apparatus: Agilent 6110 Quadropole LC/MS
HPLC: Agilent 1200 series
Column: XBridge C18 (3.5 μm, 4.6 mm x 50 mm)

Method D

Detection apparatus: ACQUITY™ SQ deteceter (Waters Corporation)
HPLC: ACQUITY™ UPLC SYSTEM
Column: Waters ACQUITY™ UPLC BEH C18 (1.7 pm, 2.1 mm x 30 mm)

High performance liquid chromatograph-mass spectrometer; Measurement conditions for LC/MS are shown below, observed values of mass spectrometry [MS(m/z)] are shown in $MH^+$, and retention times are shown in Rt (minutes). In each observed value, measurement conditions used for the measurement are described as any one of A to D.

Method A

Solvents: Solution A; 0.06 % formic acid/$H_2O$, Solution B; 0.06 % formic acid/acetonitrile
Gradient condition: 0.0 to 1.3 minutes (linear gradient of B from 2 % to 96 %)
Flow rate: 0.8 mL/min; Detection UV: 220 nm and 254 nm; Temperature: 40°C

Method B

Solvents: Solution A; 0.05 % TFA/$H_2O$, Solution B; acetonitrile
Gradient condition: 0.0 to 1.7 minutes (linear gradient of B from 10 % to 99 %)
Flow rate: 0.5 mL/min; Detection UV: 220 nm; Temperature: 40°C

Method C

Solvents: Solution A; 10 mM $NH_4HCO_3$/$H_2O$, Solution B; acetonitrile
Gradient condition: 0.0 to 0.2 minutes (5 % B), 0.2 to 1.5 minutes (linear gradient of B from 5 % to 95 %), 1.5 to 2.8 minutes (95 % B)
Flow rate: 1.8 mL/min; Detection UV: 214 nm and 254 nm; Temperature 50°C

Method D

Solvents: Solution A; 0.05 % formic acid/$H_2O$, Solution B; acetonitrile
Gradient condition: 0.0 to 1.3 minutes (linear gradient of B from 10 % to 95 %), 1.3 to 1.5 minutes (10 % B)
Flow rate: 0.8 mL/min; Detection UV: 220 nm and 254 nm;
Temperature: 40°C

Reference example 1

2-Chloro-N-(4-cyanophenyl) acetamide

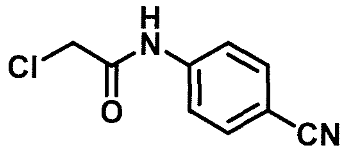

To a suspension of 4-aminobenzonitrile (25.2 g) and potassium carbonate (35.4 g) in acetone (200 ml) was added dropwise 2-chloroacetyl chloride (28.9 g) at 0°C, and the mixture was heated under reflux for 2 hours. After cooling to room temperature, the reaction mixture was poured slowly into water (400 ml), resulting in precipitation of a solid. The precipitated solid was filtered, washed with water, and dried under reduced pressure to obtain the titled compound (35.0 g).

$^1$H-NMR (400 MHz, DMSO-$d_6$) δ: 10.71 (s, 1H), 7.80 (d, J = 9.2 Hz, 2H), 7.76 (d, J = 9.2 Hz, 2H), 4.30 (s, 2H).

Reference example 2

6-(4-Methylpiperidin-1-yl)pyridazin-3(2H)-one

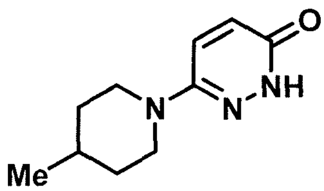

To a solution of 3,6-dichloropyridazine (34.3 g) in dimethylformamide (288 mL) were added 4-methylpiperidine (27.4 g) and triethylamine (48.1 mL). The reaction mixture was stirred at 80°C for 8 hours, and concentrated under reduced pressure. After the addition of saturated aqueous sodium bicarbonate (200 mL) and water (200 mL) thereto, the mixture was extracted twice with ethyl acetate. The obtained organic layers were combined, dried over sodium sulfate, and concentrated. The residue was dissolved in acetic acid (460 mL), and the mixture was heated under reflux for 37 hours. The reaction mixture was cooled to room temperature, and then concentrated under reduced pressure. To the residue were added 10 % aqueous sodium hydroxide (300 mL) and diethyl ether (150 mL), resulting in precipitation of a solid. The precipitated solid was filtered, washed sequentially with water, diethyl ether, and ethyl acetate, and dried under reduced pressure to obtain the titled compound (31.6 g).

$^1$H-NMR (400 MHz, $CDCl_3$) δ: 7.17 (d, J = 10.5 Hz, 1H), 6.83 (d, J = 10.1 Hz, 1H), 3.73 (dt, J = 12.9, 2.4 Hz, 2H), 2.71 (td, J = 12.6, 2.6 Hz, 2H), 1.72-1.65 (m, 2H), 1.59-1.47 (m, 1H), 1.25 (dd, J = 12.3, 4.1 Hz, 1H), 1.19 (dd, J = 12.1, 4.3 Hz, 1H), 0.95 (d, J = 6.4 Hz, 3H).

Reference example 3

2-(3-Chloro-6-oxopyridazin-1(6H)-yl)-N-(4-cyanophenyl)acetamide

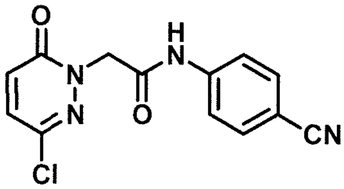

To a solution of the compound of Reference example 1 (16.7 g) in dimethylformamide (240 mL) were added potassium carbonate (23.7 g) and 6-chloropyridazin-3(2H)-one (14.8 g). After stirring at room temperature for 6 hours, water (360 mL) was added to the mixture, resulting in precipitation of a solid, and the precipitated solid was filtered. After washing with water, the solid was dried under reduced pressure to obtain the titled compound (18.4 g).

$^1$H-NMR (400 MHz, DMSO-$d_6$) δ: 10.78 (s, 1H), 7.79 (dt, J = 8.8, 2.1 Hz, 2H), 7.73 (dt, J = 9.0, 2.1 Hz, 2H), 7.64 (d, J = 9.6 Hz, 1H), 7.11 (d, J = 10.1 Hz, 1H), 4.90 (s, 2H).

Reference example 4

2-(4,4-Difluorocyclohex-1-en-1-yl)-4,4,5,5-tetramethyl-1,3,2-dioxaborolane

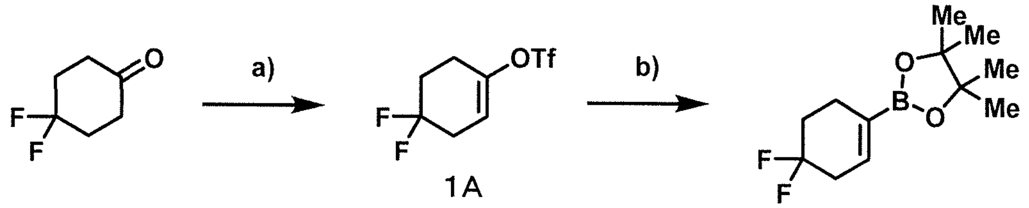


1A

a) To a solution of 4,4-difluorocyclohexanone (25.0 g) in 1,2-dichloroethane (373 mL) were added 2-chloropyridine (26.5 g) and trifluoromethanesulfonic anhydride (63.1 g), and the mixture was stirred at 50°C for 6 hours. After cooling to 0°C, hexane (750 mL) was added thereto, resulting in precipitation of a solid, and the precipitated solid was filtered out. The filtrate was concentrated under reduced pressure to obtain Compound 1A (46.1 g).

$^1$H-NMR (400 MHz, $CDCl_3$) δ: 5.67-5.63 (m, 1H), 2.69 (td, J = 13.5, 2.9 Hz, 2H), 2.60-2.55 m, 2H), 2.24-2.14 (m, 2H).

b) To a solution of Compound 1A (46.1 g) and bis(pinacolato)diboron (52.8 g) in 1,4-dioxane (577 mL) were added potassium acetate (42.5 g) and 1,1'-bis(diphenylphosphino)ferrocene palladium dichloride (6.34 g), and the mixture was heated under reflux for 2 hours. After cooling to room temperature, the mixture was filtered through Celite, and the filtrate was concentrated under reduced pressure. After the addition of ethyl acetate (1.5 L), the organic layer was washed with water (300 mL) and brine (200 mL). The organic layer was dried over anhydrous sodium sulfate, filtered, and concentrated under reduced pressure. The residue was purified by silica gel chromatography (solvent; hexane:ethyl acetate = 100:0, then 90:10) to obtain the titled compound (39.8 g).

$^1$H-NMR (400 MHz, $CDCl_3$) δ: 6.37-6.35 (m, 1H), 2.60-2.50 (m, 2H), 2.41-2.36 (m, 2H), 2.00-1.89 (m, 2H), 1.24 (s, 12H).

Reference example 5

6-(4,4-Dimethylcyclohexyl)pyridazin-3(2H)-one

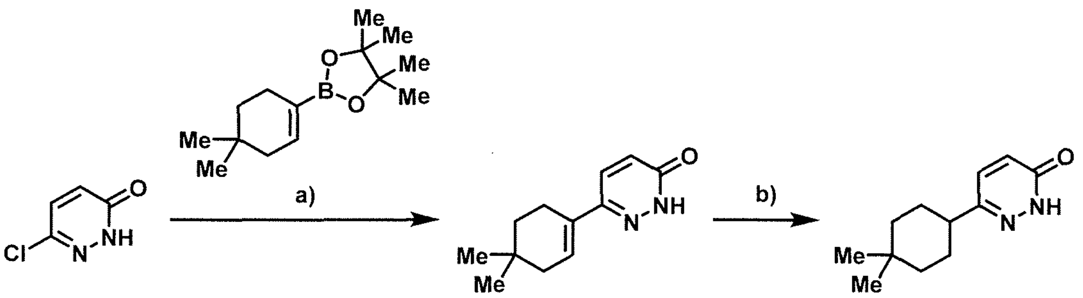


2A

a) 6-Chloropyridazin-3(2H)-one (497 mg), 2-(4,4-dimethylcyclohex-1-en-1-yl)-4,4,5,5-tetramethyl-1,3,2-dioxaborolane (900 mg), and 2 mol/L aqueous sodium carbonate (4.76 mL) were suspended in 1,2-dimethoxyethane (17 mL). To the mixture was added 1,1'-bis(diphenylphosphino)ferrocene palladium dichloride (279 mg). The mixture was stirred under nitrogen atmosphere at 80°C for 6 hours. After cooling to room temperature, water was added thereto,

and the mixture was extracted with ethyl acetate. The organic layer was dried over anhydrous sodium sulfate, filtered, and concentrated under reduced pressure. The residue was purified by silica gel column chromatography (solvent; chloroform:methanol = 99:1, then 93:7) to obtain Compound 2A (88 mg).

$^1$H-NMR (400 MHz, DMSO-$d_6$) δ: 7.80 (d, J = 10.1 Hz, 1H), 6.80 (d, J = 9.6 Hz, 1H), 6.43-6.40 (m, 1H), 2.35-2.30 (m, 2H), 1.98 (dt, J = 4.1, 1.8 Hz, 2H), 1.42 (t, J = 6.4 Hz, 2H), 0.91 (s, 6H).

b) A suspension of Compound 2A (88 mg) and 10 % palladium/carbon (20 mg) in methanol (20 mL) was stirred under hydrogen atmosphere at room temperature for 10 hours. Pd/C was filterd through Celite, and the filtrate was washed with methanol. The filtrate was concentrated to obtain the titled compound (89 mg).

$^1$H-NMR (400 MHz, $CDCl_3$) δ: 10.66 (s, 1H), 7.19 (d, J = 10.1 Hz, 1H), 6.89 (d, J = 9.6 Hz, 1H), 2.42 (tt, J = 11.8, 4.0 Hz, 1H), 1.71-1.67 (m, 2H), 1.63-1.55 (m, 2H), 1.52-1.45 (m, 2H), 1.27 (td, J = 12.3, 4.0 Hz, 2H), 0.93 (s, 3H), 0.92 (s, 3H) .

Reference example 6

[3-(4-Methylpiperidin-1-yl)-6-oxopyridazin-1(6H)-yl]acetic acid

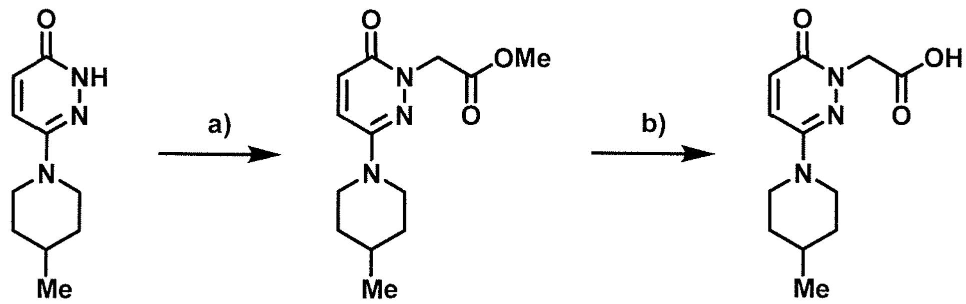


3A

a) The compound of Reference example 2 (2.5 g), methyl bromoacetate (2.8 g), and potassium carbonate (3.6 g) in dimethylformamide (26 mL) were stirred at room temperature for 2.5 hours. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 30:70) to obtain Compound 3A (3.2 g).

$^1$H-NMR (400 MHz, $CDCl_3$) δ: 7.16 (d, J = 10.1 Hz, 1H), 6.84 (d, J = 10.1 Hz, 1H), 4.75 (s, 2H), 3.77-3.74 (m, 2H), 3.76 (s, 3H), 2.71 (td, J = 12.5, 2.4 Hz, 2H), 1.69 (d, J = 12.5 Hz, 2H), 1.59-1.48 (m, 1H), 1.28-1.18 (m, 2H), 0.96 (d, J = 6.4 Hz, 3H).

b) Compound 3A (3.2 g) was dissolved in a mixed solvent of methanol (20 mL) and THF (20 mL), and 2 mol/L aqueous sodium hydroxide (30 mL) was added thereto with ice cooling. Then, the mixture was stirred at room temperature for 30 minutes. The organic solvent of the reaction solution was removed under reduced pressure, and to the resulting aqueous layer was added 1 mol/L hydrochloric acid to adjust pH to 3, resulting in precipitation of a solid. The precipitated solid was filtered, washed with water, and dried under reduced pressure to obtain the titled compound (3.0 g).

$^1$H-NMR (400 MHz, $CD_3OD$) δ: 7.50 (d, J = 10.1 Hz, 1H), 6.86 (d, J = 10.1 Hz, 1H), 4.72 (s, 2H), 3.92-3.88 (br, 2H), 2.76 (td, J = 12.7, 2.6 Hz, 2H), 1.73-1.68 (br, 2H), 1.64-1.51 (m, 1H), 1.23 (ddd, J = 24.4, 12.7, 3.9 Hz, 2H), 0.97 (d, J = 6.4 Hz, 3H).

Reference example 7

4-(4-Methylphenyl)-5,6,7,8-tetrahydrophthalazin-1(2H)-one

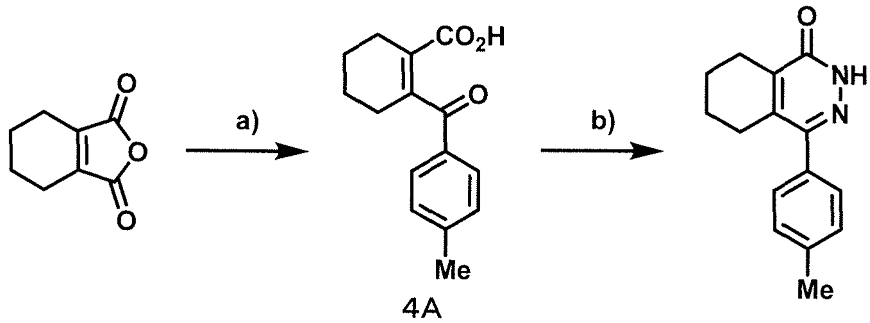


4A

a) To a solution of 4,5,6,7-tetrahydroisobenzofuran-1,3-dione (5.0 g) in tetrahydrofuran (329 mL) was added dropwise a solution of p-tolylmagnesium bromide in tetrahydrofuran (32.9 mL), and the mixture was stirred at room temperature for 23 hours. To the reaction mixture was added 1 mol/L hydrochloric acid, resulting in precipitation of a solid, and the precipitated solid was filtered and washed with water. The resulting solid was purified by silica gel column chromatography (solvent; chloroform:methanol = 9:1) to obtain Compound 4A (10.7 g).

$^1$H-NMR (400 MHz, $CDCl_3$) δ: 7.40-7.31 (m, 2H), 7.15 (d, J = 8.5 Hz, 2H), 2.42-2.20 (m, 6H), 1.98-1.43 (m, 5H).

b) To a solution of Compound 4A (7.61 g) in ethanol (156 mL) was added hydrazine monohydrate (3.03 mL), and the mixture was heated under reflux for 5 hours. After cooling to room temperature, saturated aqueous sodium bicarbonate was added thereto, and the ethanol was removed by concentration under reduced pressure, resulting in precipitation of a solid. The precipitated solid was filtered, washed with water, and dried under reduced pressure to obtain the titled compound (6.22 g).

$^1$H-NMR (400 MHz, $CDCl_3$) δ: 10.42 (s, 1H), 7.26-7.25 (m, 2H), 7.23-7.21 (m, 2H), 2.64-2.62 (m, 2H), 2.40-2.37 (m, 5H), 1.79-1.77 (m, 2H), 1.68-1.65 (m, 2H).

Reference example 8

6-(2-Azaspiro[4.4]nonan-2-yl)-4-methylpyridazin-3(2H)-one

Reference example 9

6-(2-Azaspiro[4.4]nonan-2-yl)-5-methylpyridazin-3(2H)-one

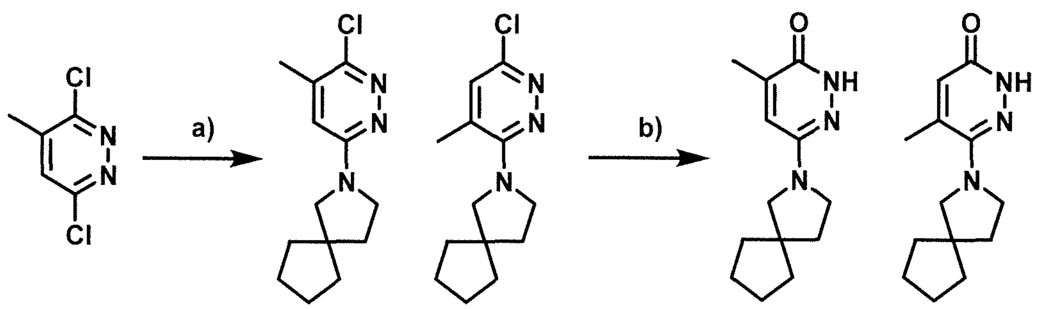


5A 5B

a) To a solution of 3,6-dichloro-4-methylpyridazine (0.40 g) in dimethylformamide (4 mL) was added 2-azaspiro[4.4]nonane (0.31 g) and triethylamine (1.03 mL). The reaction mixture was stirred at 80°C for 6 hours. After the addition of saturated aqueous sodium bicarbonate (20 mL) and water (20 mL), the mixture was extracted twice with ethyl acetate. The combined organic layer was dried over sodium sulfate and concentrated. The residue was purified by silica gel column chromatography (solvent; hexane:ethyl acetate = 90:10, and then 30:70) to obtain Compound 5A (250 mg) and Compound 5B (260 mg) .

b) Compound 5A was dissolved in acetic acid (3 mL), and the reaction mixture was subjected to microwave irradiation and stirred at 200°C for 2 hours. The reaction mixture was cooled to room temperature, and toluene was added thereto. The mixture was concentrated under reduced pressure and purified by silica gel column chromatography

(solvent; chloroform:methanol = 100:0, then 98:2) to obtain 6-(2-azaspiro[4.4]nonan-2-yl)-4-methylpyridazin-3(2H)-one (220 mg) .

LC-MS: $[M+H]^+$ / Rt (min) 234.2 / 0.832 (Method A)

Similarly, 6-(2-azaspiro[4.4]nonan-2-yl)-5-methylpyridazin-3(2H)-one (195 mg) was obtained from Compound 5B.

LC-MS: $[M+H]^+$ / Rt (min) 234.2 / 0.839 (Method A)

Reference example 10

6-(6-Azaspiro[3.4]octan-6-yl)-4-methoxypyridazin-3(2H)-one

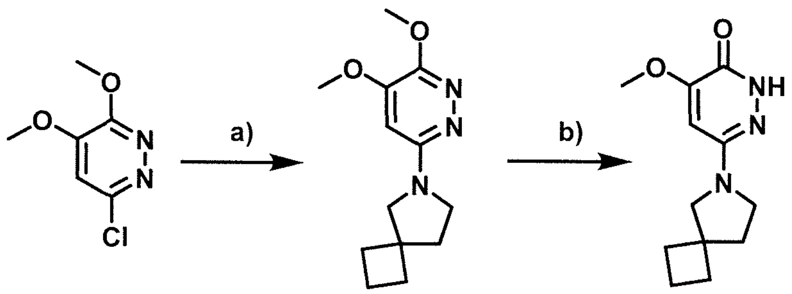


6A

a) To a solution of 6-chloro-3,4-dimethoxypyridazine (0.25 g) in toluene (2.5 mL) were added 6-azaspiro[3.4] octane (0.23 g), potassium tert-butoxide (0.14 g), and 2,2'-bis(biphenylphosphino)-1,1'-binaphthalene (6.4 mg). The reaction mixture was subjected to microwave irradiation, and stirred at 80°C for one hour. After the reaction mixture was cooled to room temperature, water (20 mL) was added thereto, and the mixture was extracted twice with ethyl acetate. The combined organic layer was dried over sodium sulfate, and then concentrated. The residue was purified by silica gel column chromatography (solvent; hexane:ethyl acetate = 90:10, then 15:85) to obtain Compound 6A (44 mg). LC-MS: $[M+H]^+$ / Rt (min) 250.2 / 0.506 (Method A)

b) Compound 6A (80 mg) was dissolved in dioxane (500 $\mu$L) and concentrated hydrochloric acid (2 mL). The reaction mixture was subjected to microwave irradiation, and stirred at 100°C for 2 hours. The reaction mixture was cooled to room temperature, and then concentrated under reduced pressure. The residue was poured into water, resulting in precipitation of a solid, and the precipitated solid was filtered to obtain the titled compound (53 mg).

LC-MS: $[M+H]^+$ / Rt (min) 236.2 / 0.527 (Method A)

Reference example 11

1,1-Difluoro-4,4-dimethyl-6-azaspiro[2.5]octane hydrochloride

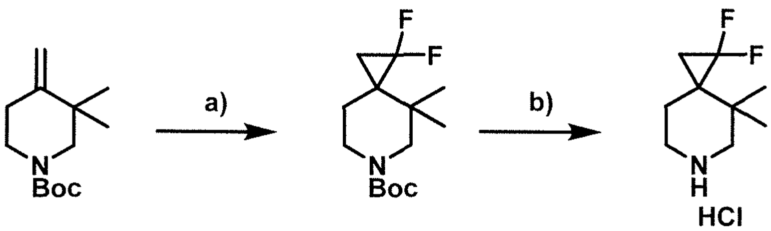


7A

a) To a solution of tert-butyl 3,3-dimethyl-4-methylidenepiperidine-1-carboxylate (4.6 g) in tetrahydrofuran (29.2 mL) were added (trifluoromethyl)trimethylsilane (10.54 ml) and sodium iodide (1.53 g), and the mixture was heated under

reflux for 33 hours. To the reaction mixture was added heptane (10 ml), and the mixture was concentrated under reduced pressure. The residue was dissolved in ethyl acetate. The organic layer was washed with water, saturated aqueous sodium thiosulfate, and brine, dried over anhydrous magnesium sulfate, and concentrated under reduced pressure. The residue was purified by silica gel column chromatography (solvent; hexane:ethyl acetate = 95:5, and then 75:25) to obtain Compound 7A (4.2 g).

$^1$H-NMR (400 MHz, $CDCl_3$) δ: 3.87 (br s, 1H), 3.43 (br s, 1H), 3.06-2.99 (m, 1H), 2.87-2.83 (m, 1H), 1.97-1.90 (m, 1H), 1.46 (s, 9H), 1.44-1.40 (m, 1H), 1.31-1.23 (m, 1H), 1.06 (s, 3H), 0.92-0.86 (m, 4H).

b) Compound 7A (4.2 g) was dissolved in cyclopentyl methyl ether (10 ml). To the mixture was added a solution of hydrogen chloride in cyclopentyl methyl ether (18.5 mL), and the mixture was stirred for 5 hours, resulting in precipitation of a solid. The precipitated solid was filtered, and dried to obtain the titled compound (3.71 g). $^1$H-NMR (400 MHz, DMSO-$d_6$) δ: 9.59 (s, 1H), 8.96 (s, 1H), 3.16-3.12 (m, 1H), 2.94-2.91 (m, 1H), 2.88-2.70 (m, 2H), 2.09-2.02 (m, 1H), 1.70-1.64 (m, 1H), 1.46-1.40 (m, 1H), 1.32-1.26 (m, 1H), 1.17 (s, 3H), 0.89 (s, 3H).

Reference example 12

7-Chloro-2-methyl-1,3-benzoxazole-5-amine

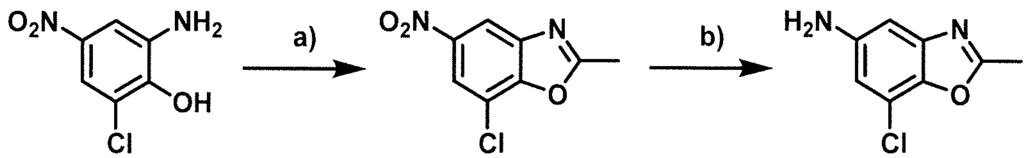


8A

a) To a solution of 2-amino-6-chloro-4-nitrophenol (0.5 g) in DMF (15 mL) were added triethyl orthoacetate (1.72 g) and p-toluenesulfonic acid (0.23 g), and the mixture was stirred at 70°C for 4 hours. To the reaction mixture was added saturated aqueous sodium bicarbonate, and the mixture was extracted with ethyl acetate. The organic layer was washed with brine, dried over anhydrous sodium sulfate, and concentrated under reduced pressure. The residue was purified by silica gel column chromatography (solvents; hexane:ethyl acetate = 80:20) to obtain Compound 8A (0.37 g).

$^1$H-NMR (400 MHz, $CDCl_3$) δ: 8.46 (d, J = 1.8 Hz, 1H), 8.30 (d, J = 2.4 Hz, 1H), 2.75 (s, 3H).

b) Compound 8A (62.5 mg) was dissolved in a mixed solvent of methanol (4 ml) and water (1 ml), and reduced iron (164 mg) and ammonium chloride (157 mg) were added thereto. The mixture was stirred at 70°C for 2 hours. Insoluble substances were filtered out through Celite, and the organic layer was concentrated. The residue was dissolved again in ethyl acetate. The mixture was washed with water and brine, and dried over anhydrous sodium sulfate, and concentrated under reduced pressure to obtain the titled compound (43.7 mg) .

$^1$H-NMR (400 MHz, $CDCl_3$) δ: 6.81 (d, J = 2.1 Hz, 1H), 6.66 (d, J = 1.8 Hz, 1H), 3.69 (br s, 2H), 2.61 (s, 3H).

Reference example 13

[1,2,4]Triazolo[1,5-a]pyridine-7-amine dihydrochloride

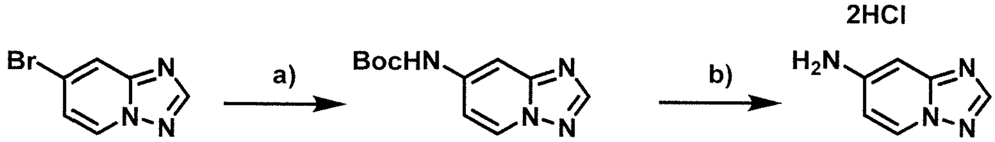


9A

a) A solution of 7-bromo[1,2,4]triazolo[1,5-a]pyridine (975 mg), tert-butyl carbamate (865 mg), sodium t-butoxide (710 mg), [(2-di-tert-butylphosphino-2',4',6'-triisopropylbiphenyl)-2-(2'-amino-1,1'-biphenyl)]palladium(II) methanesulfonate (196 mg) in toluene (33 mL) was stirred at 100°C for 2 hours. To the reaction mixture was added water, and the mixture was extracted with a mixed solvent of chloroform and ethanol (3:1). The organic layer was washed with brine, dried over anhydrous sodium sulfate, and concentrated under reduced pressure. The residue was purified by amino silica gel column chromatography (solvent; hexane:ethyl acetate = 75:25, and then ethyl acetate) to obtain the titled compound 9A (780 mg) .

$^1$H-NMR (400 MHz, $CDCl_3$) δ: 8.43 (d, J = 7.3 Hz, 1H), 8.23 (s, 1H), 7.70 (d, J = 2.4 Hz, 1H), 7.18 (dd, J = 7.3, 2.4 Hz, 1H), 6.79 (s, 1H), 1.52 (s, 9H).

b) Compound 9A (780 mg) was suspended in ethyl acetate (3 ml), and a solution of hydrogen chloride in dioxane (16 ml) was added thereto. The mixture was stirred at 40°C for 3 hours. The reaction mixture was concentrated under reduced pressure, and the following steps were repeated twice: toluene was added thereto, and the mixture was concentrated under reduced pressure. To the residue was added ethyl acetate, and the mixture was stirred, and then filtered to obtain the titled compound (499 mg).

$^1$H-NMR (400 MHz, $CD_3OD$) δ: 8.71 (s, 1H), 8.54 (d, J = 7.3 Hz, 1H), 6.90 (dd, J = 7.3, 2.1 Hz, 1H), 6.71 (d, J = 2.1 Hz, 1H) .

Reference example 14

4-(Morpholin-4-yl)-2,3-dihydro-1H-indole dihydrochloride

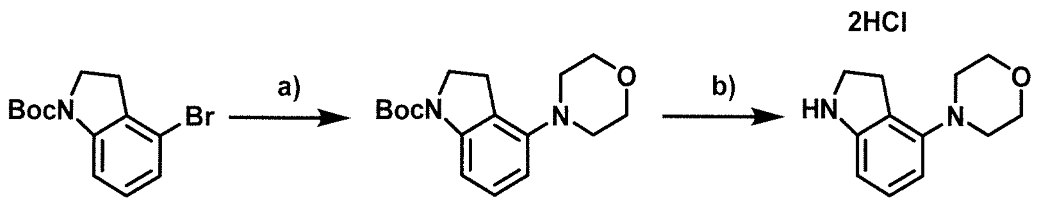


10A

a) A solution of tert-butyl 4-bromo-2,3-dihydro-1H-indole-1-carboxylate (1.55 g), morpholine (1.81 g), tris(dibenzylideneacetone)dipalladium(0) (0.48 g), (R)-(+)-2,2'-bis(diphenylphosphino)-1,1-binaphthyl (0.324 g), and sodium t-butoxide (0.999 g) in toluene (17.3 mL) was stirred at 100°C for 2 hours. Insoluble substances were filtered out through Celite, and the organic layer was concentrated. To the residue was added saturated aqueous ammonium chloride, and the mixture was extracted with ethyl acetate. The organic layer was washed with brine, dried over anhydrous sodium sulfate, and concentrated under reduced pressure. The residue was purified by silica gel column chromatography (solvent; hexane:ethyl acetate = 80:20) to obtain Compound 10A (1.07 g).

LC-MS: $[M+H]^+$ / Rt (min) 305.2 / 1.984 (Method B)

b) Compound 10A (1.07 g) was dissolved in ethyl acetate (15 ml), and a solution of hydrogen chloride in ethyl acetate (15 ml) was added thereto. The mixture was stirred at room temperature for 20 hours, and concentrated under reduced pressure. To the residue was added ethyl acetate, and the mixture was filtered to obtain the titled compound (0.92 g). $^1$H-NMR (400 MHz, $CD_3OD$) δ: 7.48 (t, J = 8.2 Hz, 1H), 7.27 (t, J = 7.9 Hz, 2H), 3.94-3.92 (m, 4H), 3.87 (t, J = 7.6 Hz, 2H), 3.41 (t, J = 7.3 Hz, 2H), 3.26-3.24 (m, 4H).

Reference example 15

4-(Pyridazin-4-yl)-2,3-dihydro-1H-indole hydrochloride

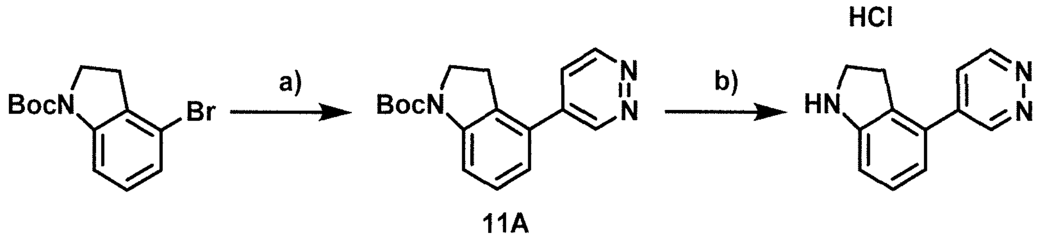


a) A solution of tert-butyl 4-bromo-2,3-dihydro-1H-indole-1-carboxylate (2.62 g), 4-(4,4,5,5-tetramethyl-1,3,2-dioxaborolan-2-yl)pyridazine (2.17 g), bis(di-tert-butyl(4-dimethylaminophenyl)phosphine)dichloropalladium(II) (0.62 g), 2 mol/L aqueous potassium acetate (13 ml) in acetonitrile (35 mL) was stirred at 90°C for 4 hours. To the reaction mixture was added water, and the mixture was extracted with ethyl acetate. The organic layer was washed with brine, dried over anhydrous sodium sulfate, and concentrated under reduced pressure. The residue was purified by silica gel column chromatography (solvent; ethyl acetate, and then ethyl acetate:methanol = 95:5) to obtain Compound 11A (2.30 g).

LC-MS: $[M+H]^+$ / Rt (min) 298.2 / 0.891 (Method A)

b) Compound 11A (2.30 g) was dissolved in chloroform (35 ml). After the addition of a solution of hydrogen chloride in ethyl acetate (4 mol/L, 35 ml), the mixture was stirred at room temperature for 3 hours. The reaction mixture was concentrated under reduced pressure, and the following steps were repeated twice: toluene was added to the mixture, and the mixture was concentrated under reduced pressure to obtain the titled compound (1.8 g).

LC-MS: $[M+H]^+$ / Rt (min) 198.2 / 0.333 (Method A)

Reference example 16

[3-(4-Methylcyclohex-1-en-1-yl)-6-oxopyridazin-1(6H)-yl]acetic acid

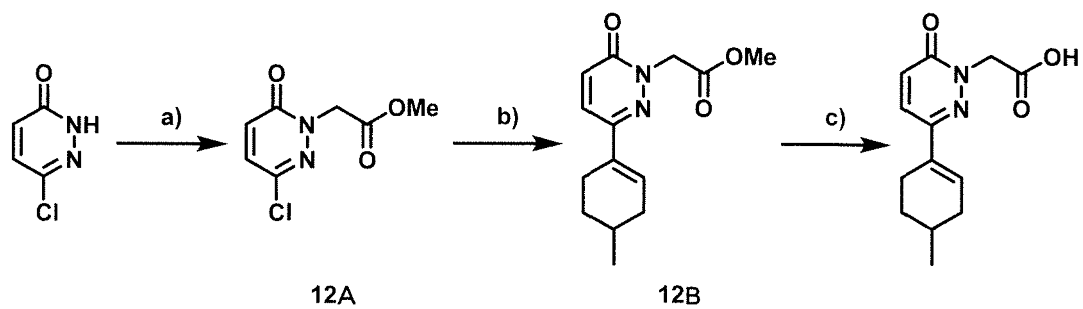


a) According to the method of a) in Reference example 6, Compound 12A (49.4 g) was obtained using 6-chloropyridazin-3(2H)-one (44.0 g).

$^1$H-NMR (400 MHz, DMSO-$d_6$) δ: 7.64 (d, J = 9.8 Hz, 1H), 7.13 (d, J = 9.8 Hz, 1H), 4.85 (s, 2H), 3.69 (s, 3H).

b) A solution of Compound 12A (2.15 g), 4,4,5,5-tetramethyl-2-(4-methylcyclohex-1-en-1-yl)-1,3,2-dioxaborolane (3.06 g) and 2 mol/L aqueous potassium acetate (15.92 mL) was suspended in acetonitrile (140 mL), and bis(di-tert-butyl(4-dimethylaminophenyl)phosphine)dichloropalladium(II) (301 mg) was added thereto. The mixture was stirred under a nitrogen atmosphere at 90°C for 5 hours. After cooling to room temperature, water was added thereto, and the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 90:10, and then 70:30) to obtain Compound 12B (2.40 g).

LC-MS: $[M+H]^+$ / Rt (min) 263.2 / 0.935 (Method A) c) According to the method of b) in Reference example 6, the titled compound (1.5 g) was obtained using Compound 12B (2.40 g).

LC-MS: $[M+H]^+$ / Rt (min) 249.2 / 0.835 (Method A)

Reference example 17

2-(Trifluoromethyl)imidazo[1,2-a]pyridine-7-amine

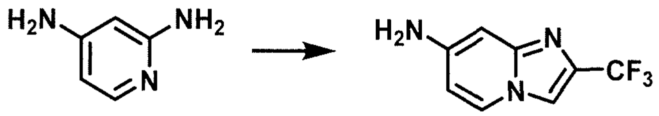

Pyridine-2,4-diamine (300 mg) and sodium bicarbonate (462 mg) was suspended in ethanol (9.1 mL), and 3-chloro-1,1,1-trifluoropropan-2-one was added thereto. The mixture was heated under reflux for 4 hours. After cooling to room temperature, water was added to the reaction mixture, and the mixture was extracted with a mixed solvent of chloroform and ethanol (3:1). The organic layer was dried over anhydrous sodium sulfate and concentrated under reduced pressure. The residue was purified by amino silica gel column chromatography (solvent; hexane:ethyl acetate = 50:50, and then ethyl acetate) to obtain the titled compound (250 mg) .

LC-MS: $[M+H]^+$ / Rt (min) 202.1 / 0.344 (Method A)

Reference example 18

7-Fluoro-1,3-benzoxazole-5-amine

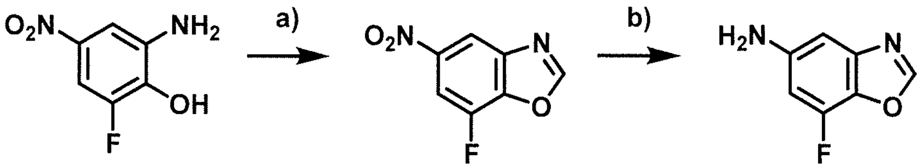


13A

a) 2-Amino-6-fluoro-4-nitrophenol (507 mg) and triethoxymethane (0.98 mL) were dissolved in chloroform (14.7 mL) and acetic acid (0.68 mL). The mixture was heated under reflux for 5 hours. After cooling to room temperature, the mixture was concentrated under reduced pressure. The residue was purified by silica gel column chromatography (solvent; hexane:ethyl acetate = 83:17, and then 75:25) to obtain Compound 13A (330 mg).
LC-MS: $[M+H]^+$ / Rt (min) 183.0 / 0.749 (Method A)
b) Compound 13A (330 mg) was dissolved in methanol (9.0 mL) and palladium/carbon (96 mg) was added thereto. The mixture was stirred at room temperature for 3 hours. Insoluble substances were filtered through Celite, and the organic layer was concentrated to obtain the titled compound (268 mg) .

LC-MS: $[M+H]^+$ / Rt (min) 153.0 / 0.445 (Method A)

Reference example 19

2-(Difluoromethyl)-1,3-benzoxazole-5-amine

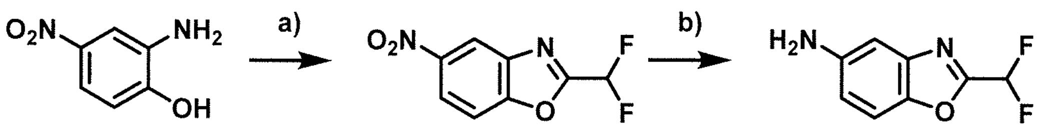


14A

a) 2-Amino-4-nitrophenol (300 mg), triethylamine (1.36 mL), triphenylphosphine (1.28 g), and difluoroacetic acid (0.12 mL) were dissolved in carbon tetrachloride (6.5 mL), and the mixture was heated under reflux for 3 hours. After cooling to room temperature, to the reaction mixture was added water, and the mixture was extracted with a mixed solvent of chloroform and methanol (10:1). The organic layer was dried over anhydrous sodium sulfate, and concentrated under reduced pressure. The residue was purified by silica gel column chromatography (solvate; hexane:ethyl acetate = 75:25, and then ethyl acetate) to obtain Compound 14A (331 mg). LC-MS: $[M+H]^+$ / Rt (min) 215.1 / 0.850 (Method A)

b) According to the method of b) in Reference example 18, the titled compound (268 mg) was obtained using Compound 14A (330 mg).

LC-MS: $[M+H]^+$ / Rt (min) 185.1 / 0.505 (Method A)

Reference example 20

7-Fluoro-2-methoxy-1,3-benzoxazole-5-amine

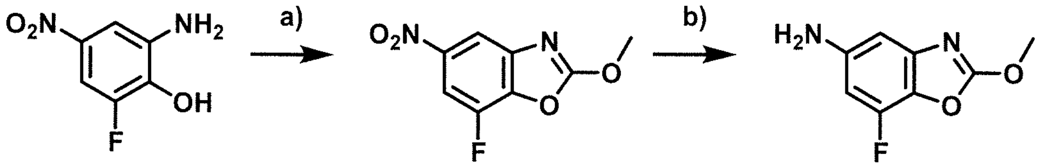


15A

a) According to the method of a) in Reference example 18, Compound 15A (318 mg) was obtained by using 2-amino-6-fluoro-4-nitrophenol (303 mg) and tetramethyl orthocarbonate (0.47 mL) .

LC-MS: $[M+H]^+$ / Rt (min) 213.1 / 0.843 (Method A) b) According to the method of b) in Reference example 18, the titled compound (248 mg) was obtained by using Compound 120A (318 mg) .

LC-MS: $[M+H]^+$ / Rt (min) 183.0 / 0.517 (Method A)

Reference example 21

2-Methyl[1,3]thiazolo[5,4-b]pyridine-6-amine

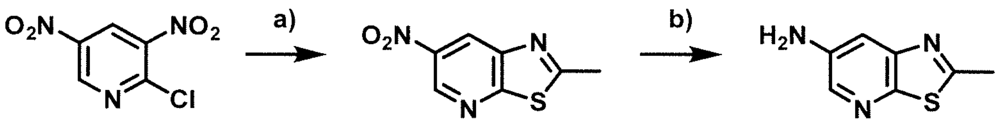


16A

a) 2-Chloro-3,5-dinitropyridine (300 mg) was dissolved in sulfolane (9.8 mL), and thioacetamide (1.48 g) was added thereto. The mixture was heated under reflux at 110°C for 3 hours. After cooling to room temperature, water was added to the reaction mixture, and the mixture was extracted with ethyl acetate. The organic layer was dried over anhydrous sodium sulfate, and concentrated under reduced pressure. The residue was purified by silica gel column chromatography (solvent; hexane:ethyl acetate = 90:10, and then 75:25) to obtain Compound 16A (0.23 g). LC-MS: $[M+H]^+$ / Rt (min) 196.1 / 0.667 (Method A)

b) Compound 16A (177 mg) was dissolved in ethanol (3.8 mL), and water (1.3 mL), ammonium chloride (485 mg), and reduced iron (253 mg) were added thereto. The mixture was heated under reflux for 3 hours. Insoluble substances were filtered through Celite, and the organic layer was concentrated under reduced pressure. To the residue was added saturated aqueous sodium bicarbonate, and the mixture was extracted with chloroform. The organic layer was dried over anhydrous sodium sulfate, and concentrated under reduced pressure to obtain the titled compound (97 mg).

LC-MS: $[M+H]^+$ / Rt (min) 166.0 / 0.423 (Method A)

Reference example 22

2-(5-Amino-1,3-benzoxazol-2-yl)propan-2-ol

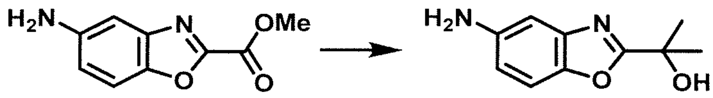

Methyl 5-amino-1,3-benzoxazole-2-carboxylate (200 mg) and cerium(III) chloride (1.03 g) were dissolved in tetrahydrofuran (6.9 mL). While stirring at 0°C, 3 mol/L methylmagnesium bromide (1.39 ml) was added thereto, and the mixture was stirred at 0°C for 2 hours. To the reaction mixture was added saturated aqueous ammonium chloride, and the mixture was extracted with chloroform. The organic layer was washed with saturated aqueous sodium bicarbonate and brine, dried over anhydrous sodium sulfate, and concentrated under reduced pressure. The residue was purified by silica gel column chromatography (solvent; ethyl acetate, and then ethyl acetate:methanol = 96:4) to obtain the titled compound (10.0 mg).
LC-MS: $[M+H]^+$ / Rt (min) 193.1 / 0.317 (Method A)

Reference example 23

1-(5-Methoxy-2-methylpyridin-3-yl)piperazine trihydrochloride

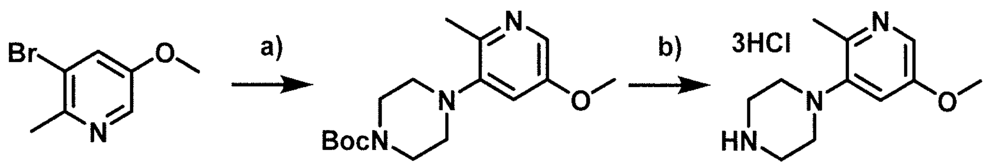


17A

a) 1-Boc-piperazine (277 mg), tris(dibenzylideneacetone)dipalladium(0) (91 mg), 4,5-bis(diphenylphosphino)-9,9-dimethylxanthene (172 mg), and sodium t-butoxide (190 mg) were suspended in toluene (10 ml). 3-Bromo-5-methoxy-2-methylpyridine (200 mg) was added thereto, and the mixture was stirred at 70°C for one hour. After cooling to room temperature, ethyl acetate was added to the reaction mixture. Insoluble substances were filtered through Celite, and the organic layer was concentrated under reduced pressure. The residue was purified by silica gel column chromatography (solvent; hexane:ethyl acetate = 70:30, and then 50:50) to obtain Compound 17A (296 g).
$^1H$-NMR (400 MHz, $CDCl_3$) $\delta$: 7.94 (d, J = 2.4 Hz, 1H), 6.86 (d, J = 2.4 Hz, 1H), 3.84 (s, 3H), 3.60-3.57 (m, 4H), 2.86-2.84 (m, 4H), 2.48 (s, 3H), 1.49 (s, 9H).
b) Compound 17A (296 mg) was dissolved in methanol (5 mL), and 2 mol/L solution of hydrogen chloride in methanol (9.6 mL) was added thereto while stirring at 0°C. The mixture was stirred at 50°C for 3 hours. After cooling to room temperature, the precipitated solid was filtered, and the resulting solid was dried under reduced pressure to obtain the titled compound (271.7 mg).

$^1H$-NMR (400 MHz, $CD_3OD$) $\delta$: 8.16 (d, J = 2.4 Hz, 1H), 7.83 (d, J = 2.4 Hz, 1H), 4.03 (s, 3H), 3.47-3.45 (m, 4H), 3.37-3.35 (m, 4H), 2.68 (s, 3H).

Reference example 24

(2R,6S)-2,6-Dimethyl-1-(pyridin-3-yl)piperazine trihydrochloride

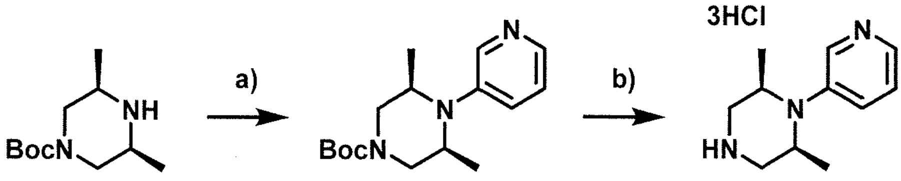


18A

a) 0.5 mol/L Potassium hexamethyldisilazide (2.57 ml), tert-butyl (3R,5S)-3,5-dimethylpiperazine-1-carboxylate (250 mg), and 3-bromopyridine (184 mg) were suspended in 1,4-dioxane (10 ml), and the mixture was stirred at 100°C for 8 hours. After cooling to room temperature, water was added thereto, and the mixture was extracted with ethyl acetate. The organic layer was washed with brine, dried over anhydrous sodium sulfate, filtered, and then concentrated under reduced pressure. The residue was purified by silica gel column chromatography (solvent; hexane:ethyl acetate = 80:20, and then 40:60) to obtain Compound 18A (114 mg).
LC-MS: $[M+H]^+$ / Rt (min) 292.0 / 0.689 (Method A)
b) According to the method of b) in Reference example 15, the titled compound (75 mg) was obtained by using Compound 18A (114 mg).

LC-MS: $[M+H]^+$ / Rt (min) 192.2 / 0.149 (Method A)

Reference example 25

4-[(Morpholin-4-yl)methyl]-2,3-dihydro-1H-indole

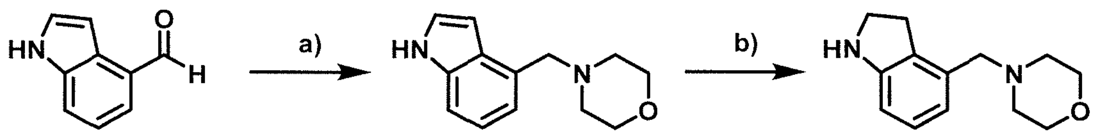


19A

a) 2,3-Dihydro-1H-indole-4-carbaldehyde (147 mg) was dissolved in dichloromethane (2 ml), and morpholine (88 mg) and sodium triacetoxyborohydride (322 mg) were added thereto. The mixture was stirred at room temperature for 4 hours. To the reaction mixture was added saturated aqueous sodium bicarbonate, and the mixture was extracted with ethyl acetate. The organic layer was washed with brine, dried over anhydrous sodium sulfate, filtered, and then concentrated under reduced pressure to obtain Compound 19A (209 mg).
LC-MS: $[M+H]^+$ / Rt (min) 217.2 / 0.292 (Method A)
b) Compound 19A (209 mg) were dissolved in acetic acid (3 ml), and sodium cyanoborohydride (182 mg) was added thereto, and the mixture was stirred at room temperature for 4 hours. The mixture was concentrated under reduced pressure, and the following steps were repeated twice: toluene was added thereto, and the mixture was concentrated under reduced pressure. To the residue was added saturated aqueous sodium bicarbonate, and the mixture was extracted with ethyl acetate. The organic layer was washed with brine, dried over anhydrous sodium sulfate, filtered, and then concentrated under reduced pressure to obtain the titled compound (256 mg). LC-MS: $[M+H]^+$ / Rt (min) 219.2 / 0.131 (Method A)

Reference example 26

4-[(1H-Imidazol-1-yl)methyl]-2,3-dihydro-1H-indole hydrochloride

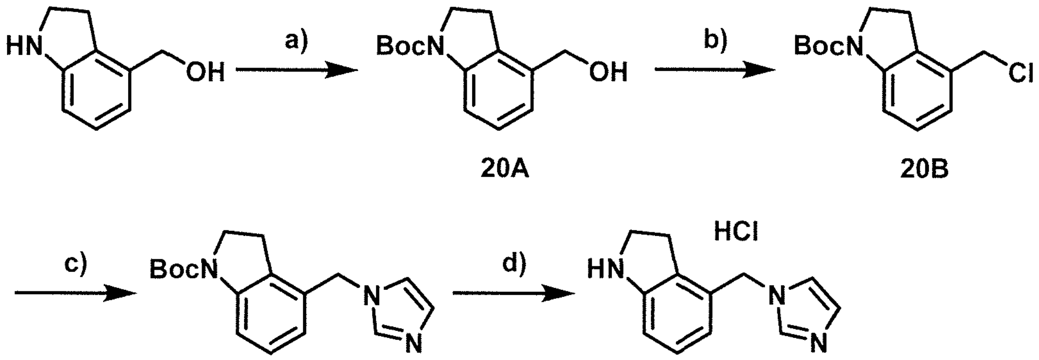


20A

20B

20C

a) (2,3-Dihydro-1H-indol-4-yl)methanol (0.92 g) was dissolved in tetrahydrofuran (2 ml), and di-tert-butyl dicarbonate (1.48 g) was added thereto. The mixture was stirred at room temperature overnight. The reaction mixture was concentrated under reduced pressure, resulting in precipitation of a solid, and the resulting residue was purified by silica gel column chromatography (solvent; hexane, and then hexane:ethyl acetate = 70:30) to obtain Compound 20A (1.54 g).
LC-MS: $[M+H-tBu]^+$ / Rt (min) 194.1 / 0.866 (Method A)
b) Compound 20A (1.54 g) was dissolved in dichloromethane (20 ml), and thionyl chloride (0.57 ml) was added thereto while stirring at 0°C. The mixture was warmed to room temperature, and stirred overnight. The reaction mixture was concentrated under reduced pressure, and the following steps were repeated twice: toluene was added thereto, and the mixture was concentrated under reduced pressure to obtain Compound 20B (1.65 g).
LC-MS: $[M+H-tBu]^+$ / Rt (min) 212.1 / 1.169 (Method A)
c) Compound 20B (200 mg) was dissolved in dimethylformamide (3 ml), and imidazole (2.5 g) was added thereto. The mixture was stirred at 80°C for 2 hours. Water was added thereto, and the mixture was extracted with ethyl acetate. The organic layer was washed with water and brine, dried over anhydrous sodium sulfate, filtered, and concentrated under reduced pressure. The residue was purified by silica gel column chromatography (solvent; ethyl acetate, and then ethyl acetate:methanol = 95:5) to obtain Compound 20C (80 mg) .
LC-MS: $[M+H]^+$ / Rt (min) 300.3 / 0.683 (Method A)
d) Compound 20C (80 mg) were dissolved in chloroform (3 ml), and a solution of hydrogen chloride in ethyl acetate (0.54 mL) were added thereto. The reaction mixture was stirred at room temperature for one hour, and concentrated under reduced pressure. The following steps were repeated twice: to the residue was added toluene, and the mixture was concentrated to obtain the titled compound (53 mg).

LC-MS: $[M+H]^+$ / Rt (min) 200.1 / 0.115 (Method A)

Reference example 27

Methyl (3-chloro-5-methyl-6-oxopyridazin-1(6H)-yl)acetate

Reference example 28

Methyl (3-chloro-4-methyl-6-oxopyridazin-1(6H)-yl)acetate

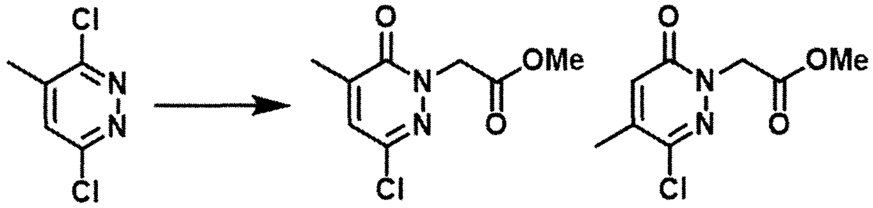

Reference example 27 Reference example 28

3,6-Dichloro-4-methylpyridazine (650 mg) was dissolved in acetic acid (6 mL), and the reaction mixture was subjected to microwave irradiation, and stirred at 200°C for 2 hours. After cooling to room temperature, the following steps were repeated three times: toluene was added thereto, and the mixture was concentrated under reduced pressure. The residue was dissolved in dimethylformamide (3 mL), and methyl bromoacetate (855 mg) and potassium carbonate (1.10 g) were added thereto. The mixture was stirred at room temperatuer overnight. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 90:10, and then 25:75) to obtain the compound of Reference example 27 (374 mg) and the compound of Reference example 28 (190 mg), respectively. Reference example 27 LC-MS: $[M+H]^+$ / Rt (min) 217.1 / 0.610

(Method A)

Reference example 28 LC-MS: $[M+H]^+$ / Rt (min) 217.1 / 0.598 (Method A)

Reference example 29

4,4,5,5-Tetramethyl-2-(4-methylcyclopent-1-en-1-yl)-1,3,2-dioxaborolane

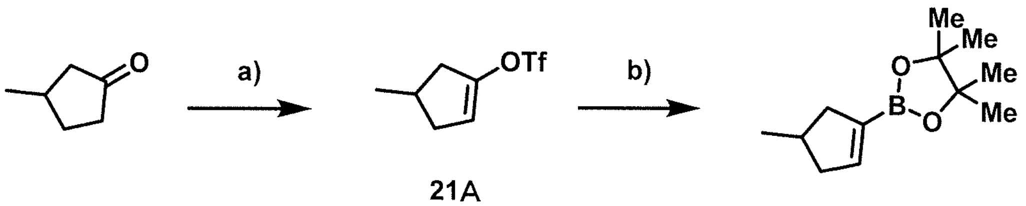


21A

a) A solution of 4,4-dimethyl-2-cyclopenten-1-one (750 mg) in tetrahydrofuran (40 mL) was stirred at -78°C, and 1 mol/L solution of lithium tri-sec-butylborohydride in tetrahydrofuran (7.8 ml) was added thereto. The mixture was stirred at -78°C for one hour. To the reaction mixture was added a solution of 2-[N,N-bis(trifluoromethylsulfonyl)amino]pyridine (2.80 g) in tetrahydrofuran (10 ml), and the mixture was stirred at room temperature for one hour. To the reaction mixture was added water, and the mixture was extracted with ethyl acetate. The organic layer was washed with 2 mol/L aqueous sodium hydroxide (30 mL) and brine, dried over anhydrous sodium sulfate, filtered, and concentrated under reduced pressure. The residue was purified by silica gel column chromatography (solvent; hexane:ethyl acetate = 99:1) to obtain Compound 21A (310 mg).
$^1$H-NMR (400 MHz, $CDCl_3$) δ: 5.57-5.55 (m, 1H), 2.78-2.70 (m, 1H), 2.63-2.49 (m, 2H), 2.22-2.15 (m, 1H), 2.02-1.95 (m, 1H), 1.11 (d, J = 6.8 Hz, 3H).
b) According to the method of b) in Reference example 4, the titled compound (150 mg) was obtained by using Compound 21A (300 mg).

$^1$H-NMR (400 MHz, $CDCl_3$) δ: 6.48-6.43 (1H, m), 2.67-2.52 (2H, m), 2.42-2.28 (1H, m), 2.08-1.95 (2H, m), 1.33-1.21 (12H, m), 1.04-0.99 (3H, m).

Reference example 30

2-(1,1-Difluorospiro[2.5]oct-5-en-6-yl)-4,4,5,5-tetramethyl-1,3,2-dioxaborolane

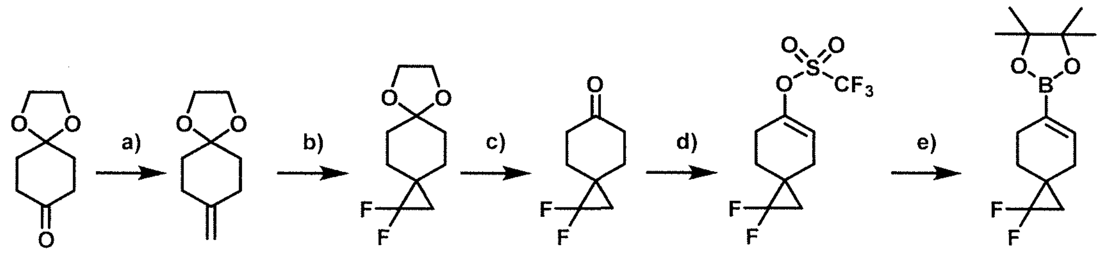


22A 22B 22C 22D

a) To a suspension of methyltriphenylphosphonium bromide (121.0 g) in toluene (570 mL) was added potassium tert-butoxide (37.9 g), and the mixture was stirred at room temperature for one hour. To the reaction mixture was added a solution of 1,4-dioxaspiro[4.5]decan-8-one (24.0 g) in toluene (1000 ml), and the mixture was stirred at room temperature for 2 hours. To the reaction mixture was added sandy magnesium chloride (64.4 g), and the mixture was stirred at 60°C for 2 hours. To the reaction mixture was added acetone (13.54 ml), and the mixture was stirred at 60°C for 2 hours, and then at room temperature overnight. The reaction mixture was filtered, and the solid was washed with heptane (400 ml). The filtrate was concentrated under reduced pressure. To the residue was added hexane, and the mixture was stirred for a while. Insoluble substances were filtered, and the filtrate was concentrated under reduced pressure. The residue was purified by silica gel column chromatography (solvate; hexane, and then hexane:ethyl acetate = 85:15) to obtain Compound 22A (20.8 g).

$^1$H-NMR (400 MHz, $CDCl_3$) δ: 4.65 (s, 2H), 3.96 (s, 4H), 2.27 (t, J = 6.5 Hz, 4H), 1.69 (t, J = 6.5 Hz, 4H).

b) According to the method of a) in Reference example 11, Compound 22B (24.0 g) was obtained by using Compound 22A (20.8 g).

$^1$H-NMR (400 MHz, $CDCl_3$) δ: 3.96 (s, 4H), 1.74-1.66 (m, 8H), 1.05 (t, J = 8.2 Hz, 2H).

c) Compound 22B (23.8 g) was dissolved in a mixed solvent of acetone (180 ml) and water (120 ml). p-Toluenesulfonic acid monohydrate (2.22 g) was added thereto, and the mixture was heated under reflux for 4 hours. After cooling to room temperature, saturated aqueous sodium bicarbonate was added thereto, and the acetone layer was removed under reduced pressure. The aqueous layer was extracted twice with ethyl acetate. The combined organic layer was dried over anhydrous magnesium sulfate, filtered, and concentrated under reduced pressure. The residue was purified by silica gel column chromatography (solvent; hexane:ethyl acetate = 90:10, and then hexane:ethyl acetate = 75:25) to obtain Compound 22C (15.2 g).

$^1$H-NMR (400 MHz, $CDCl_3$) δ: 2.48-2.37 (m, 4H), 1.98-1.89 (m, 4H), 1.25-1.21 (m, 2H).

d) A solution of Compound 22C (4.77 g) and 2,6-lutidine in dichloromethane (48 ml) was stirred at 0°C, and trifluoromethanesulfonic anhydride (10.57 ml) was added thereto. The mixture was warmed, and heated under reflux for 3 hours. After cooling to room temperature, saturated aqueous sodium bicarbonate was added thereto, and the dichloromethane layer was removed under reduced pressure. The aqueous layer was extracted twice with ethyl acetate. The combined organic layer was washed sequentially with 1 mol/l aqueous hydrochloric acid, water, and brine, dried over anhydrous magnesium sulfate, filtered, and concentrated under reduced pressure. The residue was purified by silica gel column chromatography (solvent; hexane:ethyl acetate = 95:5, and then hexane:ethyl acetate = 77:23) to obtain Compound 22D (6.61 g).

$^1$H-NMR (400 MHz, $CDCl_3$) δ: 5.78 (t, J = 3.7 Hz, 1H), 2.45-2.35 (m, 3H), 2.19-2.14 (m, 1H), 1.86-1.81 (m, 2H), 1.21-1.11 (m, 2H).

e) To a solution of Compound 22D (6.61 g), triphenylphosphine (593 mg), potassium phenoxide (2.99 g), 4,4,4',4',5,5,5',5'-octamethyl-2,2'-bi(1,3,2-dioxaborolane) (6.03 g) in toluene (113 ml) was added bis(triphenylphosphine)palladium(II) dichloride (794 mg), and the mixture was stirred at 50°C for 4 hours. To the reaction mixture were added potassium phenoxide (1.14 g) and tetrakis(triphenylphosphine)palladium(0) (784 mg), and the mixture was stirred at 50°C for 1.5 hours. After cooling to room temperature, water and ethyl acetate were added thereto, and the mixture was filtered through Celite. The organic layer was washed with 1 M aqueous sodium carbonate and brine, dried over anhydrous magnesium sulfate, filtered, and concentrated under reduced pressure. The residue was purified by silica gel column chromatography (solvent; hexane, and then hexane:ethyl acetate = 80:20), and purified again by silica gel column chromatography (solvent; hexane:toluene = 50:50, and then hexane:toluene:ethyl

acetate = 45:50:5) to obtain the titled compound (3.72 g). $^1$H-NMR (400 MHz, $CDCl_3$) δ: 6.53-6.50 (m, 1H), 2.36-2.14 (m, 3H), 2.05-2.00 (m, 1H), 1.67-1.55 (m, 2H), 1.25 (s, 12H), 1.09-0.97 (m, 2H).

Reference example 31

8-Fluoro-[1,2,4]triazolo[1,5-a]pyridine-7-amine

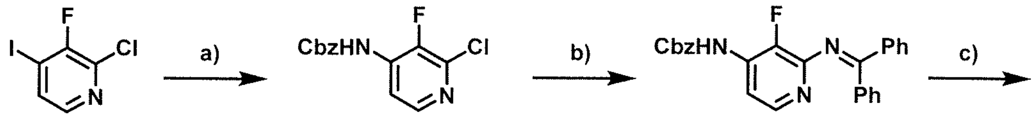


23A 23B

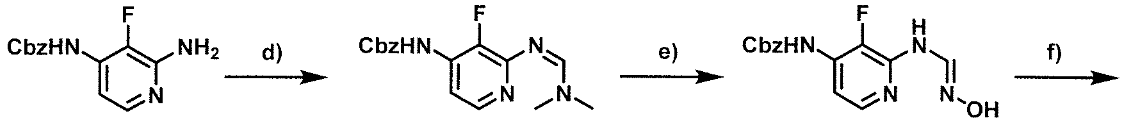


23C 23D 23E

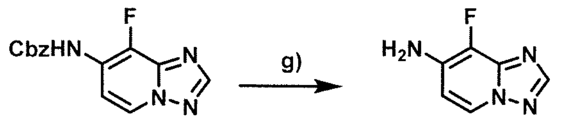


23F

a) A solution of 2-chloro-3-fluoro-4-iodopyridine (3.1 g), benzyl carbamate (2.28 g), 4,5-bis(diphenylphosphino)-9,9-dimethylxanthene (1.74 g), and tris(dibenzylideneacetone)dipalladium (1.32 g) in toluene (80 mL) was stirred at 100°C for 8 hours. After cooling to room temperature, water and ethyl acetate were added thereto, and the mixture was filtered through Celite. The aqueous layer was extracted with ethyl acetate. The organic layer was dried over anhydrous sodium sulfate, and concentrated under reduced pressure. The residue was purified by silica gel column chromatography (solvent; hexane:ethyl acetate = 87:13, and then hexane:ethyl acetate = 67:33) to obtain Compound 23A (1.8 g).
LC-MS: $[M+H]^+$ / Rt (min) 281.1 / 0.948 (Method A)
b) A solution of Compound 23A (1.25 g), benzophenone imine (1.12 mL), sodium t-butoxide (642 mg), and [(2-di-tert-butylphosphino-2',4',6'-triisopropylbiphenyl)-2-(2'-amino-1,1'-biphenyl)]palladium(II) methanesulfonate (354 mg) in toluene (18 ml) was stirred at 150°C for 5 hours under microwave irradiation. After cooling to room temperature, water and ethyl acetate were added thereto, and the mixture was filtered through Celite. The aqueous layer was extracted with ethyl acetate. The organic layer was dried over anhydrous sodium sulfate, and concentrated under reduced pressure. The residue was purified by amino silica gel column chromatography (solvent; hexane:ethyl acetate = 87:13, and then hexane:ethyl acetate = 67:33) to obtain Compound 23B (490 mg).
LC-MS: $[M+H]^+$ / Rt (min) 426.3 / 1.117 (Method A)
c) Compound 23B (780 mg) was dissolved in tetrahydrofuran (5.3 ml), and 1 mol/L aqueous hydrochloric acid (5.3 ml) was added thereto. The mixture was stirred at room temperature for 4 hours. To the reaction mixture was added saturated sodium bicarbonate, and the mixture was extracted with a mixed solvent of chloroform and ethanol (3:1). The organic layer was dried oved anhydrous sodium sulfate, and concentrated under reduced pressure. The residue was purified by amino silica gel column chromatography (solvent; hexane:ethyl acetate = 87:13, and then ethyl acetate) to obtain Compound 23C (179 mg).
LC-MS: $[M+H]^+$ / Rt (min) 262.1 / 0.542 (Method A)
d) Compound 23C (179 mg) was dissolved in 2-propanol (2.7 ml), and N,N-dimethylformamide dimethyl acetal (0.119 ml) was added thereto. The mixture was stirred at 100°C for 3 hours. To the reaction mixture was added toluene (2.7 mL), and the mixture was concentrated under reduced pressure to obtain Compound 23D (212 mg).
LC-MS: $[M+H]^+$ / Rt (min) 317.2 / 0.598 (Method A)
e) Compound 23D (178 mg) was dissolved in 2-propanol (2.7 ml), and 50 % aqueous hydroxyamine (0.05 ml) was

added thereto. The mixture was stirred at 60°C for 6 hours. To the reaction mixture was added water, and the mixture was extracted with chloroform. The organic layer was dried over anhydrous sodium sulfate, and concentrated under reduced pressure. The residue was purified by silica gel column chromatography (solvent; hexane:ethyl acetate = 67:33, and then hexane:ethyl acetate = 30:70) to obtain Compound 23E (146 mg).
LC-MS: $[M+H]^+$ / Rt (min) 305.2 / 0.782 (Method A)
f) Compound 23E (171 mg) was dissolved in tetrahydrofuran (5.6 ml), and trifluoroacetic anhydride (0.119 ml) was added thereto. The mixture was stirred at room temperature for 21 hours. To the reaction mixture was added saturated sodium bicarbonate, and the mixture was extracted with chloroform. The organic layer was dried over anhydrous sodium sulfate, and concentrated under reduced pressure. The residue was purified by silica gel column chromatography (solvent; hexane:ethyl acetate = 83:17, and then hexane:ethyl acetate = 50:50) to obtain Compound 23F (82 mg).
LC-MS: $[M+H]^+$ / Rt (min) 287.2 / 0.742 (Method A)
g) Compound 23F (90 mg) was dissolved in methanol (1.6 ml), and 10 % palladium/carbon (17 mg) was added thereto. The mixture was stirred under hydrogen atmosphere at room temperature for 4 hours. The palladium/carbon was filtered through Celite, and washed with methanol. The filtrate was concentrated to obtain the titled compound (47 mg).

LC-MS: $[M+H]^+$ / Rt (min) 153.0 / 0.294 (Method A)

Reference example 32

[6-(4,4-Dimethylpiperidin-1-yl)-1H-pyrazolo[3,4-b]pyrazin-1-yl]acetic acid hydrochloride

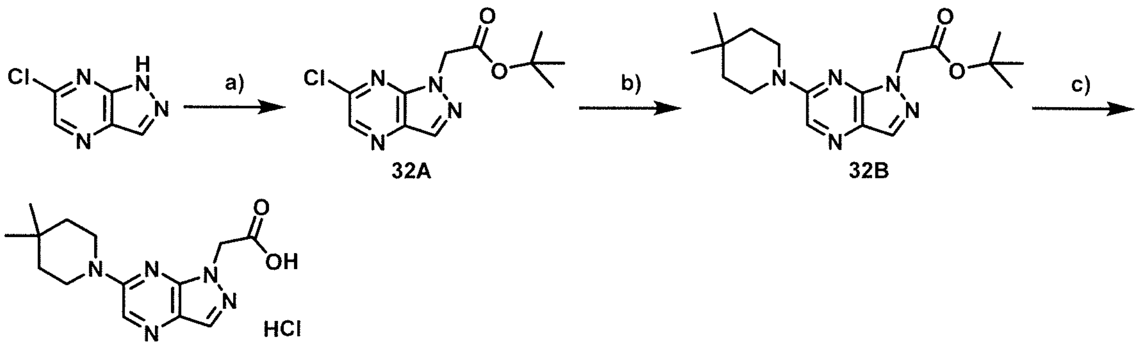

32A
32B

a) To a solution of 6-chloro-1H-pyrazolo{3,4-b}pyrazine (2.50 g) in dimethylformamide (65 mL) were added cesium carbonate (7.38 g) and tert-butyl bromoacetate (3.08 mL), and the mixture was stirred at room temperature for 4 hours. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 83: 17, then 0:100) to obtain Compound 32A (2.26 g).
LC-MS: $[M+H]^+$ / Rt (min) 268.9 / 0.945 (Method A)
b) To a solution of Compound 32A (200 mg) in N-methylpyrrolidone (2 mL) were added potassium carbonate (309 mg) and 4,4-dimethylpiperidine hydrochloride (134 mg), and the mixture was stirred at 50°C for 2 hours. Water was added thereto, and the mixture was extracted with ethyl acetate. The organic layer was washed with brine, dried over anhydrous sodium sulfate, filtered, and concentrated under reduced pressure. The residue was purified by silica gel chromatography (solvent; hexane:ethyl acetate = 90:10, and then 20:80) to obtain Compound 32B (246 mg).
LC-MS: $[M+H]^+$ / Rt (min) 346.0 / 1.146 (Method A)
c) To Compound 32B (246 mg) was added 4 mol/L hydrogen chloride in dioxane (5 mL), and the mixture was stirred at 55°C for 8 hours. The reaction mixture was concentrated under reduced pressure, and the following steps were repeated twice: toluene was added thereto, and the mixture was concentrated under reduced pressure to obtain the titled compound (200 mg).

LC-MS: $[M+H]^+$ / Rt (min) 290.0 / 0.801 (Method A)

Reference example 33

[6-(4-Methylpiperidin-1-yl)-1H-pyrazolo[3,4-b]pyrazin-1-yl]acetic acid hydrochloride

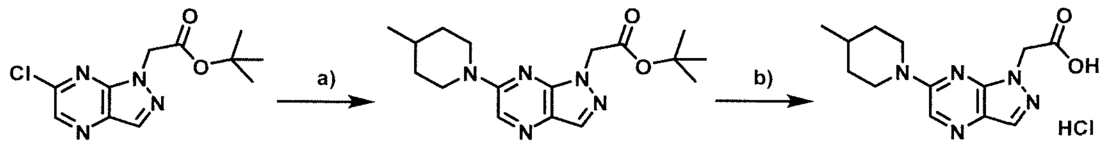


32A 33A

a) To a solution of Compound 32A (330 mg) in toluene (5 mL) were added sodium tert-butoxide (236 mg), 4-methylpiperidine (0.30 mL), and [(2-di-tert-butylphosphino-2',4',6'-triisopropylbiphenyl)-2-(2'-amino-1,1'-biphenyl)]palladium(II) methanesulfonate (98 mg), and the mixture was stirred at 120°C for 6 hours. Water was added thereto, and the mixture was extracted with ethyl acetate. The organic layer was washed with brine, dried over anhydrous sodium sulfate, filtered, and concentrated under reduced pressure. The residue was purified by silica gel chromatography (solvent; hexane:ethyl acetate = 90:10, and then 0:100) to obtain Compound 33A (161 mg).
LC-MS: $[M+H]^+$ / Rt (min) 332.3 / 2.14 (Method B)
b) According to the method of c) in Reference example 32, the titled compound (150 mg) was obtained by using Compound 33A (161 mg).

LC-MS: $[M+H]^+$ / Rt (min) 276.3 / 1.93 (Method B)

Reference example 34

[6-(4-Methylcyclohex-1-en-1-yl)-1H-pyrazolo[3,4-b]pyrazin-1-yl]acetic acid

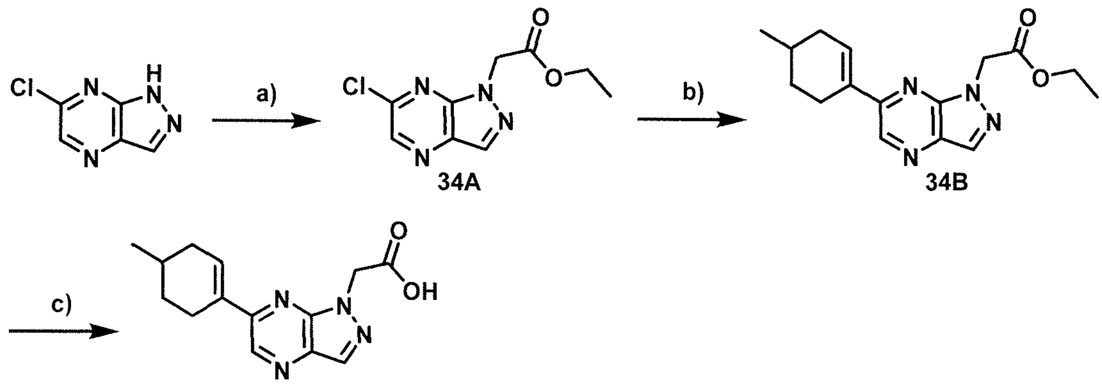


34A 34B

a) According to the method of a) in Reference example 32, Compound 34A (12.5 g) was obtained by using 6-chloro-1H-pyrazolo{3,4-b}pyrazine (15.0 g) and ethyl chloroacetate (11.6 mL).
LC-MS: $[M+H]^+$ / Rt (min) 240.9 / 0.766 (Method A)
b) To a solution of Compound 34A (11.3 g) in acetonitrile (200 mL) were added 2-(4-methylcyclohex-1-en-1-yl)-4,4,5,5-tetramethyl-1,3,2-dioxaborolane (12.6 g), 2 mol/L aqueous potassium acetate (65.9 mL), and bis[di-tert-butyl(4-dimethylaminophenyl)phosphine]dichloropalladium(II) (3.7 g), and the mixture was stirred at 100°C for 5 hours. Water was added thereto, and the mixture was extracted with ethyl acetate. The organic layer was washed with brine, dried over anhydrous sodium sulfate, filtered, and concentrated under reduced pressure. The residue was purified by silica gel chromatography (solvent; hexane:ethyl acetate = 90:10, and then 75:25) to obtain Compound 34B (14.0 g).
LC-MS: $[M+H]^+$ / Rt (min) 302.0 / 1.108 (Method A)
c) To a solution of Compound 34B (14.0 g) in ethanol (400 mL) was added 4 mol/L aqueous sodium hydroxide (117 mL), and the mixture was stirred at room temperature for 2 hours. To the reaction solution was added 2 mol/L

hydrochloric acid to adjust pH to 3. The solution was extracted with ethyl acetate. The organic layer was dried over anhydrous sodium sulfate, filtered, and concentrated under reduced pressure. To the residue were added ethyl acetate, and the mixture was stirred for one hour, resulting in precipitation of a solid. The precipitated solid was filtered to obtain the titled compound (10.1 g).

LC-MS: $[M+H]^+$ / Rt (min) 272.9 / 0.863 (Method A)

Reference example 35

2-(6-Chloro-1H-pyrazolo[3,4-b]pyrazin-1-yl)-N-([1,2,4]triazolo[1,5-a]pyridin-7-yl)acetamide

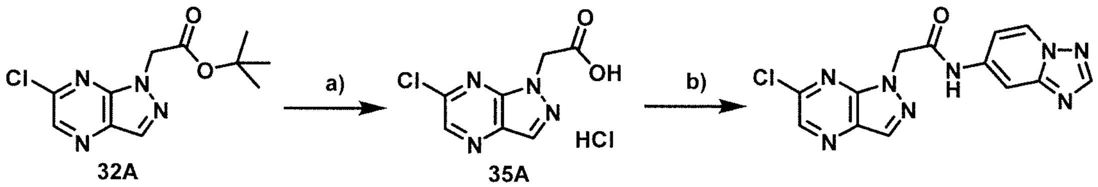

32A 35A

a) According to the method of c) in Reference example 32, Compound 35A (333 mg) was obtained using Compound 32A (560 mg) .
LC-MS: $[M+H]^+$ / Rt (min) 212.8 / 0.486 (Method A)
b) To a solution of Compound 35A (518 mg) in dichloromethane (15 mL) were added [1,2,4]triazolo[1,5-a]pyridine-7-amine dihydrochloride (517 mg), WSC (518 mg), and N,N-dimethyl-4-aminopyridine (1270 mg), and the mixture was stirred at room temperature for 3 hours. Water was added thereto, and the mixture was extracted with ethyl acetate. The organic layer was washed with brine, dried over anhydrous sodium sulfate, filtered, and concentrated under reduced pressure. The residue was purified by silica gel chromatography (solvent; hexane:ethyl acetate = 100:0, and then 96:4) to obtain the titled compound (180 mg).

LC-MS: $[M+H]^+$ / Rt (min) 328.9 / 0.566 (Method A)

Reference example 36

[6-(4-Methylcyclohexyl)-1H-pyrazolo[3,4-b]pyrazin-1-yl]acetic acid hydrochloride

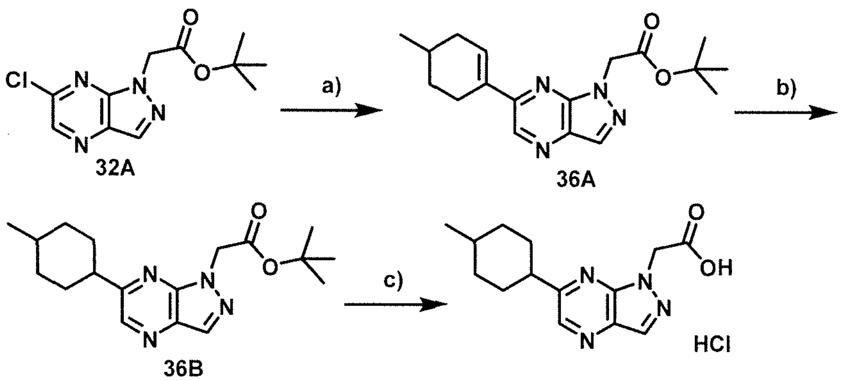

32A 36A 36B

a) According to the method of b) in Reference example 34, Compound 36A (1150 mg) was obtained by using Compound 32A (940 mg).
LC-MS: $[M+H]^+$ / Rt (min) 329.0 / 1.236 (Method A)
b) To a solution of Compound 36A (350 mg) in methanol (5 mL) was added 10 % palladium/carbon (100 mg), and the mixture was stirred under hydrogen atmosphere at room temperature for 5 hours. The palladium/carbon was filtered through Celite, and washed with methanol. The filtrate was concentrated under reduced pressure. The

residue was purified by silica gel chromatography (solvent; hexane:ethyl acetate = 90:10) to obtain Compound 36B (310 mg).
LC-MS: $[M+H]^+$ / Rt (min) 331.0 / 1.237 (Method A)
c) According to the method of c) in Reference example 32, the titled compound (257 mg) was obtained by using Compound 36B (310 mg).

LC-MS: $[M+H]^+$ / Rt (min) 275.0 / 0.861 (Method A)

Reference example 37

2-(6-Chloro-1H-pyrazolo[3,4-b]pyrazin-1-yl)-N-(4-cyanophenyl)acetamide

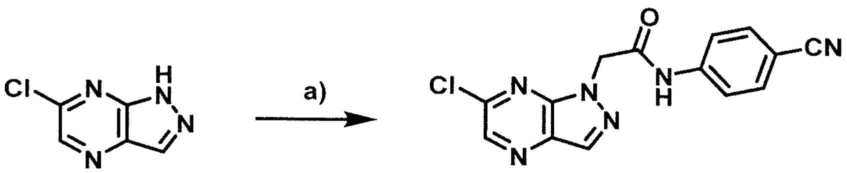


a) According to the method of a) in Reference example 32, the titled compound (21.0 mg) was obtained by using 6-chloro-1H-pyrazolo{3,4-b}pyrazine (20.0 mg) and the compound of Reference example 1 (27.7 mg).

LC-MS: $[M+H]^+$ / Rt (min) 313.1 / 0.784 (Method A)

Reference example 38

2-[6-(Methanesulfonyl)-1H-pyrazolo[3,4-d]pyrimidin-1-yl]-N-([1,2,4]triazolo[1,5-a]pyridin-7-yl)acetamide

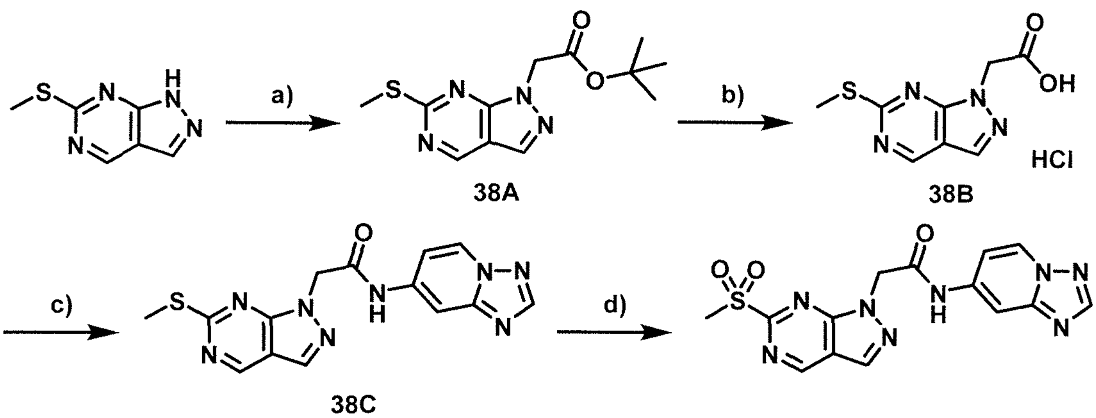

38A
38B
38C

a) To a solution of 6-(methylthio)-1H-pyrazolo[3,4-d]pyrimidine (866 mg) in dimethylformamide (5.2 mL) were added tert-butyl 2-bromoacetate (0.917 mL) and potassium carbonate (1080 mg), and the mixture was stirred at room temperature for 16 hours. Water was added thereto, and the mixture was extracted with ethyl acetate. The organic layer was washed with brine, dried over anhydrous sodium sulfate, filtered, and concentrated under reduced pressure. The residue was purified by silica gel chromatography (solvent; hexane:ethyl acetate = 75:25, and then 50:50) to obtain Compound 38A (772 mg).
LC-MS: $[M+H]^+$ / Rt (min) 280.9 / 0.911 (Method A)
b) According to the method of c) in Reference example 32, Compound 38B (620 mg) was obtained by using Compound 38A (772 mg).
LC-MS: $[M+H]^+$ / Rt (min) 224.9 / 0.504 (Method A)
c) According to the method of b) in Reference example 35, Compound 38C (177 mg) was obtained by using Compound 38B (200 mg).
LC-MS: $[M+H]^+$ / Rt (min) 340.9 / 0.548 (Method A)
d) To a solution of Compound 38C (78 mg) in tetrahydrofuran (2.3 mL) was added meta-chloroperbenzoic acid (169 mg), and the mixture was stirred at room temperature for 16 hours. To the reaction solution were assed saturated aqueous sodium bicarbonate and saturated aqueous sodium thiosulfate, and the mixture was extracted with chloroform. The organic layer was washed with brine, dried over anhydrous sodium sulfate, filtered, and concentrated under reduced pressure. The residue was purified by silica gel chromatography (solvent; chloroform:methanol = 99:1, then 90:10) to obtain the titled compound (100 mg).

LC-MS: $[M+H]^+$ / Rt (min) 372.9 / 0.430 (Method A)

Reference example 39

[6-(4-Methylpiperidin-1-yl)-1H-pyrazolo[3,4-b]pyrimidin-1-yl]acetic acid hydrochloride

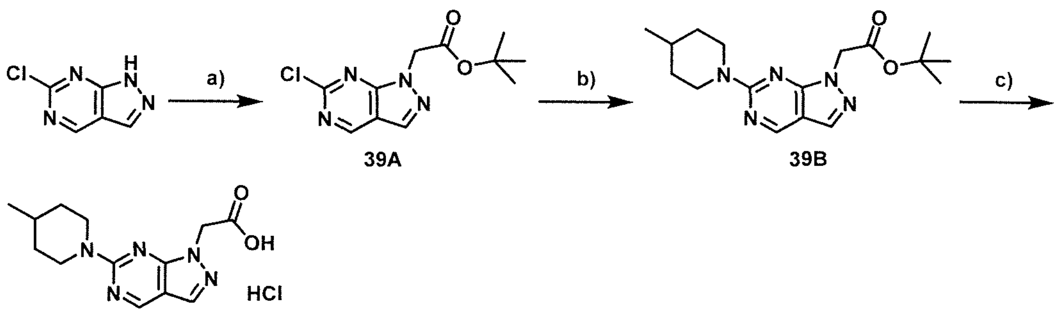


a) According to the method of a) in Reference example 32, Compound 39A (1.25 g) was obtained by using 6-chloro-1H-pyrazolo{3,4-b}pyrimidine (1.24 g) and tert-butyl bromoacetate (12.0 mL).
LC-MS: $[M+H]^+$ / Rt (min) 269.2 / 0.859 (Method A)
b) According to the method of b) in Reference example 32, Compound 39B (133 mg) was obtained by using Compound 39A (300 mg) and 4-methylpiperidine (0.79 mL).
LC-MS: $[M+H]^+$ / Rt (min) 332.2 / 1.185 (Method A)
c) According to the method of c) in Reference example 32, the titled compound (109 mg) was obtained by using Compound 39B (133 mg).

LC-MS: $[M+H]^+$ / Rt (min) 276.1 / 0.728 (Method D)

Reference example 40

[6-(4-Methylcyclohex-1-en-1-yl)-1H-pyrazolo[3,4-b]pyrimidin-1-yl]acetic acid hydrochloride

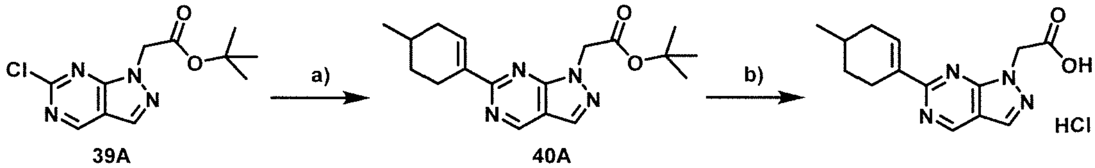


a) According to the method of b) in Reference example 34, Compound 40A (290 mg) was obtained by using Compound 39A (400 mg).
LC-MS: $[M+H]^+$ / Rt (min) 329.4 / 1.184 (Method D)
b) According to the method of c) in Reference example 32, the titled compound (240 mg) was obtained by using Compound 40A (290 mg) .

LC-MS: $[M+H]^+$ / Rt (min) 273.1 / 0.758 (Method D)

Reference example 41

2-(6-Chloro-1H-pyrazolo[3,4-b]pyrimidin-1-yl)-N-([1,2,4]triazolo[1,5-a]pyridin-7-yl) acetamide

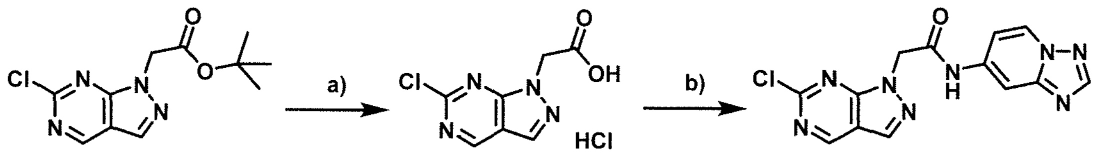


39A 41A

a) According to the method of c) in Reference example 32, Compound 41A (820 mg) was obtained by using Compound 39A (1.0 g).
LC-MS: $[M+H]^+$ / Rt (min) 212.9 / 0.403 (Method A)
b) According to the method of b) in Reference example 35, the titled compound (57 mg) was obtained by using Compound 41A (50 mg) .

LC-MS: $[M+H]^+$ / Rt (min) 328.9 / 0.566 (Method A)

Reference example 42

[6-(4-Methylpiperidin-1-yl)-1H-pyrazolo[3,4-b]pyridin-1-yl]acetic acid hydrochloride

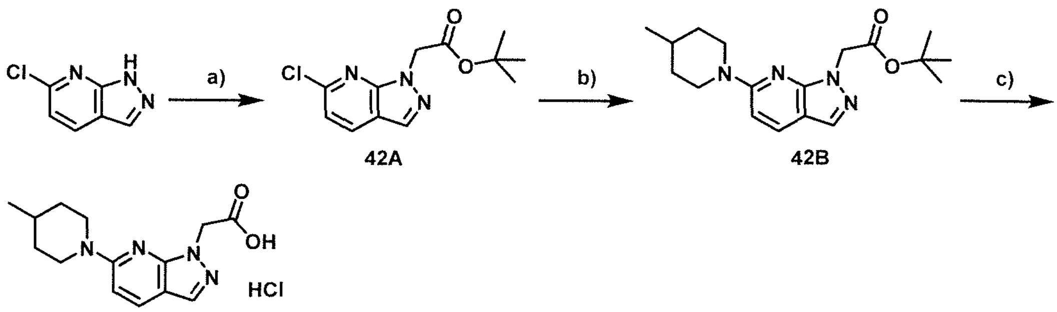


42A 42B

a) According to the method of a) in Reference example 32, Compound 42A (0.96 g) was obtained by using 6-chloro-1H-pyrazolo{3,4-b}pyridine (1.0 g) and tert-butyl bromoacetate (1.43 mL).
LC-MS: $[M+H]^+$ / Rt (min) 269.2 / 0.859 (Method A)
b) According to the method of b) in Reference example 32, Compound 42B (1.19 g) was obtained by using Compound 42A (0.96 g) and 4-methylpiperidine (2.54 mL).
LC-MS: $[M+H]^+$ / Rt (min) 331.3 / 1.206 (Method A)
c) According to the method of c) in Reference example 32, the titled compound (0.86 g) was obtained by using Compound 42B (1.19 g).

LC-MS: $[M+H]^+$ / Rt (min) 275.2 / 0.865 (Method A)

Reference example 43

[6-(4-Methylcyclohex-1-en-1-yl)-1H-pyrazolo[3,4-b]pyridin-1-yl]acetic acid hydrochloride

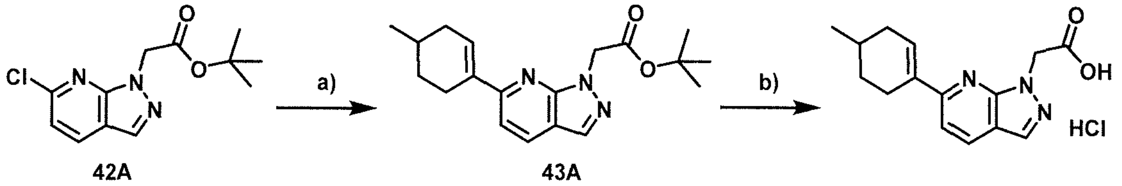

a) According to the method of b) in Reference example 34, Compound 43A (1030 mg) was obtained by using Compound 42A (922 mg).
LC-MS: $[M+H]^+$ / Rt (min) 328.3 / 1.252 (Method A)
b) According to the method of c) in Reference example 32, the titled compound (840 mg) was obtained by using Compound 43A (1030 mg).

LC-MS: $[M+H]^+$ / Rt (min) 272.1 / 0.941 (Method A)

Reference example 44

2-(6-Chloro-1H-pyrazolo[3,4-b]pyridin-1-yl)-N-([1,2,4]triazolo[1,5-a]pyridin-7-yl)acetamide

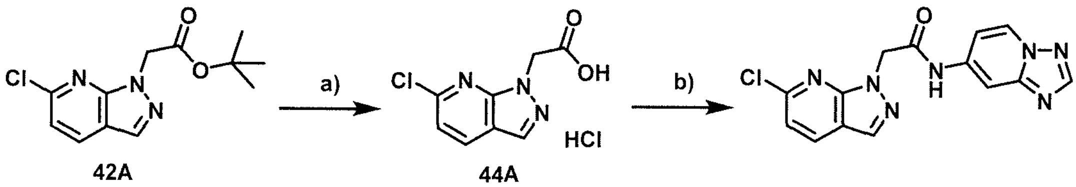

a) According to the method of c) in Reference example 32, Compound 44A (1.06 g) was obtained by using Compound 42A (1.82 g).
LC-MS: $[M+H]^+$ / Rt (min) 211.9 / 0.559 (Method A)
b) According to the method of b) in Reference example 35, the titled compound (763 mg) was obtained by using Compound 44A (550 mg).

LC-MS: $[M+H]^+$ / Rt (min) 327.9 / 0.621 (Method A)

Reference example 45

[6-(4-Methylcyclohex-1-en-1-yl)-1H-indazol-1-yl]acetic acid hydrochloride

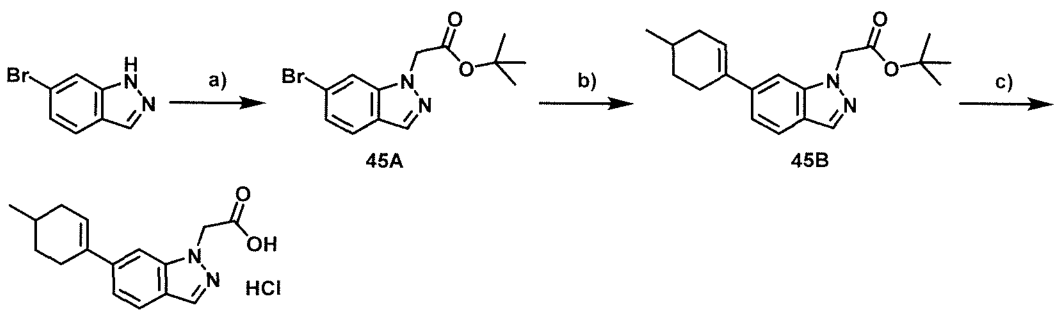

a) According to the method of a) in Reference example 32, Compound 45A (1.1 g) was obtained by using 6-bromo-1H-indazole (1.0 g) and tert-butyl bromoacetate (0.89 mL).
LC-MS: $[M+H]^+$ / Rt (min) 313.1 / 1.025 (Method A)
b) According to the method of b) in Reference example 34, Compound 45B (503 mg) was obtained by using Compound 45A (500 mg).

LC-MS: $[M+H]^+$ / Rt (min) 328.3 / 1.252 (Method A)

c) According to the method of c) in Reference example 32, the titled compound (390 mg) was obtained by using Compound 45B (503 mg).

LC-MS: $[M+H]^+$ / Rt (min) 271.2 / 0.980 (Method A)

Example 1

N-(4-Cyanophenyl)-2-[3-(4-methylpiperidin-1-yl)-6-oxopyridazin-1(6H)-yl]acetamide

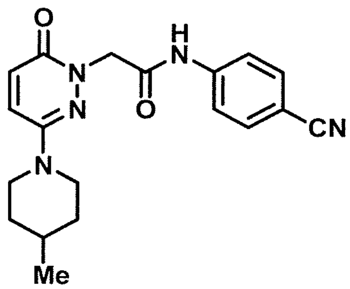

To a solution of the compound of Reference example 2 (1.0 g) in dimethylformamide (14 mL) were added potassium carbonate (1.43 g) and the compound of Reference example 1 (1.0 g). After stirring at room temperature for 24 hours, water was added thereto, and the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 2:3, and then ethyl acetate) to obtain the titled compound (1.21 g). $^1H$-NMR (400 MHz, $CDCl_3$) $\delta$: 9.79 (s, 1H), 7.63 (dt, J = 9.0, 2.0 Hz, 2H), 7.55 (dt, J = 8.5, 1.8 Hz, 2H), 7.24 (d, J = 9.8 Hz, 1H), 6.93 (d, J = 10.4 Hz, 1H), 4.84 (s, 2H), 3.84 (d, J = 13.4 Hz, 2H), 2.77 (td, J = 12.8, 2.4 Hz, 2H), 1.75-1.68 (m, 2H), 1.58-1.53 (m, 1H), 1.25 (dd, J = 12.5, 4.0 Hz, 1H), 1.19 (dd, J = 12.2, 4.3 Hz, 1H), 0.97 (d, J = 6.7 Hz, 3H) .

Example 2

N-(4-Cyanophenyl)-2-[3-(4,4-dimethylpiperidin-1-yl)-6-oxopyridazin-1(6H)-yl]acetamide

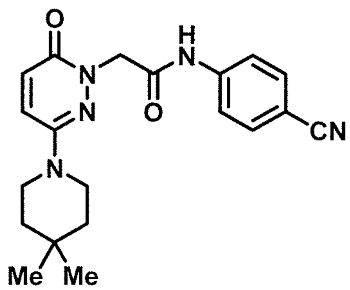

A solution of the compound of Reference example 3 (60 mg), 4,4-dimethylpiperidine hydrochloride (93 mg), and diisopropylethylamine (1 mL) in dimethylacetamide (0.5 mL) was stirred at 150°C for 11 hours. After the completion of the reaction, water was added thereto, and the mixture was extracted with ethyl acetate. The organic layer was dried over anhydrous sodium sulfate, filtered, and concentrated under reduced pressure. The residue was purified by reversed-phase HPLC (eluent; 0.035 % trifluoroacetic acid in acetonitrile/water), and then by amino silica gel column chromatography (solvent; chloroform:methanol = 99:1, and then 93:7) to obtain the titled compound (22 mg).

$^1H$-NMR (400 MHz, $CDCl_3$) $\delta$: 9.78 (s, 1H), 7.65 (d, J = 8.5 Hz, 2H), 7.57 (d, J = 8.5 Hz, 2H), 7.25 (d, J = 9.8 Hz, 1H), 6.94 (d, J = 10.4 Hz, 1H), 4.84 (s, 2H), 3.32-3.28 (m, 4H), 1.46-1.42 (m, 4H), 0.98 (s, 6H).

Examples 3 - 36

According to the method of Example 1 or 2 and common reaction conditions, the compounds of Examples 3 to 36 were obtained by using each corresponding material compound.

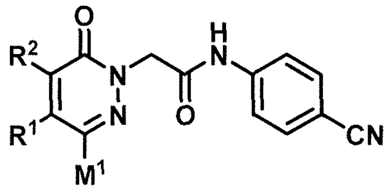

| Example | $M^1$ | $R^1$ | $R^2$ | LC-MS: $[M+H]^+$ / Rt (min) (Method) |
|---|---|---|---|---|
| 3 | 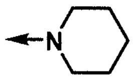 | H | H | 338.3 / 0.810 (Method A) |
| 4 | 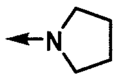 | H | H | 324.2 / 0.730 (Method A) |
| 5 | 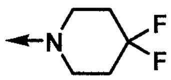 | H | H | 374.2 / 0.778 (Method A) |
| 6 | 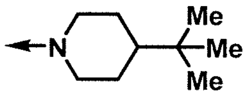 | H | H | 394.4 / 1.05 (Method A) |
| 7 | 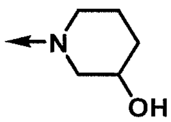 | H | H | 354.3 / 0.586 (Method A) |
| 8 | 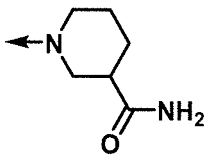 | H | H | 381.3 / 0.552 (Method A) |
| 9 | 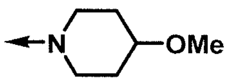 | H | H | 368.3 / 0.689 (Method A) |
| 10 | 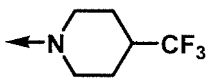 | H | H | 406.3 / 0.864 (Method A) |
| 11 | 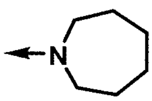 | H | H | 352.3 / 0.860 (Method A) |
| 12 | 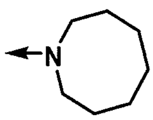 | H | H | 366.4 / 0.872 (Method A) |

(continued)

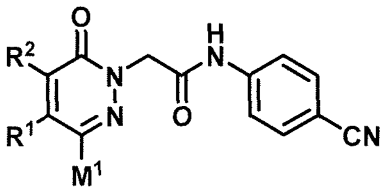

| Example | $M^1$ | $R^1$ | $R^2$ | LC-MS: $[M+H]^+$ / Rt (min) (Method) |
|---|---|---|---|---|
| 13 | 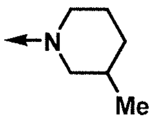 | H | H | 352.3 / 0.874 (Method A) |
| 14 | 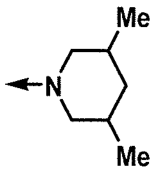 | H | H | 366.3 / 0.945 (Method A) |
| 15 | 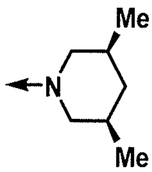 | H | H | 366.4 / 0.951 (Method A) |
| 16 | 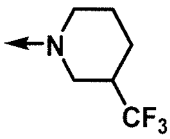 | H | H | 406.3 / 0.875 (Method A) |
| 17 | 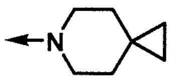 | H | H | 364.3 / 0.885 (Method A) |
| 18 | 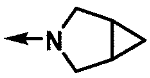 | H | H | 336.2 / 0.795 (Method A) |
| 19 | 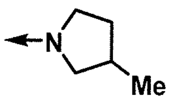 | H | H | 338.2 / 0.836 (Method A) |
| 20 | 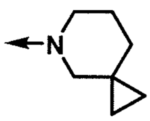 | H | H | 364.3 / 0.907 (Method A) |
| 21 | 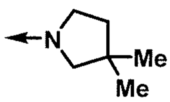 | H | H | 352.3 / 0.895 (Method A) |

(continued)

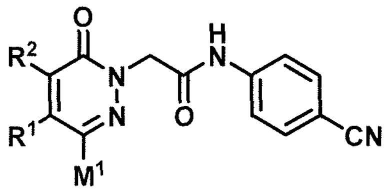

| Example | $M^1$ | $R^1$ | $R^2$ | LC-MS: $[M+H]^+$ / Rt (min) (Method) |
|---|---|---|---|---|
| 22 | 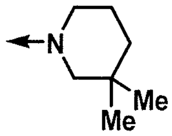 | H | H | 366.3 / 0.970 (Method A) |
| 23 | 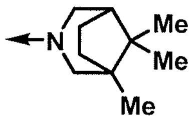 | H | H | 406.4 / 1.079 (Method A) |
| 24 | 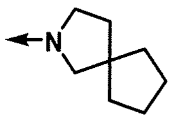 | H | H | 378.3 / 0.979 (Method A) |
| 25 | 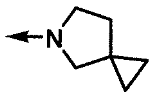 | H | H | 350.2 / 0.938 (Method A) |
| 26 | 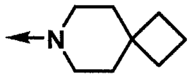 | H | H | 378.3 / 1.001 (Method A) |
| 27 | 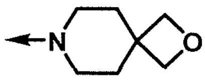 | H | H | 380.3 / 0.698 (Method A) |
| 28 | 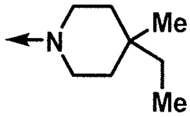 | H | H | 380.3 / 1.029 (Method A) |
| 29 | 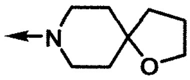 | H | H | 394.3 / 0.790 (Method A) |
| 30 | 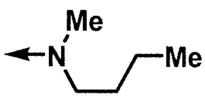 | H | H | 340.3 / 0.890 (Method A) |
| 31 | 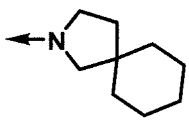 | H | H | 392.3 / 1.033 (Method A) |

(continued)

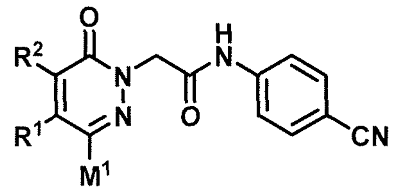

| Example | $M^1$ | $R^1$ | $R^2$ | LC-MS: $[M+H]^+$ / Rt (min) (Method) |
|---|---|---|---|---|
| 32 | 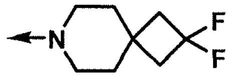 | H | H | 414.3 / 0.908 (Method A) |
| 33 | 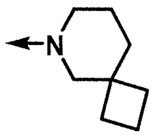 | H | H | 378.3 / 1.004 (Method A) |
| 34 | 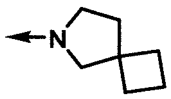 | H | H | 364.3 / 0.938 (Method A) |
| 35 | 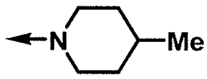 | Me | Me | 380.3 / 1.05 (Method A) |
| 36 | 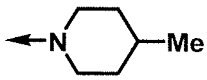 | $-(CH_2)_4-$ | | 406.3 / 1.14 (Method A) |

Example 37

N-(4-Cyanophenyl)-2-[3-(4,4-difluorocyclohex-1-en-1-yl)-6-oxopyridazin-1(6H)-yl]acetamide

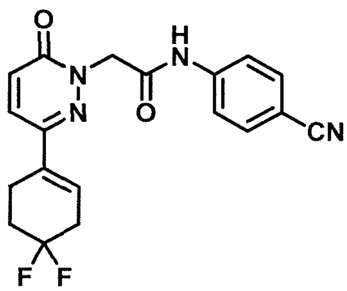

The compound of Reference example 3 (1.58 g), the compound of Reference example 4 (1.74 g), and 2 mol/L aqueous sodium carbonate (6.85 mL) were suspended in 1,2-dimethoxyethane (25 mL), and 1,1'- bis(diphenylphosphino)ferrocene palladium dichloride (401 mg) was added thereto. The mixture was stirred under a nitrogen atmosphere at 80°C for 4 hours. After cooling to room temperature, water was added thereto, and the mixture was extracted with ethyl acetate. The organic layer was dried over anhydrous sodium sulfate, filtered, and concentrated under reduced pressure. The residue was purified by silica gel column chromatography (solvent; chloroform:methanol = 99:1, and then 93:7), and recrystallized with ethanol (60 mL)-acetonitrile (20 mL) to obtain the titled compound (1.64 g).

$^1H$-NMR (400 MHz, $CDCl_3$) δ: 9.37 (s, 1H), 7.65-7.58 (m, 3H), 7.54 (d, J = 8.7 Hz, 2H), 7.04 (d, J = 9.6 Hz,

1H), 6.22 (s, 1H), 4.99 (s, 2H), 2.82-2.71 (m, 4H), 2.20-2.10 (m, 2H).

Example 38

N-(4-Cyanophenyl)-2-[3-(4,4-dimethylcyclohexyl)-6-oxopyridazin-1(6H)-yl]acetamide

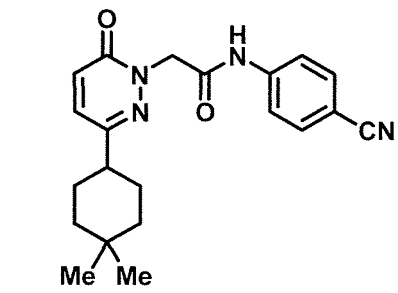

To a solution of the compound of Reference example 5 (40 mg) in dimethylformamide (2 mL) were added potassium carbonate (54 mg) and the compound of Reference example 1 (45 mg). After stirring the mixture at room temperature for 6 hours, water was added thereto, and the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 1:1, and then 1:4) to obtain the titled compound (60 mg).

$^1$H-NMR (400 MHz, $CDCl_3$) $\delta$: 9.54 (s, 1H), 7.62 (dt, J = 9.0, 2.2 Hz, 2H), 7.54 (dt, J = 9.1, 2.0 Hz, 2H), 7.27 (d, J = 9.5 Hz, 1H), 7.00 (d, J = 9.6 Hz, 1H), 4.95 (s, 2H), 2.48 (tt, J = 11.9, 3.7 Hz, 1H), 1.73-1.69 (m, 2H), 1.64-1.57 (td, J = 12.8, 3.63 Hz, 2H), 1.55-1.46 (m, 2H), 1.28 (td, J = 13.2, 4.1 Hz, 2H), 0.94 (s, 3H), 0.93 (s, 3H).

Examples 39 - 49

According to the method of Example 37 or 38 and common reaction conditions, the compounds of Examples 39 to 49 were obtained by using each corresponding material compound.

| 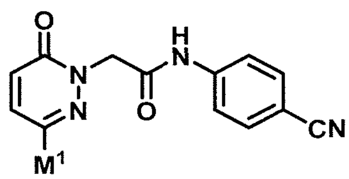 | | |
|---|---|---|
| **Example** | $M^1$ | **LC-MS: [M+H]$^+$ / Rt (min) (Method)** |
| **39** | 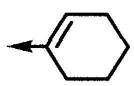 | **335.2 / 0.901 (Method A)** |
| **40** | 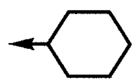 | **337.2 / 0.920 (Method A)** |
| **41** | 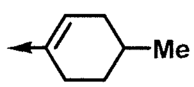 | **349.3 / 0.978 (Method A)** |
| **42** | 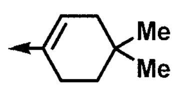 | **363.3 / 1.03 (Method A)** |

(continued)

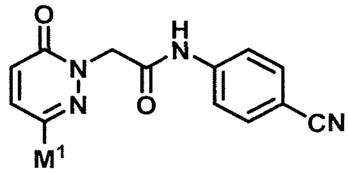

| Example | $M^1$ | LC-MS: $[M+H]^+$ / Rt (min) (Method) |
|---|---|---|
| 43 | 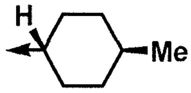 | 351.2 / 0.968 (Method A) |
| 44 | 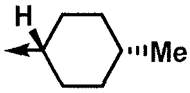 | 351.2 / 0.985 (Method A) |
| 45 | 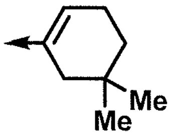 | 363.3 / 1.046 (Method A) |
| 46 | 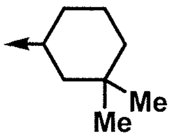 | 365.3 / 1.058 (Method A) |
| 47 | 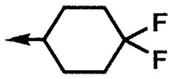 | 373.2 / 0.858 (Method A) |
| 48 | 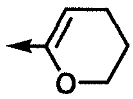 | 337.2 / 0.777 (Method A) |
| 49 | 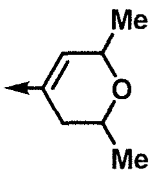 | 365.2 / 0.817 (Method A) |

Example 50

N-(1,3-Benzooxazol-5-yl)-2-[3-(methylpiperidin-1-yl)-6-oxopyridazin-1(6H)-yl]acetamide

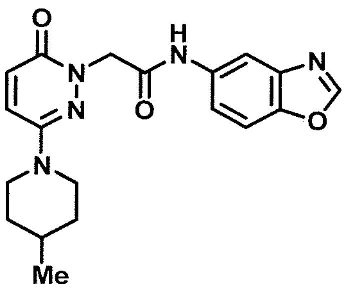

To a suspension of the compound of Reference example 6 (50 mg), 1,3-benzoxazole-5-amine (32 mg), and HATU (91 mg) in acetonitrile (1.5 mL) was added N,N-diisopropylethylamine (0.34 mL), and the mixture was stirred at room temperature for 2 hours. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; ethyl acetate:methanol = 100:0, and then 92:8) to obtain the titled compound (38 mg).

$^1$H-NMR (400 MHz, $CDCl_3$) δ: 9.35 (s, 1H), 8.09 (s, 1H), 8.06 (s, 1H), 7.48 (s, 2H), 7.23 (d, J = 9.8 Hz, 1H), 6.94 (d, J = 9.8 Hz, 1H), 4.86 (s, 2H), 3.86-3.83 (m, 2H), 2.80-2.74 (m, 2H), 1.73-1.70 (m, 2H), 1.61-1.53 (m, 1H), 1.28-1.18 (m, 2H), 0.97 (d, J = 6.4 Hz, 3H).

Examples 51 - 99

According to the method of Example 50 and common reaction conditions, the compounds of Examples 51 to 99 were obtained by using each corresponding material compound.

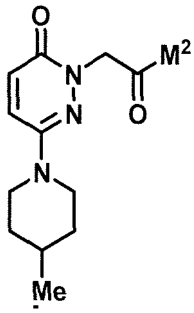

| Example | $M^2$ | Analytical data |
|---|---|---|
| 51 | 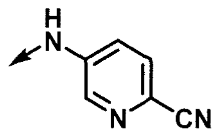 | $^1$H-NMR (400 MHz, $CDCl_3$) δ: 10.14 (br, 1H), 8.63 (s, 1H), 8.29 (d, J = 8.5 Hz, 1H), 7.61 (d, J = 8.5 Hz, 1H), 7.28-7.25 (m, 1H), 6.95 (d, J = 10.1 Hz, 1H), 4.87 (s, 2H), 3.87-3.84 (m, 2H), 2.82-2.75 (m, 2H), 1.74-1.71 (m, 2H), 1.60-1.54 (m, 1H), 1.28-1.17 (m, 2H), 0.97 (d, J = 6.4 Hz, 3H). |
| 52 | 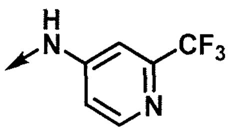 | $^1$H-NMR (400 MHz, $CDCl_3$) δ: 10.21 (s, 1H), 8.50 (d, J = 5.5 Hz, 1H), 7.83 (s, 1H), 7.60 (d, J = 5.9 Hz, 1H), 7.26 (d, J = 10.0 Hz, 1H), 6.95 (d, *J* = 9.6 Hz, 1H), 4.87 (s, 2H), 3.82 (d, J = 12.8 Hz, 2H), 2.76 (td, *J* = 12.7, 2.3 Hz, 2H), 1.70 (d, J = 12.3 Hz, 2H), 1.59-1.50 (m, 1H), 1.24 (dd, *J* = 12.1, 3.4 Hz, 1H), 1.18 (dd, *J* = 12.1, 3.4 Hz, 1H), 0.95 (d, *J* = 6.4 Hz, 3H). |
| 53 | 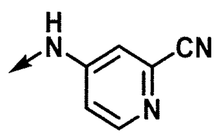 | $^1$H-NMR (400 MHz, DMSO-$d_6$) δ: 11.01 (s, 1H), 8.59 (d, *J* = 5.5 Hz, 1H), 8.08 (d, *J* = 1.8 Hz, 1H), 7.76 (dd, *J* = 5.8, 2.1 Hz, 1H), 7.57 (d, *J* = 10.4 Hz, 1H), 6.85 (d, J = 9.8 Hz, 1H), 4.75 (s, 2H), 3.81 (d, J = 12.8 Hz, 2H), 2.66 (t, J = 11.3 Hz, 2H), 1.63 (d, J = 12.2 Hz, 2H), 1.55-1.46 (m, 1H), 1.15 (dd, J = 11.9, 4.0 Hz, 1H), 1.09 (dd, J = 12.5, 3.4 Hz, 1H), 0.90 (d, J = 6.1 Hz, 3H). |

(continued)

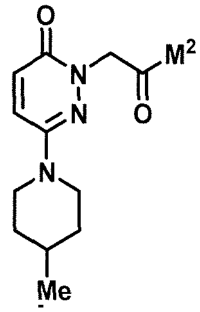

| Example | $M^2$ | Analytical data |
|---|---|---|
| 54 | 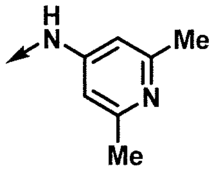 | $^1$H-NMR (400 MHz, $CDCl_3$) δ: 7.34 (s, 2H), 7.26 (d, J = 10.3 Hz, 1H), 7.16 (br, 1H), 6.90 (d, J = 10.4 Hz, 1H), 4.91 (s, 2H), 3.81 (d, J = 13.4 Hz, 2H), 2.75 (td, J = 12.8, 2.4 Hz, 2H), 2.54 (s, 6H), 1.70 (d, J = 12.8 Hz, 2H), 1.58-1.51 (m, 1H), 1.23 (dd, J = 12.2, 3.7 Hz, 1H), 1.17 (dd, J = 12.5, 3.4 Hz, 1H), 0.95 (d, J = 6.1 Hz, 3H). |
| 55 | 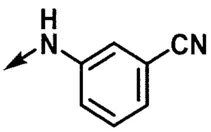 | LC-MS: $[M+H]^+$ / Rt (min) 352.2 / 1.83 (Method B) |
| 56 | 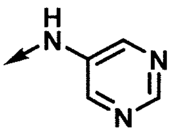 | LC-MS: $[M+H]^+$ / Rt (min) 329.2 / 1.60 (Method B) |
| 57 | 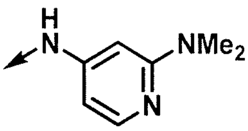 | LC-MS: $[M+H]^+$ / Rt (min) 371.2 / 1.55 (Method B) |
| 58 | 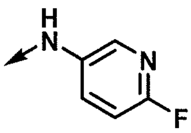 | LC-MS: $[M+H]^+$ / Rt (min) 346.2 / 1.74 (Method B) |
| 59 | 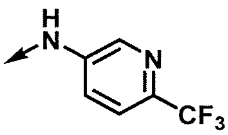 | $^1$H-NMR (400 MHz, $CDCl_3$) δ: 9.95 (br, 1H), 8.64 (s, 1H), 8.30-8.28 (m, 1H), 7.60-7.56 (m, 1H), 7.26 (d, J = 10.1 Hz, 1H), 6.95 (d, J = 10.1, 1H), 4.87 (s, 2H), 3.86-3.83 (m, 2H), 2.80-2.75 (m, 2H), 1.72 (d, J = 12.3 Hz, 2H), 1.58-1.53 (m, 1H), 1.28-1.17 (m, 2H), 0.97 (d, J = 6.4 Hz, 3H). |
| 60 | 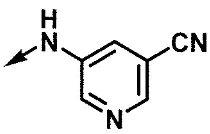 | $^1$H-NMR (400 MHz, $CDCl_3$) δ: 10.06 (br, 1H), 8.70 (d, J = 2.1 Hz, 1H), 8.56 (d, J = 2.1 Hz, 1H), 8.52 (t, J = 2.1 Hz, 1H), 7.26 (d, J = 9.6 Hz, 1H), 6.95 (d, J = 9.6 Hz, 1H), 4.86 (s, 2H), 3.85 (d, J = 12.8 Hz, 2H), 2.78 (t, J = 12.8 Hz, 2H), 1.72 (d, J = 12.3 Hz, 2H), 1.60-1.55 (m, 1H), 1.28-1.18 (m, 2H), 0.97 (d, J = 6.4 Hz, 3H). |

(continued)

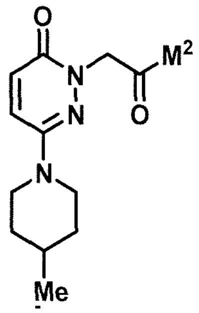

| Example | $M^2$ | Analytical data |
|---|---|---|
| 61 | 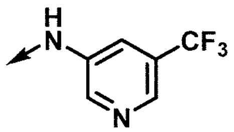 | 1H-NMR (400 MHz, $CDCl_3$) δ: 9.88 (br, 1H), 8.74 (s, 1H), 8.56 (s, 1H), 8.40 (s, 1H), 7.24 (d, J = 9.9 Hz, 1H), 6.94 (d, J = 9.9 Hz, 1H), 4.85 (s, 2H), 3.85-3.82 (m, 2H), 2.80-2.73 (m, 2H), 1.73-1.69 (m, 2H), 1.58-1.53 (m, 1H), 1.26-1.15 (m, 2H), 0.95 (d, J = 6.4 Hz, 3H). |
| 62 | 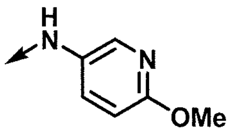 | LC-MS: $[M+H]^+$ / Rt (min) 358.2 / 1.73 (Method B) |
| 63 | 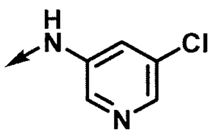 | $^1$H-NMR (400 MHz, $CDCl_3$) δ: 9.76 (br, 1H), 8.41 (d, J = 1.8 Hz, 1H), 8.25 (d, J = 1.8 Hz, 1H), 8.22 (d, J = 1.8 Hz, 1H), 7.25 (d, J = 9.8 Hz, 1H), 6.94 (d, J = 9.8 Hz, 1H), 4.85 (s, 2H), 3.86-3.82 (m, 2H), 2.81-2.74 (m, 2H), 1.73-1.70 (m, 2H), 1.61-1.53 (m, 1H), 1.27-1.17 (m, 2H), 0.97 (d, J = 6.7 Hz, 3H). |
| 64 | 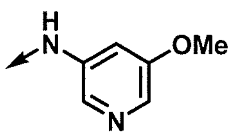 | LC-MS: [M+H]+ / Rt (min) 358.2 / 1.55 (Method B) |
| 65 | 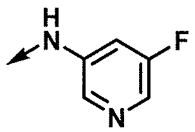 | LC-MS: [M+H]+ / Rt (min) 346.2 / 1.73 (Method B) |
| 66 | 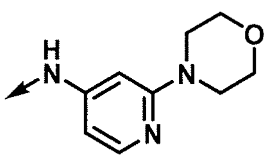 | $^1$H-NMR (400 MHz, $CDCl_3$) δ: 9.37 (br, 1H), 8.04 (d, J = 5.5 Hz, 1H), 7.23 (d, J = 9.8 Hz, 1H), 7.08 (d, J = 1.5 Hz, 1H), 6.92 (d, J = 9.8 Hz, 1H), 6.63 (dd, J = 5.5, 1.5 Hz, 1H), 4.81 (s, 2H), 3.85-3.78 (m, 6H), 3.49-3.47 (m, 4H), 2.80-2.73 (m, 2H), 1.73-1.70 (m, 2H), 1.58-1.53 (m, 1H), 1.27-1.17 (m, 2H), 0.97 (d, J = 6.7 Hz, 3H). |
| 67 | 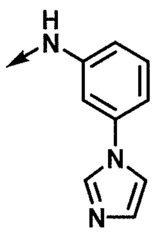 | $^1$H-NMR (400 MHz, $CDCl_3$) δ: 9.57 (br, 1H), 7.84-7.82 (m, 2H), 7.36-7.34 (m, 2H), 7.26 (d, J = 2.7 Hz, 1H), 7.24 (d, J = 10.1 Hz, 1H), 7.18 (s, 1H), 7.10-7.06 (m, 1H), 6.94 (d, J = 10.1 Hz, 1H), 4.85 (s, 2H), 3.86-3.83 (m, 2H), 2.81-2.74 (m, 2H), 1.73-1.70 (m, 2H), 1.62-1.54 (m, 1H), 1.30-1.18 (m, 2H), 0.97 (d, J = 6.4 Hz, 3H). |

(continued)

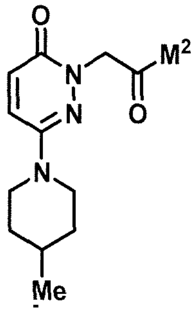

| Example | $M^2$ | Analytical data |
|---|---|---|
| 68 | 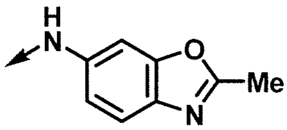 | $^1$H-NMR (400 MHz, $CDCl_3$) δ: 9.39 (br, 1H), 8.10 (d, J = 2.0 Hz, 1H), 7.50 (d, J = 8.6 Hz, 1H), 7.23 (d, J = 10.1 Hz, 1H), 7.12 (dd, J = 8.6, 2.0 Hz, 1H), 6.93 (d, J = 10.1 Hz, 1H), 4.85 (s, 2H), 3.86-3.83 (m, 2H), 2.81-2.74 (m, 2H), 2.61 (s, 3H), 1.73-1.70 (m, 2H), 1.60-1.52 (m, 1H), 1.28-1.18 (m, 2H), 0.97 (d, J = 6.4 Hz, 3H). |
| 69 | 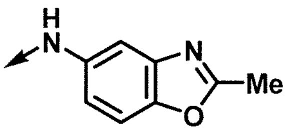 | $^1$H-NMR (400 MHz, $CDCl_3$) δ: 9.23 (br, 1H), 7.90 (s, 1H), 7.40-7.34 (m, 2H), 7.22 (d, J = 9.9 Hz, 1H), 6.93 (d, J = 9.9 Hz, 1H), 4.85 (s, 2H), 3.86-3.83 (m, 2H), 2.80-2.74 (m, 2H), 2.61 (s, 3H), 1.73-1.70 (m, 2H), 1.60-1.52 (m, 1H), 1.28-1.18 (m, 2H), 0.97 (d, J = 6.4 Hz, 3H). |
| 70 | 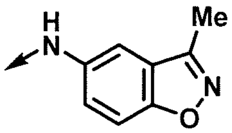 | $^1$H-NMR (400 MHz, $CDCl_3$) δ: 9.50 (br, 1H), 8.08 (s, 1H), 7.43-7.42 (m, 2H), 7.24 (d, J = 10.1 Hz, 1H), 6.94 (d, J = 10.1 Hz, 1H), 4.87 (s, 2H), 3.87-3.83 (m, 2H), 2.781-2.74 (m, 2H), 2.53 (s, 3H), 1.74-1.70 (m, 2H), 1.60-1.53 (m, 1H), 1.28-1.18 (m, 2H), 0.97 (d, J = 6.4 Hz, 3H). |
| 71 | 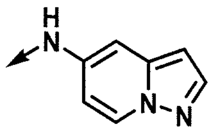 | $^1$H-NMR (400 MHz, $CDCl_3$) δ: 9.59 (br, 1H), 8.32 (d, J = 7.8 Hz, 1H), 7.99 (d, J = 1.8 Hz, 1H), 7.87 (d, J = 2.3 Hz, 1H), 7.24 (d, J = 10.3 Hz, 1H), 6.94 (d, J = 10.3 Hz, 1H), 6.72 (dd, J = 7.8, 2.3 Hz, 1H), 6.38 (d, J = 1.8 Hz, 1H), 4.85 (s, 2H), 3.87-3.83 (m, 2H), 2.81-2.74 (m, 2H), 1.74-1.70 (m, 2H), 1.61-1.53 (m, 1H), 1.28-1.18 (m, 2H), 0.97 (d, J = 6.9 Hz, 3H). |
| 72 | 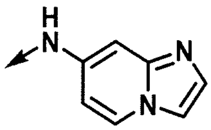 | $^1$H-NMR (400 MHz, $CDCl_3$) δ: 9.72 (br, 1H), 7.95 (d, J = 7.3 Hz, 1H), 7.87 (br, 1H), 7.52 (br s, 1H), 7.43 (s, 1H), 7.23 (d, J = 10.1 Hz, 1H), 6.99 (dd, J = 7.3, 2.4 Hz, 1H), 6.92 (d, J = 10.1 Hz, 1H), 4.86 (s, 2H), 3.85-3.82 (m, 2H), 2.80-2.73 (m, 2H), 1.73-1.70 (m, 2H), 1.61-1.52 (m, 1H), 1.27-1.17 (m, 2H), 0.96 (d, J = 6.7 Hz, 3H). |
| 73 | 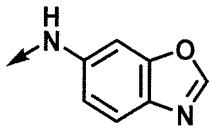 | $^1$H-NMR (400 MHz, $CDCl_3$) δ: 9.52 (br, 1H), 8.24 (d, J = 1.8 Hz, 1H), 8.04 (s, 1H), 7.65 (d, J = 8.5 Hz, 1H), 7.23 (d, J = 10.4 Hz, 1H), 7.19 (dd, J = 8.5, 1.8 Hz, 1H), 6.94 (d, J = 10.4 Hz, 1H), 4.86 (s, 2H), 3.87-3.83 (m, 2H), 2.81-2.74 (m, 2H), 1.73-1.71 (m, 2H), 1.61-1.52 (m, 1H), 1.28-1.19 (m, 2H), 0.97 (d, J = 6.7 Hz, 3H). |
| 74 | 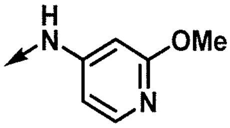 | LC-MS: [M+H]+ / Rt (min) 358.2 / 1.59 (Method B) |

(continued)

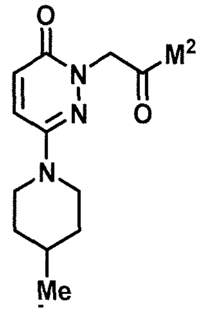

| Example | $M^2$ | Analytical data |
|---|---|---|
| 75 | 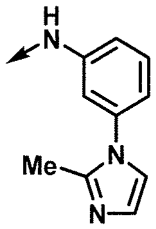 | LC-MS: [M+H]+ / Rt (min) 407.2 / 1.57 (Method B) |
| 76 | 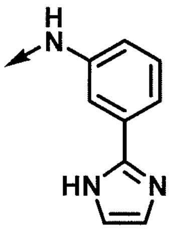 | LC-MS: [M+H]+ / Rt (min) 393.2 / 1.55 (Method B) |
| 77 | 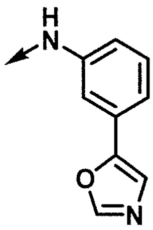 | LC-MS: [M+H]+ / Rt (min) 394.2 / 1.83 (Method B) |
| 78 | 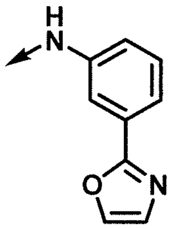 | LC-MS: [M+H]+ / Rt (min) 394.2 / 1.85 (Method B) |
| 79 | 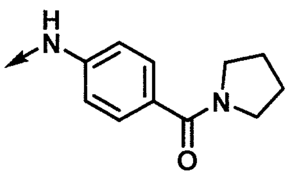 | $^1$H-NMR (400 MHz, $CDCl_3$) $\delta$: 9.36 (br, 1H), 7.55 (d, J = 8.5 Hz, 2H), 7.48 (d, J = 8.5 Hz, 2H), 7.22 (d, J = 10.1 Hz, 1H), 6.92 (d, J = 10.1 Hz, 1H), 4.83 (s, 2H), 3.85-3.82 (m, 2H), 3.63-3.60 (m, 2H), 3.44-3.41 (m, 2H), 2.79-2.73 (m, 2H), 1.95-1.90 (m, 2H), 1.88-1.84 (m, 2H), 1.73-1.69 (br m, 2H), 1.60-1.51 (br m, 1H), 1.27-1.18 (m, 2H), 0.96 (d, J = 6.4 Hz, 3H). |

(continued)

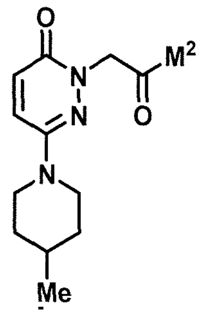

| Example | $M^2$ | Analytical data |
|---|---|---|
| 80 | 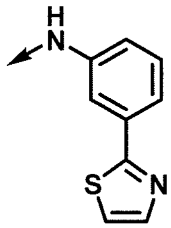 | $^1$H-NMR (400 MHz, $CDCl_3$) δ: 9.21 (br, 1H), 8.06-8.05 (m, 1H), 7.85-7.84 (m, 1H), 7.72-7.68 (m, 2H), 7.40-7.36 (m, 1H), 7.33 (d, J = 3.1 Hz, 1H), 7.23 (d, J = 9.8 Hz, 1H), 6.94 (d, J = 9.8 Hz, 1H), 4.85 (s, 2H), 3.86-3.83 (m, 2H), 2.81-2.74 (m, 2H), 1.73-1.71 (m, 2H), 1.62-1.52 (m, 1H), 1.28-1.18 (m, 2H), 0.97 (d, J = 6.7 Hz, 3H). |
| 81 | 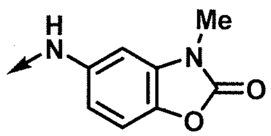 | $^1$H-NMR (400 MHz, $CDCl_3$) δ: 9.40 (br, 1H), 7.63 (d, J = 2.1 Hz, 1H), 7.24 (d, J = 10.1 Hz, 1H), 7.07 (d, J = 8.5 Hz, 1H), 6.93 (d, J = 10.1 Hz, 1H), 6.92 (dd, J = 8.5, 2.1 Hz, 1H), 4.84 (s, 2H), 3.86-3.83 (m, 2H), 3.37 (s, 3H), 2.81-2.74 (m, 2H), 1.73-1.71 (br, 2H), 1.62-1.53 (m, 1H), 1.28-1.17 (m, 2H), 0.97 (d, J = 6.7 Hz, 3H). |
| 82 | 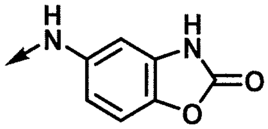 | $^1$H-NMR (400 MHz, CD3OD) δ: 7.60 (br, 1H), 7.52 (d, J = 10.1 Hz, 1H), 7.15 (d, J = 8.7 Hz, 1H), 7.10 (d, J = 8.7 Hz, 1H), 6.89 (d, J = 10.1 Hz, 1H), 4.82 (s, 2H), 3.93-3.90 (m, 2H), 2.81-2.73 (m, 2H), 1.71-1.70 (m, 2H), 1.56 (br, 1H), 1.28-1.18 (m, 2H), 0.96 (d, J = 6.4 Hz, 3H). |
| 83 | 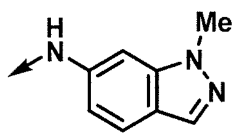 | $^1$H-NMR (400 MHz, $CDCl_3$) δ: 9.43 (br, 1H), 8.09 (s, 1H), 7.87 (s, 1H), 7.58 (d, J = 8.5 Hz, 1H), 7.23 (d, J = 10.1 Hz, 1H), 6.94 (d, J = 10.1 Hz, 1H), 6.89 (dd, J = 8.5, 1.2 Hz, 1H), 4.87 (s, 2H), 4.02 (s, 3H), 3.86-3.83 (m, 2H), 2.81-2.74 (m, 2H), 1.73-1.70 (m, 2H), 1.62-1.52 (m, 1H), 1.28-1.18 (m, 2H), 0.97 (d, J = 6.7 Hz, 3H). |
| 84 | 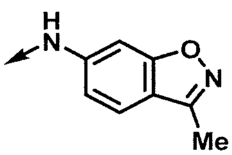 | $^1$H-NMR (400 MHz, $CDCl_3$) δ: 9.71 (br, 1H), 8.08 (s, 1H), 7.47 (d, J = 8.7 Hz, 1H), 7.24 (d, J = 10.3 Hz, 1H), 7.20 (d, J = 8.7 Hz, 1H), 6.94 (d, J = 10.3 Hz, 1H), 4.86 (s, 2H), 3.86-3.83 (m, 2H), 2.81-2.74 (m, 2H), 2.53 (s, 3H), 1.73-1.70 (m, 2H), 1.60-1.53 (m, 1H), 1.28-1.17 (m, 2H), 0.97 (d, J = 6.4 Hz, 3H). |
| 85 | 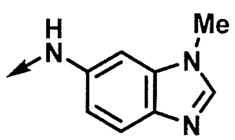 | $^1$H-NMR (400 MHz, $CDCl_3$) δ: 9.37 (br, 1H), 8.08 (s, 1H), 7.77-7.75 (br, 1H), 7.65-7.62 (m, 1H), 7.23 (d, J = 10.1 Hz, 1H), 6.99-6.97 (br, 1H), 6.94 (d, J = 10.1 Hz, 1H), 4.88 (s, 2H), 3.86-3.83 (m, 2H), 3.76 (br, 3H), 2.80-2.74 (m, 2H), 1.73-1.70 (br, 2H), 1.60-1.52 (m, 1H), 1.28-1.18 (m, 2H), 0.97 (d, J = 6.9 Hz, 3H). |
| 86 | 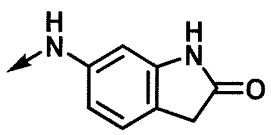 | $^1$H-NMR (400 MHz, CD3OD) δ: 7.52 (d, J = 10.1 Hz, 1H), 7.39 (d, J = 1.8 Hz, 1H), 7.17 (d, J = 7.9 Hz, 1H), 7.03 (dd, J = 7.9, 1.8 Hz, 1H), 6.89 (d, J = 10.1 Hz, 1H), 4.82 (s, 2H), 3.93-3.90 (m, 2H), 3.47 (s, 2H), 2.80-2.73 (m, 2H), 1.72-1.69 (m, 2H), 1.61-1.52 (m, 1H), 1.28-1.18 (m, 2H), 0.96 (d, J = 6.1 Hz, 3H). |

(continued)

| 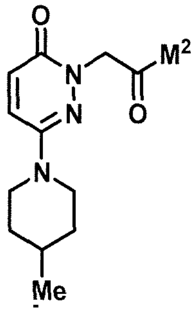 | | |
|---|---|---|
| Example | $M^2$ | Analytical data |
| 87 | 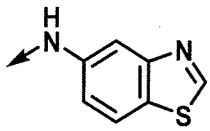 | $^1$H-NMR (400 MHz, $CDCl_3$) δ: 9.43 (br, 1H), 8.98 (s, 1H), 8.38 (d, J = 1.8 Hz, 1H), 7.84 (d, J = 8.5 Hz, 1H), 7.61 (dd, J = 8.5, 1.8 Hz, 1H), 7.23 (d, J = 10.1 Hz, 1H), 6.95 (d, J = 10.1 Hz, 1H), 4.88 (s, 2H), 3.87-3.83 (m, 2H), 2.81-2.74 (m, 2H), 1.73-1.70 (m, 2H), 1.62-1.53 (m, 1H), 1.28-1.18 (m, 2H), 0.97 (d, J = 6.1 Hz, 3H). |
| 88 | 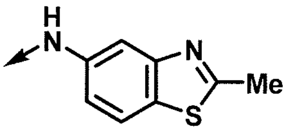 | $^1$H-NMR (400 MHz, $CDCl_3$) δ: 9.30 (br, 1H), 8.18 (d, J = 1.8 Hz, 1H), 7.70 (d, J = 8.6 Hz, 1H), 7.51 (dd, J = 8.6, 1.8 Hz, 1H), 7.22 (d, J = 10.1 Hz, 1H), 6.93 (d, J = 10.1 Hz, 1H), 4.86 (s, 2H), 3.86-3.83 (m, 2H), 2.81 (s, 3H), 2.80-2.73 (m, 2H), 1.73-1.70 (m, 2H), 1.61-1.52 (m, 1H), 1.28-1.18 (m, 2H), 0.96 (d, J = 6.7 Hz, 3H). |
| 89 | 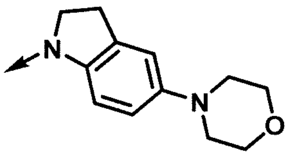 | $^1$H-NMR (DMSO-$d_6$) δ: 7.83 (d, J = 9.2 Hz, 1H), 7.56 (d, J = 9.8 Hz, 1H), 6.89 (s, 1H), 6.85 (d, J = 9.8 Hz, 1H), 6.73 (dd, J = 8.5, 2.4 Hz, 1H), 4.82 (s, 2H), 4.18-4.10 (m, 2H), 3.83-3.77 (m, 2H), 3.74-3.68 (m, 4H), 3.33-3.27 (m, 1H), 3.19-3.10 (m, 2H), 3.07-3.00 (m, 4H), 2.70-2.60 (m, 2H), 1.68-1.59 (m, 2H), 1.56-1.45 (br, 1H), 1.20-1.08 (m, 2H), 0.91 (3H, d, J = 6.7 Hz). |
| 90 | 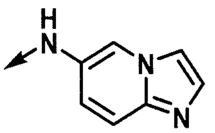 | $^1$H-NMR (400 MHz, CD3OD) δ: 9.21 (s, 1H), 7.89 (s, 1H), 7.62-7.53 (m, 3H), 7.35 (d, J = 9.5 Hz, 1H), 6.90 (d, J = 9.5 Hz, 1H), 4.87 (s, 2H), 3.94-3.90 (m, 2H), 2.80-2.74 (m, 2H), 1.72-1.68 (m, 2H), 1.57 (s, 1H), 1.28-1.17 (m, 2H), 0.96 (d, J = 6.7 Hz, 3H). |
| 91 | 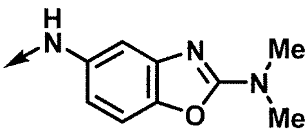 | $^1$H-NMR (400 MHz, $CDCl_3$) δ: 8.97 (br, 1H), 7.52 (s, 1H), 7.20 (d, J = 9.8 Hz, 1H), 7.14-7.14 (br, 2H), 6.91 (d, J = 9.8 Hz, 1H), 4.83 (s, 2H), 3.85-3.82 (m, 2H), 3.18 (s, 6H), 2.79-2.72 (m, 2H), 1.72-1.69 (m, 2H), 1.61-1.51 (m, 1H), 1.28-1.18 (m, 2H), 0.97 (d, J = 6.7 Hz, 3H). |
| 92 | 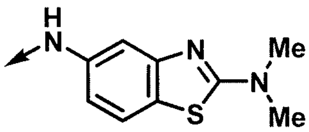 | $^1$H-NMR (400 MHz, $CDCl_3$) δ: 9.38 (br, 1H), 7.63 (d, J = 2.4 Hz, 1H), 7.42 (dd, J = 8.5, 2.4 Hz, 1H), 7.24 (d, J = 10.1 Hz, 1H), 7.20 (d, J = 8.5 Hz, 1H), 6.93 (d, J = 10.1 Hz, 1H), 4.82 (s, 2H), 3.86-3.83 (m, 2H), 3.28 (s, 3H), 2.81-2.74 (m, 2H), 2.46 (s, 3H), 1.73-1.70 (m, 2H), 1.61-1.52 (m, 1H), 1.28-1.18 (m, 2H), 0.97 (d, J = 6.1 Hz, 3H). |
| 93 | 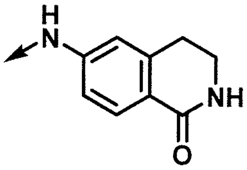 | $^1$H-NMR (400 MHz, $CDCl_3$) δ: 9.48 (br, 1H), 7.99 (d, J = 8.5 Hz, 1H), 7.63 (d, J = 1.8 Hz, 1H), 7.31 (dd, J = 8.5, 1.8 Hz, 1H), 7.23 (d, J = 9.9 Hz, 1H), 6.93 (d, J = 9.9 Hz, 1H), 5.77 (s, 1H), 4.83 (s, 2H), 3.86-3.82 (m, 2H), 3.55-3.51 (m, 2H), 2.98-2.95 (m, 2H), 2.81-2.74 (m, 2H), 1.73-1.71 (m, 2H), 1.61-1.54 (m, 1H), 1.28-1.17 (m, 2H), 0.97 (d, J = 6.9 Hz, 3H). |

(continued)

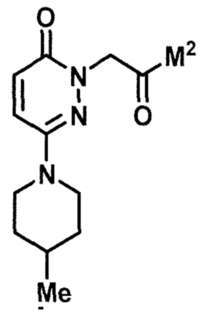

| Example | $M^2$ | Analytical data |
|---|---|---|
| 94 | 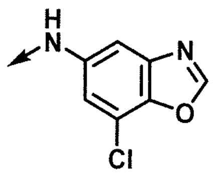 | $^1$H-NMR (400 MHz, $CDCl_3$) δ: 9.53 (br, 1H), 8.09 (s, 1H), 7.95 (d, J = 1.8 Hz, 1H), 7.62 (d, J = 1.8 Hz, 1H), 7.24 (d, J = 10.1 Hz, 1H), 6.95 (d, J = 10.1 Hz, 1H), 4.85 (s, 2H), 3.87-3.83 (m, 2H), 2.81-2.74 (m, 2H), 1.74-1.71 (m, 2H), 1.62-1.50 (m, 1H), 1.28-1.18 (m, 2H), 0.97 (d, J = 6.9 Hz, 3H). |
| 95 | 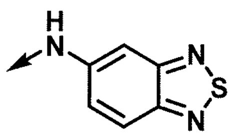 | $^1$H-NMR ($CDCl_3$) δ: 9.80 (s, 1H), 8.50 (d, J = 2.0 Hz, 1H), 7.88 (d, J = 9.9 Hz, 1H), 7.50 (dd, J = 9.1, 2.0 Hz, 1H), 7.25 (d, J = 9.1 Hz, 1H), 6.96 (d, J = 9.9 Hz, 1H), 4.89 (s, 2H), 3.87-3.84 (m, 2H), 2.82-2.75 (m, 2H), 1.74-1.71 (m, 2H), 1.61-1.53 (m, 1H), 1.28-1.18 (m, 2H), 0.97 (d, J = 6.9 Hz, 3H). |
| 96 | 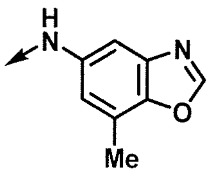 | $^1$H-NMR (400 MHz, $CDCl_3$) δ: 9.25 (br, 1H), 8.04 (s, 1H), 7.89 (s, 1H), 7.28 (br, 1H), 7.22 (d, J = 10.1 Hz, 1H), 6.94 (d, J = 10.1 Hz, 1H), 4.85 (s, 2H), 3.86-3.83 (m, 2H), 2.81-2.74 (m, 2H), 2.49 (s, 3H), 1.73-1.70 (br, 2H), 1.61-1.53 (m, 1H), 1.28-1.18 (m, 2H), 0.97 (d, J = 6.7 Hz, 3H). |
| 97 | 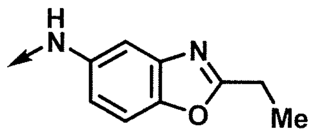 | $^1$H-NMR (400 MHz, $CDCl_3$) δ: 9.20 (br, 1H), 7.92 (d, J = 1.2 Hz, 1H), 7.40-7.35 (m, 2H), 7.22 (d, J = 10.4 Hz, 1H), 6.93 (d, J = 10.4 Hz, 1H), 4.85 (s, 2H), 3.86-3.83 (br, 2H), 2.94 (q, J = 7.7 Hz, 2H), 2.80-2.74 (m, 2H), 1.73-1.70 (br, 2H), 1.58-1.53 (m, 1H), 1.43 (t, J = 7.7 Hz, 3H), 1.28-1.18 (m, 2H), 0.97 (d, J = 6.7 Hz, 3H). |
| 98 | 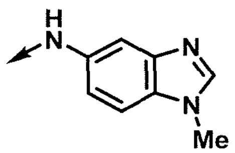 | $^1$H-NMR (400 MHz, $CDCl_3$) δ: 9.08 (s, 1H), 7.93 (s, 1H), 7.83 (s, 1H), 7.49 (d, J = 8.5 Hz, 1H), 7.29-7..26 (m, 1H), 7.20 (d, J = 9.8 Hz, 1H), 6.92 (d, J = 9.8 Hz, 1H), 4.86 (s, 2H), 3.85-3.82 (m, 2H), 3.80 (s, 3H), 2.79-2.73 (m, 2H), 1.73-1.70 (m, 2H), 1.60-1.51 (m, 1H), 1.28-1.18 (m, 2H), 0.96 (d, J = 6.7 Hz, 3H). |
| 99 | 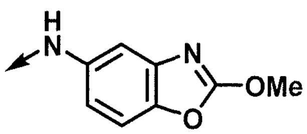 | $^1$H-NMR (400 MHz, $CDCl_3$) δ: 9.19 (br, 1H), 7.79 (s, 1H), 7.24 (s, 1H), 7.23 (s, 1H), 7.22 (d, J = 10.4 Hz, 1H), 6.92 (d, J = 10.4 Hz, 1H), 4.84 (s, 2H), 4.20 (s, 3H), 3.85-3.82 (m, 2H), 2.80-2.73 (m, 2H), 1.73-1.70 (m, 2H), 1.61-1.52 (m, 1H), 1.28-1.17 (m, 2H), 0.97 (d, J = 6.7 Hz, 3H). |

Examples 100 to 135

According to the method of Example 2, 37, 38, or 50 and corresponding reaction conditions, the compounds of Examples 100 to 135 were obtained by using each corresponding material compound.

| Example | Chemical structure | Analytical data |
|---|---|---|
| 100 | 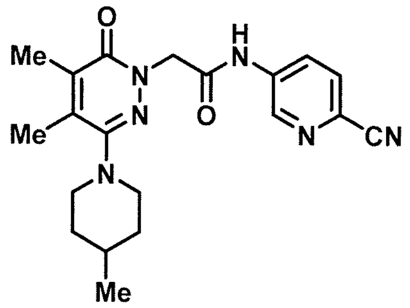 | $^1$H-NMR (DMSO-d6) δ: 10.97 (br, 1H), 8.85 (d, J = 2.7 Hz, 1H), 8.22 (dd, J = 8.5, 2.5 Hz, 1H), 7.99 (d, J = 8.2 Hz, 1H), 4.81 (s, 2H), 3.20-3.13 (m, 2H), 2.59-2.52 (m, 2H), 2.14 (3H, s), 2.03 (3H, s), 1.72-1.64 (m, 2H), 1.53-1.41 (br, 1H), 1.31-1.19 (m, 2H), 0.94 (3H, d, J = 6.4 Hz). |
| 101 | 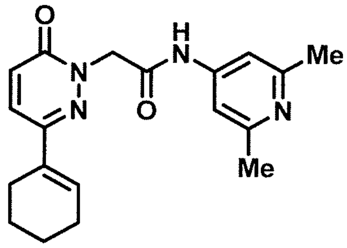 | LC-MS: [M+H] + / Rt (min) 339.2 / 0.638 (Method A) |
| 102 | 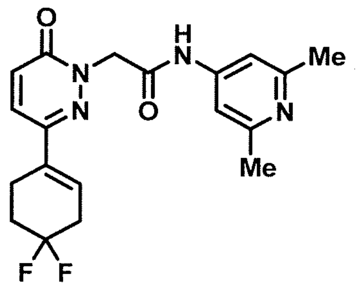 | LC-MS: [M+H] + / Rt (min) 375.3 / 0.595 (Method A) |
| 103 | 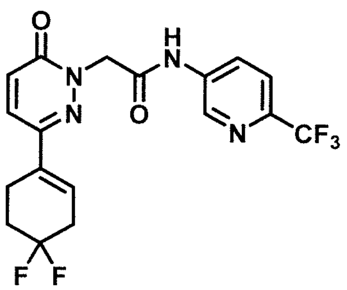 | $^1$H-NMR (400 MHz, $CDCl_3$) δ: 9.51 (s, 1H), 8.60 (d, J = 2.3 Hz, 1H), 8.26 (dd, J = 8.7, 2.3 Hz, 1H), 7.63 (d, J = 10.1 Hz, 1H), 7.54 (d, J = 8.2 Hz, 1H), 7.03 (d, J = 9.6 Hz, 1H), 6.21 (br, 1H), 5.00 (s, 2H), 2.79 - 2.68 (m, 4H), 2.19-2.06 (m, 2H). |
| 104 | 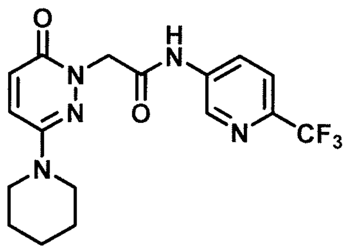 | LC-MS: [M+H] + / Rt (min) 382.2 / 0.860 (Method A) |

(continued)

| Example | Chemical structure | Analytical data |
|---|---|---|
| 105 | 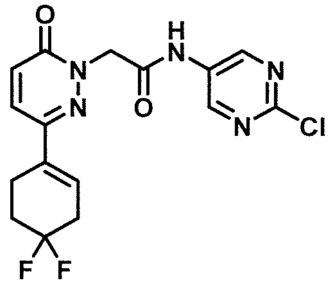 | LC-MS: [M+H] + / Rt (min) 382.2 / 0.771 (Method A) |
| 106 | 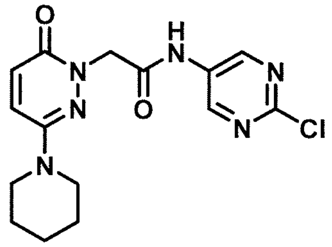 | LC-MS: [M+H] + / Rt (min) 349.2 / 0.734 (Method A) |
| 107 | 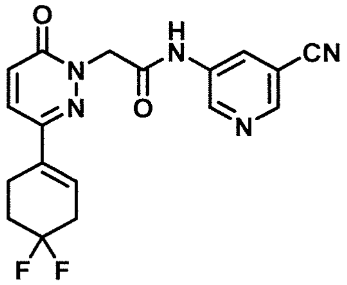 | $^1$H-NMR (400 MHz, $CDCl_3$) δ: 9.59 (s, 1H), 8.66 (d, J = 2.4 Hz, 1H), 8.54 (d, J = 1.8 Hz, 1H), 8.44 (t, J = 2.1 Hz, 1H), 7.64 (d, J = 9.8 Hz, 1H), 7.04 (d, J = 9.8 Hz, 1H), 6.22 (1H, bs), 5.00 (s, 2H), 2.79-2.70 (m, 4H), 2.19-2.09 (m, 2H). |
| 108 | 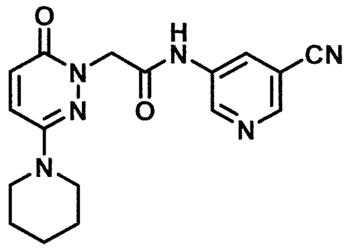 | LC-MS: [M+H] + / Rt (min) 339.2 / 0.713 (Method A) |
| 109 | 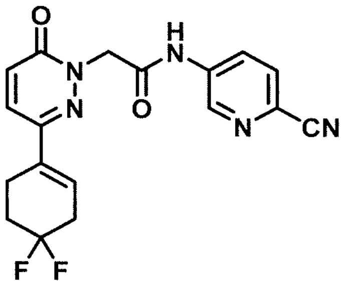 | $^1$H-NMR (400 MHz, DMSO-$d_6$) δ: 11.03 (s, 1H), 8.85 (d, J = 2.7 Hz, 1H), 8.21 (dd, J = 8.7, 1.8 Hz, 1H), 7.99 (d, J = 8.7 Hz, 1H), 7.93 (d, J = 10.1 Hz, 1H), 6.97 (d, J = 9.6 Hz, 1H), 6.45 (br, 1H), 4.94 (s, 2H), 2.78 (t, J = 13.7 Hz, 2H), 2.63-2.57 (m, 2H), 2.18-2.07 (m, 2H). |

(continued)

| Example | Chemical structure | Analytical data |
|---|---|---|
| 110 | 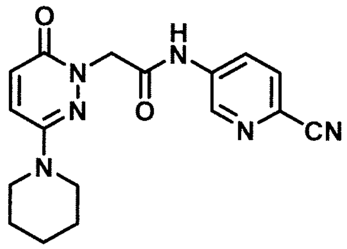 | LC-MS: [M+H] + / Rt (min) 339.2 / 0.743 (Method A) |
| 111 | 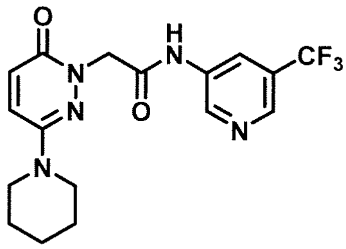 | LC-MS: [M+H] + / Rt (min) 382.3 / 0.840 (Method A) |
| 112 | 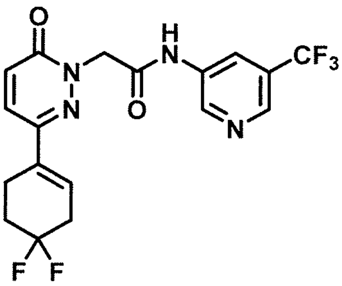 | $^1$H-NMR (400 MHz, $CDCl_3$) δ: 9.45 (s, 1H), 8.64 (s, 1H), 8.49 (s, 1H), 8.31 (s, 1H), 7.58 (dd, J = 10.1, 2.1 Hz, 1H), 6.99 (dd, J = 9.8, 1.8 Hz, 1H), 6.17 (br, 1H), 4.96 (s, 2H), 2.74-2.64 (m, 4H), 2.13-2.03 (m, 2H). |
| 113 | 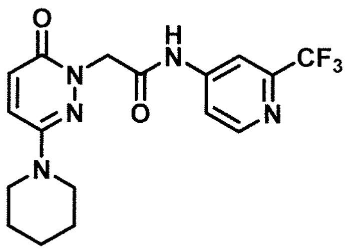 | LC-MS: [M+H] + / Rt (min) 382.3 / 0.845 (Method A) |
| 114 | 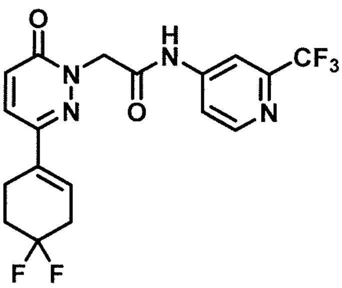 | $^1$H-NMR (400 MHz, $CDCl_3$) δ: 9.66 (s, 1H), 8.51 (d, J = 5.9 Hz, 1H), 7.78 (d, J = 1.8 Hz, 1H), 7.64 (d, J = 9.6 Hz, 1H), 7.57 (dd, J = 5.3, 2.1 Hz, 1H), 7.04 (d, J = 9.6 Hz, 1H), 6.22 (br, 1H), 4.99 (s, 2H), 2.79-2.70 (m, 4H), 2.18-2.08 (m, 2H). |

(continued)

| Example | Chemical structure | Analytical data |
|---|---|---|
| 115 | 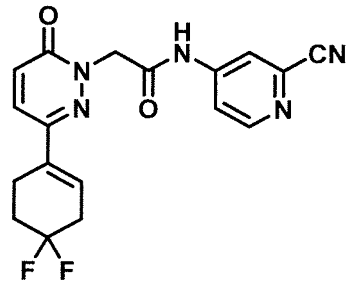 | $^1$H-NMR (400 MHz, DMSO-$d_6$) δ: 11.12 (s, 1H), 8.60 (d, J = 5.9 Hz, 1H), 8.08 (d, J = 2.3 Hz, 1H), 7.94 (d, J = 10.1 Hz, 1H), 7.76 (dd, J = 5.5, 2.3 Hz, 1H), 6.99 (d, J = 10.1 Hz, 1H), 6.45 (br, 1H), 4.94 (s, 2H), 2.78 (t, J = 13.7 Hz, 2H), 2.63-2.57 (m, 2H), 2.18-2.08 (m, 2H). |
| 116 | 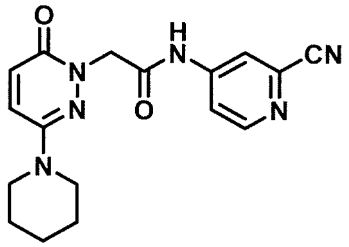 | LC-MS: [M+H] + / Rt (min) 339.2 / 0.743 (Method A) |
| 117 | 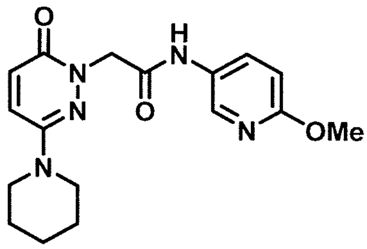 | LC-MS: [M+H] + / Rt (min) 344.3 / 0.721 (Method A) |
| 118 | 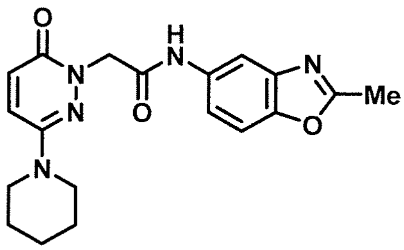 | LC-MS: [M+H] + / Rt (min) 368.3 / 0.747 (Method A) |
| 119 | 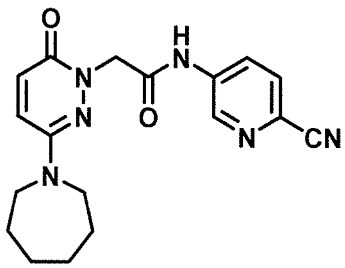 | $^1$H-NMR (400 MHz, $CDCl_3$) δ: 10.29 (s, 1H), 8.64 (d, J = 1.8 Hz, 1H), 8.28 (dd, J = 8.7, 2.7 Hz, 1H), 7.59 (d, J = 8.7 Hz, 1H), 7.15 (d, J = 10.1 Hz, 1H), 6.95 (d, J = 10.1 Hz, 1H), 4.87 (s, 2H), 3.47 (t, J = 5.9 Hz, 4H), 1.73 (br, 4H), 1.56-1.53 (m, 4H). |

(continued)

| Example | Chemical structure | Analytical data |
|---|---|---|
| 120 | 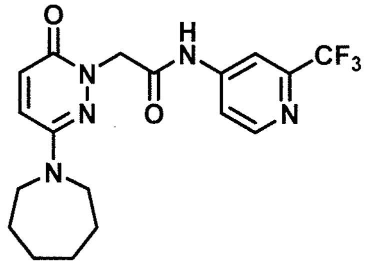 | $^1$H-NMR (400 MHz, $CDCl_3$) δ: 10.31 (s, 1H), 8.54 (d, J = 5.5 Hz, 1H), 7.85 (d, J = 1.8 Hz, 1H), 7.66 (dd, J = 5.5, 1.8 Hz, 1H), 7.15 (d, J = 10.1 Hz, 1H), 6.95 (d, J = 10.1 Hz, 1H), 4.86 (s, 2H), 3.47 (t, J = 5.9 Hz, 4H), 1.73 (br, 4H), 1.56-1.53 (m, 4H). |
| 121 | 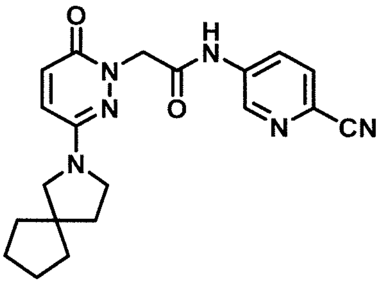 | $^1$H-NMR (400 MHz, DMSO-$d_6$) δ: 10.91 (s, 1H), 8.85 (d, J = 2.4 Hz, 1H), 8.22 (dd, J = 8.5, 2.4 Hz, 1H), 7.99 (d, J = 9.2 Hz, 1H), 7.26 (d, J = 9.8 Hz, 1H), 6.85 (d, J = 10.4 Hz, 1H), 4.75 (s, 2H), 3.36 (t, J = 6.7 Hz, 2H), 3.16 (s, 2H), 1.80 (t, J = 6.7 Hz, 2H), 1.68-1.48 (m, 8H). |
| 122 | 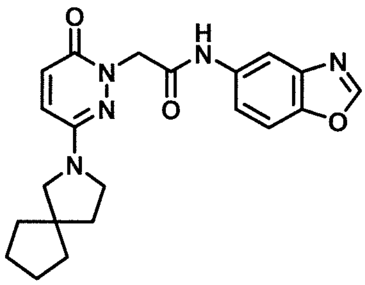 | $^1$H-NMR (400 MHz, DMSO-$d_6$) δ: 10.35 (s, 1H), 8.70 (s, 1H), 8.12 (d, J = 2.4 Hz, 1H), 7.72 (d, J = 8.5 Hz, 1H), 7.51 (dd, J = 8.9, 2.1 Hz, 1H), 7.25 (d, J = 9.8 Hz, 1H), 6.85 (d, J = 9.8 Hz, 1H), 4.71 (s, 2H), 3.40-3.34 (m, 2H), 3.17 (s, 2H), 1.80 (t, J = 7.0 Hz, 2H), 1.68-1.49 (m, 8H). |
| 123 | 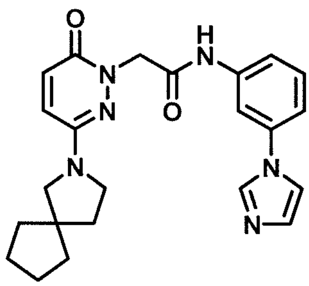 | $^1$H-NMR (400 MHz, DMSO-$d_6$) δ: 10.43 (s, 1H), 8.14 (s, 1H), 7.89 (s, 1H), 7.62 (s, 1H), 7.49-7.44 (m, 2H), 7.35-7.31 (m, 1H), 7.24 (d, J = 9.6 Hz, 1H), 7.11 (s, 1H), 6.85 (d, J = 10.1 Hz, 1H), 4.71 (s, 2H), 3.38-3.34 (m, 2H), 3.16 (s, 2H), 1.80 (t, J = 6.9 Hz, 2H), 1.64-1.53 (m, 8H). |
| 124 | 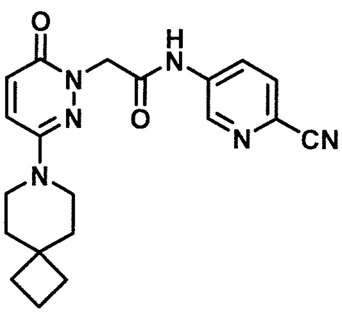 | $^1$H-NMR (400 MHz, DMSO-$d_6$) δ: 10.92 (s, 1H), 8.85 (d, J = 2.3 Hz, 1H), 8.22 (dd, J = 8.2, 2.3 Hz, 1H), 7.99 (d, J = 8.7 Hz, 1H), 7.58 (d, J = 10.1 Hz, 1H), 6.85 (d, J = 10.1 Hz, 1H), 4.76 (s, 2H), 3.17-3.15 (m, 4H), 1.88-1.82 (m, 2H), 1.76-1.72 (m, 4H), 1.59-1.56 (m, 4H). |

(continued)

| Example | Chemical structure | Analytical data |
|---|---|---|
| 125 | 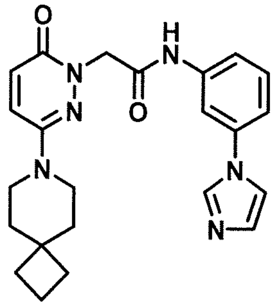 | $^1$H-NMR (400 MHz, DMSO-$d_6$) δ: 10.45 (s, 1H), 8.14 (s, 1H), 7.89 (s, 1H), 7.62 (s, 1H), 7.57 (d, J = 10.1 Hz, 1H), 7.49-7.44 (m, 2H), 7.34-7.31 (m, 1H), 7.10 (s, 1H), 6.85 (d, J = 10.1 Hz, 1H), 4.72 (s, 2H), 3.16 (m, 4H), 1.89-1.82 (m, 2H), 1.77-1.72 (m, 4H), 1.58 (m, 4H). |
| 126 | 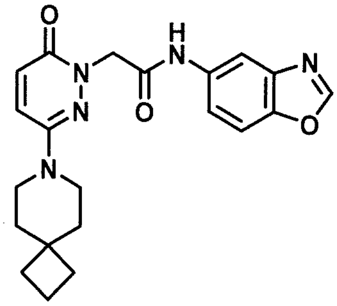 | $^1$H-NMR (400 MHz, DMSO-$d_6$) δ: 10.37 (s, 1H), 8.71 (s, 1H), 8.11 (d, J = 1.8 Hz, 1H), 7.72 (d, J = 8.5 Hz, 1H), 7.57 (d, J = 9.8 Hz, 1H), 7.51 (dd, J = 8.9, 2.1 Hz, 1H), 6.85 (d, J = 9.8 Hz, 1H), 4.72 (s, 2H), 3.18-3.15 (m, 4H), 1.90-1.82 (m, 2H), 1.77-1.72 (m, 4H), 1.60-1.56 (m, 4H). |
| 127 | 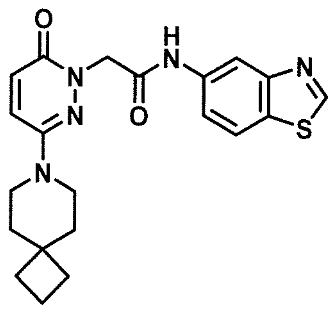 | $^1$H-NMR (400 MHz, DMSO-$d_6$) δ: 10.45 (s, 1H), 9.37 (s, 1H), 8.44 (d, J = 1.8 Hz, 1H), 8.09 (d, J = 9.2 Hz, 1H), 7.61-7.56 (m, 2H), 6.86 (d, J = 10.4 Hz, 1H), 4.75 (s, 2H), 3.19-3.14 (m, 4H), 1.90-1.82 (m, 2H), 1.77-1.72 (m, 4H), 1.60-1.56 (m, 4H). |
| 128 | 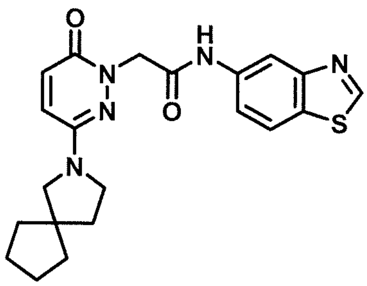 | $^1$H-NMR (400 MHz, DMSO-$d_6$) δ: 10.43 (s, 1H), 9.37 (s, 1H), 8.45 (d, J = 1.8 Hz, 1H), 8.09 (d, J = 8.7 Hz, 1H), 7.60 (dd, J = 8.7, 1.8 Hz, 1H), 7.26 (d, J = 10.1 Hz, 1H), 6.86 (d, J = 9.6 Hz, 1H), 4.74 (s, 2H), 3.40-3.36 (m, 2H), 3.18 (s, 2H), 1.83-1.78 (m, 2H), 1.66-1.52 (m, 8H). |
| 129 | 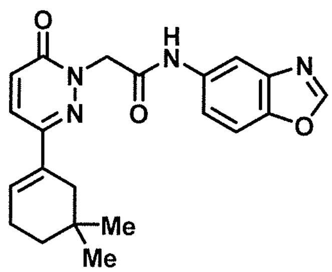 | $^1$H-NMR (400 MHz, DMSO-$d_6$) δ: 10.47 (s, 1H), 8.70 (s, 1H), 8.10 (d, J = 1.8 Hz, 1H), 7.89 (d, J = 9.8 Hz, 1H), 7.72 (d, J = 9.2 Hz, 1H), 7.51 (dd, J = 9.2, 1.8 Hz, 1H), 6.93 (d, J = 9.8 Hz, 1H), 6.56-6.52 (m, 1H), 4.88 (s, 2H), 2.27-2.20 (m, 2H), 2.14 (s, 2H), 0.92 (s, 6H). |

(continued)

| Example | Chemical structure | Analytical data |
|---|---|---|
| 130 | 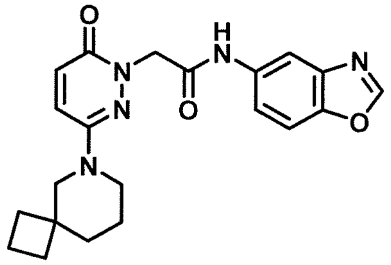 | $^1$H-NMR (400 MHz, DMSO-$d_6$) δ: 10.36 (s, 1H), 8.69 (s, 1H), 8.10 (d, J = 1.8 Hz, 1H), 7.71 (d, J = 9.2 Hz, 1H), 7.61 (d, J = 10.6 Hz, 1H), 7.51 (dd, J = 9.2, 1.8 Hz, 1H), 6.84 (d, J = 9.8 Hz, 1H), 4.72 (s, 2H), 3.15-3.10 (m, 5H), 1.94-1.46 (m, 11H). |
| 131 | 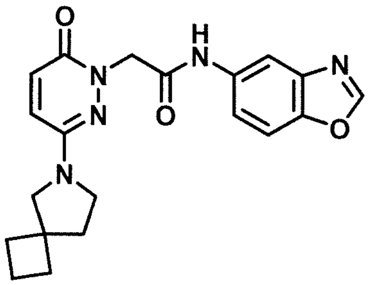 | $^1$H-NMR (400 MHZ, DMSO-$d_6$) δ: 10.33 (s, 1H), 8.70 (d, J = 1.2 Hz, 1H), 8.11 (s, 1H), 7.71 (d, J = 8.7 Hz, 1H), 7.51 (d, J = 9.1 Hz, 1H), 7.23 (d, J = 10.1 Hz, 1H), 6.84 (d, J = 10.1 Hz, 1H), 4.70 (s, 2H), 3.33-3.26 (m, 3H), 2.03-1.80 (m, 9H). |
| 132 | 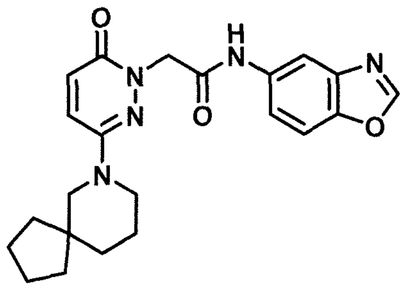 | $^1$H-NMR (400 MHz, DMSO-$d_6$) δ: 10.37 (s, 1H), 8.71 (s, 1H), 8.11 (d, J = 1.8 Hz, 1H), 7.72 (d, J = 8.7 Hz, 1H), 7.56 (d, J = 10.1 Hz, 1H), 7.52 (dd, J = 8.9, 1.8 Hz, 1H), 6.84 (d, J = 10.1 Hz, 1H), 4.72 (s, 2H), 3.21-3.18 (m, 2H), 3.00 (s, 2H), 1.63-1.52 (m, 6H), 1.49-1.43 (m, 4H), 1.33-1.27 (m, 2H). |
| 133 | 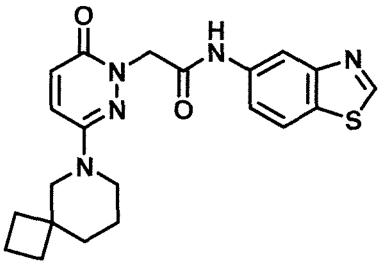 | $^1$H-NMR (400 MHz, $CDCl_3$) δ: 9.42 (br, 1H), 8.98 (s, 1H), 8.39 (d, J = 2.0 Hz, 1H), 7.85 (d, J = 8.7 Hz, 1H), 7.62 (dd, J = 8.7, 2.0 Hz, 1H), 7.26 (d, J = 9.8 Hz, 1H), 6.95 (d, J = 9.8 Hz, 1H), 4.89 (s, 2H), 3.21 (br s, 2H), 3.18 (s, 2H), 1.94-1.88 (m, 2H), 1.79-1.73 (m, 4H), 1.60-1.59 (m, 4H). |
| 134 | 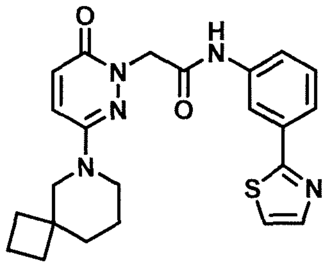 | LC-MS: [M+H] + / Rt (min) 436.2 / 2.00 (Method B) |

(continued)

| Example | Chemical structure | Analytical data |
|---|---|---|
| 135 | 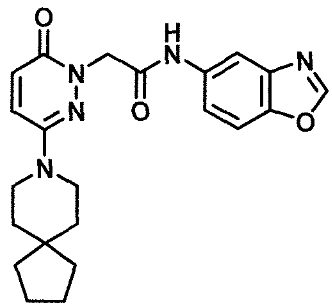 | $^1$H-NMR (400 MHz, DMSO-$d_6$) $\delta$: 10.38 (s, 1H), 8.71 (s, 1H), 8.12 (d, J = 1.8 Hz, 1H), 7.72 (d, J = 8.5 Hz, 1H), 7.58 (d, J = 10.4 Hz, 1H), 7.52 (dd, J = 8.5, 1.8 Hz, 1H), 6.86 (d, J = 10.4 Hz, 1H), 4.73 (s, 2H), 3.25-3.21 (m, 4H), 1.60-1.56 (m, 4H), 1.48-1.40 (m, 8H). |

Examples 136 - 159

According to the method of Example 37 or 50 and common reaction conditions, the compounds of Examples 136 to 159 were obtained by using each corresponding material compound.

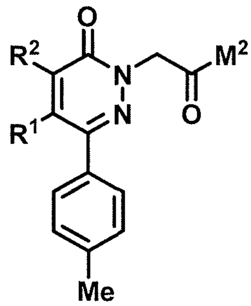

| Example | $M^2$ | $R^1$ | $R^2$ | Analytical data |
|---|---|---|---|---|
| 136 | 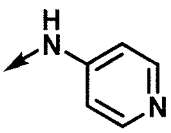 | H | H | LC-MS: [M+H] + / Rt (min) 322.2 / 0.613 (Method A) |
| 137 | 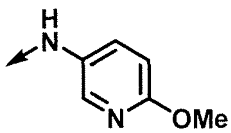 | H | H | LC-MS: [M+H] + / Rt (min) 322.2 / 0.613 (Method A) |
| 138 | 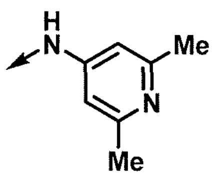 | H | H | LC-MS: [M+H] + / Rt (min) 322.2 / 0.613 (Method A) |
| 139 | 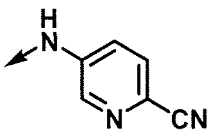 | H | H | LC-MS: [M+H] + / Rt (min) 322.2 / 0.613 (Method A) |

(continued)

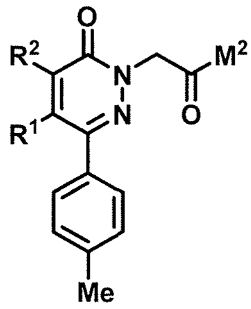

| Example | $M^2$ | $R^1$ | $R^2$ | Analytical data |
|---|---|---|---|---|
| 140 | 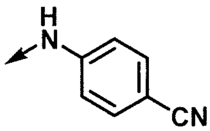 | H | H | LC-MS: [M+H] + / Rt (min) 322.2 / 0.613 (Method A) |
| 141 | 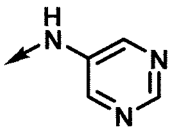 | H | H | LC-MS: [M+H] + / Rt (min) 322.2 / 0.613 (Method A) |
| 142 | 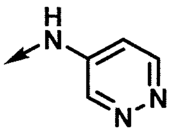 | H | H | LC-MS: [M+H] + / Rt (min) 322.2 / 0.613 (Method A) |
| 143 | 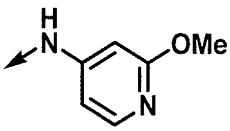 | H | H | LC-MS: [M+H] + / Rt (min) 322.2 / 0.613 (Method A) |
| 144 | 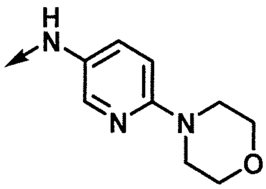 | H | H | LC-MS: [M+H] + / Rt (min) 322.2 / 0.613 (Method A) |
| 145 | 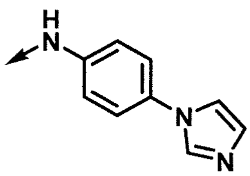 | H | H | LC-MS: [M+H] + / Rt (min) 322.2 / 0.613 (Method A) |
| 146 | 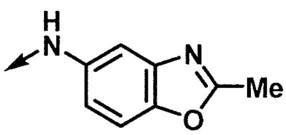 | H | H | LC-MS: [M+H] + / Rt (min) 322.2 / 0.613 (Method A) |

(continued)

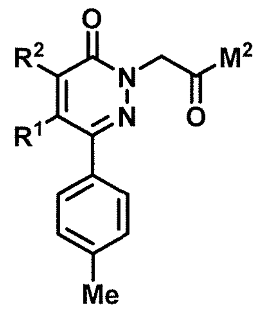

| Example | $M^2$ | $R^1$ | $R^2$ | Analytical data |
|---|---|---|---|---|
| 147 | 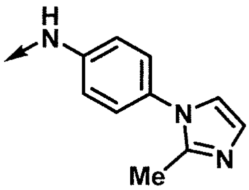 | H | H | LC-MS: [M+H] + / Rt (min) 322.2 / 0.613 (Method A) |
| 148 | 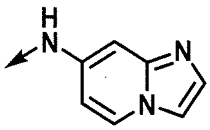 | H | H | LC-MS: [M+H] + / Rt (min) 322.2 / 0.613 (Method A) |
| 149 | 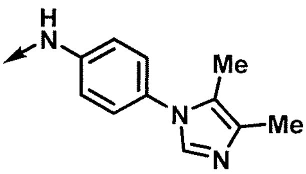 | H | H | LC-MS: [M+H] + / Rt (min) 322.2 / 0.613 (Method A) |
| 150 | 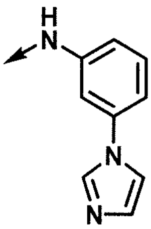 | H | H | LC-MS: [M+H] + / Rt (min) 322.2 / 0.613 (Method A) |
| 151 | 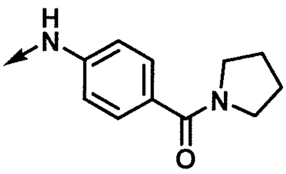 | H | H | LC-MS: [M+H] + / Rt (min) 322.2 / 0.613 (Method A) |
| 152 | 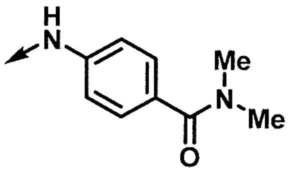 | H | H | LC-MS: [M+H] + / Rt (min) 322.2 / 0.613 (Method A) |

(continued)

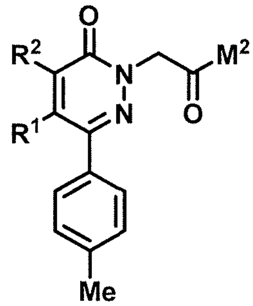

| Example | $M^2$ | $R^1$ | $R^2$ | Analytical data |
|---|---|---|---|---|
| 153 | 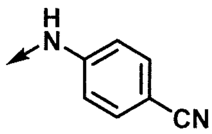 | Me | Me | LC-MS: [M+H] + / Rt (min) 322.2 / 0.613 (Method A) |
| 154 | 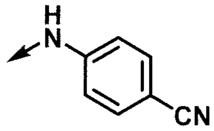 | Me | H | LC-MS: [M+H] + / Rt (min) 322.2 / 0.613 (Method A) |
| 155 | 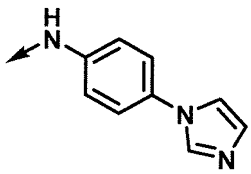 | - $(CH_2)_4$- | | LC-MS: [M+H] + / Rt (min) 322.2 / 0.613 (Method A) |
| 156 | 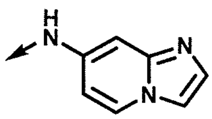 | Me | Me | LC-MS: [M+H] + / Rt (min) 388.2 / 1.584 (Method B) |
| 157 | 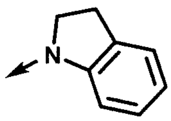 | H | H | LC-MS: $[M+H]^+$ / Rt (min) 346.2 / 0.966 (Method A) |
| 158 | 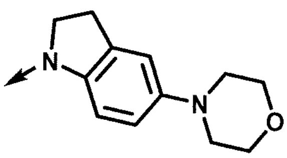 | H | H | $^1$H-NMR (400 MHz, DMSO-$d_6$) δ: 8.09 (d, J = 9.8 Hz, 1H), 7.83-7.78 (m, 3H), 7.31 (d, J = 7.9 Hz, 2H), 7.10 (d, J = 9.8 Hz, 1H), 6.91 (d, J = 1.8 Hz, 1H), 6.73 (dd, J = 8.5, 2.4 Hz, 1H), 5.13 (s, 2H), 4.22 (t, J = 8.2 Hz, 2H), 3.72 (t, J = 4.6 Hz, 4H), 3.18 (t, J = 8.2 Hz, 2H), 3.04 (t, J = 4.9 Hz, 4H), 2.36 (s, 3H). |
| 159 | 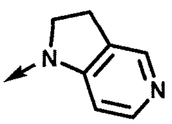 | H | H | $^1$H-NMR (400MHz, DMSO-$d_6$) δ: 8.43 (s, 1H), 8.32 (d, J = 4.9 Hz, 1H), 8.11 (d, J = 9.8 Hz, 1H), 7.81-7.78 (m, 3H), 7.31 (d, J = 8.5 Hz, 2H), 7.12 (d, J = 9.8 Hz, 1H), 5.21 (s, 2H), 4.32 (t, J = 8.5 Hz, 2H), 3.31-3.25 (m, 2H), 2.36 (s, 3H). |

Examples 160 - 192

According to the method of Example 1, 2, or 50 and common reaction conditions, the compounds of Examples

160 to 192 were obtained by using each corresponding material compound.

| Example | Chemical structure | Analytical data |
|---|---|---|
| 160 | 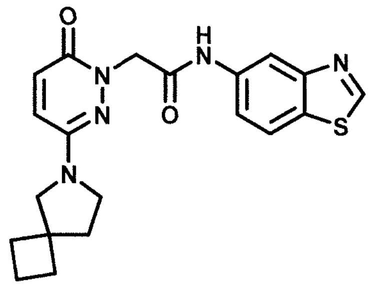 | $^1$H-NMR (400 MHz, $CDCl_3$) δ: 9.50 (br s, 1H), 8.98 (s, 1H), 8.38 (d, J = 2.3 Hz, 1H), 7.84 (d, J = 8.7 Hz, 1H), 7.62 (dd, J = 8.7, 2.3 Hz, 1H), 6.99 (d, J = 9.9 Hz, 1H), 6.95 (d, J = 9.9 Hz, 1H), 4.88 (s, 2H), 3.39 (t, J = 6.9 Hz, 2H), 3.35 (s, 2H), 2.07-1.91 (m, 8H). |
| 161 | 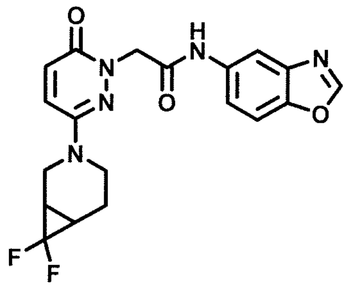 | $^1$H-NMR (400 MHz, $CDCl_3$) δ: 9.35 (s, 1H), 8.06-8.00 (m, 2H), 7.43-7.37 (m, 2 H), 7.13 (d, J = 10.0 Hz, 1H), 6.93 (d, J = 10.0 Hz, 1H), 4.85 (s, 2H), 3.81 (dd, J = 14.4, 7.0 Hz, 1H), 3.48-2.40 (m, 1H), 3.28-3.12 (m, 2H), 2.00-1.92 (m, 1H), 1.86-1.68 (m, 3H). |
| 162 | 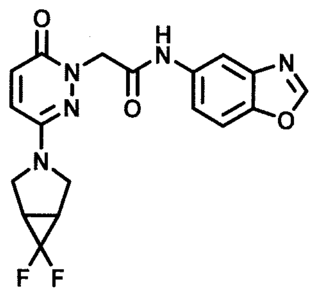 | $^1$H-NMR (400 MHz, $CDCl_3$) δ: 9.52 (s, 1H), 8.01 (d, J = 12.2 Hz, 2H), 7.42-7.35 (m, 2H), 6.92 (s, 2H), 4.85 (s, 2H), 3.75-3.58 (m, 4H), 2.37 (d, J = 11.6 Hz, 2H). |
| 163 | 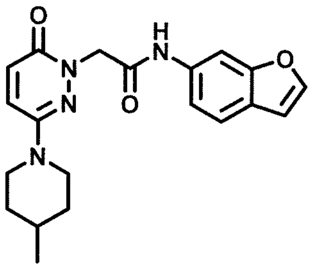 | $^1$H-NMR (400 MHz, $CDCl_3$) δ: 9.27 (br s, 1H), 8.04 (br s, 1H), 7.57 (d, J = 1.8 Hz, 1H), 7.46 (d, J = 8.5 Hz, 1H), 7.22 (d, J = 9.8 Hz, 1H), 7.14 (dd, J = 8.5, 1.8 Hz, 1H), 6.93 (d, J = 9.8 Hz, 1H), 6.70-6.69 (br m, 1H), 4.85 (s, 2H), 3.86-3.83 (m, 2H), 2.80-2.73 (m, 2H), 1.73-1.70 (m, 2H), 1.59-1.51 (m, 1H), 1.28-1.18 (m, 2H), 0.97 (d, J = 6.7 Hz, 3H). |
| 164 | 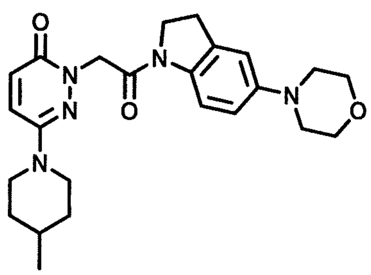 | LC-MS: $[M+H]^+$ / Rt (min) 438.4 / 0.858 (Method A) |

(continued)

| Example | Chemical structure | Analytical data |
|---|---|---|
| 165 | 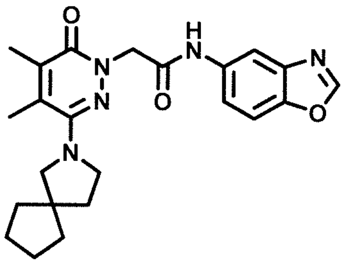 | $^1$H-NMR (400MHz, DMSO-$d_6$) δ: 10.36 (s, 1H), 8.70 (s, 1H), 8.11 (d, J = 2.4 Hz, 1H), 7.71 (d, J = 8.5 Hz, 1H), 7.50 (dd, J = 8.9, 2.1 Hz, 1H), 4.72 (s, 2H), 3.32-3.28 (m, 2H), 3.06 (s, 2H), 2.16 (s, 3H), 2.04 (s, 3H), 1.73 (t, J = 7.0 Hz, 2H), 1.63-1.46 (m, 8H). |
| 166 | 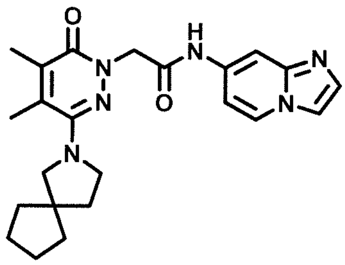 | $^1$H-NMR (400MHz, DMSO-$d_6$) δ: 10.44 (s, 1H), 8.44 (d, J = 7.3 Hz, 1H), 7.90 (s, 1H), 7.81 (s, 1H), 7.44 (d, J = 1.2 Hz, 1H), 6.94 (dd, J = 7.3, 1.8 Hz, 1H), 4.73 (s, 2H), 3.32-3.29 (m, 2H), 3.07 (s, 2H), 2.16 (s, 3H), 2.04 (s, 3H), 1.73 (t, J = 7.0 Hz, 2H), 1.63-1.46 (m, 8H). |
| 167 | 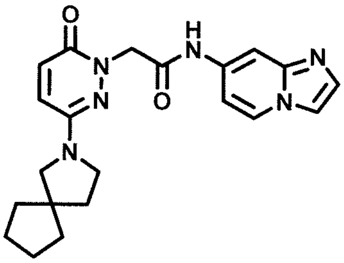 | $^1$H-NMR (400 MHz, $CDCl_3$) δ: 9.67 (s, 1H), 7.99 (d, J = 7.3 Hz, 1H), 7.86 (d, J = 1.8 Hz, 1H), 7.55 (s, 1H), 7.47 (s, 1H), 7.04 (dd, J = 7.3, 1.8 Hz, 1H), 7.00 (d, J = 10.1 Hz, 1H), 6.95 (d, J = 10.1 Hz, 1H), 4.85 (s, 2H), 3.44 (t, J = 6.9 Hz, 2H), 3.23 (s, 2H), 1.87 (t, J = 6.9 Hz, 2H), 1.71-1.56 (m, 8H). |
| 168 | 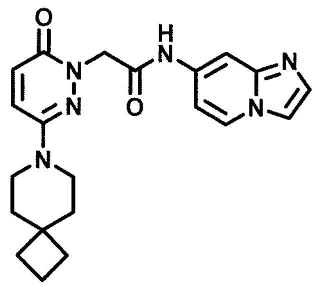 | $^1$H-NMR (400 MHz, $CDCl_3$) δ: 9.54 (br s, 1H), 7.99 (d, J = 7.3 Hz, 1H), 7.86 (d, J = 1.8 Hz, 1H), 7.56 (s, 1H), 7.47 (s, 1H), 7.24 (d, J = 10.1 Hz, 1H), 7.02 (dd, J = 7.3, 1.8 Hz, 1H), 6.93 (d, J = 10.1 Hz, 1H), 4.85 (s, 2H), 3.25-3.23 (m, 4H), 1.94-1.88 (m, 2H), 1.81-1.78 (m, 4H), 1.66-1.64 (m, 4H). |
| 169 | 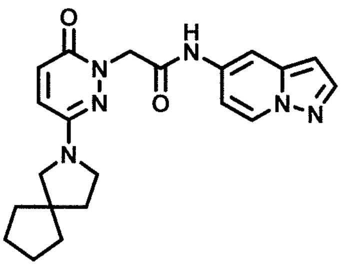 | $^1$H-NMR (400 MHz, $CDCl_3$) δ: 9.69 (br s, 1H), 8.32 (d, J = 7.5 Hz, 1H), 7.99 (d, J = 2.4 Hz, 1H), 7.87 (d, J = 2.3 Hz, 1H), 7.00 (d, J = 10.1 Hz, 1H), 6.95 (d, J = 10.1 Hz, 1H), 6.72 (dd, J = 7.5, 2.3 Hz, 1H), 6.39 (d, J = 2.4 Hz, 1H), 4.84 (s, 2H), 3.44 (t, J = 7.0 Hz, 2H), 3.22 (s, 2H), 1.87 (t, J = 7.0 Hz, 2H), 1.71-1.66 (m, 4H), 1.62-1.56 (m, 4H). |

(continued)

| Example | Chemical structure | Analytical data |
|---|---|---|
| 170 | 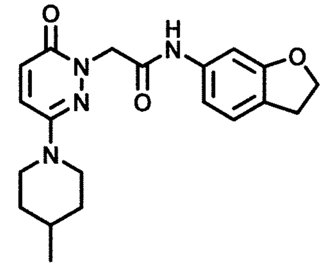 | $^1$H-NMR (400 MHz, $CDCl_3$) δ: 8.99 (br s, 1H), 7.20 (d, J = 9.8 Hz, 1H), 7.10-7.10 (br m, 1H), 7.07 (d, J = 7.9 Hz, 1H), 6.92-6.89 (m, 2H), 4.80 (s, 2H), 4.55 (t, J = 8.7 Hz, 2H), 3.84-3.81 (m, 2H), 3.14 (t, J = 8.7 Hz, 2H), 2.79-2.72 (m, 2H), 1.72-1.69 (m, 2H), 1.58-1.53 (m, 1H), 1.28-1.17 (m, 2H), 0.96 (d, J = 6.7 Hz, 3H). |
| 171 | 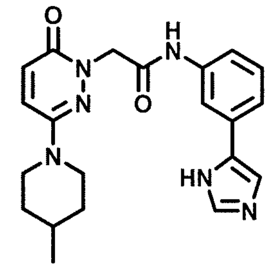 | $^1$H-NMR (400 MHz, $CDCl_3$) δ: 7.67 (s, 1H), 7.63 (br s, 1H), 7.46-7.44 (br m, 1H), 7.37 (br s, 1H), 7.28 (s, 1H), 7.24 (br s, 1H), 7.22 (d, J = 9.8 Hz, 1H), 6.91 (d, J = 9.8 Hz, 1H), 4.88 (s, 2H), 3.85-3.83 (br m, 2H), 2.81-2.74 (m, 2H), 1.73-1.70 (m, 2H), 1.66-1.49 (m, 1H), 1.29-1.19 (m, 2H), 0.97 (d, J = 6.7 Hz, 3H). |
| 172 | 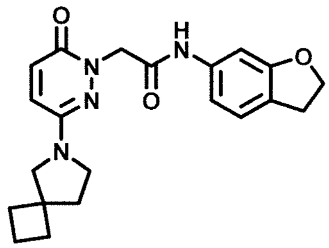 | $^1$H-NMR (400 MHz, $CDCl_3$) δ: 9.08 (br s, 1H), 7.10 (d, J = 1.2 Hz, 1H), 7.06 (d, J = 7.9 Hz, 1H), 6.97-6.90 (m, 3H), 4.80 (s, 2H), 4.55 (t, J = 8.7 Hz, 2H), 3.37 (t, J = 6.7 Hz, 2H), 3.34 (s, 2H), 3.13 (t, J = 8.7 Hz, 2H), 2.04-1.91 (m, 8H). |
| 173 | 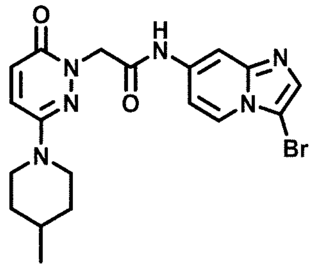 | $^1$H-NMR (400 MHz, $CDCl_3$) δ: 9.74 (br s, 1H), 7.95-7.93 (m, 2H), 7.51 (s, 1H), 7.25 (d, J = 10.1 Hz, 1H), 7.08 (dd, J = 7.9, 1.8 Hz, 1H), 6.94 (d, J = 10.1 Hz, 1H), 4.86 (s, 2H), 3.87-3.83 (m, 2H), 2.81-2.74 (m, 2H), 1.73-1.70 (m, 2H), 1.62-1.54 (m, 1H), 1.28-1.18 (m, 2H), 0.97 (d, J = 6.7 Hz, 3H). |
| 174 | 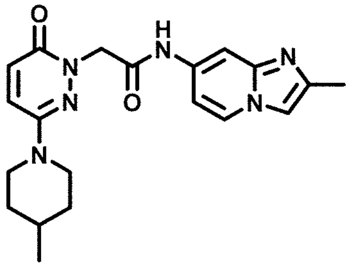 | $^1$H-NMR (400 MHz, $CDCl_3$) δ: 9.46 (br s, 1H), 7.89 (d, J = 7.3 Hz, 1H), 7.72 (d, J = 2.4 Hz, 1H), 7.23 (d, J = 10.4 Hz, 1H), 7.21 (br s, 1H), 6.99-6.96 (m, 1H), 6.93 (d, J = 10.4 Hz, 1H), 4.84 (s, 2H), 3.86-3.83 (m, 2H), 2.81-2.74 (m, 2H), 2.41 (s, 3H), 1.73-1.70 (m, 2H), 1.63-1.53 (m, 1H), 1.28-1.18 (m, 2H), 0.97 (d, J = 6.7 Hz, 3H). |

(continued)

| Example | Chemical structure | Analytical data |
|---|---|---|
| 175 | 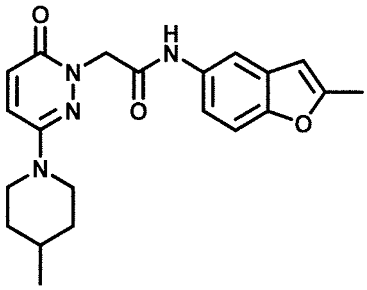 | $^1$H-NMR (400 MHz, $CDCl_3$) δ: 9.04 (br s, 1H), 7.78 (d, J = 2.1 Hz, 1H), 7.28 (d, J = 8.5 Hz, 1H), 7.21 (d, J = 9.8 Hz, 1H), 7.18 (dd, J = 8.5, 2.1 Hz, 1H), 6.92 (d, J = 9.8 Hz, 1H), 6.31 (s, 1H), 4.84 (s, 2H), 3.85 - 3.82 (m, 2H), 2.80-2.73 (m, 2H), 2.42 (s, 3H), 1.73-1.69 (m, 2H), 1.61-1.53 (m, 1H), 1.28-1.18 (m, 2H), 0.97 (d, J = 6.7 Hz, 3H). |
| 176 | 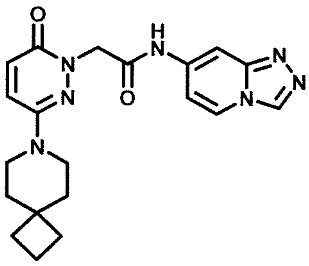 | $^1$H-NMR (400 MHz, $CD_3OD$) δ: 9.05 (s, 1H), 8.43 (d, J = 7.3 Hz, 1H), 8.20 (d, J = 1.8 Hz, 1H), 7.54 (d, J = 9.8 Hz, 1H), 7.10 (dd, J = 7.3, 1.8 Hz, 1H), 6.90 (d, J = 9.8 Hz, 1H), 4.87 (s, 2H), 3.29-3.26 (m, 4H), 1.97-1.89 (m, 2H), 1.84-1.79 (m, 4H), 1.67-1.65 (m, 4H). |
| 177 | 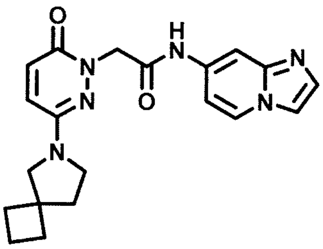 | $^1$H-NMR (400 MHz, $CDCl_3$) δ: 9.62 (br s, 1H), 7.99 (d, J = 7.3 Hz, 1H), 7.85-7.84 (br m, 1H), 7.55 (d, J = 1.5 Hz, 1H), 7.47 (s, 1H), 7.03 (dd, J = 7.3, 1.5 Hz, 1H), 6.99 (d, J = 9.8 Hz, 1H), 6.94 (d, J = 9.8 Hz, 1H), 4.85 (s, 2H), 3.39 (t, J = 6.7 Hz, 2H), 3.35 (s, 2H), 2.06-1.91 (m, 8H). |
| 178 | 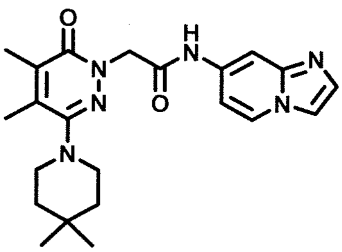 | LC-MS: [M+H] + / Rt (min) 409.3 / 1.71 (Method B) |
| 179 | 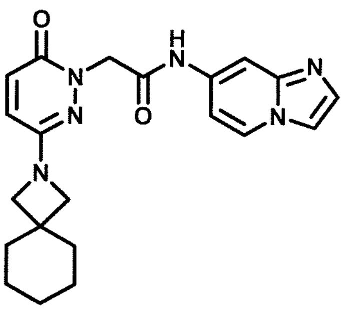 | LC-MS: [M+H] + / Rt (min) 393.4 / 0.628 (Method A) |

(continued)

| Example | Chemical structure | Analytical data |
|---|---|---|
| 180 | 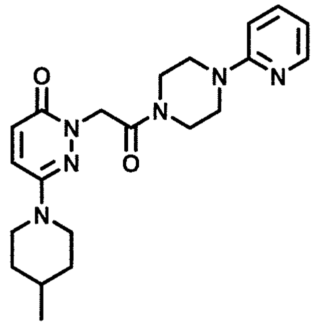 | LC-MS: [M+H] + / Rt (min) 397.1 / 0.609 (Method A) |
| 181 | 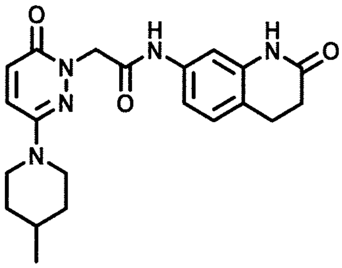 | $^1$H-NMR (400 MHz, $CDCl_3$) δ: 9.26 (br s, 1H), 7.50 (s, 1H), 7.25 (d, J = 2.0 Hz, 1H), 7.22 (d, J = 9.8 Hz, 1H), 7.05 (d, J = 8.1 Hz, 1H), 6.93 (d, J = 9.8 Hz, 1H), 6.92 (dd, J = 8.1, 2.0 Hz, 1H), 4.82 (s, 2H), 3.85-3.82 (m, 2H), 2.91-2.89 (m, 2H), 2.76 (td, J = 12.8, 2.4 Hz, 2H), 2.61-2.58 (m, 2H), 1.73-1.69 (m, 2H), 1.60-1.53 (m, 1H), 1.27-1.17 (m, 2H), 0.97 (d, J = 6.1 Hz, 3H). |
| 182 | 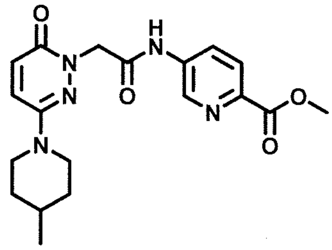 | LC-MS: [M+H] + / Rt (min) 386.2 / 1.695 (Method B) |
| 183 | 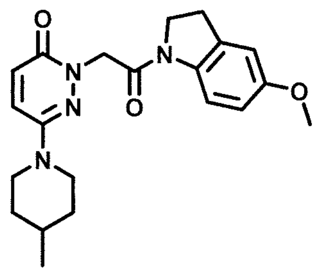 | $^1$H-NMR (400 MHz, $CDCl_3$) δ: 8.11 (d, J = 8.5 Hz, 1H), 7.18 (d, J = 9.8 Hz, 1H), 6.87 (d, J = 9.8 Hz, 1H), 6.75 (br s, 1H), 6.70-6.68 (br m, 1H), 4.88 (s, 2H), 4.17-4.13 (m, 2H), 3.77 (s, 3H), 3.77-3.75 (m, 2H), 3.25-3.20 (m, 2H), 2.75-2.68 (m, 2H), 1.70-1.67 (m, 2H), 1.56-1.49 (m, 1H), 1.28-1.18 (m, 2H), 0.95 (d, J = 6.7 Hz, 3H). |
| 184 | 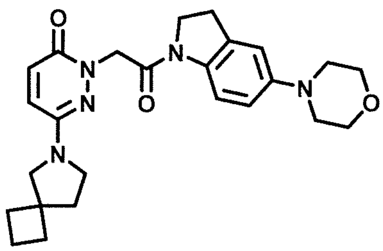 | $^1$H-NMR (400 MHz, $CDCl_3$) δ: 8.10 (d, J = 8.5 Hz, 1H), 6.94 (d, J = 9.8 Hz, 1H), 6.88 (d, J = 9.8 Hz, 1H), 6.78 (br s, 1H), 6.74-6.71 (br m, 1H), 4.88 (s, 2H), 4.16-4.11 (m, 2H), 3.86-3.83 (m, 4H), 3.37-3.33 (m, 2H), 3.30 (s, 2H), 3.24-3.20 (m, 2H), 3.10-3.08 (m, 4H), 2.04-1.86 (m, 8H). |

(continued)

| Example | Chemical structure | Analytical data |
|---|---|---|
| 185 | 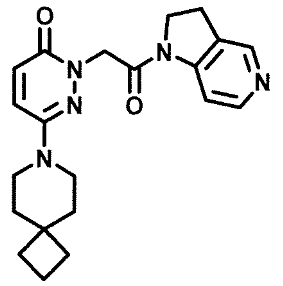 | $^1$H-NMR (400 MHz, $CDCl_3$) δ: 8.41 (s, 1H), 8.38 (d, J = 5.5 Hz, 1H), 8.00 (br s, 1H), 7.21-7.19 (m, 1H), 6.88 (d, J = 9.8 Hz, 1H), 4.89 (br s, 2H), 4.21 (t, J = 8.5 Hz, 2H), 3.33-3.29 (m, 2H), 3.19-3.16 (m, 4H), 1.92-1.87 (m, 2H), 1.80-1.76 (m, 4H), 1.65-1.62 (m, 4H). |
| 186 | 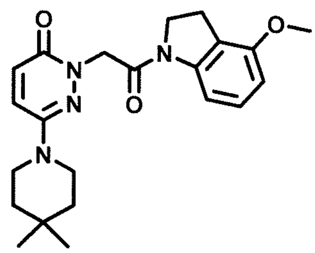 | $^1$H-NMR (400 MHz, $CDCl_3$) δ: 7.82 (d, J = 8.5 Hz, 1H), 7.19-7.12 (m, 2H), 6.88 (d, J = 9.8 Hz, 1H), 6.58 (d, J = 8.5 Hz, 1H), 4.88 (s, 2H), 4.17 (t, J = 8.5 Hz, 2H), 3.84 (s, 3H), 3.25-3.22 (m, 4H), 3.17 (t, J = 8.5 Hz, 2H), 1.44-1.42 (m, 4H), 0.96 (s, 6H). |
| 187 | 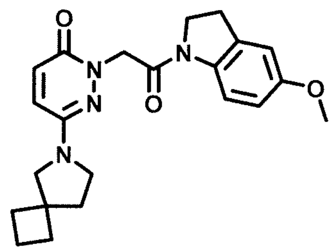 | $^1$H-NMR (400 mHz, $CDCl_3$) δ: 8.12 (d, J = 8.9 Hz, 1H), 6.94 (d, J = 10.1 Hz, 1H), 6.89 (d, J = 10.1 Hz, 1H), 6.75-6.75 (br m, 1H), 6.69 (dd, J = 8.9, 2.4 Hz, 1H), 4.88 (s, 2H), 4.15 (t, J = 8.5 Hz, 2H), 3.77 (s, 3H), 3.35 (t, J = 6.7 Hz, 2H), 3.31 (s, 2H), 3.23 (t, J = 8.5 Hz, 2H), 2.05-1.88 (m, 8H). |
| 188 | 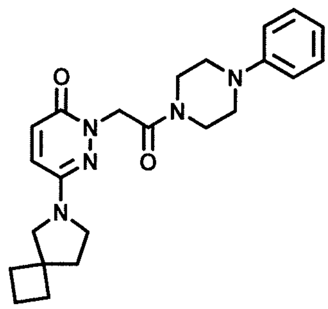 | $^1$H-NMR (400 MHz, $CDCl_3$) δ: 7.31-7.28 (m, 2H), 6.94-6.89 (m, 4H), 6.86 (d, J = 10.4 Hz, 1H), 4.88 (s, 2H), 3.82-3.79 (m, 2H), 3.67-3.65 (m, 2H), 3.35 (t, J = 6.7 Hz, 2H), 3.30 (s, 2H), 3.23-3.18 (m, 4H), 2.04-1.88 (m, 8H). |
| 189 | 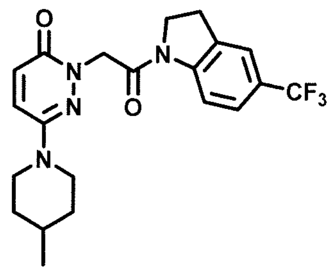 | $^1$H-NMR (400 MHz, $CDCl_3$) δ: 8.28-8.26 (br m, 1H), 7.45-7.43 (m, 2H), 7.20 (d, J = 10.4 Hz, 1H), 6.88 (d, J = 10.4 Hz, 1H), 4.91 (s, 2H), 4.25-4.21 (m, 2H), 3.78-3.75 (m, 2H), 3.33-3.29 (m, 2H), 2.76-2.69 (m, 2H), 1.71-1.67 (m, 2H), 1.57-1.48 (m, 1H), 1.28-1.18 (m, 2H), 0.96 (d, J = 6.7 Hz, 3H). |

(continued)

| Example | Chemical structure | Analytical data |
|---|---|---|
| 190 | 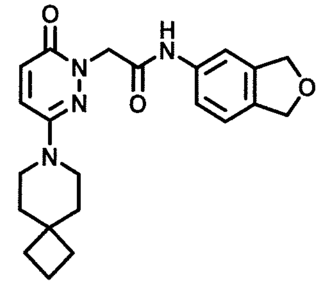 | $^1$H-NMR (400 MHz, $CDCl_3$) δ: 9.23 (br s, 1H), 7.57 (s, 1H), 7.29-7.26 (m, 1H), 7.22 (d, J = 9.8 Hz, 1H), 7.13 (d, J = 7.9 Hz, 1H), 6.92 (d, J = 9.8 Hz, 1H), 5.05 (s, 4H), 4.82 (s, 2H), 3.24-3.21 (m, 4H), 1.96-1.88 (m, 2H), 1.81-1.77 (m, 4H), 1.66-1.63 (m, 4H). |
| 191 | 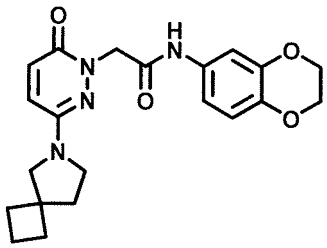 | $^1$H-NMR (400 MHz, $CDCl_3$) δ: 8.96 (s, 1H), 7.18 (d, J = 2.4 Hz, 1H), 7.00-6.88 (m, 3H), 6.76 (d, J = 8.5 Hz, 1H), 4.79 (s, 2H), 4.23-4.20 (m, 4H), 3.37 (t, J = 7.0 Hz, 2H), 3.33 (s, 2H), 2.07-1.90 (m, 8H). |
| 192 | 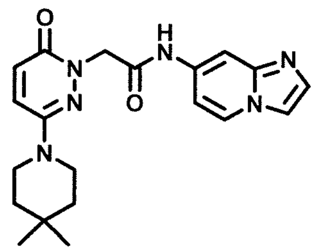 | $^1$H-NMR (400 MHz, $CDCl_3$) δ: 9.62 (br s, 1H), 8.02 (d, J = 7.3 Hz, 1H), 7.90 (s, 1H), 7.54 (s, 1H), 7.51 (s, 1H), 7.27-7.24 (m, 1H), 7.04 (d, J = 6.1 Hz, 1H), 6.94 (d, J = 10.4 Hz, 1H), 4.87 (s, 2H), 3.32-3.29 (m, 4H), 1.46-1.43 (m, 4H), 0.98 (s, 6H). |

Examples 193 - 238

According to the method of Example 1, 2, or 50 and common reaction conditions, the compounds of Examples 193 to 238 were obtained by using each corresponding material compound.

| Example | Chemical structure | Analytical data |
|---|---|---|
| 193 | 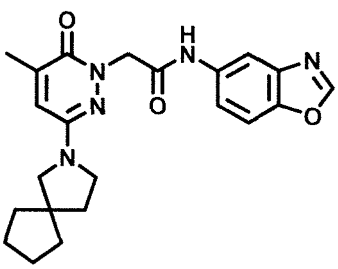 | $^1$H-NMR (400 MHz, DMSO-$d_6$) δ: 10.33 (s, 1H), 8.70 (s, 1H), 8.12 (d, J = 1.8 Hz, 1H), 7.72 (d, J = 8.5 Hz, 1H), 7.51 (dd, J = 8.8, 2.1 Hz, 1H), 7.16 (d, J = 1.2 Hz, 1H), 4.71 (s, 2H), 3.35 (t, J = 6.7 Hz, 2H), 3.16 (s, 2H), 2.08 (s, 3H), 1.80 (t, J = 7.0 Hz, 2H), 1.65-1.52 (m, 8H). |

(continued)

| Example | Chemical structure | Analytical data |
|---|---|---|
| 194 | 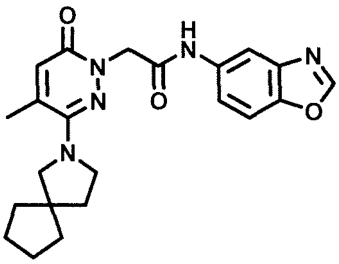 | $^1$H-NMR (400 MHz, DMSO-$d_6$) δ: 10.34 (s, 1H), 8.70 (s, 1H), 8.10 (d, J = 1.8 Hz, 1H), 7.71 (d, J = 8.6 Hz, 1H), 7.50 (dd, J = 8.6, 1.8 Hz, 1H), 6.71 (d, J = 1.2 Hz, 1H), 4.70 (s, 2H), 3.39 (t, J = 6.7 Hz, 2H), 3.14 (s, 2H), 2.25 (s, 3H), 1.74 (t, J = 7.0 Hz, 2H), 1.63-1.46 (m, 8H). |
| 195 | 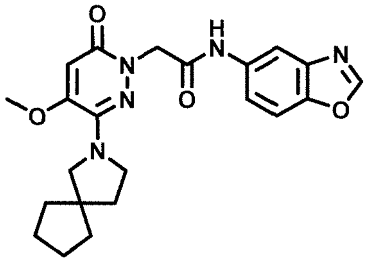 | $^1$H-NMR (400 MHz, DMSO-$d_6$) δ: 10.31 (s, 1H), 8.70 (s, 1H), 8.11 (d, J = 1.8 Hz, 1H), 7.71 (d, J = 8.7 Hz, 1H), 7.51 (dd, J = 8.7, 1.8 Hz, 1H), 6.20 (s, 1H), 4.66 (s, 2H), 3.83 (s, 3H), 3.45 (t, J = 7.1 Hz, 2H), 3.20 (s, 2H), 1.71 (t, J = 7.1 Hz, 2H), 1.63-1.48 (m, 8H). |
| 196 | 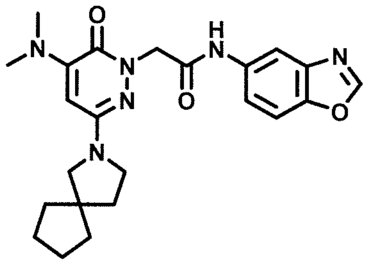 | $^1$H-NMR (400 MHz, DMSO-$d_6$) δ: 10.29 (s, 1H), 8.70 (s, 1H), 8.12 (d, J = 1.8 Hz, 1H), 7.71 (d, J = 8.5 Hz, 1H), 7.51 (dd, J = 8.8, 2.1 Hz, 1H), 5.83 (s, 1H), 4.66 (s, 2H), 3.35-3.31 (m, 2H), 3.14 (s, 2H), 3.04 (s, 6H), 1.77 (t, J = 6.7 Hz, 2H), 1.65-1.49 (m, 8H). |
| 197 | 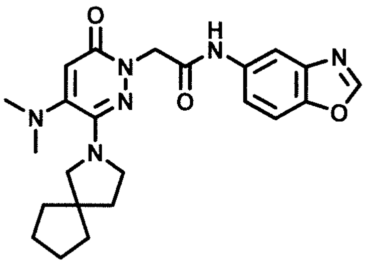 | $^1$H-NMR (400 MHz, DMSO-$d_6$) δ: 10.35 (s, 1H), 8.70 (s, 1H), 8.12 (d, J = 1.8 Hz, 1H), 7.71 (d, J = 9.1 Hz, 1H), 7.51 (dd, J = 8.8, 2.1 Hz, 1H), 5.98 (s, 1H), 4.67 (s, 2H), 3.24 (t, J = 7.3 Hz, 2H), 3.04 (s, 2H), 2.79 (s, 6H), 1.73 (t, J = 7.0 Hz, 2H), 1.63-1.47 (m, 8H). |
| 198 | 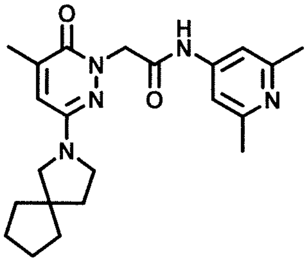 | $^1$H-NMR (400 MHz, DMSO-$d_6$) δ: 10.36 (s, 1H), 7.21 (s, 2H), 7.16-7.14 (m, 1H), 4.68 (s, 2H), 3.35-3.31 (m, 2H), 3.14 (s, 2H), 2.35 (s, 6H), 2.06 (s, 3H), 1.79 (t, J = 6.7 Hz, 2H), 1.66-1.49 (m, 8H). |

(continued)

| Example | Chemical structure | Analytical data |
|---|---|---|
| 199 | 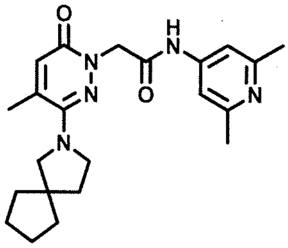 | $^{1}$H-NMR (400 MHz, DMSO-$d_6$) δ: 10.38 (s, 1H), 7.20 (s, 2H), 6.70 (d, J = 1.2 Hz, 1H), 4.68 (s, 2H), 3.38 (t, J = 7.0 Hz, 2H), 3.13 (s, 2H), 2.35 (s, 6H), 2.25 (s, 3H), 1.74 (t, J = 7.0 Hz, 2H), 1.63-1.47 (m, 8H). |
| 200 | 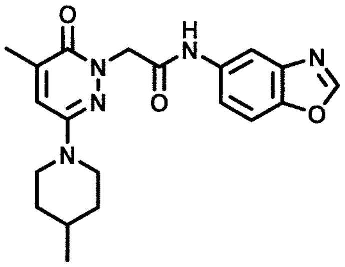 | $^{1}$H-NMR (400 MHz, DMSO-$d_6$) δ: 10.35 (s, 1H), 8.70 (s, 1H), 8.11 (d, J = 1.8 Hz, 1H), 7.72 (d, J = 8.6 Hz, 1H), 7.53-7.47 (m, 2H), 4.73 (s, 2H), 3.81 (d, J = 13.4 Hz, 2H), 2.68-2.61 (m, 2H), 2.07 (s, 3H), 1.64 (d, J = 12.2 Hz, 2H), 1.57-1.45 (m, 1H), 1.19-1.09 (m, 2H), 0.91 (d, J = 6.2 Hz, 3H). |
| 201 | 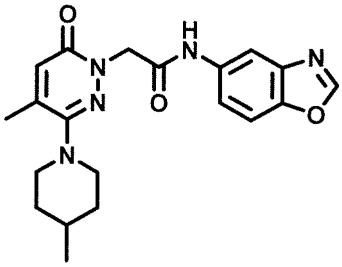 | $^{1}$H-NMR (400 MHz, DMSO-$d_6$) δ: 10.39 (s, 1H), 8.71 (s, 1H), 8.11 (d, J = 1.8 Hz, 1H), 7.72 (d, J = 9.2 Hz, 1H), 7.51 (dd, J = 8.9, 2.1 Hz, 1H), 6.78 (d, J = 1.2 Hz, 1H), 4.75 (s, 2H), 3.23 (d, J = 12.8 Hz, 2H), 2.63-2.52 (m, 2H), 2.20 (s, 3H), 1.67 (d, J = 10.4 Hz, 2H), 1.55-1.43 (m, 1H), 1.29-1.19 (m, 2H), 0.94 (d, J = 6.7 Hz, 3H). |
| 202 | 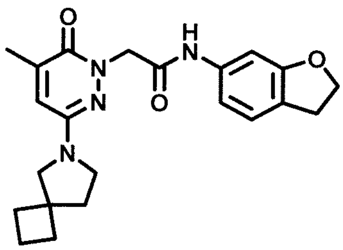 | $^{1}$H-NMR (400 MHz, $CDCl_3$) δ: 9.11 (br s, 1H), 7.08-7.05 (br m, 2H), 6.93 (dd, J = 8.2, 2.0 Hz, 1H), 6.81 (d, J = 2.0 Hz, 1H), 4.80 (s, 2H), 4.55 (t, J = 8.7 Hz, 2H), 3.36 (t, J = 6.7 Hz, 2H), 3.32 (s, 2H), 3.13 (t, J = 8.7 Hz, 2H), 2.23 (d, J = 1.2 Hz, 3H), 2.06-1.90 (m, 8H). |
| 203 | 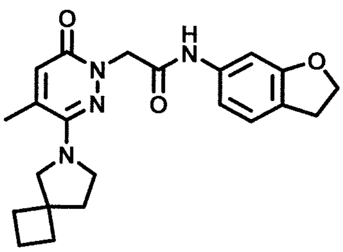 | $^{1}$H-NMR (400 MHz, $CDCl_3$) δ: 9.17 (br s, 1H), 7.11 (br s, 1H), 7.07-7.05 (br m, 1H), 6.90 (dd, J = 7.9, 1.8 Hz, 1H), 6.71 (br s, 1H), 4.78 (s, 2H), 4.55 (t, J = 8.7 Hz, 2H), 3.38 (t, J = 6.7 Hz, 2H), 3.33 (s, 2H), 3.13 (t, J = 8.7 Hz, 2H), 2.28 (d, J = 1.2 Hz, 3H), 2.06-1.86 (m, 8H) . |

(continued)

| Example | Chemical structure | Analytical data |
|---|---|---|
| 204 | 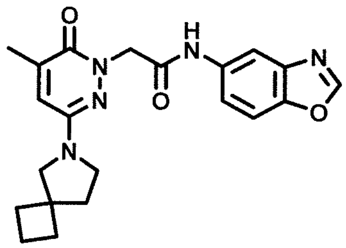 | $^1$H-NMR (400 MHz, DMSO-$d_6$) δ: 10.34 (s, 1H), 8.71 (s, 1H), 8.12 (d, J = 2.4 Hz, 1H), 7.72 (d, J = 8.5 Hz, 1H), 7.52 (dd, J = 8.9, 2.1 Hz, 1H), 7.15 (d, J = 1.2 Hz, 1H), 4.72 (s, 2H), 3.31-3.28 (m, 4H), 2.08 (d, J = 1.2 Hz, 3H), 2.03-1.80 (m, 8H). |
| 205 | 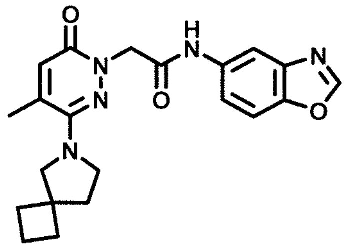 | $^1$H-NMR (400 MHz, DMSO-$d_6$) δ: 10.35 (s, 1H), 8.71 (s, 1H), 8.11 (d, J = 1.8 Hz, 1H), 7.72 (d, J = 8.6 Hz, 1H), 7.51 (dd, J = 8.9, 2.1 Hz, 1H), 6.71 (s, 1H), 4.70 (s, 2H), 3.35-3.28 (m, 4H), 2.25 (s, 3H), 2.03-1.76 (m, 8H). |
| 206 | 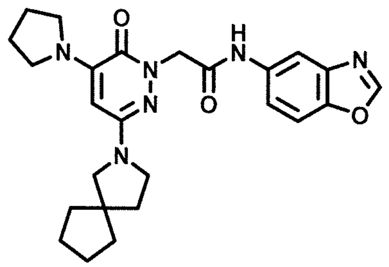 | $^1$H-NMR (400 MHz, DMSO-$d_6$) δ: 10.33 (s, 1H), 8.71 (s, 1H), 8.12 (d, J = 1.8 Hz, 1H), 7.72 (d, J = 8.5 Hz, 1H), 7.51 (dd, J = 8.5, 1.8 Hz, 1H), 5.78 (s, 1H), 4.67 (s, 2H), 3.29-3.25 (m, 4H), 3.10 (t, J = 7.3 Hz, 2H), 2.95 (s, 2H), 1.91-1.87 (m, 4H), 1.75 (t, J = 7.3 Hz, 2H), 1.61-1.51 (m, 8H). |
| 207 | 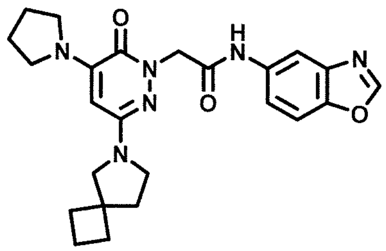 | $^1$H-NMR (400 MHz, DMSO-$d_6$) δ: 10.28 (s, 1H), 8.70 (s, 1H), 8.13 (d, J = 1.8 Hz, 1H), 7.71 (d, J = 8.6 Hz, 1H), 7.51 (dd, J = 8.9, 2.1 Hz, 1H), 5.52 (s, 1H), 4.63 (s, 2H), 3.57 (s, 4H), 3.29-3.23 (m, 4H), 2.01-1.79 (m, 12H). |
| 208 | 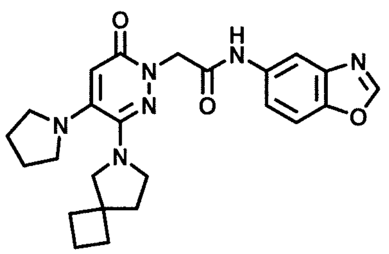 | $^1$H-NMR (400 MHz, DMSO-$d_6$) δ: 10.33 (s, 1H), 8.70 (s, 1H), 8.12 (d, J = 1.8 Hz, 1H), 7.71 (d, J = 8.6 Hz, 1H), 7.51 (dd, J = 8.9, 2.1 Hz, 1H), 5.78 (s, 1H), 4.66 (s, 2H), 3.26-3.22 (m, 4H), 3.08 (s, 2H), 3.04 (t, J = 7.2 Hz, 2H), 2.03-1.76 (m, 12H). |

(continued)

| Example | Chemical structure | Analytical data |
|---|---|---|
| 209 | 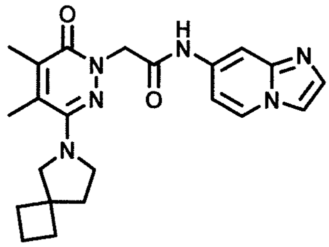 | $^1$H-NMR (400 MHz, DMSO-$d_6$) δ: 10.45 (s, 1H), 8.45 (d, J = 7.3 Hz, 1H), 7.91 (d, J = 1.2 Hz, 1H), 7.82 (s, 1H), 7.45 (d, J = 1.2 Hz, 1H), 6.95 (dd, J = 7.3, 1.8 Hz, 1H), 4.74 (s, 2H), 3.26-3.21 (m, 4H), 2.16 (s, 3H), 2.05 (s, 3H), 2.01-1.78 (m, 8H). |
| 210 | 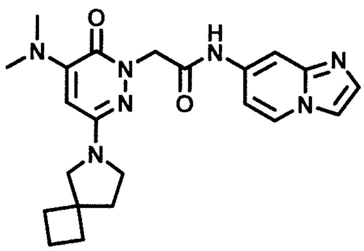 | LC-MS: [M+H] $^+$ / Rt (min) 422.4 / 0.695 (Method A) |
| 211 | 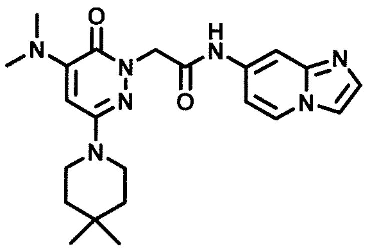 | $^1$H-NMR (400 MHz, $CDCl_3$) δ: 9.42 (br s, 1H), 7.99 (d, J = 7.3 Hz, 1H), 7.71 (br m, 1H), 7.55 (br m, 1H), 7.46 (s, 1H), 7.16 (dd, J = 7.3, 1.8 Hz, 1H), 5.99 (s, 1H), 4.83 (s, 2H), 3.27-3.24 (m, 4H), 3.13 (s, 6H), 1.46-1.43 (m, 4H), 0.97 (s, 6H). |
| 212 | 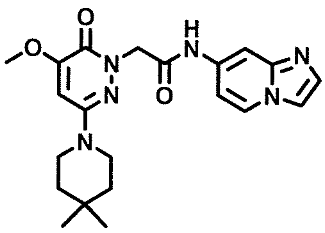 | LC-MS: [M+H] $^+$ / Rt (min) 411.4 / 0.618 (Method A) |
| 213 | 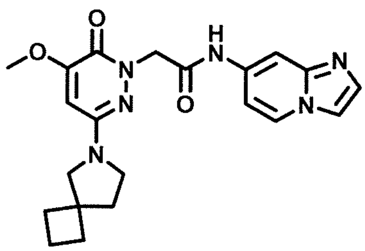 | LC-MS: [M+H] $^+$ / Rt (min) 409.4 / 0.608 (Method A) |

(continued)

| Example | Chemical structure | Analytical data |
|---|---|---|
| 214 | 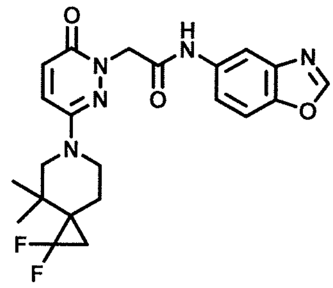 | $^1$H-NMR (400 MHz, $CDCl_3$) δ: 9.37 (s, 1H), 8.04 (d, J = 9.2 Hz, 2H), 7.42 (s, 2H), 7.20 (d, J = 10.0 Hz, 1H), 6.94 (d, J = 10.0 Hz, 1H), 4.85 (s, 2H), 3.70-3.65 (m, 1H), 3.21 (d, J = 12.6 Hz, 1H), 3.00-2.95 (m, 1H), 2.84 (d, J = 12.6 Hz, 1H), 2.08 - 2.04 (m, 1H), 1.48-1.32 (m, 2H), 1.11 (s, 3H), 0.96-0.84 (m, 1H), 0.92 (s, 3H). |
| 215 | 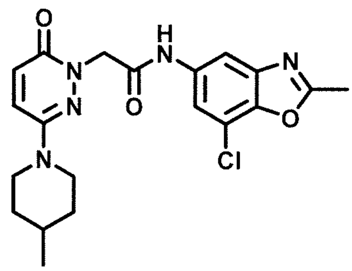 | $^1$H-NMR (400 MHz, $CDCl_3$) δ: 9.41 (br s, 1H), 7.76 (d, J = 1.8 Hz, 1H), 7.53 (d, J = 1.8 Hz, 1H), 7.23 (d, J = 10.1 Hz, 1H), 6.94 (d, J = 10.1 Hz, 1H), 4.84 (s, 2H), 3.84 (d, J = 12.8 Hz, 2H), 2.81-2.74 (m, 2H), 2.65 (s, 3H), 1.74-1.70 (m, 2H), 1.59-1.53 (m, 1H), 1.30-1.18 (m, 2H), 0.97 (d, J = 6.7 Hz, 3H). |
| 216 | 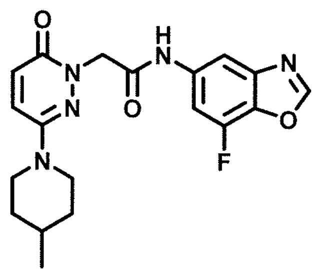 | $^1$H-NMR (400 MHz, $CDCl_3$) δ: 9.53 (br s, 1H), 8.07 (s, 1H), 7.75 (d, J = 1.4 Hz, 1H), 7.53-7.50 (m, 1H), 7.24 (d, J = 10.4 Hz, 1H), 6.94 (d, J = 10.4 Hz, 1H), 4.85 (s, 2H), 3.87-3.83 (m, 2H), 2.80-2.74 (m, 2H), 1.74-1.70 (m, 2H), 1.61-1.50 (m, 1H), 1.31-1.18 (m, 2H), 0.97 (d, J = 6.7 Hz, 3H). |
| 217 | 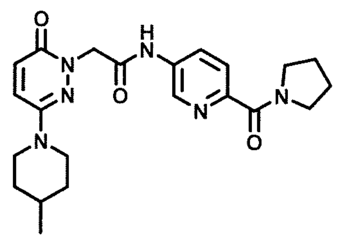 | $^1$H-NMR (400 MHz, $CDCl_3$) δ: 9.65 (s, 1H), 8.64 (d, J = 2.7 Hz, 1H), 8.10 (dd, J = 8.5, 2.7 Hz, 1H), 7.84 (d, J = 8.5 Hz, 1H), 7.25 (d, J = 10.4 Hz, 1H), 6.95 (d, J = 10.4 Hz, 1H), 4.86 (s, 2H), 3.87-3.83 (m, 2H), 3.78-3.74 (m, 2H), 3.68-3.65 (m, 2H), 2.81-2.74 (m, 2H), 1.93-1.89 (m, 4H), 1.74-1.70 (m, 2H), 1.58-1.53 (m, 1H), 1.28-1.18 (m, 2H), 0.97 (d, J = 6.7 Hz, 3H). |
| 218 | 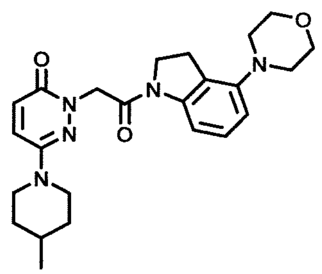 | $^1$H-NMR (400 MHz, $CDCl_3$) δ: 7.94-7.92 (br m, 1H), 7.19-7.14 (m, 2H), 6.87 (d, J = 9.8 Hz, 1H), 6.68 (d, J = 7.9 Hz, 1H), 4.89 (s, 2H), 4.17-4.13 (m, 2H), 3.85-3.83 (m, 4H), 3.77-3.74 (m, 2H), 3.19-3.15 (m, 2H), 2.99-2.97 (m, 4H), 2.75-2.68 (m, 2H), 1.70-1.67 (m, 2H), 1.60-1.49 (m, 1H), 1.28-1.18 (m, 2H), 0.95 (d, J = 6.1 Hz, 3H). |

(continued)

| Example | Chemical structure | Analytical data |
|---|---|---|
| 219 | 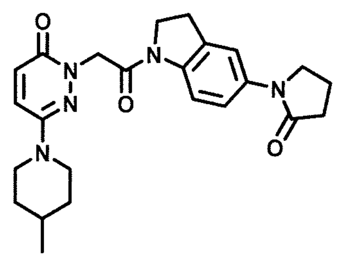 | $^1$H-NMR (400 MHz, $CDCl_3$) δ: 8.15 (d, J = 9.0 Hz, 1H), 7.79 (s, 1H), 7.18 (d, J = 10.1 Hz, 1H), 7.09 (dd, J = 9.0, 2.1 Hz, 1H), 6.88 (d, J = 10.1 Hz, 1H), 4.89 (s, 2H), 4.19-4.14 (m, 2H), 3.86-3.82 (m, 2H), 3.76 (br m, 2H), 3.29-3.25 (br m, 2H), 2.75-2.68 (br m, 2H), 2.62-2.58 (m, 2H), 2.18-2.11 (m, 2H), 1.70-1.67 (m, 2H), 1.56-1.49 (m, 1H), 1.28-1.18 (m, 2H), 0.96 (d, J = 6.7 Hz, 3H). |
| 220 | 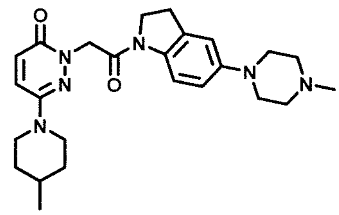 | $^1$H-NMR (400 MHz, $CDCl_3$) δ: 8.07 (d, J = 9.1 Hz, 1H), 7.17 (d, J = 10.1 Hz, 1H), 6.87 (d, J = 10.1 Hz, 1H), 6.79 (br s, 1H), 6.75 (d, J = 8.4 Hz, 1H), 4.87 (s, 2H), 4.13 (t, J = 8.4 Hz, 2H), 3.77-3.74 (br m, 2H), 3.21 (t, J = 8.2 Hz, 2H), 3.16-3.14 (m, 4H), 2.74-2.68 (m, 2H), 2.58 (d, J = 9.8 Hz, 4H), 2.35 (s, 3H), 1.71-1.66 (m, 2H), 1.57-1.47 (m, 1H), 1.27-1.17 (m, 2H), 0.95 (d, J = 6.1 Hz, 3H). |
| 221 | 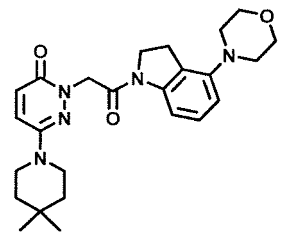 | $^1$H-NMR (400 MHz, $CDCl_3$) δ: 7.93 (d, J = 7.9 Hz, 1H), 7.20-7.14 (m, 2H), 6.88 (d, J = 9.8 Hz, 1H), 6.69 (d, J = 7.9 Hz, 1H), 4.89 (s, 2H), 4.18-4.13 (m, 2H), 3.85-3.83 (m, 4H), 3.25-3.20 (m, 4H), 3.19-3.15 (m, 2H), 2.99-2.97 (m, 4H), 1.44-1.41 (m, 4H), 0.96 (s, 6H). |
| 222 | 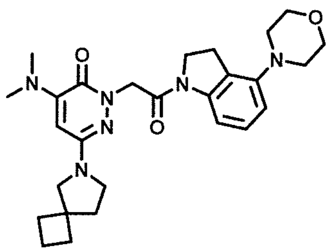 | LC-MS: $[M+H]^+$ / Rt (min) 493.4 / 1.077 (Method A) |
| 223 | 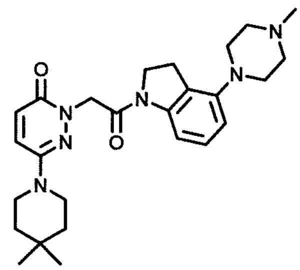 | $^1$H-NMR (400 MHz, $CDCl_3$) δ: 7.91 (d, J = 7.9 Hz, 1H), 7.18 (d, J = 10.1 Hz, 1H), 7.14 (t, J = 7.9 Hz, 1H), 6.88 (d, J = 10.1 Hz, 1H), 6.69 (d, J = 7.9 Hz, 1H), 4.89 (s, 2H), 4.14 (t, J = 8.2 Hz, 2H), 3.24-3.22 (m, 4H), 3.16 (t, J = 8.2 Hz, 2H), 3.03-3.00 (m, 4H), 2.57 (br s, 4H), 2.36 (s, 3H), 1.44-1.41 (br m, 4H), 0.96 (s, 6H). |

(continued)

| Example | Chemical structure | Analytical data |
|---|---|---|
| 224 | 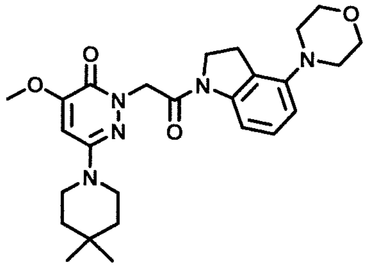 | $^1$H-NMR (400MHz, DMSO-d$_6$) δ: 7.67 (d, J = 8.0 Hz, 1H), 7.11 (t, J = 7.6 Hz, 1H ), 6.74 (s, 1H), 6.67 (d, J = 7.6 Hz, 1H), 4.84 (s, 2H), 4.18-4.14 (m, , 2H), 3.82 (s, 3H), 3.73-3.71 (m, 4H), 3.24-3.21 (m, 4H), 3.12-3.07 (m, 2H), 2.93-2.90 (m, 4H), 1.38-1.35 (m, 4H), 0.93 (s, 6H). |
| 225 | 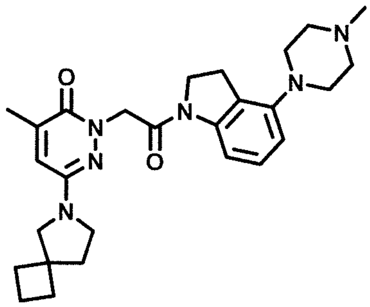 | $^1$H-NMR (400 MHz, CDCl$_3$) δ: 7.89 (d, J = 7.9 Hz, 1H), 7.13-7.09 (m, 1H), 6.82 (br s, 1H), 6.66 (d, J = 7.9 Hz, 1H), 4.89 (s, 2H), 4.17-4.12 (m, 2H), 3.34 (t, J = 6.7 Hz, 2H), 3.30 (s, 2H), 3.12-3.08 (m, 2H), 3.02 (br s, 4H), 2.77 (br s, 4H), 2.51 (br s, 3H), 2.21 (d, J = 1.2 Hz, 3H), 2.05-1.86 (m, 8H). |
| 226 | 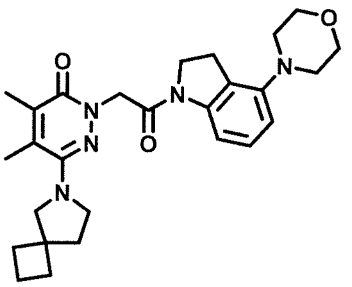 | $^1$H-NMR (400 MHz, DMSO-d$_6$) δ: 7.68 (d, J = 7.9 Hz, 1H), 7.12 (t, J = 7.9 Hz, 1H), 6.67 (d, J = 7.9 Hz, 1H), 4.86 (s, 2H), 4.18 (t, J = 8.2 Hz, 2H), 3.73 (t, J = 4.6 Hz, 4H), 3.25-3.20 (m, 4H), 3.11 (t, J = 8.2 Hz, 2H), 2.92 (t, J = 4.3 Hz, 4H), 2.16 (s, 3H), 2.05 (s, 3H), 2.00-1.78 (m, 8H). |
| 227 | 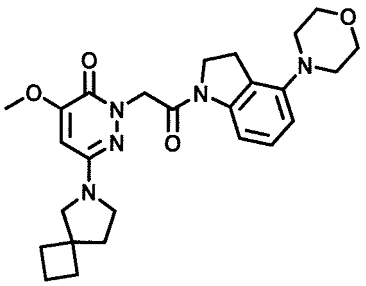 | $^1$H-NMR (400 MHz, DMSO-d$_6$) δ: 7.67 (d, J = 8.0 Hz, 1H), 7.11 (t, J = 8.0 Hz, 1H), 6.66 (d, J = 8.0 Hz, 1H), 6.39 (s, 1H), 4.82 (s, 2H), 4.18-4.14 (m, 2H), 3.83 (s, 3H), 3.73-3.71 (m, 4H), 3.30-3.28 (m, 2H), 3.12-3.07 (m, 2H), 2.93-2.90 (m, 5H), 2.00-1.84 (m, 9H). |
| 228 | 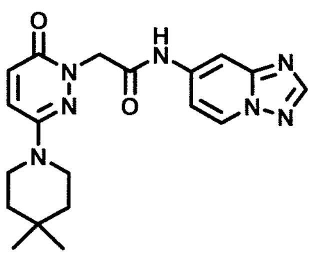 | $^1$H-NMR (400 MHz, CDCl$_3$) δ: 10.26 (s, 1H), 8.39 (d, J = 7.3 Hz, 1H), 8.25 (s, 1H), 8.12 (d, J = 1.8 Hz, 1H), 7.28-7.25 (m, 1H), 7.22 (d, J = 7.3 Hz, 1H), 6.95 (d, J = 9.8 Hz, 1H), 4.91 (s, 2H), 3.35-3.25 (m, 4H), 1.48-1.38 (m, 4H), 0.98 (s, 6H). |

(continued)

| Example | Chemical structure | Analytical data |
|---|---|---|
| 229 | 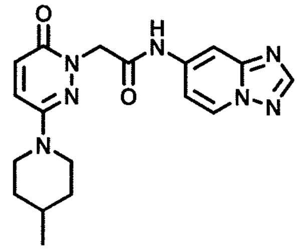 | $^1$H-NMR (400 MHz, $CDCl_3$) δ: 10.01 (s, 1H), 8.42 (d, J = 7.3 Hz, 1H), 8.24 (s, 1H), 8.11 (d, J = 2.4 Hz, 1H), 7.35-7.21 (m, 1H), 7.12 (dd, J = 7.6, 2.1 Hz, 1H), 6.95 (d, J = 9.8 Hz, 1H), 4.87 (s, 2H), 3.85 (d, J = 13.4 Hz, 2H), 2.78 (td, J = 12.8, 2.4 Hz, 2H), 1.72 (d, J = 12.8 Hz, 2H), 1.27-1.17 (m, 3H), 0.97 (d, J = 6.1 Hz, 3H). |
| 230 | 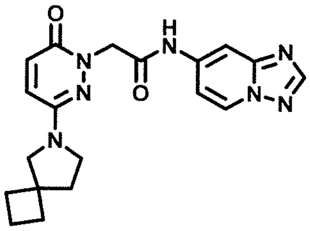 | $^1$H-NMR (400 MHz, $CDCl_3$) δ: 10.11 (s, 1H), 8.44 (d, J = 7.3 Hz, 1H), 8.26 (s, 1H), 8.12 (d, J = 1.8 Hz, 1H), 7.15 (dd, J = 7.3, 2.4 Hz, 1H), 7.05 (d, J = 10.4 Hz, 1H), 6.95 (d, J = 10.4 Hz, 1H), 4.89 (s, 2H), 3.40 (t, J = 7.0 Hz, 2H), 3.36 (s, 2H), 2.10-1.90 (m, 8H). |
| 231 | 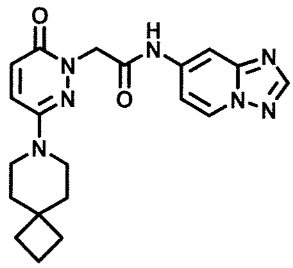 | $^1$H-NMR (400 MHz, $CDCl_3$) δ: 10.07 (s, 1H), 8.40 (d, J = 7.3 Hz, 1H), 8.24 (s, 1H), 8.10 (d, J = 1.2 Hz, 1H), 7.30-7.24 (m, 1H),7.15-7.11 (m, 1H), 6.95 (d, J = 9.8 Hz, 1H), 4.89 (s, 2H), 3.27-3.24 (m, 4H), 1.98-1.89 (m, 2H), 1.85-1.77 (m, 4H), 1.68-1.65 (m, 4H). |
| 232 | 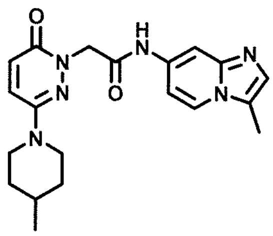 | LC-MS: $[M+H]^+$ / Rt (min) 381.2 / 1.54 (Method B) |
| 233 | 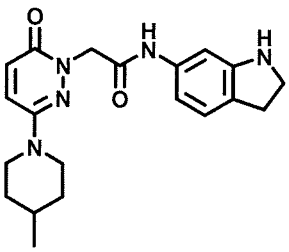 | $^1$H-NMR (400 MHz, $CDCl_3$) δ: 8.87 (s, 1H), 7.20 (d, J = 10.1 Hz, 1H), 7.05 (d, J = 1.8 Hz, 1H), 6.98 (d, J = 7.6 Hz, 1H), 6.90 (d, J = 10.1 Hz, 1H), 6.64 (dd, J = 7.6, 1.8 Hz, 1H), 4.79 (s, 2H), 3.84-3.77 (m, 3H), 3.54 (t, J = 8.2 Hz, 2H), 2.95 (t, J = 8.2 Hz, 2H), 2.79-2.72 (m, 2H), 1.72-1.69 (m, 2H), 1.58-1.51 (m, 1H), 1.27-1.17 (m, 2H), 0.96 (d, J = 6.7 Hz, 3H). |
| 234 | 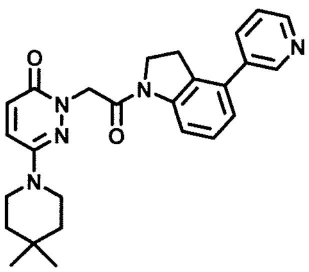 | $^1$H-NMR (400 MHz, DMSO-$d_6$) δ: 8.72 (s, 1H), 8.60 (d, J = 4.9 Hz, 1H), 8.07 (d, J = 7.9 Hz, 1H), 7.94 (d, J = 7.9 Hz, 1H), 7.59 (d, J = 9.8 Hz, 1H), 7.51 (dd, J = 7.9, 4.9 Hz, 1H), 7.32 (t, J = 7.9 Hz, 1H), 7.13 (d, J = 7.3 Hz, 1H), 6.87 (d, J = 9.8 Hz, 1H), 4.89 (s, 2H), 4.21 (t, J = 8.2 Hz, 2H), 3.29-3.23 (m, 6H), 1.39-1.36 (m, 4H), 0.94 (s, 6H). |

(continued)

| Example | Chemical structure | Analytical data |
|---|---|---|
| 235 | 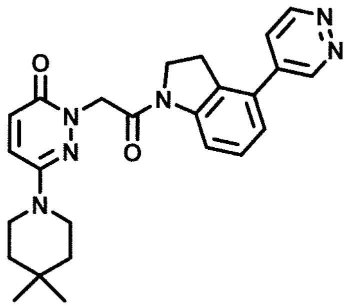 | $^1$H-NMR (400 MHz, DMSO-$d_6$) δ: 9.45 (dd, J = 2.4, 1.2 Hz, 1H), 9.32 (dd, J = 5.5, 1.2 Hz, 1H), 8.15 (d, J = 7.9 Hz, 1H), 7.87 (dd, J = 4.9, 2.4 Hz, 1H), 7.59 (d, J = 10.4 Hz, 1H), 7.38 (t, J = 7.9 Hz, 1H), 7.28-7.25 (m, 1H), 6.87 (d, J = 10.4 Hz, 1H), 4.91 (s, 2H), 4.23 (t, J = 8.2 Hz, 2H), 3.36 (t, J = 8.2 Hz, 2H), 3.26-3.23 (m, 4H), 1.37 (t, J = 5.8 Hz, 4H), 0.94 (s, 6H). |
| 236 | 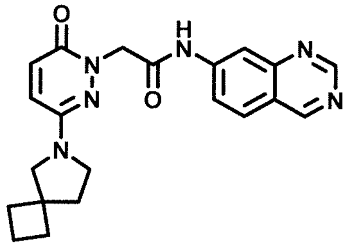 | $^1$H-NMR (400 MHz, $CDCl_3$) δ: 10.18 (s, 1H), 9.15 (s, 2H), 8.23 (s, 1H), 7.75-7.65 (m, 2H), 7.01-6.92 (m, 2H), 4.93 (s, 2H), 3.41-3.35 (m, 2H), 2.08-1.87 (m, 10H). |
| 237 | 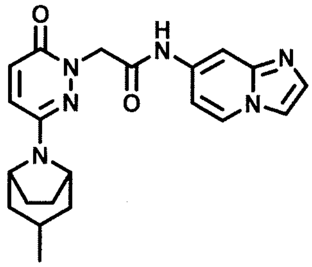 | $^1$H-NMR (400 MHz, $CD_3OD$)δ: 8.32 (s, 1H), 7.97 (s, 1H), 7.70 (s, 1H), 7.52-7.37 (m, 2H), 7.06 (s, 1H), 6.89 (s, 1H), 4.95-4.85 (m, 2H), 4.27 (s, 2H), 2.15-1.90 (m, 3H), 1.90-1.70 (m, 3H), 1.55-1.40 (m, 3H), 1.24 (d, J = 6.0 Hz, 3H). |
| 238 | 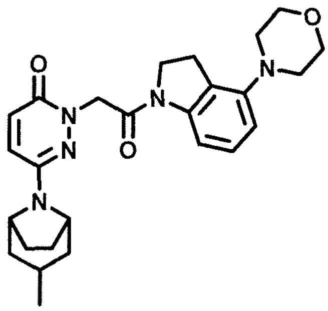 | $^1$H-NMR (400 MHz, $CDCl_3$) δ: 7.98 (d, J = 7.3 Hz, 1H), 7.18 (t, J = 8.2 Hz, 1H), 7.06 (d, J = 9.8 Hz, 1H), 6.88 (d, J = 9.8 Hz, 1H), 6.74 (d, J = 7.3 Hz, 1H), 4.90 (s, 2H), 4.19-4.09 (m, 4H), 3.89 (t, J = 25.6 Hz, 4H), 3.24 (s, 2H), 3.04 (s, 4H), 2.05-1.90 (m, 2H), 1.78-1.70 (m, 2H), 1.30-1.22 (m, 3H), 0.90-0.85 (m, 2H), 0.83 (d, J = 6.7 Hz, 3H). |

Examples 239 - 243

According to the method of Example 1, 37, or 50 and common reaction conditions, the compounds of Examples 239 to 243 were obtained by using each corresponding material compound.

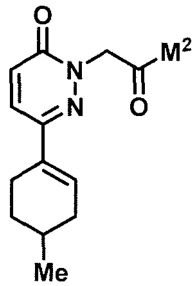

| Example | $M^2$ | Analytical data |
|---|---|---|
| 239 | 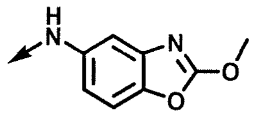 | $^1$H-NMR (400 MHz, $CDCl_3$) δ: 8.87 (s, 1H), 7.76 (s, 1H), 7.60 (d, J = 9.8 Hz, 1H), 7.24 (br s, 2H), 6.98 (d, J = 9.8 Hz, 1H), 6.37 (br s, 1H), 4.97 (s, 2H), 4.21 (s, 3H), 2.65-2.62 (br m, 1H), 2.37-2.30 (br m, 2H), 1.89-1.81 (br m, 2H), 1.76-1.67 (br m, 1H), 1.34-1.24 (m, 1H), 1.01 (d, J = 6.1 Hz, 3H). |
| 240 | 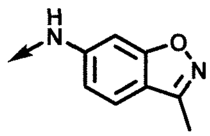 | $^1$H-NMR (400 MHz, $CDCl_3$) δ: 9.18 ( s, 1H), 8.02-7.99 (m, H1), 7.59 (d, J = 9.8 Hz, 1H), 7.48 (d, J = 8.6 Hz, 1H), 7.26-7.22 ( m, H1), 6.98 (d, J = 9.8 Hz, 1H), 6.38 (s, 1H), 4.98 (s, 2H), 2.68-2.59 (m, 1H), 2.52 (s, 3H), 2.43-2.31 (m, 2H), 1.92-1.82 (m, 2H), 1.8-1.70 (m, 1H), 1.39-1.27 (m, 1H), 1.02 (d, J = 6.1 Hz, 3H). |
| 241 | 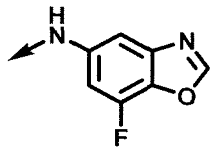 | $^1$H-NMR (400 MHz, $CDCl_3$) δ: 9.28 (s, 1H), 8.06 (s, 1H), 7.71 (s, 1H), 7.62 (d, J = 9.8 Hz, 1H), 7.50 (d, J = 11.6 Hz, 1H), 6.99 (d, J = 9.8 Hz, 1H), 6.38 (s, 1H), 4.99 (s, 2H), 2.73-2.57 (m, 1H), 2.38-2.27 (m, 2H), 1.90-1.78 (m, 2H), 1.75-1.57 (m, 2H), 1.01 (d, J = 6.7 Hz, 3H). |
| 242 | 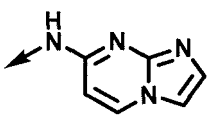 | $^1$H-NMR (400 MHz, $CDCl_3$) δ: 9.38 (s, 1H), 8.33 (d, J = 7.3 Hz, 1H), 7.99 (d, J = 7.3 Hz, 1H), 7.68 (d, J = 1.2 Hz, 1H), 7.59 (d, J = 9.8 Hz, 1H), 7.44 (d, J = 1.2 Hz, 1H), 6.97 (d, J = 9.8 Hz, 1H), 6.38 (s, 1H), 5.14 (s, 2H), 2.65-2.55 (m, 1H), 2.37-2.25 (m, 2H), 1.91-1.80 (m, 2H), 1.78-1.65 (m, 1H), 1.35-1.20 (m, 1H), 1.00 (d, J = 6.1 Hz, 3H). |
| 243 | 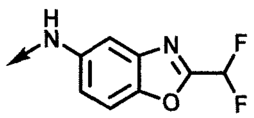 | $^1$H-NMR (400 MHz, $CDCl_3$) δ: 9.34 (s, 1H), 8.11 (s, 1H), 7.62 (d, J = 9.8 Hz, 1H), 7.48-7.42 (m, 2H), 6.99 (d, J = 9.8 Hz, 1H), 6.76 (t, J = 52.4 Hz, 1H), 6.38 (s, 1H), 5.02 (s, 2H), 2.68-2.53 (m, 1H), 2.40-2.28 (m, 2H), 1.90-1.79 (m, 2H), 1.48-1.42 (m, 1H), 1.35-1.24 (m, 1H), 1.01 (d, J = 6.1 Hz, 3H). |

Example 244

N-[2-(Dimethylamino)-1,3-benzooxazol-5-yl]-2-[3-(4-methylcyclohex-1-en-1-yl)-6-oxopyridazin-1(6H)-yl]acetamide

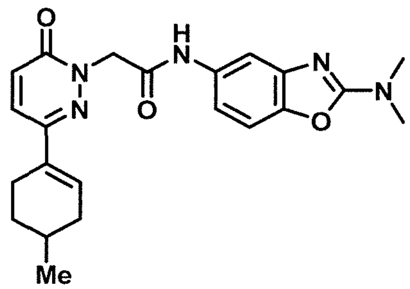

To a suspension of the compound of Reference example 16 (45 mg), $N^2$,$N^2$-dimethyl-1,3-benzoxazole-2,5-diamine (39 mg), and HATU (90 mg) in acetonitrile (1.8 mL) was added N,N-diisopropylethylamine (0.13 mL), and the mixture was stirred at room temperature for 2 hours. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; ethyl acetate:methanol = 100:0, and then 96:4) to obtain the titled compound (75 mg) .

$^1$H-NMR (400 MHz, $CDCl_3$) δ: 8.68 (s, 1H), 7.60 (d, J = 9.8 Hz, 1H), 7.50 (s, 1H), 7.21-7.14 (m, 2H), 6.98 (d, J = 9.8 Hz, 1H), 6.38 (s, 1H), 4.97 (s, 2H), 3.20 (s, 6H), 2.70-2.60 (m, 1H), 2.41-2.30 (m, 2H), 1.92-1.82 (m, 2H), 1.80-1.67 (m, 1H), 1.35-1.25 (m, 1H), 1.03 (d, J = 6.1 Hz, 3H).

<u>Examples 245 - 424</u>

According to the method of Example 1, 37, or 50 and common reaction conditions, the compounds of Examples 245 to 424 were obtained by using each corresponding material compound.

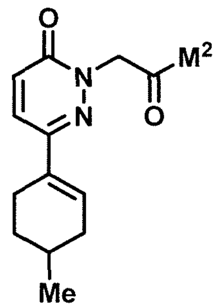

| Example | $M^2$ | Analytical data |
|---|---|---|
| 245 | 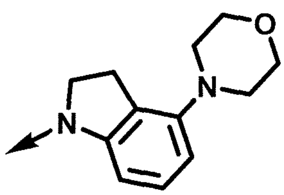 | $^1$H-NMR (400 MHz, $CDCl_3$) δ: 7.91 (d, J = 7.9 Hz, 1H), 7.55 (d, J = 9.8 Hz, 1H), 7.16 (t, J = 8.2 Hz, 1H), 6.92 (d, J = 9.8 Hz, 1H), 6.69 (d, J = 7.9 Hz, 1H), 6.33-6.32 (br m, 1H), 5.01 (d, J = 3.7 Hz, 2H), 4.19 (t, J = 8.2 Hz, 2H), 3.87-3.84 (m, 4H), 3.19 (t, J = 8.2 Hz, 2H), 2.99-2.97 (m, 4H), 2.61-2.56 (m, 1H), 2.34-2.25 (m, 2H), 1.88-1.78 (m, 2H), 1.74-1.65 (m, 1H), 1.32-1.22 (m, 1H), 0.99 (d, J = 6.1 Hz, 3H). |
| 246 | 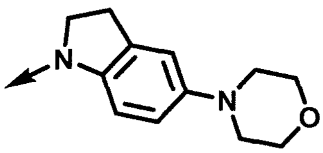 | LC-MS: $[M+H]^+$ / Rt (min) 435.4 / 1.308 (Method A) |
| 247 | 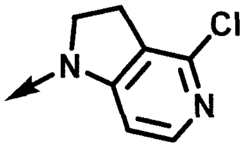 | LC-MS: $[M+H]^+$ / Rt (min) 385.3 / 1.019 (Method A) |

(continued)

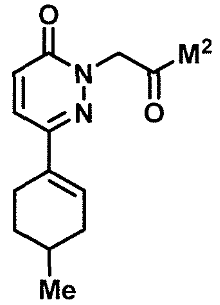

| Example | $M^2$ | Analytical data |
|---|---|---|
| 248 | 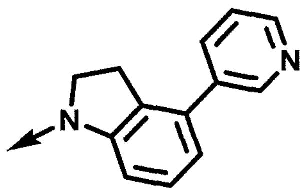 | $^1$H-NMR (400 MHz, DMSO-$d_6$) δ: 8.73 (d, J = 1.8 Hz, 1H), 8.60 (d, J = 4.9 Hz, 1H), 8.06 (d, J = 7.9 Hz, 1H), 7.95 (d, J = 7.9 Hz, 1H), 7.90 (d, J = 10.4 Hz, 1H), 7.51 (dd, J = 7.9, 4.9 Hz, 1H), 7.32 (t, J = 7.6 Hz, 1H), 7.14 (d, J = 7.3 Hz, 1H), 6.96 (d, J = 9.8 Hz, 1H), 6.55 (s, 1H), 5.07 (s, 2H), 4.25 (t, J = 8.5 Hz, 2H), 3.32-3.27 (m, 2H), 2.67-2.54 (m, 2H), 2.37-2.19 (m, 2H), 1.87-1.62 (m, 3H), 0.98 (d, J = 6.7 Hz, 3H). |
| 249 | 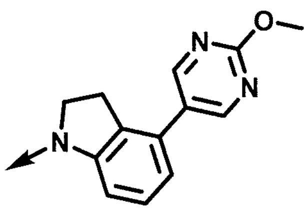 | $^1$H-NMR (400 MHz, DMSO-$d_6$) δ: 8.79 (s, 2H), 8.05 (d, J = 7.9 Hz, 1H), 7.89 (d, J = 10.4 Hz, 1H), 7.31 (t, J = 7.9 Hz, 1H), 7.15 (d, J = 7.3 Hz, 1H), 6.95 (d, J = 9.8 Hz, 1H), 6.56-6.52 (m, 1H), 5.06 (s, 2H), 4.25 (t, J = 8.2 Hz, 2H), 3.98 (s, 3H), 3.34-3.29 (m, 2H), 2.56-2.51 (m, 1H), 2.36-2.18 (m, 2H), 1.87-1.60 (m, 3H), 1.29-1.19 (m, 1H), 0.97 (d, J = 6.7 Hz, 3H). |
| 250 | 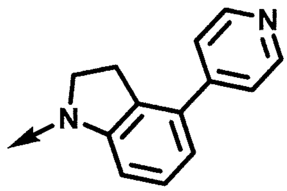 | $^1$H-NMR (400 MHz, DMSO-$d_6$) δ: 8.67-8.65 (m, 2H), 8.09 (d, J = 7.9 Hz, 1H), 7.89 (d, J = 9.8 Hz, 1H), 7.55-7.53 (m, 2H), 7.33 (t, J = 7.9 Hz, 1H), 7.17 (d, J = 6.7 Hz, 1H), 6.96 (d, J = 10.4 Hz, 1H), 6.56-6.53 (m, 1H), 5.07 (s, 2H), 4.25 (t, J = 8.2 Hz, 2H), 3.35-3.31 (m, 1H), 2.56-2.51 (m, 1H), 2.37-2.18 (m, 2H), 1.87-1.61 (m, 3H), 1.29-1.18 (m, 1H), 1.16-1.12 (m, 1H), 0.97 (d, J = 6.1 Hz, 3H). |
| 251 | 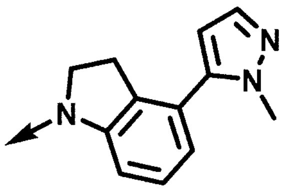 | LC-MS: $[M+H]^+$ / Rt (min) 430.2 / 0.965 (Method A) |
| 252 | 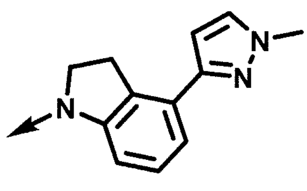 | $^1$H-NMR (400 MHz, DMSO-$d_6$) δ: 7.95 (d, J = 7.9 Hz, 1H), 7.89 (d, J = 9.8 Hz, 1H), 7.78 (d, J = 2.4 Hz, 1H), 7.45 (d, J = 7.9 Hz, 1H), 7.20 (t, J = 7.9 Hz, 1H), 6.95 (d, J = 10.4 Hz, 1H), 6.67 (d, J = 2.4 Hz, 1H), 6.55-6.53 (m, 1H), 5.05 (s, 2H), 4.27 (t, J = 8.2 Hz, 2H), 3.91 (s, 3H), 3.46 (t, J = 8.5 Hz, 2H), 2.58-2.51 (m, 1H), 2.37-2.18 (m, 2H), 1.88-1.62 (m, 3H), 1.29-1.18 (m, 1H), 0.97 (d, J = 5.8 Hz, 3H). |
| 253 | 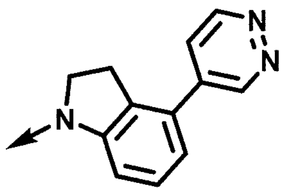 | $^1$H-NMR (400 MHz, $CDCl_3$) δ: 9.32-9.26 (m, 2H), 8.34 (d, J = 8.5 Hz, 1H), 7.58-7.52 (m, 2H), 7.35 (t, J = 7.9 Hz, 1H), 7.11 (d, J = 7.7 Hz, 1H), 6.93 (d, J = 9.5 Hz, 1H), 6.35-6.33 (m, 1H), 5.09-5.00 (m, 2H), 4.25 (t, J = 8.2 Hz, 2H), 3.36 (t, J = 8.2 Hz, 2H), 2.62-2.54 (m, 1H), 2.37-2.25 (m, 2H), 2.02-1.64 (m, 4H), 1.00 (d, J = 6.5 Hz, 3H). |

(continued)

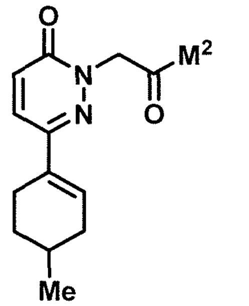

| Example | $M^2$ | Analytical data |
|---|---|---|
| 254 | 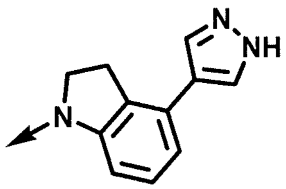 | LC-MS: $[M+H]^+$ / Rt (min) 416.3 / 0.933 (Method A) |
| 255 | 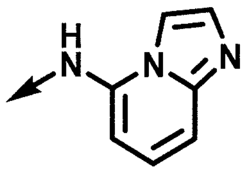 | $^1$H-NMR (400 MHz, $CDCl_3$) δ: 10.20 (s, 1H), 7.72 (s, 1H), 7.66 (t, J = 4.0 Hz, 2H), 7.45 (t, J = 9.1 Hz, 2H), 7.22 (dd, J = 8.8, 7.6 Hz, 1H), 7.08 (d, J = 9.8 Hz, 1H), 6.41 (s, 1H), 5.09 (s, 2H), 2.68-2.59 (m, 1H), 2.40-2.28 (m, 2H), 1.91-1.80 (m, 2H), 1.79-1.65 (m, 1H), 1.39-1.22 (m, 1H), 1.01 (d, J = 6.7 Hz, 3H). |
| 256 | 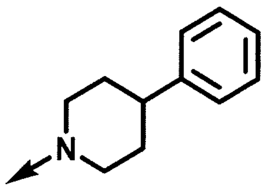 | LC-MS: $[M+H]^+$ / Rt (min) 392.2 / 2.01 (Method B) |
| 257 | 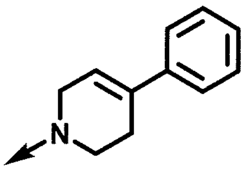 | $^1$H-NMR (400 MHz, $CDCl_3$) δ: 7.53 (d, J = 9.8 Hz, 1H), 7.41-7.28 (m, 5H), 6.91 (d, J = 9.8 Hz, 1H), 6.31 (br s, 1H), 6.08-6.02 (br m, 1H), 5.05 (d, J = 3.7 Hz, 1H), 4.99 (d, J = 3.7 Hz, 1H), 4.27 (d, J = 2.8 Hz, 1H), 4.20 (d, J = 2.8 Hz, 1H), 3.87-3.84 (br m, 1H), 3.74-3.71 (br m, 1H), 2.66-2.56 (m, 3H), 2.34-2.26 (m, 2H), 1.87-1.78 (m, 2H), 1.73-1.65 (m, 1H), 1.32-1.22 (m, 1H), 0.99 (d, J = 6.7 Hz, 3H). |
| 258 | 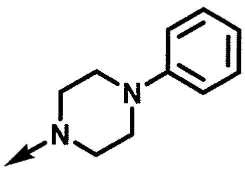 | $^1$H-NMR (400 MHz, $CDCl_3$) δ: 7.53 (d, J = 9.8 Hz, 1H), 7.33-7.29 (m, 2H), 7.04-6.92 (m, 3H), 6.90 (d, J = 9.8 Hz, 1H), 6.32 (br s, 1H), 5.00 (br s, 2H), 3.84-3.72 (br m, 4H), 3.28-3.22 (br m, 4H), 2.62-2.56 (m, 1H), 2.35-2.25 (m, 2H), 1.87-1.79 (m, 2H), 1.74-1.65 (m, 1H), 1.33-1.22 (m, 1H), 1.00 (d, J = 6.1 Hz, 3H). |
| 259 | 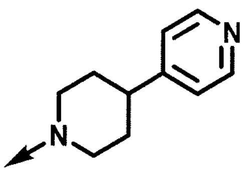 | $^1$H-NMR (400 MHz, $CDCl_3$) δ: 8.60-8.56 (m, 2H), 7.54 (dd, J = 9.8, 1.2 Hz, 1H), 7.36-7.25 (br m, 2H), 6.90 (d, J = 9.8 Hz, 1H), 6.32 (br s, 1H), 5.08-5.02 (m, 1H), 4.96-4.90 (br m, 1H), 4.81-4.77 (br m, 1H), 4.01-3.98 (br m, 1H), 3.30-3.24 (br m, 1H), 2.92-2.79 (br m, 1H), 2.77-2.71 (br m, 1H), 2.61-2.57 (br m, 1H), 2.35-2.29 (br m, 2H), 2.00-1.91 (m, 2H), 1.87-1.70 (m, 5H), 1.33-1.24 (m, 1H), 1.00 (d, J = 6.1 Hz, 3H). |

(continued)

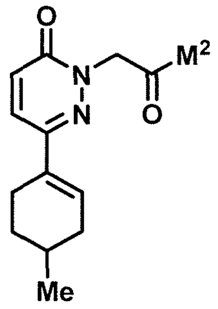

| Example | $M^2$ | Analytical data |
| --- | --- | --- |
| 260 | 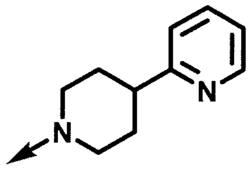 | $^1$H-NMR (400 MHz, $CDCl_3$) δ: 8.59-8.57 (m, 1H), 7.82-7.78 (br m, 1H), 7.53 (d, J = 10.1 Hz, 1H), 7.30-7.27 (br m, 2H), 6.89 (d, J = 10.1 Hz, 1H), 6.32-6.30 (m, 1H), 5.05-4.93 (m, 2H), 4.77-4.74 (br m, 1H), 3.99-3.95 (br m, 1H), 3.34-3.27 (m, 1H), 3.19-3.11 (br m, 1H), 2.83-2.75 (m, 1H), 2.62-2.57 (br m, 1H), 2.35-2.28 (m, 2H), 2.14-2.11 (br m, 1H), 2.03-2.00 (br m, 1H), 1.92-1.77 (m, 4H), 1.75-1.66 (m, 1H), 1.33-1.23 (br m, 1H), 1.00 (d, J = 6.7 Hz, 3H). |
| 261 | 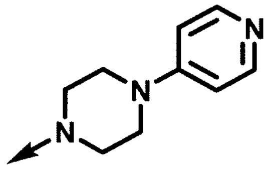 | $^1$H-NMR (400 MHz, $CD_3OD$) δ: 8.17 (d, J = 7.0 Hz, 2H), 7.85 (d, J = 9.8 Hz, 1H), 7.02 (d, J = 7.0 Hz, 2H), 6.94 (d, J = 9.8 Hz, 1H), 6.52-6.50 (br m, 1H), 5.11 (s, 2H), 3.84-3.77 (br m, 4H), 3.73-3.71 (br m, 2H), 3.66-3.63 (br m, 2H), 2.65-2.58 (m, 1H), 2.40-2.26 (m, 2H), 1.91-1.81 (m, 2H), 1.72 (br s, 1H), 1.35-1.25 (m, 1H), 1.02 (d, J = 6.7 Hz, 3H). |
| 262 | 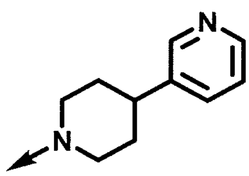 | $^1$H-NMR (400 MHz, $CD_3OD$) δ: 8.62 (br s, 1H), 8.53 (br s, 1H), 8.14-8.08 (m, 1H), 7.85 (d, J = 9.8 Hz, 1H), 7.68-7.63 (m, 1H), 6.95 (d, J = 9.8 Hz, 1H), 6.52-6.50 (br m, 1H), 5.18 (dd, J = 15.9, 3.7 Hz, 1H), 5.05-5.00 (m, 1H), 4.68-4.64 (br m, 1H), 4.17-4.12 (m, 1H), 3.38-3.30 (br m, 2H), 3.10-3.03 (m, 1H), 2.88-2.81 (m, 1H), 2.66-2.60 (br m, 1H), 2.41-2.28 (m, 2H), 2.02-1.83 (m, 4H), 1.78-1.67 (m, 2H), 1.36-1.26 (m, 1H), 1.02 (d, J = 6.7 Hz, 3H). |
| 263 | 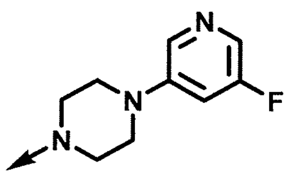 | LC-MS: $[M+H]^+$ / Rt (min) 412.3 / 0.803 (Method A) |
| 264 | 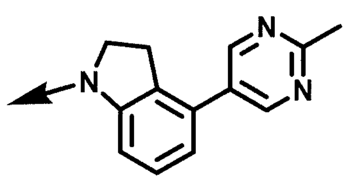 | $^1$H-NMR (400 MHz, DMSO-$d_6$) δ: 8.87 (s, 2H), 8.08 (d, J = 7.9 Hz, 1H), 7.89 (d, J = 9.8 Hz, 1H), 7.33 (t, J = 7.9 Hz, 1H), 7.18 (d, J = 6.7 Hz, 1H), 6.96 (d, J = 9.8 Hz, 1H), 6.54 (s, 1H), 5.07 (s, 2H), 4.25 (t, J = 8.2 Hz, 2H), 3.36-3.33 (m, 2H), 2.70-2.64 (m, 4H), 2.37-2.18 (m, 2H), 1.88-1.62 (m, 3H), 1.29-1.20 (m, 1H), 0.98 (d, J = 6.7 Hz, 3H). |
| 265 | 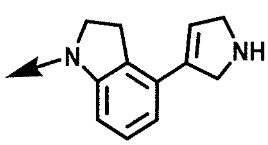 | LC-MS: $[M+H]^+$ / Rt (min) 417.4 / 0.728 (Method A) |

(continued)

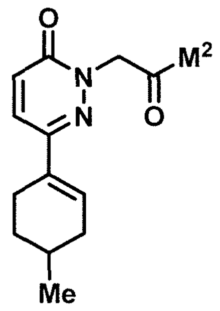

| Example | $M^2$ | Analytical data |
|---|---|---|
| 266 | 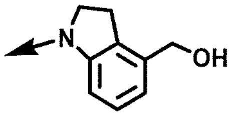 | LC-MS: $[M+H]^+$ / Rt (min) 380.4 / 0.892 (Method A) |
| 267 | 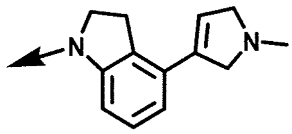 | LC-MS: $[M+H]^+$ / Rt (min) 431.4 / 0.793 (Method A) |
| 268 | 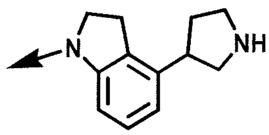 | LC-MS: $[M+H]^+$ / Rt (min) 419.5 / 0.767 (Method A) |
| 269 | 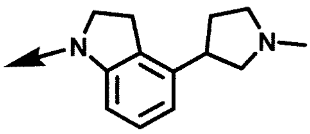 | $^1$H-NMR (400 MHz, DMSO-$d_6$) $\delta$: 7.88 (d, J = 9.8 Hz, 1H), 7.81 (d, J = 7.9 Hz, 1H), 7.12 (t, J = 7.6 Hz, 1H), 7.03 (d, J = 7.9 Hz, 1H), 6.94 (d, J = 9.8 Hz, 1H), 6.54-6.52 (m, 1H), 5.02 (s, 2H), 4.22 (t, J = 8.5 Hz, 2H), 3.39-3.32 (m, 3H), 3.19 (t, J = 8.2 Hz, 2H), 2.81 (t, J = 8.5 Hz, 1H), 2.67-2.42 (m, 5H), 2.36-2.18 (m, 3H), 1.85-1.61 (m, 4H), 1.28-1.17 (m, 1H), 0.97 (d, J = 6.7 Hz, 3H). |
| 270 | 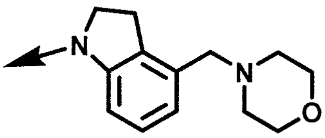 | $^1$H-NMR (400 MHz, DMSO-$d_6$) $\delta$: 7.88 (d, J = 9.8 Hz, 2H), 7.12 (t, J = 7.6 Hz, 1H), 6.98-6.93 (m, 2H), 6.55-6.52 (m, 1H), 5.03 (s, 2H), 4.24 (t, J = 8.5 Hz, 2H), 3.56 (t, J = 4.3 Hz, 4H), 3.42 (s, 2H), 3.23 (t, J = 8.2 Hz, 2H), 2.55-2.50 (m, 1H), 2.38-2.16 (m, 6H), 1.87-1.74 (m, 2H), 1.72-1.60 (m, 1H), 1.28-1.17 (m, 1H), 0.97 (d, J = 6.7 Hz, 3H). |
| 271 | 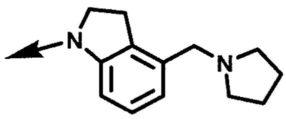 | LC-MS: $[M+H]^+$ / Rt (min) 433.4 / 0.848 (Method A) |
| 272 | 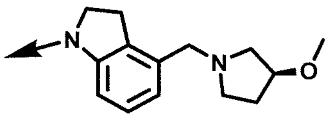 | $^1$H-NMR (400 MHz, $CDCl_3$) $\delta$: 8.09 (d, J = 7.9 Hz, 1H), 7.55 (d, J = 10.4 Hz, 1H), 7.13 (t, J = 7.9 Hz, 1H), 7.00 (d, J = 7.3 Hz, 1H), 6.92 (d, J = 9.8 Hz, 1H), 6.33 (s, 1H), 5.05-4.97 (m, 2H), 4.19 (t, J = 8.2 Hz, 2H), 3.95-3.89 (m, 1H), 3.59-3.52 (m, 2H), 2.76-2.44 (m, 5H), 2.34-2.27 (m, 2H), 2.11-2.02 (m, 1H), 1.87-1.62 (m, 10H), 0.99 (d, J = 6.7 Hz, 3H). |

(continued)

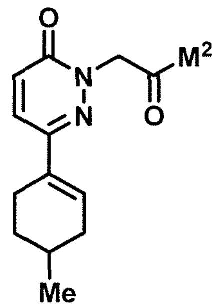

| Example | $M^2$ | Analytical data |
|---|---|---|
| 273 | 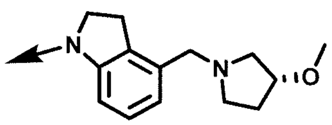 | $^1$H-NMR (400 MHz, $CDCl_3$) δ: 8.09 (d, J = 7.9 Hz, 1H), 7.55 (d, J = 9.8 Hz, 1H), 7.15-7.11 (m, 1H), 7.02-6.98 (m, 1H), 6.92 (d, J = 9.8 Hz, 1H), 6.32 (s, 1H), 5.06-4.97 (m, 2H), 4.19 (t, J = 8.2 Hz, 2H), 3.94-3.89 (m, 1H), 3.60-3.53 (m, 3H), 2.76-2.45 (m, 6H), 2.35-2.27 (m, 2H), 1.86-1.57 (m, 9H), 0.99 (d, J = 6.7 Hz, 3H). |
| 274 | 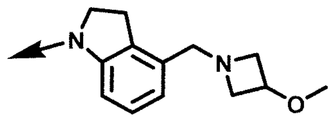 | $^1$H-NMR (400 MHz, DMSO-$d_6$) δ: 7.87 (t, J = 9.4 Hz, 2H), 7.11 (t, J = 7.9 Hz, 1H), 6.96-6.93 (m, 2H), 6.53 (s, 1H), 5.02 (s, 2H), 4.23 (t, J = 8.2 Hz, 2H), 4.00-3.94 (m, 1H), 3.53 (s, 4H), 3.49-3.45 (m, 4H), 3.20-3.15 (m, 5H), 2.87-2.83 (m, 2H), 2.70-2.54 (m, 1H), 2.36-2.17 (m, 2H), 1.88-1.74 (m, 2H), 1.70-1.60 (m, 1H), 1.29-1.18 (m, 1H), 0.97 (d, J = 6.7 Hz, 3H). |
| 275 | 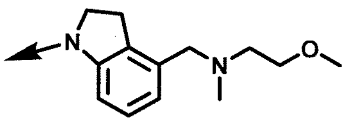 | $^1$H-NMR (400 MHz, DMSO-$d_6$) δ: 7.90-7.86 (m, 2H), 7.11 (t, J = 7.6 Hz, 1H), 6.97-6.93 (m, 2H), 6.53 (s, 1H), 5.03 (s, 2H), 4.23 (t, J = 8.5 Hz, 2H), 3.46-3.42 (m, 4H), 3.23-3.19 (m, 5H), 2.53-2.51 (m, 3H), 2.36-2.17 (m, 2H), 2.14 (s, 3H), 1.86-1.74 (m, 2H), 1.72-1.60 (m, 1H), 1.28-1.17 (m, 1H), 0.97 (d, J = 6.1 Hz, 3H). |
| 276 | 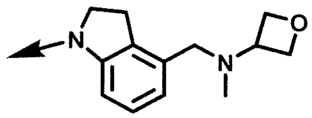 | $^1$H-NMR (400 MHz, DMSO-$d_6$) δ: 7.91-7.87 (m, 2H), 7.13 (t, J = 7.6 Hz, 1H), 6.96 (t, J = 7.9 Hz, 2H), 6.54 (s, 1H), 5.03 (s, 2H), 4.51 (t, J = 6.4 Hz, 2H), 4.43 (t, J = 6.1 Hz, 2H), 4.25 (t, J = 8.5 Hz, 2H), 3.61-3.54 (m, 1H), 3.30-3.25 (m, 4H), 2.56-2.52 (m, 1H), 2.36-2.18 (m, 2H), 1.95 (s, 3H), 1.87-1.75 (m, 2H), 1.73-1.61 (m, 1H), 1.29-1.17 (m, 1H), 0.97 (d, J = 6.1 Hz, 3H). |
| 277 | 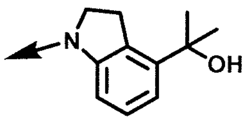 | $^1$H-NMR (400 MHz, DMSO-$d_6$) δ: 7.93-7.87 (m, 2H), 7.12-7.07 (m, 2H), 6.94 (d, J = 10.4 Hz, 1H), 6.55-6.52 (m, 1H), 5.03 (s, 2H), 4.97 (s, 1H), 4.17 (t, J = 8.2 Hz, 2H), 3.42 (t, J = 8.5 Hz, 2H), 2.57-2.53 (m, 1H), 2.37-2.17 (m, 2H), 1.87-1.61 (m, 3H), 1.46 (s, 6H), 1.30-1.18 (m, 1H), 0.97 (d, J = 6.1 Hz, 3H). |
| 278 | 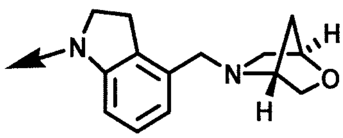 | $^1$H-NMR (400 MHz, DMSO-$d_6$) δ: 7.89-7.85 (m, 2H), 7.11 (t, J = 7.6 Hz, 1H), 7.02 (d, J = 7.9 Hz, 1H), 6.94 (d, J = 9.8 Hz, 1H), 6.54 (s, 1H), 5.03 (s, 2H), 4.35 (s, 1H), 4.24 (t, J = 8.5 Hz, 2H), 3.91 (d, J = 7.3 Hz, 1H), 3.71-3.62 (m, 2H), 3.53-3.51 (m, 1H), 3.41 (s, 1H), 3.22 (t, J = 8.5 Hz, 2H), 2.72 (d, J = 8.5 Hz, 1H), 2.55-2.51 (m, 1H), 2.43 (d, J = 9.8 Hz, 1H), 2.36-2.13 (m, 2H), 1.87-1.74 (m, 3H), 1.71-1.56 (m, 2H), 1.28-1.18 (m, 1H), 0.97 (d, J = 6.7 Hz, 3H). |

(continued)

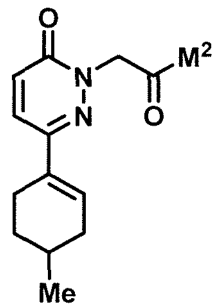

| Example | $M^2$ | Analytical data |
|---|---|---|
| 279 | 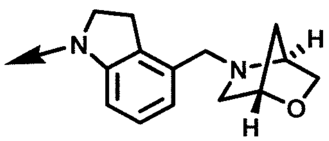 | $^1$H-NMR (400 MHz, DMSO-$d_6$) δ: 7.88-7.83 (m, 2H), 7.10 (t, J = 7.6 Hz, 1H), 7.01 (d, J = 7.9 Hz, 1H), 6.93 (d, J = 9.8 Hz, 1H), 6.52 (s, 1H), 5.01 (s, 2H), 4.33 (s, 1H), 4.22 (t, J = 8.5 Hz, 2H), 3.89 (d, J = 7.3 Hz, 1H), 3.70-3.60 (m, 2H), 3.52-3.50 (m, 1H), 3.39 (s, 1H), 3.20 (t, J = 8.2 Hz, 2H), 2.70 (d, J = 8.5 Hz, 1H), 2.55-2.51 (m, 1H), 2.41 (d, J = 9.8 Hz, 1H), 2.35-2.16 (m, 2H), 1.84-1.73 (m, 3H), 1.70-1.56 (m, 2H), 1.27-1.18 (m, 1H), 0.96 (d, J = 6.7 Hz, 3H). |
| 280 | 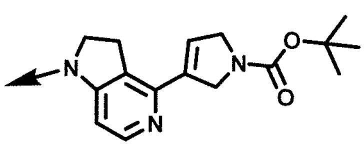 | LC-MS: $[M+H]^+$ / Rt (min) 518.4 / 1.075 (Method A) |
| 281 | 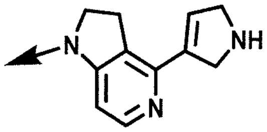 | LC-MS: $[M+H]^+$ / Rt (min) 418.5 / 0.884 (Method A) |
| 282 | 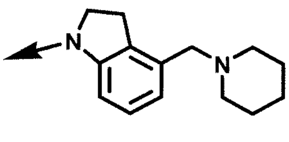 | $^1$H-NMR (400 MHz, DMSO-$d_6$) δ: 7.89-7.85 (m, 2H), 7.11 (t, J = 7.6 Hz, 1H), 6.96-6.93 (m, 2H), 6.53 (s, 1H), 5.02 (s, 2H), 4.23 (t, J = 8.2 Hz, 2H), 3.37 (s, 2H), 3.21 (t, J = 8.5 Hz, 2H), 2.55-2.52 (m, 1H), 2.37-2.16 (m, 6H), 1.87-1.60 (m, 3H), 1.51-1.34 (m, 6H), 1.28-1.18 (m, 1H), 0.97 (d, J = 6.7 Hz, 3H). |
| 283 | 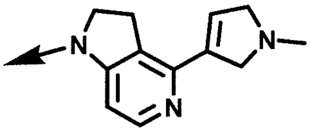 | $^1$H-NMR (400 MHz, DMSO-$d_6$) δ: 8.31-8.28 (m, 1H), 7.88 (d, J = 10.4 Hz, 1H), 7.73 (d, J = 4.9 Hz, 1H), 6.95 (d, J = 9.8 Hz, 1H), 6.53 (s, 1H), 6.30 (s, 1H), 5.07 (s, 2H), 4.30 (t, J = 8.2 Hz, 2H), 3.87-3.84 (m, 2H), 3.64-3.61 (m, 2H), 3.36-3.31 (m, 2H), 2.54-2.50 (m, 1H), 2.42 (s, 3H), 2.35-2.16 (m, 2H), 1.86-1.73 (m, 2H), 1.70-1.60 (m, 1H), 1.29-1.16 (m, 1H), 0.96 (d, J = 6.7 Hz, 3H). |
| 284 | 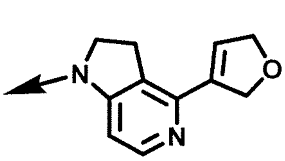 | $^1$H-NMR (400 MHz, DMSO-$d_6$) δ: 8.30 (d, J = 5.5 Hz, 1H), 7.89 (d, J = 9.8 Hz, 1H), 7.76 (d, J = 5.5 Hz, 1H), 6.95 (d, J = 9.8 Hz, 1H), 6.54-6.49 (m, 2H), 5.08 (s, 2H), 5.02-4.99 (m, 2H), 4.84-4.81 (m, 2H), 4.33 (t, J = 8.2 Hz, 2H), 3.40-3.34 (m, 2H), 2.35-2.16 (m, 2H), 1.86-1.74 (m, 2H), 1.70-1.60 (m, 1H), 1.28-1.16 (m, 2H), 0.96 (d, J = 6.1 Hz, 3H). |

(continued)

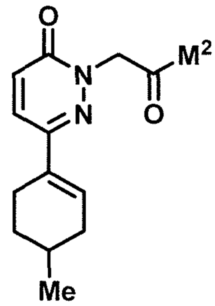

| Example | $M^2$ | Analytical data |
|---|---|---|
| 285 | 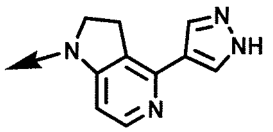 | $^1$H-NMR (400 MHz, DMSO-$d_6$) δ: 13.15 (s, 1H), 8.30 (d, J = 5.5 Hz, 1H), 8.20 (s, 1H), 8.02 (s, 1H), 7.88 (d, J = 9.8 Hz, 1H), 7.67 (d, J = 4.9 Hz, 1H), 6.95 (d, J = 9.8 Hz, 1H), 6.53 (s, 1H), 5.08 (s, 2H), 4.34 (t, J = 8.2 Hz, 2H), 3.38 (t, J = 8.5 Hz, 2H), 2.54-2.50 (m, 1H), 2.36-2.15 (m, 2H), 1.86-1.72 (m, 2H), 1.70-1.59 (m, 1H), 1.29-1.16 (m, 1H), 0.96 (d, J = 6.7 Hz, 3H). |
| 286 | 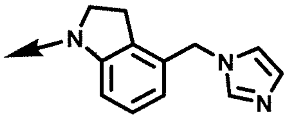 | $^1$H-NMR (400 MHz, DMSO-$d_6$) δ: 7.92-7.87 (m, 2H), 7.73 (s, 1H), 7.18-7.13 (m, 2H), 6.96-6.92 (m, 2H), 6.77 (d, J = 7.3 Hz, 1H), 6.53 (s, 1H), 5.19 (s, 2H), 5.03 (s, 2H), 4.25 (t, J = 8.2 Hz, 2H), 3.14 (t, J = 8.2 Hz, 2H), 2.69-2.53 (m, 1H), 2.38-2.15 (m, 2H), 1.88-1.59 (m, 3H), 1.29-1.17 (m, 1H), 0.97 (d, J = 6.7 Hz, 3H). |
| 287 | 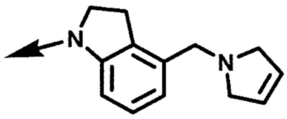 | $^1$H-NMR (400 MHz, $CDCl_3$) δ: 8.26 (d, J = 7.9 Hz, 1H), 7.71 (d, J = 9.8 Hz, 1H), 7.30 (t, J = 7.9 Hz, 1H), 7.20 (d, J = 7.9 Hz, 1H), 7.08 (d, J = 10.4 Hz, 1H), 6.49 (s, 1H), 5.95 (s, 2H), 5.22-5.13 (m, 2H), 4.36 (t, J = 8.2 Hz, 2H), 3.92 (s, 2H), 3.68-3.63 (m, 1H), 3.47 (t, J = 8.2 Hz, 2H), 2.79-2.70 (m, 1H), 2.52-2.40 (m, 2H), 2.04-1.70 (m, 6H), 1.49-1.38 (m, 1H), 1.16 (d, J = 6.7 Hz, 3H). |
| 288 | 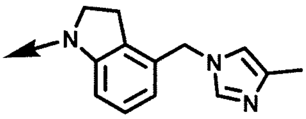 | LC-MS: $[M+H]^+$ / Rt (min) 444.4 / 0.827 (Method A) |
| 289 | 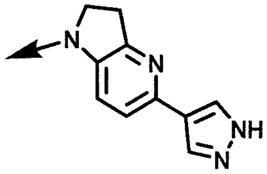 | LC-MS: $[M+H]^+$ / Rt (min) 417.3 / 0.826 (Method A) |
| 290 | 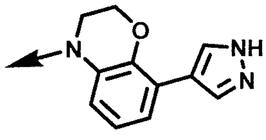 | $^1$H-NMR (400 MHz, DMSO-$d_6$) δ: 12.93 (s, 1H), 8.14 (s, 1H), 7.96 (s, 1H), 7.86 (d, J = 9.8 Hz, 1H), 7.66-7.45 (m, 2H), 6.91-6.86 (m, 2H), 6.52 (s, 1H), 5.15 (s, 2H), 4.45-4.41 (m, 2H), 3.95 (s, 2H), 2.58-2.51 (m, 1H), 2.37-2.18 (m, 2H), 1.87-1.61 (m, 3H), 1.29-1.16 (m, 1H), 0.97 (d, J = 6.7 Hz, 3H). |
| 291 | 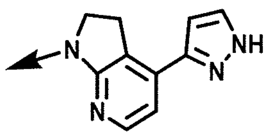 | $^1$H-NMR (400 MHz, DMSO-$d_6$) δ: 13.36 (s, 1H), 8.20 (s, 2H), 8.11 (d, J = 5.5 Hz, 1H), 7.88 (d, J = 10.4 Hz, 1H), 7.35 (d, J = 5.5 Hz, 1H), 6.93 (d, J = 9.8 Hz, 1H), 6.54-6.52 (m, 1H), 5.48 (s, 2H), 4.05 (t, J = 8.5 Hz, 2H), 2.37-2.15 (m, 2H), 1.87-1.60 (m, 3H), 1.30-1.15 (m, 4H), 0.97 (d, J = 6.7 Hz, 3H). |

(continued)

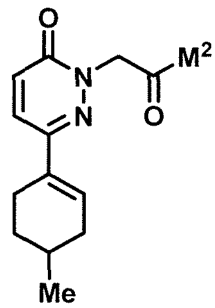

| Example | $M^2$ | Analytical data |
|---|---|---|
| 292 | 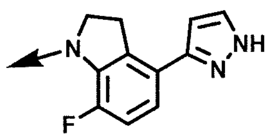 | $^1$H-NMR (400 MHz, DMSO-$d_6$) δ: 13.07 (s, 1H), 8.07 (br s, 2H), 7.88 (d, J = 10.0 Hz, 1H), 7.37-7.33 (m, 1H), 7.10 (t, J = 9.5 Hz, 1H), 6.94 (d, J = 9.8 Hz, 1H), 6.54 (s, 1H), 5.07 (s, 2H), 4.23 (t, J = 7.6 Hz, 2H), 3.28-3.16 (m, 2H), 2.37-2.17 (m, 2H), 1.87-1.59 (m, 3H), 1.29-1.24 (m, 2H), 0.97 (d, J = 6.7 Hz, 3H). |
| 293 | 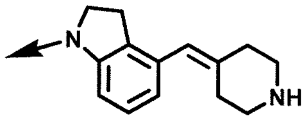 | LC-MS: $[M+H]^+$ / Rt (min) 445.5 / 0.864 (Method A) |
| 294 | 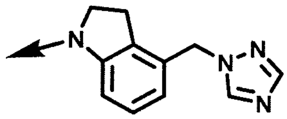 | $^1$H-NMR (400 MHz, DMSO-$d_6$) δ: 8.62 (s, 1H), 7.96 (s, 1H), 7.91-7.84 (m, 2H), 7.13 (t, J = 7.9 Hz, 1H), 6.92 (d, J = 9.8 Hz, 1H), 6.82 (d, J = 7.3 Hz, 1H), 6.51 (s, 1H), 5.38 (s, 2H), 5.00 (s, 2H), 4.23 (t, J = 8.5 Hz, 2H), 3.29-3.27 (m, 1H), 3.17 (t, J = 8.5 Hz, 2H), 2.34-2.13 (m, 2H), 1.83-1.58 (m, 3H), 1.25-1.17 (m, 1H), 0.94 (d, J = 6.7 Hz, 3H). |
| 295 | 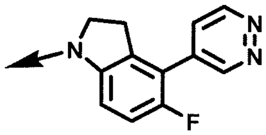 | $^1$H-NMR (400 MHz, DMSO-$d_6$) δ: 9.40-9.37 (m, 2H), 8.09 (dd, J = 8.9, 4.6 Hz, 1H), 7.90-7.87 (m, 2H), 7.24 (t, J = 9.5 Hz, 1H), 6.95 (d, J = 9.8 Hz, 1H), 6.55-6.53 (m, 1H), 5.06 (s, 2H), 4.27 (t, J = 8.2 Hz, 2H), 3.24 (t, J = 8.2 Hz, 2H), 2.56-2.52 (m, 1H), 2.37-2.18 (m, 2H), 1.88-1.61 (m, 3H), 1.29-1.20 (m, 1H), 0.98 (d, J = 6.7 Hz, 3H). |
| 296 | 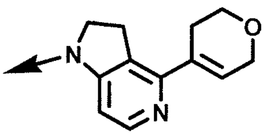 | LC-MS: $[M+H]^+$ / Rt (min) 433.4 / 0.700 (Method A) |
| 297 | 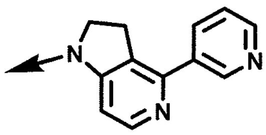 | $^1$H-NMR (400 MHz, $CDCl_3$) δ: 8.99 (d, J = 1.8 Hz, 1H), 8.68 (dd, J = 4.9, 1.8 Hz, 1H), 8.54 (d, J = 5.5 Hz, 1H), 8.15-8.08 (m, 2H), 7.61 (d, J = 10.0 Hz, 1H), 7.45 (dd, J = 7.6, 4.6 Hz, 1H), 6.96 (d, J = 10.0 Hz, 1H), 6.39-6.36 (m, 1H), 5.07 (s, 2H), 4.31 (t, J = 8.2 Hz, 2H), 3.52 (t, J = 8.2 Hz, 2H), 2.63-2.58 (m, 1H), 2.38-2.29 (m, 2H), 1.90-1.83 (m, 2H), 1.36-1.26 (m, 2H), 1.03 (d, J = 6.1 Hz, 3H). |
| 298 | 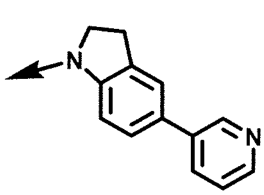 | $^1$H-NMR (400 MHz, DMSO-$d_6$) δ: 8.85 (d, J = 1.8 Hz, 1H), 8.50 (dd, J = 4.6, 1.5 Hz, 1H), 8.05-8.01 (m, 2H), 7.87 (d, J = 9.8 Hz, 1H), 7.65 (s, 1H), 7.53 (dd, J = 8.5, 1.8 Hz, 1H), 7.43 (dd, J = 7.9, 4.9 Hz, 1H), 6.94 (d, J = 9.8 Hz, 1H), 6.53-6.51 (m, 1H), 5.04 (s, 2H), 4.27 (t, J = 8.5 Hz, 2H), 3.29-3.24 (m, 2H), 2.55-2.50 (m, 1H), 2.35-2.15 (m, 2H), 1.86-1.60 (m, 3H), 1.27-1.18 (m, 1H), 0.95 (d, J = 6.1 Hz, 3H). |

(continued)

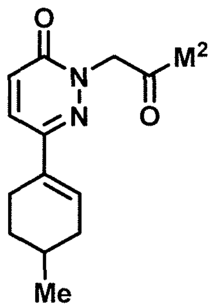

| Example | $M^2$ | Analytical data |
|---|---|---|
| 299 | 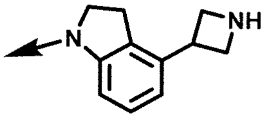 | LC-MS: $[M+H]^+$ / Rt (min) 405.3 / 0.657 (Method A) |
| 300 | 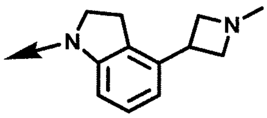 | LC-MS: $[M+H]^+$ / Rt (min) 419.3 / 0.670 (Method A) |
| 301 | 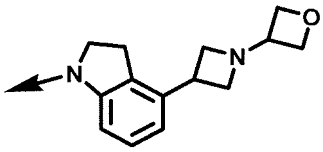 | LC-MS: $[M+H]^+$ / Rt (min) 461.4 / 0.787 (Method A) |
| 302 | 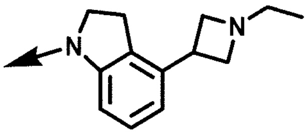 | $^1$H-NMR (400 MHz, $CDCl_3$) δ: 8.00 (d, J = 8.5 Hz, 1H), 7.48 (d, J = 9.8 Hz, 1H), 7.12 (t, J = 7.9 Hz, 1H), 6.93-6.84 (m, 2H), 6.26 (s, 1H), 4.96-4.91 (m, 2H), 4.12 (t, J = 7.9 Hz, 2H), 3.86-3.66 (m, 3H), 3.11-3.01 (m, 3H), 2.55-2.46 (m, 3H), 2.30-1.85 (m, 3H), 1.81-1.57 (m, 3H), 1.25-1.15 (m, 1H), 1.00-0.92 (m, 6H). |
| 303 | 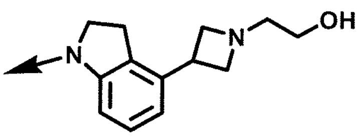 | LC-MS: $[M+H]^+$ / Rt (min) 449.4 / 0.750 (Method A) |
| 304 | 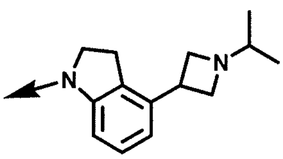 | $^1$H-NMR (400 MHz, DMSO-$d_6$) δ: 7.85 (dd, J = 21.7, 8.9 Hz, 2H), 7.15 (t, J = 7.9 Hz, 1H), 7.01 (d, J = 7.3 Hz, 1H), 6.94 (d, J = 9.8 Hz, 1H), 6.54-6.52 (m, 1H), 5.02 (s, 2H), 4.21 (t, J = 8.5 Hz, 2H), 3.64-3.53 (m, 3H), 3.08 (t, J = 7.9 Hz, 2H), 2.99-2.95 (m, 2H), 2.68-2.64 (m, 1H), 2.35-2.21 (m, 3H), 1.86-1.75 (m, 2H), 1.70-1.63 (m, 1H), 1.28-1.19 (m, 1H), 0.97 (d, J = 6.1 Hz, 3H), 0.87 (d, J = 6.1 Hz, 6H). |
| 305 | 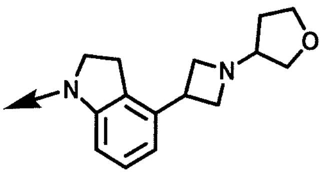 | LC-MS: $[M+H]^+$ / Rt (min) 475.4 / 0.908 (Method A) |

(continued)

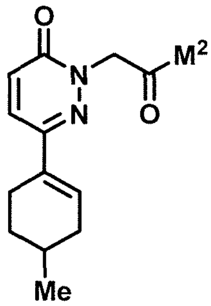

| Example | $M^2$ | Analytical data |
|---|---|---|
| 306 | 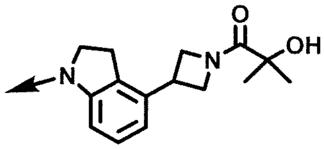 | $^1$H-NMR (400 MHz, DMSO-$d_6$) δ: 7.90-7.86 (m, 2H), 7.21 (t, J = 7.9 Hz, 1H), 7.11 (d, J = 7.3 Hz, 1H), 6.95 (d, J = 9.8 Hz, 1H), 6.55-6.52 (m, 1H), 5.12 (s, 1H), 5.03 (s, 2H), 4.78 (t, J = 8.9 Hz, 1H), 4.43-4.39 (m, 1H), 4.24 (t, J = 8.5 Hz, 3H), 3.91-3.86 (m, 2H), 3.15-3.10 (m, 2H), 2.36-2.18 (m, 2H), 1.87-1.75 (m, 2H), 1.72-1.60 (m, 1H), 1.31-1.21 (m, 7H), 0.97 (d, J = 6.7 Hz, 3H) . |
| 307 | 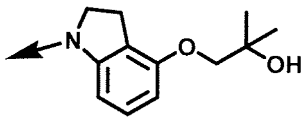 | $^1$H-NMR (400 MHz, $CDCl_3$) δ: 7.83 (d, J = 8.5 Hz, 1H), 7.57 (d, J = 9.8 Hz, 1H), 7.15 (t, J = 8.2 Hz, 1H), 6.94 (d, J = 9.8 Hz, 1H), 6.59 (d, J = 7.9 Hz, 1H), 6.35 (s, 1H), 5.06-4.98 (m, 2H), 4.22 (t, J = 8.5 Hz, 2H), 3.85 (s, 2H), 3.24 (t, J = 8.5 Hz, 2H), 2.65-2.56 (m, 1H), 2.38-2.27 (m, 2H), 2.01 (br s, 2H), 1.89-1.81 (m, 2H), 1.76-1.67 (m, 1H), 1.37 (s, 6H), 1.01 (d, J = 6.1 Hz, 3H). |
| 308 | 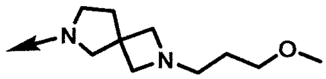 | LC-MS: $[M+H]^+$ / Rt (min) 415.4 / 0.671 (Method A) |
| 309 | 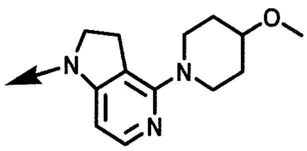 | $^1$H-NMR (400 MHz, DMSO-$d_6$) δ: 7.94 (d, J = 5.5 Hz, 1H), 7.87 (d, J = 9.8 Hz, 1H), 7.40 (d, J = 5.5 Hz, 1H), 6.93 (d, J = 9.8 Hz, 1H), 6.52 (s, 1H), 5.02 (s, 2H), 4.22-4.18 (m, 2H), 3.69-3.63 (m, 2H), 3.39-3.32 (m, 1H), 3.25 (s, 3H), 3.16 (t, J = 7.8 Hz, 2H), 3.03-2.96 (m, 2H), 2.35-2.15 (m, 2H), 1.94-1.73 (m, 4H), 1.69-1.58 (m, 1H), 1.50-1.41 (m, 2H), 1.23-1.21 (m, 2H), 0.96 (d, J = 6.7 Hz, 3H). |
| 310 | 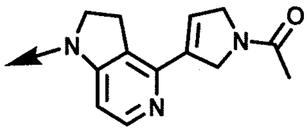 | LC-MS: $[M+H]^+$ / Rt (min) 460.4 / 0.788 (Method A) |
| 311 | 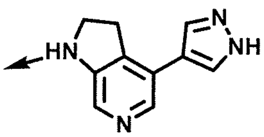 | $^1$H-NMR (400 MHz, DMSO-$d_6$) δ: 13.19 (s, 1H), 8.94 (s, 1H), 8.54 (s, 1H), 8.22-8.01 (m, 2H), 7.87 (d, J = 9.8 Hz, 1H), 6.94 (d, J = 9.8 Hz, 1H), 6.52 (s, 1H), 5.06 (s, 2H), 4.29 (t, J = 8.5 Hz, 2H), 3.44-3.33 (m, 2H), 2.35-2.15 (m, 2H), 1.86-1.57 (m, 4H), 1.29-1.16 (m, 1H), 0.95 (d, J = 6.1 Hz, 3H). |
| 312 | 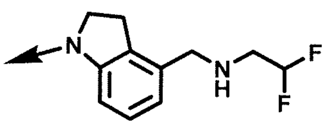 | $^1$H-NMR (400 MHz, DMSO-$d_6$) δ: 7.90-7.85 (m, 2H), 7.13 (t, J = 7.6 Hz, 1H), 7.03 (d, J = 7.3 Hz, 1H), 6.95 (d, J = 9.8 Hz, 1H), 6.54 (s, 1H), 6.17-5.85 (m, 1H), 5.03 (s, 2H), 4.24 (t, J = 8.2 Hz, 2H), 3.72 (s, 2H), 3.20 (t, J = 8.2 Hz, 2H), 2.86 (t, J = 15.9 Hz, 2H), 2.68-2.54 (m, 2H), 2.36-2.15 (m, 2H), 1.88-1.60 (m, 3H), 1.29-1.19 (m, 1H), 0.97 (d, J = 6.1 Hz, 3H). |

(continued)

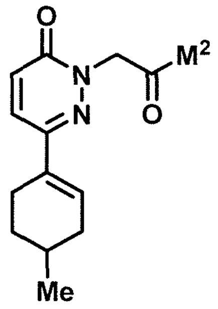

| Example | $M^2$ | Analytical data |
|---|---|---|
| 313 | 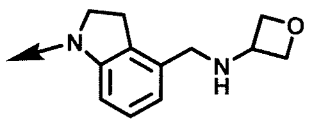 | $^1$H-NMR (400 MHz, DMSO-$d_6$) δ: 7.90-7.84 (m, 2H), 7.11 (t, J = 7.6 Hz, 1H), 7.01-6.93 (m, 2H), 6.54 (s, 1H), 5.03 (s, 2H), 4.58-4.54 (m, 2H), 4.31-4.22 (m, 4H), 3.92-3.82 (m, 1H), 3.58 (s, 2H), 3.21 (t, J = 8.2 Hz, 2H), 2.85-2.75 (m, 1H), 2.37-2.16 (m, 2H), 1.88-1.58 (m, 3H), 1.30-1.17 (m, 1H), 0.97 (d, J = 6.1 Hz, 3H). |
| 314 | 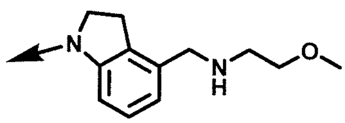 | $^1$H-NMR (400 MHz, DMSO-$d_6$) δ: 7.90-7.83 (m, 2H), 7.12 (t, J = 7.9 Hz, 1H), 7.02 (d, J = 7.3 Hz, 1H), 6.95 (d, J = 10.4 Hz, 1H), 6.54 (s, 1H), 5.03 (s, 2H), 4.23 (t, J = 8.5 Hz, 2H), 3.67 (s, 2H), 3.40 (t, J = 5.8 Hz, 2H), 3.25-3.16 (m, 5H), 2.65 (t, J = 5.5 Hz, 2H), 2.37-2.17 (m, 2H), 2.09-1.59 (m, 4H), 1.28-1.17 (m, 1H), 0.97 (d, J = 6.7 Hz, 3H). |
| 315 | 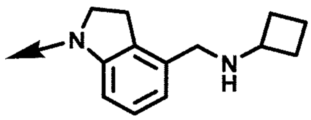 | $^1$H-NMR (400 MHz, DMSO-$d_6$) δ: 7.90-7.83 (m, 2H), 7.10 (t, J = 7.9 Hz, 1H), 7.00 (d, J = 7.3 Hz, 1H), 6.94 (d, J = 9.8 Hz, 1H), 6.54 (s, 1H), 5.03 (s, 2H), 4.23 (t, J = 8.2 Hz, 2H), 3.55 (s, 2H), 3.22-3.11 (m, 3H), 2.36-2.04 (m, 5H), 1.85-1.49 (m, 7H), 1.28-1.18 (m, 1H), 0.97 (d, J = 6.7 Hz, 3H). |
| 316 | 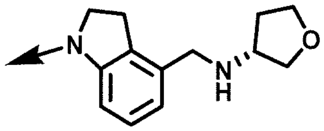 | $^1$H-NMR (400 MHz, DMSO-$d_6$) δ: 7.89-7.84 (m, 2H), 7.12 (t, J = 7.6 Hz, 1H), 7.03 (d, J = 7.3 Hz, 1H), 6.94 (d, J = 9.8 Hz, 1H), 6.53 (s, 1H), 5.03 (s, 2H), 4.24 (t, J = 8.2 Hz, 2H), 3.78-3.59 (m, 5H), 3.47-3.42 (m, 1H), 3.30-3.19 (m, 3H), 2.36-2.11 (m, 3H), 1.97-1.62 (m, 5H), 1.28-1.18 (m, 1H), 0.97 (d, J = 6.7 Hz, 3H). |
| 317 | 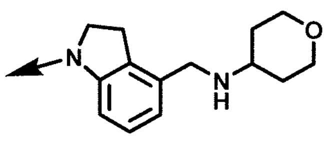 | $^1$H-NMR (400 MHz, DMSO-$d_6$) δ: 7.90-7.83 (m, 2H), 7.13-7.03 (m, 2H), 6.94 (d, J = 9.8 Hz, 1H), 6.53 (s, 1H), 5.03 (s, 2H), 4.24 (t, J = 8.2 Hz, 2H), 3.85-3.80 (m, 2H), 3.68 (s, 2H), 3.31-3.19 (m, 4H), 2.63-2.55 (m, 1H), 2.36-2.18 (m, 2H), 1.96-1.62 (m, 6H), 1.33-1.18 (m, 3H), 0.97 (d, J = 6.1 Hz, 3H). |
| 318 | 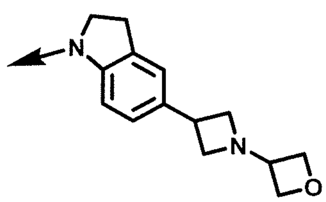 | $^1$H-NMR (400 MHz, $CDCl_3$) δ: 8.14-8.09 (br m, 1H), 7.56 (d, J = 9.8 Hz, 1H), 7.18-7.16 (br m, 1H), 7.08-7.04 (br m, 1H), 6.92 (d, J = 9.8 Hz, 1H), 6.33 (br s, 1H), 5.01 (s, 2H), 4.77-4.74 (m, 1H), 4.63-4.60 (m, 1H), 4.50-4.41 (m, 1H), 4.23-4.17 (m, 2H), 4.14-4.10 (m, 1H), 4.03 (s, 2H), 3.92-3.84 (m, 2H), 3.30-3.25 (m, 2H), 2.60-2.56 (br m, 1H), 2.34-2.26 (br m, 2H), 1.87-1.79 (br m, 2H), 1.74-1.65 (m, 1H), 1.30-1.24 (m, 3H), 0.99 (d, J = 6.7 Hz, 3H). |

(continued)

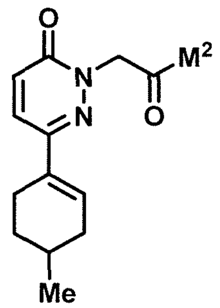

| Example | $M^2$ | Analytical data |
|---|---|---|
| 319 | 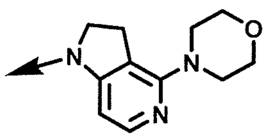 | $^1$H-NMR (400 MHz, $CDCl_3$) δ: 8.09-8.07 (br m, 1H), 7.69 (br s, 1H), 7.57 (d, J = 9.8 Hz, 1H), 6.92 (d, J = 9.8 Hz, 1H), 6.35-6.33 (br m, 1H), 5.00 (br s, 2H), 4.24-4.20 (br m, 2H), 3.83 (t, J = 4.9 Hz, 4H), 3.38-3.24 (br m, 6H), 2.61-2.54 (br m, 1H), 2.36-2.26 (br m, 2H), 1.88-1.78 (m, 2H), 1.75-1.66 (br m, 1H), 1.33-1.23 (m, 1H), 1.00 (d, J = 6.7 Hz, 3H). |
| 320 | 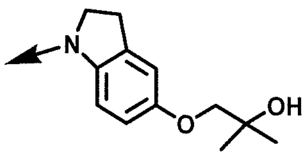 | $^1$H-NMR (400 MHz, $CDCl_3$) δ: 8.09 (d, J = 8.6 Hz, 1H), 7.55 (d, J = 9.8 Hz, 1H), 6.92 (d, J = 9.8 Hz, 1H), 6.79 (d, J = 2.4 Hz, 1H), 6.71 (dd, J = 8.6, 2.4 Hz, 1H), 6.33 (br s, 1H), 5.04-4.96 (m, 2H), 4.19 (t, J = 8.3 Hz, 2H), 3.76 (s, 2H), 3.25 (t, J = 8.3 Hz, 2H), 2.62-2.56 (br m, 1H), 2.36-2.27 (br m, 2H), 2.23 (s, 1H), 1.88-1.79 (m, 2H), 1.74-1.66 (br m, 1H), 1.33 (s, 6H), 1.31-1.22 (m, 1H), 1.00 (d, J = 6.7 Hz, 3H). |
| 321 | 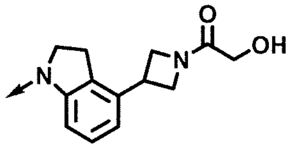 | LC-MS: $[M+H]^+$ / Rt (min) 463.1 / 0.799 (Method A) |
| 322 | 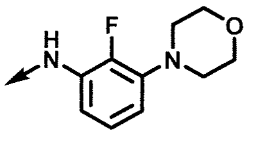 | $^1$H-NMR (400 MHz, $CDCl_3$) δ: 8.92 (s, 1H), 8.03-7.93 (m, 1H), 7.60 (d, J = 5.1 Hz, 1H), 7.06-7.00 (m, 1H), 6.98 (d, J = 5.1 Hz, 1H), 6.86-6.75 (m, 1H), 6.42-6.32 (m, 1H), 5.00 (d, J = 14.6 Hz, 1H), 4.95 (d, J = 14.6 Hz, 1H), 3.93-3.85 (m, 4H), 3.15-3.07 (m, 4H), 2.68-2.55 (m, 1H), 2.40-2.25 (m, 2H), 1.92-1.80 (m, 2H), 1.80-1.65 (m, 2H), 1.01 (d, J = 6.1 Hz, 3H). |
| 323 | 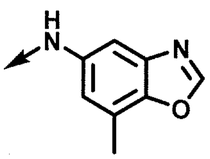 | $^1$H-NMR (400 MHz, $CDCl_3$) δ: 9.09 (s, 1H), 8.05 (s, 1H), 7.87-7.83 (m, 1H), 7.60 (d, J = 9.8 Hz, 1H), 7.29 (s, 1H), 6.98 (d, J = 9.8 Hz, 1H), 6.37 (s, 1H), 5.00 (s, 2H), 2.70-2.58 (m, 1H), 2.46 (s, 3H), 2.36-2.30 (m, 2H), 1.92-1.79 (m, 2H), 1.77-1.69 (m, 2H), 1.02 (d, J = 6.1 Hz, 3H). |
| 324 | 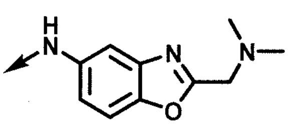 | $^1$H-NMR (400 MHz, $CDCl_3$) δ: 9.10 (s, 1H), 8.06 (s, 1H), 7.63 (t, J = 7.6 Hz, 1H), 7.48-7.44 (m, 2H), 7.01 (d, J = 9.8 Hz, 1H), 6.40 (s, 1H), 5.03 (s, 2H), 4.11-4.06 (m, 2H), 2.73-2.61 (m, 7H), 2.38-2.26 (m, 2H), 2.94-1.80 (m, 2H), 1.80-1.69 (m, 2H), 1.03 (d, J = 6.1 Hz, 3H). |
| 325 | 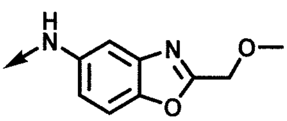 | $^1$H-NMR (400 MHz, $CDCl_3$) δ: 9.17 (s, 1H), 7.98 (s, 1H), 7.60 (d, J = 10.4 Hz, 1H), 7.45-7.35 (m, 2H), 6.98 (t, J = 10.7 Hz, 1H), 6.36 (s, 1H), 5.00 (s, 2H), 4.69 (s, 2H), 3.51 (s, 3H), 2.67-2.57 (m, 1H), 2.39-2.328 (m, 2H), 1.90-1.79 (m, 2H), 1.78-1.62 (m,, 1H), 1.33-1.21 (m, 1H), 1.00 (d, J = 6.7 Hz, 3H). |

(continued)

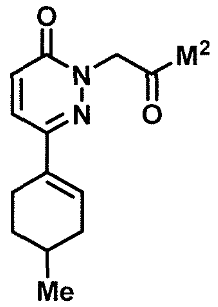

| Example | $M^2$ | Analytical data |
|---|---|---|
| 326 | 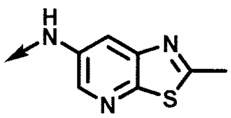 | $^1$H-NMR (400 MHz, $CDCl_3$) δ: 9.43 (s, 1H), 8.61-8.57 (m, 2H), 7.64 (d, J = 9.8 Hz, 1H), 7.02 (d, J = 9.8 Hz, 1H), 6.40 (s, 1H), 5.05 (s, 2H), 2.84 (s, 3H), 2.82 (s, 1H), 2.70-2.60 (m, 1H), 2.43-2.29 (m, 2H), 1.92-1.83 (m, 2H), 1.65-1.75 (m, 1H), 1.38-1.25 (m, 1H), 1.01 (t, J = 7.3 Hz, 3H). |
| 327 | 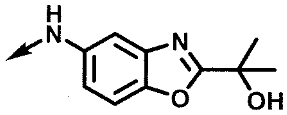 | $^1$H-NMR (400 MHz, $CDCl_3$) δ: 9.16 (s, 1H), 7.90 (s, 1H), 7.60 (d, J = 7.0 Hz, 1H), 7.46-7.39 (m, 2H), 7.29-7.37 (m, 1H), 6.36 (s, 1H), 5.00 (s, 2H), 2.38-2.25 (m, 3H), 1.90-1.79 (m, 3H), 1.75 (s, 1H), 1.73 (t, 6H), 0.99 (t, J = 7.3 Hz, 3H). |
| 328 | 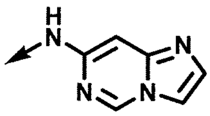 | $^1$H-NMR (400 MHz, $CD_3OD$) δ: 9.13 (s, 1H), 8.17 (s, 1H), 7.85 (d, J = 9.2 Hz, 2H), 7.53 (s, 1H), 6.95 (d, J = 9.8 Hz, 1H), 6.51 (s, 1H), 5.02 (s, 2H), 2.67-2.57 (m, 1H), 2.42-2.28 (m, 2H), 1.90-1.80 (m, 2H), 1.80-1.65 (s, 1H), 1.35-1.25 (m, 1H), 1.01 (d, J = 6.7 Hz, 3H). |
| 329 | 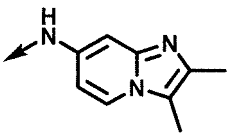 | $^1$H-NMR (400 MHz, $CD_3OD$) δ: 7.98 (d, J = 7.3 Hz, 1H), 7.85-7.80 (m, 2H), 7.09-7.02 (m, 1H), 6.93 (d, J = 9.8 Hz, 1H), 6.53-6.45 (m, 1H), 4.97 (s, 2H), 2.67-2.55 (m, 1H), 2.36 (s, 3H), 2.36-2.26 (m, 2H), 2.30 (s, 3H), 1.87-1.80 (m, 2H), 1.73-1.68 (m, 1H), 0.98 (t, J = 7.0 Hz, 3H). |
| 330 | 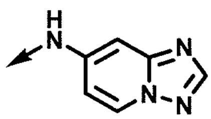 | $^1$H-NMR (400 MHz, $CDCl_3$) δ: 9.98 (s, 1H), 8.32 (d, J = 4.3 Hz, 1H), 8.23 (s, 1H), 8.01 (s, 1H), 7.63 (d, J = 9.8 Hz, 1H), 7.09 (dd, J = 7.3, 2.4 Hz, 1H), 6.98 (d, J = 9.8 Hz, 1H), 6.40 (s, 1H), 5.03 (s, 2H), 2.65-2.55 (m, 1H), 2.40-2.25 (m, 2H), 1.91-1.80 (m, 2H), 1.80-1.65 (m, 1H), 1.34-1.20 (m, 1H), 1.00 (d, J = 6.7 Hz, 3H). |
| 331 | 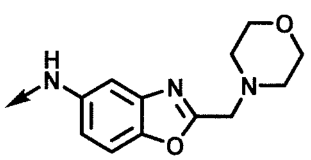 | $^1$H-NMR (400 MHz, $CDCl_3$) δ: 9.12 (s, 1H), 7.94 (s, 1H), 7.59 (d, J = 9.8 Hz, 1H), 7.41-7.34 (m, 2H), 6.97 (d, J = 9.8 Hz, 1H), 6.36 (s, 1H), 4.99 (s, 2H), 3.83 (s, 2H), 3.78-3.74 (m, 4H), 2.68-2.58 (m, 5H), 2.38-2.28 (m, 2H), 1.90-1.81 (m, 2H), 1.75-1.65 (m, 1H), 1.35-1.26 (m, 1H), 0.99 (t, J = 8.6 Hz, 3H). |
| 332 | 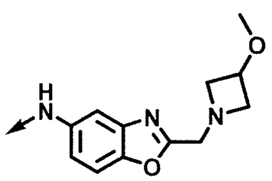 | $^1$H-NMR (400 MHz, $CDCl_3$) δ: 9.08 (s, 1H), 7.93 (s, 1H), 7.59 (d, J = 9.8 Hz, 1H), 7.41-7.33 (m, 2H), 6.98 (d, J = 7.9 Hz, 1H), 6.36 (s, 1H), 4.98 (s, 2H), 4.17-4.05 (m, 2H), 3.92-3.88 (m, 2H), 3.85-3.76 (m, 2H), 3.24 (s, 3H), 3.21-3.11 (m, 2H), 2.68-2.57 (m, 1H), 2.38-2.29 (m, 2H), 1.90-1.79 (m, 2H), 1.78-1.60 (m, 1H), 0.98 (d, J = 6.4 Hz, 3H). |

(continued)

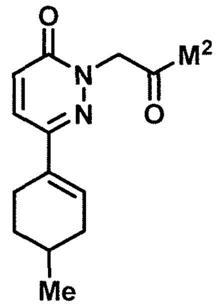

| Example | $M^2$ | Analytical data |
|---|---|---|
| 333 | 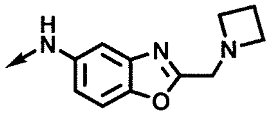 | $^1$H-NMR (400 MHz, $CD_3OD$) δ: 8.14 (d, J = 1.8 Hz, 1H), 7.84 (d, J = 9.8 Hz, 1H), 7.59 (d, J = 8.6 Hz, 1H), 7.49 (dd, J = 8.9, 2.1 Hz, 1H), 6.93 (d, J = 9.5 Hz, 1H), 6.50 (s, J = 3.1 Hz, 1H), 4.97 (s, 2H), 4.83 (s, 2H), 4.50-4.40 (m, 2H), 4.40-4.29 (m, 2H), 3.32-3.28 (m, 5H), 2.70-2.45 (m, 3H), 1.89-1.878 (m, 2H), 1.35-1.26 (m, 1H), 1.00 (d, J = 6.7 Hz, 3H). |
| 334 | 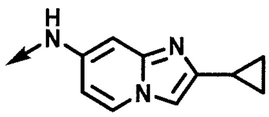 | $^1$H-NMR (400 MHz, $CDCl_3$) δ: 10.11 (s, 1H), 7.72 (d, J = 11.3 Hz, 1H), 7.61 (s, 1H), 7.54 (d, J = 9.2 Hz, 1H), 7.11 (s, 1H), 7.06-7.02 (m, 1H), 6.89 (d, J = 4.9 Hz, 1H), 6.30 (s, 1H), 4.95 (s, 2H), 2.65-2.41 (m, 2H), 2.35-2.20 (m, 2H), 2.00-1.91 (m, 1H), 1.86-1.75 (m, 2H), 1.75-1.65 (m, 1H), 0.98 (d, J = 6.7 Hz, 3H), 0.94-0.83 (m, 4H). |
| 335 | 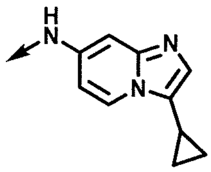 | $^1$H-NMR (400 MHz, $CDCl_3$) δ: 10.06 (s, 1H), 7.97 (d, J = 7.3 Hz, 1H), 7.82 (s, 1H), 7.65-7.56 (m, 1H), 7.25-7.15 (m, 2H), 6.99-6.93 (m, 1H), 6.35 (s, 1H), 5.05 (s, 2H), 2.65-2.56 (m, 1H), 2.50-2.22 (m, 3H), 1.90-1.60 (m, 3H), 1.34-1.20 (m, 1H), 0.98 (d, J = 5.8 Hz, 3H), 1.02-0.92 (m, 1H), 0.88 (t, J = 6.7 Hz, 1H), 0.69-0.62 (m, 2H). |
| 336 | 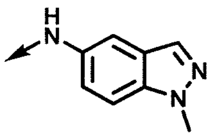 | $^1$H-NMR (400 MHz, $CDCl_3$) δ: 8.93 (s, 1H), 8.04 (d, J = 1.2 Hz, 1H), 7.91 (s, 1H), 7.61 (d, J = 9.8 Hz, 1H), 7.41-7.38 (m, 1H), 7.29 (d, J = 8.5 Hz, 1H), 6.99 (d, J = 9.8 Hz, 1H), 6.38 (s, 1H), 4.98 (s, 2H), 2.70-2.59 (m, 1H), 2.41-2.28 (m, 2H), 1.93-1.79, 2H), 1.55-1.42 (m, 4H), 1.38-1.23 (m, 1H), 1.01 (d, J = 6.7 Hz, 3H). |
| 337 | 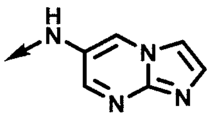 | $^1$H-NMR (400 MHz, $CDCl_3$) δ: 11.01 (s, 1H), 9.45 (s, 1H), 8.57 (s, 1H), 7.69 (s, 1H), 7.57 (d, J = 9.1 Hz, 1H), 7.43 (s, 1H), 6.94 (d, J = 9.8 Hz, 1H), 6.33 (s, 1H), 5.14 (s, 2H), 2.63-2.51 (m, 1H), 2.38-2.25 (m, 2H), 1.90-1.75 (m, 2H), 1.75-1.60 (m, 1H), 1.30-1.21 (m, 1H), 0.98 (d, J = 6.1 Hz, 3H). |
| 338 | 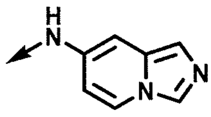 | $^1$H-NMR (400 MHz, $CDCl_3$) δ: 9.52 (s, 1H), 7.94 (s, 1H), 7.79 (s, 1H), 7.68 (d, J = 7.3 Hz, 1H), 7.61 (d, J = 4.9 Hz, 1H), 7.19 (s, 1H), 6.96 (d, J = 9.8 Hz, 1H), 6.56 (dd, J = 7.6, 2.1 Hz, 1H), 6.37 (s, 1H), 4.99 (s, 2H), 2.67-2.56 (m, 1H), 2.39-2.25 (m, 2H), 2.25-2.08 (m, 1H), 1.89-1.78 (m, 2H), 1.35-1.21(m, 1H), 0.99 (d, J = 6.7 Hz, 3H). |
| 339 | 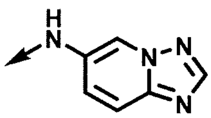 | $^1$H-NMR (400 MHz, $CDCl_3$) δ: 9.65 (s, 1H), 9.48 (s, 1H), 8.31 (s, 1H), 7.67 (dd, J = 13.7, 9.5 Hz, 2H), 7.41-7.35 (m, 1H), 7.01 (d, J = 9.8 Hz, 1H), 6.40 (s, 1H), 5.03 (s, 2H), 2.68-2.57 (m, 1H), 2.40-2.31 (m, 2H), 1.93-1.83 (m, 2H), 1.35-1.21 (m, 1H), 1.01 (d, J = 6.7 Hz, 3H). |

(continued)

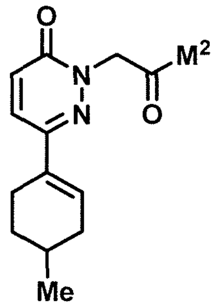

| Example | $M^2$ | Analytical data |
|---|---|---|
| 340 | 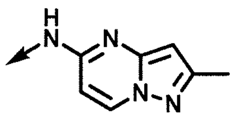 | $^1$H-NMR (400 MHz, $CDCl_3$) δ: 8.99 (s, 1H), 8.46 (d, J = 7.3 Hz, 1H), 7.75 (d, J = 6.7 Hz, 1H), 7.59 (d, J = 9.8 Hz, 1H), 6.98 (d, J = 9.8 Hz, 1H), 6.38 (s, 1H), 6.18 (s, 1H), 4.99 (s, 2H), 2.65-2.56 (m, 1H), 2.46 (s, 3H), 2.40-2.28 (m, 2H), 1.89-1.80 (m, 1H), 1.78-1.65 (m, 2H), 1.35-1.20 (m, 1H), 1.01 (d, J = 6.7 Hz, 3H). |
| 341 | 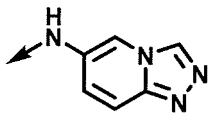 | $^1$H-NMR (400 MHz, $CDCl_3$) δ: 10.90-10.70 (m, 1H), 9.48-9.35 (m, 1H), 9.15-9.00 (m, 1H), 7.95-7.50 (m, H), 7.61 (d, J = 9.8 Hz, 1H), 6.98 (d, J = 9.8 Hz, 1H), 6.36 (s, 1H), 5.12 (s, 2H), 2.65-2.40 (m, 2H), 2.39-2.23 (m, 2H), 1.90-1.79 (m, 1H), 1.75-1.65 (m, 1H), 0.99 (d, J = 6.1 Hz, 3H). |
| 342 | 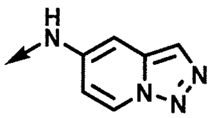 | $^1$H-NMR (400 MHz, $CDCl_3$) δ: 9.67 (s, 1H), 8.58 (d, J = 7.9 Hz, 1H), 8.19 (d, J = 1.2 Hz, 1H), 7.90 (s, 1H), 7.64 (t, J = 8.2 Hz, 1H), 7.01 (d, J = 9.8 Hz, 1H), 6.93 (dd, J = 7.6, 2.1 Hz, 1H), 6.40 (s, 1H), 5.01 (s, 2H), 2.70-2.55 (m, 1H), 2.40-2.25 (m, 2H), 1.90-1.64 (m, 2H), 1.35-1.26 (m, 1H), 1.02 (t, J = 8.5 Hz, 5H). |
| 343 | 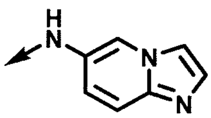 | $^1$H-NMR (400 MHz, $CDCl_3$) δ: 9.70 (s, 1H), 9.11 (s, 1H), 7.64-7.52 (m, 3H), 7.47 (d, J = 9.2 Hz, 1H), 7.08-6.95 (m, 2H), 6.38 (s, 1H), 5.03 (s, 2H), 2.70-2.55 (m, 1H), 2.40-2.28 (m, 1H), 2.25-2.10 (m, 2H), 1.93-1.78 (m, 1H), 1.78-1.65 (m, 1H), 1.35-1.21 (m, 1H), 1.01 (d, J = 6.7 Hz, 3H). |
| 344 | 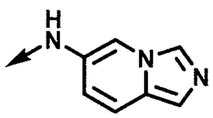 | $^1$H-NMR (400 MHz, $CDCl_3$) δ: 9.96 (s, 1H), 9.02 (s, 1H), 8.49 (s, 1H), 7.60 (d, J = 9.8 Hz, 1H), 7.47 (s, 1H), 7.33 (d, J = 9.8 Hz, 1H), 6.98 (d, J = 9.5 Hz, 1H), 6.80 (d, J = 9.2 Hz, 1H), 6.35 (s, 1H), 5.08 (s, 2H), 2.65-2.53 (m, 1H), 2.40-2.25 (m, 2H), 1.90-1.78 (m, 2H), 1.73-1.65 (m, 1H), 1.33-1.25 (m, 1H), 1.01 (d, J = 6.7 Hz, 3H). |
| 345 | 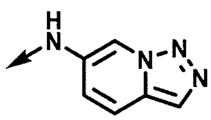 | $^1$H-NMR (400 MHz, $CD_3OD$) δ: 9.61 (s, 1H), 8.08 (s, 1H), 7.86 (d, J = 4.9 Hz, 2H), 7.32 (d, J = 7.9 Hz, 1H), 6.96 (d, J = 9.8 Hz, 1H), 6.53 (s, 1H), 5.01 (s, 2H), 2.65-2.51 (m, 1H), 2.42-2.30 (m, 2H), 1.90-1.75 (m, 2H), 1.75-1.65 (m, 1H), 1.40-1.25 (m, 1H), 1.01 (d, J = 6.7 Hz, 3H). |
| 346 | 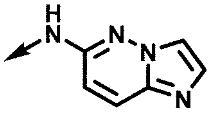 | $^1$H-NMR (400 MHz, $CD_3OD$) δ: 9.31 (s, 1H), 8.04 (d, J = 9.8 Hz, 1H), 7.91 (d, J = 9.8 Hz, 1H), 7.79 (s, 1H), 7.70 (d, J = 1.2 Hz, 1H), 7.62 (d, J = 9.8 Hz, 1H), 7.01 (d, J = 9.8 Hz, 1H), 6.39 (s, 1H), 5.02 (s, 2H), 2.70-2.58 (m, 1H), 2.45-2.30 (m, 2H), 1.95-1.83 (m, 2H), 1.80-1.65 (m, 1H), 1.35-1.20 (m, 1H), 1.01 (d, J = 6.7 Hz, 3H). |

(continued)

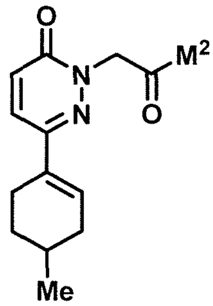

| Example | $M^2$ | Analytical data |
|---|---|---|
| 347 | 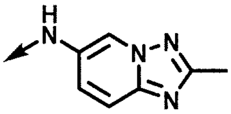 | $^1H$-NMR (400 MHz, $CDCl_3$) $\delta$: 9.53 (s, 1H), 9.30 (s, 1H), 7.64 (d, J = 9.8 Hz, 1H), 7.46 (dd, J = 9.4, 2.7 Hz, 1H), 7.21 (d, J = 9.8 Hz, 1H), 6.99 (d, J = 9.8 Hz, 1H), 6.39 (s, 1H), 5.01 (s, 2H), 2.68-2.58 (m, 1H), 2.54 (s, 3H), 2.40-2.38 (m, 2H), 1.92-1.80 (m, 2H), 1.80-1.65 (m, 1H), 1.35-1.21 (m, 1H), 1.01 (d, J = 6.7 Hz, 3H). |
| 348 | 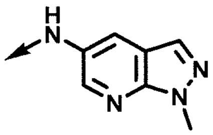 | $^1H$-NMR (400 MHz, $CDCl_3$) $\delta$: 9.18 (s, 1H), 8.48 (d, J = 1.8 Hz, 1H), 8.41 (d, J = 2.4 Hz, 1H), 7.90 (s, 1H), 7.63 (d, J = 9.8 Hz, 1H), 7.00 (d, J = 9.8 Hz, 1H), 6.39 (s, 1H), 5.02 (s, 2H), 4.11 (s, 3H), 2.70-2.59 (m, 1H), 2.40-2.38 (m, 2H), 1.90-1.80 (m, 2H), 1.79-1.63 (m, 1H), 1.36-1.24 (m, 1H), 1.01 (d, J = 6.7 Hz, 3H). |
| 349 | 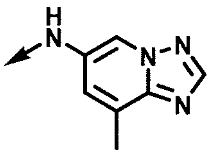 | $^1H$-NMR (400 MHz, $CDCl_3$) $\delta$: 9.46 (s, 1H), 9.29 (s, 1H), 8.23 (s, 1H), 7.65 (d, J = 9.8 Hz, 1H), 7.04 (s, 1H), 7.01 (d, J = 9.8 Hz, 1H), 6.40 (s, 1H), 5.01 (s, 2H), 2.68-2.58 (m, 1H), 2.56 (s, 3H), 2.40-2.38 (m, 2H), 1.90-1.79 (m, 2H), 1.78-1.64 (m, 1H), 1.38-1.29 (m, 1H), 1.01 (d, J = 6.7 Hz, 3H). |
| 350 | 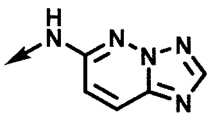 | $^1H$-NMR (400 MHz, $CDCl_3$) $\delta$: 9.67 (s, 1H), 8.50 (d, J = 9.8 Hz, 1H), 8.41 (d, J = 15.2 Hz, 1H), 8.09 (d, J = 9.8 Hz, 1H), 7.61 (d, J = 9.8 Hz, 1H), 7.00 (d, J = 9.8 Hz, 1H), 6.38 (d, J = 2.4 Hz, 1H), 5.11 (s, 2H), 2.65-2.55 (m, 1H), 2.40-2.25 (m, 2H), 1.93-1.81 (m, 2H), 1.75-1.60 (m, 1H), 1.35-1.22 (m, 1H), 1.00 (d, J = 6.4 Hz, 3H). |
| 351 | 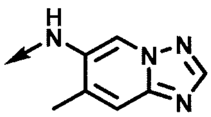 | $^1H$-NMR (400 MHz, $CDCl_3$) $\delta$: 9.52 (s, 1H), 9.15 (s, 1H), 8.23 (s, 1H), 7.61 (d, J = 9.8 Hz, 1H), 7.50 (s, 1H), 6.99 (d, J = 9.8 Hz, 1H), 6.38 (s, 1H), 5.04 (s, 2H), 2.68-2.57 (m, 1H), 2.40 (s, 3H), 2.40-2.30 (m, 2H), 1.91-1.80 (m, 2H), 1.80-1.65 (m, 1H), 1.35-1.25 (m, 1H), 1.01 (d, J = 6.7 Hz, 3H). |
| 352 | 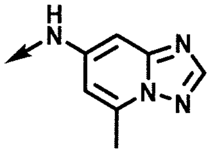 | $^1H$-NMR (400 MHz, $CDCl_3$) $\delta$: 9.41 (s, 1H), 8.25 (s, 1H), 7.90 (s, 1H), 7.61 (d, J = 10.4 Hz, 1H), 6.99 (d, J = 4.9 Hz, 2H), 6.38 (s, 1H), 4.99 (s, 2H), 2.73 (s, 3H), 2.68-2.58 (m, 1H), 2.43-2.30 (m, 2H), 1.93-1.82 (m, 2H), 1.78-1.72 (m, 1H), 1.38-1.25 (m, 1H), 1.02 (d, J = 6.7 Hz, 3H). |
| 353 | 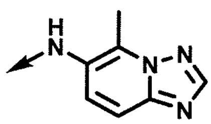 | $^1H$-NMR (400 MHz, $CDCl_3$) $\delta$: 8.82 (s, 1H), 8.33 (s, 1H), 7.81 (d, J = 9.8 Hz, 1H), 7.63 (dd, J = 13.1, 9.4 Hz, 2H), 7.01 (d, J = 9.8 Hz, 1H), 6.40 (s, 1H), 5.05 (s, 2H), 2.73 (s, 3H), 2.68-2.58 (m, 1H), 2.40-2.25 (m, 2H), 1.93-1.80 (m, 2H), 1.79-1.65 (,m 1H), 1.34-1.21 (m, 1H), 1.01 (d, J = 6.7 Hz, 3H). |

(continued)

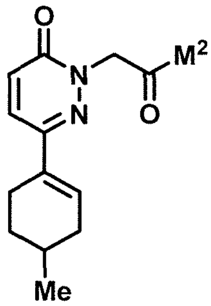

| Example | $M^2$ | Analytical data |
|---|---|---|
| 354 | 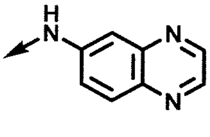 | $^1$H-NMR (400 MHz, $CDCl_3$) δ: 9.99 (s, 1H), 8.68 (s, 1H), 8.63 (s, 1H), 8.29 (t, J = 7.6 Hz, 1H), 7.83-7.78 (m, 2H), 7.61-7.55 (m, 1H), 7.00-6.94 (m, 1H), 6.32 (s, 1H), 5.10 (s, 2H), 2.65-2.53 (m, 1H), 2.33-2.22 (m, 2H), 1.90-1.78 (m, 2H), 1.78-1.60 (m, 1H), 1.31-1.20 (m, 1H), 1.00 (d, J = 6.5 Hz, 3H). |
| 355 | 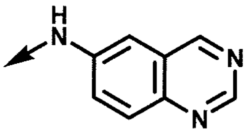 | $^1$H-NMR (400 MHz, $CDCl_3$) δ: 10.00 (s, 1H), 9.10 (d, J = 3.7 Hz, 1H), 8.97 (t, J = 4.3 Hz, 1H), 8.22 (s, 1H), 7.77 (d, J = 6.7 Hz, 1H), 7.71-7.60 (m, 2H), 7.02 (dd, J = 9.8, 3.7 Hz, 1H), 6.39 (s, 1H), 5.14 (s, 2H), 2.68-2.57 (m, 1H), 2.40-2.31 (m, 2H), 1.95-1.90 (m, 2H), 1.80-1.65 (m, 1H), 1.35-1.21 (m, 1H), 1.02 (d, J = 6.0 Hz, 3H). |
| 356 | 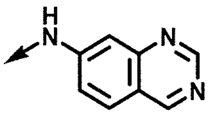 | $^1$H-NMR (400 MHz, $CDCl_3$) δ: 9.59 (s, 1H), 9.27 (s, 2H), 8.28 (s, 1H), 7.85 (q, J = 6.7 Hz, 2H), 7.65 (d, J = 9.8 Hz, 1H), 7.03 (d, J = 9.8 Hz, 1H), 6.41 (s, 1H), 5.05 (s, 2H), 2.70-2.60 (m, 1H), 2.41-2.30 (m, 2H), 1.95-1.83 (m, 2H), 1.80-1.68 (m, 1H), 1.35-1.21 (m, 1H), 1.03 (d, J = 6.1 Hz, 3H). |
| 357 | 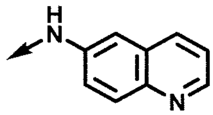 | $^1$H-NMR (400 MHz, $CDCl_3$) δ: 9.65 (s, 1H), 8.93 (s, 1H), 8.40 (d, J = 8.5 Hz, 1H), 8.20 (d, J = 7.3 Hz, 1H), 7.94 (d, J = 7.9 Hz, 1H), 7.72-7.64 (m, 2H), 7.48 (s, 1H), 7.06 (d, J = 9.8 Hz, 1H), 6.41 (s, 1H), 5.12 (s, 2H), 2.73-2.60 (m, 1H), 2.43-2.30 (m, 2H),1.93-1.83 (m, 2H), 1.80-1.70 (m, 1H), 1.37-1.22 (m, 1H), 1.05 (d, J = 6.0 Hz, 3H). |
| 358 | 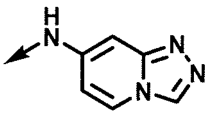 | $^1$H-NMR (400 MHz, DMSO-$d_6$) δ: 10.78 (s, 1 H), 9.11 (s, 1 H), 8.54-8.44 (m, 1 H), 8.09-8.02 (m, 1 H), 7.93-7.82 (m, 1 H), 7.01-6.89 (m, 1 H), 6.54 (s, 1 H), 4.91 (s, 2 H), 2.35-2.16 (m, 2 H), 1.91-1.75 (m, 2 H), 1.70-1.60 (m, 1 H), 1.29-1.16 (m, 1 H), 0.99 (d, J = 8.0 Hz, 3H), |
| 359 | 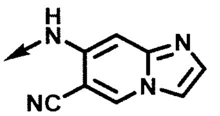 | $^1$H-NMR (400 MHz, $CDCl_3$) δ: 9.16 (s, 1H), 8.60 (s, 1H), 8.49 (s, 1H), 7.71 (d, J = 1.2 Hz, 1H), 7.63 (d, J = 9.8 Hz, 1H), 7.57 (s, 1H), 7.04 (d, J = 9.8 Hz, 1H), 6.40 (s, 1H), 5.10 (s, 2H), 2.68-2.59 (m, 1H), 2.41-2.28 (m, 2H), 1.93-1.80 (m, 2H), 1.65-1.78 (m, 1H), 1.39-1.22 (m, 1H), 1.01 (d, J = 6.7 Hz, 3H). |
| 360 | 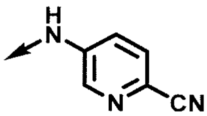 | $^1$H-NMR (400 MHz, $CDCl_3$) δ: 9.77 (s, 1H), 8.62 (d, J = 2.4 Hz, 1H), 8.29 (dd, J = 8.5, 2.4 Hz, 1H), 7.67-7.58 (m, 2H), 7.01 (d, J = 9.8 Hz, 1H), 6.40 (s, 1H), 5.01 (s, 2H), 2.68-2.57 (m, 1H), 2.41-2.27 (m, 2H), 1.93-1.80 (m, 2H), 1.80-1.65 (m, 1H), 1.35-1.21 (m, 1H), 1.01 (d, J = 6.7 Hz, 3H). |

(continued)

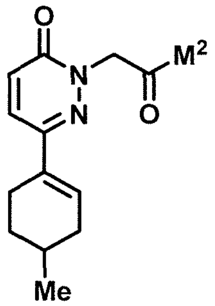

| Example | $M^2$ | Analytical data |
|---|---|---|
| 361 | 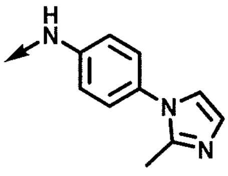 | $^1$H-NMR (400 MHz, $CDCl_3$) δ: 9.42 (s, 1H), 7.69 (d, J = 8.6 Hz, 2H), 7.62 (d, J = 9.8 Hz, 1H), 7.21 (d, J = 8.6 Hz, 2H), 7.09 (s, 1H), 7.10-6.90 (m, 2H), 6.38 (s, 1H), 5.00 (s, 2H), 2.68-2.58 (m, 1H), 2.40-2.25 (m, 2H), 1.95-1.80 (m, 2H), 1.80-1.60 (m, 1H), 1.31-1.23 (m, 1H), 1.01 (d, J = 6.7 Hz, 3H). |
| 362 | 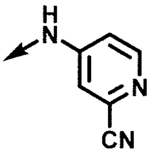 | $^1$H-NMR (400 MHz, $CDCl_3$) δ: 9.90 (s, 1H), 8.51 (d, J = 6.1 Hz, 1H), 7.93 (d, J = 1.8 Hz, 1H), 7.66 (d, J = 9.8 Hz, 1H), 7.60-7.56 (m, 1H), 7.01 (d, J = 9.8 Hz, 1H), 6.41 (s, 1H), 4.99 (s, 2H), 2.67-2.56 (m, 1H), 2.41-2.28 (m, 2H), 1.93-1.81 (m, 2H), 1.80-1.65 (m, 1H), 1.32-1.25 (m, 1H), 1.01 (d, J = 6.7 Hz, 3H). |
| 363 | 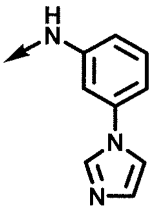 | $^1$H-NMR (400 MHz, $CDCl_3$) δ: 9.79 (s, 1H), 8.29 (s, 1H), 7.83 (s, 1H), 7.63 (d, J = 9.8 Hz, 1H), 7.46 (d, J = 7.9 Hz, 1H), 7.32 (t, J = 7.9 Hz, 1H), 7.28 (s, 1H), 7.23 (s, 1H), 7.09 (d, J = 7.9 Hz, 1H), 6.97 (d, J = 9.8 Hz, 1H), 6.41-6.35 (m, 1H), 5.06 (s, 2H), 2.67-2.56 (m, 1H), 2.41-2.28 (m, 2H), 1.90-1.80 (m, 2H), 1.79-1.65 (m, 1H), 1.33-1.23 (m, 1H), 1.00 (d, J = 6.7 Hz, 3H). |
| 364 | 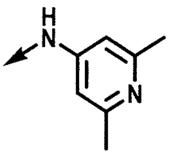 | $^1$H-NMR (400 MHz, $CDCl_3$) δ: 9.35 (s, 1H), 7.63 (d, J = 9.8 Hz, 1H), 7.18 (s, 2H), 7.00 (d, J = 9.8 Hz, 1H), 6.40 (s, 1H), 4.97 (s, 2H), 2.67-2.58 (m, 1H), 2.50 (s, 6H), 2.41-2.30 (m, 2H), 1.93-1.83 (m, 2H), 1.79-1.65 (m, 1H), 1.35-1.22 (m, 1H), 1.02 (d, J = 6.7 Hz, 3H). |
| 365 | 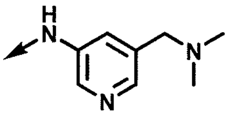 | $^1$H-NMR (400 MHz, $CDCl_3$) δ: 9.20 (s, 1H), 8.61 (s, 1H), 8.25 (s, 1H), 7.99 (s, 1H), 7.60 (d, J = 9.8 Hz, 1H), 6.97 (d, J = 9.8 Hz, 1H), 6.37 (s, 1H), 4.99 (s, 2H), 3.44 (s, 2H), 2.66-2.55 (m, 1H), 2.38-2.28 (m, 2H), 2.25 (s, 6H), 2.15-2.00 (m, 1H), 1.87-1.81 (m, 1H), 1.78-1.65 (s, 1H), 1.33-1.25 (m, 1H), 1.00 (d, J = 6.7 Hz, 3H). |
| 366 | 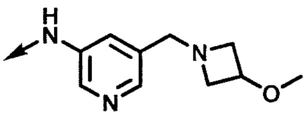 | $^1$H-NMR (400 MHz, $CDCl_3$) δ: 9.07 (s, 1H), 8.55 (d, J = 2.4 Hz, 1H), 8.23 (d, J = 1.8 Hz, 1H), 7.97 (d, J = 9.2 Hz, 1H), 7.61 (d, J = 9.8 Hz, 1H), 6.98 (d, J = 9.8 Hz, 1H), 6.38 (s, 1H), 4.99 (s, 2H), 3.63-3.53 (m, 4H), 3.25 (s, 3H), 2.97-2.93 (m, 2H), 2.67-2.56 (m, 1H), 2.36-2.31 (m, 2H), 2.15-2.00 (m, 1H), 1.90-1.75 (m, 1H), 1.78-1.66 (m, 1H), 1.38-1.25 (m, 1H), 1.01 (d, J = 6.7 Hz, 3H). |

(continued)

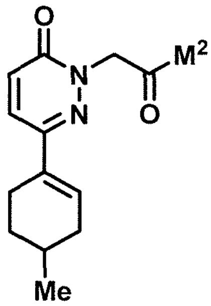

| Example | $M^2$ | Analytical data |
|---|---|---|
| 367 | 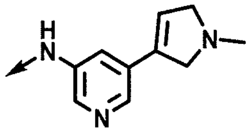 | $^1$H-NMR (400 MHz, $CDCl_3$) δ: 9.38 (s, 1H), 8.44 (s, 1H), 8.26 (s, 1H), 8.02 (s, 1H), 7.62 (d, J = 9.8 Hz, 1H), 6.98 (d, J = 9.8 Hz, 1H), 6.38 (s, 1H), 6.20 (s, 1H), 4.99 (s, 2H), 3.90 (s, 2H), 3.76 (s, 2H), 2.68-2.55 (m, 4H), 2.40-2.25 (m, 2H), 1.91-1.80 (m, 2H), 1.75-1.66 (m, 1H), 1.34-1.20 (m, 1H), 1.00 (d, J = 6.1 Hz, 3H). |
| 368 | 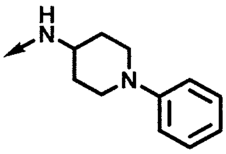 | LC-MS: $[M+H]^+$ / Rt (min) 407.4 / 1.79 (Method C) |
| 369 | 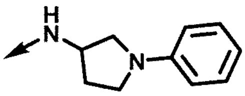 | LC-MS: $[M+H]^+$ / Rt (min) 393.4 / 1.82 (Method C) |
| 370 | 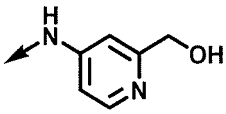 | $^1$H-NMR (400 MHz, $CDCl_3$) δ: 9.44 (s, 1H), 8.36 (d, J = 5.5 Hz, 1H), 7.63 (d, J = 9.8 Hz, 1H), 7.41 (s, 1H), 7.29 (dd, J = 5.8, 2.1 Hz, 1H), 6.99 (t, J = 4.9 Hz, 1H), 6.39 (s, 1H), 4.98 (s, 2H), 4.67 (s, 2H), 3.75-3.65 (m, 1H), 2.67-2.57 (m, 1H), 2.40-2.28 (m, 2H), 1.93-1.78 (m, 2H), 1.77-1.65 (m, 1H), 1.34-1.21 (m, 1H), 1.01 (q, J = 6.3 Hz, 3H). |
| 371 | 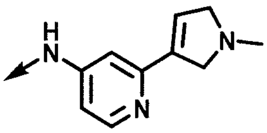 | $^1$H-NMR (400 MHz, $CDCl_3$) δ: 9.49 (s, 1H), 8.35 (d, J = 5.5 Hz, 1H), 7.63 (d, J = 10.4 Hz, 1H), 7.52 (s, 1H), 7.30-7.23 (m, 1H), 6.99 (d, J = 9.8 Hz, 1H), 6.45-6.33 (m, 2H), 4.97 (s, 2H), 4.01 (d, J = 1.8 Hz, 2H), 3.81 (s, 2H), 2.70-2.55 (m, 4H), 2.40-2.25 (m, 2H), 1.93-1.80 (m, 2H), 1.80-1.65 (m, 1H), 1.35-1.20 (m, 1H), 1.01 (d, J = 6.7 Hz, 3H). |
| 372 | 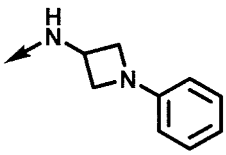 | LC-MS: $[M+H]^+$ / Rt (min) 379.1 / 1.83 (Method C) |
| 373 | 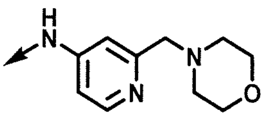 | $^1$H-NMR (400 MHz, $CDCl_3$) δ: 9.78 (s, 1H),8.27 (d, J = 5.5 Hz, 1H), 7.61 (dd, J = 10.1, 4.6 Hz, 1H), 7.47 (d, J = 1.8 Hz, 1H), 7.22-7.18 (m, 1H), 6.95 (t, J = 7.9 Hz, 1H), 6.35 (s, 1H), 4.97 (q, J = 12.8 Hz, 2H), 3.70-3.64 (m, 4H), 3.48 (s, 2H), 2.60-2.53 (m, 1H), 2.48-2.38 (m, 4H), 2.37-2.26 (m, 2H), 1.86-1.79 (m, 2H), 1.74-1.62 (m, 1H), 1.35-1.22 (m, 1H), 0.96 (d, J = 5.8 Hz, 3H). |

(continued)

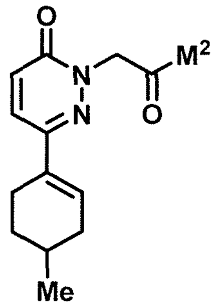

| Example | $M^2$ | Analytical data |
|---|---|---|
| 374 | 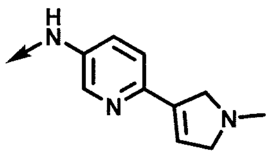 | $^1$H-NMR (400 MHz, $CDCl_3$) δ: 9.74 (s, 1H), 8.45 (s, 1H), 8.07 (d, J = 8.6 Hz, 1H), 7.62 (dd, J = 9.5, 3.4 Hz, 1H), 7.00-6.94 (m, 2H), 6.37 (s, 1H), 6.27 (s, 1H), 4.99 (s, 2H), 4.44 (s, 2H), 4.29 (s, 2H), 2.97 (s, 3H), 2.67-2.57 (m, 1H), 2.40-2.25 (m, 2H), 1.93-1.78 (m, 2H), 1.79-1.65 (m, 1H), 1.35-1.21 (m, 1H), 1.01 (d, J = 6.1 Hz, 3H). |
| 375 | 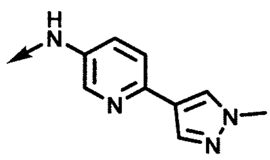 | $^1$H-NMR (400 MHz, $CDCl_3$) δ: 9.13 (s, 1H), 8.51 (d, J = 2.4 Hz, 1H), 8.04 (dd, J = 8.5, 2.4 Hz, 1H), 7.85 (d, J = 14.0 Hz, 2H), 7.62 (d, J = 9.8 Hz, 1H), 7.35 (d, J = 8.5 Hz, 1H), 6.99 (d, J = 9.8 Hz, 1H), 6.38 (s, 1H), 4.99 (s, 2H), 3.92 (s, 3H), 2.68-2.57 (m, 1H), 2.40-2.30 (m, 2H), 1.91-1.79 (m, 2H), 1.78-1.64 (m, 1H), 1.34-1.22 (m, 1H), 1.01 (d, J = 6.7 Hz, 3H). |
| 376 | 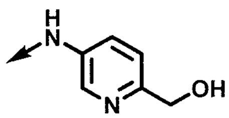 | $^1$H-NMR (400 MHz, $CDCl_3$) δ: 9.19 (s, 1H), 8.57 (d, J = 2.4 Hz, 1H), 7.98 (d, J = 9.1 Hz, 1H), 7.63 (d, J = 9.8 Hz, 1H), 7.18 (t, J = 7.6 Hz, 1H), 7.00 (t, J = 4.9 Hz, 1H), 6.39 (s, 1H), 4.99 (s, 2H), 4.75-4.65 (m, 2H), 3.49 (s, 1H), 2.69-2.52 (m, 1H), 2.40-2.28 (m, 2H), 1.93-1.79 (m, 2H), 1.78-1.63 (m, 1H), 1.35-1.21 (m, 1H), 1.01 (d, J = 6.1 Hz, 3H). |
| 377 | 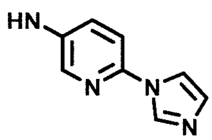 | $^1$H-NMR (400 MHz, $CDCl_3$) δ: 9.54 (s, 1H), 8.45 (s, 1H), 8.27-8.15 (m, 2H), 7.64 (d, J = 9.8 Hz, 1H), 7.56-7.41 (m, 1H), 7.25-7.18 (m, 1H), 7.17 (s, 1H), 7.00 (d, J = 9.8 Hz, 1H), 6.39 (s, 1H), 5.01 (s, 2H), 2.68-2.57 (m, 1H), 2.40-2.27 (m, 2H), 1.93-1.80 (m, 2H), 1.79-1.62 (m, 1H), 1.35-1.21 (m, 1H), 1.01 (d, J = 6.1 Hz, 3H). |
| 378 | 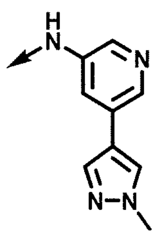 | $^1$H-NMR (400 MHz, $CDCl_3$) δ: 9.08 (s, 1H), 8.46 (d, J = 1.8 Hz, 1H), 8.39 (d, J = 2.4 Hz, 1H), 8.23 (t, J = 2.1 Hz, 1H), 7.77 (s, 1H), 7.66 (s, 1H), 7.62 (d, J = 9.8 Hz, 1H), 7.00 (d, J = 5.2 Hz, 1H), 6.39 (s, 1H), 4.99 (s, 2H), 3.94 (s, 3H), 2.67-2.51 (m, 1H), 2.40-2.29 (m, 2H), 1.90-1.80 (m, 2H), 1.78-1.65 (m, 1H) 1.35-1.21 (m, 1H), 1.01 (d, J = 6.7 Hz, 3H). |
| 379 | 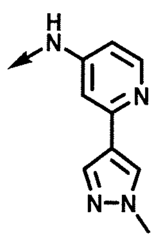 | $^1$H-NMR (400 MHz, $CDCl_3$) δ: 9.33 (s, 1H), 8.37 (d, J = 6.1 Hz, 1H), 7.91 (s, 1H), 7.85 (s, 1H), 7.68-7.60 (m,2H), 7.21-7.17 (m, 1H), 7.00 (d, J = 9.8 Hz, 1H), 6.38 (s, 1H), 4.98 (s, 2H), 3.92 (s, 3H), 2.67-2.56 (m, 1H), 2.40-2.26 (m, 2H), 1.90-1.80 (m, 2H), 1.75-.66 (m, 1H), 1.35-1.21 (m, 1H), 1.01 (d, J = 6.7 Hz, 3H). |

(continued)

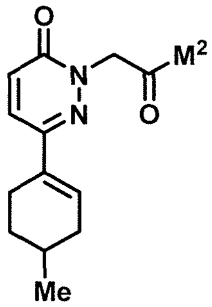

| Example | $M^2$ | Analytical data |
|---|---|---|
| 380 | 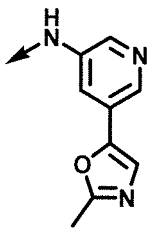 | $^1$H-NMR (400 MHz, $CDCl_3$) δ: 9.33 (s, 1H), 8.54 (s, 1H), 8.46 (s, 1H), 8.34 (s, 1H), 7.63 (d, J = 9.8 Hz, 1H), 7.27 (s, 1H), 7.00 (d, J = 9.8 Hz, 1H), 6.39 (s, 1H), 5.03 (s, 2H), 2.68-2.58 (m, 1H), 2.52 (s, 3H), 2.40-2.28 (m, 2H), 1.91-1.80 (m, 2H), 1.78-1.65 (m, 1H), 1.35-1.21 (m, 1H), 1.01 (d, J = 6.7 Hz, 3H). |
| 381 | 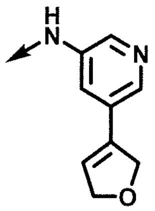 | $^1$H-NMR (400 MHz, $CDCl_3$) δ: 9.27 (s, 1H), 8.43 (s, 1H), 8.26 (s, 1H), 8.08 (s, 1H), 7.63 (d, J = 9.1 Hz, 1H), 6.99 (d, J = 10.4 Hz, 1H), 6.41-6.32 (m, 2H), 5.00 (s, 2H), 4.95 (d, J = 4.3 Hz, 2H), 4.84 (d, J = 4.9 Hz, 2H), 2.68-2.57 (m, 1H), 2.40-2.27 (m, 2H), 1.92-1.80 (m, 2H), 1.79-1.65 (m, 1H), 1.34-1.24 (m, 1H), 1.01 (d, J = 6.7 Hz, 3H). |
| 382 | 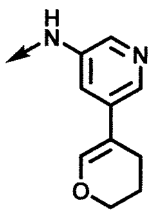 | $^1$H-NMR (400 MHz, $CDCl_3$) δ: 9.55 (s, 1H), 8.35 (d, J = 2.4 Hz, 1H), 8.22 (t, J = 3.0 Hz, 1H), 7.99 (d, J = 1.8 Hz, 1H), 7.59 (d, J = 10.1, 1H), 6.97-6.91 (m, 2H), 6.35 (s, 1H), 5.00 (s, 2H), 4.03-3.97 (m, 2H), 3.00 (s, 1H), 2.65-2.53 (m, 1H), 2.38-2.25 (m, 4H), 1.99-1.78 (m, 4H), 1.75-1.62 (m, 1H), 1.35-1.21 (m, 1H), 0.99 (d, J = 6.0 Hz, 3H). |
| 383 | 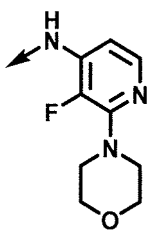 | $^1$H-NMR (400 MHz, $CDCl_3$) δ: 9.27 (s, 1H), 7.91 (d, J = 5.5 Hz, 1H), 7.81 (t, J = 4.9 Hz, 1H), 7.61 (d, J = 9.8 Hz, 1H), 7.00 (t, J = 4.9 Hz, 1H), 6.38 (s, 1H), 4.99 (s, 2H), 3.84-3.76 (m, 4H), 3.48-3.40 (m, 4H), 2.65-2.55 (m, 1H), 2.40-2.27 (m, 2H), 1.92-1.80 (m, 2H), 1.80-1.65 (m, 1H), 1.34-1.26 (m, 1H), 1.01 (d, J = 6.7 Hz, 3H). |
| 384 | 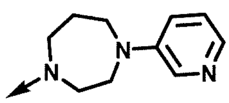 | LC-MS: $[M+H]^+$ / Rt (min) 408.4 / 1.56 (Method C) |
| 385 | 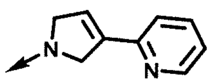 | LC-MS: $[M+H]^+$ / Rt (min) 377.4 / 1.64 (Method C) |
| 386 | 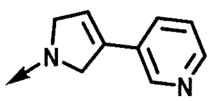 | LC-MS: $[M+H]^+$ / Rt (min) 377.3 / 1.57 (Method C) |

(continued)

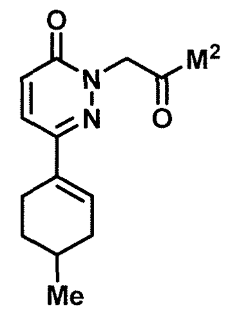

| Example | $M^2$ | Analytical data |
|---|---|---|
| 387 | 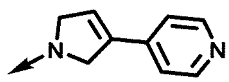 | LC-MS: $[M+H]^+$ / Rt (min) 377.3 / 1.55 (Method C) |
| 388 | 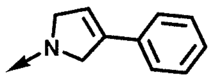 | LC-MS: $[M+H]^+$ / Rt (min) 376.3 / 1.83 (Method C) |
| 389 | 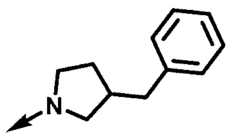 | LC-MS: $[M+H]^+$ / Rt (min) 392.3 / 1.88 (Method C) |
| 390 | 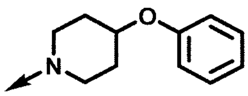 | LC-MS: $[M+H]^+$ / Rt (min) 408.3 / 1.86 (Method C) |
| 391 | 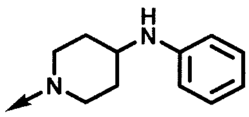 | LC-MS: $[M+H]^+$ / Rt (min) 407.4 / 1.80 (Method C) |
| 392 | 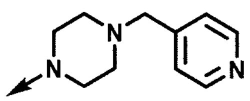 | LC-MS: $[M+H]^+$ / Rt (min) 408.3 / 1.55 (Method C) |
| 393 | 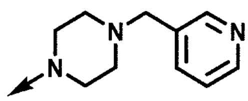 | LC-MS: $[M+H]^+$ / Rt (min) 408.4 / 1.54 (Method C) |
| 394 | 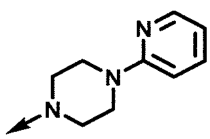 | $^1$H-NMR (400 MHz, DMSO-$d_6$) $\delta$: 8.14-8.12 (m, 1H), 7.83 (d, J = 10.0 Hz, 1H), 7.58-7.53 (m, 1H), 6.89 (d, J = 9.6 Hz, 1H), 6.87-6.84 (m, 1H), 6.68-6.65 (m, 1H), 6.51-6.48 (m, 1H), 4.97 (s, 2H), 3.65-3.49 (m, 8H), 2.68-2.64 (m, 1H), 2.34-2.27 (m, 1H), 2.25-2.15 (m, 1H), 1.84-1.73 (m, 2H), 1.71-1.60 (m, 1H), 1.28-1.17 (m, 1H), 0.96 (d, J = 6.8 Hz, 3H). |
| 395 | 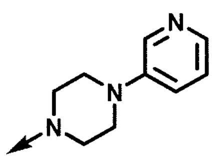 | $^1$H-NMR (400 MHz, DMSO-$d_6$) $\delta$: 8.33 (d, J = 2.8 Hz, 1H), 8.03-8.02 (m, 1H), 7.83 (d, J = 10.4 Hz, 1H), 7.37-7.36 (m, 1H), 7.25-7.21 (m, 1H), 6.89 (d, J = 10.0 Hz, 1H), 6.51-6.48 (m, 1H), 4.98 (s, 2H), 3.69-3.66 (m, 2H), 3.61-3.59 (m, 2H), 3.29-3.26 (m, 2H), 3.21-3.18 (m, 2H), 2.48-2.45 (m, 1H), 2.35-2.28 (m, 1H), 2.24-2.15 (m, 1H), 1.84-1.73 (m, 2H), 1.68-1.61 (m, 1H), 1.26-1.16 (m, 1H), 0.96 (d, J = 6.0 Hz, 3H). |

(continued)

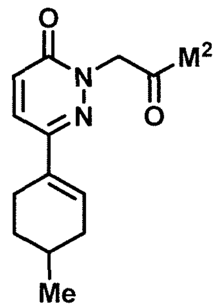

| Example | $M^2$ | Analytical data |
|---|---|---|
| 396 | 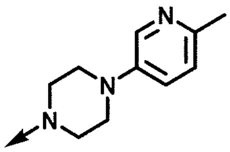 | LC-MS: $[M+H]^+$ / Rt (min) 408.4 / 0.643 (Method A) |
| 397 | 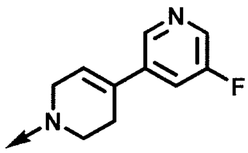 | LC-MS: $[M+H]^+$ / Rt (min) 409.3 / 0.932 (Method A) |
| 398 | 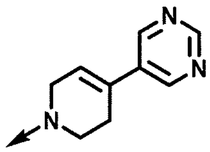 | LC-MS: $[M+H]^+$ / Rt (min) 392.4 / 1.239 (Method A) |
| 399 | 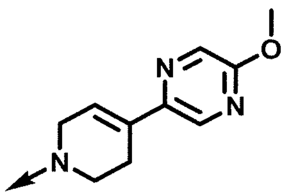 | $^1$H-NMR (400 MHz, DMSO-$d_6$) δ: 8.40-8.37 (m, 1H), 8.29-8.28 (m, 1H), 7.83 (d, J = 10.0 Hz, 1H), 6.89 (d, J = 10.0 Hz, 1H), 6.65-6.63 (m, 1H), 6.51-6.48 (m, 1H), 5.02 (s, 1H), 4.96 (s, 1H), 4.31-4.28 (m, 1H), 4.15-4.12 (m, 1H), 3.91 (s, 3H), 3.75-3.66 (m, 2H), 2.69-2.63 (m, 2H), 2.57-2.51 (m, 1H), 2.34-2.14 (m, 2H), 1.84-1.70 (m, 2H), 1.70-1.59 (m, 1H), 1.26-1.16 (m, 1H), 0.95 (d, J = 6.8 Hz, 3H). |
| 400 | 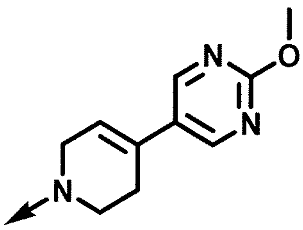 | $^1$H-NMR (400 MHz, DMSO-$d_6$) δ: 8.71 (d, J = 7.2 Hz, 2H), 7.83 (d, J = 10.0 Hz, 1H), 6.89 (d, J = 10.0 Hz, 1H), 6.51-6.48 (m, 1H), 6.30-6.26 (m, 1H), 4.98 (d, J = 22 Hz, 2H), 4.26-4.23 (m, 1H), 4.11-4.08 (m, 1H), 3.91 (s, 3H), 3.73 (t, J = 5.6 Hz, 1H), 3.68 (t, J = 5.6 Hz, 1H), 3.40-3.33 (m, 1H), 2.63-2.58 (m, 1H), 2.47-2.44 (m, 1H), 2.33-2.26 (m, 1H), 2.24-2.14 (m, 1H), 1.84-1.72 (m, 2H), 1.68-1.60 (m, 1H), 1.26-1.15 (m, 1H), 0.95 (d, J = 6.8 Hz, 3H). |
| 401 | 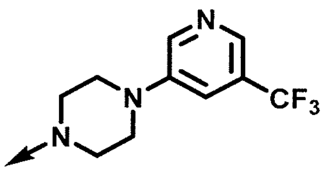 | LC-MS: $[M+H]^+$ / Rt (min) 462.4 / 1.021 (Method A) |

(continued)

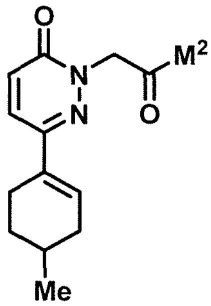

| Example | $M^2$ | Analytical data |
|---|---|---|
| 402 | 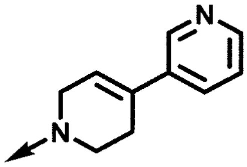 | $^1$H-NMR (400 MHz, DMSO-$d_6$) δ: 8.70-8.68 (m, 1H), 8.48-8.47 (m, 1H), 7.87-7.82 (m, 2H), 7.39-7.36 (m, 1H), 6.90 (d, J = 10.0 Hz, 1H), 6.51-6.48 (m, 1H), 6.31-6.29 (m, 1H), 4.99 (d, J = 23.2 Hz, 2H), 4.28-4.26 (m, 1H), 4.13-4.10 (m, 1H), 3.73 (t, J = 5.6 Hz, 1H), 3.69 (t, J = 5.6 Hz, 1H), 3.39-3.33 (m, 1H), 2.68-2.61 (m, 1H), 2.47-2.44 (m, 1H), 2.34-2.26 (m, 1H), 2.24-2.14 (m, 1H), 1.85-1.72 (m, 2H), 1.69-1.60 (m, 1H), 1.26-1.16 (m, 1H), 0.95 (d, J = 6.8 Hz, 3H). |
| 403 | 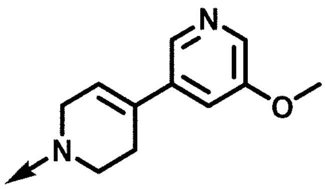 | $^1$H-NMR (400 MHz, DMSO-$d_6$) δ: 8.30-8.27 (m, 1H), 8.20-8.19 (m, 1H), 7.84 (d, J = 10.0 Hz, 1H), 7.41-7.38 (m, 1H), 6.90 (d, J = 10.0 Hz, 1H), 6.51-6.48 (m, 1H), 6.34-6.31 (m, 1H), 4.99 (d, J = 22 Hz, 2H), 4.28-4.25 (m, 1H), 4.13-4.10 (m, 1H), 3.85 (s, 3H), 3.73 (t, J = 5.6 Hz, 1H), 3.68 (t, J = 5.6 Hz, 1H), 3.40-3.33 (m, 1H), 2.68-2.61 (m, 1H), 2.47-2.44 (m, 1H), 2.34-2.26 (m, 1H), 2.24-2.14 (m, 1H), 1.85-1.72 (m, 2H), 1.69-1.60 (m, 1H), 1.26-1.16 (m, 1H), 0.95 (d, J = 6.8 Hz, 3H). |
| 404 | 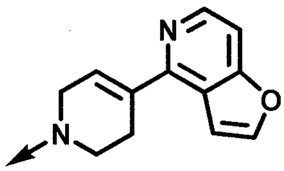 | LC-MS: $[M+H]^+$ / Rt (min) 431.4 / 0.860 (Method A) |
| 405 | 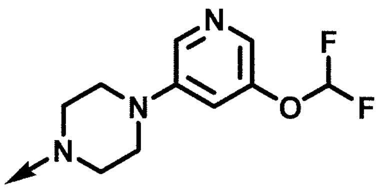 | $^1$H-NMR (400 MHz, DMSO-$d_6$) δ: 8.24-8.22 (m, 1H), 7.90-7.89 (m, 1H), 7.83 (d, J = 9.6 Hz, 1H), 7.18 (t, J = 2.4 Hz, 1H), 6.90 (d, J = 10.0 Hz, 1H), 6.51-6.48 (m, 1H), 4.98 (s, 2H), 3.69-3.65 (m, 2H), 3.61-3.57 (m, 2H), 3.39-3.34 (m, 2H), 3.29-3.25 (m, 2H), 2.67-2.63 (m, 1H), 2.54-2.50 (m, 1H), 2.35-2.30 (m, 1H), 2.26-2.14 (m, 1H), 1.85-1.72 (m, 2H), 1.70-1.60 (m, 1H), 1.27-1.17 (m, 1H), 0.96 (d, J = 6.8 Hz, 3H). |
| 406 | 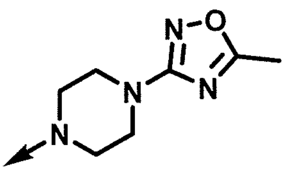 | LC-MS: $[M+H]^+$ / Rt (min) 399.3 / 0.868 (Method A) |
| 407 | 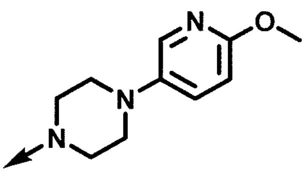 | LC-MS: $[M+H]^+$ / Rt (min) 424.3 / 0.679 (Method A) |

(continued)

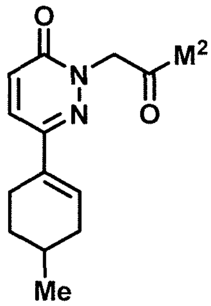

| Example | M2 | Analytical data |
|---|---|---|
| 408 | 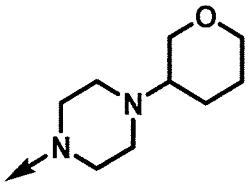 | LC-MS: [M+H]+ / Rt (min) 401.4 / 0.621 (Method A) |
| 409 | 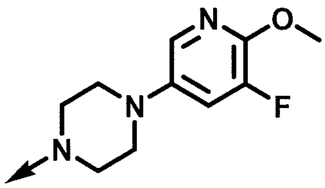 | LC-MS: [M+H]+ / Rt (min) 442.4 / 0.994 (Method A) |
| 410 | 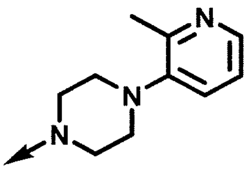 | LC-MS: [M+H]+ / Rt (min) 408.4 / 0.704 (Method A) |
| 411 | 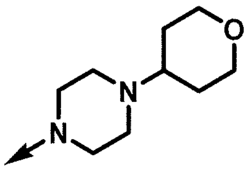 | LC-MS: [M+H]+ / Rt (min) 401.4 / 0.624 (Method A) |
| 412 | 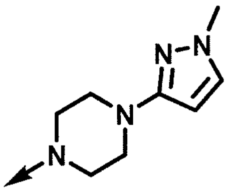 | LC-MS: [M+H]+ / Rt (min) 397.3 / 0.828 (Method A) |
| 413 | 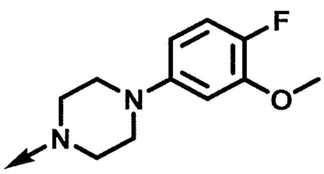 | LC-MS: [M+H]+ / Rt (min) 441.4 / 0.993 (Method D) |

(continued)

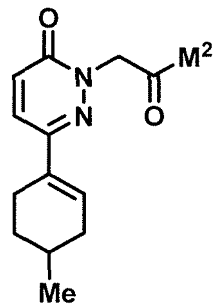

| Example | $M^2$ | Analytical data |
|---|---|---|
| 414 | 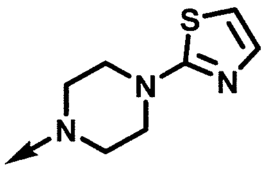 | $^1$H-NMR (400 MHz, DMSO-$d_6$) δ: 7.83 (d, J = 9.6 Hz, 1H), 7.19 (d, J = 3.6 Hz, 1H), 6.91-6.89 (m, 1H), 6.88 (s, 1H), 6.51-6.47 (m, 1H), 4.97 (s, 2H), 3.68-3.65 (m, 2H), 3.60-3.58 (m, 2H), 3.51-3.48 (m, 2H), 3.41-3.39 (m, 2H), 2.48-2.45 (m, 1H), 2.35-2.26 (m, 1H), 2.26-2.14 (m, 1H), 1.85-1.73 (m, 2H), 1.69-1.60 (m, 1H), 1.26-1.18 (m, 1H), 0.96 (d, J = 6.4 Hz, 3H). |
| 415 | 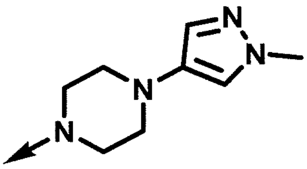 | LC-MS: $[M+H]^+$ / Rt (min) 397.3 / 0.787 (Method A) |
| 416 | 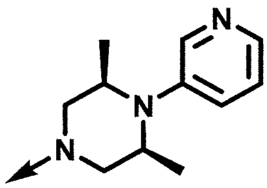 | $^1$H-NMR (400 MHz, DMSO-$d_6$) δ: 8.36 (d, J = 2.8 Hz, 1H), 8.03-8.01 (m, 1H), 7.84 (d, J = 9.6 Hz, 1H), 7.39-7.36 (m, 1H), 7.25-7.21 (m, 1H), 6.89 (d, J = 10.0 Hz, 1H), 6.52-6.49 (m, 1H), 5.01-4.90 (m, 2H), 4.48-4.39 (m, 1H), 4.26-4.17 (m, 1H), 3.68-3.59 (m, 2H), 3.34-3.32 (m, 1H), 3.00-2.81 (m, 2H), 2.35-2.26 (m, 1H), 2.26-2.16 (m, 1H), 1.86-1.74 (m, 2H), 1.70-1.61 (m, 1H), 1.48-1.34 (m, 3H), 1.30-1.18 (m, 4H), 0.96 (d, J = 6.8 Hz, 3H). |
| 417 | 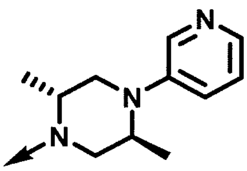 | LC-MS: $[M+H]^+$ / Rt (min) 422.4 / 0.676 (Method A) |
| 418 | 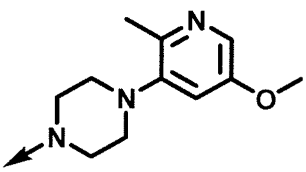 | $^1$H-NMR (400 MHz, $CDCl_3$) δ: 7.96 (d, J = 2.4 Hz, 1H), 7.54 (d, J = 9.8 Hz, 1H), 6.91-6.89 (br m, 2H), 6.32 (br s, 1H), 5.00 (d, J = 3.7 Hz, 2H), 3.86 (s, 3H), 3.83-3.80 (br m, 2H), 3.70-3.68 (br m, 2H), 3.00-2.92 (m, 4H), 2.62-2.60 (m, 1H), 2.58-2.56 (m, 1H), 2.52 (s, 3H), 1.87-1.66 (m, 4H), 1.33-1.23 (m, 1H), 1.00 (d, J = 6.7 Hz, 3H). |
| 419 | 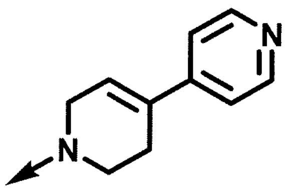 | $^1$H-NMR (400 MHz, $CDCl_3$) δ: 8.64 (d, J = 6.1 Hz, 2H), 7.59 (br s, 2H), 7.55 (d, J = 9.8 Hz, 1H), 6.90 (d, J = 9.8 Hz, 1H), 6.60-6.51 (br m, 1H), 6.33-6.31 (m, 1H), 5.05-4.98 (m, 2H), 4.40-4.34 (m, 2H), 3.92-3.78 (m, 1H), 2.75-2.70 (br m, 1H), 2.64-2.53 (br m, 2H), 2.35-2.24 (br m, 3H), 1.88-1.78 (br m, 2H), 1.75-1.65 (br m, 1H), 1.32-1.22 (m, 1H), 0.99 (d, J = 6.1 Hz, 3H). |

(continued)

| 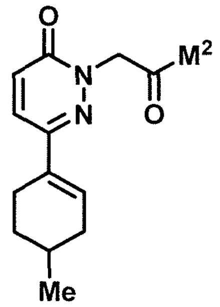 | | |
|---|---|---|
| Example | $M^2$ | Analytical data |
| 420 | 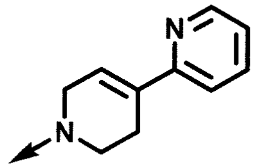 | LC-MS: $[M+H]^+$ / Rt (min) 391.2 / 1.62 (Method B) |
| 421 | 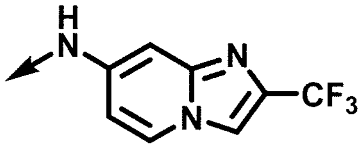 | $^1$H-NMR (400 MHz, $CDCl_3$) δ: 9.57 (s, 1H), 7.96 (s, 1H), 7.92 (d, J = 7.6 Hz, 1H), 7.68 (s, 1H), 7.65 (d, J = 10.0 Hz, 1H), 7.02-6.97 (m, 2H), 6.40 (s, 1H), 5.00 (s, 2H), 2.67-2.57 (m, 1H), 2.40-2.25 (m, 2H), 1.90-1.85 (m, 2H), 1.77-1.67 (m, 1H), 1.35-1.25 (m, 1H), 1.01 (d, J = 6.7 Hz, 3H). |
| 422 | 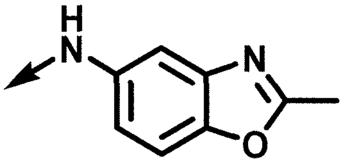 | $^1$H-NMR (400 MHz, $CDCl_3$) δ: 8.73 (s, 1H), 7.84 (d, J = 2.4 Hz, 1H), 7.56 (d, J = 9.8 Hz, 1H), 7.41 (dd, J = 8.6, 2.4 Hz, 1H), 7.35 (d, J = 8.6 Hz, 1H), 6.96 (d, J = 9.8 Hz, 1H), 6.36 (s, 1H), 4.96 (s, 2H), 2.68-2.60 (m, 1H), 2.60 (s, 3H), 2.45-2.30 (m, 2H), 1.91-1.81 (m, 2H), 1.79-1.71 (m, 1H), 1.37-1.27 (m, 1H), 1.02 (d, J = 6.7 Hz, 3H). |
| 423 | 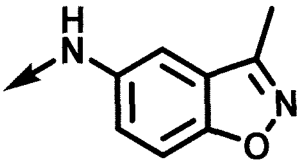 | $^1$H-NMR (400 MHz, $CDCl_3$) δ: 8.97 (s, 1H), 8.05 (s, 1H), 7.58 (d, J = 9.8 Hz, 1H), 7.43 (s, 2H), 6.98 (t, J = 6.7 Hz, 1H), 6.38 (s, 1H), 4.98 (s, 2H), 2.69-2.60 (m, 1H), 2.53 (s, 3H), 2.43-2.32 (m, 2H), 1.93-1.82 (m, 2H), 1.80-1.70 (m, 1H), 1.39-1.25 (m, 1H), 1.03 (d, J = 6.7 Hz, 3H). |
| 424 | 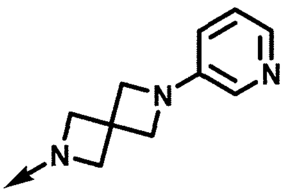 | LC-MS: $[M+H]^+$ / Rt (min) 406.3 / 0.577 (Method D) |

Examples 425 - 445

According to the method of Example 1, 37, or 50 and common reaction conditions, the compounds of Examples 425 to 445 were obtained by using each corresponding material compound.

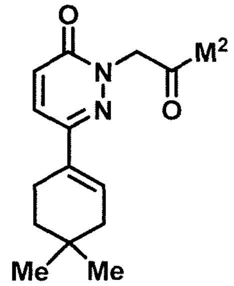

| Example | $M^2$ | Analytical data |
|---|---|---|
| 425 | 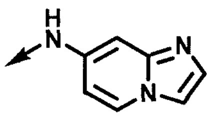 | $^1$H-NMR (400 MHz, DMSO-$d_6$) δ: 10.55 (s, 1H), 8.45 (d, J = 7.6 Hz, 1H), 7.88-7.91 (m, 2H), 7.82 (s, 1H), 7.45-7.44 (m, 1H), 6.93-6.96 (m, 2H), 6.49-6.52 (m, 1H), 4.89 (s, 2H), 2.32-2.38 (m, 2H), 1.99-2.03 (m, 2H), 1.43 (t, J = 6.8 Hz, 2H), 0.92 (s, 6H). |
| 426 | 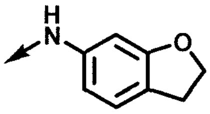 | $^1$H-NMR (400 MHz, $CDCl_3$) δ: 8.70 (br s, 1H), 7.60 (d, J = 10.1 Hz, 1H), 7.09-7.06 (m, 2H), 6.96 (d, J = 10.1 Hz, 1H), 6.91 (dd, J = 8.2, 2.1 Hz, 1H), 6.34-6.32 (m, 1H), 4.93 (s, 2H), 4.55 (t, J = 8.7 Hz, 2H), 3.14 (t, J = 8.7 Hz, 2H), 2.48-2.44 (m, 2H), 2.04-2.02 (m, 2H), 1.48 (t, J = 6.4 Hz, 2H), 0.95 (s, 6H). |
| 427 | 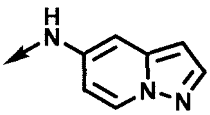 | $^1$H-NMR (400 MHz, $CDCl_3$) δ: 8.34 (d, J = 7.3 Hz, 1H), 7.99 (s, 1H), 7.88 (d, J = 2.4 Hz, 1H), 7.64 (d, J = 9.8 Hz, 1H), 7.00 (d, J = 9.8 Hz, 1H), 6.73-6.71 (m, 1H), 6.40 (d, J = 2.4 Hz, 1H), 6.37-6.34 (br m, 1H), 4.98 (s, 2H), 2.49-2.45 (m, 2H), 2.04-2.03 (m, 2H), 1.51-1.48 (br m, 2H), 0.96 (s, 6H). |
| 428 | 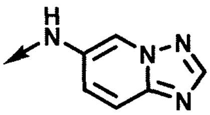 | $^1$H-NMR (400 MHz, $CDCl_3$) δ: 9.54 (s, 1H), 9.47 (s, 1H), 8.29 (d, J = 4.3 Hz, 1H), 7.68-7.63 (m, 2H), 7.31-7.28 (m, 1H), 7.02 (t, d = 6.7 Hz, 1H), 6.37 (s, 1H), 5.03 (s, 2H), 2.50-2.46 (m, 2H), 2.08-2.03 (m, 2H), 1.53-1.547 (m, 2H), 0.96 (s, 6H). |
| 429 | 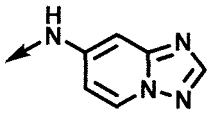 | $^1$H-NMR (400 MHz, $CDCl_3$) δ: 9.69 (s, 1H), 8.42 (d, J = 7.3 Hz, 1H), 8.24 (s, 1H), 8.08 (d, J = 1.8 Hz, 1H), 7.66 (d, J = 9.8 Hz, 1H), 7.13 (dd, J = 7.6, 2.1 Hz, 1H), 7.01 (d, J = 9.8 Hz, 1H), 6.36 (s, 1H), 5.01 (s, 2H), 2.50-2.43 (m, 2H), 2.15-2.03 (m, 2H), 1.53-1.45 (m, 2H), 0.95 (s, 6H). |
| 430 | 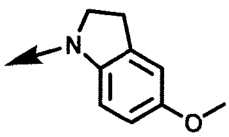 | $^1$H-NMR (400 MHz, DMSO-$d_6$) δ: 7.89-7.84 (m, 2H), 6.93 (d, J = 10.0 Hz, 1H), 6.88-6.86 (m, 1H), 6.70 (dd, J = 2.4, 4.0 Hz, 1H), 6.50-6.47 (m, 1H), 5.00 (s, 2H), 4.20 (t, J = 8.0 Hz, 2H), 3.71 (s, 3H), 3.17 (t, J = 8.0 Hz, 2H), 2.36-2.31 (m, 2H), 2.03-1.99 (m, 2H), 1.42 (t, J = 6.8 Hz, 2H), 0.91 (s, 6H). |
| 431 | 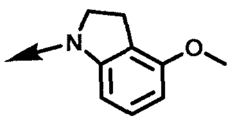 | $^1$H-NMR (400 MHz, DMSO-$d_6$) δ: 7.88 (d, J = 10.0 Hz, 1H), 7.57 (d, J = 8.0 Hz, 1H), 7.13 (t, J = 8.0 Hz, 1H), 6.93 (d, J = 9.6 Hz, 1H), 6.70 (d, J = 8.0 Hz, 1H), 6.50-6.47 (m, 1H), 5.01 (s, 2H), 4.25-4.20 (m, 2H), 3.79 (s, 3H), 3.09-3.04 (m, 2H), 2.36-2.31 (m, 2H), 2.02-1.99 (m, 2H), 1.44-1.40 (m, 2H), 0.91 (s, 6H). |
| 432 | 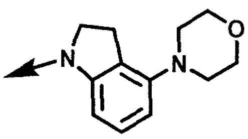 | $^1$H-NMR (400 MHz, $CDCl_3$) δ: 7.91 (d, J = 8.4 Hz, 1H), 7.57 (d, J = 10.1 Hz, 1H), 7.16 (t, J = 8.4 Hz, 1H), 6.92 (d, J = 10.1 Hz, 1H), 6.69 (d, J = 7.3 Hz, 1H), 6.30-6.28 (m, 1H), 5.02 (s, 2H), 4.19 (t, J = 8.2 Hz, 2H), 3.86-3.83 (m, 4H), 3.19 (t, J = 8.2 Hz, 2H), 2.99-2.97 (m, 4H), 2.44-2.40 (m, 2H), 2.03-2.01 (m, 2H), 1.45 (t, J = 6.4 Hz, 2H), 0.94 (s, 6H). |

(continued)

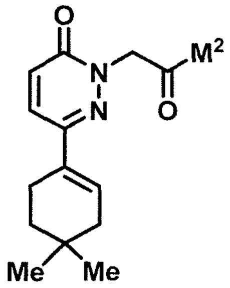

| Example | $M^2$ | Analytical data |
|---|---|---|
| 433 | 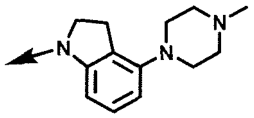 | $^1$H-NMR (400 MHz, DMSO-$d_6$) δ: 7.89 (d, J = 10.0 Hz, 1H), 7.64 (d, J = 8.0 Hz, 1H), 7.09 (t, J = 8.0 Hz, 1H), 6.93 (d, J = 9.6 Hz, 1H), 6.66 (d, J = 8.0 Hz, 1H), 6.51-6.48 (m, 1H), 5.01 (s, 2H), 4.19 (t, J = 8.0 Hz, 2H), 3.09 (t, J = 8.0 Hz, 2H), 2.95-2.90 (m, 4H), 2.47-2.43 (m, 4H), 2.36-2.30 (m, 2H), 2.22 (s, 3H), 2.02-1.99 (m, 2H), 1.42 (t, J = 6.8 Hz, 2H), 0.91 (s, 6H). |
| 434 | 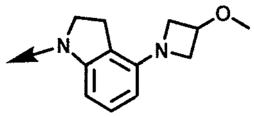 | LC-MS: $[M+H]^+$ / Rt (min) 449.2 / 1.098 (Method A) |
| 435 | 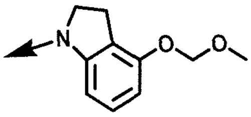 | $^1$H-NMR (400 MHz, $CDCl_3$) δ: 7.84 (d, J = 7.9 Hz, 1H), 7.56 (d, J = 9.8 Hz, 1H), 7.12 (t, J = 8.2 Hz, 1H), 6.92 (d, J = 9.8 Hz, 1H), 6.78 (d, J = 7.9 Hz, 1H), 6.30-6.28 (m, 1H), 5.20 (s, 2H), 5.01 (s, 2H), 4.21 (t, J = 8.5 Hz, 2H), 3.49 (s, 3H), 3.23 (t, J = 8.5 Hz, 2H), 2.42 (br s, 2H), 2.03-2.01 (m, 2H), 1.46 (t, J = 6.4 Hz, 2H), 0.94 (s, 6H). |
| 436 | 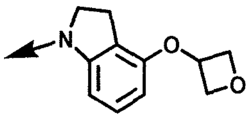 | LC-MS: $[M+H]^+$ / Rt (min) 436.4 / 1.054 (Method A) |
| 437 | 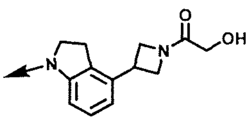 | LC-MS: $[M+H]^+$ / Rt (min) 477.4 / 0.895 (Method A) |
| 438 | 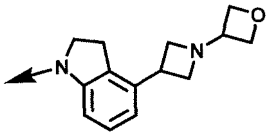 | $^1$H-NMR (400 MHz, $CDCl_3$) δ: 8.02 (d, J = 7.9 Hz, 1H), 7.50 (d, J = 9.8 Hz, 1H), 7.13 (t, J = 7.9 Hz, 1H), 6.93 (d, J = 7.9 Hz, 1H), 6.85 (d, J = 9.8 Hz, 1H), 6.24-6.21 (m, 1H), 4.94 (s, 2H), 4.69 (t, J = 6.7 Hz, 2H), 4.54 (t, J = 6.1 Hz, 2H), 4.14 (t, J = 8.5 Hz, 2H), 3.86 (s, 4H), 3.33 (s, 2H), 3.07 (t, J = 8.2 Hz, 2H), 2.37-2.33 (m, 2H), 1.96-1.94 (m, 2H), 1.39 (t, J = 6.4 Hz, 2H), 0.87 (s, 6H). |
| 439 | 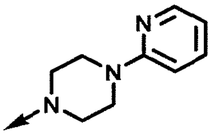 | LC-MS: $[M+H]^+$ / Rt (min) 408.4 / 0.770 (Method A) |
| 440 | 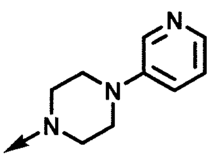 | $^1$H-NMR (400 MHz, DMSO-$d_6$) δ: 8.33-8.32 (m, 1H), 8.03-8.02 (m, 1H), 7.85 (d, J = 10.0 Hz, 1H), 7.37-7.34 (m, 1H), 7.25-7.21 (m, 1H), 6.90 (d, J = 9.6 Hz, 1H), 6.48-6.45 (m, 1H), 4.98 (s, 2H), 3.69-3.66 (m, 2H), 3.62-3.58 (m, 2H), 3.29-3.26 (m, 2H), 3.22-3.17 (m, 2H), 2.34-2.29 (m, 2H), 2.01-1.98 (m, 2H), 1.41 (t, J = 6.0 Hz, 2H), 0.91 (s, 6H). |

(continued)

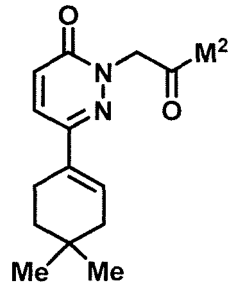

| Example | $M^2$ | Analytical data |
|---|---|---|
| 441 | 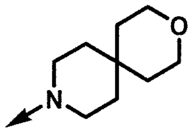 | LC-MS: $[M+H]^+$ / Rt (min) 400.4 / 0.947 (Method A) |
| 442 | 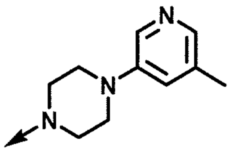 | $^1$H-NMR (400 MHz, DMSO-$d_6$) δ: 8.14-8.12 (m, 1H), 7.87 (s, 1H), 7.85 (d, J = 10.0 Hz, 1H), 7.20-7.18 (m, 1H), 6.90 (d, J = 9.6 Hz, 1H), 6.48-6.44 (m, 1H), 4.98 (s, 2H), 3.68-3.64 (m, 2H), 3.61-3.57 (m, 2H), 3.29-3.24 (m, 2H), 3.20-3.16 (m, 2H), 2.34-2.29 (m, 2H), 2.24 (s, 3H), 2.02-1.98 (m, 2H), 1.41 (t, J = 6.4 Hz, 2H), 0.91 (s, 6H). |
| 443 | 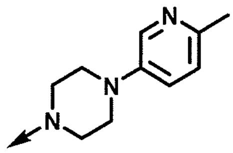 | LC-MS: $[M+H]^+$ / Rt (min) 422.4 / 0.754 (Method A) |
| 444 | 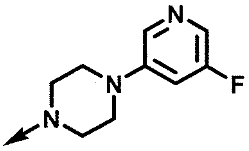 | LC-MS: $[M+H]^+$ / Rt (min) 426.4 / 0.858 (Method D) |
| 445 | 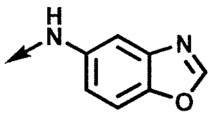 | $^1$H-NMR (400 MHz, DMSO-$d_6$) δ: 10.49 (s, 1H), 8.71-8.70 (m, 1H), 8.11-8.10 (m, 1H), 7.90-7.87 (m, 1H), 7.73-7.70 (m, 1H), 7.53-7.49 (m, 1H), 6.95-6.92 (m, 1H), 6.51-6.49 (m, 1H), 4.89-4.86 (m, 2H), 2.37-2.31 (m, 2H), 2.03-1.99 (m, 2H), 1.44-1.40 (m, 2H), 0.93-0.89 (m, 6H). |

Examples 446 - 455

According to the method of Example 1, 37, or 50 and common reaction conditions, the compounds of Examples 446 to 455 were obtained by using each corresponding material compound.

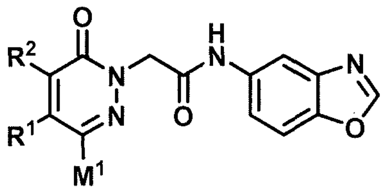

| Example | $M^1$ | $R^1$ | $R^2$ | Analytical data |
|---|---|---|---|---|
| 446 | 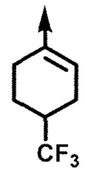 | H | H | $^1$H-NMR (400 MHz, $CDCl_3$) δ: 8.93 (s, 1H), 8.06 (s, 2H), 7.59 (d, J = 9.8 Hz, 1H), 7.51-7.44 (m, 2H), 7.01 (d, J = 9.8 Hz, 1H), 6.37-6.33 (m, 1H), 4.98 (s, 2H), 2.86-2.77 (m, 1H), 2.55-2.45 (m, 1H), 2.45-2.25 (m, 3H), 2.20-2.12 (m, 1H), 1.64-1.58 (m, 1Hz). |
| 447 | 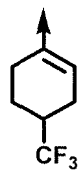 | H | OMe | $^1$H-NMR (400 MHz, $CDCl_3$) δ: 9.10 (s, 1H), 8.08-8.05 (m, 2H), 7.48 (s, 2H), 6.45-6.40 (s, 1H), 6.27 (s, 1H), 4.95 (s, 2H), 3.87 (s, 3H), 2.67-2.57 (m, 1H), 2.42-2.225 (m, 4H), 2.16-2.08 (m, 1H), 1.70-1.60 (m, 1H). |
| 448 | 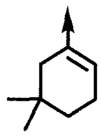 | H | OMe | $^1$H-NMR (400 MHz, $CDCl_3$) δ: 9.35 (s, 1H), 8.01 (d, J = 5.5 Hz, 2H), 7.48-7.35 (m, 2H), 6.33-6.27 (m, 2H), 6.21 (s, 1H), 4.93 (s, 2H), 3.82 (s, 3H), 2.24-2.17 (m, 2H), 2.10 (s, 2H), 1.37 (t, J = 6.4 Hz, 2H), 1.21 (s, 6H). |
| 449 | 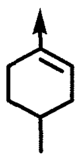 | H | OMe | $^1$H-NMR (400 MHz, $CDCl_3$) δ: 9.13 (s, 1H), 8.07 (s, 2H), 7.49 (s, 2H), 6.38 (s, 1H), 6.25 (s, 1H), 4.95 (s, 2H), 3.86 (s, 3H), 2.48-2.28 (m, 3H), 1.87-1.67 (m, 2H), 1.38-1.23 (m, 2H), 1.00 (d, J = 6.1 Hz, 3H). |
| 450 | 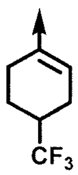 | OMe | H | $^1$H-NMR (400 MHz, $CDCl_3$) δ: 9.07 (s, 1H), 8.07-8.06 (m, 2H), 7.48-7.47 (m, 2H), 6.43-6.41 (m, 1H), 6.27 (s, 1H), 4.94 (s, 2H), 3.87 (s, 3H), 2.65-2.59 (m, 1H), 2.49-2.26 (m, 4H), 2.15-2.10 (m, 1H), 1.69-1.58 (m, 1H). |
| 451 | 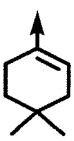 | OMe | H | $^1$H-NMR (400 MHz, $CDCl_3$) δ: 9.35 (s, 1H), 8.08-8.05 (m, 2H), 7.44 (s, 1H), 6.40 (s, 1H), 6.28 (s, 1H), 5.00 (s, 2H), 3.88 (s, 3H), 2.45-2.38 (m, 2H), 2.05-2.00 (m, 2H), 1.49 (t, J = 6.4 Hz, 2H), 0.98 (s, 6H). |
| 452 | 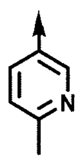 | H | H | $^1$H-NMR (400 MHz, $CDCl_3$) δ: 9.13 (s, 1H), 8.74 (s, 1H), 7.98-7.93 (m, 1H), 7.93-7.86 (m, 2H), 7.68-7.64 (m, 1H), 7.49-7.39 (m, 1H), 7.29-7.38 (m, 1H), 7.13-7.05 (m, 1H), 6.99-6.94 (m, 1H), 4.94 (s, 2H), 2.44 (s, 3H). |

(continued)

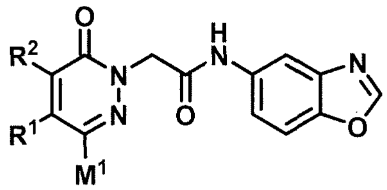

| Example | $M^1$ | $R^1$ | $R^2$ | Analytical data |
|---|---|---|---|---|
| 453 | 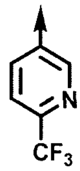 | H | H | $^1$H-NMR (400 MHz, $CDCl_3$) δ: 9.10 (s, 1H), 8.74 (s, 1H), 8.34 (d, J = 7.9 Hz, 1H), 8.13-8.03 (m, 2H), 7.85-7.75 (m, 2H), 7.65-7.35 (m, 3H), 5.10 (s, 2H). |
| 454 | 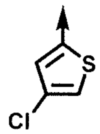 | H | H | $^1$H-NMR (400 MHz, $CD_3OD$) δ: 8.45 (s, 1H), 8.11 (d, J = 1.8 Hz, 1H), 7.99 (dd, J = 9.8, 4.9 Hz, 1H), 7.63 (d, J = 8.6 Hz, 1H), 7.60-7.53 (m, 2H), 7.39 (d, J = 1.2 Hz, 1H), 7.09 (dd, J = 9.8, 4.3 Hz, 1H), 5.04 (s, 2H). |
| 455 | 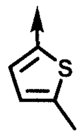 | H | H | $^1$H-NMR (400 MHz, $CDCl_3$) δ: 8.95 (s, 1H), 8.04 (s, 2H), 7.63 (d, J = 9.8 Hz, 1H), 7.50-7.40 (m, 2H), 7.20 (q, J = 4.7 Hz, 1H), 7.06 (t, J = 11.0 Hz, 1H), 6.72 (t, J = 1.8 Hz, 1H), 5.00 (s, 2H), 7.49 (s, 3H). |

Examples 456 - 467

According to the method of Example 1, 37, or 50 and common reaction conditions, the compounds of Examples 456 to 467 were obtained by using each corresponding material compound.

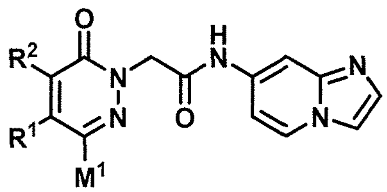

| Example | $M^1$ | $R^1$ | $R^2$ | Analytical data |
|---|---|---|---|---|
| 456 | 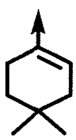 | H | OMe | $^1$H-NMR (400 MHz, $CDCl_3$) δ: 9.98 (s, 1H), 8.06-7.98 (m, 1H), 7.88 (s, 1H), 7.57 (s, 1H), 7.54-7.48 (m, 1H), 7.26-7.17 (m, 1H), 6.35-6.39 (m, 1H), 6.27 (s, 1H), 5.06-5.02 (m, 2H), 3.89 (s, 3H), 2.44-2.39 (m, 2H), 2.05-1.99 (m, 2H), 1.49 (t, J = 6.4 Hz, 2H), 0.99 (s, 6H). |
| 457 | 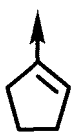 | H | H | $^1$H-NMR (400 MHz, $CDCl_3$) δ: 10.40 (s, 1H), 7.89 (d, J = 7.6 Hz, 1H), 7.85 (s, 1H), 7.62 (d, J = 9.6 Hz, 1H), 7.53 (s, 1H), 7.41 (s, 1H), 7.12 (dd, J = 7.6, 2.1 Hz, 1H), 6.98 (d, J = 9.6 Hz, 1H), 6.36 (s, 1H), 5.07 (s, 2H), 2.79-2.71 (m, 2H), 2.64-2.57 (m, 2H), 2.04 (q, J = 7.2 Hz, 2H). |

(continued)

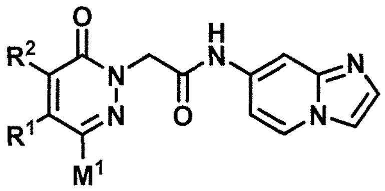

| Example | $M^1$ | $R^1$ | $R^2$ | Analytical data |
|---|---|---|---|---|
| 458 | 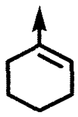 | H | H | $^1$H-NMR (400 MHz, $CDCl_3$) δ: 10.38 (s, 1H), 7.86-7.80 (m, 2H), 7.56 (d, J = 9.8 Hz, 1H), 7.46 (s, 1H), 7.34 (s, 1H), 7.05 (dd, J =7.2, 2.0 Hz, 1H), 6.93 (d, J = 9.8 H, 1H z), 6.36 (s, 1H), 5.02 (s, 2H), 2.40 (s, 4H), 1.75-1.60 (m, 4H). |
| 459 | 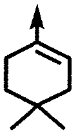 | OMe | H | $^1$H-NMR (400 MHz, $CDCl_3$) δ: 9.58 (s, 1H), 7.97-7.83 (m, 1H), 7.81 (s, 1H), 7.49 (s, 1H), 7.39 (s, 1H), 6.98 (d, J = 6.7 Hz, 1H), 6.36-6.30 (m, 1H), 6.23 (s, 1H), 4.94 (s, 2H), 3.86 (s, 3H), 2.40-2.35 (m, 2H), 2.00-1.96 (m, 2H), 1.44 (2H, t, J = 6.7 Hz), 0.94 (s, 6H). |
| 460 | 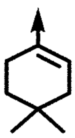 | H | Me | $^1$H-NMR (400 MHz, DMSO-$d_6$) δ: 10.57 (s, 1H) 8.40 (d, J = 7.3 Hz, 1H), 7.85 (s, 1H), 7.75 (d, J = 7.3 Hz, 2H), 7.39 (s, 1H), 6.92 (dd, J = 7.3, 1.8 Hz, 1H), 6.41 (s, 1H), 4.83 (s, 2H), 2.28 (s, 2H), 2.04 (s, 3H), 1.95 (s, 2H), 1.38-1.34 (m, 2H), 0.85 (s, 6H). |
| 461 | 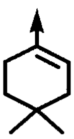 | Me | H | $^1$H-NMR (400 MHz, $CDCl_3$) δ: 10.10 (s, 1H), 7.98-7.93 (m, 2H), 7.50-7.43 (m, 2H), 7.16-7.08 (s, 1H), 6.83 (s, 1H), 5.78 (s, 1H), 5.03 (s, 2H), 2.30-2.23 (m, 5H), 1.98 (s, 2H), 1.51-1.48 (m, 2H), 0.98 (s, 6H). |
| 462 | 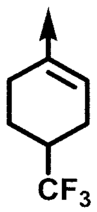 | H | OMe | $^1$H-NMR (400 MHz, DMSO-$d_6$) δ: 10.55 (s, 1H), 8.46 (d, J = 7.3 Hz, 1H), 7.91 (s, 1H), 7.83 (s, 1H), 7.46 (s, 1H), 6.95 (d, J = 7.3 Hz, 1H), 6.37 (s, 2H), 4.86 (s, 2H), 3.85 (s, 3H), 2.67-2.53 (m, 2H), 2.46-2.16 (m, 3H), 2.05-1.99 (m, 1H), 1.57-1.45 (m, 1H). |
| 463 | 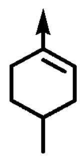 | H | OMe | $^1$H-NMR (400 MHz, $CDCl_3$) δ: 9.39 (br s, 1H), 7.97 (d, J = 7.3 Hz, 1H), 7.82 (s, 1H), 7.55 (s, 1H), 7.46 (s, 1H), 7.06-7.04 (br m, 1H), 6.38-6.37 (br m, 1H), 6.25 (s, 1H), 4.94 (s, 2H), 3.86 (s, 3H), 2.47-2.28 (m, 3H), 1.85-1.78 (m, 2H), 1.37-1.24 (m, 2H), 1.00 (d, J = 6.1 Hz, 3H). |
| 464 | 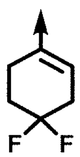 | H | H | $^1$H-NMR (400 MHz, DMSO-$d_6$) δ: 10.58 (s, 1H), 8.46 (dd, J = 7.3, 1.2 Hz, 1H), 7.95-7.90 (m, 2H), 7.83-7.82 (m, 1H), 7.46 (d, J = 1.2 Hz, 1H), 7.00 (d, J = 9.8 Hz, 1H), 6.96 (dd, J = 7.3, 2.4 Hz, 1H), 6.46 (s, 1H), 4.92 (s, 2H), 2.83-2.75 (m, 2H), 2.66-2.60 (m, 2H), 2.19-2.09 (m, 2H). |

(continued)

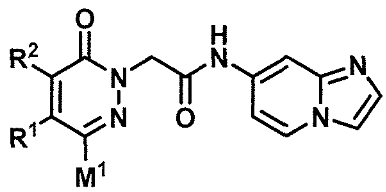

| Example | $M^1$ | $R^1$ | $R^2$ | Analytical data |
|---|---|---|---|---|
| 465 | 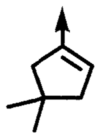 | H | H | LC-MS: [M+H] $^+$ / Rt (min) 364.3 / 0.582 (Method D) |
| 466 | 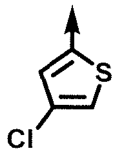 | H | OMe | $^1$H-NMR (400 MHz, $CDCl_3$) δ: 9.86 (s, 1H), 7.90 (s, 1H), 7.78 (s, 1H), 7.62 (s, 1H), 7.52 (d, J = 6.7 Hz, 1H), 7.39 (s, 1H), 7.18 (s, 1H), 7.11 (d, J = 6.7 Hz, 1H), 6.34 (s, 1H), 5.02 (s, 2H), 3.99 (s, 3H). |
| 467 | 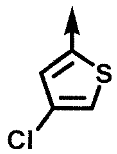 | H | H | $^1$H-NMR (400 MHz, $CD_3OD$) δ: 8.38 (d, J = 7.3 Hz, 1H), 8.01 (d, J = 9.8 Hz, 2H), 7.75 (s, 1H), 7.57 (d, J = 1.2 Hz, 1H), 7.48 (dd, J = 8.8, 1.5 Hz, 1H), 7.40 (d, J = 1.2 Hz, 1H), 7.10 (t, J = 4.9 Hz, 2H), 5.05 (s, 2H). |

Examples 468 - 481

According to the method of Example 1, 37, 38, or 50, and common reaction conditions, the compounds of Examples 468 to 481 were obtained by using each corresponding material compounds.

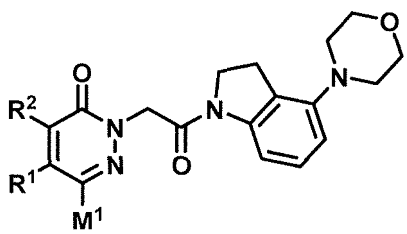

| Example | $M^1$ | $R^1$ | $R^2$ | Analytical data |
|---|---|---|---|---|
| 468 | 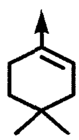 | H | OMe | $^1$H-NMR (400 MHz, $CDCl_3$) δ: 7.91 (d, J = 8.0 Hz, 1H), 7.15 (t, J = 8.0 Hz, 1H), 6.68 (d, J = 8.0 Hz, 1H), 6.27 (s, 1H), 6.19 (s, 1H), 4.98 (s, 2H), 4.16 (t, J = 8.0 Hz, 2H), 3.90-3.78 (m, 7H), 3.17 (t, J = 8.0 Hz, 2H), 2.98 (m, 4H), 2.36 (s, 2H), 1.98 (m, 2H), 1.45 (t, J = 6.4 Hz, 2H), 0.95 (6H, s). |
| 469 | 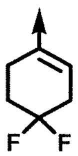 | H | H | LC-MS: [M+H] $^+$ / Rt (min) 457.4 / 0.824 (Method D) |

(continued)

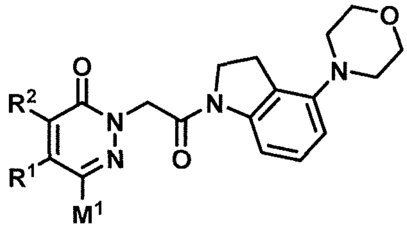

| Example | $M^1$ | $R^1$ | $R^2$ | Analytical data |
|---|---|---|---|---|
| 470 | 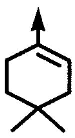 | OMe | H | $^1$H-NMR (400 MHz, $CDCl_3$) δ: 7.89 (d, J = 8.0 Hz, 1H), 7.13 (t, J = 8.0 Hz, 1H), 6.66 (d, J = 8.0 Hz, 1H), 6.25 (s, 1H), 6.17 (s, 1H), 4.97 (s, 2H), 4.14 (t, J = 8.0 Hz, 2H), 3.90-3.78 (m, 7H), 3.15 (t, J = 8.0 Hz, 2H), 2.96 (m, 4H), 2.34 (s, 2H), 1.96 (m, 2H), 1.43 (t, J = 6.4 Hz, 2H), 0.93 (6H, s). |
| 471 | 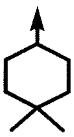 | H | H | $^1$H-NMR (400 MHz, $CDCl_3$) δ: 7.84 (d, J = 7.9 Hz, 1H), 7.56 (d, J = 9.8 Hz, 1H), 7.12 (t, J = 8.2 Hz, 1H), 6.92 (d, J = 9.8 Hz, 1H), 6.78 (d, J = 7.9 Hz, 1H), 6.30-6.28 (m, 1H), 5.20 (s, 2H), 5.01 (s, 2H), 4.21 (t, J = 8.5 Hz, 2H), 3.49 (s, 3H), 3.23 (t, J = 8.5 Hz, 2H), 2.42 (br s, 2H), 2.03-2.01 (m, 2H), 1.46 (t, J = 6.4 Hz, 2H), 0.94 (s, 6H). |
| 472 | 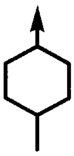 | H | H | $^1$H-NMR (400 MHz, $CDCl_3$) δ: 7.91 (d, J = 7.3 Hz, 1H), 7.24-7.13 (m, 2H), 6.93 (dd, J = 9.1, 1.2 Hz, 1H), 6.69 (d, J = 7.9 Hz, 1H), 5.00 (d, J = 5.5 Hz, 2H), 4.18 (t, J = 8.2 Hz, 2H), 3.86-3.83 (m, 4H), 3.19 (t, J = 8.2 Hz, 2H), 2.99-2.97 (br m, 4H), 2.67-2.44 (m, 1H), 1.93-1.90 (br m, 1H), 1.82-1.61 (m, 4H), 1.48-0.99 (m, 4H), 0.96-0.91 (m, 3H). |
| 473 | 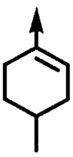 | H | OMe | $^1$H-NMR (400 MHz, $CDCl_3$) δ: 7.91 (br m, 1H), 7.15 (br m, 1H), 6.68 (br m, 1H), 6.28 (s, 1H), 6.19 (s, 1H), 4.98 (s, 2H), 4.18-4.14 (m, 2H), 3.85-3.81 (m, 7H), 3.19-3.15 (m, 2H), 2.99-2.97 (m, 4H), 2.44-2.25 (m, 3H), 1.84-1.73 (m, 3H), 1.35-1.24 (m, 1H), 0.99 (d, J = 6.7 Hz, 3H). |
| 474 | 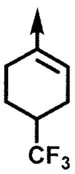 | H | OMe | $^1$H-NMR (400 MHz, DMSO-$d_6$) δ: 7.67 (d, J = 7.9 Hz, 1H), 7.12 (t, J = 7.9 Hz, 1H), 6.68 (d, J = 7.9 Hz, 1H), 6.37-6.34 (m, 2H), 5.00 (s, 2H), 4.20 (t, J = 8.2 Hz, 2H), 3.85 (s, 3H), 3.73 (t, J = 4.3 Hz, 4H), 3.12 (t, J = 8.2 Hz, 2H), 2.93 (t, J = 4.6 Hz, 4H), 2.68 - 2.52 (m, 2H), 2.47-2.30 (m, 2H), 2.24-2.15 (m, 1H), 2.04-1.98 (m, 1H), 1.55-1.44 (m, 1H). |
| 475 | 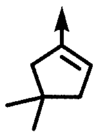 | H | H | $^1$H-NMR (400 MHz, DMSO-$d_6$) δ: 7.90 (d, J = 9.6 Hz, 1H), 7.66 (d, J = 8.0 Hz, 1H), 7.11 (t, J = 8.0 Hz, 1H), 6.95 (d, J = 9.6 Hz, 1H), 6.68 (d, J = 8.0 Hz, 1H), 6.46 (s, 1H), 5.00 (s, 2H), 4.20 (t, J = 8.0 Hz, 2H), 3.74-3.70 (m, 4H), 3.11 (t, J = 8.0 Hz, 2H), 2.94-2.90 (m, 4H), 2.44-2.41 (m, 2H), 2.35-2.31 (m, 2H), 1.09 (s, 6H). |

(continued)

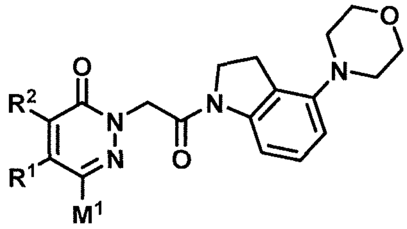

| Example | $M^1$ | $R^1$ | $R^2$ | Analytical data |
|---|---|---|---|---|
| 476 | 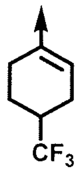 | OMe | H | $^1$H-NMR (400 MHz, $CDCl_3$) $\delta$: 7.90 (d, J = 7.9 Hz, 1H), 7.15 (t, J = 7.9 Hz, 1H), 6.69 (d, J = 7.3 Hz, 1H), 6.35 (br s, 1H), 6.21 (s, 1H), 4.98 (s, 2H), 4.17 (t, J = 8.2 Hz, 2H), 3.85-3.83 (m, 4H), 3.83 (s, 3H), 3.18 (t, J = 8.2 Hz, 2H), 2.99-2.97 (m, 4H), 2.61-2.56 (m, 1H), 2.48-2.25 (m, 4H), 2.12-2.06 (m, 1H), 1.67-1.56 (m, 1H). |
| 477 | 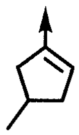 | H | H | $^1$H-NMR (400 MHz, DMSO-$d_6$) $\delta$: 7.90 (d, J = 9.6 Hz, 1H), 7.66 (d, J = 8.0 Hz, 1H), 7.11 (t, J = 8.0 Hz, 1H), 6.95 (d, J = 10.0 Hz, 1H), 6.68 (d, J = 8.0 Hz, 1H), 6.51-6.49 (m, 1H), 5.01 (s, 2H), 4.20 (t, J = 8.0 Hz, 3H), 3.73-3.71 (m, 4H), 3.15-3.10 (m, 2H), 2.94-2.90 (m, 4H), 2.67-2.63 (m, 1H), 2.54-2.52 (m, 1H), 2.24-2.16 (m, 1H), 2.15-2.06 (m, 1H), 1.03 (d, J = 6.8 Hz, 3H). |
| 478 | 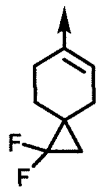 | H | H | $^1$H-NMR (400 MHz, DMSO-$d_6$) $\delta$: 7.91 (d, J = 9.8 Hz, 1H), 7.68 (d, J = 7.9 Hz, 1H), 7.12 (t, J = 7.9 Hz, 1H), 6.97 (d, J = 9.8 Hz, 1H), 6.69 (d, J = 7.9 Hz, 1H), 6.61-6.58 (m, 1H), 5.04 (s, 2H), 4.22 (t, J = 8.2 Hz, 2H), 3.74-3.71 (m, 4H), 3.13 (t, J = 8.2 Hz, 2H), 2.95-2.91 (m, 4H), 2.49-2.41 (m, 3H), 2.34-2.22 (m, 1H), 1.77-1.70 (m, 2H), 1.38-1.23 (m, 2H). |
| 479 | 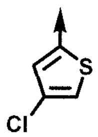 | H | OMe | LC-MS: $[M+H]^+$ / Rt (min) 487.3 / 0.982 (Method A) |
| 480 | 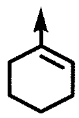 | H | H | LC-MS: $[M+H]^+$ / Rt (min) 421.4 / 1.72 (Method C) |
| 481 | 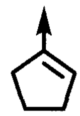 | H | H | LC-MS: $[M+H]^+$ / Rt (min) 407.3 / 1.65 (Method C) |

Examples 482 - 490

According to the method of Example 1, 37, or 50, and common reaction conditions, the compounds of Examples 482 to 490 were obtained by using each corresponding material compounds.

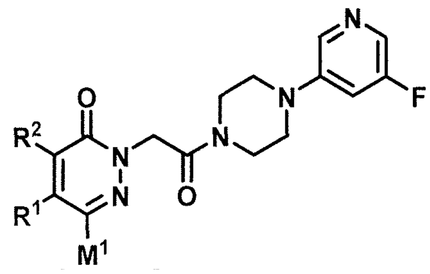

| Example | $M^1$ | $R^1$ | $R^2$ | Analytical data |
|---|---|---|---|---|
| 482 | 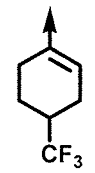 | H | OMe | $^1$H-NMR (400 MHz, DMSO-$d_6$) δ: 8.21-8.20 (m, 1H), 7.95 (d, J = 2.4 Hz, 1H), 7.29 (dt, J = 2.4, 6.4 Hz, 1H), 6.32-6.29 (m, 2H), 4.96 (s, 2H), 3.82 (s, 3H), 3.67-3.64 (m, 2H), 3.59-3.57 (m, 2H), 3.38-3.34 (m, 2H), 3.28-3.26 (m, 2H), 2.61-2.51 (m, 1H), 2.48-2.41 (m, 1H), 2.41-2.29 (m, 2H), 2.23-2.13 (m, 1H), 2.03-1.96 (m, 1H), 1.53-1.43 (m, 1H). |
| 483 | 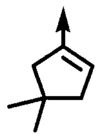 | H | H | $^1$H-NMR (400 MHz, DMSO-$d_6$) δ: 8.21 (s, 1H), 7.96 (s, 1H), 7.89-7.85 (m, 1H), 7.32-7.26 (m, 1H), 6.94-6.90 (m, 1H), 6.43 (s, 1H), 4.97 (s, 2H), 3.69-3.57 (m, 4H), 3.40-3.34 (m, 3H), 2.67-2.63 (m, 1H), 2.43-2.40 (m, 1H), 2.34-2.31 (m, 3H), 1.08 (s, 6H). |
| 484 | 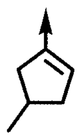 | H | H | $^1$H-NMR (400 MHz, DMSO-$d_6$) δ: 8.21 (s, 1H), 7.96-7.95 (m, 1H), 7.87 (d, J = 10.0 Hz, 1H), 7.31-7.26 (m, 1H), 6.91 (d, J = 9.6 Hz, 1H), 6.48-6.46 (s, 1H), 4.98-4.97 (m, 2H), 3.69-3.65 (m, 2H), 3.61-3.57 (m, 2H), 3.39-3.35 (m, 2H), 3.29-3.26 (m, 2H), 2.81-2.64 (m, 2H), 2.46-2.39 (m, 1H), 2.22-2.15 (m, 1H), 2.14-2.05 (m, 1H), 1.03 (d, J = 7.2 Hz, 3H). |
| 485 | 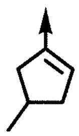 | H | OMe | LC-MS: $[M+H]^+$ / Rt (min) 428.3 / 0.866 (Method A) |
| 486 | 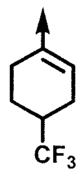 | OMe | H | $^1$H-NMR (400 MHz, $CDCl_3$) δ: 8.14-8.13 (m, 1H), 8.01 (d, J = 2.4 Hz, 1H), 6.88 (dt, J = 11.6, 2.4 Hz, 1H), 6.33-6.32 (br m, 1H), 6.18 (s, 1H), 4.97 (s, 2H), 3.83 (s, 3H), 3.81-3.70 (br m, 4H), 3.34-3.25 (m, 4H), 2.60-2.54 (m, 1H), 2.48-2.24 (m, 4H), 2.12-2.06 (m, 1H), 1.67-1.58 (m, 1H). |
| 487 | 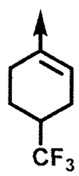 | H | H | $^1$H-NMR (400 MHz, $CDCl_3$) δ: 8.14-8.13 (br m, 1H), 8.01 (d, J = 2.1 Hz, 1H), 7.53 (d, J = 10.1 Hz, 1H), 6.93 (d, J = 10.1 Hz, 1H), 6.89 (dt, J = 11.0, 2.1 Hz, 1H), 6.32-6.30 (br m, 1H), 5.02 (d, J = 15.2 Hz, 1H), 4.98 (d, J = 15.2 Hz, 1H), 3.84-3.71 (m, 4H), 3.36-3.26 (m, 4H), 2.80-2.75 (m, 1H), 2.52-2.45 (m, 1H), 2.37-2.29 (m, 3H), 2.15-2.11 (m, 1H), 1.64-1.55 (m, 1H). |
| 488 | 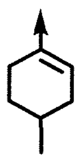 | H | OMe | $^1$H-NMR (400 MHz, $CDCl_3$) δ: 8.13-8.12 (br m, 1H), 8.00 (d, J = 2.4 Hz, 1H), 6.88 (dt, J = 11.6, 2.4 Hz, 1H), 6.26 (d, J = 4.9 Hz, 1H), 6.16 (s, 1H), 4.97 (s, 2H), 3.81 (s, 3H), 3.81-3.68 (m, 4H), 3.33-3.24 (m, 4H), 2.44-2.24 (m, 3H), 1.85-1.71 (m, 3H), 1.35-1.25 (m, 1H), 0.99 (d, J = 6.7 Hz, 3H). |

(continued)

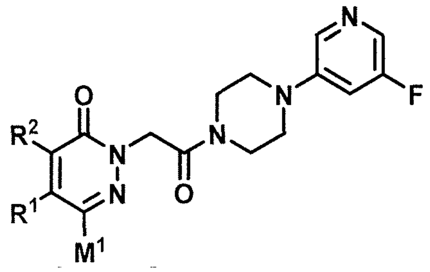

| Example | $M^1$ | $R^1$ | $R^2$ | Analytical data |
|---|---|---|---|---|
| 489 | 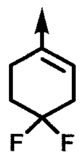 | H | H | LC-MS: $[M+H]^+$ / Rt (min) 434.3 / 0.662 (Method A) |
| 490 | 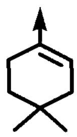 | OMe | H | $^1$H-NMR (400 MHz, $CDCl_3$) δ: 8.13 (t, J = 1.8 Hz, 1H), 8.01 (d, J = 2.4 Hz, 1H), 6.88 (dt, J = 11.0, 2.4 Hz, 1H), 6.26-6.24 (m, 1H), 6.17 (s, 1H), 4.97 (s, 2H), 3.82 (s, 3H), 3.70 (s, 2H), 3.36-3.22 (m, 4H), 2.40-2.30 (m, 2H), 1.99-1.94 (m, 2H), 1.47-1.43 (m, 2H), 0.95 (s, 6H), 0.90-0.85 (m, 2H). |

Examples 491 - 534

According to the method of Example 1, 37, or 50, and common reaction conditions, the compounds of Examples 491 to 534 were obtained by using corresponding material compounds.

| Example | Chemical structure | Analytical data |
|---|---|---|
| 491 | 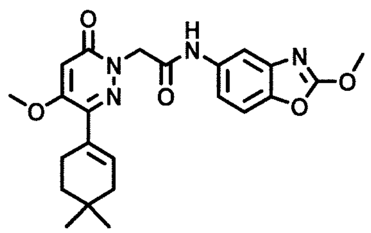 | $^1$H-NMR (400 MHz, $CDCl_3$) δ: 9.02 (s, 1H), 7.78 (s, 1H), 7.24-7.22 (m, 2H), 6.37 (s, 1H), 6.25 (s, 1H), 4.95 (s, 2H), 4.22 (s, 3H), 3.87 (s, 3H), 2.43-2.37 (m, 2H), 2.04-1.99 (m, 2H), 1.49 (t, J = 6.4 Hz, 2H), 0.98 (s, 6H). |
| 492 | 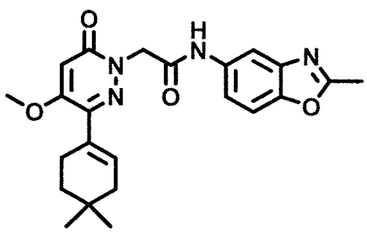 | $^1$H-NMR (400 MHz, $CDCl_3$) δ: 9.07 (s, 1H), 7.87 (s, 1H), 7.45-7.30 (m, 2H), 6.40-6.31 (m, 1H), 6.27-6.21 (m, 1H), 4.94 (s, 2H), 3.85 (s, 3H), 2.62 (s, 3H), 2.39 (s, 2H), 2.00 (s, 2H), 1.53-1.45 (m, 2H), 0.97 (s, 6H). |
| 493 | 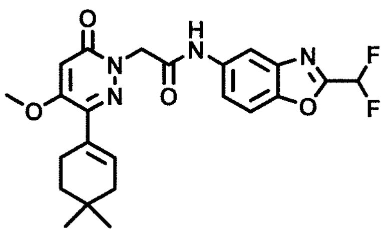 | $^1$H-NMR (400 MHz, $CDCl_3$) δ: 9.38 (s, 1H), 8.16 (s, 1H), 7.52 (s, 1H), 6.79 (t, J = 57.4 Hz, 1H), 6.38 (s, 1H), 6.27 (s, 1H), 4.98 (s, 2H), 3.88 (s, 3H), 2.42-2.39 (m, 2H), 2.04-2.00 (m, 2H), 1.50 (t, J = 6.4 Hz, 2H), 0.98 (s, 6H). |

(continued)

| Example | Chemical structure | Analytical data |
|---|---|---|
| 494 | 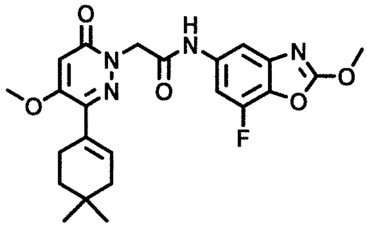 | $^1$H-NMR (400 MHz, $CDCl_3$) δ: 9.34 (s, 1H), 7.39 (d, J = 1.5 Hz, 1H), 7.29 (dd, J = 11.6, 1.5 Hz, 1H), 6.35-6.33 (m, 1H), 6.23 (s, 1H), 4.93 (s, 2H), 4.21 (s, 3H), 3.85 (s, 3H), 2.42-2.35 (m, 2H), 2.02-1.98 (m, 2H), 1.47 (t, J = 6.5 Hz, 2H), 0.95 (s, 6H). |
| 495 | 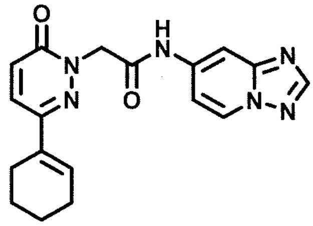 | $^1$H-NMR (400 MHz, $CDCl_3$) δ: 9.70 (s, 1H), 8.43 (d, J = 7.3 Hz, 1H), 8.26 (s, 1H), 8.09 (s, 1H), 7.66 (d, J = 9.8 Hz, 1H), 7.15 (d, J = 7.9 Hz, 1H), 7.03 (d, J = 9.1 Hz, 1H), 6.45 (s, 1H), 5.03 (s, 2H), 2.49-2.43 (m, 2H), 2.33-2.23 (m, 2H), 1.82-1.63 (m, 4H). |
| 496 | 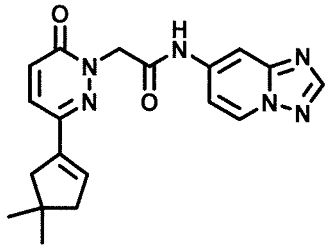 | $^1$H-NMR (400 MHz, $CDCl_3$) δ: 10.13 (s, 1H), 8.30 (d, J = 7.3 Hz, 1H), 8.20 (d, J = 5.5 Hz, 1H), 7.99 (s, 1H), 7.60 (d, J = 10.7 Hz, 1H), 7.14 (dt, J = 16.9, 6.9 Hz, 1H), 7.03-6.95 (m, 1H), 6.26 (s, 1H), 5.04 (s, 2H), 2.54 (s, 2H), 2.39 (s, 2H), 1.18 (s, 6H). |
| 497 | 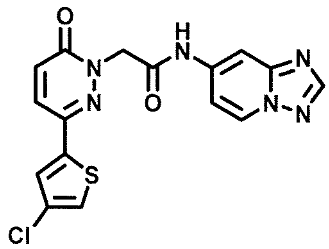 | $^1$H-NMR (400 MHz, DMSO-$d_6$) δ: 10.97 (s, 1H), 8.86 (d, J = 7.9 Hz, 1H), 8.37 (s, 1H), 8.13 (dd, J = 8.8, 5.8 Hz, 2H), 7.82 (d, J = 1.2 Hz, 1H), 7.69 (d, J = 1.2 Hz, 1H), 7.21 (dd, J = 7.3, 1.8 Hz, 1H), 7.14 (d, J = 9.8 Hz, 1H), 4.98 (s, 2H). |
| 498 | 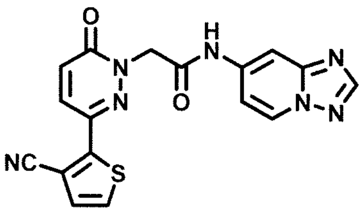 | $^1$H-NMR (400 MHz, $CDCl_3$) δ: 9.25 (s, 1H), 8.46 (d, J = 7.3 Hz, 1H), 8.28 (s, 1H), 8.18 (d, J = 9.8 Hz, 1H), 8.10 (d, J = 1.8 Hz, 1H), 7.50 (d, J = 5.5 Hz, 1H), 7.36 (d, J = 4.9 Hz, 1H), 7.25-7.18 (m, 2H), 5.12 (s, 2H). |
| 499 | 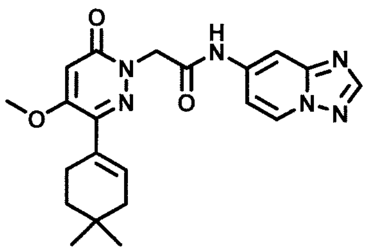 | $^1$H-NMR (400 MHz, $CDCl_3$) δ: 10.20 (s, 1 H), 8.27-8.22 (m, 1 H), 8.15 (s, 1 H), 8.00-7.93 (m, 1H), 7.09-7.02 (m, 1 H), 6.30 (s, 1 H), 6.21 (s, 1 H), 4.98 (s, 2 H), 3.83 (s, 3 H), 2.34 (s, 2 H), 1.95 (s, 2 H), 1.50-1.38 (m, 2 H), 0.94 (s, 6 H). |

(continued)

| Example | Chemical structure | Analytical data |
|---|---|---|
| 500 | 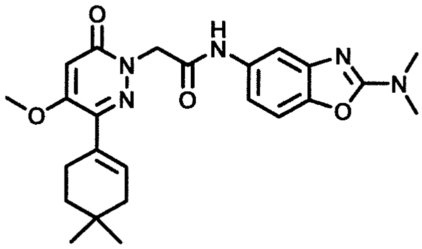 | $^1$H-NMR (400 MHz, $CD_3OD$) δ:8.89 (s, 1 H), 7.49-7.41 (m, 1 H), 7.19-7.12 (m, 1 H), 7.12-7.03 (m, 1 H), 6.30 (s, 1 H), 6.18 (s, 1 H), 4.88 (s, 2 H), 3.77 (s, 3 H), 3.14 (s, 6 H), 2.33 (s, 2 H), 1.99-1.92 (m, 2 H), 1.49-1.38 (m, 2 H), 0.93 (s, 6 H). |
| 501 | 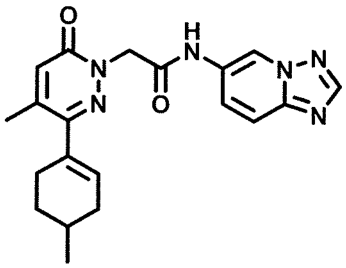 | $^1$H-NMR (400 MHz, $CDCl_3$) δ: 10.00 (s, 1 H), 9.31-9.20 (m, 1 H), 8.08 (s, 1 H), 7.45-7.35 (m, 1 H), 7.23-7.15 (m, 1 H), 6.75 (s, 1 H), 5.75 (s, 1 H), 4.96 (s, 2 H), 2.34-2.14 (m, 6 H), 1.86-1.61 (m, 3 H), 1.38-1.23 (m, 1 H), 0.95 (d, J = 8.0 Hz, 3H), |
| 502 | 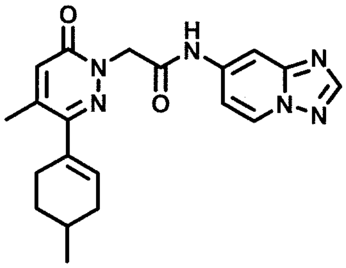 | $^1$H-NMR (400 MHz, $CDCl_3$) δ: 10.10 (s, 1 H), 8.25-8.22 (m, 1 H), 8.11 (s, 1 H), 7.96-7.90 (m, 1 H), 7.06 - 7.00 (m, 1 H), 6.76 (s, 1 H), 5.76 (s, 1 H), 4.95 (s, 2 H), 2.30-2.14 (m, 6 H), 1.79-1.64 (m, 3 H), 1.35-1.23 (m, 1 H), 0.96 (d, J = 8.0 Hz, 3H), |
| 503 | 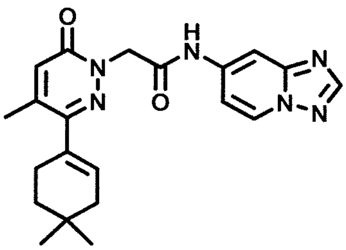 | $^1$H-NMR (400 MHz, $CDCl_3$) δ: 8.71-8.66 (m, 1 H), 8.31 (s, 1 H), 8.23-8.20 (m, 1H), 7.32-7.26 (m, 1 H), 6.85 (s, 1 H), 5.86-5.81 (m, 1 H), 5.00 (s, 2 H), 2.36-2.29 (m, 2 H), 2.26 (s, 3 H), 2.03-1.97 (m, 2 H), 1.55-1.48 (m, 2 H), 0.99 (s, 6 H). |
| 504 | 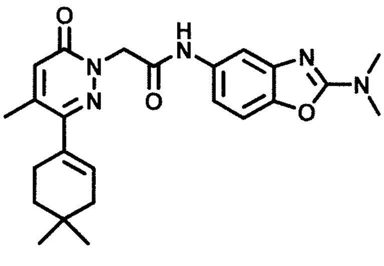 | $^1$H-NMR (400 MHz, $CDCl_3$) 5:7.59-7.55 (m, 1 H), 7.24-7.20 (m, 1 H), 7.19-7.15 (m, 1 H), 6.82 (s, 1 H), 5.84-5.79 (m, 1 H), 4.93 (s, 2 H), 3.16 (s, 6 H), 2.35-2.28 (m, 2 H), 2.23 (s, 3 H), 2.01-1.96 (m, 2 H), 1.53-1.47 (m, 2 H), 0.99 (s, 6 H). |

(continued)

| Example | Chemical structure | Analytical data |
|---|---|---|
| 505 | 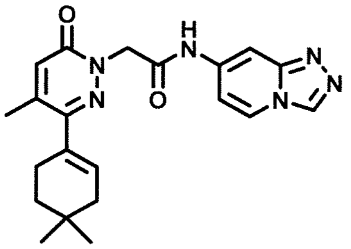 | $^1$H-NMR (400 MHz, $CD_3OD$) δ: 9.05 (s, 1 H), 8.46-8.39 (m, 1 H), 8.26-8.15 (m, 1 H), 7.16-7.02 (m, 1 H), 6.91-6.80 (m, 1 H), 4.98 (s, 2 H), 2.36-2.29 (m, 2 H), 2.27 (s, 3 H), 2.02-1.98 (m, 2 H), 1.55-1.48 (m, 2 H), 0.99 (s, 6 H). |
| 506 | 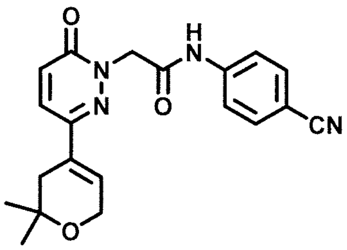 | $^1$H-NMR (400 MHz, $CDCl_3$) δ:9.33 (s, 1 H), 7.58-7.51 (m, 3 H), 7.49-7.45 (m, 2 H), 6.99-6.93 (m, 1 H), 6.96 (s, 1 H), 4.92 (s, 2 H), 3.88-3.77 (m, 2 H), 2.44-2.36 (m, 2 H), 1.37 (s, 6 H). |
| 507 | 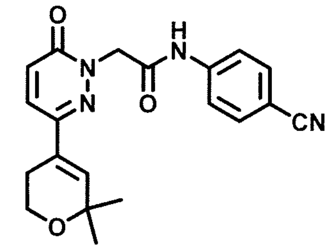 | $^1$H-NMR (400 MHz, $CDCl_3$) δ:9.55 (s, 1 H), 7.61-7.57 (m, 1 H), 7.50-7.46 (m, 2 H), 7.45-7.39 (m, 2 H), 6.99-6.95 (m, 1 H), 6.32 (s, 1 H), 4.94 (s, 2 H), 4.33-4.28 (m, 2 H), 2.38-2.32 (m, 2 H), 1.21 (s, 6 H). |
| 508 | 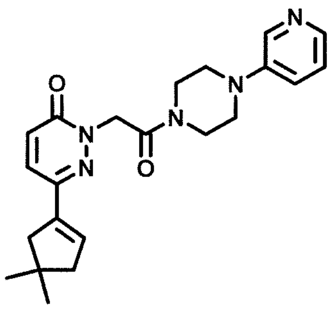 | LC-MS: [M+H] + / Rt (min) 394.4 / 0.690 (Method A) |
| 509 | 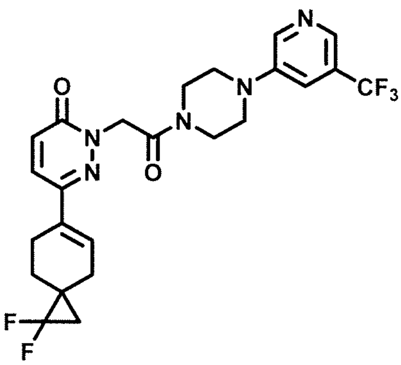 | LC-MS: [M+H] + / Rt (min) 510.3 / 0.944 (Method A) |

(continued)

| Example | Chemical structure | Analytical data |
|---|---|---|
| 510 | 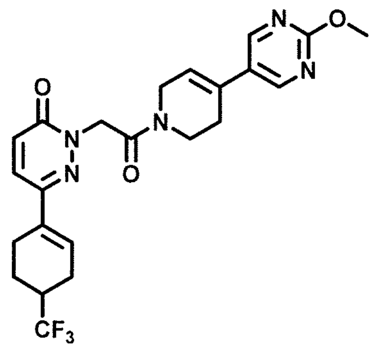 | LC-MS: [M+H] + / Rt (min) 476.3 / 0.876 (Method A) |
| 511 | 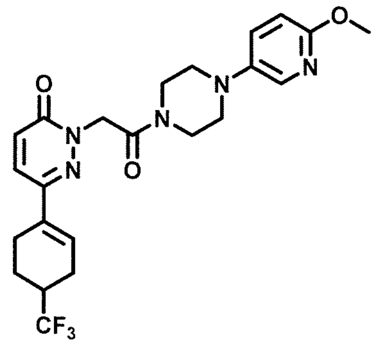 | $^1$H-NMR (400 MHz, DMSO-$d_6$) δ: 7.86 (d, J = 10.0 Hz, 1H), 7.82 (d, J = 6.0 Hz, 1H), 6.93 (d, J = 10.0 Hz, 1H), 6.59-6.57 (m, 1H), 6.54-6.51 (m, 1H), 6.14-6.13 (m, 1H), 4.98 (s, 2H), 3.77 (s, 3H), 3.66-3.61 (m, 2H), 3.57-3.52 (m, 2H), 3.42-3.38 (m, 2H), 3.34-3.31 (m, 2H), 2.67-2.63 (m, 1H), 2.63-2.59 (m, 1H), 2.47-2.42 (m, 1H), 2.33-2.18 (m, 2H), 2.06-2.00 (m, 1H), 1.54-1.43 (m, 1H). |
| 512 | 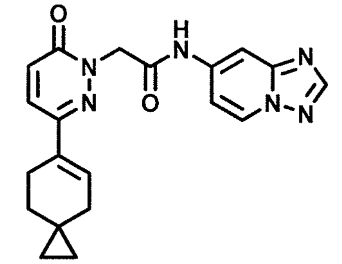 | $^1$H-NMR (400 MHz, DMSO-$d_6$) δ: 10.92 (s, 1H), 8.86 (d, J = 7.3 Hz, 1H), 8.37 (d, J = 9.8 Hz, 1H), 8.12 (d, J = 1.8 Hz, 1H), 7.91 (d, J = 9.8 Hz, 1H), 7.21 (dd, J = 7.3, 1.8 Hz, 1H), 6.97 (d, J = 9.8 Hz, 1H), 6.61 (d, J = 3.7 Hz, 1H), 4.94 (s, 2H), 2.45-2.42 (m, 2H), 2.13-2.12 (m, 2H), 1.46 (t, J = 6.4 Hz, 2H), 0.36-0.31 (m, 4H). |
| 513 | 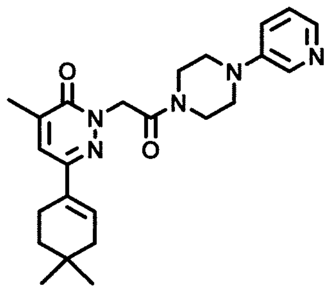 | LC-MS: [M+H] + / Rt (min) 422.4 / 0.776 (Method A) |
| 514 | 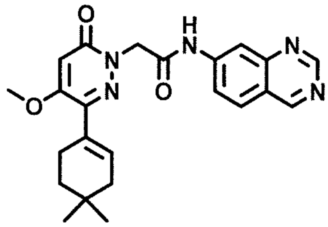 | LC-MS: [M+H] + / Rt (min) 420.1 / 0.914 (Method A) |

(continued)

| Example | Chemical structure | Analytical data |
|---|---|---|
| 515 | 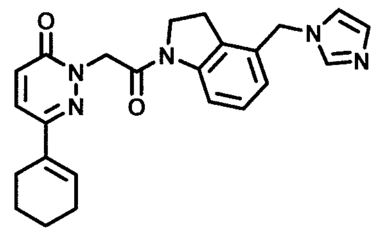 | LC-MS: [M+H] + / Rt (min) 416.3 / 0.671 (Method A) |
| 516 | 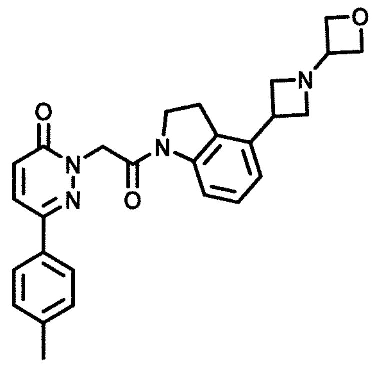 | $^1$H-NMR (400 MHz, DMSO-$d_6$) δ: 8.23 (d, J = 9.8 Hz, 1H), 7.98 (d, J = 7.9 Hz, 1H), 7.93 (d, J = 7.9 Hz, 2H), 7.44 (d, J = 7.9 Hz, 2H), 7.30 (t, J = 7.9 Hz, 1H), 7.25-7.19 (m, 2H), 5.29 (s, 2H), 4.70 (t, J = 6.7 Hz, 2H), 4.51 (t, J = 5.8 Hz, 2H), 4.39 (t, J = 8.5 Hz, 2H), 3.89-3.80 (m, 4H), 3.34-3.28 (m, 2H), 3.26-3.21 (m, 2H), 2.49 (s, 3H). |
| 517 | 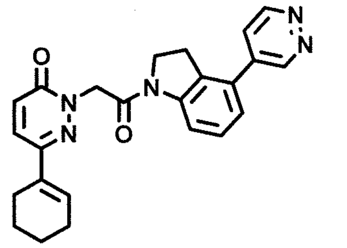 | LC-MS: [M+H] + / Rt (min) 414.0 / 0.781 (Method A) |
| 518 | 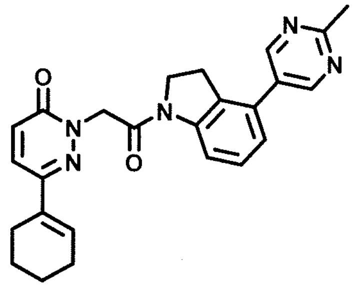 | $^1$H-NMR (400 MHz, DMSO-$d_6$) δ: 8.85 (s, 2H), 8.05 (d, J = 7.9 Hz, 1H), 7.86 (d, J = 9.8 Hz, 1H), 7.31 (t, J = 7.9 Hz, 1H), 7.15 (d, J = 7.3 Hz, 1H), 6.93 (d, J = 9.8 Hz, 1H), 6.56-6.53 (m, 1H), 5.04 (s, 2H), 4.22 (t, J = 8.2 Hz, 2H), 3.33-3.27 (m, 2H), 2.66 (s, 3H), 2.33-2.28 (m, 2H), 2.22-2.17 (m, 2H), 1.66-1.53 (m, 4H). |
| 519 | 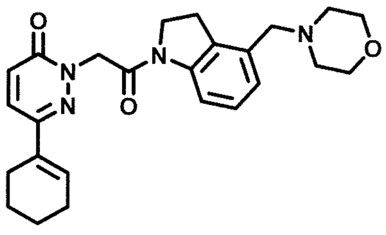 | $^1$H-NMR (400 MHz, DMSO-$d_6$) δ: 7.85 (d, J = 9.8 Hz, 2H), 7.09 (t, J = 7.6 Hz, 1H), 6.93 (t, J = 9.2 Hz, 2H), 6.55-6.53 (m, 1H), 5.00 (s, 2H), 4.21 (t, J = 8.5 Hz, 2H), 3.55-3.52 (m, 4H), 3.40 (s, 2H), 3.21 (t, J = 8.5 Hz, 2H), 2.34-2.27 (m, 6H), 2.21-2.16 (m, 2H), 1.66-1.53 (m, 4H). |

(continued)

| Example | Chemical structure | Analytical data |
|---|---|---|
| 520 | 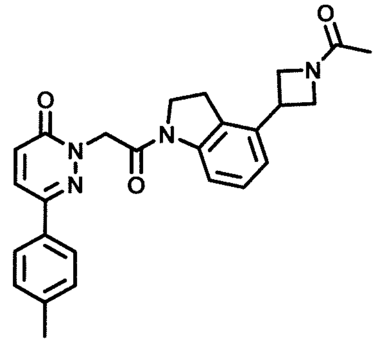 | $^1$H-NMR (400 MHz, DMSO-$d_6$) δ: 8.10 (d, J = 9.8 Hz, 1H), 7.89 (d, J = 7.9 Hz, 1H), 7.79 (d, J = 8.5 Hz, 2H), 7.31 (d, J = 7.9 Hz, 2H), 7.21 (t, J = 7.9 Hz, 1H), 7.14-7.10 (m, 2H), 5.16 (s, 2H), 4.49 (t, J = 8.2 Hz, 1H), 4.30-4.18 (m, 4H), 3.94-3.85 (m, 2H), 3.14 (t, J = 8.5 Hz, 2H), 2.35 (s, 3H), 1.80 (s, 3H). |
| 521 | 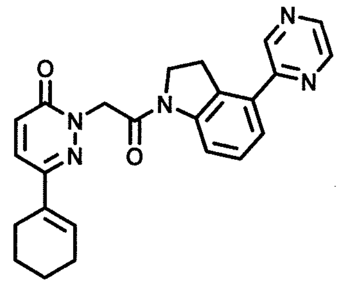 | $^1$H-NMR (400 MHz, DMSO-$d_6$) δ: 9.05 (d, J = 1.8 Hz, 1H), 8.74 (t, J = 2.1 Hz, 1H), 8.62 (d, J = 2.4 Hz, 1H), 8.12 (d, J = 7.9 Hz, 1H), 7.86 (d, J = 9.8 Hz, 1H), 7.52 (d, J = 7.9 Hz, 1H), 7.33 (t, J = 7.9 Hz, 1H), 6.94 (d, J = 9.8 Hz, 1H), 6.56-6.54 (m, 1H), 5.04 (s, 2H), 4.25 (t, J = 8.5 Hz, 2H), 3.49 (t, J = 8.4 Hz, 2H), 2.33-2.28 (m, 2H), 2.23-2.16 (m, 2H), 1.67-1.53 (m, 4H). |
| 522 | 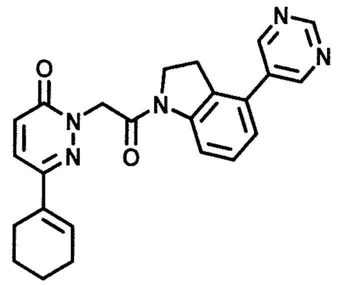 | $^1$H-NMR (400 MHz, DMSO-$d_6$) δ: 9.20 (s, 1H), 8.98 (s, 2H), 8.07 (d, J = 7.9 Hz, 1H), 7.86 (d, J = 9.8 Hz, 1H), 7.33 (t, J = 7.6 Hz, 1H), 7.19 (d, J = 6.7 Hz, 1H), 6.93 (d, J = 9.8 Hz, 1H), 6.56-6.53 (m, 1H), 5.05 (s, 2H), 4.23 (t, J = 8.2 Hz, 2H), 3.34-3.26 (m, 2H), 2.33-2.28 (m, 2H), 2.21-2.18 (m, 2H), 1.66-1.55 (m, 4H). |
| 523 | 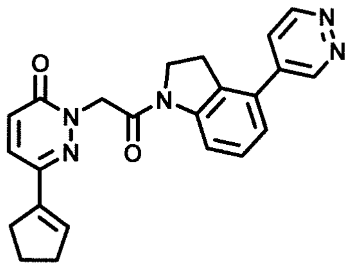 | $^1$H-NMR (400 MHz, DMSO-$d_6$) δ: 9.47-9.45 (m, 1H), 9.33-9.31 (m, 1H), 8.14 (d, J = 7.9 Hz, 1H), 7.94 (d, J = 9.8 Hz, 1H), 7.89-7.87 (m, 1H), 7.38 (t, J = 7.6 Hz, 1H), 7.28 (d, J = 6.7 Hz, 1H), 6.98 (d, J = 9.8 Hz, 1H), 6.62-6.59 (m, 1H), 5.08 (s, 2H), 4.27 (t, J = 8.2 Hz, 2H), 3.38 (t, J = 8.2 Hz, 2H), 3.32-3.29 (m, 2H), 2.63-2.58 (m, 2H), 1.97-1.89 (m, 2H). |
| 524 | 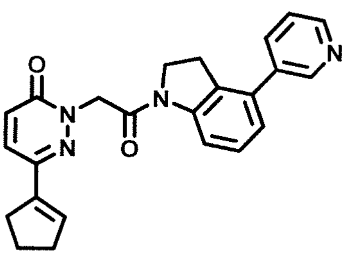 | $^1$H-NMR (400 MHz, DMSO-$d_6$) δ: 8.70-8.69 (m, 1H), 8.58 (dd, J = 4.9, 1.8 Hz, 1H), 8.04 (d, J = 7.9 Hz, 1H), 7.94-7.90 (m, 2H), 7.48 (dd, J = 8.2, 5.2 Hz, 1H), 7.29 (t, J = 7.9 Hz, 1H), 7.11 (d, J = 6.7 Hz, 1H), 6.96 (d, J = 9.8 Hz, 1H), 6.59-6.56 (m, 1H), 5.03 (s, 2H), 4.22 (t, J = 8.2 Hz, 2H), 3.28-3.25 (m, 2H), 2.66-2.56 (m, 4H), 1.95-1.87 (m, 2H). |

(continued)

| Example | Chemical structure | Analytical data |
|---|---|---|
| 525 | 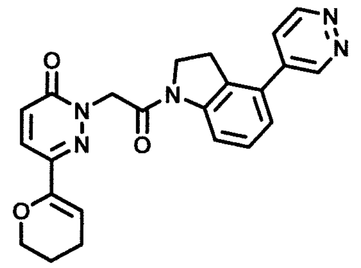 | LC-MS: [M+H] + / Rt (min) 416.3 / 0.740 (Method A) |
| 526 | 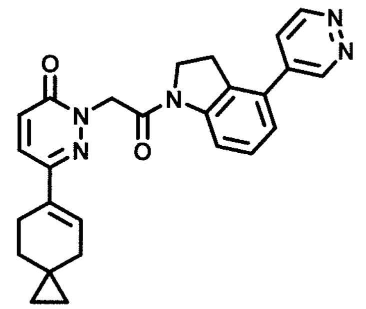 | $^1$H-NMR (400 MHz, DMSO-$d_6$) δ: 9.44 (dd, J = 2.4, 1.2 Hz, 1H), 9.29 (dd, J = 5.5, 1.2 Hz, 1H), 8.12 (d, J = 7.9 Hz, 1H), 7.88 (d, J = 9.8 Hz, 1H), 7.85 (dd, J = 5.5, 2.4 Hz, 1H), 7.36 (t, J = 7.9 Hz, 1H), 7.25 (d, J = 6.7 Hz, 1H), 6.94 (d, J = 9.8 Hz, 1H), 6.58-6.56 (m, 1H), 5.06 (s, 2H), 4.25 (t, J = 8.2 Hz, 2H), 3.36 (t, J = 8.5 Hz, 2H), 3.28-3.26 (m, 2H), 2.12-2.09 (m, 2H), 1.43 (t, J = 6.4 Hz, 2H), 0.31 (d, J = 6.1 Hz, 4H) . |
| 527 | 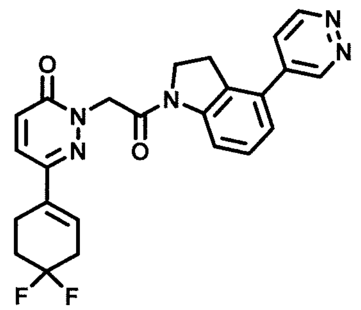 | LC-MS: [M+H] + / Rt (min) 450.3 / 0.773 (Method A) |
| 528 | 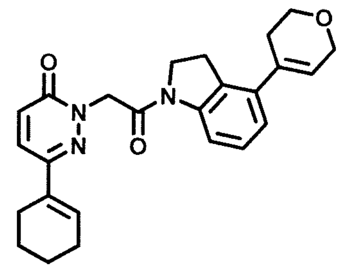 | $^1$H-NMR (400 MHz, DMSO-$d_6$) δ: 7.93-7.87 (m, 2H), 7.17 (t, J = 7.9 Hz, 1H), 6.98-6.94 (m, 2H), 6.58-6.55 (m, 1H), 5.92-5.90 (m, 1H), 5.04 (s, 2H), 4.24-4.19 (m, 4H), 3.81 (t, J = 5.2 Hz, 2H), 3.25 (t, J = 8.2 Hz, 2H), 2.39-2.21 (m, 6H), 1.67-1.58 (m, 4H). |
| 529 | 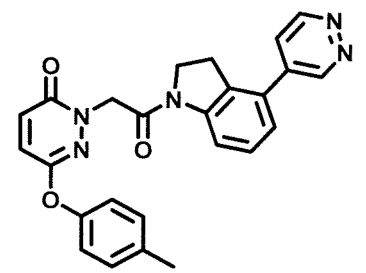 | $^1$H-NMR (400 MHz, DMSO-$d_6$) δ: 9.44 (dd, J = 2.4, 1.2 Hz, 1H), 9.32-9.30 (m, 1H), 8.13 (d, J = 7.9 Hz, 1H), 7.86 (dd, J = 5.5, 2.4 Hz, 1H), 7.47 (d, J = 9.8 Hz, 1H), 7.38 (t, J = 7.9 Hz, 1H), 7.28-7.25 (m, 1H), 7.19 (d, J = 7.9 Hz, 2H), 7.14 (d, J = 9.8 Hz, 1H), 7.09-7.05 (m, 2H), 4.89 (s, 2H), 4.19 (t, J = 8.5 Hz, 2H), 3.34-3.30 (m, 2H), 2.27 (s, 3H). |

(continued)

| Example | Chemical structure | Analytical data |
|---|---|---|
| 530 | 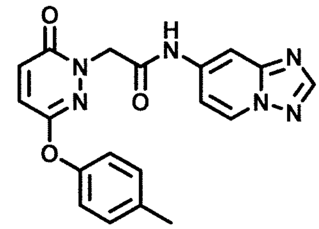 | $^1$H-NMR (400 MHz, DMSO-$d_6$) δ: 10.79 (s, 1H), 8.82 (d, J = 7.3 Hz, 1H), 8.35 (s, 1H), 8.07 (d, J = 1.8 Hz, 1H), 7.44 (d, J = 9.8 Hz, 1H), 7.18-7.04 (m, 6H), 4.72 (s, 2H), 2.24 (s, 3H). |
| 531 | 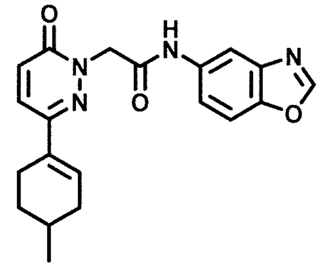 | $^1$H-NMR (400 MHz, DMSO-$d_6$) δ: 10.49 (s, 1H), 8.71-8.70 (m, 1H), 8.11-8.10 (m, 1H), 7.90-7.87 (m, 1H), 7.73-7.70 (m, 1H), 7.53-7.49 (m, 1H), 6.95-6.92 (m, 1H), 6.51-6.49 (m, 1H), 4.89-4.86 (m, 2H), 2.37-2.31 (m, 2H), 2.03-1.99 (m, 2H), 1.44-1.40 (m, 2H), 0.93-0.89 (m, 6H). |
| 532 | 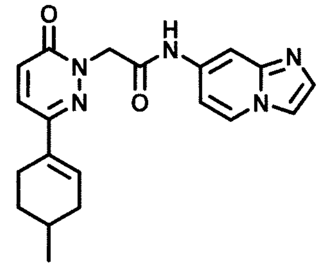 | $^1$H-NMR (400 MHz, DMSO-$d_6$) δ: 10.53 (s, 1H), 8.46-8.44 (m, 1H), 7.88 (d, J = 9.6 Hz, 2H), 7.82 (s, 1H), 7.45-7.44 (m, 1H), 6.96-6.93 (m, 2H), 6.55-6.51 (m, 1H), 4.88 (s, 2H), 2.56-2.52 (m, 1H), 2.36-2.18 (m, 2H), 1.86-1.74 (m, 2H), 1.70-1.61 (m, 1H), 1.28-1.18 (m, 1H), 0.96 (d, J = 6.8 Hz, 3H). |
| 533 | 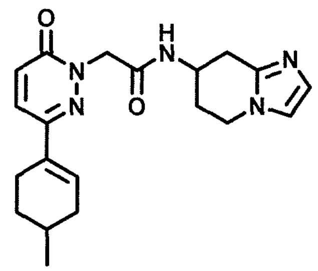 | $^1$H-NMR (400 MHz, $CDCl_3$) δ: 7.55 (d, J = 9.8 Hz, 1H), 7.00-6.98 (m, 1H), 6.88 (d, J = 9.8 Hz, 1H), 6.83-6.79 (m, 2H), 6.34 (s, 1H), 4.83-4.74 (m, 2H), 4.42-4.38 (m, 1H), 4.09-3.96 (m, 2H), 3.24-3.17 (m, 1H), 2.82-2.75 (m, 1H), 2.61-2.54 (m, 1H), 2.35-2.07 (m, 4H), 1.87-1.81 (m, 3H), 1.33-1.24 (m, 2H), 1.01 (d, J = 6.7 Hz, 3H). |
| 534 | 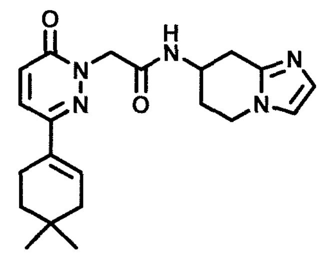 | $^1$H-NMR (400 MHz, $CDCl_3$) δ: 7.57 (d, J = 9.8 Hz, 1H), 6.98 (d, J = 1.2 Hz, 1H), 6.89 (d, J = 9.8 Hz, 1H), 6.80 (d, J = 1.2 Hz, 1H), 6.78 (d, J = 7.3 Hz, 1H), 6.32-6.30 (m, 1H), 4.83-4.74 (m, 2H), 4.44-4.36 (m, 1H), 4.09-3.96 (m, 2H), 3.23-3.17 (m, 1H), 2.81-2.74 (m, 1H), 2.44-2.40 (m, 2H), 2.26-2.19 (m, 1H), 2.16-2.06 (m, 1H), 2.04-2.02 (m, 2H), 1.48 (t, J = 6.4 Hz, 2H), 0.95 (s, 6H). |

Examples 535 and 536

Cis-trans isomers of the compound (35.5 mg) obtained in Example 472 were resolved by chiral column chromatography to obtaine the following compounds (Example 535 and 536):

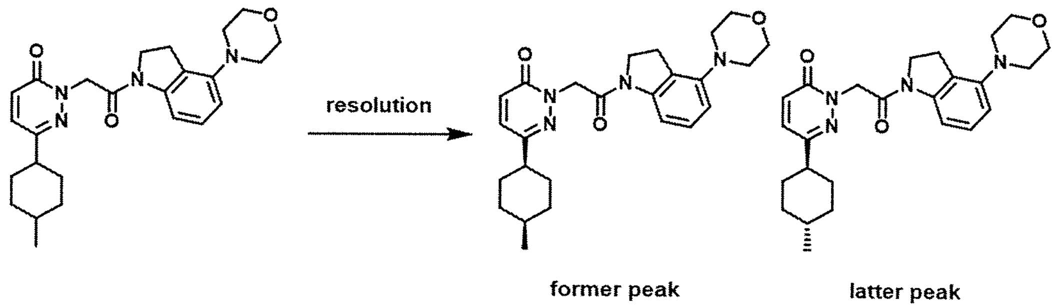


[Resolution conditions]

Detection apparatus: SPD-M20A (Shimadzu Corporation)
HPLC: LC-20AT (Shimadzu Corporation)
Column: CHIRALPAK IA (Daicel Corporation) (S - 5 μm, 20 x 250 mm)
Elution condition: 0.0 - 60.0 (min): A/B = 55:45
Solvent A: hexane with 0.1 % diethylamine
Solvent B: (isopropylalcohol:methanol = 2:1) with 0.1 % diethylamine
Flow rate: 10 ml/min
UV: 220 nm
Column temperature: 40°C

| | Example | Retention time (min.) | Yield (mg) | Purity |
|---|---|---|---|---|
| Former peak | 535 | 31.5 | 13.5 | 99.9% |
| Latter peak | 536 | 41.5 | 14.0 | 99.8% |

Examples 537 and 538

Optical isomers of the compound (14.1 mg) obtained in Example 330 were resolved by chiral column chromatography to obtain the following compounds (Examples 537 and 538):

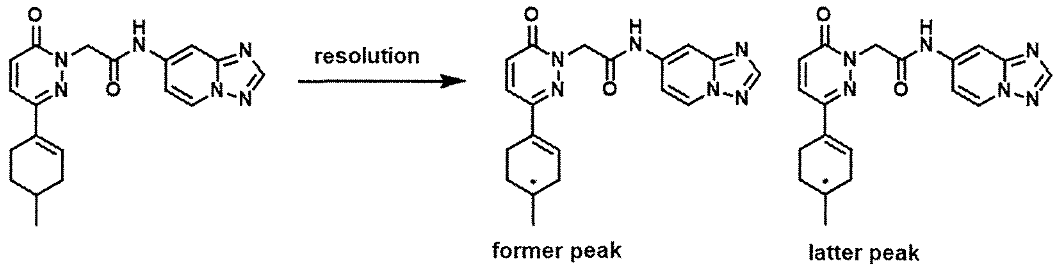


[Resolution conditions]

Detection apparatus: SPD-M20A (Shimadzu Corporation)
HPLC: LC-20AT (Shimadzu Corporation)
Column: CHIRALPAK AY-H (Daicel Corporation) (S - 5 pm, 20 x 250 mm)
Elution condition: 0.0 - 80.0 (min): A/B = 65:35
Solvent A: hexane
Solvent B: isopropylalcohol
Flow rate: 10 ml/min
UV: 220 nm
Column temperature: 40°C

| | Example | Retention time (min) | Yield (mg) | Optical purity |
|---|---|---|---|---|
| Former peak | 537 | 43.5 | 4.5 | 87.8%ee |
| Latter peak | 538 | 56 | 4.8 | 98.6%ee |

Examples 539 and 540

Optical isomers of the compound (13.7 mg) obtained in Example 339 were resolved by chiral column chromatography to obtain the following compounds (Examples 539 and 540):

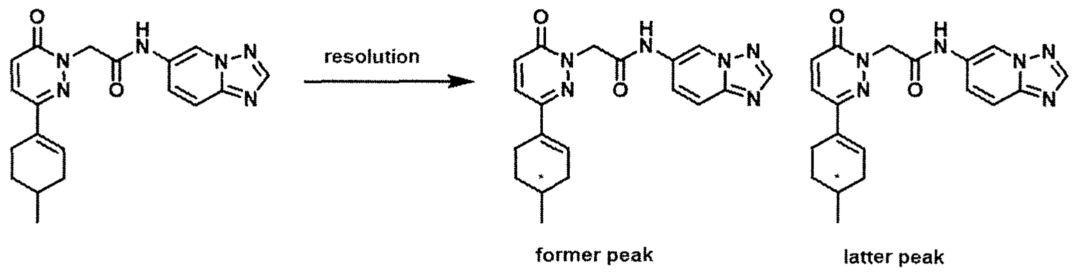


[Resolution conditions]

Detection apparatus: SPD-M20A (Shimadzu Corporation)
HPLC: LC-20AT (Shimadzu Corporation)
Column : CHIRALPAK AY-H (Daicel Corporation) (S - 5 μm, 20 x 250 mm)
Elution condition: 0.0 - 80.0 (min): A/B = 50:50
Solvent A: hexane
Solvent B: isopropylalcohol
Flow rate: 10 ml/min
UV: 220 nm
Column temperature: 40°C

| | Example | Retention time (min.) | Yield (mg) | Optical purity |
|---|---|---|---|---|
| Former peak | 539 | 39.8 | 4.5 | 99%ee |
| Latter peak | 540 | 52.5 | 7.6 | 98.2%ee |

Examples 541 to 571

According to the method of Example 50 and common reaction conditions, the compounds of Examples 541 to 571 were obtained by using each corresponding material compound.

| Example | Chemical structure | Analytical data |
|---|---|---|
| 541 | 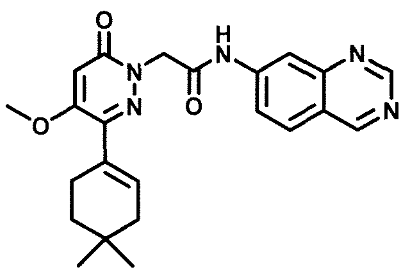 | LC-MS: [M+H] $^{+}$ / Rt (min) 420.1 / 0.914 (Method A) |

(continued)

| Example | Chemical structure | Analytical data |
|---|---|---|
| 542 | 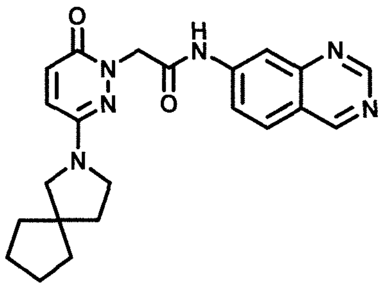 | LC-MS: [M+H] + / Rt (min) 391.3 / 0.761 (Method A) |
| 543 | 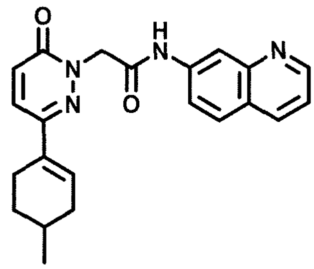 | LC-MS: [M+H] + / Rt (min) 375.1 / 0.661 (Method B) |
| 544 | 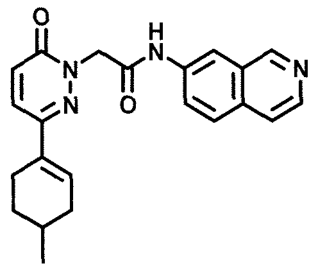 | LC-MS: [M+H] + / Rt (min) 375.0 / 0.745 (Method A) |
| 545 | 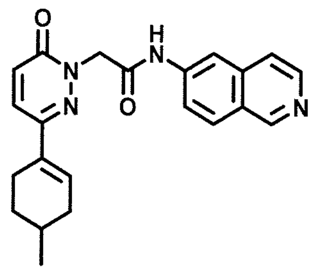 | LC-MS: [M+H] + / Rt (min) 375.0 / 0.725 (Method A) |
| 546 | 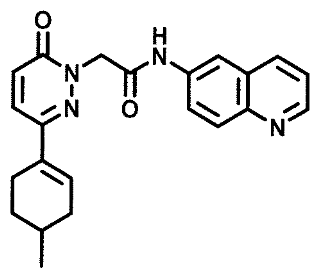 | LC-MS: [M+H] + / Rt (min) 375.0 / 0.784 (Method A) |

(continued)

| Example | Chemical structure | Analytical data |
|---|---|---|
| 547 | 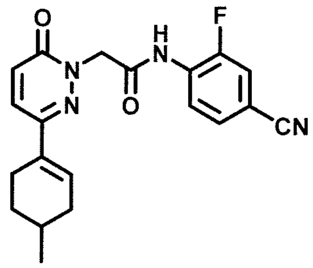 | $^1$H-NMR (400 MHz, $CDCl_3$) δ: 9.36 (s, 1H), 8.52 (t, J = 8.2 Hz, 1H), 7.61 (d, J = 10.4 Hz, 1H), 7.42 (d, J = 8.4 Hz, 1H), 7.37 (dd, J = 10.4, 2.0 Hz), 7.00 (d, J = 9.8 Hz, 1H), 6.38 (s, 1H), 4.99 (s, 2H), 2.66-2.57 (m, 1H), 2.39-2.27 (m, 2H), 1.91-1.80 (m, 2H), 1.77-1.65 (m, 1H), 1.42-1.23 (m, 1H), 1.01 (d, J = 6.7 Hz, 3H). |
| 548 | 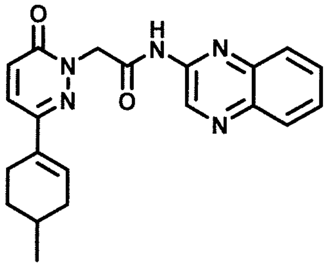 | LC-MS: [M+H] + / Rt (min) 376.0 / 0.995 (Method A) |
| 549 | 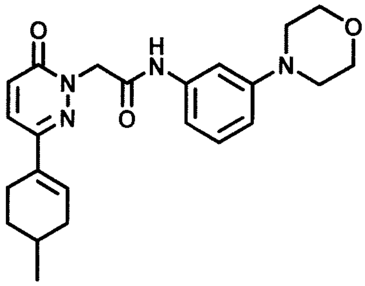 | LC-MS: [M+H] + / Rt (min) 409.1 / 0.958 (Method A) |
| 550 | 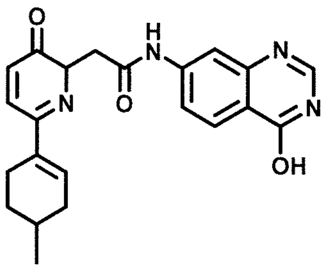 | LC-MS: [M+H] + / Rt (min) 392.0 / 0.791 (Method A) |
| 551 | 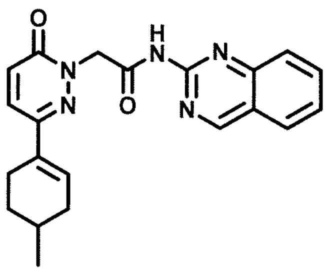 | LC-MS: [M+H] + / Rt (min) 376.1 / 0.964 (Method A) |

(continued)

| Example | Chemical structure | Analytical data |
|---|---|---|
| 552 | 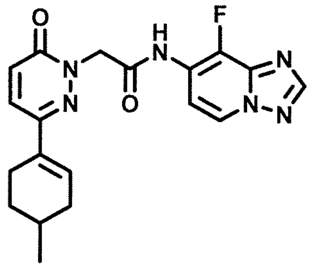 | LC-MS: [M+H] $^{+}$ / Rt (min) 383.0 / 0.838 (Method A) |
| 553 | 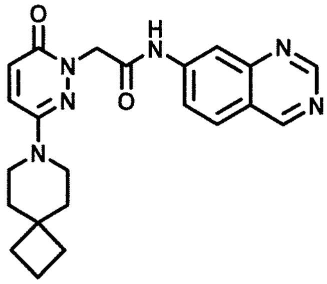 | LC-MS: [M+H] $^{+}$ / Rt (min) 405.0 / 0.834 (Method A) |
| 554 | 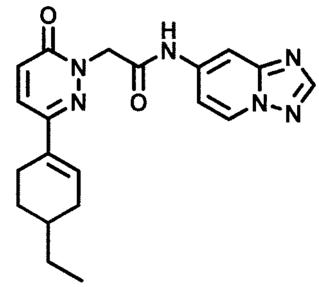 | $^{1}$H-NMR (400 MHz, $CDCl_3$) δ: 9.95 (s, 1H), 8.36 (d, J = 7.3 Hz, 1H), 8.23 (s, 1H), 8.05 (d, J = 1. 8 Hz, 1H), 7.66 (d, J = 9.8 Hz, 1H), 7.13 (dd, J = 7.3, 1.8 Hz, 1H), 7.01 (d, J = 9.8 Hz, 1H), 6.42 (s, 1H), 5.04 (s, 2H), 2.72-2.59 (m, 1H), 2.47-2.26 (m, 2H), 1.98-1.83 (m, 2H), 1.58-1.45 (m, 1H), 1.43-1.25 (m, 3H), 0.93 (t, J = 7.2 Hz, 3H). |
| 555 | 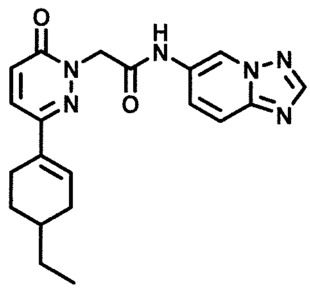 | $^{1}$H-NMR (400 MHz, $CDCl_3$) δ: 9.77 (s, 1H), 9.37 (s, 1H), 8.22 (s, 1H), 7.66 (d, J = 9.6 Hz, 1H), 7.56 (d, J = 9.6 Hz, 1H), 7.30-7.25 (m, 1H), 7.02 (t, J = 10.1 Hz, 1H), 6.41 (s, 1H), 5.05 (s, 2H), 2.70-2.59 (m, 1H), 2.45-2.23 (m, 2H), 1.97-1.80 (m, 2H), 1.59-1.42 (s, 1H), 1.41-1.24 (m, 3H), 0.95 (t, J = 7.2 Hz, 3H). |
| 556 | 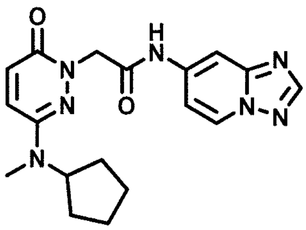 | LC-MS: [M+H] $^{+}$ / Rt (min) 368.2 / 0.693 (Method A) |

(continued)

| Example | Chemical structure | Analytical data |
|---|---|---|
| 557 | 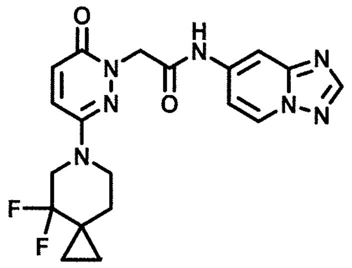 | LC-MS: [M+H] + / Rt (min) 416.2 / 0.710 (Method A) |
| 558 | 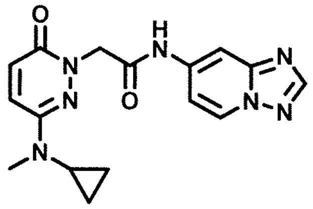 | LC-MS: [M+H] + / Rt (min) 340.1 / 0.609 (Method A) |
| 559 | 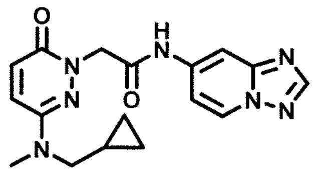 | LC-MS: [M+H] + / Rt (min) 354.2 / 0.651 (Method A) |
| 560 | 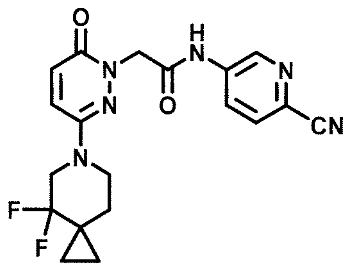 | LC-MS: [M+H] + / Rt (min) 401.1 / 0.804 (Method A) |
| 561 | 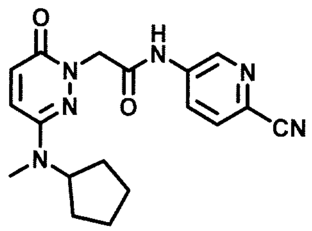 | LC-MS: [M+H] + / Rt (min) 353.2 / 0.834 (Method A) |
| 562 | 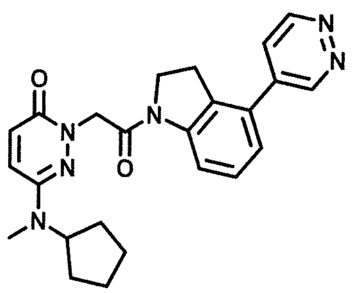 | LC-MS: [M+H] + / Rt (min) 431.3 / 0.822 (Method A) |

(continued)

| Example | Chemical structure | Analytical data |
|---|---|---|
| 563 | 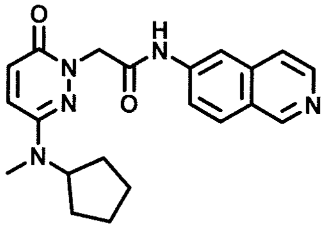 | $^1$H-NMR (400 MHz, $CDCl_3$) δ: 9.85 (s, 1H), 9.10 (s, 1H), 8.43 (d, J = 6.1 Hz, 1H), 8.24 (s, 1H), 7.86 (d, J = 8.6 Hz, 1H), 7.54-7.51 (m, 2H), 7.23 (d, J = 10.4 Hz, 1H), 6.96 (d, J = 9.8 Hz, 1H), 4.90 (s, 2H), 4.37-4.29 (m, 1H), 2.82 (s, 3H), 1.91-1.79 (m, 2H), 1.76-1.65 (m, 2H), 1.40-1.21 (m, 2H), 0.94-0.80 (m, 2H). |
| 564 | 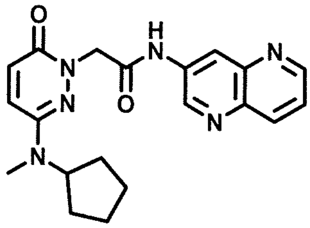 | LC-MS: [M+H] $^+$ / Rt (min) 379.3 / 0.749 (Method A) |
| 565 | 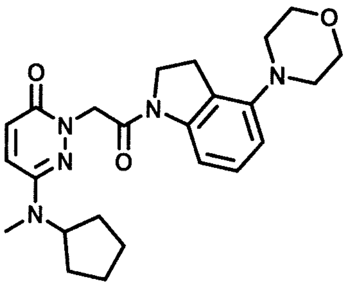 | $^1$H-NMR (400 MHz, $CDCl_3$) δ: 7.94 (d, J = 7.9 Hz, 1H), 7.20-7.13 (m, 2H), 6.88 (d, J = 9.8 Hz, 1H), 6.68 (d, J = 7.9 Hz, 1H), 4.89 (s, 2H), 4.30-4.20 (m, 1H), 3.88-3.82 (m, 4H), 3.16 (t, J = 8.2 Hz, 2H), 3.02-2.93 (m, 4H), 2.77 (s, 3H), 1.90-1.77 (m, 2H), 1.75-1.63 (m, 2H), 1.63-1.50 (m, 6H). |
| 566 | 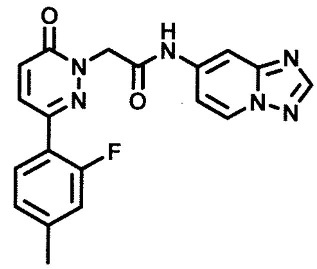 | LC-MS: [M+H] $^+$ / Rt (min) 379.3 / 0.721 (Method A) |
| 567 | 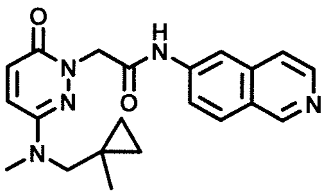 | $^1$H-NMR (400 MHz, $CDCl_3$) δ: 9.71 (s, 1H), 9.09 (s, 1H), 8.43 (d, J = 5.5 Hz, 1H), 8.19 (d, J = 1.8 Hz, 1H), 7.83 (d, J = 9.2 Hz, 1H), 7.57-7.50 (m, 2H), 7.21 (d, J = 9.6 Hz, 1H), 6.95 (d, J = 9.6 Hz, 1H), 4.90 (s, 2H), 3.25 (s, 2H), 3.00 (s, 3H), 1.03 (s, 3H), 0.45-0.42 (m, 2H), 0.39-0.36 (m, 2H). |
| 568 | 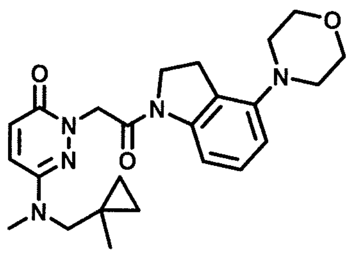 | $^1$H-NMR (400 MHz, $CDCl_3$) δ: 7.92 (s, 1H), 7.17-7.13 (m, 2H), 6.85 (d, J = 9.8 Hz, 1H), 6.68 (d, J = 7.9 Hz, 1H), 4.90 (s, 2H), 3.90-3.79 (m, 4H), 3.25-3.07 (m, 2H), 3.18 (s, 2H), 3.05-2.95 (m, 4H), 2.98 (s, 3H), 1.70-1.55 (m, 2H), 1.03 (s, 3H), 0.45-0.38 (m, 2H), 0.367-0.32 (m, 2H). |

(continued)

| Example | Chemical structure | Analytical data |
|---|---|---|
| 569 | 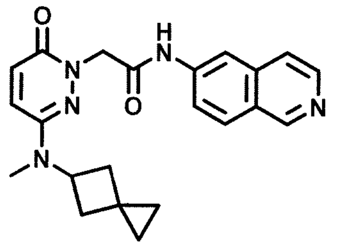 | $^1$H-NMR (400 MHz, $CDCl_3$) δ: 9.91 (s, 1H), 9.04 (s, 1H), 8.38 (d, J = 5.5 Hz, 1H), 8.18 (s, 1H), 7.79 (d, J = 8.6 Hz, 1H), 7.48 (dd, J = 8.4, 2.0 Hz, 1H), 7.43 (d, J = 6.0 Hz, 1H), 7.21 (d, J = 10.4 Hz, 1H), 6.96 (d, J = 10.0 Hz, 1H), 4.93 (s, 2H), 4.50-4.40 (m, 1H), 2.93 (s, 3H), 2.44-2.39 (m, 2H), 2.23-2.18 (m, 2H), 0.55-0.51 (m, 2H), 0.45-0.39 (m, 2H). |
| 570 | 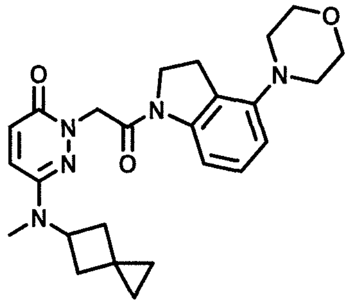 | $^1$H-NMR (400 MHz, $CDCl_3$) δ: 7.95-7.91 (m, 1H), 7.18-7.10 (m, 2H), 6.87 (d, J = 10.1 Hz, 1H), 6.67 (d, J = 8.2 Hz, 1H), 4.88 (s, 2H), 4.40-4.30 (m, 1H), 3.85-3.80 (m, 4H), 3.18-3.10 (m, 2H), 2.98-2.93 (m, 4H), 2.85 (s, 3H), 2.85-2.77 (m, 2H), 2.40-2.32 (m, 1H), 2.17-2.10 (m, 1H), 2.05-2.00 (m, 1H), 1.85-1.70 (m, 1H), 0.50-0.45 (m, 2H), 0.40-0.35 (m, 2H). |
| 571 | 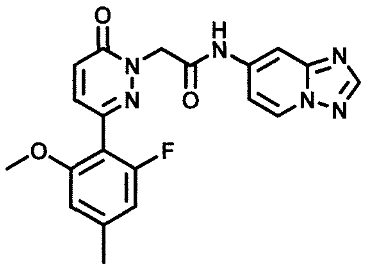 | LC-MS: $[M+H]^+$ / Rt (min) 409.4 / 0.714 (Method A) |

Example 572

2-[6-(4-Methylpiperidin-1-yl)-1H-pyrazolo[3,4-b]pyrazin-1-yl]-N-([1,2,4]triazolo[1,5-a]pyridin-7-yl)acetamide

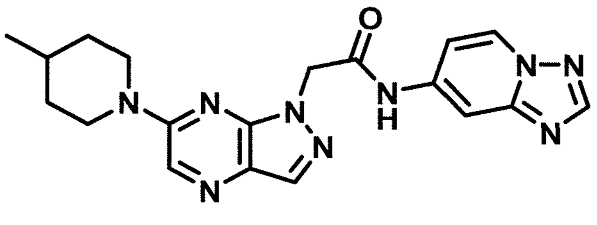

To a solution of the compound of Reference example 33 (50.0 mg) in dichloromethane (2 mL) were added imidazo[1,2-A]pyridine-7-amine hydrochloride (66.4 mg), WSC (61.5 mg), and 4-dimethylaminopyridine (118.0 mg), and the mixture was stirred at room temperature for 2 hours. Water was added thereto, and the mixture was extracted with ethyl acetate. The organic layer was washed with brine, dried over anhydrous sodium sulfate, and concentrated under reduced pressure. The residue was purified by amino silica gel column chromatography (solvent; ethyl acetate, and then ethyl acetate:methanol = 90:10) to obtain the titled compound (14.0 mg) .

$^1$H-NMR (400 MHz, DMSO-$d_6$) δ: 10.96 (s, 1H), 8.86 (d, J = 7.3 Hz, 1H), 8.42 (s, 1H), 8.37 (s, 1H), 8.13-8.06 (m, 2H), 7.22 (dd, dd, J = 7.6, 2.1 Hz, 1H), 5.18 (s, 2H), 4.51-4.44 (m, 2H), 3.00-2.87 (m, 2H), 1.72-1.59 (m, 3H), 1.16-1.02 (m, 2H), 0.88 (d, J = 6.1 Hz, 3H).

Example 573

2-{6-[Methyl(2-methylpropyl)amino]-1H-pyrazolo[3,4-b]pyrazin-1-yl}-N-([1,2,4]triazolo[1,5-a]pyridin-7-yl)acetamide

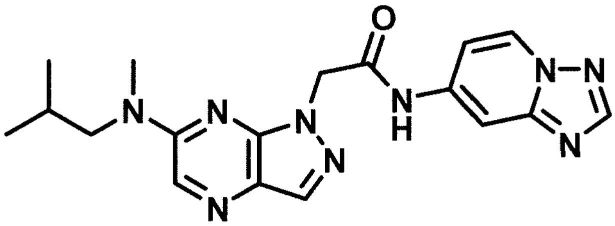

To a solution of the compound of Reference example 35 (15.0 mg) in dimethylformamide (2 mL) were added N,2-dimethylpropane-1-amine hydrochloride (6.8 mg) and potassium carbonate (18.9 mg), and the mixture was stirred at 50°C for 2 hours. Water was added thereto, and the mixture was extracted with ethyl acetate. The organic layer was washed with brine, dried over anhydrous sodium sulfate, and concentrated under reduced pressure. The residue was purified by silica gel column chromatography (solvent; ethyl acetate, and then ethyl acetate:methanol = 98:2) to obtain the titled compound (5.0 mg).

$^1$H-NMR (400 MHz, DMSO-$d_6$) δ: 10.96 (s, 1H), 8.87 (d, J = 7.3 Hz, 1H), 8.38 (s, 1H), 8.26 (s, 1H), 8.12 (d, J = 1.8 Hz, 1H), 8.08 (s, 1H), 7.23 (dd, J = 7.6, 1.8 Hz, 1H), 5.18 (s, 2H), 3.44 (d, J = 7.3 Hz, 2H), 3.15 (s, 3H), 2.08-1.98 (m, 1H), 0.87-0.80 (m, 6H).

Example 574

2-[6-(2-Fluoro-4-methylphenyl)-1H-pyrazolo[3,4-b]pyrazin-1-yl]-N-([1,2,4]triazolo[1,5-a]pyridin-7-yl)acetamide

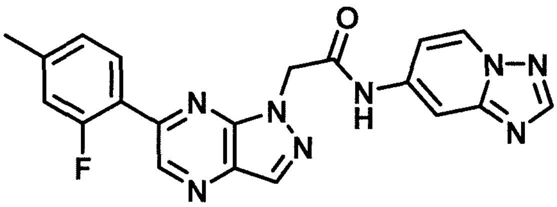

To a solution of the compound of Reference example 35 (25.0 mg) in 1,4-dioxane (2 mL) were added (2-fluoro-4-methylphenyl)boronic acid (15.2 mg) and chloro(2-dicyclohexylphosphino-2',4',6'-triisopropyl-1,1'-biphenyl)[2-(2'-amino-1,1'-biphenyl)]palladium(II) (6.0 mg), and the mixture was stirred at 80°C for 7 hours. Water was added thereto, and the mixture was extracted with ethyl acetate. The organic layer was washed with brine, dried over anhydrous sodium sulfate, and concentrated under reduced pressure. The residue was purified by silica gel column chromatography (solvent; ethyl acetate, and then ethyl acetate:methanol = 98:2) to obtain the titled compound (5.0 mg).

$^1$H-NMR (400 MHz, DMSO-$d_6$) δ: 11.14 (s, 1H), 9.03 (d, J = 2.4 Hz, 1H), 8.86 (d, J = 7.3 Hz, 1H), 8.59 (s, 1H), 8.40 (s, 1H), 8.09 (d, J = 2.4 Hz, 1H), 7.88 (t, J = 7.9 Hz, 1H), 7.26-7.19 (m, 3H), 5.51 (s, 2H), 2.38 (s, 3H).

Example 575

2-[6-(5-Methyl-1,3-thiazol-2-yl)-1H-pyrazolo[3,4-b]pyrazin-1-yl]-N-([1,2,4]triazolo[1,5-a]pyridin-7-yl)acetamide

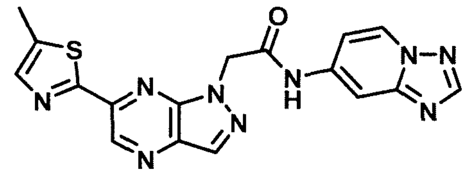

To a solution of the compound of Reference example 35 (15.0 mg) in 1,2-dimethoxyethane (1 mL) were 5-methyl-2-(tributylstannyl)thiazole (39.0 mg) and tetrakis(triphenylphosphine)palladium(0) (5.3 mg), and the mixture was subjected to microwave irradiation, and stirred at 150°C for 20 minutes. Water was added thereto, and the mixture was extracted with chloroform. The organic layer was washed with brine, dried over anhydrous sodium sulfate, and concentrated under reduced pressure. The residue was purified by silica gel column chromatography (solvent; chloroform:methanol = 98: 2, and then chloroform:methanol = 90:10) to obtain the titled compound (5.8 mg).
LC-MS: $[M+H]^+$ / Rt (min) 391.9 / 0.650 (Method A)

Example 576

2-(6-Cyclobutyl-1H-pyrazolo[3,4-b]pyrazin-1-yl)-N-([1,2,4]triazolo[1,5-a]pyridin-7-yl)acetamide

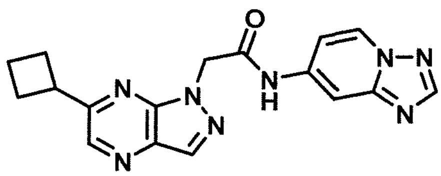

To a solution of the compound of Reference example 35 (60.0 mg) in tetrahydrofuran (2 mL) were added 0.5 mol/L cyclobutylzinc bromide (0.548 mL) and bis(tri-tert-butylphosphine)palladium(0) (18.7 mg), and the mixture was stirred at room temperature for 3 hours. Water was added thereto, and the mixture was extracted with ethyl acetate. The organic layer was washed with brine, dried over anhydrous sodium sulfate, and concentrated under reduced pressure. The residue was purified by silica gel column chromatography (solvent; ethyl acetate, and then ethyl acetate:methanol = 90:10) to obtain the titled compound (27.0 mg).
$^1$H-NMR (400 MHz, DMSO-$d_6$) δ: 11.10 (s, 1H), 8.88 (d, J = 7.3 Hz, 1H), 8.63 (s, 1H), 8.48 (s, 1H), 8.38 (s, 1H), 8.11 (d, J = 2.1 Hz, 1H), 7.24 (dd, J = 7.6, 2.1 Hz, 1H), 5.46 (s, 2H), 3.97-3.89 (m, 1H), 2.41-2.31 (m, 4H), 2.11-1.99 (m, 1H), 1.92-1.83 (m, 1H).

Examples 577 - 640

According to the methods of Reference examples 32 - 36 or Examples 572 - 576, and common reaction conditions, the compounds of Examples 577 - 640 were obtained by using each corresponding material compound.

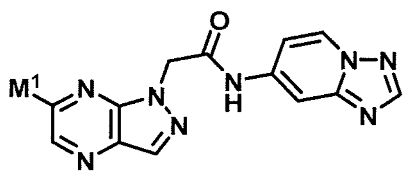

| Example | $M^1$ | Analytical data |
|---|---|---|
| 577 | 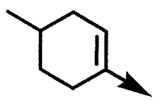 | $^1$H-NMR (400 MHz, DMSO-$d_6$) δ: 11.02 (s, 1H), 8.89 (s, 1H), 8.78 (d, J = 7.3 Hz, 1H), 8.37 (s, 1H), 8.29 (s, 1H), 8.01 (d, J = 1.2 Hz, 1H), 7.16-7.13 (m, 1H), 6.93-6.91 (m, 1H), 5.36-5.34 (m, 2H), 2.69-2.64 (m, 1H), 2.34-2.27 (m, 2H), 1.84-1.72 (m, 2H), 1.67-1.60 (m, 1H), 1.27-1.17 (m, 1H), 0.90 (d, J = 6.7 Hz, 3H). |

(continued)

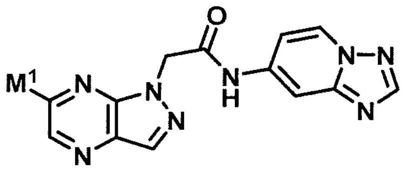

| Example | $M^1$ | Analytical data |
|---|---|---|
| 578 | 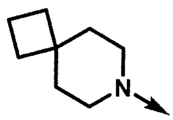 | $^1$H-NMR (400 MHz, $CDCl_3$) δ: 9.05 (s, 1H), 8.46 (d, J = 7.3 Hz, 1H), 8.27 (d, J = 4.3 Hz, 2H), 8.17 (s, 1H), 8.08 (d, J = 1.8 Hz, 1H), 7.13 (dd, J = 7.3, 1.8 Hz, 1H), 5.15 (s, 2H), 3.71-3.68 (m, 4H), 2.01-1.93 (m, 2H), 1.87-1.82 (m, 4H), 1.72-1.68 (m, 4H). |
| 579 | 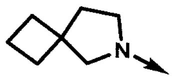 | $^1$H-NMR (400 MHz, DMSO-$d_6$) δ: 10.35 (s, 1H), 8.24-8.22 (m, 1H), 7.73 (s, 1H), 7.48 (dd, J = 2.3, 0.9 Hz, 1H), 7.45 (s, 1H), 7.41 (s, 1H), 6.60 (dd, J = 7.5, 2.3 Hz, 1H), 4.54 (s, 2H), 2.92-2.87 (m, 4H), 1.39-1.19 (m, 8H). |
| 580 | 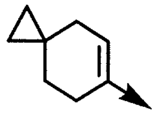 | $^1$H-NMR (400 MHz, DMSO-$d_6$) δ: 11.08 (s, 1H), 8.99 (s, 1H), 8.87 (d, J = 7.3 Hz, 1H), 8.47 (s, 1H), 8.37 (s, 1H), 8.09 (d, J = 1.8 Hz, 1H), 7.23 (dd, J = 7.3, 1.8 Hz, 1H), 7.05-7.03 (m, 1H), 5.44 (s, 2H), 2.67-2.64 (m, 2H), 2.19-2.18 (m, 2H), 1.51 (t, J = 6.1 Hz, 2H), 0.38-0.32 (m, 4H). |
| 581 | 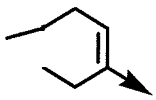 | $^1$H-NMR (400 MHz, DMSO-$d_6$) δ: 11.10 (s, 1H), 8.99 (s, 1H), 8.86 (d, J = 7.3 Hz, 1H), 8.46 (s, 1H), 8.37 (s, 1H), 8.09 (d, J = 2.1 Hz, 1H), 7.23 (dd, J = 7.3, 2.1 Hz, 1H), 7.00-6.98 (m, 1H), 5.43 (s, 2H), 2.60-2.57 (m, 2H), 2.09-2.08 (m, 2H), 1.49 (t, J = 6.4 Hz, 2H), 0.94 (s, 6H). |
| 582 | 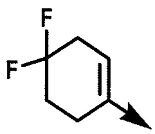 | $^1$H-NMR (400 MHz, DMSO-$d_6$) δ: 11.06 (s, 1H), 9.05-9.02 (m, 1H), 8.87 (d, J = 6.7 Hz, 1H), 8.51 (s, 1H), 8.37 (s, 1H), 8.09 (d, J = 1.8 Hz, 1H), 7.23 (dd, J = 7.6, 2.1 Hz, 1H), 6.88 (s, 1H), 5.45 (s, 2H), 2.90-2.82 (m, 4H), 2.25-2.15 (m, 2H). |
| 583 | 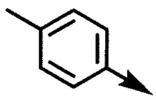 | $^1$H-NMR (400 MHz, DMSO-$d_6$) δ: 11.07 (s, 1H), 9.30 (s, 1H), 8.86 (d, J = 7.3 Hz, 1H), 8.53 (s, 1H), 8.36 (s, 1H), 8.16 (d, J = 7.3 Hz, 2H), 8.09 (s, 1H), 7.36 (d, J = 6.7 Hz, 2H), 7.23 (d, J = 6.7 Hz, 1H), 5.51 (s, 2H), 2.37 (s, 3H). |
| 584 | 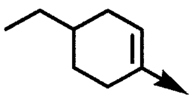 | $^1$H-NMR (400 MHz, DMSO-$d_6$) δ: 11.04 (s, 1H), 8.95 (s, 1H), 8.85 (d, J = 7.9 Hz, 1H), 8.44 (s, 1H), 8.36 (s, 1H), 8.08 (d, J = 1.8 Hz, 1H), 7.21 (dd, J = 7.3, 1.8 Hz, 1H), 6.99 (s, 1H), 5.41 (s, 2H), 2.74 (d, J = 14.6 Hz, 1H), 2.44-2.36 (m, 2H), 1.90-1.86 (m, 2H), 1.47 (s, 1H), 1.35-1.25 (m, 3H), 0.92-0.86 (m, 3H). |
| 585 | 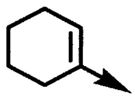 | $^1$H-NMR (400 MHz, DMSO-$d_6$) δ: 11.03 (s, 1H), 8.95 (s, 1H), 8.85 (d, J = 7.3 Hz, 1H), 8.44 (s, 1H), 8.36 (s, 1H), 8.08 (d, J = 2.4 Hz, 1H), 7.21 (dd, J = 7.3, 2.4 Hz, 1H), 7.01 (s, 1H), 5.41 (s, 2H), 2.54 (s, 2H), 2.26 (s, 2H), 1.71-1.60 (m, 4H). |
| 586 | 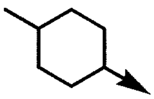 | LC-MS: $[M+H]^+$ / Rt (min) 391.0 / 0.830, 0.854 (Method A) (cis-trans mixture) |

(continued)

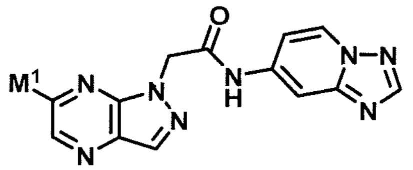

| Example | $M^1$ | Analytical data |
|---|---|---|
| 587 | 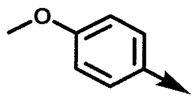 | $^1$H-NMR (400 MHz, DMSO-$d_6$) δ: 11.13 (s, 1H), 9.30 (s, 1H), 8.88 (d, J = 7.3 Hz, 1H), 8.52 (s, 1H), 8.38 (s, 1H), 8.27-8.23 (m, 2H), 8.11 (d, J = 2.4 Hz, 1H), 7.26 (dd, J = 7.6, 2.4 Hz, 1H), 7.13-7.09 (m, 2H), 5.52 (s, 2H), 3.84 (s, 3H). |
| 588 | 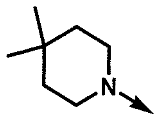 | $^1$H-NMR (400 MHz, DMSO-$d_6$) δ: 10.99 (s, 1H), 8.87 (d, J = 8.5 Hz, 1H), 8.43 (s, 1H), 8.38 (s, 1H), 8.12 (d, J = 1.8 Hz, 1H), 8.09 (s, 1H), 7.23 (dd, J = 7.6, 1.8 Hz, 1H), 5.19 (s, 2H), 3.73-3.69 (m, 4H), 1.38-1.35 (m, 4H), 0.95 (s, 6H). |
| 589 | 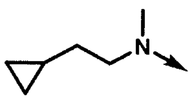 | $^1$H-NMR (400 MHz, DMSO-$d_6$) δ: 10.97 (s, 1H), 8.87 (d, J = 6.7 Hz, 1H), 8.38 (s, 1H), 8.25 (s, 1H), 8.12 (d, J = 2.4 Hz, 1H), 8.08 (s, 1H), 7.23 (dd, J = 7.3, 2.4 Hz, 1H), 5.18 (s, 2H), 3.67 (t, J = 7.3 Hz, 2H), 3.15 (s, 3H), 1.44 (q, J = 7.1 Hz, 2H), 0.65-0.58 (m, 1H), 0.30-0.25 (m, 2H), -0.01--0.05 (m, 2H). |
| 590 | 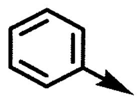 | $^1$H-NMR (400 MHz, DMSO-$d_6$) δ: 11.13 (s, 1H), 9.35 (s, 1H), 8.88 (d, J = 7.3 Hz, 1H), 8.58 (s, 1H), 8.37 (s, 1H), 8.29-8.26 (m, 2H), 8.11 (d, J = 2.4 Hz, 1H), 7.60-7.53 (m, 3H), 7.25 (dd, J = 7.6, 2.4 Hz, 1H), 5.54 (s, 2H). |
| 591 | 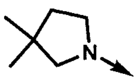 | LC-MS: $[M+H]^+$ / Rt (min) 392.0 / 0.724 (Method A) |
| 592 | 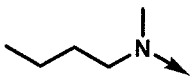 | $^1$H-NMR (400 MHz, DMSO-$d_6$) δ: 10.97 (s, 1H), 8.87 (d, J = 7.3 Hz, 1H), 8.12 (d, J = 2.4 Hz, 1H), 7.99 (s, 1H), 7.96 (s, 1H), 7.74 (t, J = 5.5 Hz, 1H), 7.23 (dd, J = 7.3, 2.4 Hz, 1H), 5.14 (s, 2H), 3.27-3.21 (m, 2H), 3.12-3.06 (m, 1H), 1.68-1.56 (m, 1H), 1.44-1.26 (m, 1H), 1.16-1.02 (m, 1H), 0.88-0.75 (m, 6H). |
| 593 | 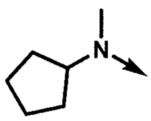 | $^1$H-NMR (400 MHz, $CDCl_3$) δ: 9.15 (s, 1H), 8.45 (d, J = 7.3 Hz, 1H), 8.26 (s, 1H), 8.18 (s, 1H), 8.16 (s, 1H), 8.08 (d, J = 1.8 Hz, 1H), 7.14 (dd, J = 7.3, 2.3 Hz, 1H), 5.17 (s, 2H), 5.02-4.93 (m, 1H), 3.07 (s, 3H), 2.01-1.93 (m, 2H), 1.76-1.67 (m, 6H). |
| 594 | 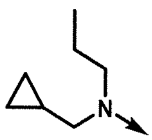 | $^1$H-NMR (400 MHz, $CDCl_3$) δ: 9.12 (s, 1H), 8.46 (d, J = 7.3 Hz, 1H), 8.27 (s, 1H), 8.16 (s, 1H), 8.15 (s, 1H), 8.08 (d, J = 2.4 Hz, 1H), 7.15 (dd, J = 7.3, 2.4 Hz, 1H), 5.14 (s, 2H), 3.62-3.58 (m, 2H), 3.52 (d, J = 6.7 Hz, 2H), 1.77-1.69 (m, 2H), 1.16-1.07 (m, 1H), 0.98 (t, J = 7.3 Hz, 3H), 0.60-0.55 (m, 2H), 0.35-0.31 (m, 2H). |
| 595 | 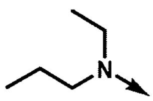 | $^1$H-NMR (400 MHz, DMSO-$d_6$) δ: 10.97 (s, 1H), 8.87 (d, J = 7.3 Hz, 1H), 8.38 (s, 1H), 8.21 (s, 1H), 8.12 (d, J = 2.4 Hz, 1H), 8.07 (s, 1H), 7.23 (dd, J = 7.3, 2.4 Hz, 1H), 5.17 (s, 2H), 3.60 (q, J = 7.3 Hz, 2H), 3.49 (t, J = 7.3 Hz, 2H), 1.63-1.53 (m, 2H), 1.12 (t, J = 7.3 Hz, 3H), 0.83 (t, J = 7.3 Hz, 3H). |

(continued)

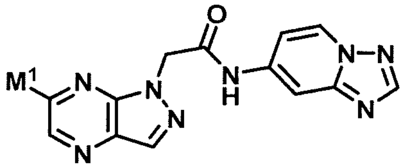

| Example | $M^1$ | Analytical data |
|---|---|---|
| 596 | 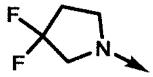 | LC-MS: $[M+H]^+$ / Rt (min) 400.0 / 0.616 (Method A) |
| 597 | 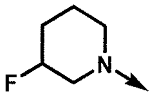 | LC-MS: $[M+H]^+$ / Rt (min) 396.1 / 0.602 (Method A) |
| 598 | 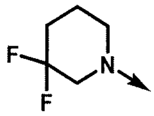 | LC-MS: $[M+H]^+$ / Rt (min) 414.1 / 0.635 (Method A) |
| 599 | 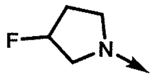 | LC-MS: $[M+H]^+$ / Rt (min) 382.0 / 0.556 (Method A) |
| 600 | 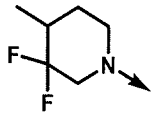 | LC-MS: $[M+H]^+$ / Rt (min) 428.0 / 0.703 (Method A) |
| 601 | 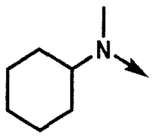 | $^1$H-NMR (400 MHz, $CDCl_3$) δ: 9.26 (br s, 1H), 8.44 (d, J = 7.3 Hz, 1H), 8.25 (s, 1H), 8.14 (s, 2H), 8.07 (d, J = 2.4 Hz, 1H), 7.15 (dd, J = 7.3, 2.4 Hz, 1H), 5.15 (s, 2H), 4.36 (br s, 1H), 3.06 (s, 3H), 1.95-1.72 (m, 6H), 1.59-1.39 (m, 4H). |
| 602 | 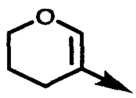 | $^1$H-NMR (400 MHz, DMSO-$d_6$) δ: 11.05 (s, 1H), 8.87-8.83 (m, 2H), 8.36-8.35 (m, 2H), 8.08 (d, J = 1.8 Hz, 1H), 7.92 (s, 1H), 7.21 (dd, J = 7.3, 1.8 Hz, 1H), 5.36 (s, 2H), 4.06 (t, J = 5.2 Hz, 2H), 2.51-2.48 (m, 2H), 1.93-1.87 (m, 2H). |
| 603 | 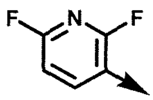 | LC-MS: $[M+H]^+$ / Rt (min) 407.9 / 0.675 (Method A) |
| 604 | 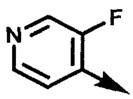 | LC-MS: $[M+H]^+$ / Rt (min) 390.0 / 0.568 (Method A) |
| 605 | 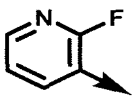 | LC-MS: $[M+H]^+$ / Rt (min) 390.0 / 0.587 (Method A) |
| 606 | 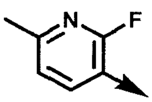 | LC-MS: $[M+H]^+$ / Rt (min) 404.0 / 0.638 (Method A) |

(continued)

| 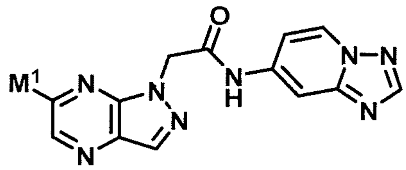 | | |
|---|---|---|
| Example | $M^1$ | Analytical data |
| 607 | 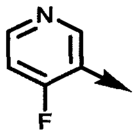 | LC-MS: $[M+H]^+$ / Rt (min) 389.9 / 0.545 (Method A) |
| 608 | 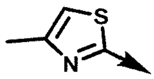 | LC-MS: $[M+H]^+$ / Rt (min) 391.9 / 0.651 (Method A) |
| 609 | 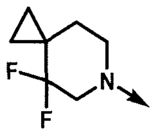 | $^1H$-NMR (400 MHz, $CDCl_3$) δ: 8.97 (s, 1H), 8.45 (d, J = 7.3 Hz, 1H), 8.31 (s, 1H), 8.26 (s, 1H), 8.21 (s, 1H), 8.06 (d, J = 1.8 Hz, 1H), 7.13 (dd, J = 7.8, 2.3 Hz, 1H), 5.17 (s, 2H), 4.05 (t, J = 10.7 Hz, 2H), 3.91 (t, J = 5.5 Hz, 2H), 1.76 (t, J = 5.5 Hz, 2H), 1.01 (t, J = 5.5 Hz, 2H), 0.55-0.53 (m, 2H). |
| 610 | 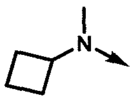 | $^1H$-NMR (400 MHz, DMSO-$d_6$) δ: 10.94 (s, 1H), 8.85 (d, J = 7.3 Hz, 1H), 8.35 (s, 1H), 8.23 (s, 1H), 8.10-8.05 (m, 2H), 7.21 (dd, J = 7.3, 2.4 Hz, 1H), 5.17 (s, 2H), 4.80-4.72 (m, 1H), 3.07 (s, 3H), 2.20-2.11 (m, 4H), 1.73-1.51 (m, 2H). |
| 611 | 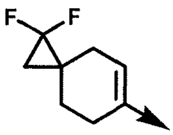 | $^1H$-NMR (400 MHz, DMSO-$d_6$) δ: 11.09 (s, 1H), 9.02 (s, 1H), 8.87 (d, J = 7.3 Hz, 1H), 8.49 (s, 1H), 8.38 (s, 1H), 8.10 (d, J = 2.4 Hz, 1H), 7.23 (dd, J = 7.3, 2.4 Hz, 1H), 7.06-7.04 (m, 1H), 5.45 (s, 2H), 2.72-2.67 (m, 2H), 2.55-2.51 (m, 1H), 2.36-2.31 (m, 1H), 1.86-1.75 (m, 2H), 1.41-1.30 (m, 2H). |
| 612 | 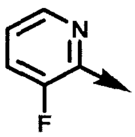 | LC-MS: $[M+H]^+$ / Rt (min) 390.0 / 0.571 (Method A) |
| 613 | 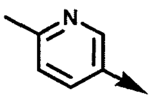 | LC-MS: $[M+H]^+$ / Rt (min) 386.0 / 0.450 (Method A) |
| 614 | 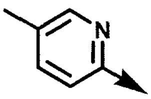 | LC-MS: $[M+H]^+$ / Rt (min) 386.0 / 0.629 (Method A) |
| 615 | 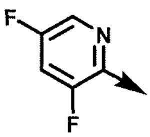 | LC-MS: $[M+H]^+$ / Rt (min) 408.2 / 0.631 (Method A) |
| 616 | 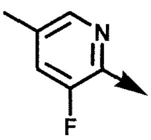 | LC-MS: $[M+H]^+$ / Rt (min) 404.0 / 0.636 (Method A) |

(continued)

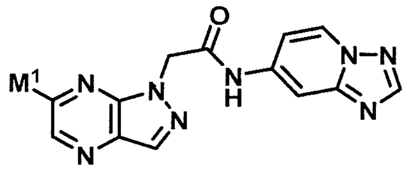

| Example | $M^1$ | Analytical data |
|---|---|---|
| 617 | 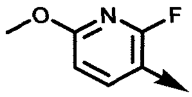 | LC-MS: $[M+H]^+$ / Rt (min) 420.0 / 0.719 (Method A) |
| 618 | 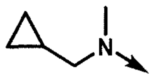 | $^1$H-NMR (400 MHz, DMSO-$d_6$) δ: 11.01 (s, 1H), 8.88-8.86 (m, 1H), 8.37 (s, 1H), 8.29 (s, 1H), 8.12 (d, J = 1.8 Hz, 1H), 8.09 (s, 1H), 7.24 (dd, J = 7.3, 2.4 Hz, 1H), 5.19 (s, 2H), 3.53 (d, J = 6.7 Hz, 2H), 3.19 (s, 3H), 1.12-1.02 (m, 1H), 0.40-0.36 (m, 2H), 0.29-0.25 (m, 2H). |
| 619 | 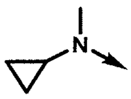 | $^1$H-NMR (400 MHz, DMSO-$d_6$) δ: 11.01 (s, 1H), 8.88-8.86 (m, 1H), 8.37 (s, 1H), 8.29 (s, 1H), 8.12 (d, J = 1.8 Hz, 1H), 8.09 (s, 1H), 7.24 (dd, J = 7.3, 2.4 Hz, 1H), 5.19 (s, 2H), 3.53 (d, J = 6.7 Hz, 2H), 3.19 (s, 3H), 1.12-1.02 (m, 1H), 0.40-0.36 (m, 2H), 0.29-0.25 (m, 2H). |
| 620 | 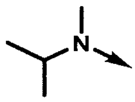 | $^1$H-NMR (400 MHz, $CDCl_3$) δ: 9.10 (s, 1H), 8.46 (d, J = 7.3 Hz, 1H), 8.27 (s, 1H), 8.17 (s, 1H), 8.16 (s, 1H), 8.08 (d, J = 2.4 Hz, 1H), 7.14 (dd, J = 7.3, 2.4 Hz, 1H), 5.16 (s, 2H), 4.98-4.90 (m, 1H), 3.04 (s, 3H), 1.26 (d, J = 6.7 Hz, 6H). |
| 621 | 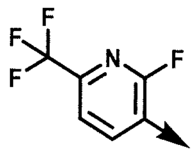 | LC-MS: $[M+H]^+$ / Rt (min) 457.9 / 0.809 (Method A) |
| 622 | 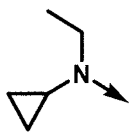 | LC-MS: $[M+H]^+$ / Rt (min) 378.0 / 0.699 (Method A) |
| 623 | 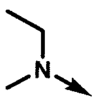 | $^1$H-NMR (400 MHz, DMSO-$d_6$) δ: 10.98 (s, 1H), 8.86 (d, J = 7.3 Hz, 1H), 8.37 (s, 1H), 8.24 (s, 1H), 8.11 (d, J = 1.8 Hz, 1H), 8.08 (s, 1H), 7.23 (dd, J = 7.3, 1.8 Hz, 1H), 5.17 (s, 2H), 3.65 (q, J = 7.1 Hz, 2H), 3.12 (s, 3H), 1.10 (t, J = 7.1 Hz, 3H). |
| 624 | 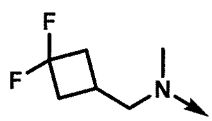 | $^1$H-NMR (400 MHz, DMSO-$d_6$) δ: 10.98 (s, 1H), 8.85 (d, J = 7.3 Hz, 1H), 8.36 (s, 1H), 8.27 (s, 1H), 8.10-8.09 (m, 2H), 7.22 (dd, J = 7.3, 2.4 Hz, 1H), 5.19 (s, 2H), 3.77 (d, J = 6.1 Hz, 2H), 3.17 (s, 3H), 2.61-2.50 (m, 3H), 2.47-2.31 (m, 2H). |
| 625 | 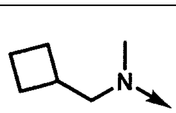 | $^1$H-NMR (400 MHz, DMSO-$d_6$) δ: 10.98 (s, 1H), 8.86 (d, J = 7.3 Hz, 1H), 8.36 (s, 1H), 8.24 (s, 1H), 8.12 (d, J = 2.4 Hz, 1H), 8.07 (s, 1H), 7.23 (dd, J = 7.3, 2.4 Hz, 1H), 5.18 (s, 2H), 3.65 (d, J = 7.3 Hz, 2H), 3.12 (s, 3H), 2.67-2.59 (m, 1H), 1.92-1.82 (m, 2H), 1.75-1.65 (m, 4H). |

(continued)

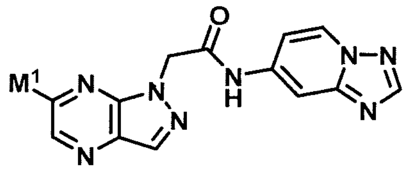

| Example | $M^1$ | Analytical data |
|---|---|---|
| 626 | 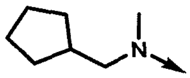 | $^1$H-NMR (400 MHz, DMSO-$d_6$) δ: 10.98 (s, 1H), 8.86 (d, J = 7.3 Hz, 1H), 8.36 (s, 1H), 8.23 (s, 1H), 8.12 (d, J = 2.4 Hz, 1H), 8.07 (s, 1H), 7.22 (dd, J = 7.3, 2.4 Hz, 1H), 5.17 (s, 2H), 3.56 (d, J = 7.3 Hz, 2H), 3.14 (s, 3H), 2.28-2.16 (m, 1H), 1.62-1.50 (m, 2H), 1.50-1.42 (m, 2H), 1.35-1.23 (m, 2H), 1.19-1.09 (m, 2H). |
| 627 | 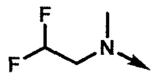 | $^1$H-NMR (400 MHz, DMSO-$d_6$) δ: 10.96 (s, 1H), 8.86 (d, J = 7.3 Hz, 1H), 8.37 (s, 1H), 8.36 (s, 1H), 8.16 (s, 1H), 8.10 (d, J = 2.4 Hz, 1H), 7.22 (dd, J = 7.3, 2.4 Hz, 1H), 6.42-6.12 (m, 1H), 5.22 (s, 2H), 4.12-4.03 (m, 2H), 3.24 (s, 3H). |
| 628 | 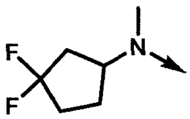 | $^1$H-NMR (400 MHz, $CDCl_3$) δ: 9.08 (s, 1H), 8.47 (d, J = 7.3 Hz, 1H), 8.28 (s, 1H), 8.22 (s, 1H), 8.19 (s, 1H), 8.08 (d, J = 1.8 Hz, 1H), 7.16 (dd, J = 7.3, 1.8 Hz, 1H), 5.46-5.37 (m, 1H), 5.23-5.14 (m, 2H), 3.12 (s, 3H), 2.58-2.45 (m, 1H), 2.39-2.26 (m, 1H), 2.24-2.08 (m, 3H), 2.04-1.90 (m, 1H). |
| 629 | 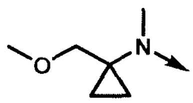 | $^1$H-NMR (400 MHz, $CDCl_3$) δ: 9.04 (s, 1H), 8.61 (s, 1H), 8.46 (d, J = 7.3 Hz, 1H), 8.27 (s, 1H), 8.20 (s, 1H), 8.07 (d, J = 2.4 Hz, 1H), 7.13 (dd, J = 7.3, 2.4 Hz, 1H), 5.17 (s, 2H), 3.52 (s, 2H), 3.30 (s, 3H), 3.24 (s, 3H), 1.25 (s, 2H), 1.04 (s, 2H). |
| 630 | 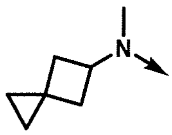 | $^1$H-NMR (400 MHz, DMSO-$d_6$) δ: 10.99 (s, 1H), 8.85 (d, J = 7.3 Hz, 1H), 8.35 (s, 1H), 8.25 (s, 1H), 8.09-8.08 (m, 2H), 7.21 (dd, J = 7.3, 2.4 Hz, 1H), 5.18 (s, 2H), 5.03-4.94 (m, 1H), 3.13 (s, 3H), 2.45-2.40 (m, 2H), 2.22-2.17 (m, 2H), 0.41-0.29 (m, 4H). |
| 631 | 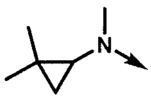 | $^1$H-NMR (400 MHz, $CDCl_3$) δ: 9.10 (s, 1H), 8.46 (d, J = 7.3 Hz, 1H), 8.33 (s, 1H), 8.27 (s, 1H), 8.20 (s, 1H), 8.07 (d, J = 1.8 Hz, 1H), 7.15 (dd, J = 7.3, 1.8 Hz, 1H), 5.19 (s, 2H), 3.19 (s, 3H), 2.63-2.60 (m, 1H), 1.60 (d, J = 7.3 Hz, 2H), 1.00 (s, 3H), 0.95-0.86 (m, 2H), 0.70 (t, J = 4.9 Hz, 1H). |
| 632 | 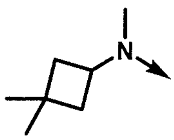 | $^1$H-NMR (400 MHz, $CDCl_3$) δ: 9.08 (s, 1H), 8.46 (d, J = 7.3 Hz, 1H), 8.27 (s, 1H), 8.19 (s, 1H), 8.13 (s, 1H), 8.08 (d, J = 2.4 Hz, 1H), 7.14 (dd, J = 7.3, 2.4 Hz, 1H), 5.17 (s, 2H), 4.75-4.67 (m, 1H), 3.16 (s, 3H), 2.27-2.21 (m, 2H), 2.04-1.97 (m, 2H), 1.27 (s, 3H), 1.17 (s, 3H). |
| 633 | 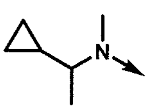 | $^1$H-NMR (400 MHz, $CDCl_3$) δ: 9.16 (s, 1H), 8.49 (d, J = 7.3 Hz, 1H), 8.30 (s, 1H), 8.19 (s, 1H), 8.16 (s, 1H), 8.11 (d, J = 2.1 Hz, 1H), 7.16 (dd, J = 7.3, 2.1 Hz, 1H), 5.16 (s, 2H), 4.11-4.06 (m, 1H), 3.19 (s, 3H), 1.32 (d, J = 6.7 Hz, 3H), 1.11-1.02 (m, 1H), 0.74-0.67 (m, 1H), 0.54-0.41 (m, 2H), 0.29-0.22 (m, 1H). |
| 634 | 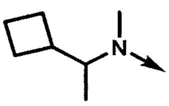 | $^1$H-NMR (400 MHz, $CDCl_3$) δ: 9.18 (s, 1H), 8.49 (d, J = 7.3 Hz, 1H), 8.30 (s, 1H), 8.22 (s, 1H), 8.21 (s, 1H), 8.13 (d, J = 2.4 Hz, 1H), 7.17 (dd, J = 7.3, 2.4 Hz, 1H), 5.21 (s, 2H), 3.00 (s, 3H), 2.63-2.53 (m, 1H), 2.21-2.14 (m, 1H), 1.95-1.83 (m, 4H), 1.35-1.27 (m, 2H), 1.15 (d, J = 6.7 Hz, 3H). |

(continued)

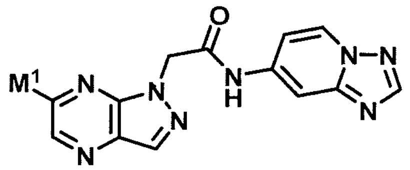

| Example | $M^1$ | Analytical data |
|---|---|---|
| 635 | 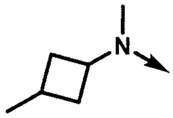 | LC-MS: $[M+H]^+$ / Rt (min) 392.2 / 0.756 (Method A) |
| 636 | 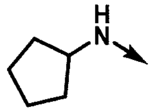 | $^1$H-NMR (400 MHz, $CDCl_3$) δ: 9.10 (s, 1H), 8.46 (d, J = 7.3 Hz, 1H), 8.26 (d, J = 7.9 Hz, 1H), 8.16 (s, 1H), 8.08 (d, J = 2.4 Hz, 1H), 7.87 (s, 1H), 7.14 (dd, J = 7.3, 2.4 Hz, 1H), 5.17 (s, 2H), 5.09 (d, J = 6.7 Hz, 1H), 4.35-4.32 (m, 1H), 2.18-2.12 (m, 2H), 1.81-1.48 (m, 6H). |
| 637 | 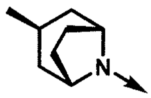 | $^1$H-NMR (400 MHz, $CDCl_3$) δ: 9.16 (s, 1H), 8.46 (d, J = 7.3 Hz, 1H), 8.27 (s, 1H), 8.15 (d, J = 2.3 Hz, 1H), 8.09-8.08 (m, 2H), 7.14 (dd, J = 7.5, 2.1 Hz, 1H), 5.15 (s, 2H), 4.71 (br s, 2H), 2.32-2.26 (m, 1H), 2.13-2.03 (m, 2H), 1.99-1.95 (m, 1H), 1.90-1.86 (m, 1H), 1.73-1.67 (m, 2H), 1.43-1.38 (m, 2H), 1.18 (d, J = 7.3 Hz, 1H), 0.82 (d, J = 6.9 Hz, 2H). |
| 638 | 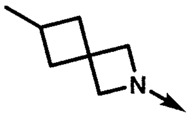 | $^1$H-NMR (400 MHz, DMSO-$d_6$) δ: 10.95 (s, 1H), 8.86 (d, J = 7.9 Hz, 1H), 8.37 (s, 1H), 8.10 (s, 2H), 7.86 (s, 1H), 7.22 (dd, J = 7.6, 2.1 Hz, 1H), 5.17 (s, 2H), 4.14 (s, 2H), 4.05 (s, 2H), 2.34-2.31 (m, 2H), 2.24-2.17 (m, 1H), 1.80-1.75 (m, 2H), 1.01 (d, J = 6.7 Hz, 3H). |
| 639 | 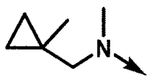 | $^1$H-NMR (400 MHz, DMSO-$d_6$) δ: 11.06 (s, 1H), 8.97 (d, J = 7.3 Hz, 1H), 8.48 (s, 1H), 8.41 (s, 1H), 8.22 (d, J = 1.8 Hz, 1H), 8.19 (s, 1H), 7.34 (dd, J = 7.5, 2.1 Hz, 1H), 5.29 (s, 2H), 3.70 (s, 2H), 3.28 (s, 3H), 1.01 (s, 3H), 0.56 (dd, J = 5.3, 4.3 Hz, 2H), 0.33 (dd, J = 5.7, 4.3 Hz, 2H). |
| 640 | 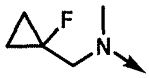 | $^1$H-NMR (400 MHz, DMSO-$d_6$) δ: 10.94 (s, 1H), 8.86 (d, J = 7.3 Hz, 1H), 8.36 (d, J = 7.2 Hz, 2H), 8.11 (s, 1 H), 8.11 (d, J = 3.2 Hz, 1H), 7.22 (dd, J = 7.3, 1.8 Hz, 1H), 5.18 (s, 2H), 4.12 (d, J = 21.4 Hz, 2H), 3.25 (s, 3H), 0.98-0.88 (m, 2H), 0.86-0.78 (m, 2H). |

Examples 641 - 648

According to the methods of Reference examples 32 - 36 or Examples 572 to 576, and common reaction conditions, the compounds of Examples 641 - 648 were obtained by using each corresponding material compound.

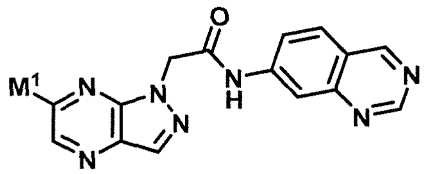

| Example | $M^1$ | Analytical data |
|---|---|---|
| 641 | 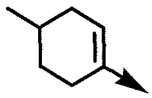 | $^1$H-NMR (400 MHz, DMSO-$d_6$) δ: 11.08 (s, 1H), 9.44 (d, J = 1.2 Hz, 1H), 9.17 (d, J = 2.4 Hz, 1H), 8.96 (d, J = 1.8 Hz, 1H), 8.45 (d, J = 1.8 Hz, 1H), 8.32 (s, 1H), 8.11 (dd, J = 8.5, 1.8 Hz, 1H), 7.80 (dd, J = 6.7, 1.8 Hz, 1H), 7.02-6.94 (m, 1H), 5.45 (d, J = 1.2 Hz, 2H), 2.80-2.61 (m, 1H), 2.46-2.27 (m, 2H), 1.93-1.76 (m, 2H), 1.76-1.62 (m, 1H), 1.35-1.22 (m, 1H), 0.99-0.94 (m, 3H). |
| 642 | 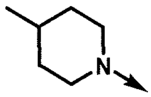 | LC-MS: $[M+H]^+$ / Rt (min) 403.3 / 1.70 (Method B) |
| 643 | 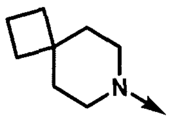 | $^1$H-NMR (400 MHz, $CDCl_3$) δ: 9.26 (d, J = 4.9 Hz, 2H), 9.07 (s, 1H), 8.26-8.18 (m, 3H), 7.87 (d, J = 9.2 Hz, 1H), 7.81-7.78 (m, 1H), 5.19 (s, 2H), 3.71-3.68 (m, 4H), 2.01-1.92 (m, 2H), 1.87-1.83 (m, 4H), 1.72-1.68 (m, 4H). |
| 644 | 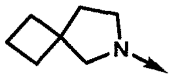 | $^1$H-NMR (400 MHz, DMSO-$d_6$) δ: 11.02 (s, 1H), 9.44 (s, 1H), 9.18 (s, 1H), 8.35 (d, J = 1.8 Hz, 1H), 8.12 (d, J = 8.7 Hz, 1H), 8.08 (s, 1H), 8.04 (s, 1H), 7.82 (dd, J = 8.7, 1.8 Hz, 1H), 5.20 (s, 2H), 3.55-3.50 (m, 4H), 2.00-1.80 (m, 8H). |
| 645 | 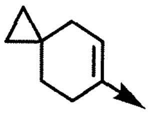 | $^1$H-NMR (400 MHz, DMSO-$d_6$) δ: 11.11 (s, 1H), 9.45 (s, 1H), 9.18 (s, 1H), 8.99 (s, 1H), 8.51-8.47 (m, 1H), 8.34 (d, J = 1.8 Hz, 1H), 8.12 (d, J = 8.5 Hz, 1H), 7.82 (dd, J = 8.5, 1.8 Hz, 1H), 7.06-7.03 (m, 1H), 5.49-5.45 (m, 2H), 2.67-2.63 (m, 2H), 2.19-2.17 (m, 2H), 1.51 (t, J = 6.1 Hz, 2H), 0.37-0.32 (m, 4H). |
| 646 | 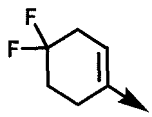 | $^1$H-NMR (400 MHz, DMSO-$d_6$) δ: 11.13 (s, 1H), 9.45 (s, 1H), 9.18 (s, 1H), 9.04 (s, 1H), 8.51 (s, 1H), 8.34 (d, J = 1.8 Hz, 1H), 8.13 (d, J = 9.2 Hz, 1H), 7.82 (dd, J = 9.2, 1.8 Hz, 1H), 6.89 (s, 1H), 5.48 (s, 2H), 2.86-2.81 (m, 4H), 2.25-2.14 (m, 2H). |
| 647 | 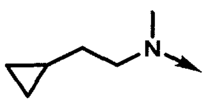 | $^1$H-NMR (400 MHz, DMSO-$d_6$) δ: 11.00 (s, 1H), 9.42 (s, 1H), 9.16 (s, 1H), 8.33 (d, J = 1.8 Hz, 1H), 8.23 (s, 1H), 8.09 (d, J = 8.5 Hz, 1H), 8.06 (s, 1H), 7.79 (dd, J = 8.5, 1.8 Hz, 1H), 5.19 (s, 2H), 3.65 (t, J = 7.1 Hz, 2H), 3.12 (s, 3H), 1.41 (q, J = 7.1 Hz, 2H), 0.62-0.53 (m, 1H), 0.26-0.21 (m, 2H), -0.05--0.09 (m, 2H). |
| 648 | 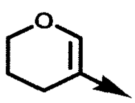 | $^1$H-NMR (400 MHz, DMSO-$d_6$) δ: 11.08 (s, 1H), 9.43 (s, 1H), 9.16 (s, 1H), 8.86 (s, 1H), 8.36 (s, 1H), 8.32 (d, J = 2.4 Hz, 1H), 8.11 (d, J = 8.6 Hz, 1H), 7.92 (s, 1H), 7.80 (s, 1H), 5.40 (s, 2H), 4.06 (t, J = 5.2 Hz, 2H), 2.51-2.49 (m, 2H), 1.92-1.86 (m, 2H). |

Examples 649 - 668

According to the methods of Reference examples 32 - 36 or Examples 572 - 576, and common reaction conditions, the compounds of Examples 649 - 668 were obtained by using each corresponding material compound.

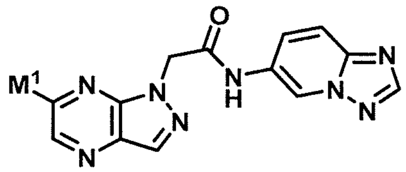

| Example | M1 | Analytical data |
|---|---|---|
| 649 | 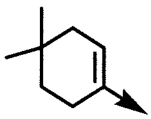 | 1H-NMR (400 MHz, DMSO-$d_6$) δ: 10.91 (s, 1H), 9.35-9.34 (m, 1H), 8.98 (s, 1H), 8.45 (s, 1H), 8.43 (s, 1H), 7.87 (d, J = 9.8 Hz, 1H), 7.64 (dd, J = 9.8, 2.1 Hz, 1H), 7.00-6.98 (m, 1H), 5.41 (s, 2H), 2.60-2.56 (m, 2H), 2.09-2.08 (m, 2H), 1.49 (t, J = 6.4 Hz, 2H), 0.94 (s, 6H). |
| 650 | 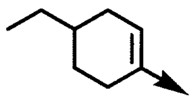 | 1H-NMR (400 MHz, DMSO-$d_6$) δ: 10.84 (s, 1H), 9.27-9.26 (m, 1H), 8.89 (s, 1H), 8.37 (s, 1H), 8.35 (s, 1H), 7.78 (d, J = 9.8 Hz, 1H), 7.56 (dd, J = 9.8, 2.1 Hz, 1H), 6.93 (s, 1H), 5.33 (s, 2H), 2.68 (d, J = 16.5 Hz, 1H), 2.38-2.31 (m, 2H), 1.84-1.80 (m, 2H), 1.40 (s, 1H), 1.28-1.14 (m, 3H), 0.85-0.78 (m, 3H). |
| 651 | 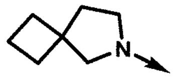 | 1H-NMR (400 MHz, DMSO-$d_6$) δ: 10.74 (s, 1H), 9.29 (d, J = 1.8 Hz, 1H), 8.35 (s, 1H), 8.00 (s, 1H), 7.96 (s, 1H), 7.79 (d, J = 9.2 Hz, 1H), 7.56 (dd, J = 9.8, 1.8 Hz, 1H), 5.08 (s, 2H), 3.48-3.42 (m, 4H), 1.94-1.73 (m, 8H). |
| 652 | 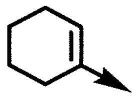 | LC-MS: $[M+H]^+$ / Rt (min) 375.0 / 0.901 (Method A) |
| 653 | 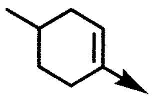 | 1H-NMR (400 MHz, DMSO-$d_6$) δ: 10.84 (s, 1H), 9.27-9.26 (m, 1H), 8.89 (s, 1H), 8.37 (s, 1H), 8.35 (s, 1H), 7.79 (d, J = 9.8 Hz, 1H), 7.55 (dd, J = 9.8, 1.8 Hz, 1H), 6.93-6.91 (m, 1H), 5.33 (s, 2H), 2.70-2.64 (m, 1H), 2.46-2.43 (m, 1H), 2.35-2.27 (m, 1H), 1.85-1.74 (m, 2H), 1.68-1.59 (m, 1H), 1.28-1.17 (m, 1H), 0.91 (d, J = 6.1 Hz, 3H). |
| 654 | 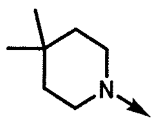 | 1H-NMR (400 MHz, DMSO-$d_6$) δ: 10.86 (s, 1H), 9.38-9.37 (m, 1H), 8.43 (d, J = 1.8 Hz, 2H), 8.09 (s, 1H), 7.87 (d, J = 9.2 Hz, 1H), 7.64 (dd, J = 9.2, 1.8 Hz, 1H), 5.17 (s, 2H), 3.73-3.69 (m, 4H), 1.38-1.35 (m, 4H), 0.95 (s, 6H). |
| 655 | 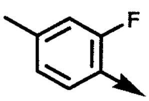 | LC-MS: $[M+H]^+$ / Rt (min) 403.0 / 0.789 (Method A) |
| 656 | 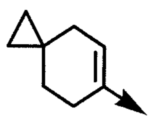 | LC-MS: $[M+H]^+$ / Rt (min) 401.0 / 0.836 (Method A) |
| 657 | 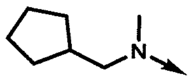 | 1H-NMR (400 MHz, DMSO-$d_6$) δ: 10.84 (s, 1H), 9.39-9.38 (m, 1H), 8.43 (s, 1H), 8.24 (s, 1H), 8.08 (s, 1H), 7.87 (d, J = 9.8 Hz, 1H), 7.64 (dd, J = 9.8, 2.1 Hz, 1H), 5.17 (s, 2H), 3.57 (d, J = 7.3 Hz, 2H), 3.15 (s, 3H), 2.29-2.17 (m, 1H), 1.59-1.52 (m, 2H), 1.50-1.40 (m, 2H), 1.34-1.25 (m, 2H), 1.18-1.09 (m, 2H). |
| 658 | 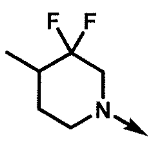 | LC-MS: $[M+H]^+$ / Rt (min) 428.0 / 0.708 (Method A) |

(continued)

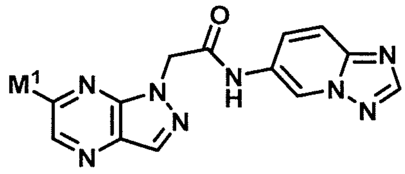

| Example | $M^1$ | Analytical data |
|---|---|---|
| 659 | 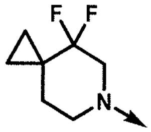 | $^1H$-NMR (400 MHz, $CDCl_3$) δ: 9.46 (s, 1H), 8.78 (s, 1H), 8.31 (s, 1H), 8.30 (s, 1H), 8.20 (s, 1H), 7.66 (d, J = 10.1 Hz, 1H), 7.25-7.23 (m, 1H), 5.17 (s, 2H), 4.05 (t, J = 10.7 Hz, 2H), 3.91 (t, J = 5.5 Hz, 2H), 1.76 (t, J = 5.5 Hz, 2H), 1.00 (t, J = 5.5 Hz, 2H), 0.55-0.03 (m, 2H). |
| 660 | 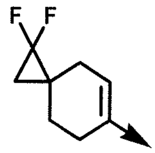 | $^1H$-NMR (400 MHz, DMSO-$d_6$) δ: 10.94 (s, 1H), 9.35 (dd, J = 1.8, 0.9 Hz, 1H), 9.01 (s, 1H), 8.49 (s, 1H), 8.43 (s, 1H), 7.87 (dd, J = 9.6, 0.9 Hz, 1H), 7.64 (dd, J = 9.6, 1.8 Hz, 1H), 7.06-7.04 (m, 1H), 5.43 (s, 2H), 2.72-2.68 |
| 661 | 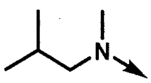 | $^1H$-NMR (400 MHz, $CDCl_3$) δ: 9.49 (d, J = 1.8 Hz, 1H), 8.90 (s, 1H), 8.31 (s, 1H), 8.16 (s, 1H), 8.15 (s, 1H), 7.67 (d, J = 9.8 Hz, 1H), 7.27-7.24 (m, 1H), 5.16 (s, 2H), 3.46 (d, J = 7.3 Hz, 2H), 3.23 (s, 3H), 2.20-2.07 (m, 1H), 0.96 (d, J = 6.1 Hz, 6H). |
| 662 | 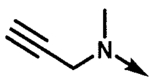 | $^1H$-NMR (400 MHz, $CDCl_3$) δ: 9.50 (d, J = 1.2 Hz, 1H), 8.82 (s, 1H), 8.34 (s, 1H), 8.27 (s, 1H), 8.25 (s, 1H), 7.70 (d, J = 8.5 Hz, 1H), 7.29 (dd, J = 9.8, 1.8 Hz, 1H), 5.23 (s, 2H), 4.50 (d, J = 2.4 Hz, 2H), 3.34 (s, 3H), 2.26 (t, J = 2.4 Hz, 1H) . |
| 663 | 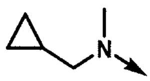 | $^1H$-NMR (400 MHz, DMSO-$d_6$) δ: 10.83 (s, 1H), 9.37 (d, J = 1.8 Hz, 1H), 8.43 (s, 1H), 8.29 (s, 1H), 8.09 (s, 1H), 7.87 (d, J = 9.8 Hz, 1H), 7.64 (dd, J = 9.8, 1.8 Hz, 1H), 5.17 (s, 2H), 3.53 (d, J = 6.7 Hz, 2H), 3.19 (s, 3H), 1.12-1.03 (m, 1H), 0.40-0.35 (m, 2H), 0.31-0.25 (m, 2H). |
| 664 | 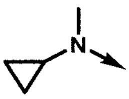 | $^1H$-NMR (400 MHz, DMSO-$d_6$) δ: 10.84 (s, 1H), 9.37-9.36 (m, 1H), 8.56 (s, 1H), 8.43 (s, 1H), 8.14 (s, 1H), 7.87 (d, J = 9.8 Hz, 1H), 7.64 (dd, J = 9.8, 1.8 Hz, 1H), 5.20 (s, 2H), 3.11 (s, 3H), 2.87-2.81 (m, 1H), 1.02-0.97 (m, 2H), 0.76-0.72 (m, 2H). |
| 665 | 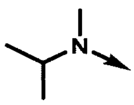 | LC-MS: $[M+H]^+$ / Rt (min) 366.0 / 0.658 (Method A) |
| 666 | 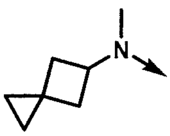 | $^1H$-NMR (400 MHz, DMSO-$d_6$) δ: 10.94 (s, 1H), 9.48 (s, 1H), 8.54 (s, 1H), 8.38 (s, 1H), 8.21 (s, 1H), 7.98 (d, J = 9.8 Hz, 1H), 7.74 (dd, J = 9.5, 2.1 Hz, 1 H), 5.29 (s, 2H), 5.15-5.09 (m, 1H), 3.26 (s, 3H), 2.56-2.53 (m, 2H), 2.35-2.30 (m, 2H), 0.54-0.50 (m, 2H), 0.46-0.42 (m, 2H). |
| 667 | 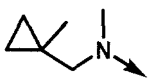 | $^1H$-NMR (400 MHz, DMSO-$d_6$) δ: 10.92 (s, 1H), 9.47 (d, J = 1.4 Hz, 1H), 8.53 (s, 1H), 8.41 (s, 1H), 8.19 (s, 1H), 7.97 (d, J = 9.6 Hz, 1H), 7.74 (dd, J = 9.6, 2.3 Hz, 1H), 5.27 (s, 2H), 3.70 (s, 2H), 3.28 (s, 3H), 1.01 (s, 3H), 0.56 (dd, J = 5.3, 4.3 Hz, 2H), 0.33 (dd, J = 5.7, 4.3 Hz, 2H). |

(continued)

| 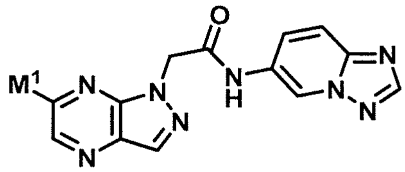 | | |
|---|---|---|
| Example | $M^1$ | Analytical data |
| 668 | 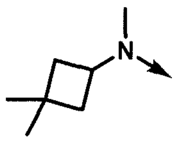 | $^1$H-NMR (400 MHz, DMSO-$d_6$) δ: 10.84 (s, 1H), 9.38 (s, 1H), 8.43 (s, 1H), 8.24 (s, 1H), 8.09 (s, 1H), 7.86 (d, J = 9.2 Hz, 1H), 7.64 (dd, J = 9.8, 1.8 Hz, 1H), 5.17 (s, 2H), 4.77-4.68 (m, 1H), 3.06 (s, 3H), 2.09-2.04 (m, 2H), 1.92 (dd, J = 11.6, 9.2 Hz, 2H), 1.07 (s, 3H), 1.02 (s, 3H). |

Examples 669 - 684

According to the methods of Reference examples 32 - 36 or Examples 572 - 576, and common reaction conditions, the compounds of Examples 669 - 684 were obtained by using each corresponding material compound.

| 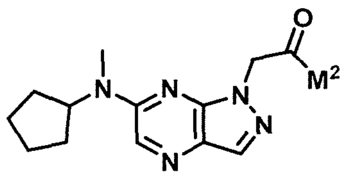 | | |
|---|---|---|
| Example | $M^2$ | Analytical data |
| 669 | 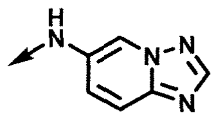 | $^1$H-NMR (400 MHz, DMSO-$d_6$) δ: 10.86 (s, 1H), 9.40-9.37 (m, 1H), 8.44 (s, 1H), 8.31 (s, 1H), 8.08 (s, 1H), 7.87 (d, J = 10.4 Hz, 1H), 7.65 (dd, J = 9.5, 2.1 Hz, 1H), 5.17 (s, 2H), 4.98-4.90 (m, 1H), 3.00 (s, 3H), 1.82-1.78 (m, 2H), 1.71-1.48 (m, 6H). |
| 670 | 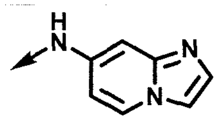 | LC-MS: $[M+H]^+$ / Rt (min) 391.2 / 0.546 (Method A) |
| 671 | 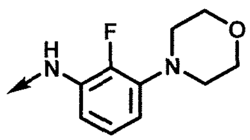 | $^1$H-NMR (400 MHz, $CDCl_3$) δ: 8.63 (s, 1H), 8.20-8.18 (m, 2H), 7.98-7.94 (m, 1H), 7.07-7.02 (m, 1H), 6.72-6.68 (m, 1H), 5.19 (s, 2H), 5.05-4.96 (m, 1H), 3.88-3.84 (m, 4H), 3.08 (s, 3H), 3.07-3.03 (m, 4H), 2.02-1.98 (m, 2H), 1.86-1.61 (m, 6H). |
| 672 | 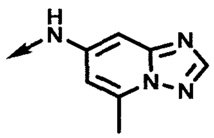 | $^1$H-NMR (400 MHz, $CDCl_3$) δ: 8.96 (s, 1H), 8.28 (s, 1H), 8.19 (d, J = 4.3 Hz, 2H), 7.97-7.93 (m, 1H), 6.98-6.94 (m, 1H), 5.16 (s, 2H), 5.02-4.97 (m, 1H), 3.09 (s, 3H), 2.75 (s, 3H), 2.01-1.92 (m, 2H), 1.82-1.63 (m, 6H). |
| 673 | 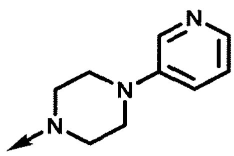 | LC-MS: $[M+H]^+$ / Rt (min) 421.1 / 0.607 (Method A) |

(continued)

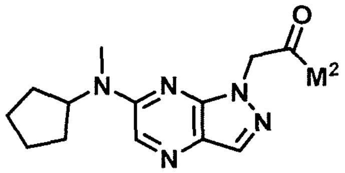

| Example | $M^2$ | Analytical data |
|---|---|---|
| 674 | 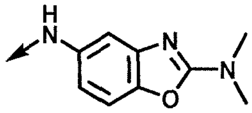 | LC-MS: $[M+H]^+$ / Rt (min) 435.3 / 0.860 (Method A) |
| 675 | 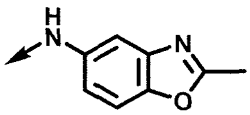 | $^1$H-NMR (400 MHz, $CDCl_3$) $\delta$: 8.51 (s, 1H), 8.17 (d, J = 8.6 Hz, 2H), 7.84 (s, 1H), 7.38-7.30 (m, 2H), 5.16 (s, 2H), 5.03-4.97 (m, 1H), 3.07 (s, 3H), 2.61 (s, 3H), 2.03-1.94 (m, 2H), 1.85-1.63 (m, 6H). |
| 676 | 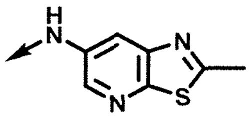 | LC-MS: $[M+H]^+$ / Rt (min) 423.0 / 0.838 (Method A) |
| 677 | 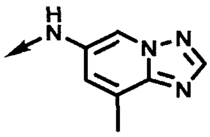 | $^1$H-NMR (400 MHz, $CDCl_3$) $\delta$: 9.33 (s, 1H), 8.76 (s, 1H), 8.28 (s, 1H), 8.19 (d, J = 5.5 Hz, 2H), 7.01 (d, J = 6.7 Hz, 1H), 5.17 (s, 2H), 5.02-4.98 (m, 1H), 3.08 (s, 3H), 2.61 (s, 3H), 2.00-1.92 (m, 2H), 1.85-1.64 (m, 6H). |
| 678 | 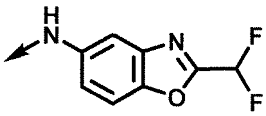 | LC-MS: $[M+H]^+$ / Rt (min) 441.9 / 0.948 (Method A) |
| 679 | 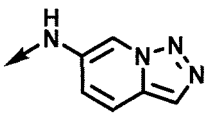 | LC-MS: $[M+H]^+$ / Rt (min) 392.0 / 0.797 (Method A) |
| 680 | 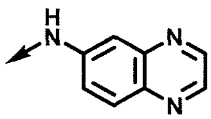 | LC-MS: $[M+H]^+$ / Rt (min) 403.0 / 0.835 (Method A) |
| 681 | 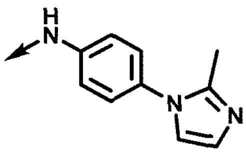 | LC-MS: $[M+H]^+$ / Rt (min) 431.0 / 0.657 (Method A) |
| 682 | 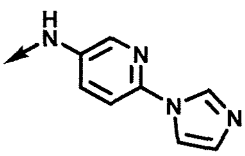 | LC-MS: $[M+H]^+$ / Rt (min) 418.0 / 0.645 (Method A) |

(continued)

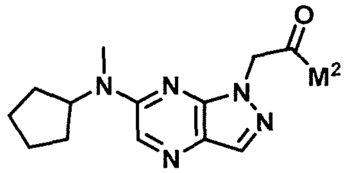

| Example | $M^2$ | Analytical data |
|---|---|---|
| 683 | 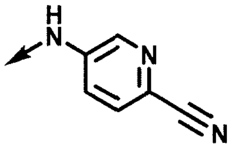 | $^1$H-NMR (400 MHz, $CDCl_3$) δ: 9.17 (s, 1H), 8.57 (d, J = 3.2 Hz, 1H), 8.32 (dd, J = 8.5, 2.5 Hz, 1H), 8.18 (s, 1H), 8.16 (s, 1H), 7.64 (d, J = 8.7 Hz, 1H), 5.14 (s, 2H), 5.00-4.92 (m, 1H), 3.06 (s, 3H), 1.98-1.91 (m, 2H), 1.81-1.76 (m, 2H), 1.73-1.62 (m, 4H). |
| 684 | 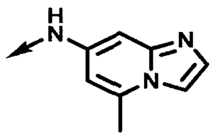 | $^1$H-NMR (400 MHz, $CDCl_3$) δ: 8.99-8.90 (m, 1H), 8.14 (d, J = 9.1 Hz, 2H), 7.69 (s, 1H), 7.60 (s, 1H), 7.36 (s, 1H), 6.91 (s, 1H), 5.14 (s, 2H), 4.99-4.93 (m, 1H), 3.04 (s, 3H), 2.52 (s, 3H), 1.99-1.90 (m, 2H), 1.78-1.66 (m, 6H). |

Examples 685 - 698

According to the methods of Reference examples 32 - 36 or Examples 572 - 576, and common reaction conditions, the compounds of Examples 685 - 698 were obtained by using each corresponding material compound.

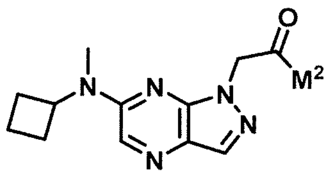

| Example | $M^2$ | Analytical data |
|---|---|---|
| 685 | 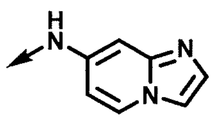 | $^1$H-NMR (400 MHz, DMSO-$d_6$) δ: 10.61 (s, 1H), 8.44 (d, J = 7.3 Hz, 1H), 8.23 (s, 1H), 8.07 (s, 1H), 7.88 (d, J = 1.8 Hz, 1H), 7.81 (s, 1H), 7.43 (d, J = 1.2 Hz, 1H), 6.96 (dd, J = 7.3, 2.4 Hz, 1H), 5.13 (s, 2H), 4.81-4.73 (m, 1H), 3.08 (s, 3H), 2.20-2.13 (m, 4H), 1.67-1.52 (m, 2H). |
| 686 | 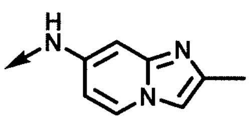 | $^1$H-NMR (400 MHz, $CDCl_3$) δ: 8.66 (s, 1H), 8.16 (s, 1H), 8.14 (s, 1H), 7.91 (d, J = 7.3 Hz, 1H), 7.65 (d, J = 2.4 Hz, 1H), 7.24 (s, 1H), 6.96 (dd, J = 7.3, 2.4 Hz, 1H), 5.15 (s, 2H), 4.81-4.72 (m, 1H), 3.17 (s, 3H), 2.41 (s, 3H), 2.38-2.30 (m, 2H), 2.28-2.17 (m, 2H), 1.83-1.72 (m, 2H). |
| 687 | 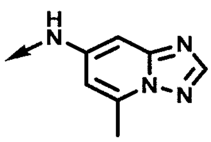 | $^1$H-NMR (400 MHz, $CDCl_3$) δ: 9.03 (s, 1H), 8.27 (s, 1H), 8.17 (d, J = 1.2 Hz, 1H), 8.13 (d, J = 1.8 Hz, 1H), 7.92 (d, J = 1.2 Hz, 1H), 6.99 (s, 1H), 5.16 (s, 2H), 4.80-4.71 (m, 1H), 3.16 (s, 3H), 2.74 (s, 3H), 2.37-2.30 (m, 2H), 2.27-2.17 (m, 2H), 1.82-1.71 (m, 2H). |

(continued)

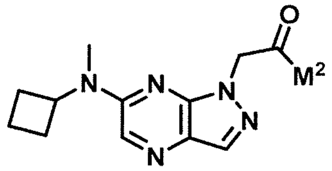

| Example | $M^2$ | Analytical data |
|---|---|---|
| 688 | 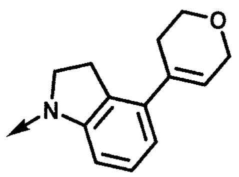 | $^1$H-NMR (400 MHz, DMSO-$d_6$) δ: 8.22 (s, 1H), 8.06 (s, 1H), 7.86 (d, J = 7.9 Hz, 1H), 7.12 (t, J = 7.9 Hz, 1H), 6.94 (d, J = 7.3 Hz, 1H), 5.90-5.88 (m, 1H), 5.30 (s, 2H), 4.81-4.73 (m, 1H), 4.27 (t, J = 7.9 Hz, 2H), 4.21-4.18 (m, 2H), 3.79 (t, J = 5.5 Hz, 2H), 3.27-3.22 (m, 2H), 3.07 (s, 3H), 2.38-2.34 (m, 2H), 2.20-2.12 (m, 4H), 1.69-1.53 (m, 2H). |
| 689 | 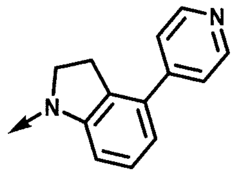 | $^1$H-NMR (400 MHz, DMSO-$d_6$) δ: 8.68-8.66 (m, 2H), 8.25 (s, 1H), 8.10 (s, 1H), 8.06 (d, J = 7.9 Hz, 1H), 7.55-7.54 (m, 2H), 7.32 (t, J = 7.9 Hz, 1H), 7.16 (d, J = 6.7 Hz, 1H), 5.36 (s, 2H), 4.84-4.76 (m, 1H), 4.33 (t, J = 8.5 Hz, 2H), 3.36-3.32 (m, 2H), 3.10 (s, 3H), 2.24-2.15 (m, 4H), 1.72-1.59 (m, 2H). |
| 690 | 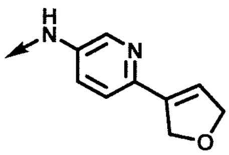 | LC-MS: $[M+H]^+$ / Rt (min) 406.0 / 0.878 (Method A) |
| 691 | 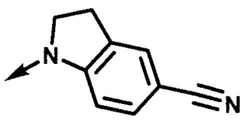 | $^1$H-NMR (400 MHz, DMSO-$d_6$) δ: 8.22 (s, 1H), 8.07 (s, 1H), 8.00 (d, J = 8.5 Hz, 1H), 7.71-7.70 (m, 1H), 7.63-7.60 (m, 1H), 5.35 (s, 2H), 4.81-4.72 (m, 1H), 4.35 (t, J = 8.5 Hz, 2H), 3.25 (t, J = 8.5 Hz, 2H), 3.07 (s, 3H), 2.21-2.12 (m, 4H), 1.69-1.54 (m, 2H). |
| 692 | 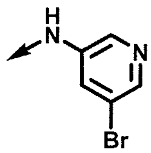 | LC-MS: $[M+H]^+$ / Rt (min) 415.9 / 1.000 (Method A) |
| 693 | 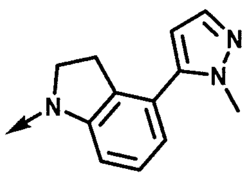 | $^1$H-NMR (400 MHz, $CDCl_3$) δ: 8.29 (d, J = 7.9 Hz, 1H), 8.12-8.11 (m, 2H), 7.58 (d, J = 1.8 Hz, 1H), 7.36-7.31 (m, 1H), 7.04-7.02 (m, 1H), 6.28 (d, J = 1.8 Hz, 1H), 5.27 (s, 2H), 4.77-4.69 (m, 1H), 4.24 (t, J = 8.5 Hz, 2H), 3.81 (s, 3H), 3.20 (t, J = 8.5 Hz, 2H), 3.16 (s, 3H), 2.38-2.31 (m, 2H), 2.31-2.18 (m, 2H), 1.85-1.72 (m, 2H). |
| 694 | 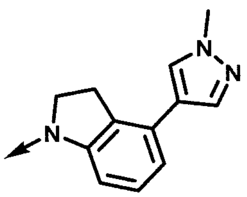 | $^1$H-NMR (400 MHz, $CDCl_3$) δ: 8.15-8.10 (m, 3H), 7.73 (s, 1H), 7.58 (s, 1H), 7.24-7.17 (m, 2H), 5.27 (s, 2H), 4.77-4.68 (m, 1H), 4.27 (t, J = 8.2 Hz, 2H), 4.00 (s, 3H), 3.39 (t, J = 8.5 Hz, 2H), 3.15 (s, 3H), 2.37-2.30 (m, 2H), 2.29-2.18 (m, 2H), 1.83-1.69 (m, 2H). |

(continued)

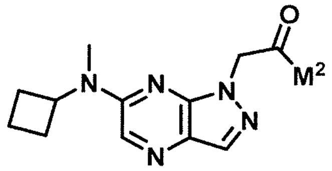

| Example | $M^2$ | Analytical data |
|---|---|---|
| 695 | 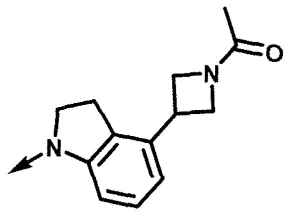 | $^1$H-NMR (400 MHz, $CDCl_3$) δ: 8.12 (d, J = 7.9 Hz, 1H), 8.07 (d, J = 4.3 Hz, 2H), 7.23 (d, J = 7.9 Hz, 1H), 7.08 (d, J = 7.9 Hz, 1H), 5.22 (s, 2H), 4.73-4.64 (m, 1H), 4.50 (t, J = 8.5 Hz, 1H), 4.39 (t, J = 9.5 Hz, 1H), 4.23-4.18 (m, 3H), 4.13-4.07 (m, 1H), 3.88-3.80 (m, 1H), 3.17 (t, J = 8.5 Hz, 2H), 3.11 (s, 3H), 2.34-2.25 (m, 2H), 2.23-2.15 (m, 2H), 1.92 (s, 3H), 1.80-1.70 (m, 2H). |
| 696 | 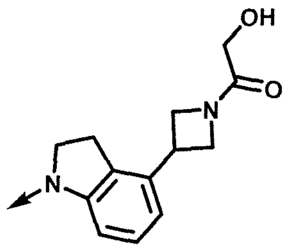 | LC-MS: $[M+H]^+$ / Rt (min) 476.3 / 0.738 (Method A) |
| 697 | 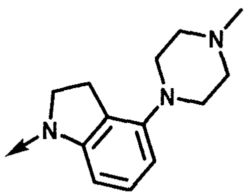 | $^1$H-NMR (400 MHz, $CDCl_3$) δ: 8.10 (s, 1H), 8.09 (s, 1H), 7.91 (d, J = 7.9 Hz, 1H), 7.16 (t, J = 7.9 Hz, 1H), 6.73 (d, J = 7.9 Hz, 1H), 5.24 (s, 2H), 4.76-4.67 (m, 1H), 4.20 (t, J = 7.9 Hz, 2H), 3.21 (t, J = 7.9 Hz, 2H), 3.14 (s, 3H), 3.06-3.03 (m, 4H), 2.60 (s, 4H), 2.39 (s, 3H), 2.36-2.27 (m, 2H), 2.25-2.17 (m, 2H), 1.83-1.71 (m, 2H). |
| 698 | 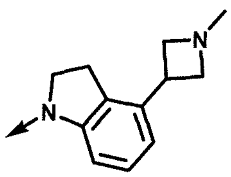 | $^1$H-NMR (400 MHz, $CDCl_3$) δ: 8.10-8.07 (m, 3H), 7.21 (t, J = 7.9 Hz, 1H), 7.04 (d, J = 7.9 Hz, 1H), 5.24 (s, 2H), 4.76-4.67 (m, 1H), 4.21 (t, J = 8.5 Hz, 2H), 3.84-3.70 (m, 3H), 3.18-3.12 (m, 7H), 2.39 (s, 3H), 2.36-2.27 (m, 2H), 2.25-2.17 (m, 2H), 1.83-1.76 (m, 2H). |

Examples 699 - 734

According to the methods of Reference examples 32 - 36 or Examples 572 - 576, and common reaction conditions, the compounds of Examples 699 - 734 were obtained by using each corresponding material compound.

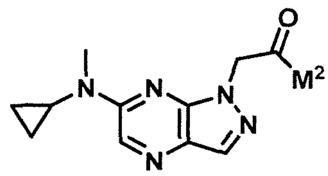

| Example | $M^2$ | Analytical data |
|---|---|---|
| 699 | 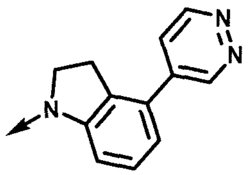 | $^1$H-NMR (400 MHz, DMSO-$d_6$) δ: 9.45-9.44 (m, 1H), 9.31 (dd, J = 5.5, 1.2 Hz, 1H), 8.55 (s, 1H), 8.13 (s, 1H), 8.09 (d, J = 7.9 Hz, 1H), 7.87 (dd, J = 5.5, 2.4 Hz, 1H), 7.35 (t, J = 7.9 Hz, 1H), 7.27-7.25 (m, 1H), 5.38 (s, 2H), 4.34 (t, J = 8.2 Hz, 2H), 3.38 (t, J = 7.9 Hz, 2H), 3.10 (s, 3H), 2.85-2.80 (m, 1H), 1.01-0.94 (m, 2H), 0.75-0.71 (m, 2H). |
| 700 | 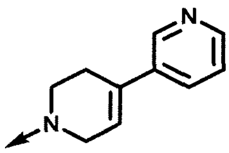 | LC-MS: $[M+H]^+$ / Rt (min) 390.0 / 0.551 (Method A) |
| 701 | 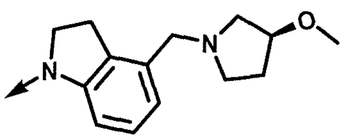 | $^1$H-NMR (400 MHz, $CDCl_3$) δ: 8.56 (s, 1H), 8.10 (s, 1H), 8.07 (d, J = 7.9 Hz, 1H), 7.12 (t, J = 7.9 Hz, 1H), 7.04-6.95 (m, 1H), 5.23 (s, 2H), 4.19 (t, J = 8.5 Hz, 2H), 3.94-3.89 (m, 1H), 3.59-3.52 (m, 2H), 3.32-3.26 (m, 5H), 3.16 (s, 3H), 2.80-2.62 (m, 3H), 2.54-2.43 (m, 2H), 2.11-2.02 (m, 1H), 1.82-1.74 (m, 1H), 1.03-0.98 (m, 2H), 0.79-0.71 (m, 2H). |
| 702 | 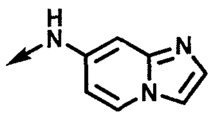 | $^1$H-NMR (400 MHz, DMSO-$d_6$) δ: 10.62 (s, 1H), 8.53 (s, 1H), 8.44 (d, J = 7.3 Hz, 1H), 8.11 (s, 1H), 7.87-7.86 (m, 1H), 7.80 (d, J = 1.2 Hz, 1H), 7.43 (d, J = 1.2 Hz, 1H), 6.95 (dd, J = 7.3, 2.4 Hz, 1H), 5.14 (s, 2H), 3.09 (s, 3H), 2.84-2.79 (m, 1H), 0.99-0.94 (m, 2H), 0.73-0.70 (m, 2H). |
| 703 | 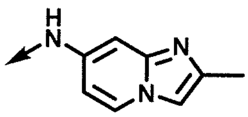 | $^1$H-NMR (400 MHz, $CDCl_3$) δ: 8.68 (s, 1H), 8.61 (s, 1H), 8.19 (s, 1H), 7.90 (d, J = 7.3 Hz, 1H), 7.63 (d, J = 2.4 Hz, 1H), 7.23 (s, 1H), 6.97 (dd, J = 7.3, 2.4 Hz, 1H), 5.15 (s, 2H), 3.22 (s, 3H), 2.82-2.77 (m, 1H), 2.40 (s, 3H), 1.08-1.03 (m, 2H), 0.83-0.79 (m, 2H). |
| 704 | 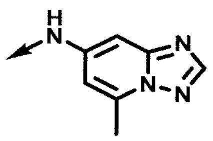 | $^1$H-NMR (400 MHz, $CDCl_3$) δ: 8.93 (s, 1H), 8.62 (s, 1H), 8.28 (s, 1H), 8.21 (s, 1H), 7.92 (d, J = 2.4 Hz, 1H), 6.98 (d, J = 1.2 Hz, 1H), 5.17 (s, 2H), 3.22 (s, 3H), 2.83-2.78 (m, 1H), 2.75 (s, 3H), 1.08-1.03 (m, 2H), 0.83-0.79 (m, 2H). |
| 705 | 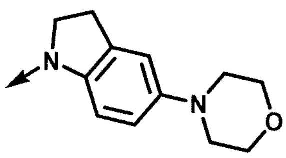 | $^1$H-NMR (400 MHz, DMSO-$d_6$) δ: 8.52 (s, 1H), 8.09 (s, 1H), 7.75 (d, J = 9.2 Hz, 1H), 6.88 (d, J = 2.4 Hz, 1H), 6.68 (dd, J = 9.2, 2.4 Hz, 1H), 5.26 (s, 2H), 4.24 (t, J = 8.5 Hz, 2H), 3.70-3.67 (m, 4H), 3.15 (t, J = 8.5 Hz, 2H), 3.08 (s, 3H), 3.02-2.99 (m, 4H), 2.83-2.78 (m, 1H), 0.99-0.94 (m, 2H), 0.73-0.69 (m, 2H). |
| 706 | 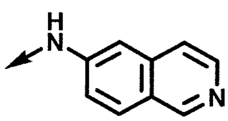 | $^1$H-NMR (400 MHz, $CDCl_3$) δ: 9.13 (s, 1H), 8.72 (s, 1H), 8.62 (s, 1H), 8.47 (d, J = 6.1 Hz, 1H), 8.22-8.17 (m, 2H), 7.87 (d, J = 8.6 Hz, 1H), 7.57 (d, J = 5.5 Hz, 1H), 7.48 (dd, J = 8.9, 2.1 Hz, 1H), 5.19 (s, 2H), 3.24 (s, 3H), 2.83-2.78 (m, 1H), 1.06 (t, J = 5.8 Hz, 2H), 0.85-0.79 (m, 2H). |

(continued)

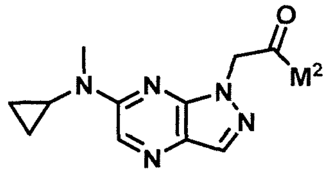

| Example | $M^2$ | Analytical data |
|---|---|---|
| 707 | 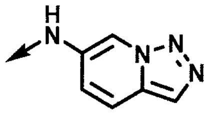 | LC-MS: $[M+H]^+$ / Rt (min) 364.0 / 0.787 (Method A) |
| 708 | 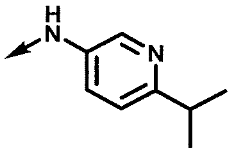 | LC-MS: $[M+H]^+$ / Rt (min) 366.0 / 0.796 (Method A) |
| 709 | 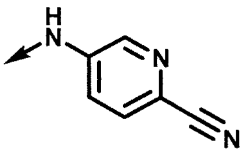 | LC-MS: $[M+H]^+$ / Rt (min) 348.9 / 0.994 (Method A) |
| 710 | 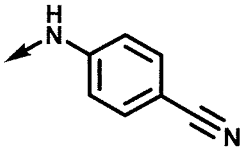 | LC-MS: $[M+H]^+$ / Rt (min) 347.9 / 1.076 (Method A) |
| 711 | 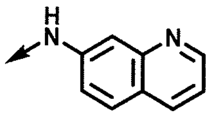 | $^1$H-NMR (400 MHz, $CDCl_3$) δ: 8.86 (dd, J = 4.3, 1.6 Hz, 1H), 8.76 (s, 1H), 8.61 (s, 1H), 8.21 (s, 1H), 8.14-8.12 (m, 1H), 8.07 (dd, J = 8.2, 0.9 Hz, 1H), 7.80-7.72 (m, 2H), 7.31 (dd, J = 8.2, 4.1 Hz, 1H), 5.2 (s, 2H), 3.22 (s, 3H), 2.82-2.77 (m, 1H), 1.07-1.00 (m, 2H), 0.83-0.78 (m, 2H). |
| 712 | 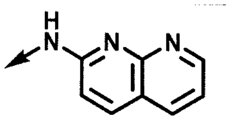 | LC-MS: $[M+H]^+$ / Rt (min) 375.2 / 0.647 (Method A) |
| 713 | 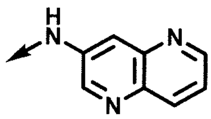 | $^1$H-NMR (400 MHz, $CDCl_3$) δ: 9.03 (s, 1H), 8.95 (d, J = 4.1 Hz, 1H), 8.91 (d, J = 1.4 Hz, 1H), 8.77 (s, 1H), 8.63 (s, 1H), 8.33 (d, J = 8.7 Hz, 1H), 8.24 (s, 1H), 7.55 (dd, J = 8.2, 4.1 Hz, 1H), 5.24 (s, 2H), 3.23 (s, 3H), 2.85-2.77 (m, 1H), 1.06 (q, J = 6.4 Hz, 2H), 0.82 (d, J = 7.3 Hz, 2H). |
| 714 | 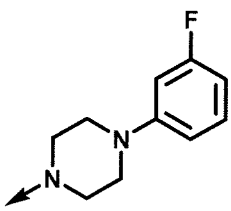 | $^1$H-NMR (400 MHz, $CDCl_3$) δ: 8.56 (s, 1H), 8.08 (s, 1H), 7.24-7.18 (m, 1H), 6.68-6.65 (m, 1H), 6.61-6.56 (m, 2H), 5.21 (s, 2H), 3.79-3.72 (m, 4H), 3.21-3.18 (m, 7H), 2.78-2.73 (m, 1H), 1.04-0.99 (m, 2H), 0.80-0.76 (m, 2H). |

(continued)

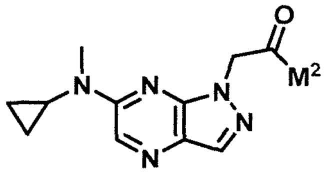

| Example | $M^2$ | Analytical data |
|---|---|---|
| 715 | 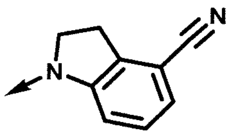 | $^1$H-NMR (400 MHz, $CDCl_3$) δ: 8.60 (s, 1H), 8.40 (d, J = 7.3 Hz, 1H), 8.14 (s, 1H), 7.33-7.30 (m, 2H), 5.27 (s, 2H), 4.33 (t, J = 8.5 Hz, 2H), 3.50 (t, J = 8.5 Hz, 2H), 3.20 (s, 3H), 2.81-2.76 (m, 1H), 1.07-1.02 (m, 2H), 0.83-0.79 (m, 2H). |
| 716 | 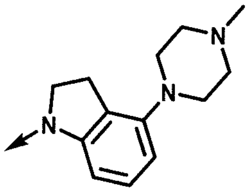 | $^1$H-NMR (400 MHz, $CDCl_3$) δ: 8.60 (s, 1H), 8.40 (d, J = 7.3 Hz, 1H), 8.14 (s, 1H), 7.33-7.30 (m, 2H), 5.27 (s, 2H), 4.33 (t, J = 8.5 Hz, 2H), 3.50 (t, J = 8.5 Hz, 2H), 3.20 (s, 3H), 2.81-2.76 (m, 1H), 1.07-1.02 (m, 2H), 0.83-0.79 (m, 2H). |
| 717 | 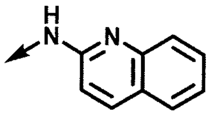 | LC-MS: $[M+H]^+$ / Rt (min) 374.2 / 0.900 (Method A) |
| 718 | 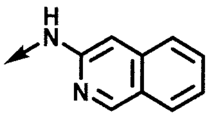 | LC-MS: $[M+H]^+$ / Rt (min) 374.2 / 0.917 (Method A) |
| 719 | 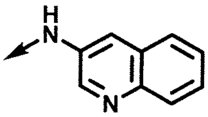 | $^1$H-NMR (400 MHz, $CDCl_3$) δ: 8.81 (s, 1H), 8.71 (d, J = 2.4 Hz, 1H), 8.67-8.63 (m, 2H), 8.24 (s, 1H), 8.03 (d, J = 8.6 Hz, 1H), 7.80 (d, J = 7.9 Hz, 1H), 7.65-7.60 (m, 1H), 7.55-7.51 (m, 1H), 5.22 (s, 2H), 3.24 (s, 3H), 2.85-2.77 (m, 1H), 1.08-1.03 (m, 2H), 0.84-0.78 (m, 2H). |
| 720 | 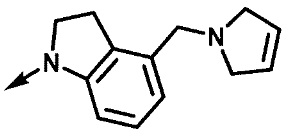 | LC-MS: $[M+H]^+$ / Rt (min) 430/3 / 0.599 (Method A) |
| 721 | 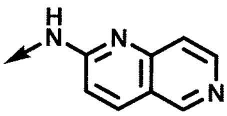 | LC-MS: $[M+H]^+$ / Rt (min) 375.2 / 0.584 (Method A) |
| 722 | 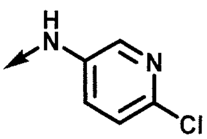 | $^1$H-NMR (400 MHz, $CDCl_3$) δ: 8.71 (s, 1H), 8.62 (s, 1H), 8.32 (d, J = 3.1 Hz, 1H), 8.20 (s, 1H), 8.08 (dd, J = 8.5, 3.1 Hz, 1H), 7.28-7.26 (m, 1H), 5.15 (s, 2H), 3.22 (s, 3H), 2.83-2.78 (m, 1H), 1.07-1.05 (m, 2H), 0.83-0.79 (m, 2H). |

(continued)

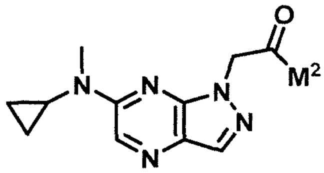

| Example | $M^2$ | Analytical data |
|---|---|---|
| 723 | 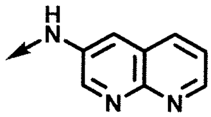 | $^1$H-NMR (400 MHz, $CDCl_3$) δ: 9.33 (s, 1H), 9.05-9.01 (m, 1H), 8.88 (d, J = 3.2 Hz, 1H), 8.86-8.82 (m, 1H), 8.61 (s, 1H), 8.22 (s, 1H), 8.17 (d, J = 7.3 Hz, 1H), 7.48 (dd, J = 8.2, 4.1 Hz, 1H), 5.25 (s, 2H), 3.21 (s, 3H), 2.82-2.78 (m, 1H), 1.04-1.01 (m, 2H), 0.90-0.84 (m, 2H). |
| 724 | 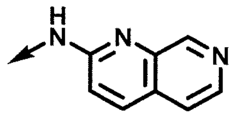 | LC-MS: $[M+H]^+$ / Rt (min) 375.2 / 0.685 (Method A) |
| 725 | 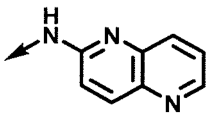 | LC-MS: $[M+H]^+$ / Rt (min) 375.2 / 0.734 (Method A) |
| 726 | 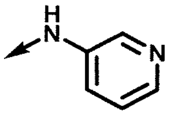 | $^1$H-NMR (400 MHz, $CDCl_3$) δ: 8.64 (s, 1H), 8.60 (s, 1H), 8.50 (d, J = 2.7 Hz, 1H), 8.33 (dd, J = 4.6, 1.4 Hz, 1H), 8.18 (s, 1H), 8.10-8.08 (m, 1H), 7.23 (dd, J = 4.3, 2.2 Hz, 1H), 5.16 (s, 2H), 3.21 (s, 3H), 2.82-2.77 (m, 1H), 1.06-1.04 (m, 2H), 0.81-0.79 (m, 2H). |
| 727 | 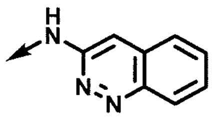 | LC-MS: $[M+H]^+$ / Rt (min) 375.2 / 0.816 (Method A) |
| 728 | 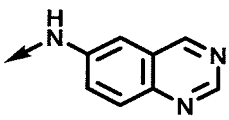 | $^1$H-NMR (400 MHz, $CDCl_3$) δ: 9.35 (s, 1H), 9.25 (s, 1H), 8.94 (s, 1H), 8.62 (s, 1H), 8.45 (d, J = 2.7 Hz, 1H), 8.22 (d, J = 4.6 Hz, 1H), 7.97 (d, J = 8.7 Hz, 1H), 7.70 (dd, J = 9.1, 2.7 Hz, 1H), 5.22 (s, 2H), 3.22 (s, 3H), 2.83-2.78 (m, 1H), 1.10-1.04 (m, 2H), 0.83-0.75 (m, 2H). |
| 729 | 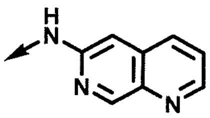 | LC-MS: $[M+H]^+$ / Rt (min) 375.2 / 0.752 (Method A) |
| 730 | 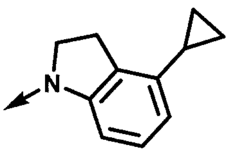 | $^1$H-NMR (400 MHz, $CDCl_3$) δ: 8.55 (s, 1H), 8.10 (s, 1H), 7.98 (d, J = 7.9 Hz, 1H), 7.07 (t, J = 7.9 Hz, 1H), 6.58 (d, J = 7.9 Hz, 1H), 5.23 (s, 2H), 4.30-4.15 (m, 2H), 3.33 (t, J = 8.2 Hz, 2H), 3.15 (s, 3H), 2.76-2.71 (m, 1H), 1.85-1.70 (m, 1H), 1.03-0.97 (m, 2H), 0.96-0.91 (m, 2H), 0.79-0.75 (m, 2H), 0.71-0.66 (m, 2H). |
| 731 | 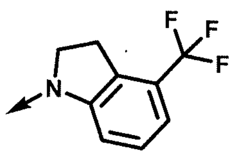 | LC-MS: $[M+H]^+$ / Rt (min) 417.2 / 1.054 (Method A) |

(continued)

| 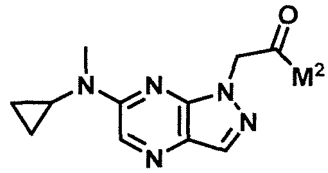 | | |
|---|---|---|
| Example | $M^2$ | Analytical data |
| 732 | 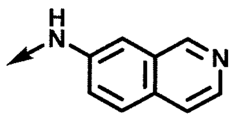 | $^1$H-NMR (400 MHz, $CDCl_3$) δ: 9.16 (s, 1H), 8.86 (s, 1H), 8.60 (s, 1H), 8.44-8.42 (m, 1H), 8.33 (s, 1H), 8.21 (s, 1H), 7.72 (d, J = 8.6 Hz, 1H), 7.61-7.53 (m, 2H), 5.20 (s, 2H), 3.21 (s, 3H), 2.83-2.75 (m, 1H), 1.06-1.00 (m, 2H), 0.85-0.75 (m, 2H). |
| 733 | 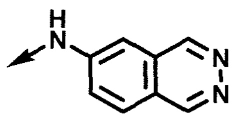 | $^1$H-NMR (400 MHz, $CDCl_3$) δ: 9.49 (s, 1H), 9.41 (s, 1H), 9.18 (s, 1H), 8.63 (s, 1H), 8.43-8.41 (d, 1H), 8.23 (s, 1H), 7.89 (d, J = 8.6 Hz, 1H), 7.77 (dd, J = 8.6, 1.8 Hz, 1H), 5.22 (s, 2H), 3.22 (s, 3H), 2.84-2.77 (m, 1H), 1.08-1.02 (m, 2H), 0.83-0.76 (m, 2H). |
| 734 | 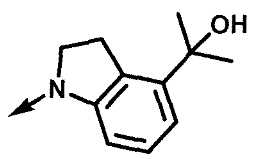 | $^1$H-NMR (400 MHz, $CDCl_3$) δ: 8.51 (s, 1H), 8.12 (d, J = 7.9 Hz, 1H), 8.07 (s, 1H), 7.13-7.02 (m, 2H), 5.11 (s, 2H), 4.10-4.00 (m, 2H), 3.55-3.45 (m, 2H), 3.13 (s, 3H), 2.75-2.69 (m, 1H), 2.01 (d, J = 10.4 Hz, 1H), 1.58 (s, 6H), 1.00-0.93 (m, 2H), 0.77-0.73 (m, 2H). |

Examples 735 - 761

According to the methods of Reference examples 32 - 36 or Examples 572 - 576, and common reaction conditions, the compounds of Examples 735 - 761 were obtained by using each corresponding material compound.

| Example | Chemical structure | Analytical data |
|---|---|---|
| 735 | 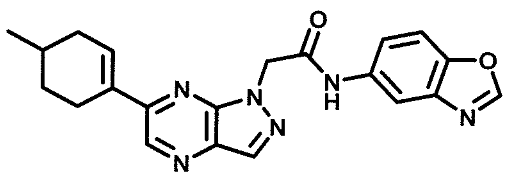 | $^1$H-NMR (400 MHz, $CDCl_3$) δ: 10.64 (s, 1H), 8.96 (d, J = 1.8 Hz, 1H), 8.70 (d, J = 2.4 Hz, 1H), 8.44 (d, J = 2.4 Hz, 1H), 8.08 (s, 1H), 7.73 (dd, J = 8.8, 2.1 Hz, 1H), 7.55-7.49 (m, 1H), 7.03-6.95 (m, 1H), 5.36 (d, J = 1.2 Hz, 2H), 2.82-2.62 (m, 1H), 2.47-2.28 (m, 2H), 1.96-1.79 (m, 2H), 1.79-1.65 (m, 1H), 1.38-1.22 (m, 1H), 0.99 (d, J = 15.2 Hz, 3H). |
| 736 | 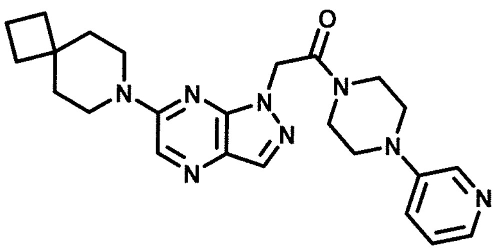 | $^1$H-NMR (400 MHz, $CDCl_3$) δ: 8.31 (s, 1H), 8.20-8.15 (m, 2H), 8.03 (s, 1H), 7.20-7.18 (m, 2H), 5.19 (s, 2H), 3.80-3.75 (m, 4H), 3.65-3.62 (m, 4H), 3.21 (s, 4H), 1.98-1.90 (m, 2H), 1.84-1.81 (m, 4H), 1.69-1.66 (m, 4H). |
| 737 | 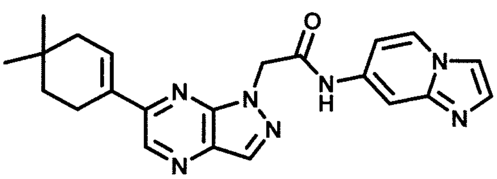 | $^1$H-NMR (400 MHz, DMSO-$d_6$) δ: 10.72 (s, 1H), 8.91-8.89 (m, 1H), 8.39-8.37 (m, 1H), 8.36 (s, 1H), 7.80 (d, J = 1.8 Hz, 1H), 7.75 (d, J = 1.2 Hz, 1H), 7.37 (d, J = 1.2 Hz, 1H), 6.92-6.89 (m, 2H), 5.31 (s, 2H), 2.52-2.50 (m, 2H), 2.02-1.99 (m, 2H), 1.41 (t, J = 6.4 Hz, 2H), 0.88-0.81 (m, 6H). |

(continued)

| Example | Chemical structure | Analytical data |
|---|---|---|
| 738 | 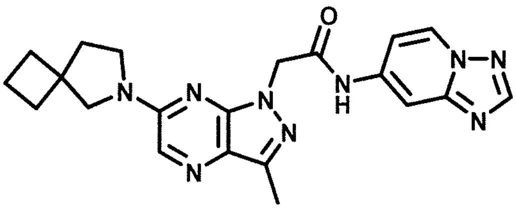 | $^1$H-NMR (400 MHz, DMSO-$d_6$) δ: 10.94 (s, 1H), 8.87 (d, J = 7.3 Hz, 1H), 8.37 (s, 1H), 8.12 (d, J = 2.3 Hz, 1H), 7.97 (s, 1H), 7.24 (dd, J = 7.3, 2.3 Hz, 1H), 5.09 (s, 2H), 3.55-3.51 (m, 4H), 2.41 (s, 3H), 2.04-1.85 (m, 8H). |
| 739 | 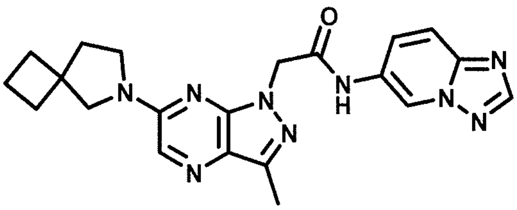 | $^1$H-NMR (400 MHz, DMSO-$d_6$) δ: 10.79 (s, 1H), 9.38 (d, J = 1.8 Hz, 1H), 8.43 (s, 1H), 7.96 (s, 1H), 7.87 (d, J = 9.6 Hz, 1H), 7.65 (dd, J = 9.6, 1.8 Hz, 1H), 5.08 (s, 2H), 3.55-3.52 (m, 4H), 2.41 (s, 3H), 2.02-1.82 (m, 8H). |
| 740 | 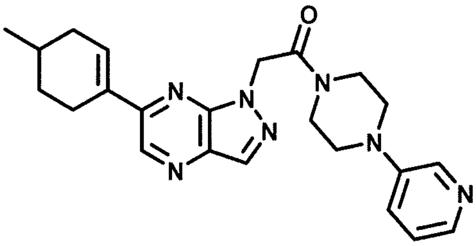 | $^1$H-NMR (400 MHz, DMSO-$d_6$) δ: 8.92 (s, 1H), 8.39 (s, 1H), 8.34 (d, J = 2.4 Hz, 1H), 8.03 (dd, J = 4.9, 1.2 Hz, 1H), 7.39-7.35 (m, 1H), 7.26-7.22 (m, 1H), 6.96-6.95 (m, 1H), 5.53-5.51 (m, 2H), 3.80 (t, J = 4.9 Hz, 2H), 3.60 (t, J = 4.9 Hz, 2H), 3.34 (t, J = 4.9 Hz, 2H), 3.20 (t, J = 4.9 Hz, 2H), 2.75-2.70 (m, 1H), 2.48-2.33 (m, 2H), 1.92-1.66 (m, 3H), 1.34-1.23 (m, 1H), 0.99-0.91 (m, 3H). |
| 741 | 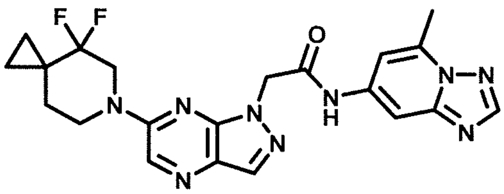 | LC-MS: [M+H]$^+$ / Rt (min) 454.0 / 0.809 (Method A) |
| 742 | 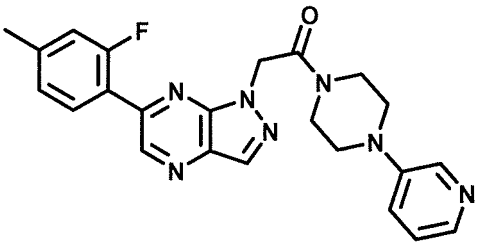 | $^1$H-NMR (400 MHz, DMSO-$d_6$) δ: 9.00 (d, J = 2.4 Hz, 1H), 8.54 (s, 1H), 8.34 (d, J = 2.4 Hz, 1H), 8.03 (dd, J = 4.3, 1.2 Hz, 1H), 7.86 (t, J = 7.9 Hz, 1H), 7.38-7.34 (m, 1H), 7.28-7.19 (m, 3H), 5.62 (s, 2H), 3.80 (t, J = 4.9 Hz, 2H), 3.59 (t, J = 4.9 Hz, 2H), 3.35 (t, J = 4.9 Hz, 2H), 3.20 (t, J = 4.9 Hz, 2H), 2.40 (s, 3H). |
| 743 | 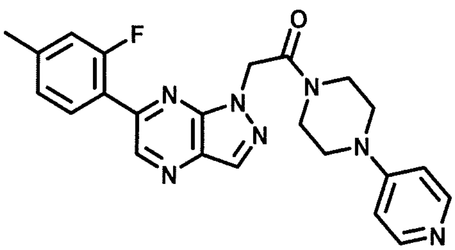 | $^1$H-NMR (400 MHz, DMSO-$d_6$) δ: 9.00 (d, J = 2.4 Hz, 1H), 8.54 (s, 1H), 8.19-8.17 (m, 2H), 7.86 (t, J = 8.2 Hz, 1H), 7.27-7.19 (m, 2H), 6.85-6.84 (m, 2H), 5.62 (s, 2H), 3.79 (t, J = 4.9 Hz, 2H), 3.56 (t, J = 4.9 Hz, 2H), 3.50 (t, J = 4.9 Hz, 2H), 3.36 (t, J = 4.9 Hz, 2H), 2.40 (s, 3H). |

(continued)

| Example | Chemical structure | Analytical data |
|---|---|---|
| 744 | 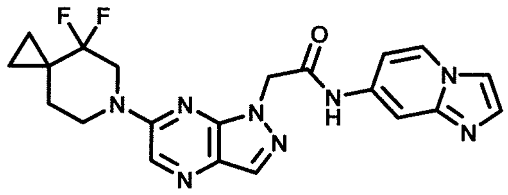 | LC-MS: $[M+H]^+$ / Rt (min) 439.0 / 0.622 (Method A) |
| 745 | 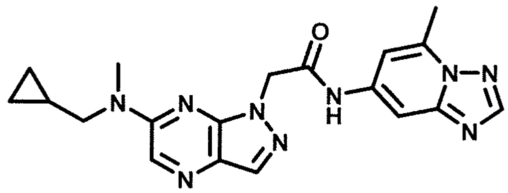 | $^1$H-NMR (400 MHz, $CDCl_3$) $\delta$: 8.96 (s, 1H), 8.31 (s, 1H), 8.23 (s, 1H), 8.22 (s, 1H), 7.96 (d, J = 1.8 Hz, 1H), 7.01 (d, J = 1.2 Hz, 1H), 5.19 (s, 2H), 3.59 (d, J = 6.7 Hz, 2H), 3.31 (s, 3H), 2.78 (s, 3H), 1.18-1.08 (m, 1H), 0.63-0.58 (m, 2H), 0.38-0.34 (m, 2H). |
| 746 | 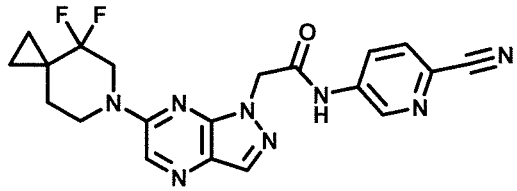 | $^1$H-NMR (400 MHz, $CDCl_3$) $\delta$: 9.10 (s, 1H), 8.60 (d, J = 2.3 Hz, 1H), 8.35-8.32 (m, 2H), 8.22 (s, 1H), 7.66 (d, J = 8.7 Hz, 1H), 5.18 (s, 2H), 4.07 (dd, J = 11.0, 5.5 Hz, 2H), 3.93-3.91 (m, 2H), 1.78 (dd, J = 5.5, 2.7 Hz, 2H), 1.03-1.02 (m, 2H), 0.56-0.54 (m, 2H). |
| 747 | 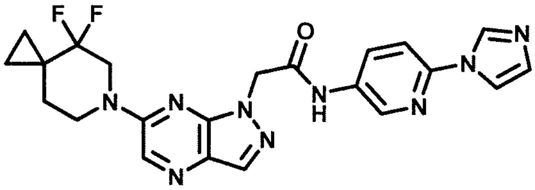 | $^1$H-NMR (400 MHz, $CDCl_3$) $\delta$: 8.80 (s, 1H), 8.44 (d, J = 2.4 Hz, 1H), 8.33 (s, 1H), 8.26-8.23 (m, 3H), 7.58 (dd, J = 1.2, 0.6 Hz, 1H), 7.32 (d, J = 8.6 Hz, 1H), 7.18 (s, 1H), 5.19 (s, 2H), 4.08 (dd, J = 11.0, 5.5 Hz, 2H), 3.93 (dd, J = 5.5, 2.8 Hz, 2H), 1.78 (dd, J = 5.5, 2.8 Hz, 2H), 1.04-1.02 (m, 2H), 0.56-0.54 (m, 2H). |
| 748 | 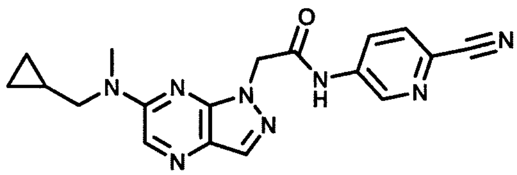 | $^1$H-NMR (400 MHz, $CDCl_3$) $\delta$: 9.19 (s, 1H), 8.59 (d, J = 2.3 Hz, 1H), 8.34 (dd, J = 8.7, 2.7 Hz, 1H), 8.18 (d, J = 9.6 Hz, 2H), 7.65 (d, J = 8.7 Hz, 1H), 5.16 (s, 2H), 3.56 (d, J = 6.4 Hz, 2H), 3.27 (s, 3H), 1.15-1.04 (m, 1H), 0.64-0.52 (m, 2H), 0.33-0.32 (m, 2H). |
| 749 | 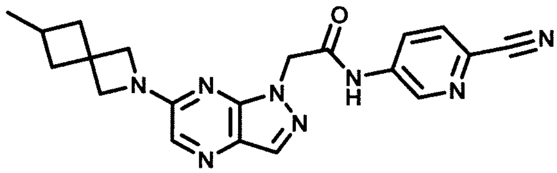 | $^1$H-NMR (400 MHz, $CDCl_3$) $\delta$: 9.14 (s, 1H), 8.57 (d, J = 2.4 Hz, 1H), 8.34 (dd, J = 8.5, 2.4 Hz, 1H), 8.16 (s, 1H), 7.77 (s, 1H), 7.65 (d, J = 8.5 Hz, 1H), 5.14 (s, 2H), 4.22 (s, 2H), 4.12 (s, 2H), 2.40-2.31 (m, 3H), 1.86 (dd, J = 11.3, 8.2 Hz, 2H), 1.09 (d, J = 6.7 Hz, 3H). |
| 750 | 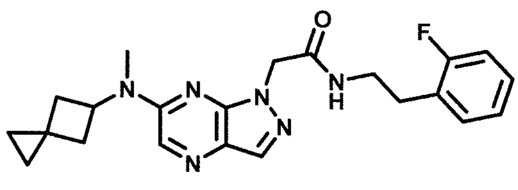 | $^1$H-NMR (400 MHz, DMSO-$d_6$) $\delta$: 8.25 (s, 1H), 8.20 (t, J = 5.5 Hz, 1 H), 8.06 (s, 1H), 7.42-7.40 (m, 1H), 7.32-7.23 (m, 3H), 5.07 (quintet, J = 8.1 Hz, 1H), 4.85 (s, 2H), 3.17 (s, 3H), 2.85 (t, J = 7.3 Hz, 2 H), 2.51-2.47 (m, 4 H), 2.31-2.26 (m, 2H), 0.56-0.52 (m, 2H), 0.46-0.42 (m, 2H). |

(continued)

| Example | Chemical structure | Analytical data |
|---|---|---|
| 751 | 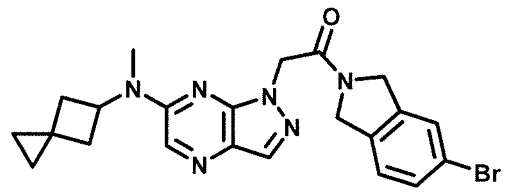 | $^1$H-NMR (400 MHz, DMSO-$d_6$) δ: 8.25 (s, 1H), 8.07 (s, 1H), 7.62 (s, 1H), 7.54-7.51 (m, 1H), 7.36 (d, J = 8.2 Hz, 1H), 5.24 (d, J = 2.3 Hz, 2H), 5.08 (s, 1H), 5.06-5.02 (m, 2H), 4.65 (d, J = 18.3 Hz, 2H), 3.16 (s, 3H), 2.51-2.45 (m, 2H), 2.27-2.22 (m, 2H), 0.48-0.44 (m, 2H), 0.42-0.38 (m, 2H). |
| 752 | 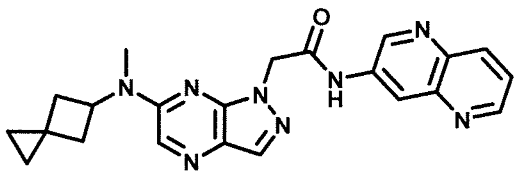 | $^1$H-NMR (400 MHz, $CDCl_3$) δ: 9.05 (s, 1H), 8.95 (dd, J = 4.3, 1.2 Hz, 1H), 8.91 (d, J = 2.4 Hz, 1H), 8.84 (s, 1H), 8.76 (d, J = 2.4 Hz, 1H), 8.33 (dd, J = 8.6, 1.8 Hz, 1H), 8.02 (s, 1H), 7.56 (dd, J = 8.6, 4.3 Hz, 1H), 5.65-5.60 (m, 1H), 5.21 (s, 2H), 3.29 (s, 3H), 2.58-2.53 (m, 2H), 2.29-2.24 (m, 2H), 0.60-0.52 (m, 2H), 0.49-0.42 (m, 2H). |
| 753 | 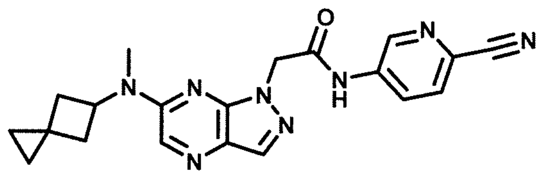 | $^1$H-NMR (400 MHz, DMSO-$d_6$) δ: 11.10 (s, 1H), 8.87 (d, J = 2.7 Hz, 1H), 8.27 (s, 1H), 8.24 (dd, J = 8.7, 2.3 Hz, 1H), 8.10 (s, 1H), 8.00 (d, J = 8.7 Hz, 1H), 5.20 (s, 2H), 5.05-4.96 (m, 1H), 3.15 (s, 3H), 2.48-2.43 (m, 2H), 2.25-2.20 (m, 2H), 0.47-0.43 (m, 2H), 0.39-0.35 (m, 2H). |
| 754 | 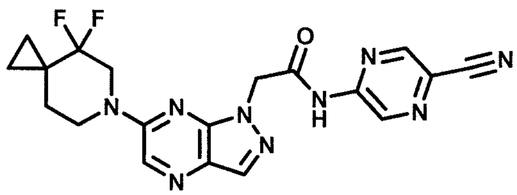 | $^1$H-NMR (400 MHz, DMSO-$d_6$) δ: 11.82 (s, 1H), 9.32 (d, J = 1.2 Hz, 1H), 9.00 (d, J = 1.8 Hz, 1H), 8.57 (s, 1H), 8.15 (s, 1H), 5.33 (s, 2H), 4.14 (dd, J = 11.3, 5.6 Hz, 2H), 3.88 (dd, J = 5.2, 2.6 Hz, 2H), 1.67 (dd, J = 5.5, 2.7 Hz, 2H), 0.83 (dd, J = 6.4, 4.6 Hz, 2H), 0.57 (d, J = 4.9 Hz, 2H). |
| 755 | 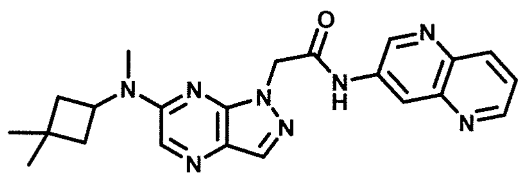 | $^1$H-NMR (400 MHz, DMSO-$d_6$) δ: 11.09 (s, 1H), 9.07 (d, J = 2.7 Hz, 1H), 8.94 (dd, J = 4.3, 1.6 Hz, 1H), 8.73 (d, J = 1.8 Hz, 1H), 8.37 (dd, J = 6.9, 0.9 Hz, 1H), 8.26 (s, 1H), 8.11 (s, 1H), 7.68 (dd, J = 8.5, 4.3 Hz, 1H), 5.24 (s, 2H), 4.78-4.69 (m, 1H), 3.07 (s, 3H), 2.10-2.05 (m, 2H), 1.95-1.89 (m, 2H), 1.06 (s, 3H), 1.01 (s, 3H). |
| 756 | 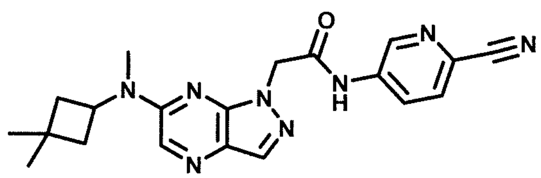 | $^1$H-NMR (400 MHz, DMSO-$d_6$) δ: 11.12 (s, 1H), 8.86 (d, J = 1.8 Hz, 1H), 8.25-8.22 (m, 2H), 8.08 (s, 1H), 7.99 (d, J = 7.8 Hz, 1H), 5.18 (s, 2H), 4.72 (tt, J = 8.4, 8.4 Hz, 1H), 3.06 (s, 3H), 2.09-2.04 (m, 2H), 1.98-1.90 (m, 2H), 1.10 (s, 3H), 1.06 (s, 3H). |
| 757 | 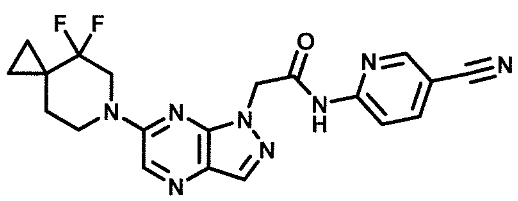 | $^1$H-NMR (400 MHz, DMSO-$d_6$) δ: 11.44 (s, 1H), 8.82 (d, J = 1.2 Hz, 1H), 8.56 (s, 1H), 8.24 (dd, J = 8.9, 2.1 Hz, 1H), 8.11 (dd, J = 8.9, 4.4 Hz, 2H), 5.28 (s, 2H), 4.14 (dd, J = 11.3, 5.6 Hz, 2H), 3.87 (dd, J = 5.2, 2.6 Hz, 2H), 1.66 (dd, J = 5.2, 2.6 Hz, 2H), 0.83 (dd, J = 5.2, 2.6 Hz, 2H), 0.57 (s, 2H). |

(continued)

| Example | Chemical structure | Analytical data |
|---|---|---|
| 758 | 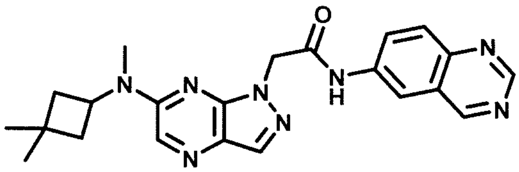 | $^1$H-NMR (400 MHz, DMSO-$d_6$) δ: 10.92 (s, 1H), 9.51 (s, 1H), 9.19 (s, 1H), 8.51 (d, J = 1.8 Hz, 1H), 8.24 (s, 1H), 8.09 (s, 1H), 8.06-7.99 (m, 2H), 5.20 (s, 2H), 4.77-4.68 (m, 1H), 3.06 (s, 3H), 2.09-2.04 (m, 2H), 1.94-1.89 (m, 2H), 1.06 (s, 3H), 1.01 (s, 3H). |
| 759 | 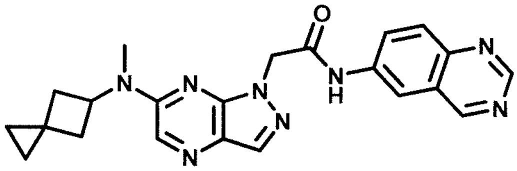 | $^1$H-NMR (400 MHz, DMSO-$d_6$) δ: 11.04 (s, 1H), 9.62 (s, 1H), 9.30 (s, 1H), 8.62 (d, J = 1.8 Hz, 1H), 8.37 (s, 1H), 8.21 (s, 1H), 8.17-8.11 (m, 2H), 5.32 (s, 2H), 5.15-5.07 (m, 1H), 3.25 (s, 3H), 2.57-2.52 (m, 2H), 2.35-2.30 (m, 2H), 0.51-0.47 (m, 2H), 0.43-0.39 (m, 2H). |
| 760 | 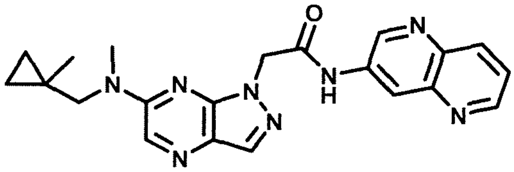 | $^1$H-NMR (400 MHz, DMSO-$d_6$) δ: 11.17 (s, 1H), 9.17 (d, J = 2.7 Hz, 1H), 9.04 (dd, J = 4.1, 1.8 Hz, 1H), 8.81 (d, J = 1.8 Hz, 1H), 8.47 (d, J = 9.1 Hz, 1H), 8.42 (s, 1H), 8.20 (s, 1H), 7.78 (dd, J = 8.2, 4.1 Hz, 1H), 5.34 (s, 2H), 3.70 (s, 2H), 3.28 (s, 3H), 1.00 (s, 3H), 0.57-0.55 (m, 2H), 0.33-0.30 (m, 2H). |
| 761 | 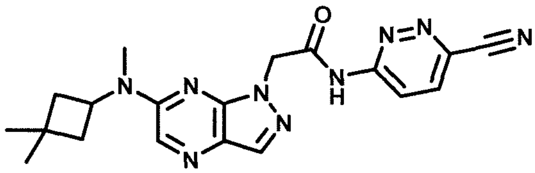 | LC-MS: $[M+H]^+$ / Rt (min) 392.4 / 0.914 (Method A) |

Example 762

2-[6-(2-Cyclopropylethoxy)-1H-pyrazolo[3,4-d]pyrimidin-1-yl]-N-([1,2,4]triazolo[1,5-a]pyridin-7-yl)acetamide

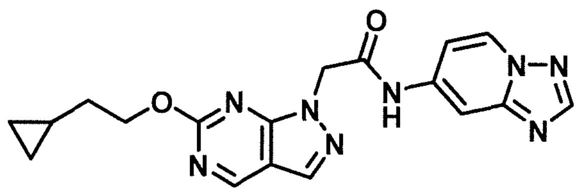

To a solution of 2-cyclopropylethanol (18.6 mg) in dimethylformamide (0.54 mL) was added 55 % sodium hydride (11.7 mg), and the solution was stirred at room temperature for 10 minutes. To the reaction solution was added the compound of Reference example 38 (20.0 mg), and the solution was stirred at 60°C for 2 hours. The reaction solution was concentrated under reduced pressure. The residue was purified by silica gel column chromatography (solvent; chloroform, and then chloroform:methanol = 90:10) to obtain the titled compound (10.3 mg).

$^1$H-NMR (400 MHz, $CDCl_3$) δ: 9.18 (s, 1H), 9.02 (s, 1H), 8.48 (d, J = 7.4 Hz, 1H), 8.29 (s, 1H), 8.20 (s, 1H), 8.02 (s, 1H), 7.31 (d, J = 7.4 Hz, 1H), 5.28 (s, 2H), 4.54 (t, J = 6.7 Hz, 2H), 1.85-1.73 (m, 1H), 0.89-0.86 (m, 2H), 0.52-0.47 (m, 2H), 0.18-0.12 (m, 2H).

Examples 763 - 804

According to the methods of Reference examples 38 - 41 or Examples 572 - 576 and 762, and common reaction

conditions, the compounds of Examples 763 - 804 were obtained by using each corresponding material compound.

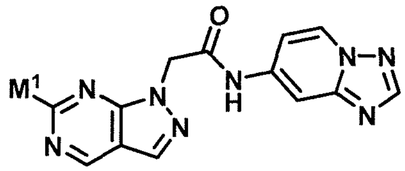

| Example | $M^1$ | Analytical data |
|---|---|---|
| 763 | 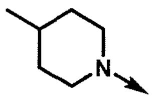 | $^1$H-NMR (400 MHz, DMSO-$d_6$) δ: 10.95 (s, 1H), 8.88 (s, 1H), 8.87-8.85 (m, 1H), 8.37 (s, 1H), 8.11 (d, J = 1.2 Hz, 1H), 8.02 (s, 1H), 7.22 (dd, J = 7.3, 2.4 Hz, 1H), 5.15 (s, 2H), 4.80-4.69 (m, 2H), 2.94-2.83 (m, 2H), 1.70-1.57 (m, 3H), 1.09-0.94 (m, 2H), 0.88 (d, J = 6.1 Hz, 3H). |
| 764 | 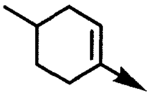 | $^1$H-NMR (400 MHz, $CDCl_3$) δ: 9.19 (s, 1H), 8.94 (br s, 1H), 8.47 (d, J = 7.3 Hz, 1H), 8.28 (s, 1H), 8.25 (s, 1H), 7.99 (d, J = 1.8 Hz, 1H), 7.54-7.49 (m, 1H), 7.20 (dd, J = 7.3, 2.4 Hz, 1H), 5.35 (s, 2H), 2.95-2.84 (m, 1H), 2.62-2.41 (m, 2H), 2.02-1.88 (m, 2H), 1.88-1.71 (m, 1H), 1.47-1.32 (m, 1H), 1.05 (d, J = 6.1 Hz, 3H). |
| 765 | 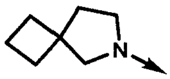 | $^1$H-NMR (400 MHz, $CDCl_3$) δ: 8.97 (s, 1H), 8.76 (s, 1H), 8.40 (d, J = 7.3 Hz, 1H), 8.21 (s, 1H), 7.94 (s, 2H), 7.14-7.11 (m, 1H), 5.08 (s, 2H), 3.63-3.54 (m, 4H), 2.01-1.88 (m, 8H). |
| 766 | 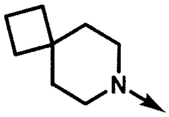 | $^1$H-NMR (400 MHz, $CDCl_3$) δ: 8.98 (s, 1H), 8.72 (s, 1H), 8.40 (d, J = 7.3 Hz, 1H), 8.21 (s, 1H), 7.94 (d, J = 1.8 Hz, 1H), 7.91 (s, 1H), 7.12 (dd, J = 7.3, 2.4 Hz, 1H), 5.06 (s, 2H), 3.80-3.77 (m, 4H), 1.88-1.84 (m, 2H), 1.79-1.75 (m, 4H), 1.59-1.52 (m, 4H). |
| 767 | 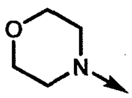 | $^1$H-NMR (400 MHz, $CDCl_3$) δ: 8.88 (s, 1H), 8.76 (s, 1H), 8.41 (d, J = 7.3 Hz, 1H), 8.21 (s, 1H), 7.97 (s, 1H), 7.95 (d, J = 1.8 Hz, 1H), 7.13 (dd, J = 7.3, 2.4 Hz, 1H), 5.07 (s, 2H), 4.21 (t, J = 5.2 Hz, 4H), 2.62 (t, J = 5.2 Hz, 4H). |
| 768 | 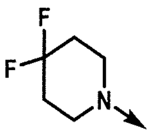 | $^1$H-NMR (400 MHz, $CDCl_3$) δ: 8.89 (s, 1H), 8.83 (s, 1H), 8.46 (d, J = 7.3 Hz, 1H), 8.26 (s, 1H), 8.04 (s, 1H), 7.99 (d, J = 1.8 Hz, 1H), 7.18 (dd, J = 7.3, 2.4 Hz, 1H), 5.13 (s, 2H), 4.10-4.07 (m, 4H), 2.06-1.99 (m, 4H). |
| 769 | 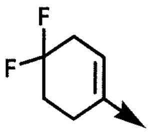 | LC-MS: $[M+H]^+$ / Rt (min) 411.0 / 0.713 (Method A) |
| 770 | 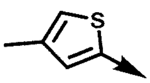 | $^1$H-NMR (400 MHz, $CDCl_3$) δ: 9.12 (s, 1H), 9.01 (s, 1H), 8.39 (d, J = 6.7 Hz, 1H), 8.20 (s, 1H), 8.18 (s, 1H), 7.95 (d, J = 1.2 Hz, 1H), 7.90 (d, J = 1.2 Hz, 1H), 7.17 (d, J = 2.4 Hz, 1H), 7.08 (s, 1H), 5.30 (s, 2H), 2.27 (s, 3H). |
| 771 | 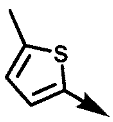 | LC-MS: $[M+H]^+$ / Rt (min) 391.0 / 0.744 (Method A) |

(continued)

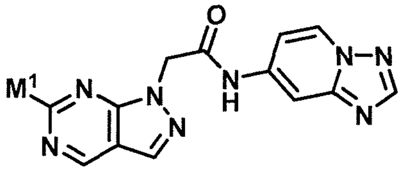

| Example | $M^1$ | Analytical data |
|---|---|---|
| 772 | 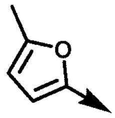 | $^1$H-NMR (400 MHz, DMSO-$d_6$) δ: 11.09 (s, 1H), 9.33 (s, 1H), 8.87 (d, J = 7.3 Hz, 1H), 8.38 (d, J = 7.9 Hz, 2H), 8.11-8.10 (m, 1H), 7.33 (d, J = 3.1 Hz, 1H), 7.25-7.22 (m, 1H), 6.36-6.34 (m, 1H), 5.44 (s, 2H), 2.39 (s, 3H). |
| 773 | 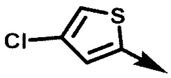 | $^1$H-NMR (400 MHz, $CDCl_3$) δ: 9.79 (s, 1H), 9.38-9.32 (m, 1H), 9.08-9.01 (m, 2H), 8.88-8.82 (m, 2H), 8.58-8.54 (m, 2H), 7.88 (s, 1H), 5.95 (s, 2H). |
| 774 | 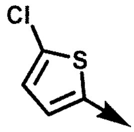 | LC-MS: $[M+H]^+$ / Rt (min) 411.0 / 0.832 (Method A) |
| 775 | 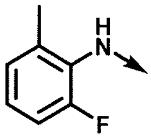 | $^1$H-NMR (400 MHz, $CDCl_3$) δ: 9.01 (s, 1H), 8.82 (s, 1H), 8.37 (d, J = 7.3 Hz, 1H), 8.21 (s, 1H), 8.01 (s, 1H), 7.87 (s, 1H), 7.14-7.7.10 (m, 1H), 7.04-7.02 (m, 2H), 6.98-6.94 (m, 1H), 5.02 (s, 2H), 2.26 (s, 3H). |
| 776 | 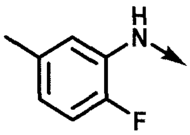 | $^1$H-NMR (400 MHz, $CDCl_3$) δ: 9.09 (s, 1H), 8.84 (s, 1H), 8.35 (d, J = 7.3 Hz, 1H), 8.18-8.17 (m, 2H), 8.03 (s, 1H), 7.92 (d, J = 1.8 Hz, 1H), 7.55 (d, J = 2.4 Hz, 1H), 7.07 (dd, J = 7.3, 2.4 Hz, 1H), 6.94 (dd, J = 11.0, 7.9 Hz, 1H), 6.76 (t, J = 5.5 Hz, 1H), 5.14 (s, 2H), 2.30 (s, 3H). |
| 777 | 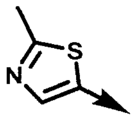 | LC-MS: $[M+H]^+$ / Rt (min) 391.8 / 0.560 (Method A) |
| 778 | 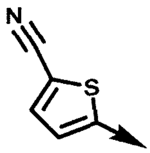 | LC-MS: $[M+H]^+$ / Rt (min) 401.9 / 0.689 (Method A) |
| 779 | 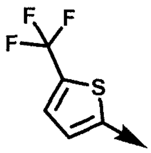 | LC-MS: $[M+H]^+$ / Rt (min) 444.9 / 0.851 (Method A) |
| 780 | 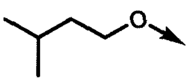 | LC-MS: $[M+H]^+$ / Rt (min) 381.0 / 0.722 (Method A) |

(continued)

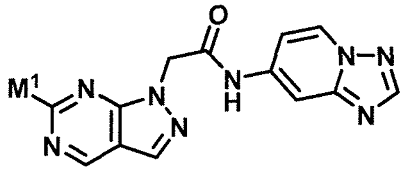

| Example | $M^1$ | Analytical data |
| --- | --- | --- |
| 781 | 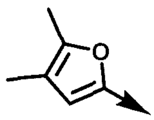 | $^1$H-NMR (400 MHz, DMSO-$d_6$) δ: 11.02 (s, 1H), 9.22 (s, 1H), 8.79 (d, J = 7.3 Hz, 1H), 8.29 (d, J = 1.8 Hz, 2H), 8.02 (d, J = 1.8 Hz, 1H), 7.17-7.14 (m, 2H), 5.34 (s, 2H), 2.22 (s, 3H), 1.91-1.90 (m, 3H). |
| 782 | 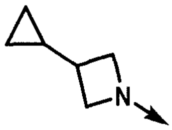 | $^1$H-NMR (400 MHz, $CDCl_3$) δ: 9.60 (s, 1H), 9.38 (s, 1H), 9.03 (d, J = 7.3 Hz, 1H), 8.84 (s, 1H), 8.58 (s, 1H), 8.55 (d, J = 2.3 Hz, 1H), 7.76 (dd, J = 7.6, 2.3 Hz, 1H), 5.70 (s, 2H), 4.83 (t, J = 8.7 Hz, 2H), 4.49-4.45 (m, 2H), 3.03-2.93 (m, 1H), 1.69-1.60 (m, 1H), 1.12-1.10 (m, 2H), 0.80-0.76 (m, 2H). |
| 783 | 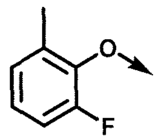 | $^1$H-NMR (400 MHz, $CDCl_3$) δ: 9.46 (s, 1H), 9.06 (s, 1H), 8.48 (d, J = 7.3 Hz, 1H), 8.30-8.24 (m, 2H), 8.00 (s, 1H), 7.31-7.27 (m, 1H), 7.21-7.15 (m, 1H), 7.12-7.02 (m, 2H), 5.28 (s, 2H), 2.28 (s, 3H). |
| 784 | 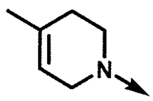 | $^1$H-NMR (400 MHz, DMSO-$d_6$) δ: 10.87 (s, 1H), 8.83 (s, 1H), 8.79 (d, J = 7.3 Hz, 1H), 8.29 (s, 1H), 8.03 (d, J = 1.8 Hz, 1H), 7.97 (s, 1H), 7.14 (dd, J = 7.3, 1.8 Hz, 1H), 5.39 (s, 1H), 5.09 (s, 2H), 4.10 (s, 2H), 3.86 (t, J = 5.8 Hz, 2H), 1.97 (s, 2H), 1.59 (s, 3H). |
| 785 | 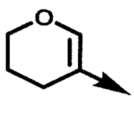 | $^1$H-NMR (400 MHz, $CDCl_3$) δ: 9.00 (s, 1H), 8.97 (s, 1H), 8.40 (d, J = 7.3 Hz, 1H), 8.21 (s, 1H), 8.15 (s, 1H), 8.12 (s, 1H), 8.04 (s, 1H), 7.14 (dd, J = 7.6, 2.1 Hz, 1H), 5.24 (s, 2H), 4.11-4.09 (m, 2H), 2.59-2.56 (m, 2H), 1.98-1.95 (m, 2H). |
| 786 | 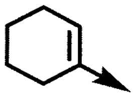 | $^1$H-NMR (400 MHz, $CDCl_3$) δ: 9.12 (s, 1H), 8.99 (s, 1H), 8.40 (d, J = 7.3 Hz, 1H), 8.21 (s, 1H), 8.18 (s, 1H), 7.92 (d, J = 1.8 Hz, 1H), 7.48 (d, J = 4.3 Hz, 1H), 7.15 (dd, J = 7.3, 2.4 Hz, 1H), 5.29 (s, 2H), 2.59-2.61 (m, 2H), 2.28-2.30 (m, 2H), 1.77-1.74 (m, 2H), 1.66-1.64 (m, 2H). |
| 787 | 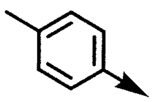 | $^1$H-NMR (400 MHz, $CDCl_3$) δ: 11.21 (s, 1H), 9.44 (s, 1H), 8.90 (d, J = 7.3 Hz, 1H), 8.44 (s, 2H), 8.41 (s, 1H), 8.14 (s, 1H), 7.35 (s, 1H), 7.33 (s, 1H), 7.29 (d, J = 10.1 Hz, 1H), 5.51 (s, 2H), 2.37 (s, 3H). |
| 788 | 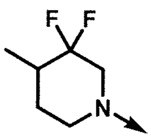 | $^1$H-NMR (400 MHz, $CDCl_3$) δ: 8.91 (s, 1H), 8.76 (d, J = 6.4 Hz, 1H), 8.40 (d, J = 8.2 Hz, 1H), 8.21 (s, 1H), 7.97 (s, 1H), 7.95 (d, J = 1.4 Hz, 1H), 7.13 (dd, J = 7.3, 2.3 Hz, 1H), 5.09-5.06 (m, 3H), 4.79 (d, J = 13.2 Hz, 1H), 3.24-3.02 (m, 2H), 2.02-1.98 (m, 1H), 1.79-1.76 (m, 1H), 1.55-1.53 (m, 1H), 1.04 (d, J = 6.9 Hz, 3H). |

(continued)

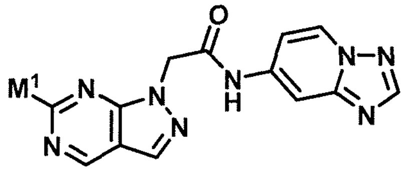

| Example | $M^1$ | Analytical data |
|---|---|---|
| 789 | 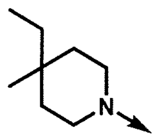 | $^1$H-NMR (400 MHz, $CDCl_3$) $\delta$: 9.04 (s, 1H), 8.72 (s, 1H), 8.39 (d, J = 4.1 Hz, 1H), 8.20 (s, 1H), 7.97-7.91 (m, 2H), 7.13 (dd, J = 7.3, 2.3 Hz, 1H), 5.07 (d, J = 11.0 Hz, 2H), 4.13-4.04 (m, 2H), 3.61-3.54 (m, 2H), 1.43-1.26 (m, 6H), 0.90 (s, 3H), 0.79 (t, J = 7.5 Hz, 3H) . |
| 790 | 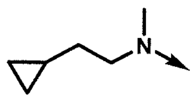 | $^1$H-NMR (400 MHz, $CDCl_3$) $\delta$: 8.99 (s, 1H), 8.74 (s, 1H), 8.39 (d, J = 7.3 Hz, 1H), 8.20 (s, 1H), 7.93-7.91 (m, 2H), 7.13 (dd, J = 7.6, 2.1 Hz, 1H), 5.06 (s, 2H), 3.73 (s, 2H), 3.18 (s, 3H), 1.50-1.45 (m, 2H), 0.62-0.59 (m, 1H), 0.39-0.34 (m, 2H), 0.01-0.02 (m, 2H). |
| 791 | 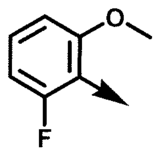 | $^1$H-NMR (400 MHz, $CDCl_3$) $\delta$: 9.32 (s, 1H), 9.20 (s, 1H), 8.35 (d, J = 8.2 Hz, 1H), 8.27 (s, 1H), 8.18 (s, 1H), 7.84 (d, J = 1.8 Hz, 1H), 7.37-7.31 (m, 1H), 7.13 (dd, J = 7.5, 2.1 Hz, 1H), 6.80-6.76 (m, 2H), 5.32 (s, 2H), 3.70 (s, 3H). |
| 792 | 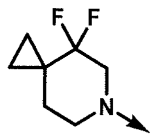 | LC-MS: $[M+H]^+$ / Rt (min) 440.2 / 0.810 (Method A) |
| 793 | 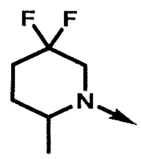 | LC-MS: $[M+H]^+$ / Rt (min) 428.1 / 0.530 (Method A) |
| 794 | 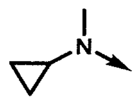 | $^1$H-NMR (400 MHz, $CDCl_3$) $\delta$: 8.99 (s, 1H), 8.82 (s, 1H), 8.40 (d, J = 7.3 Hz, 1H), 8.21 (s, 1H), 7.97 (s, 1H), 7.92 (d, J = 1.4 Hz, 1H), 7.14 (dd, J = 7.5, 2.1 Hz, 1H), 5.10 (s, 2H), 3.19 (s, 3H), 2.85-2.81 (m, 1H), 0.91-0.86 (m, 2H), 0.68-0.64 (m, 2H). |
| 795 | 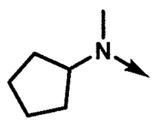 | $^1$H-NMR (400 MHz, $CDCl_3$) $\delta$: 9.00 (s, 1H), 8.79 (s, 1H), 8.45 (d, J = 7.8 Hz, 1H), 8.25 (s, 1H), 7.99 (d, J = 1.8 Hz, 1H), 7.98 (s, 1H), 7.17 (dd, J = 7.8, 2.3 Hz, 1H), 5.33-5.31 (m, 1H), 5.12 (s, 2H), 3.07 (s, 3H), 1.89-1.87 (m, 2H), 1.76-1.74 (m, 2H), 1.65-1.63 (m, 2H), 1.24-1.22 (m, 2H). |
| 796 | 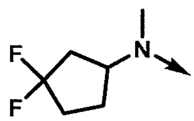 | LC-MS: $[M+H]^+$ / Rt (min) 428.1 / 0.816 (Method A) |
| 797 | 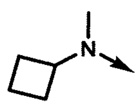 | LC-MS: $[M+H]^+$ / Rt (min) 378.1 / 0.778 (Method A) |

(continued)

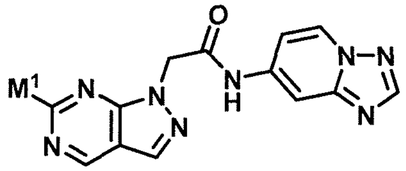

| Example | $M^1$ | Analytical data |
|---|---|---|
| 798 | 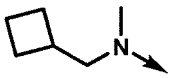 | $^1$H-NMR (400 MHz, $CDCl_3$) δ: 9.01 (s, 1H), 8.45 (t, J = 8.2 Hz, 1H), 8.25 (s, 1H), 7.99-7.97 (m, 2H), 7.17 (dd, J = 7.8, 2.3 Hz, 1H), 5.12 (s, 2H), 3.77 (d, J = 6.4 Hz, 2H), 3.20 (s, 3H), 2.72-2.68 (m, 1H), 2.05-1.99 (m, 2H), 1.88-1.76 (m, 4H). |
| 799 | 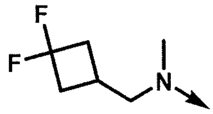 | LC-MS: $[M+H]^+$ / Rt (min) 428.1 / 0.956 (Method A) |
| 800 | 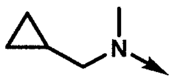 | LC-MS: $[M+H]^+$ / Rt (min) 378.1 / 0.700 (Method A) |
| 801 | 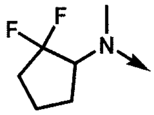 | LC-MS: $[M+H]^+$ / Rt (min) 428.1 / 0.792 (Method A) |
| 802 | 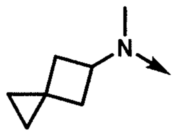 | $^1$H-NMR (400 MHz, $CDCl_3$) δ: 9.04 (s, 1H), 8.84 (s, 1H), 8.47 (d, J = 7.3 Hz, 1H), 8.28 (s, 1H), 8.02-7.99 (m, 2H), 7.20 (dd, J = 7.3, 2.4 Hz, 1H), 5.57 (br s, 1H), 5.16 (s, 2H), 3.29 (s, 3H), 2.58-2.54 (m, 2H), 2.29-2.25 (m, 2H), 0.60-0.56 (m, 2H), 0.49-0.45 (m, 2H). |
| 803 | 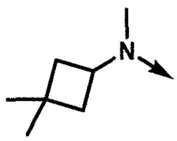 | $^1$H-NMR (400 MHz, DMSO-$d_6$) δ: 10.83 (s, 1H), 8.88 (s, 1H), 8.83 (d, J = 7.3 Hz, 1H), 8.34 (s, 1H), 8.09 (d, J = 1.8 Hz, 1H), 8.01 (s, 1H), 7.25 (dd, J = 7.3, 2.4 Hz, 1H), 5.12 (dd, J = 11.6, 5.8 Hz, 3H), 3.10 (s, 3H), 2.03-1.90 (m, 4H), 1.11 (s, 3H), 1.07 (s, 3H). |
| 804 | 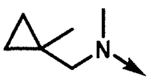 | $^1$H-NMR (400 MHz, DMSO-$d_6$) δ: 10.50 (s, 1H), 8.41 (dd, J = 11.9, 4.6 Hz, 2H), 7.92 (s, 1H), 7.66 (d, J = 1.2 Hz, 1H), 7.57 (s, 1H), 6.78 (dd, J = 7.3, 2.4 Hz, 1H), 4.71 (s, 2H), 3.20 (br s, 2H), 2.73 (br s, 3H), 0.81-0.79 (m, 2H), 0.43-0.39 (m, 5H). |

Examples 805 - 810

According to the methods of Reference examples 38 - 41 or Examples 572 - 576 and 762, and common reaction conditions, the compounds of Examples 805 - 810 were obtained by using each corresponding material compound.

<table>
<tr><td colspan="3">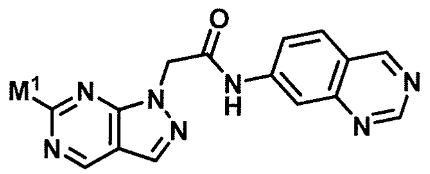</td></tr>
<tr><td>Example</td><td>M<sup>1</sup></td><td>Analytical data</td></tr>
<tr><td>805</td><td>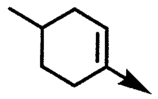</td><td>$^{1}$H-NMR (400 MHz, DMSO-$d_6$) δ: 11.08 (br s, 1H), 9.44 (s, 1H), 9.29 (s, 1H), 9.18 (s, 1H), 8.36 (s, 1H), 8.34 (d, J = 1.8 Hz, 1H), 8.12 (d, J = 9.2 Hz, 1H), 7.81 (dd, J = 8.9, 2.1 Hz, 1H), 7.39-7.35 (m, 1H), 5.42 (s, 2H), 2.85-2.73 (m, 1H), 2.45-2.29 (m, 2H), 1.93-1.76 (m, 2H), 1.75-1.63 (m, 1H), 1.34-1.20 (m, 1H), 0.97 (d, J = 6.7 Hz, 3H).</td></tr>
<tr><td>806</td><td>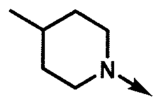</td><td>$^{1}$H-NMR (400 MHz, $CDCl_3$) δ: 9.26 (s, 1H), 9.24 (s, 1H), 9.03 (s, 1H), 8.78 (s, 1H), 8.14 (s, 1H), 7.98 (s, 1H), 7.84 (s, 2H), 5.14 (s, 2H), 4.89-4.86 (m, 2H), 2.98-2.92 (m, 2H), 1.76-1.73 (m, 2H), 1.26-1.15 (m, 2H), 0.96 (d, J = 6.1 Hz, 3H), 0.86-0.83 (m, 1H).</td></tr>
<tr><td>807</td><td>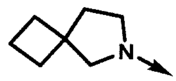</td><td>LC-MS: $[M+H]^+$ / Rt (min) 415.1 / 0.802 (Method A)</td></tr>
<tr><td>808</td><td>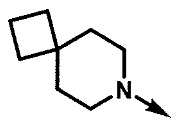</td><td>$^{1}$H-NMR (400 MHz, $CDCl_3$) δ: 9.21 (s, 1H), 9.19 (s, 1H), 8.98 (s, 1H), 8.73 (s, 1H), 8.09 (s, 1H), 7.93 (s, 1H), 7.79-7.76 (m, 2H), 5.09 (s, 2H), 3.80-3.77 (m, 4H), 1.90-1.85 (m, 2H), 1.79-1.75 (m, 4H), 1.59-1.56 (m, 4H).</td></tr>
<tr><td>809</td><td>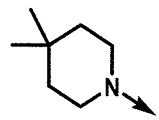</td><td>$^{1}$H-NMR (400 MHz, $CDCl_3$) δ: 9.38 (s, 1H), 9.18 (s, 1H), 9.17 (s, 1H), 8.88 (s, 1H), 8.18 (s, 1H), 7.79-7.75 (m, 2H), 7.62 (s, 1H), 4.79 (s, 2H), 3.79-3.70 (m, 4H), 1.62-1.60 (m, 4H), 1.04 (s, 6H).</td></tr>
<tr><td>810</td><td>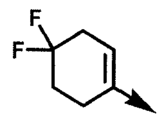</td><td>$^{1}$H-NMR (400 MHz, $CDCl_3$) δ: 9.22 (s, 1H), 9.20 (s, 1H), 9.15 (s, 1H), 8.83 (s, 1H), 8.23 (s, 1H), 8.08 (s, 1H), 7.83-7.81 (m, 2H), 7.29 (s, 1H), 5.33 (s, 2H), 2.96-2.90 (m, 2H), 2.81-2.75 (m, 2H), 2.19-2.10 (m, 2H).</td></tr>
</table>

Examples 811 - 822

According to the methods of Reference examples 38 - 41 or Examples 572 - 576 and 762, and common reaction conditions, the compounds of Examples 811 - 822 were obtained by using each corresponding material compound.

<table>
<tr><td colspan="3">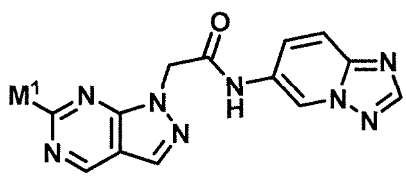</td></tr>
<tr><td>Example</td><td>M<sup>1</sup></td><td>Analytical data</td></tr>
<tr><td>811</td><td>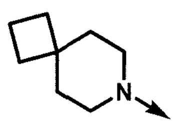</td><td>$^{1}$H-NMR (400 MHz, $CDCl_3$) δ: 9.40 (s, 1H), 8.80 (s, 1H), 8.71 (s, 1H), 8.24 (s, 1H), 7.90 (s, 1H), 7.59 (d, J = 9.8 Hz, 1H), 7.19 (s, 1H), 5.07 (s, 2H), 3.78 (t, J = 5.8 Hz, 4H), 1.87 (d, J = 9.1 Hz, 2H), 1.77 (t, J = 7.0 Hz, 4H), 1.57 (t, J = 5.5 Hz, 4H).</td></tr>
</table>

(continued)

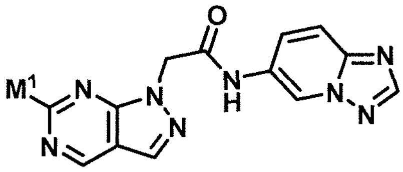

| Example | $M^1$ | Analytical data |
|---|---|---|
| 812 | 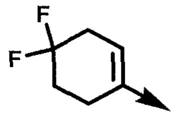 | $^1$H-NMR (400 MHz, $CDCl_3$) δ: 9.44 (s, 1H), 9.20 (s, 1H), 8.52 (s, 1H), 8.31 (s, 1H), 8.28 (s, 1H), 7.67 (d, J = 9.2 Hz, 1H), 7.37 (d, J = 14.7 Hz, 1H), 5.35 (s, 2H), 3.00-2.97 (m, 2H), 2.88-2.81 (m, 2H), 2.25-2.18 (m, 2H). |
| 813 | 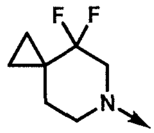 | LC-MS: $[M+H]^+$ / Rt (min) 439.9 / 0.801 (Method A) |
| 814 | 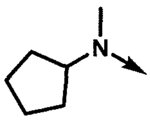 | LC-MS: $[M+H]^+$ / Rt (min) 392.0 / 0.773 (Method A) |
| 815 | 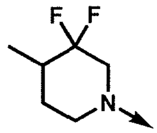 | LC-MS: $[M+H]^+$ / Rt (min) 428.0 / 0.777 (Method A) |
| 816 | 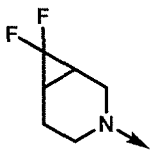 | LC-MS: $[M+H]^+$ / Rt (min) 426.1 / 0.722 (Method A) |
| 817 | 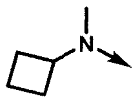 | LC-MS: $[M+H]^+$ / Rt (min) 378.0 / 0.725 (Method A) |
| 818 | 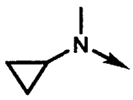 | $^1$H-NMR (400 MHz, $CDCl_3$) δ: 9.39 (s, 1H), 8.82 (s, 2H), 8.26-8.24 (m, 1H), 7.97 (s, 1H), 7.61 (s, 1H), 7.20 (br s, 1H), 5.11 (s, 2H), 3.18 (s, 3H), 2.83-2.81 (m, 1H), 0.90-0.88 (m, 2H), 0.68-0.66 (m, 2H). |
| 819 | 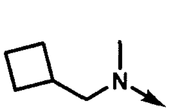 | $^1$H-NMR (400 MHz, DMSO-$d_6$) δ: 10.81 (s, 1H), 9.38 (s, 1H), 8.87 (s, 1H), 8.43 (s, 1H), 8.01 (s, 1H), 7.86 (d, J = 9.6 Hz, 1H), 7.63 (d, J = 9.6 Hz, 1H), 5.14 (s, 2H), 3.71 (br s, 2H), 3.12 (s, 3H), 2.63 (br s, 1H), 1.60-1.90 (m, 6H). |
| 820 | 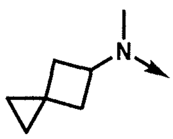 | LC-MS: $[M+H]^+$ / Rt (min) 404.1 / 0.895 (Method A) |
| 821 | 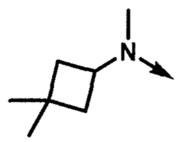 | $^1$H-NMR (400 MHz, $CDCl_3$) δ: 9.44 (d, J = 1.8 Hz, 1H), 8.85 (s, 1H), 8.78 (s, 1H), 8.29 (s, 1H), 7.96 (s, 1H), 7.64 (d, J = 8.5 Hz, 1H), 7.24-7.22 (m, 1H), 5.28-5.18 (m, 1H), 5.13 (s, 2H), 3.16 (s, 3H), 2.13-2.08 (m, 2H), 2.04-1.97 (m, 2H), 1.23 (s, 3H), 1.14 (s, 3H). |

(continued)

| 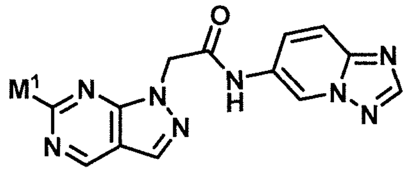 | | |
|---|---|---|
| Example | $M^1$ | Analytical data |
| 822 | 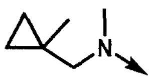 | LC-MS: $[M+H]^+$ / Rt (min) 392.3 / 0.770 (Method A) |

Examples 823 - 830

According to the methods of Reference examples 38 - 41 or Examples 572 - 576 and 762, and common reaction conditions, the compounds of Examples 823 - 830 were obtained by using each corresponding material compound.

| 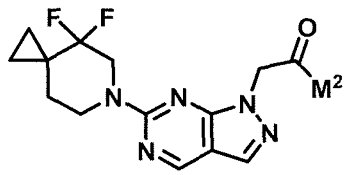 | | |
|---|---|---|
| Example | $M^2$ | Analytical data |
| 823 | 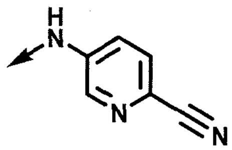 | $^1$H-NMR (400 MHz, $CDCl_3$) δ: 8.98 (s, 1H), 8.85 (s, 1H), 8.56 (d, J = 1.8 Hz, 1H), 8.36 (dd, J = 8.5, 2.4 Hz, 1H), 8.05 (s, 1H), 7.66 (d, J = 8.5 Hz, 1H), 5.15 (s, 2H), 4.27 (dd, J = 11.0, 5.5 Hz, 2H), 4.09-4.07 (m, 2H), 1.71 (dd, J = 5.5, 2.7 Hz, 2H), 1.00 (dd, J = 5.2, 2.6 Hz, 2H), 0.52 (s, 2H). |
| 824 | 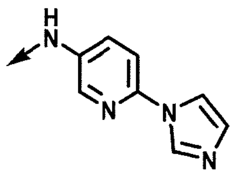 | $^1$H-NMR (400 MHz, $CDCl_3$) δ: 9.03 (s, 1H), 8.82 (s, 1H), 8.42 (d, J = 2.3 Hz, 1H), 8.28-8.25 (m, 2H), 8.03 (s, 1H), 7.57 (dd, J = 1.4, 0.7 Hz, 1H), 7.31 (d, J = 8.7 Hz, 1H), 7.17-7.17 (m, 1H), 5.16 (s, 2H), 4.26 (dd, J = 11.2, 5.6 Hz, 2H), 4.08-4.07 (m, 2H), 1.71-1.70 (m, 2H), 1.00-0.98 (m, 2H), 0.52-0.50 (m, 2H). |
| 825 | 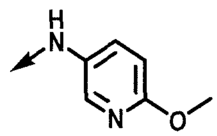 | $^1$H-NMR (400 MHz, $CDCl_3$) δ: 8.84 (s, 1H), 8.19 (s, 1H), 8.12 (d, J = 2.3 Hz, 1H), 8.02 (s, 1H), 7.82 (dd, J = 9.1, 2.7 Hz, 1H), 6.71 (d, J = 9.1 Hz, 1H), 5.12 (s, 2H), 4.27 (dd, J = 11.2, 5.6 Hz, 2H), 4.08 (dd, J = 5.5, 2.7 Hz, 2H), 3.90 (br s, 3H), 1.71 (dd, J = 5.3, 2.6 Hz, 2H), 1.00 (dd, J = 6.2, 4.8 Hz, 2H), 0.51 (s, 2H). |
| 826 | 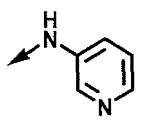 | $^1$H-NMR (400 MHz, $CDCl_3$) δ: 8.83 (s, 1H), 8.57 (s, 1H), 8.50 (d, J = 2.7 Hz, 1H), 8.34 (dd, J = 4.6, 1.4 Hz, 1H), 8.12-8.10 (m, 1H), 8.02 (s, 1H), 7.26-7.25 (m, 2H), 5.13 (s, 2H), 4.27 (dd, J = 11.2, 5.6 Hz, 2H), 4.07 (dd, J = 5.5, 2.7 Hz, 2H), 1.70 (dd, J = 5.5, 2.7 Hz, 1H), 0.99 (dd, J = 6.2, 4.8 Hz, 2H), 0.52 (d, J = 5.9 Hz, 2H). |
| 827 | 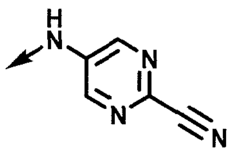 | $^1$H-NMR (400 MHz, DMSO-$d_6$) δ: 11.30 (s, 1H), 9.13 (s, 2H), 8.95 (s, 1H), 8.10 (s, 1H), 5.23 (s, 2H), 4.24 (dd, J = 11.4, 5.7 Hz, 2H), 3.99 (dd, J = 5.3, 2.6 Hz, 2H), 1.62 (dd, J = 5.5, 2.7 Hz, 2H), 0.83 (dd, J = 5.7, 4.8 Hz, 2H), 0.56 (s, 2H). |

(continued)

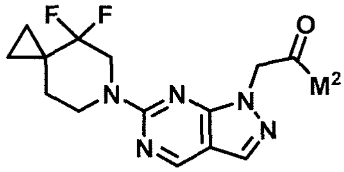

| Example | $M^2$ | Analytical data |
|---|---|---|
| 828 | 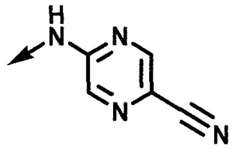 | $^1$H-NMR (400 MHz, DMSO-$d_6$) δ: 11.82 (s, 1H), 9.32 (d, J = 1.4 Hz, 1H), 9.00 (d, J = 1.4 Hz, 1H), 8.95 (s, 1H), 8.09 (s, 1H), 5.30 (s, 2H), 4.23 (dd, J = 11.4, 5.7 Hz, 2H), 3.98 (d, J = 5.5 Hz, 2H), 1.62 (dd, J = 5.3, 2.6 Hz, 2H), 0.82 (dd, J = 5.3, 2.6 Hz, 2H), 0.56 (s, 2H). |
| 829 | 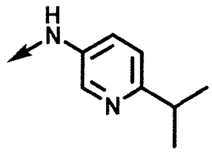 | $^1$H-NMR (400 MHz, DMSO-$d_6$) δ: 10.46 (s, 1H), 8.88 (s, 1H), 8.56 (d, J = 2.3 Hz, 1H), 8.02 (s, 1H), 7.85 (dd, J = 8.5, 2.5 Hz, 1H), 7.16 (d, J = 8.7 Hz, 1H), 5.06 (s, 2H), 4.18 (dd, J = 11.4, 5.7 Hz, 2H), 3.93 (s, 2H), 2.94-2.87 (m, 1H), 1.56 (dd, J = 5.0, 2.5 Hz, 2H), 1.14 (s, 3H), 1.12 (s, 3H), 0.77 (dd, J = 5.3, 2.6 Hz, 2H), 0.50 (s, 2H). |
| 830 | 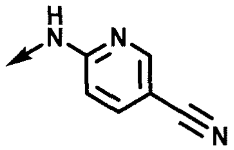 | $^1$H-NMR (400 MHz, DMSO-$d_6$) δ: 11.45 (s, 1H), 8.94 (s, 1H), 8.82 (s, 1H), 8.25 (dd, J = 8.9, 2.1 Hz, 1H), 8.09 (dd, J = 7.5, 3.8 Hz, 2H), 5.25 (s, 2H), 4.23 (dd, J = 11.2, 5.6 Hz, 2H), 4.00-3.96 (m, 2H), 1.62-1.60 (br m, 2H), 0.83-0.81 (br m, 2H), 0.57-0.55 (br m, 2H). |

Examples 831 - 851

According to the methods of Reference examples 38 - 41 or Examples 572 - 576 and 762, and common reaction conditions, the compounds of Examples 831 - 851 were obtained by using each corresponding material compound.

| Example | Chemical structure | Analytical data |
|---|---|---|
| 831 | 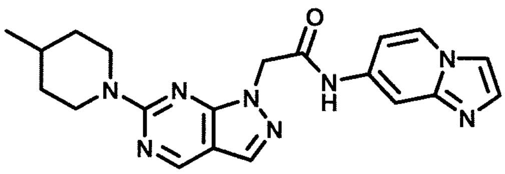 | $^1$H-NMR (400 MHz, $CDCl_3$) δ: 9.86 (s, 1H), 8.74 (s, 1H), 8.04 (d, J = 7.9 Hz, 1H), 7.92 (s, 1H), 7.84 (s, 1H), 7.58 (d, J = 1.2 Hz, 1H), 7.51 (s, 1H), 7.46 (d, J = 4.3 Hz, 1H), 5.20 (s, 2H), 4.84 (d, J = 13.4 Hz, 2H), 2.90 (dd, J = 18.3, 7.3 Hz, 2H), 2.63 (s, 3H), 1.72-1.63 (m, 3H), 1.14 (td, J = 14.3, 6.5 Hz, 2H), 0.93 (d, J = 6.7 Hz, 3H). |
| 832 | 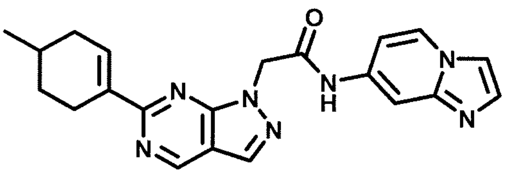 | $^1$H-NMR (400 MHz, $CDCl_3$) δ: 9.13 (s, 1H), 8.18 (s, 1H), 8.02 (d, J = 7.3 Hz, 1H), 7.78 (br s, 1H), 7.57 (s, 1H), 7.50 (s, 1H), 7.47-7.41 (m, 1H), 7.41-7.30 (m, 1H), 5.39 (s, 2H), 2.92-2.81 (m, 1H), 2.58-2.36 (m, 2H), 2.12-1.85 (m, 2H), 1.83-1.69 (m, 1H), 1.41-1.28 (m, 1H), 1.01 (d, J = 6.1 Hz, 3H). |
| 833 | 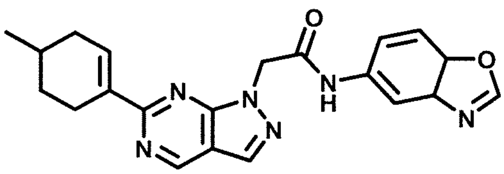 | $^1$H-NMR (400 MHz, DMSO-$d_6$) δ: 10.63 (br s, 1H), 9.27 (s, 1H), 8.70 (s, 1H), 8.34 (s, 1H), 8.08 (d, J = 1.8 Hz, 1H), 7.73 (d, J = 9.2 Hz, 1H), 7.52 (dd, J = 8.5, 1.8 Hz, 1H), 7.37 (br s, 1H), 5.33 (s, 2H), 2.86-2.74 (m, 1H), 2.44-2.33 (m, 2H), 1.95-1.78 (m, 2H), 1.78-1.64 (m, 1H), 1.35-1.23 (m, 1H), 0.98 (d, J = 6.1 Hz, 3H). |

(continued)

| Example | Chemical structure | Analytical data |
|---|---|---|
| 834 | 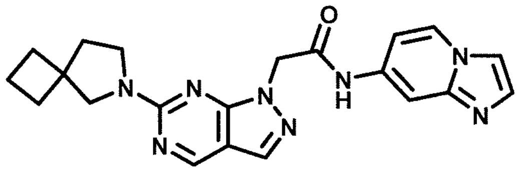 | $^1$H-NMR (400 MHz, $CDCl_3$) δ: 8.84 (s, 1H), 8.72 (s, 1H), 7.95-7.91 (m, 2H), 7.62 (s, 1H), 7.49 (s, 1H), 7.41 (s, 1H), 7.05 (dd, J = 7.3, 2.4 Hz, 1H), 5.06 (s, 2H), 3.59 (4H, br s), 1.98-1.71 (m, 8H). |
| 835 | 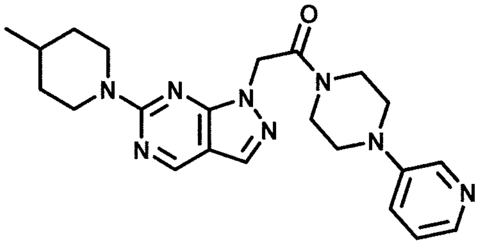 | $^1$H-NMR (400 MHz, $CDCl_3$) δ: 8.67 (s, 1H), 8.25 (s, 1H), 8.10 (s, 1H), 7.81 (s, 1H), 7.16-7.14 (m, 2H), 5.09 (s, 2H), 4.79-4.76 (m, 2H), 3.76-3.68 (m, 4H), 2.88-2.81 (m, 2H), 1.74-1.55 (m, 7H), 1.19-1.05 (m, 2H), 0.91-0.89 (m, 3H). |
| 836 | 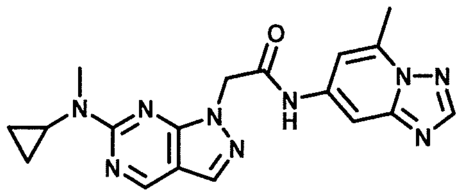 | $^1$H-NMR (400 MHz, $CDCl_3$) δ: 8.85 (s, 1H), 8.31 (s, 1H), 8.06 (s, 1H), 8.02 (s, 1H), 7.12 (s, 1H), 5.21 (s, 2H), 3.20 (s, 3H), 2.83-2.78 (m, 1H), 2.73 (s, 3H), 0.89-0.85 (m, 2H), 0.69-0.64 (m, 2H) . |
| 837 | 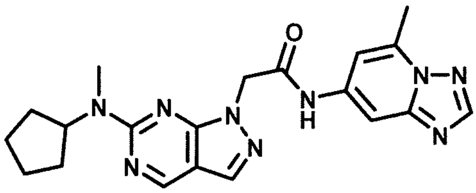 | LC-MS: $[M+H]^+$ / Rt (min) 406.1 / 0.877 (Method A) |
| 838 | 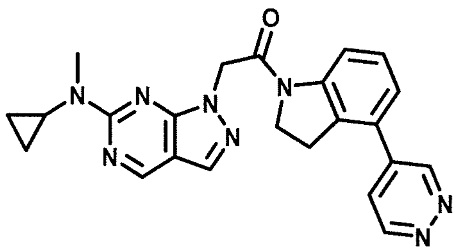 | LC-MS: $[M+H]^+$ / Rt (min) 427.1 / 0.776 (Method A) |
| 839 | 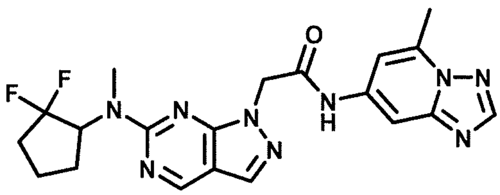 | $^1$H-NMR (400 MHz, $CDCl_3$) δ: 8.77 (br s, 2H), 8.21 (s, 1H), 7.97 (s, 1H), 7.83 (d, J = 2.3 Hz, 1H), 6.93 (d, J = 0.9 Hz, 1H), 5.08 (br s, 2H), 4.04 (br s, 1H), 3.13 (s, 3H), 2.68 (s, 3H), 2.24-1.97 (m, 4H), 1.92-1.74 (m, 2H). |
| 840 | 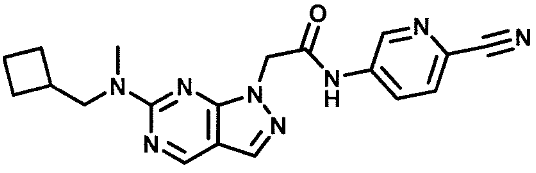 | LC-MS: $[M+H]^+$ / Rt (min) 377.2 / 0.938 (Method A) |

(continued)

| Example | Chemical structure | Analytical data |
|---|---|---|
| 841 | 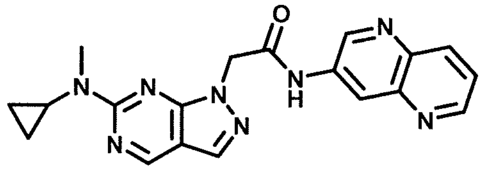 | LC-MS: $[M+H]^+$ / Rt (min) 375.1 / 0.588 (Method A) |
| 842 | 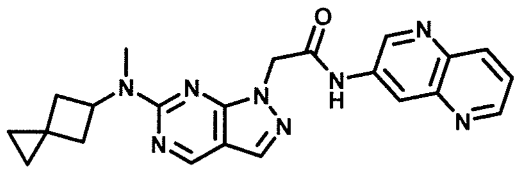 | $^1$H-NMR (400 MHz, $CDCl_3$) δ: 9.05 (s, 1H), 8.97-8.92 (m, 1H), 8.91 (d, J = 2.4 Hz, 1H), 8.84 (s, 1H), 8.77-8.75 (m, 1H), 8.35-8.31 (m, 1H), 8.04 (s, 1H), 7.56 (dd, J = 8.6, 4.3 Hz, 1H), 5.65-5.54 (m, 1H), 5.23 (s, 2H), 3.29 (s, 3H), 2.58-2.53 (m, 2H), 2.29-2.24 (m, 2H), 0.88 (t, J = 6.7 Hz, 2H), 0.60-0.54 (m, 2H), 0.48-0.42 (m, 2H). |
| 843 | 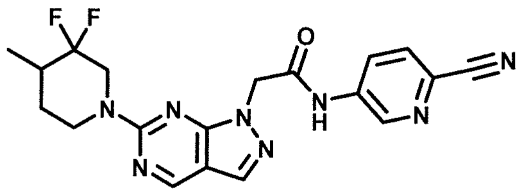 | $^1$H-NMR (400 MHz, $CDCl_3$) δ: 8.98 (s, 1H), 8.84 (s, 1H), 8.55 (d, J = 2.4 Hz, 1H), 8.36 (dd, J = 8.5, 2.4 Hz, 1H), 8.04 (s, 1H), 7.66 (d, J = 8.5 Hz, 1H), 5.16-5.13 (m, 3H), 4.86 (d, J = 13.4 Hz, 1H), 3.26 (dd, J = 29.0, 13.7 Hz, 1H), 3.15-3.08 (m, 1H), 2.13-2.09 (m, 1H), 1.85-1.83 (m, 1H), 1.62-1.55 (m, 1H), 1.12 (d, J = 6.7 Hz, 3H). |
| 844 | 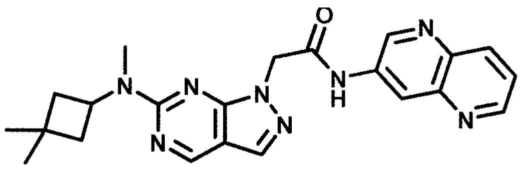 | LC-MS: $[M+H]^+$ / Rt (min) 417.1 / 0.869 (Method A) |
| 845 | 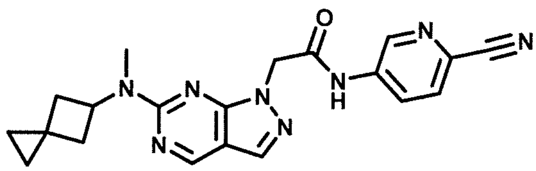 | LC-MS: $[M+H]^+$ / Rt (min) 389.2 / 0.939 (Method A) |
| 846 | 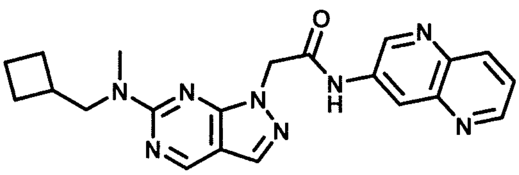 | LC-MS: $[M+H]^+$ / Rt (min) 403.1 / 0.798 (Method A) |
| 847 | 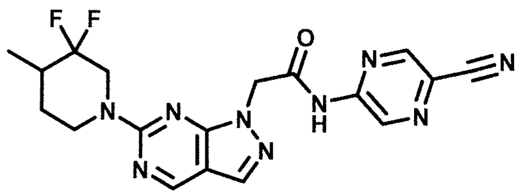 | $^1$H-NMR (400 MHz, DMSO-$d_6$) δ: 11.82 (s, 1H), 9.32 (d, J = 1.2 Hz, 1H), 9.00 (d, J = 1.2 Hz, 1H), 8.93 (s, 1H), 8.08 (s, 1H), 5.29 (s, 2H), 4.94 (s, 1H), 4.67 (d, J = 12.8 Hz, 1H), 3.41 (dd, J = 25.6, 11.6 Hz, 1H), 3.12 (dd, J = 11.3, 5.6 Hz, 1H), 2.20-2.17 (m, 1H), 1.79 (d, J = 13.4 Hz, 1H), 1.36 (dd, J = 21.4, 12.2 Hz, 1H), 0.99 (d, J = 6.7 Hz, 3H). |

(continued)

| Example | Chemical structure | Analytical data |
|---|---|---|
| 848 | 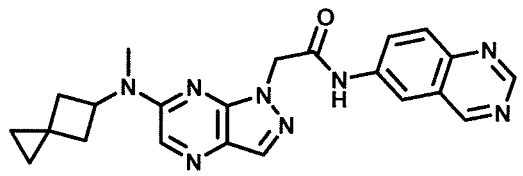 | LC-MS: [M+H]+ / Rt (min) 415.2 / 0.861 (Method A) |
| 849 | 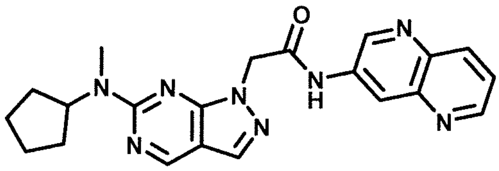 | LC-MS: [M+H]+ / Rt (min) 403.4 / 0.787 (Method A) |
| 850 | 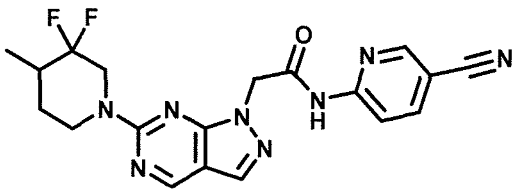 | $^1$H-NMR (400 MHz, DMSO-$d_6$) $\delta$: 11.45 (s, 1H), 8.92 (s, 1H), 8.83 (dd, J = 2.3, 0.9 Hz, 1H), 8.25 (dd, J = 8.7, 2.3 Hz, 1H), 8.10 (d, J = 8.7 Hz, 1H), 8.07 (s, 1H), 5.25 (s, 2H), 4.98-4.91 (m, 1H), 4.70-4.63 (m, 1H), 3.43 (dd, J = 10.5, 5.3 Hz, 1H), 3.36-3.28 (m, 1H), 3.12 (s, 1H), 1.81-1.74 (m, 1H), 1.39-1.35 (m, 1H), 0.99 (d, J = 6.9 Hz, 3H). |
| 851 | 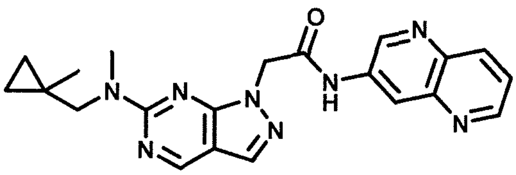 | $^1$H-NMR (400 MHz, DMSO-$d_6$) $\delta$: 11.07 (s, 1H), 9.07 (d, J = 2.4 Hz, 1H), 8.94 (dd, J = 4.3, 1.2 Hz, 1H), 8.90 (s, 1H), 8.72 (d, J = 2.4 Hz, 1H), 8.39-8.35 (m, 1H), 8.04 (s, 1H), 7.68 (dd, J = 8.6, 4.3 Hz, 1H), 5.21 (s, 2H), 3.66 (s, 2H), 3.19 (s, 3H), 0.89 (d, J = 7.9 Hz, 3H), 0.45 (s, 2H), 0.22 (s, 2H). |

Examples 852 - 863

According to the methods of Reference examples 42 - 44 or Examples 572 - 576, and common reaction conditions, the compounds of Examples 852 - 863 were obtained by using each corresponding material compound.

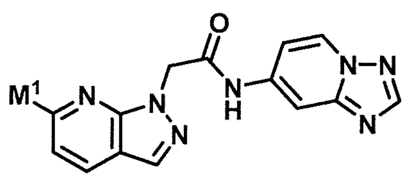

| Example | $M^1$ | Analytical data |
|---|---|---|
| 852 | 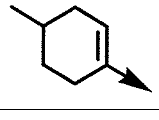 | LC-MS: [M+H]+ / Rt (min) 388.0 / 0.968 (Method A) |
| 853 | 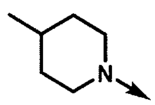 | $^1$H-NMR (400 MHz, $CDCl_3$) $\delta$: 9.24 (s, 1H), 8.45 (d, J = 7.3 Hz, 1H), 8.26 (s, 1H), 7.98 (d, J = 1.8 Hz, 1H), 7.91 (s, 1H), 7.78 (d, J = 9.2 Hz, 1H), 7.19 (dd, J = 7.3, 1.8 Hz, 1H), 6.67 (d, J = 9.2 Hz, 1H), 5.20 (s, 2H), 4.48 (d, J = 12.8 Hz, 2H), 2.96 (td, J = 12.8, 2.2 Hz, 2H), 1.78-1.65 (m, 3H), 1.31-1.16 (m, 2H), 0.97 (d, J = 6.1 Hz, 3H). |

(continued)

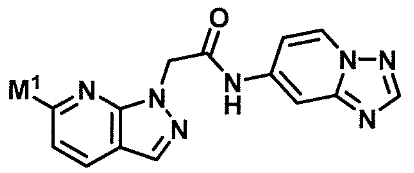

| Example | $M^1$ | Analytical data |
|---|---|---|
| 854 | 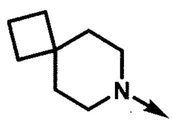 | $^1$H-NMR (400 MHz, $CDCl_3$) δ: 9.30 (s, 1H), 8.45 (d, J = 7.3 Hz, 1H), 8.26 (s, 1H), 8.00 (d, J = 2.4 Hz, 1H), 7.91 (s, 1H), 7.77 (d, J = 9.1 Hz, 1H), 7.20 (dd, J = 7.6, 2.1 Hz, 1H), 6.67 (d, J = 9.1 Hz, 1H), 5.21 (s, 2H), 3.67-3.60 (m, 4H), 1.99-1.89 (m, 2H), 1.89-1.78 (m, 6H), 1.70-1.63 (m, 2H) . |
| 855 | 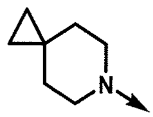 | LC-MS: $[M+H]^+$ / Rt (min) 403.1 / 0.853 (Method A) |
| 856 | 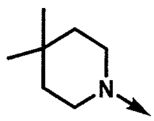 | LC-MS: $[M+H]^+$ / Rt (min) 405.1 / 0.892 (Method A) |
| 857 | 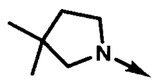 | LC-MS: $[M+H]^+$ / Rt (min) 391.0 / 0.735 (Method A) |
| 858 | 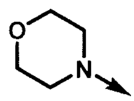 | LC-MS: $[M+H]^+$ / Rt (min) 395.0 / 0.699 (Method A) |
| 859 | 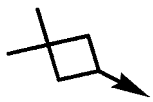 | LC-MS: $[M+H]^+$ / Rt (min) 377.0 / 0.764 (Method A) |
| 860 | 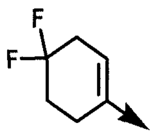 | LC-MS: $[M+H]^+$ / Rt (min) 410.0 / 0.780 (Method A) |
| 861 | 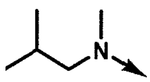 | $^1$H-NMR (400 MHz, $CDCl_3$) δ: 9.21 (s, 1H), 8.46 (d, J = 7.3 Hz, 1H), 8.27 (s, 1H), 8.00-7.98 (m, 1H), 7.92 (s, 1H), 7.78 (d, J = 9.2 Hz, 1H), 7.20 (dd, J = 7.3, 1.8 Hz, 1H), 6.55 (d, J = 8.5 Hz, 1H), 5.21 (s, 2H), 3.43 (d, J = 7.9 Hz, 2H), 3.20 (s, 3H), 2.02 (s, 1H), 0.96 (d, J = 7.6 Hz, 6H). |
| 862 | 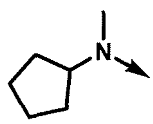 | $^1$H-NMR (400 MHz, $CDCl_3$) δ: 9.30 (s, 1H), 8.42 (d, J = 7.3 Hz, 1H), 8.24 (s, 1H), 7.99 (d, J = 2.4 Hz, 1H), 7.89 (s, 1H), 7.77-7.74 (m, 1H), 7.17 (dd, J = 7.3, 2.4 Hz, 1H), 6.56 (d, J = 8.6 Hz, 1H), 5.20 (s, 2H), 5.06-4.98 (m, 1H), 2.98 (s, 3H), 1.98-1.93 (m, 2H), 1.80-1.73 (m, 2H), 1.72-1.55 (m, 4H). |
| 863 | 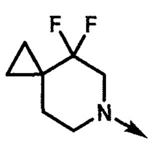 | $^1$H-NMR (400 MHz, $CDCl_3$) δ: 9.11 (s, 1H), 8.44 (d, J = 7.3 Hz, 1H), 8.26 (s, 1H), 8.01 (d, J = 1.8 Hz, 1H), 7.95 (s, 1H), 7.85 (d, J = 9.2 Hz, 1H), 7.18 (dd, J = 7.3, 2.4 Hz, 1H), 6.71 (d, J = 9.2 Hz, 1H), 5.22 (s, 2H), 4.02 (dd, J = 5.5, 5.5 Hz, 2H), 3.90-3.84 (m, 2H), 1.76-1.68 (m, 2H), 0.91-0.98 (m, 2H), 0.53-0.49 (m, 2H). |

Examples 864 - 870

According to the methods of Reference examples 42 - 44 or Examples 572 - 576, and common reaction conditions, the compounds of Examples 864 - 870 were obtained by using each corresponding material compound.

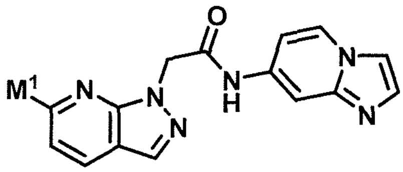

| Example | $M^1$ | Analytical data |
|---|---|---|
| 864 | 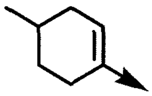 | $^1$H-NMR (400 MHz, $CDCl_3$) δ: 8.92 (br s, 1H), 8.09 (s, 1H), 8.01 (d, J = 8.5 Hz, 1H), 7.98 (d, J = 7.3 Hz, 1H), 7.65-7.62 (m, 1H), 7.56-7.53 (m, 1H), 7.46 (s, 1H), 7.41-7.37 (m, 1H), 7.09 (dd, J = 7.6, 2.1 Hz, 1H), 6.87-6.82 (m, 1H), 5.34 (s, 2H), 2.85-2.76 (m, 1H), 2.58-2.50 (m, 1H), 2.45-2.35 (m, 1H), 1.96-1.84 (m, 2H), 1.82-1.73 (m, 1H), 1.45-1.32 (m, 1H), 1.03 (d, J = 6.1 Hz, 3H). |
| 865 | 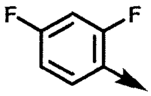 | LC-MS: $[M+H]^+$ / Rt (min) 405.3 / 0.859 (Method A) |
| 866 | 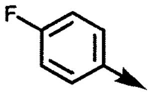 | LC-MS: $[M+H]^+$ / Rt (min) 387.3 / 0.782 (Method A) |
| 867 | 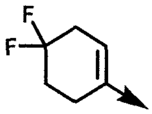 | LC-MS: $[M+H]^+$ / Rt (min) 409.3 / 0.827 (Method A) |
| 868 | 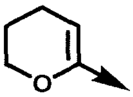 | LC-MS: $[M+H]^+$ / Rt (min) 375.3 / 0.707 (Method A) |
| 869 | 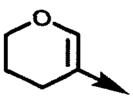 | LC-MS: $[M+H]^+$ / Rt (min) 375.3 / 0.686 (Method A) |
| 870 | 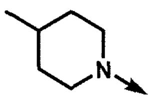 | $^1$H-NMR (400 MHz, DMSO-$d_6$) δ: 10.58 (s, 1H), 8.45 (d, J = 7.3 Hz, 1H), 7.88-7.83 (m, 4H), 7.44 (s, 1H), 6.98 (dd, J = 7.3, 1.8 Hz, 1H), 6.79 (d, J = 9.1 Hz, 1H), 5.12 (s, 2H), 4.44-4.36 (m, 2H), 2.89-2.79 (m, 2H), 1.66-1.50 (m, 3H), 1.13-0.99 (m, 2H), 0.86 (d, J = 6.1 Hz, 3H). |

Examples 871 - 875

According to the methods of Reference examples 42 - 44 or Examples 572 - 576, and common reaction conditions, the compounds of Examples 871 - 875 were obtained by using each corresponding material compound.

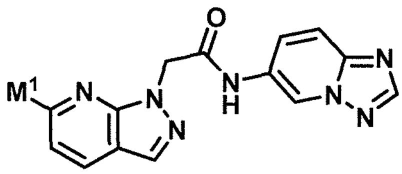

| Example | $M^1$ | Analytical data |
|---|---|---|
| 871 | 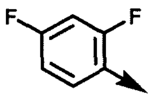 | $^1$H-NMR (400 MHz, DMSO-$d_6$) δ: 10.90 (s, 1H), 9.37-9.33 (m, 1H), 8.42 (s, 1H), 8.40 (d, J = 8.6 Hz, 1H), 8.29 (s, 1H), 8.08-8.00 (m, 1H), 7.86 (d, J = 8.6 Hz, 1H), 7.67-7.62 (m, 2H), 7.46-7.39 (m, 1H), 7.28-7.21 (m, 1H), 5.46 (s, 2H). |
| 872 | 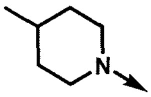 | $^1$H-NMR (400 MHz, $CDCl_3$) δ: 9.58-9.47 (m, 2H), 8.32 (s, 1H), 7.90 (s, 1H), 7.80 (d, J = 9.1 Hz, 1H), 7.68 (d, J = 9.8 Hz, 1H), 7.39-7.34 (m, 1H), 6.68 (d, J = 9.1 Hz, 1H), 5.33 (s, 2H), 4.51-4.41 (m, 2H), 3.08-2.96 (m, 2H), 1.83-1.62 (m, 3H), 1.31-1.16 (m, 2H), 0.96 (d, J = 6.7 Hz, 3H). |
| 873 | 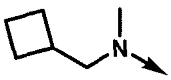 | LC-MS: $[M+H]^+$ / Rt (min) 391.3 / 0.843 (Method A) |
| 874 | 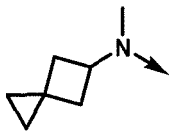 | LC-MS: $[M+H]^+$ / Rt (min) 404.3 / 0.869 (Method A) |
| 875 | 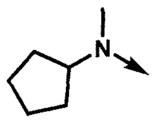 | $^1$H-NMR (400 MHz, $CDCl_3$) δ: 9.47 (s, 1H), 9.04 (s, 1H), 8.30 (s, 1H), 7.90 (s, 1H), 7.76 (d, J = 9.2 Hz, 1H), 7.64 (d, J = 9.2 Hz, 1H), 7.24-7.21 (m, 1H), 6.56 (t, J = 8.6 Hz, 1H), 5.21 (s, 2H), 5.08-4.97 (m, 1H), 3.00 (s, 3H), 1.98-1.88 (m, 2H), 1.81-1.72 (m, 2H), 1.71-1.56 (m, 4H). |

Examples 876 - 880

According to the methods of Reference examples 42 - 44 or Examples 572 - 576, and common reaction conditions, the compounds of Examples 876 - 880 were obtained by using each corresponding material compound.

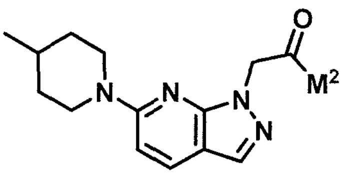

| Example | $M^2$ | Analytical data |
|---|---|---|
| 876 | 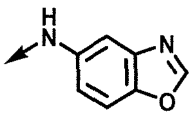 | $^1$H-NMR (400 MHz, $CDCl_3$) δ: 8.95 (s, 1H), 8.04 (s, 1H), 8.01-7.99 (m, 1H), 7.90 (s, 1H), 7.79 (d, J = 8.5 Hz, 1H), 7.45 (s, 2H), 6.68 (d, J = 9.1 Hz, 1H), 5.28 (s, 2H), 4.49-4.40 (m, 2H), 3.05-2.94 (m, 2H), 1.81-1.59 (m, 3H), 1.32-1.17 (m, 2H), 0.95 (d, J = 6.1 Hz, 3H) . |
| 877 | 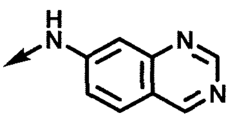 | $^1$H-NMR (400 MHz, $CDCl_3$) δ: 9.37-9.16 (m, 3H), 8.16-8.09 (m, 1H), 7.95-7.89 (m, 1H), 7.89-7.82 (m, 2H), 7.78 (dd, J = 9.1, 1.2 Hz, 1H), 6.71-6.62 (m, 1H), 5.25-5.21 (m, 2H), 4.53-4.43 (m, 2H), 3.02-2.87 (m, 2H), 1.83-1.62 (m, 3H), 1.31-1.15 (m, 2H), 0.99-0.94 (m, 3H). |

(continued)

| 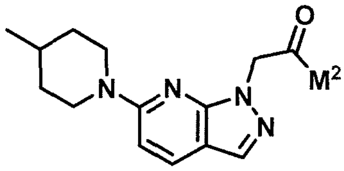 | | |
|---|---|---|
| Example | $M^2$ | Analytical data |
| 878 | 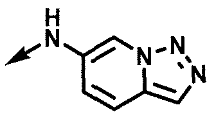 | LC-MS: $[M+H]^+$ / Rt (min) 391.0 / 0.874 (Method A) |
| 879 | 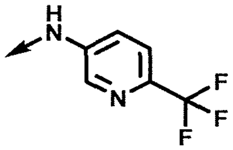 | LC-MS: $[M+H]^+$ / Rt (min) 419.1 / 1.066 (Method A) |
| 880 | 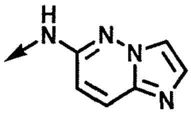 | LC-MS: $[M+H]^+$ / Rt (min) 391.0 / 0.807 (Method A) |

Examples 881 - 894

According to the methods of Reference examples 42 - 45 or Examples 572 - 576, and common reaction conditions, the compounds of Examples 881 - 894 were obtained by using each corresponding material compound.

| Example | Chemical Structure | Analytical data |
|---|---|---|
| 881 | 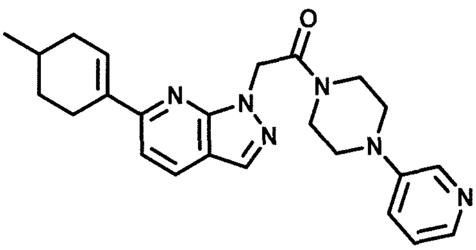 | $^1$H-NMR (400 MHz, DMSO-$d_6$) $\delta$: 8.34 (d, J = 2.4 Hz, 1H), 8.13 (d, J = 8.5 Hz, 1H), 8.08 (s, 1H), 8.03 (dd, J = 4.9, 1.2 Hz, 1H), 7.43 (d, J = 8.5 Hz, 1H), 7.39-7.34 (m, 1H), 7.24 (dd, J = 8.5, 4.9 Hz, 1H), 6.79-6.75 (m, 1H), 5.44 (s, 2H), 3.83-3.76 (m, 2H), 3.65-3.56 (m, 2H), 3.36-3.26 (m, 2H), 3.24-3.16 (m, 2H), 2.77-2.66 (m, 1H), 2.48-2.28 (m, 2H), 1.89-1.73 (m, 2H), 1.73-1.62 (m, 1H), 1.32-1.20 (m, 1H), 0.97 (d, J = 6.7 Hz, 3H). |
| 882 | 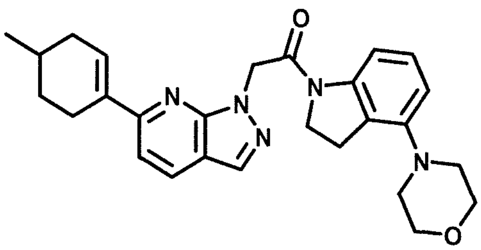 | $^1$H-NMR (400 MHz, DMSO-$d_6$) $\delta$: 8.16 (d, J = 7.9 Hz, 1H), 8.12 (s, 1H), 7.62 (d, J = 7.9 Hz, 1H), 7.44 (d, J = 8.5 Hz, 1H), 7.09 (t, J = 8.2 Hz, 1H), 6.82-6.77 (m, 1H), 6.67 (d, J = 7.9 Hz, 1H), 5.48 (s, 2H), 4.32 (t, J = 7.9 Hz, 2H), 3.76-3.68 (m, 4H), 3.15 (t, J = 8.2 Hz, 2H), 2.98-2.88 (m, 4H), 2.79-2.68 (m, 1H), 2.47-2.27 (m, 2H), 1.91-1.76 (m, 2H), 1.76-1.63 (m, 1H), 1.35-1.22 (m, 1H), 0.97 (d, J = 6.7 Hz, 3H). |

(continued)

| Example | Chemical Structure | Analytical data |
|---|---|---|
| 883 | 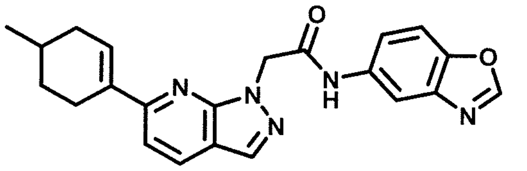 | $^1$H-NMR (400 MHz, DMSO-$d_6$) δ: 10.61 (s, 1H), 8.70 (s, 1H), 8.16 (d, J = 8.5 Hz, 1H), 8.12 (s, 1H), 8.10 (d, J = 1.8 Hz, 1H), 7.72 (d, J = 9.1 Hz, 1H), 7.53 (dd, J = 8.8, 2.1 Hz, 1H), 7.46 (d, J = 8.5 Hz, 1H), 6.84-6.78 (m, 1H), 5.32 (s, 2H), 2.79-2.70 (m, 1H), 2.47-2.28 (m, 2H), 1.91-1.76 (m, 2H), 1.76-1.63 (m, 1H), 1.34-1.21 (m, 1H), 0.97 (d, J = 6.1 Hz, 3H). |
| 884 | 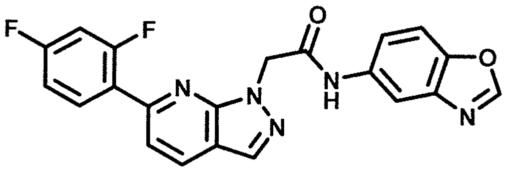 | $^1$H-NMR (400 MHz, DMSO-$d_6$) δ: 10.64 (br s, 1H), 8.70 (s, 1H), 8.39 (d, J = 7.9 Hz, 1H), 8.28 (s, 1H), 8.10 (d, J = 1.8 Hz, 1H), 8.08-8.00 (m, 1H), 7.72 (d, J = 8.6 Hz, 1H), 7.64 (dd, J = 8.3, 2.1 Hz, 1H), 7.53 (dd, J = 8.6, 1.8 Hz, 1H), 7.46-7.39 (m, 1H), 7.28-7.21 (m, 1H), 5.42 (s, 2H). |
| 885 | 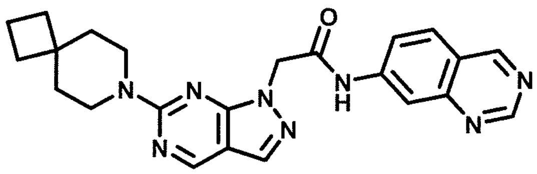 | $^1$H-NMR (400 MHz, $CDCl_3$) δ: 9.28-9.18 (m, 3H), 8.13 (s, 1H), 7.91 (s, 1H), 7.87-7.84 (m, 2H), 7.77 (d, J = 9.1 Hz, 1H), 6.66 (d, J = 9.1 Hz, 1H), 5.22 (s, 2H), 3.67-3.61 (m, 4H), 1.98-1.89 (m, 2H), 1.86-1.78 (m, 4H), 1.68-1.62 (m, 4H). |
| 886 | 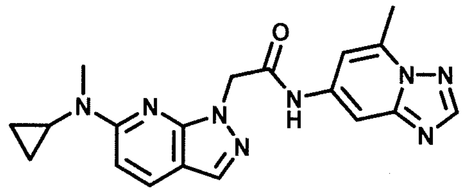 | $^1$H-NMR (400 MHz, $CDCl_3$) δ: 9.07 (s, 1H), 8.27 (s, 1H), 7.95 (s, 1H), 7.83 (s, 1H), 7.81-7.79 (m, 1H), 7.07-7.03 (m, 1H), 7.02 (m, 1H), 5.21 (s, 2H), 3.24 (s, 3H), 2.74 (s, 3H), 2.70-2.65 (m, 1H), 0.99-0.94 (m, 2H), 0.78-0.72 (m, 2H). |
| 887 | 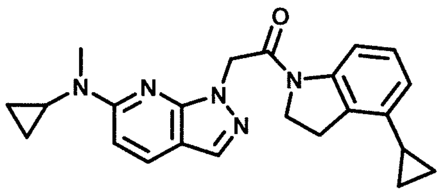 | $^1$H-NMR (400 MHz, $CDCl_3$) δ: 8.02 (d, J = 8.2 Hz, 1H), 7.86 (s, 1H), 7.77 (d, J = 9.1 Hz, 1H), 7.08 (t, J = 8.0 Hz, 1H), 6.96 (d, J = 8.7 Hz, 1H), 6.58 (d, J = 7.8 Hz, 1H), 5.27 (s, 2H), 4.19 (t, J = 8.5 Hz, 2H), 3.30 (t, J = 8.5 Hz, 2H), 3.17 (s, 3H), 2.63-2.57 (m, 1H), 1.81-1.77 (m, 1H), 0.98-0.85 (m, 4H), 0.73-0.65 (m, 4H). |
| 888 | 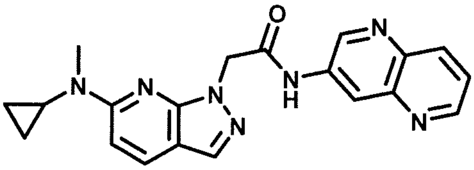 | $^1$H-NMR (400 MHz, $CDCl_3$) δ: 9.63 (s, 1H), 9.19-9.16 (m, 1H), 9.07 (d, J = 3.1 Hz, 1H), 8.86 (s, 1H), 8.59 (d, J = 8.5 Hz, 1H), 7.98 (s, 1H), 7.83 (d, J = 9.2 Hz, 1H), 7.72 (dd, J = 8.2, 4.6 Hz, 1H), 7.06 (d, J = 9.2 Hz, 1H), 5.31 (s, 2H), 3.26 (s, 3H), 2.70-2.65 (m, 1H), 1.00-0.95 (m, 2H), 0.79-0.74 (m, 2H). |

(continued)

| Example | Chemical Structure | Analytical data |
|---|---|---|
| 889 | 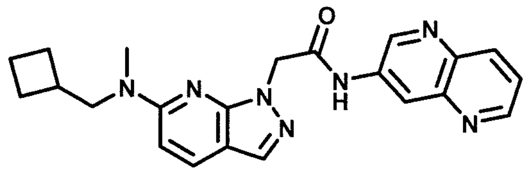 | LC-MS: $[M+H]^+$ / Rt (min) 402.2 / 0.864 (Method A) |
| 890 | 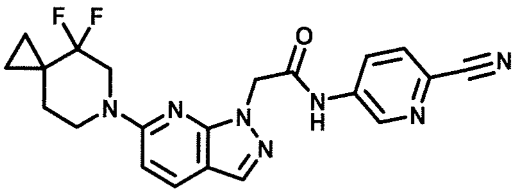 | $^1$H-NMR (400 MHz, $CDCl_3$) δ: 9.18 (s, 1H), 8.53 (d, J = 2.4 Hz, 1H), 8.36 (dd, J = 8.5, 2.4 Hz, 1H), 7.96 (s, 1H), 7.86 (d, J = 9.2 Hz, 1H), 7.65 (d, J = 8.5 Hz, 1H), 6.73 (d, J = 8.5 Hz, 1H), 5.21 (s, 2H), 4.03 (dd, J = 5.6 Hz, 5.6, 2H), 3.88-3.84 (m, 2H), 1.78-1.71 (m, 2H), 1.02-0.98 (m, 2H), 0.53-0.49 (m, 2H). |
| 891 | 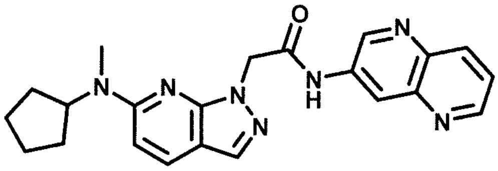 | $^1$H-NMR (400 MHz, $CDCl_3$) δ: 9.29 (s, 1H), 8.92 (dd, J = 4.0, 1.5 Hz, 1H), 8.89 (d, J = 4.3 Hz, 1H), 8.74 (d, J = 1.8 Hz, 1H), 8.30 (dd, J = 8.6, 1.8 Hz, 1H), 7.91 (s, 1H), 7.75 (d, J = 7.9, 1H), 7.52 (dd, J = 9.0, 4.9 Hz, 1H), 6.56 (d, J = 9.2 Hz, 1H), 5.25 (s, 2H), 5.08-4.97 (m, 1H), 2.98 (s, 3H), 1.97-1.87 (m, 2H), 1.79-1.72 (m, 2H), 1.71-1.55 (m, 4H), 1.26 (tt, J = 12.5, 4.4 Hz, 3H), 0.88 (q, J = 7.3 Hz, 1H). |
| 892 | 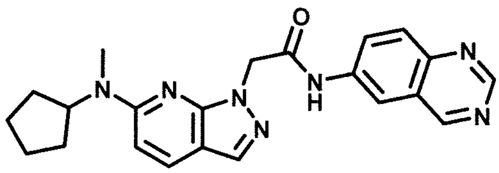 | LC-MS: $[M+H]^+$ / Rt (min) 402.3 / 0.899 (Method A) |
| 893 | 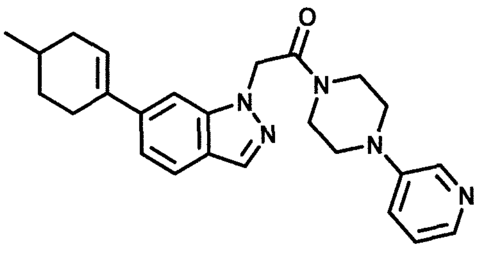 | $^1$H-NMR (400 MHz, DMSO-$d_6$) δ: 8.34 (d, J = 3.0 Hz, 1H), 8.05-8.01 (m, 1H), 7.99 (s, 1H), 7.64 (d, J = 8.5 Hz, 1H), 7.53 (s, 1H), 7.39-7.34 (m, 1H), 7.26-7.21 (m, 2H), 6.22-6.17 (m, 1H), 5.49 (s, 2H), 3.80-3.70 (m, 2H), 3.63-3.56 (m, 2H), 3.37-3.28 (m, 2H), 3.23-3.16 (m, 2H), 2.54-2.41 (m, 2H), 2.34-2.25 (m, 1H), 1.88-1.75 (m, 2H), 1.74-1.64 (m, 1H), 1.40-1.27 (m, 1H), 0.98 (d, J = 6.1 Hz, 3H). |
| 894 | 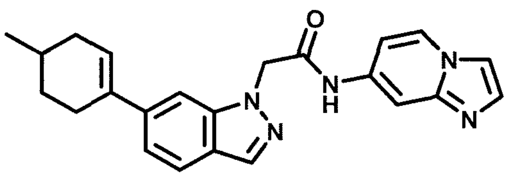 | $^1$H-NMR (400 MHz, DMSO-$d_6$) δ: 10.64 (s, 1H), 8.46 (d, J = 7.3 Hz, 1H), 8.03 (s, 1H), 7.91-7.88 (m, 1H), 7.82 (s, 1H), 7.69-7.64 (m, 2H), 7.44 (d, J = 1.2 Hz, 1H), 7.29-7.25 (m, 1H), 6.99 (dd, J = 7.3, 2.4 Hz, 1H), 6.26-6.20 (m, 1H), 5.37 (s, 2H), 2.54-2.42 (m, 2H), 2.36-2.25 (m, 1H), 1.88-1.76 (m, 2H), 1.76-1.64 (m, 1H), 1.40-1.28 (m, 1H), 0.98 (d, J = 6.1 Hz, 3H). |

Examples 895 and 896

The compound obtained in Example 577 (4.6 mg) was resolved by chiral column chromatography to obtain the following optically-active compounds.

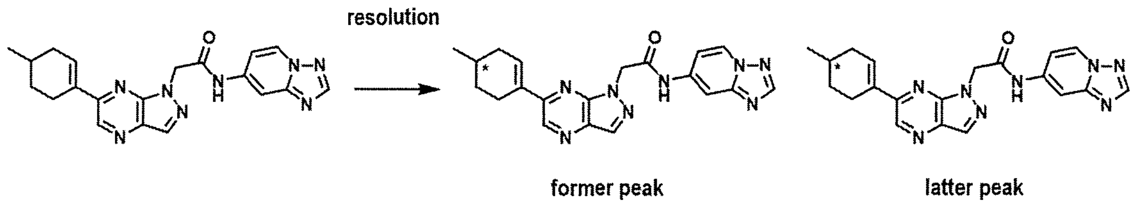


[Resolution conditions]

Detection apparatus: SPD-M20A (Shimadzu Corporation)
HPLC: LC-20AT (Shimadzu Corporation)
Column: CHIRALPAK IG (DAICEL) (20 × 250 mm)
Elution condition: 0.0 - 30.0 (min) : A/B = 40:60
Solvent A: hexane
Solvent B: isopropylalcohol
Flow rate: 1.0 ml/min
UV: 220 nm
Column temperature: 40°C

| | Example | Retention time (min) | Yield (mg) | Optical purity |
|---|---|---|---|---|
| Former peak | 895 | 20.244 | 0.8 | >95%ee |
| Latter peak | 896 | 22.450 | 0.8 | >95%ee |

Tests

Hereinafter, the results of pharmacological tests for representative compounds herein are described, and the pharmacologic effects of each compound are explained, but the present invention is not limited to the following Tests.

Test 1: Evaluation of the amplification of Nav1.1-derived voltage-gated sodium current (Nav1.1 current) by using a cell line stably expressing human Nav1.1

(1) Preparation of test compound solution

Test compounds were prepared by dissolving in DMSO at 200 times of the concentration at evaluation, and diluting the obtained solution to twice the concentration of evaluation with an extracellular fluid (135 mmol/L NaCl, 4 mmol/L KCl, 1 mmol/L $MgCl_2$, 5 mmol/L $CaCl_2$, 5 mmol/L Glucose, 10 mmol/L HEPES).

(2) Induction and measurement of Nav1.1 current

A HEK293 cell line stably expressing human Nav1.1 (cat#CYL3009, Eurofins DiscoverX Products, LLC, Human Embryonic Kidney 293) was purchased, and used in the present test. Nav1.1 current was induced by stimulating Ramp wave voltage. Detection of current which accompanied with the stimulation of voltage was carried out by a patch-clamp voltage-clamp method using HTS automated patch clamp system (SynchroPatch 768PE, Nanion Technologies GmbH, Germany). Only cells with more than 500 pA of voltage-gated sodium channel current were used for the evaluation of the activity of compounds on Nav1.1 current because there was the possibility that currents derived from endogenous voltage-gated sodium channels accounted for large proportion in cells with less than 500 pA of voltage-gated sodium channel current which was induced by stimulating Ramp wave voltage.

(3) Pharmacological effect on Nav1.1 current

The effect of test compounds on the amplification of Nav1.1-derived voltage-gated sodium current was evaluated by using a cell line stably expressing human Nav1.1 and HTS automated patch clamp system. In other words, test compounds were added in an extracellular fluid containing 1% DMSO, and evaluated as a change of the peak value of Nav1.1 current and the area under the curve (AUC).

(4) Method for pharmacological evaluation

The Nav1.1 current amplification rates of test compounds were calculated by the following formula:

Nav1.1 current amplification rate (%) = 100 x [the peak value of Nav1.1 current or the area under the curve after the addition of test compounds] / [the peak value of Nav1.1 current or the area under the curve before the addition of test compounds] - 100

Test result

The Nav1.1 current amplification rates (%) of the representative compounds of the present invention are shown in the following table.

| Example | Concentration for evaluation (μM) | Nav1.1 current amplification rate (%) | Example | Concentration for evaluation (μM) | Nav1.1 current amplification rate (%) |
|---|---|---|---|---|---|
| 1 | 10 | 246 | 36 | 10 | 312 |
| 2 | 10 | 148 | 37 | 10 | 93 |
| 3 | 10 | 40 | 38 | 10 | 174 |
| 4 | 10 | 3 | 39 | 10 | 125 |
| 5 | 10 | 6 | 40 | 10 | 20 |
| 6 | 10 | 35 | 41 | 10 | 324 |
| 7 | 10 | 3 | 42 | 10 | 216 |
| 8 | 10 | 4 | 43 | 10 | 174 |
| 9 | 10 | 11 | 44 | 10 | 59 |
| 10 | 10 | 124 | 45 | 10 | 202 |
| 11 | 10 | 205 | 46 | 10 | 85 |
| 12 | 10 | 241 | 47 | 10 | 11 |
| 13 | 10 | 76 | 48 | 10 | 33 |
| 14 | 10 | 9 | 49 | 10 | 2 |
| 15 | 10 | 10 | 50 | 10 | 155 |
| 16 | 10 | 59 | 51 | 10 | 196 |
| 17 | 10 | 212 | 52 | 10 | 248 |
| 18 | 10 | 11 | 53 | 10 | 161 |
| 19 | 10 | 98 | 54 | 10 | 20 |
| 20 | 10 | 152 | 55 | 10 | 111 |
| 21 | 10 | 130 | 56 | 10 | 10 |
| 22 | 10 | 110 | 57 | 10 | 48 |
| 23 | 10 | 28 | 58 | 10 | 26 |
| 24 | 10 | 286 | 59 | 10 | 180 |
| 25 | 10 | 163 | 60 | 10 | 57 |
| 26 | 10 | 364 | 61 | 10 | 77 |
| 27 | 10 | 5 | 62 | 10 | 13 |
| 28 | 10 | 158 | 63 | 10 | 49 |
| 29 | 10 | 20 | 64 | 10 | 11 |
| 30 | 10 | 77 | 65 | 10 | 17 |
| 31 | 10 | 220 | 66 | 10 | 49 |
| 32 | 10 | 60 | 67 | 10 | 177 |
| 33 | 10 | 220 | 68 | 10 | 42 |
| 34 | 10 | 311 | 69 | 10 | 137 |
| 35 | 10 | 301 | 70 | 10 | 68 |

| Example | Concentration for evaluation (μM) | Nav1.1 current amplification rate (%) | Example | Concentration for evaluation (μM) | Nav1.1 current amplification rate (%) |
|---|---|---|---|---|---|
| 71 | 10 | 131 | 106 | - | - |
| 72 | 10 | 119 | 107 | 10 | 37 |
| 73 | 10 | 92 | 108 | 10 | 5 |
| 74 | 10 | 70 | 109 | 10 | 57 |
| 75 | 10 | 15 | 110 | 10 | 12 |
| 76 | - | - | 111 | - | - |
| 77 | 10 | 93 | 112 | 10 | 46 |

| | | | | | |
|---|---|---|---|---|---|
| 78 | 10 | 78 | 113 | 10 | 30 |
| 79 | 10 | 165 | 114 | 10 | 83 |
| 80 | 10 | 131 | 115 | 10 | 56 |
| 81 | 10 | 69 | 116 | 10 | 12 |
| 82 | 10 | 121 | 117 | - | - |
| 83 | 10 | 45 | 118 | 10 | 7 |
| 84 | 10 | 315 | 119 | 10 | 120 |
| 85 | 10 | 15 | 120 | 10 | 176 |
| 86 | 10 | 73 | 121 | 10 | 184 |
| 87 | 10 | 255 | 122 | 10 | 173 |
| 88 | 10 | 183 | 123 | 10 | 246 |
| 89 | 10 | 187 | 124 | 10 | 205 |
| 90 | 10 | 31 | 125 | 10 | 220 |
| 91 | 10 | 93 | 126 | 10 | 167 |
| 92 | 10 | 88 | 127 | 10 | 229 |
| 93 | 10 | 65 | 128 | 10 | 240 |
| 94 | 10 | 198 | 129 | 10 | 314 |
| 95 | 10 | 221 | 130 | 10 | 181 |
| 96 | 10 | 105 | 131 | 10 | 227 |
| 97 | 10 | 296 | 132 | 10 | 108 |
| 98 | 10 | 15 | 133 | 10 | 285 |
| 99 | 10 | 103 | 134 | 10 | 44 |
| 100 | 10 | 197 | 135 | 10 | 195 |
| 101 | - | - | 136 | 10 | 63 |
| 102 | 10 | 8 | 137 | 10 | 58 |
| 103 | 10 | 58 | 138 | 10 | 58 |
| 104 | 10 | 11 | 139 | 10 | 166 |
| 105 | - | - | 140 | 10 | 93 |

| Example | Concentration for evaluation (μM) | Nav1.1 current amplification rate (%) | Example | Concentration for evaluation (μM) | Nav1.1 current amplification rate (%) |
|---|---|---|---|---|---|
| 141 | 10 | 46 | 176 | 10 | 66 |
| 142 | 10 | 34 | 177 | 10 | 169 |
| 143 | 10 | 74 | 178 | 10 | 44 |
| 144 | 10 | 48 | 179 | 10 | 27 |
| 145 | 10 | 113 | 180 | 10 | 22 |
| 146 | 10 | 148 | 181 | 10 | 118 |
| 147 | 10 | 127 | 182 | 10 | 32 |
| 148 | 10 | 97 | 183 | 10 | 95 |
| 149 | 10 | 126 | 184 | 10 | 35 |
| 150 | 10 | 157 | 185 | 10 | 22 |
| 151 | 10 | 104 | 186 | 10 | 51 |
| 152 | 10 | 50 | 187 | 10 | 37 |
| 153 | 10 | 77 | 188 | 10 | 30 |
| 154 | 10 | 75 | 189 | 10 | 3 |
| 155 | 10 | 99 | 190 | 10 | 100 |
| 156 | 10 | 33 | 191 | 3 | 52 |
| 157 | 50 | 29 | 192 | 10 | 137 |

| | | | | | |
|---|---|---|---|---|---|
| 158 | 10 | 122 | 193 | 10 | 219 |
| 159 | 10 | 32 | 194 | 10 | 252 |
| 160 | 10 | 324 | 195 | 10 | 209 |
| 161 | 10 | 21 | 196 | 10 | 225 |
| 162 | 10 | 6 | 197 | 10 | 318 |
| 163 | 10 | 118 | 198 | 10 | 36 |
| 164 | 10 | 187 | 199 | 10 | 36 |
| 165 | 10 | 146 | 200 | 10 | 103 |
| 166 | 10 | 150 | 201 | 10 | 139 |
| 167 | 10 | 129 | 202 | 10 | 131 |
| 168 | 10 | 205 | 203 | 10 | 267 |
| 169 | 10 | 155 | 204 | 10 | 189 |
| 170 | 10 | 107 | 205 | 10 | 235 |
| 171 | 3 | 2 | 206 | 10 | 154 |
| 172 | 10 | 117 | 207 | 1 | 89 |
| 173 | 10 | 112 | 208 | 10 | 200 |
| 174 | 10 | 75 | 209 | 10 | 179 |
| 175 | 10 | 44 | 210 | 10 | 178 |

| Example | Concentration for evaluation (μM) | Nav1.1 current amplification rate (%) | Example | Concentration for evaluation (μM) | Nav1.1 current amplification rate (%) |
|---|---|---|---|---|---|
| 211 | 10 | 212 | 246 | 10 | 136 |
| 212 | 10 | 74 | 247 | 10 | 53 |
| 213 | 10 | 136 | 248 | 3 | 392 |
| 214 | 10 | 64 | 249 | 3 | 584 |
| 215 | 10 | 183 | 250 | 3 | 564 |
| 216 | 10 | 121 | 251 | 3 | 307 |
| 217 | 10 | 25 | 252 | 3 | 254 |
| 218 | 1 | 155 | 253 | 3 | 455 |
| 219 | 10 | 97 | 254 | 3 | 309 |
| 220 | 10 | 50 | 255 | 10 | 32 |
| 221 | 10 | 281 | 256 | 10 | 36 |
| 222 | 1 | 137 | 257 | 3 | 45 |
| 223 | 10 | 46 | 258 | 3 | 79 |
| 224 | 10 | 118 | 259 | 10 | 71 |
| 225 | 10 | 146 | 260 | 10 | 13 |
| 226 | 10 | 319 | 261 | 10 | 60 |
| 227 | 3 | 303 | 262 | 10 | 124 |
| 228 | 1 | 29 | 263 | 10 | 342 |
| 229 | 1 | 34 | 264 | 3 | 597 |
| 230 | 1 | 33 | 265 | 3 | 110 |
| 231 | 1 | 50 | 266 | 3 | 108 |
| 232 | 10 | 37 | 267 | 3 | 231 |
| 233 | 50 | 47 | 268 | 3 | 55 |
| 234 | 1 | 240 | 269 | 3 | 127 |
| 235 | 1 | 198 | 270 | 3 | 481 |
| 236 | 1 | 214 | 271 | 3 | 48 |
| 237 | 50 | 27 | 272 | 1 | 127 |

| | | | | | |
|---|---|---|---|---|---|
| 238 | 50 | 128 | 273 | 1 | 77 |
| 239 | 10 | 310 | 274 | 1 | 60 |
| 240 | 10 | 241 | 275 | 1 | 46 |
| 241 | 10 | 260 | 276 | 1 | 207 |
| 242 | 10 | 97 | 277 | 1 | 210 |
| 243 | 10 | 276 | 278 | 1 | 204 |
| 244 | 3 | 263 | 279 | 1 | 123 |
| 245 | 1 | 393 | 280 | 1 | 10 |

| Example | Concentration for evaluation (μM) | Nav1.1 current amplification rate (%) | Example | Concentration for evaluation (μM) | Nav1.1 current amplification rate (%) |
|---|---|---|---|---|---|
| 281 | 10 | 40 | 316 | 1 | 81 |
| 282 | 1 | 26 | 317 | 1 | 41 |
| 283 | 1 | 15 | 318 | 3 | 77 |
| 284 | 1 | 91 | 319 | 1 | 134 |
| 285 | 1 | 113 | 320 | 1 | 10 |
| 286 | 1 | 848 | 321 | 3 | 478 |
| 287 | 1 | 73 | 322 | 3 | 115 |
| 288 | 1 | 115 | 323 | 3 | 154 |
| 289 | 50 | 51 | 324 | 3 | 50 |
| 290 | 3 | 94 | 325 | 3 | 233 |
| 291 | 50 | 212 | 326 | 3 | 132 |
| 292 | 1 | 46 | 327 | 3 | 124 |
| 293 | 50 | 55 | 328 | 3 | 58 |
| 294 | 1 | 271 | 329 | 3 | 100 |
| 295 | 1 | 95 | 330 | 1 | 191 |
| 296 | 1 | 87 | 331 | 3 | 64 |
| 297 | 1 | 151 | 332 | 3 | 15 |
| 298 | 1 | 51 | 333 | 3 | 25 |
| 299 | 3 | 6 | 334 | 3 | 255 |
| 300 | 3 | 140 | 335 | 3 | 187 |
| 301 | 3 | 410 | 336 | 1 | 68 |
| 302 | 3 | 104 | 337 | 1 | 52 |
| 303 | 3 | 67 | 338 | 1 | 241 |
| 304 | 10 | 167 | 339 | 1 | 127 |
| 305 | 3 | 314 | 340 | 50 | 41 |
| 306 | 3 | 532 | 341 | 50 | 202 |
| 307 | 3 | 320 | 342 | 1 | 109 |
| 308 | 50 | 9 | 343 | 1 | 44 |
| 309 | 1 | 71 | 344 | 1 | 69 |
| 310 | 1 | 87 | 345 | 1 | 232 |
| 311 | 1 | 265 | 346 | 1 | 96 |
| 312 | 1 | 80 | 347 | 1 | 37 |
| 313 | 1 | 84 | 348 | 1 | 57 |
| 314 | 1 | 9 | 349 | 1 | 101 |
| 315 | 1 | 19 | 350 | 1 | 31 |

| Example | Concentration for evaluation (μM) | Nav1.1 current amplification rate (%) | Example | Concentration for evaluation (μM) | Nav1.1 current amplification rate (%) |
|---|---|---|---|---|---|
| 351 | 50 | 98 | 386 | 50 | 126 |
| 352 | 1 | 155 | 387 | 1 | 19 |
| 353 | 50 | 32 | 388 | 10 | 37 |
| 354 | 1 | 123 | 389 | 10 | 53 |
| 355 | 1 | 130 | 390 | 10 | 50 |
| 356 | 1 | 378 | 391 | 10 | 30 |
| 357 | 50 | 43 | 392 | 10 | 53 |
| 358 | 1 | 71 | 393 | 10 | 19 |
| 359 | 10 | 26 | 394 | 10 | 98 |
| 360 | 1 | 200 | 395 | 10 | 343 |
| 361 | 1 | 101 | 396 | 10 | 136 |
| 362 | 1 | 133 | 397 | 10 | 273 |
| 363 | 1 | 174 | 398 | 10 | 175 |
| 364 | 1 | 25 | 399 | 10 | 102 |
| 365 | 50 | 32 | 400 | 10 | 238 |
| 366 | 50 | 37 | 401 | 10 | 286 |
| 367 | 1 | 38 | 402 | 10 | 323 |
| 368 | 10 | 18 | 403 | 10 | 270 |
| 369 | 50 | 10 | 404 | 10 | 42 |
| 370 | 10 | 35 | 405 | 3 | 268 |
| 371 | 50 | 64 | 406 | 50 | 68 |
| 372 | 10 | 52 | 407 | 3 | 113 |
| 373 | 10 | 35 | 408 | 50 | 80 |
| 374 | 10 | 18 | 409 | 3 | 244 |
| 375 | 10 | 173 | 410 | 3 | 66 |
| 376 | 10 | 44 | 411 | 50 | 18 |
| 377 | 1 | 134 | 412 | 50 | 36 |
| 378 | 1 | 37 | 413 | 3 | 27 |
| 379 | 10 | 147 | 414 | 10 | 40 |
| 380 | 1 | 82 | 415 | 10 | 93 |
| 381 | 1 | 153 | 416 | 10 | 383 |
| 382 | 1 | 13 | 417 | 3 | 23 |
| 383 | 1 | 84 | 418 | 3 | 105 |
| 384 | 1 | 15 | 419 | 1 | 66 |
| 385 | 50 | 42 | 420 | 10 | 39 |

| Example | Concentration for evaluation (μM) | Nav1.1 current amplification rate (%) | Example | Concentration for evaluation (μM) | Nav1.1 current amplification rate (%) |
|---|---|---|---|---|---|
| 421 | 10 | 199 | 456 | 10 | 224 |
| 422 | 10 | 287 | 457 | 50 | 29 |
| 423 | 10 | 11 | 458 | 10 | 24 |
| 424 | 10 | 40 | 459 | 10 | 118 |
| 425 | 10 | 283 | 460 | 10 | 221 |
| 426 | 10 | 244 | 461 | 10 | 337 |

| | | | | | |
|---|---|---|---|---|---|
| 427 | 10 | 172 | 462 | 10 | 124 |
| 428 | 1 | 109 | 463 | 10 | 218 |
| 429 | 1 | 101 | 464 | 10 | 18 |
| 430 | 10 | 28 | 465 | 10 | 186 |
| 431 | 10 | 11 | 466 | 10 | 233 |
| 432 | 1 | 280 | 467 | 1 | 80 |
| 433 | 10 | 192 | 468 | 1 | 275 |
| 434 | 10 | 232 | 469 | 10 | 243 |
| 435 | 10 | 262 | 470 | 10 | 522 |
| 436 | 10 | 226 | 471 | 1 | 142 |
| 437 | 3 | 582 | 472 | 10 | 387 |
| 438 | 3 | 404 | 473 | 10 | 487 |
| 439 | 10 | 87 | 474 | 10 | 235 |
| 440 | 10 | 196 | 475 | 10 | 633 |
| 441 | 10 | 30 | 476 | 10 | 343 |
| 442 | 10 | 414 | 477 | 10 | 398 |
| 443 | 10 | 114 | 478 | 3 | 345 |
| 444 | 10 | 292 | 479 | 10 | 420 |
| 445 | 10 | 320 | 480 | 1 | 110 |
| 446 | 10 | 333 | 481 | 10 | 128 |
| 447 | 10 | 148 | 482 | 10 | 219 |
| 448 | 10 | 236 | 483 | 10 | 175 |
| 449 | 10 | 250 | 484 | 10 | 224 |
| 450 | 10 | 187 | 485 | 10 | 264 |
| 451 | 10 | 365 | 486 | 10 | 148 |
| 452 | 10 | 10 | 487 | 10 | 190 |
| 453 | 10 | 15 | 488 | 10 | 376 |
| 454 | 10 | 157 | 489 | 10 | 29 |
| 455 | 10 | 131 | 490 | 10 | 469 |

| Example | Concentration for evaluation (μM) | Nav1.1 current amplification rate (%) | Example | Concentration for evaluation (μM) | Nav1.1 current amplification rate (%) |
|---|---|---|---|---|---|
| 491 | 3 | 157 | 526 | 1 | 499 |
| 492 | 3 | 222 | 527 | 1 | 324 |
| 493 | 3 | 317 | 528 | 1 | 198 |
| 494 | 3 | 255 | 529 | 1 | 43 |
| 495 | 1 | 28 | 530 | 50 | 9 |
| 496 | 1 | 37 | 531 | 1 | 183 |
| 497 | 1 | 43 | 532 | 10 | 239 |
| 498 | 10 | 24 | 533 | 10 | 22 |
| 499 | 1 | 50 | 534 | 10 | 7 |
| 500 | 1 | 14 | 535 | 10 | 277 |
| 501 | 1 | 33 | 536 | 10 | 345 |
| 502 | 1 | 49 | 537 | 1 | 99 |
| 503 | 1 | 58 | 538 | 1 | 135 |
| 504 | 1 | 27 | 539 | 1 | 80 |
| 505 | 50 | 255 | 540 | 1 | 112 |
| 506 | 50 | 10 | 541 | 1 | 325 |

| | | | | | |
|---|---|---|---|---|---|
| 507 | 10 | 10 | 542 | 1 | 214 |
| 508 | 10 | 24 | 543 | 1 | 154 |
| 509 | 3 | 238 | 544 | 1 | 208 |
| 510 | 3 | 50 | 545 | 1 | 223 |
| 511 | 3 | 37 | 546 | 1 | 59 |
| 512 | 1 | 103 | 547 | 1 | 70 |
| 513 | 10 | 262 | 548 | 1 | 41 |
| 514 | 1 | 325 | 549 | 1 | 33 |
| 515 | 1 | 255 | 550 | 1 | 31 |
| 516 | 1 | 130 | 551 | 10 | 16 |
| 517 | 1 | 359 | 552 | 1 | 24 |
| 518 | 1 | 344 | 553 | 1 | 155 |
| 519 | 1 | 135 | 554 | 1 | 115 |
| 520 | 1 | 253 | 555 | 1 | 80 |
| 521 | 1 | 358 | 556 | 1 | 13 |
| 522 | 1 | 367 | 557 | 1 | 17 |
| 523 | 1 | 129 | 558 | 10 | 11 |
| 524 | 1 | 143 | 559 | 10 | 34 |
| 525 | 10 | 18 | 560 | 1 | 12 |

| Example | Concentration for evaluation (μM) | Nav1.1 current amplification rate (%) | Example | Concentration for evaluation (μM) | Nav1.1 current amplification rate (%) |
|---|---|---|---|---|---|
| 561 | 1 | 17 | 596 | 3 | 11 |
| 562 | 1 | 247 | 597 | 3 | 32 |
| 563 | 1 | 50 | 598 | 1 | 13 |
| 564 | 10 | 73 | 599 | 50 | 26 |
| 565 | 1 | 62 | 600 | 1 | 75 |
| 566 | 1 | 98 | 601 | 1 | 10 |
| 567 | 50 | 281 | 602 | 1 | 14 |
| 568 | 1 | 42 | 603 | 1 | 11 |
| 569 | 50 | 393 | 604 | 50 | 15 |
| 570 | 1 | 99 | 605 | 50 | 109 |
| 571 | 10 | 24 | 606 | 1 | 75 |
| 572 | 1 | 49 | 607 | 50 | 37 |
| 573 | 1 | 44 | 608 | 1 | 98 |
| 574 | 1 | 303 | 609 | 1 | 84 |
| 575 | 1 | 84 | 610 | 1 | 60 |
| 576 | 3 | 14 | 611 | 1 | 113 |
| 577 | 1 | 249 | 612 | 3 | 12 |
| 578 | 1 | 57 | 613 | 3 | 20 |
| 579 | 1 | 59 | 614 | 3 | 95 |
| 580 | 1 | 134 | 615 | 3 | 20 |
| 581 | 1 | 138 | 616 | 3 | 23 |
| 582 | 1 | 68 | 617 | 1 | 41 |
| 583 | 1 | 155 | 618 | 1 | 41 |
| 584 | 1 | 53 | 619 | 1 | 107 |
| 585 | 1 | 126 | 620 | 3 | 21 |
| 586 | 1 | 25 | 621 | 3 | 69 |

| | | | | | |
|---|---|---|---|---|---|
| 587 | 1 | 22 | 622 | 3 | 14 |
| 588 | 1 | 60 | 623 | 10 | 25 |
| 589 | 1 | 44 | 624 | 1 | 61 |
| 590 | 1 | 32 | 625 | 1 | 255 |
| 591 | 1 | 22 | 626 | 1 | 200 |
| 592 | 50 | 13 | 627 | 50 | 60 |
| 593 | 1 | 78 | 628 | 1 | 14 |
| 594 | 10 | 11 | 629 | 3 | 15 |
| 595 | 3 | 14 | 630 | 1 | 120 |

| Example | Concentration for evaluation (μM) | Nav1.1 current amplification rate (%) | Example | Concentration for evaluation (μM) | Nav1.1 current amplification rate (%) |
|---|---|---|---|---|---|
| 631 | 1 | 49 | 666 | 1 | 64 |
| 632 | 1 | 164 | 667 | 1 | 45 |
| 633 | 1 | 15 | 668 | 1 | 72 |
| 634 | 1 | 13 | 669 | 1 | 30 |
| 635 | 1 | 76 | 670 | 1 | 77 |
| 636 | 10 | 13 | 671 | 1 | 34 |
| 637 | 3 | 13 | 672 | 1 | 56 |
| 638 | 10 | 58 | 673 | 3 | 20 |
| 639 | 1 | 100 | 674 | 1 | 62 |
| 640 | 1 | 38 | 675 | 1 | 36 |
| 641 | 1 | 361 | 676 | 1 | 36 |
| 642 | 1 | 252 | 677 | 1 | 35 |
| 643 | 1 | 199 | 678 | 1 | 132 |
| 644 | 1 | 197 | 679 | 1 | 63 |
| 645 | 1 | 351 | 680 | 1 | 50 |
| 646 | 1 | 148 | 681 | 1 | 26 |
| 647 | 1 | 165 | 682 | 1 | 53 |
| 648 | 1 | 53 | 683 | 1 | 63 |
| 649 | 1 | 91 | 684 | 1 | 39 |
| 650 | 1 | 26 | 685 | 1 | 41 |
| 651 | 1 | 26 | 686 | 1 | 13 |
| 652 | 1 | 53 | 687 | 1 | 67 |
| 653 | 1 | 173 | 688 | 1 | 201 |
| 654 | 1 | 23 | 689 | 1 | 286 |
| 655 | 1 | 172 | 690 | 1 | 66 |
| 656 | 1 | 70 | 691 | 3 | 11 |
| 657 | 1 | 44 | 692 | 1 | 33 |
| 658 | 1 | 33 | 693 | 1 | 13 |
| 659 | 1 | 25 | 694 | 1 | 448 |
| 660 | 1 | 64 | 695 | 1 | 219 |
| 661 | 1 | 12 | 696 | 1 | 306 |
| 662 | 50 | 47 | 697 | 1 | 21 |
| 663 | 1 | 13 | 698 | 1 | 18 |
| 664 | 1 | 34 | 699 | 1 | 366 |
| 665 | 50 | 63 | 700 | 3 | 13 |

| Example | Concentration for evaluation (μM) | Nav1.1 current amplification rate (%) | Example | Concentration for evaluation (μM) | Nav1.1 current amplification rate (%) |
|---|---|---|---|---|---|
| 701 | 1 | 38 | 736 | 1 | 13 |
| 702 | 1 | 60 | 737 | 1 | 249 |
| 703 | 1 | 27 | 738 | 3 | 22 |
| 704 | 1 | 109 | 739 | 3 | 10 |
| 705 | 1 | 54 | 740 | 1 | 91 |
| 706 | 1 | 195 | 741 | 1 | 61 |
| 707 | 1 | 62 | 742 | 1 | 32 |
| 708 | 3 | 25 | 743 | 50 | 39 |
| 709 | 1 | 44 | 744 | 1 | 147 |
| 710 | 1 | 62 | 745 | 1 | 43 |
| 711 | 1 | 100 | 746 | 1 | 102 |
| 712 | 50 | 78 | 747 | 1 | 73 |
| 713 | 1 | 112 | 748 | 1 | 17 |
| 714 | 3 | 11 | 749 | 10 | 63 |
| 715 | 1 | 13 | 750 | 10 | 27 |
| 716 | 1 | 45 | 751 | 10 | 121 |
| 717 | 3 | 23 | 752 | 1 | 135 |
| 718 | 50 | 34 | 753 | 1 | 106 |
| 719 | 1 | 78 | 754 | 1 | 14 |
| 720 | 1 | 230 | 755 | 1 | 245 |
| 721 | 1 | 17 | 756 | 1 | 104 |
| 722 | 1 | 35 | 757 | 1 | 10 |
| 723 | 1 | 95 | 758 | 1 | 61 |
| 724 | 1 | 28 | 759 | 1 | 57 |
| 725 | 10 | 51 | 760 | 1 | 104 |
| 726 | 10 | 31 | 761 | 1 | 43 |
| 727 | 10 | 71 | 762 | 50 | 37 |
| 728 | 1 | 71 | 763 | 1 | 98 |
| 729 | 10 | 55 | 764 | 1 | 164 |
| 730 | 1 | 133 | 765 | 1 | 25 |
| 731 | 10 | 102 | 766 | 1 | 41 |
| 732 | 1 | 152 | 767 | 10 | 60 |
| 733 | 1 | 76 | 768 | 1 | 18 |
| 734 | 1 | 107 | 769 | 1 | 23 |
| 735 | 1 | 321 | 770 | 1 | 35 |

| Example | Concentration for evaluation (μM) | Nav1.1 current amplification rate (%) | Example | Concentration for evaluation (μM) | Nav1.1 current amplification rate (%) |
|---|---|---|---|---|---|
| 771 | 1 | 40 | 806 | 1 | 262 |
| 772 | 50 | 35 | 807 | 1 | 86 |
| 773 | 1 | 69 | 808 | 1 | 226 |
| 774 | 1 | 50 | 809 | 10 | 64 |
| 775 | 50 | 12 | 810 | 1 | 86 |
| 776 | 50 | 17 | 811 | 1 | 23 |
| 777 | 1 | 13 | 812 | 1 | 13 |

| | | | | | |
|---|---|---|---|---|---|
| 778 | 50 | 13 | 813 | 1 | 105 |
| 779 | 1 | 44 | 814 | 1 | 87 |
| 780 | 1 | 10 | 815 | 1 | 86 |
| 781 | 50 | 15 | 816 | 1 | 13 |
| 782 | 50 | 64 | 817 | 1 | 21 |
| 783 | 3 | 11 | 818 | 50 | 195 |
| 784 | 1 | 45 | 819 | 1 | 88 |
| 785 | 3 | 17 | 820 | 1 | 50 |
| 786 | 1 | 34 | 821 | 1 | 116 |
| 787 | 1 | 50 | 822 | 1 | 42 |
| 788 | 1 | 174 | 823 | 1 | 226 |
| 789 | 1 | 36 | 824 | 1 | 240 |
| 790 | 1 | 31 | 825 | 1 | 30 |
| 791 | 50 | 14 | 826 | 1 | 31 |
| 792 | 1 | 161 | 827 | 1 | 29 |
| 793 | 50 | 55 | 828 | 1 | 108 |
| 794 | 1 | 27 | 829 | 1 | 54 |
| 795 | 1 | 111 | 830 | 1 | 54 |
| 796 | 1 | 66 | 831 | 1 | 128 |
| 797 | 1 | 66 | 832 | 1 | 204 |
| 798 | 1 | 193 | 833 | 1 | 283 |
| 799 | 1 | 35 | 834 | 1 | 26 |
| 800 | 1 | 55 | 835 | 50 | 118 |
| 801 | 1 | 122 | 836 | 1 | 19 |
| 802 | 1 | 111 | 837 | 1 | 103 |
| 803 | 1 | 222 | 838 | 0.3 | 122 |
| 804 | 1 | 93 | 839 | 1 | 143 |
| 805 | 1 | 373 | 840 | 1 | 164 |

| Example | Concentration for evaluation (μM) | Nav1.1 current amplification rate (%) | Example | Concentration for evaluation (μM) | Nav1.1 current amplification rate (%) |
|---|---|---|---|---|---|
| 841 | 1 | 28 | 876 | 1 | 98 |
| 842 | 1 | 87 | 877 | 1 | 263 |
| 843 | 1 | 171 | 878 | 1 | 42 |
| 844 | 1 | 221 | 879 | 50 | 16 |
| 845 | 1 | 76 | 880 | 50 | 42 |
| 846 | 1 | 158 | 881 | 1 | 446 |
| 847 | 1 | 132 | 882 | 1 | 88 |
| 848 | 0.3 | 22 | 883 | 1 | 164 |
| 849 | 0.3 | 57 | 884 | 1 | 34 |
| 850 | 1 | 79 | 885 | 1 | 209 |
| 851 | 1 | 89 | 886 | 1 | 125 |
| 852 | 1 | 111 | 887 | 0.3 | 173 |
| 853 | 1 | 82 | 888 | 1 | 101 |
| 854 | 1 | 45 | 889 | 1 | 151 |
| 855 | 1 | 93 | 890 | 1 | 68 |
| 856 | 1 | 50 | 891 | 1 | 81 |
| 857 | 1 | 18 | 892 | 1 | 39 |

| | | | | | |
|---|---|---|---|---|---|
| 858 | 1 | 11 | 893 | 10 | 31 |
| 859 | 50 | 39 | 894 | 10 | 151 |
| 860 | 1 | 50 | 895 | 1 | 244 |
| 861 | 1 | 78 | 896 | 1 | 241 |
| 862 | 1 | 97 | | | |
| 863 | 1 | 49 | | | |
| 864 | 1 | 160 | | | |
| 865 | 1 | 14 | | | |
| 866 | 1 | 16 | | | |
| 867 | 1 | 65 | | | |
| 868 | 1 | 19 | | | |
| 869 | 1 | 17 | | | |
| 870 | 1 | 120 | | | |
| 871 | 1 | 10 | | | |
| 872 | 1 | 38 | | | |
| 873 | 1 | 89 | | | |
| 874 | 1 | 74 | | | |
| 875 | 1 | 43 | | | |

Test 2: Evaluation of the amplification in Nav1.5-derived voltage-gated sodium current (Nav1.5 current) by using a cell line stably expressing human Nav1.5

A CHO-K1 cell line (Chinese hamster ovary) stably expressing human Nav1.5 (Gene Bank Accession No: P_000326.2) is obtained by using purchased T-Rex System (ThermoFisher Scientific, USA), and used for the present test. The effect of test compounds on the amplification of Navl.5 current is evaluated by using a cell line stably expressing human Nav1.5 and HTS automated patch clamp system, similarly in the method using Nav1.1. In other words, test compounds are added in an extracellular fluid which contained 1% DMSO and 500 nmol/L Tetrodotoxin (TTX), and evaluated as a change of the peak value of Navl.5 current and the area under the curve (AUC). The induction and the measurement of Navl.5 current, the pharmacological effect on Navl.5 current, and a method for pharmacological evaluation are carried out by the same method as in Nav1.1.

Test 3: Evaluation of the cognitive function by Novel Object

Recognition Test (hereinafter, also referred to as "NORT")

The present test is for evaluating the improvement of cognitive function with the test compounds. When an APP-Tg mouse which is an AD (Alzheimer's disease) model mouse is evaluated by NORT, the memory for a familiar object decreases depending on the time interval between the first trial run (training) and the second trial run (test). For example, if the first trial run is done and 3 hours later the second one is done, there is no difference in the searching times between novel object and known object for the APP-Tg mouse, compared with a healthy mouse. Thus, the APP-Tg mouse suffers from significant amnesia.

The APP-Tg mouse used herein was prepared by constructing an expression cassette linked to human $APP_{751}$ isoform to which Swedish (K670N/M671L) and Indiana (V717F) mutation were introduced at downstream of mouse Thy-1 promoter, then injecting the expression cassette into a mouse fertilized egg, and transplanting it to a host mouse. The prepared mouse presents with intracerebral Aβ accumulation and cognitive dysfunction from early on, and thus the mouse can be used for the evaluation of cognitive function. APP-Tg mouse has been also reported in Non-patent literature 6.

To the prepared APP-Tg mouse, the test compound was administered, and then the first trial run was done for the mouse 30 minutes after the intraperitoneal administration or after 10 days of mixed diet administration. The second trial run was done 3 hours after the first trial run, and the searching times between novel object and known object in the second trial were evaluated. The discrimination index was calculated from the searching time for novel object and known object in the second trial run, and the improvement of cognitive function of the test compound was confirmed by comparing the discrimination index with the test compound-unadministered group as an index of the cognitive function. The discrimination index was calculated by the following formula.

Discrimination index = {(Searching time for novel object) - (Searching time for known object)}/{(Searching time for novel object) + (Searching time for known object)}

According to the above method, the compound of Example 1 was intraperitoneally administered to the APP-Tg mouse at doses of 3, 10 and 30 mg/kg 30 minutes before the first trial run, and the improvement of cognitive function was evaluated. The result is shown in the following table. Here, the significant difference from the vehicle-administered group was determined using the parametric Dunnett multiple comparison test. The compound of Example 1 showed a dose-dependent improvement of cognitive function, and was confirmed to be statistically significant at doses of 10 and 30 mg/kg.

| Exampl e | Dose (mg/g i.p.) | Discrimination index | SEM | p value vs. vehicle # : p<0.05 |
|---|---|---|---|---|
| 1 | vehicle | -0.05 | 0.09 | |
| | 3 | 0.12 | 0.06 | - |
| | 10 | 0.22 | 0.07 | # |
| | 30 | 0.25 | 0.09 | # |

The present test can be also done with a combination with Elacridar (Tokyo Chemical Industry Co., Ltd.). Elacridar can inhibit P-gp and BCRP which are excretion transporters expressed in the blood-brain barrier and thereby Elacridar has the effect of improving the transferability of the test compound to the brain. Eracridal was orally administered to APP-Tg mice at 100 mg/kg 2 hours before the first trial run, and then the test compound was intraperitoneally administered to the mice 30 minutes before the first trial run according to the above method. And, the improvement of cognitive function was evaluated. The significant difference from the vehicle-administered group was determined using the parametric Dunnett multiple comparison test. The results are shown in the following table. The compounds of Examples 577, 593, and 609 showed some improvement of cognitive function, and especially the compounds of Examples 577 and 609 showed strong improvement of cognitive function.

| Exampl e | Dose (mg/g i.p.) | Discrimination index | SEM | p value vs. vehicle # : p<0.05 |
|---|---|---|---|---|
| 577 | vehicle | -0.03 | -0.05 | |
| | 10 | 0.13 | 0.10 | - |
| | 30 | 0.21 | 0.06 | # |
| 593 | vehicle | -0.03 | 0.10 | |
| | 30 | 0.11 | 0.11 | - |
| | 60 | 0.20 | 0.06 | - |
| 609 | vehicle | -0.03 | 0.10 | |
| | 30 | 0.29 | 0.07 | # |
| | 60 | 0.29 | 0.04 | # |

And, the present test can be also done with a combination with ABT (1-aminobenzotriazole) (Tokyo Chemical Industry Co., Ltd.). ABT has the effect of improving the blood concentration of the test compound by inhibiting CYP and suppressing metabolism. The test compound and ABT (1 mg / g-food) were administered with food to APP-Tg mice for 10 days, and then the improvement of cognitive function was evaluated according to the above method. The significant difference from the vehicle-administered group was determined using the parametric Dunnett multiple comparison test. The results are shown in the following table. The compounds of Examples 630, 746, 795, 819, and 821 showed some improvement of cognitive function, and especially the compounds of Examples 819 and 821 showed strong improvement of cognitive function.

| Exampl e | Dose (mg/g-food) | Discrimination index | SEM | p value vs. vehicle |
|---|---|---|---|---|
| 630 | vehicle | 0.06 | 0.10 | |
| | 0.2 | 0.04 | 0.19 | - |
| | 2 | 0.45 | 0.09 | - |
| 746 | vehicle | 0.06 | 0.10 | |
| | 0.2 | 0.28 | 0.20 | - |
| | 2 | 0.38 | 0.23 | - |
| 753 | vehicle | 0.25 | 0.13 | |
| | 0.1 | 0.21 | 0.15 | - |
| | 4 | 0.28 | 0.14 | - |
| 795 | vehicle | 0.15 | 0.08 | |
| | 0.2 | 0.30 | 0.07 | - |
| | 2 | 0.27 | 0.06 | - |
| 819 | vehicle | 0.25 | 0.13 | |
| | 0.2 | 0.47 | 0.10 | p=0.057 |
| | 2 | 0.52 | 0.10 | p=0.094 |
| 821 | vehicle | 0.15 | 0.08 | |
| | 0.2 | 0.30 | 0.03 | - |
| | 2 | 0.37 | 0.02 | p=0.074 |

The above results showed that the compounds of Examples 1, 577, 593, 609, 630, 746, 795, 819, and 821 have some improvement of cognitive function. In addition, the improvement of cognitive function of the compounds described in the other Examples can be evaluated by the same method as described above.

Test 4: Evaluation of phosphorylated tau oligomers using APP-Tg mice

This test is a test to quantify the oligomer of phosphorylated tau in the brain to evaluate the action of a compound. Although APP-Tg mice do not show neurofibrillary tangles or neuronal cell death, month-age-dependent increases in brain phosphorylated tau and its oligomers are observed compared to wild-type mice. The APP-Tg mice used herein were prepared by the method described in Test 3.

The test compound administered with food to APP-Tg mice for 7 - 10 days. At this time, in order to increase the blood concentration of the test compound, ABT was used in combination with a mixed diet of 1 mg/g-food depending on the compound. After the administration, the brain was collected, and the protein is extracted from the collected brain using TBS (Tris Buffered Saline) or sarcosyl, and then SDS-PAGE (sodium dodecyl sulfate-polyacrylamide gel electrophoresis) of the soluble or insoluble fraction was performed. After that, Western blotting was performed using an anti-oligomerized tau antibody (Anti-Tau, oligomeric, clone TOMA-1), and the effect of the test compound was evaluated by quantifying the band amount of the phosphorylated tau oligomer.

The reducing effect of phosphorylated tau oligomer was calculated by the following formula, and from the calculated values, the results were defined as follows: 0 - 25 %: +, 25 - 50 %: ++, 50 - 75 %: +++, and 75 - 100 %: ++++. The significant difference from the vehicle-administered group was determined using the Student t-test. For the band amount, the value corrected by the β-actin band amount was used.

Reducing effect of phosphorylated tau oligomer = {(band amount of oligomerized tau in vehicle-administered group - band amount of oligomerized tau in test compound-administered group) / band amount of oligomerized tau in vehicle-administered group} × 100

The results are shown in the following table. It has clarified that Examples 1, 410, 560, 565, 577, 630, 746, 753, 795, and 819 have some reducing effect of tau oligomer, and especially Examples 1, 410, 560, 565, 577, and 795 have strong reducing effect of tau oligomer.

| Example | Dose (mg/g-food) | Reducing effect of phosphorylated tau oligomer | p value vs. vehicle # : p<0.05 | with ABT 1 mg/g-food |
|---|---|---|---|---|
| 1 | 1.9 | +++ | - | - |
| | 5.8 | ++++ | # | |
| 364 | 0.6 | + | - | with ABT |
| | 2 | + | - | |
| 410 | 0.6 | +++ | p=0.08 | with ABT |
| | 2 | + | - | |
| 560 | 0.5 | ++ | - | with ABT |
| | 4.5 | ++++ | # | |
| 565 | 0.5 | +++ | - | with ABT |
| | 4.5 | ++ | - | |
| 577 | 2.2 | + | - | - |
| | 6.6 | +++ | p=0.07 | |
| 630 | 0.2 | ++ | - | with ABT |
| | 2 | ++ | - | |
| 746 | 0.2 | ++ | - | with ABT |
| | 2 | ++ | - | |
| 753 | 0.1 | ++ | - | with ABT |
| | 4 | ++ | - | |
| 795 | 0.2 | + | - | with ABT |
| | 2 | +++ | # | |
| 819 | 0.2 | ++ | - | with ABT |
| | 2 | + | - | |
| 821 | 0.2 | + | - | with ABT |
| | 2 | + | - | |

The oligomer of phosphorylated tau of the compounds described in the other Examples can be evaluated by the same method as described above.

Test 5: Evaluation of intracerebral tau aggregate accumulation and cerebral atrophy using rTg4510 mice

This test is a test to evaluate the effects of the compounds on intracerebral tau aggregate accumulation and cerebral atrophy. The rTg4510 mice overexpress the human-type FTDP-17 mutant tau in the forebrain, and month-age-dependent intracerebral tau aggregate accumulation and cerebral atrophy are observed. The rTg4510 mice used herein were produced by mating Tg (tauP301L) 4510 mice (Stock No. 015815) and CaMKII-tTA mice (Stock No. 007004) purchased from The Jackson Laboratory and breeding them.

The test compound was administered as a mixed diet to rTg4510 mice from the age of 4 months, and PET scan and MRI were performed at the ages of 4, 6 and 8 months to quantitatively evaluate the amount of the intracerebral tau aggregate accumulated and cerebral atrophy. The PET scan was performed using MicroPET Focus 220 of Siemens Medical Solutions (0.851 mm thick (intercenter) 95 slices, 19.0 cm axial field of view (FOV), and 7.6 cm intra-section FOV). The administration of radioactive compound [$^{18}$F] PM-PBB3 (precursor obtained from Aprinoia) and the PET scan were performed in dimly lit after the mice was treated with 1.5% isoflurane anesthesia to avoid photoracemization of the radioactive compound. The emission scan was performed for 60 minutes immediately after the intravenous administration of [$^{18}$F] PM-PBB3 (1 mCi) (3D list mode, energy window: 350-750 keV). Then, the list mode data sorted into 3D synograms

were converted into 2D synograms by Fourier-rebining (frames: 4 × 1, 8 × 2 and 8 × 5 minutes). The dynamic image for 60 minutes after the administration of [$^{18}$F] PM-PBB3 was reconstructed by a filter-corrected back projection with a 0.5 mm Hanning filter. Regions of interest (ROI) were set in multiple brain regions using PMOD image analysis software (PMOD Technology) with reference to the MRI templates and the PET reconstructed images. Tau aggregate accumulation was evaluated by calculating SUVR (Standardized uptake value ratio) with the cerebellum as a reference region, based on the added average of dynamic images from 40 minutes to 60 minutes.

The evaluation of cerebral atrophy was performed with horizontal superconducting magnet type MRI (7T, inner diameter 400 mm, self-shielding type, zero boil-off type). The imaging was performed under isoflurane anesthesia while maintaining the rectal temperature at 36.5 ± 0.5°C. After Tripilot acquisition, the brain images were acquired by Turbo-RARE T2 emphasis sequence (TR: 4200 ms, TE: 36 ms, Fat-Sup: on, NA: 4, RARE factor: 8, scan time: 14 min) (FOV read: 25.6 mm, FOV P1: 14.5 mm, Slice thickness: 0.5 mm, number of slices: 28). The ROI of each brain region was set in each of the acquired slices, and the volume of the brain region was calculated by summing the ROI areas of all the slices to evaluate the effect of the test compound.

According to the above method, rTg4510 mice were fed a diet containing 5.8 mg/g-food of the compound of Example 1 from the age of 4 months, and the effect of PET/MR on tau aggregate accumulation and cerebral atrophy was evaluated. The results are shown in Figures 1-3 and the table below.

In FIG. 1, the SUVR average images of tau PET in Example 1-administration and non-administration groups with rTg4510 mice at the ages of 4, 6 and 8 months are shown in the upper row, and typical T2-weighted images of the corresponding positions are shown in the lower row. It was clarified that, at the age of 4 months, there was no difference in the degree of tau PET tracer accumulation between the two administration groups and no difference in the brain volume, but in the non-administration group, an increase in the PET tracer accumulation was observed in each region with aging, and similarly, the cerebral atrophy and the enlargement of the lateral ventricle were observed, whereas they were significantly suppressed in the Example 1-administration group.

Figure 2 shows the quantitative results of volume changes in each brain region. The result showed that the cerebellar volume which is the reference region did not change with aging, but the age-dependent cerebral atrophy observed in the cerebral neocortex and hippocampus was statistically significantly suppressed in the Example 1-administration group. And, it was shown that the age-dependent increase in the lateral ventricular volume was statistically significantly suppressed in the Example 1-administration group. The results are also shown in the table below.

| month old | administration group | volume (mm$^3$) * p<0.05, ** p<0.01 vs. control | | | |
|---|---|---|---|---|---|
| | | cerebellum | cerebral neocortex | hippocampus | lateral ventricle |
| 4 | control | 33.9±0.20 | 58.2±0.38 | 12.4±0.19 | 1.04±0.046 |
| | Example 1 | 34.3±0.19 | 60.1±0.49 | 14.0±0.13 | 0.79±0.014 |
| 6 | control | 33.7±0.34 | 41.1±0.77 | 9.98±0.21 | 8.21±0.179 |
| | Example 1 | 33.9±0.18 | 50.9±0.98 | 12.6±0.22 | 1.70±0.312 |
| 8 | control | 33.7±0.19 | 33.3±0.53 | 9.51±0.20 | 8.41±0.516 |
| | Example 1 | 33.4±0.33 | 45.3±1.13 | 12.7±0.12 | 0.61±0.036 |

FIG. 3 shows SUVR in each brain region. It was shown that the age-dependent increase in SUVR observed in the cerebral neocortex and hippocampus was statistically significantly suppressed in the Example 1-administration group. The results are also shown in the table below.

| month old | administration group | SUVR (cerebellum ratio) * p<0.05, ** p<0.01 vs. control | |
|---|---|---|---|
| | | cerebral neocortex | hippocampus |
| 4 | control | 1.020±0.009 | 1.082±0.008 |
| | Example 1 | 0.986±0.009 | 1.078±0.009 |
| 6 | control | 1.196±0.012 | 1.337±0.012 |
| | Example 1 | 1.098±0.013 | 1.182±0.016 |
| 8 | control | 1.378±0.011 | 1.472±0.012 |
| | Example 1 | 1.184±0.017 | 1.284±0.024 |

In the same manner as described above, the compound of Example 577 was administered to the mice as a mixed diet at 0.7 and 6.6 mg/g-food and evaluated. In the non-administration group, the SUVR of the tau PET tracer increased monotonically with ages at 4, 6 and 8 months, but in the compound-administration group of Example 577, 3 out of 5 individuals in the 0.7 mg/g-hood administration group and 3 out of 6 in the 6.6 mg/g-hood administration group did not show any increase in the SUVR in the transition from the age of 6 months to the age of 8 months, which suggested that the accumulation of tau aggregates was suppressed. And, no suppression of cerebral atrophy was observed in the Example 577-administration group.

The compound of Example 795 was administered to the mice as a mixed diet at 3 mg/g-food in the same manner as described above, and evaluated. ABT was used in combination with a mixed diet of 1 mg/g-food. The results of the compound administration group of Example 795 at 3 mg/g-food are shown in Figures 4 and 5 and the table below.

Figure 4 shows average SUVR images of tau PET in the administration group of the compound of Example 795 at 3 mg/g-food and non-administration group of rTg4510 mice at the age of 4, 6 and 8 months. It was shown that in the non-administration group, an increase in PET tracer accumulation was observed in each brain region with aging, whereas in the compound administration group of Example 795 at 3 mg/g-food, they were suppressed. Figure 5 shows the SUVR in each brain region. It was shown that the age-dependent increase in SUVR observed in the cerebral neocortex and hippocampus tended to be suppressed in the compound administration group of Example 795 at 3 mg/g-food. The results are also shown in the table below.

| month old | administration group | SUVR(cerebellum ratio) | |
|---|---|---|---|
| | | cerebral neocortex | hippocampus |
| 4 | control | 1.034±0.049 | 1.139±0.061 |
| | Example 795 3 mg/g-food | 1.023±0.009 | 1.134±0.042 |
| 6 | control | 1.200±0.070 | 1.374±0.080 |
| | Example 795 3 mg/g-food | 1.149±0.031 | 1.308±0.044 |
| 8 | control | 1.410±0.117 | 1.583±0.152 |
| | Example 795 3 mg/g-food | 1.268±0.036 | 1.419±0.048 |

Although the age-dependent increase in lateral ventricular volume was not different in the compound administration group of Example 795 at 3 mg/g-food from the non-administration group at the age of 8 months, a tendency to suppress was observed at the age of 6 months, which suggests that cerebral atrophy was delayed.

The compound of Example 560 was administered to the mice as a mixed diet at 4.5 mg/g-hood in the same manner as described above, and evaluated. ABT was used in combination with a mixed diet of 1 mg/g-food. The results of the compound administration group of Example 560 at 4.5 mg/g-food are shown in Figures 6 and 7 and the table below.

Figure 6 shows average SUVR images of tau PET in the administration group of the compound of Example 560 at 4.5 mg/g-food and non-administration group of rTg4510 mice at the ages of 4, 6 and 8 months. It was shown that in the non-administration group, an increase in PET tracer accumulation was observed in each brain region with aging, whereas in the compound administration group of Example 560 at 4.5 mg/g-food, they were suppressed. Figure 7 shows the SUVR in each brain region. It was shown that the age-dependent increase in SUVR observed in the cerebral neocortex and hippocampus tended to be suppressed in the compound administration group of Example 560 at 4.5 mg/g-food. The results are also shown in the table below.

| month old | administration group | SUVR(cerebellum ratio) | |
|---|---|---|---|
| | | cerebral neocortex | hippocampus |
| 4 | control | 1.019±0.020 | 1.089±0.014 |
| | Example 560 4.5 mg/g-food | 1.026±0.037 | 1.103±0.036 |
| 6 | control | 1.242±0.026 | 1.377±0.040 |
| | Example 560 4.5 mg/g-food | 1.224±0.060 | 1.328±0.078 |
| 8 | control | 1.456±0.035 | 1.559±0.049 |
| | Example 560 4.5 mg/g-food | 1.351±0.049 | 1.468±0.056 |

And, the age-dependent increase in lateral ventricular volume was suppressed in the compound administration group of Example 560 at 4.5 mg/g-food, which suggests that cerebral atrophy was suppressed. The results are shown in the table below.

| month old | administration group | lateral ventricular volume ($mm^3$) |
|---|---|---|
| 4 | control | 0.50±0.05 |
| | Example 560 4.5 mg/g-food | 0.47±0.06 |
| 6 | control | 3.11±0.82 |
| | Example 560 4.5 mg/g-food | 0.34±0.10 |
| 8 | control | 3.68±0.77 |
| | Example 560 4.5 mg/g-food | 0.71±0.35 |

By the same method as described above, the evaluation of tau aggregate accumulation and cerebral atrophy in the brain can be carried out for the compounds described in other examples.

INDUSTRIAL APPLICABILITY

Nav activator may become a useful medicament for treating and/or preventing tauopathy.

## Claims

1. A medicament for treating and/or preventing tauopathy, comprising a Nav activator.

2. The medicament of claim 1, wherein the Nav activator is an activator for at least one Nav subtype selected from the group consisting of Nav1.1, Navl.2, Navl.3, and Nav1.6.

3. The medicament of claim 1, wherein the Nav activator is Nav1.1 activator.

4. The medicament of claim 3, wherein the Nav1.1 activator is a small molecule compound.

5. The medicament of Item 3, wherein the Nav1.1 activator is a compound of formula (1):

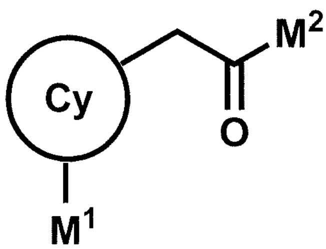

(1)

or a pharmaceutically acceptable salt thereof
wherein

$M^1$ is

(1-1) saturated or partially-unsaturated $C_{4-12}$ carbocyclyl which may be optionally substituted with the same or different 1 to 4 substituents selected from the group consisting of

(a) halogen atom,
(b) hydroxy,

(c) $C_{1-6}$ alkyl which may be optionally substituted with the same or different 1 to 3 substituents selected from the group consisting of halogen atom, hydroxy, and $C_{1-6}$ alkoxy,
(d) $C_{1-6}$ alkoxy which may be optionally substituted with the same or different 1 to 3 substituents selected from the group consisting of halogen atom, hydroxy, and $C_{1-6}$ alkoxy,
(e) amino which may be optionally substituted with the same or different 1 to 2 $C_{1-6}$ alkyl which may be optionally substituted with the same or different 1 to 3 halogen atoms, and
(f) amino-carbonyl wherein the amino may be optionally substituted with the same or different 1 to 2 $C_{1-6}$ alkyl which may be optionally substituted with the same or different 1 to 3 halogen atoms,

(1-2) 4- to 12-membered saturated or partially-unsaturated heterocyclyl which may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy,
(d) $C_{1-6}$ alkoxy which may be optionally substituted with the same or different 1 to 3 substituents selected from the group consisting of halogen atom, hydroxy, and $C_{1-6}$ alkoxy,
(e) $C_{3-6}$ cycloalkyl which may be optionally substituted with the same or different 1 to 3 halogen atoms,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-3) $C_{6-10}$ aryl which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-4) 5- to 10-membered heteroaryl which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-5) $C_{1-10}$ alkoxy which may be optionally substituted with the same or different 1 to 2 substituents selected from the group consisting of

(a) halogen atom,
(b) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy,

(1-6) $C_{6-10}$ aryloxy which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) amino which may be optionally substituted with the same or different 1 to 2 $C_{1-6}$ alkyl which may be optionally substituted with the same or different 1 to 3 halogen atoms, and
(f) amino-carbonyl wherein the amino may be optionally substituted with the same or different 1 to 2 $C_{1-6}$ alkyl which may be optionally substituted with the same or different 1 to 3 halogen atoms,

(1-7) 5- to 10-membered heteroaryloxy which may be optionally substituted with the same or different 1 to 4 substituents selected from the group consisting of

(a) halogen atom,
(b) hydroxy,
(c) cyano,
(d) $C_{1-6}$ alkyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 $C_{1-6}$ alkyl which may be optionally substituted with the same or different 1 to 3 halogen atoms, and
(g) amino-carbonyl wherein the amino may be optionally substituted with the same or different 1 to 2 $C_{1-6}$ alkyl which may be optionally substituted with the same or different 1 to 3 halogen atoms,

(1-8) $C_{1-10}$ alkyl which may be optionally substituted with the same or different 1 to 4 substituents selected from the group consisting of

(a) halogen atom,
(b) $C_{3-6}$ cycloalkyl which may be optionally substituted with the same or different 1 to 3 halogen atoms,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy,

(1-9) $C_{2-10}$ alkenyl which may be optionally substituted with the same or different 1 to 4 substituents selected from the group consisting of

(a) halogen atom,
(b) $C_{3-6}$ cycloalkyl which may be optionally substituted with the same or different 1 to 3 halogen atoms,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different

1 to 3 substituents selected from the group consisting of halogen atom, cyano, $C_{1-6}$ alkyl, and $C_{1-6}$ alkoxy,

(1-10) $C_{2-10}$ alkynyl which may be optionally substituted with the same or different 1 to 4 substituents selected from the group consisting of

(a) halogen atom,
(b) $C_{3-6}$ cycloalkyl which may be optionally substituted with the same or different 1 to 3 halogen atoms,
(c) $C_{6-10}$ aryl which may be optionally substituted with the same or different 1 to 3 substituents selected from the group consisting of halogen atom, cyano, $C_{1-6}$ alkyl, and $C_{1-6}$ alkoxy, and
(d) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy, or

(1-11) $-NR^eR^f$, wherein $R^e$ and $R^f$ are independently

(a) hydrogen atom,
(b) $C_{1-6}$ alkyl which may be optionally substituted with the same or different 1 to 3 substituents selected from the group consisting of halogen atom and $C_{2-10}$ alkynyl,
(c) $C_{3-10}$ cycloalkyl which may be optionally substituted with the same or different 1 to 3 substituents selected from the group consisting of halogen atom, $C_{1-6}$ alkyl, $C_{1-6}$ alkoxy-$C_{1-6}$ alkyl, and $C_{3-6}$ cycloalkyl,
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,
(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy;

$M^2$ is

(2-1) a group of the following formula (2a) or (2b):

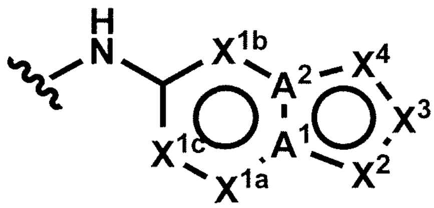
(2a)

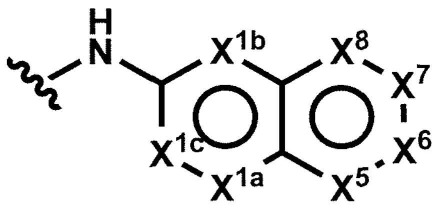
(2b)

wherein

$X^{1a}$, $X^{1b}$, $X^{1c}$, $X^5$, $X^6$, $X^7$, and $X^8$ are independently N or $CR^3$,
$X^2$, $X^3$, and $X^4$ are independently $CR^3$, O, S, N, or $NR^4$,
$A^1$ and $A^2$ are independently N or C,

wherein $X^{1a}$, $X^{1b}$, $X^{1c}$, $X^2$, $X^3$, $X^4$, $X^5$, $X^6$, $X^7$, $X^8$, $A^1$, and $A^2$ are selected so that the ring comprising them can be a 9- or 10-membered bicyclic heteroaromatic ring;

$R^3$ is

(a) hydrogen atom,
(b) halogen atom,

(c) cyano,
(d) hydroxy,
(e) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, $C_{1-6}$ alkoxy, 4- to 7-membered saturated heterocyclyl which may be optionally substituted with $C_{1-6}$ alkoxy, and amino which may be optionally substituted with the same or different 1 to 2 $C_{1-6}$ alkyl,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, $C_{1-6}$ alkoxy, and amino which may be optionally substituted with the same or different 1 to 2 $C_{1-6}$ alkyl,
(g) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl,
(h) amino which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(i) 5- or 6-membered heteroaryl which may be optionally substituted with the same or different 1 to 4 substituents selected from the group consisting of halogen atom, cyano, and $C_{1-6}$ alkyl,
(j) 4- to 7-membered saturated or partially-unsaturated heterocyclyl which may be optionally substituted with the same or different 1 to 4 substituents selected from the group consisting of halogen atom, hydroxy, $C_{1-6}$ alkyl, and $C_{1-6}$ alkoxy, or
(k) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,

$R^4$ is

(a) hydrogen atom,
(b) $C_{1-6}$ alkyl which may be optionally substituted with the same or different 1 to 3 substituents selected from the group consisting of halogen atom, hydroxy, and $C_{1-6}$ alkoxy, or
(c) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,

provided that when there are plural $R^3$ or $R^4$, each $R^3$ or each $R^4$ may be the same or different,

(2-2) a group of the following formula (2c):

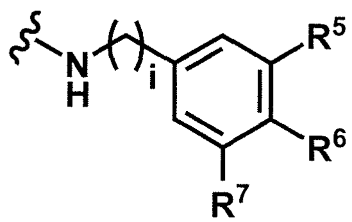

(2c)

wherein

$R^5$, $R^6$, and $R^7$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy,
(e) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, and $C_{1-6}$ alkoxy,
(f) $C_{1-6}$ alkoxy which may be optionally substituted with the same or different 1 to 3 substituents selected from the group consisting of halogen atom, hydroxy, and $C_{1-6}$ alkoxy,
(g) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,
(h) 5- or 6-membered heteroaryl which may be optionally substituted with the same or different 1 to 4 substituents selected from the group consisting of halogen atom, cyano, and $C_{1-6}$ alkyl,
(i) 4- to 7-membered saturated or partially-unsaturated heterocyclyl which may be optionally substituted with the same or different 1 to 4 substituents selected from the group consisting of halogen atom, hydroxy, $C_{1-6}$ alkyl, and $C_{1-6}$ alkoxy,
(j) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,
(k) $C_{2-7}$ alkylcarbonyl which may be optionally substituted with the same or different 1 to 3 halogen atoms, or
(l) $C_{2-7}$ alkoxycarbonyl which may be optionally substituted with the same or different 1 to 3 halogen atom,

wherein $R^5$ and $R^6$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom, oxo, $C_{1-6}$ alkyl, $C_{1-6}$ alkoxy, and $C_{2-7}$ alkoxycarbonyl,
i is 0, 1, or 2, and
the substitutable carbon atom on the ring of formula (2c) may have one fluorine atom as a substituent,

(2-3) a group of the following formula (2d), (2e), (2f), (2g), (2h), (2i), or (2j):

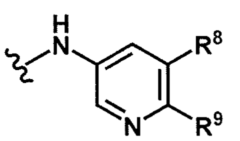
(2d)

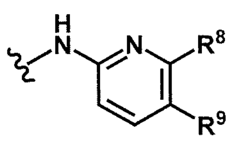
(2e)

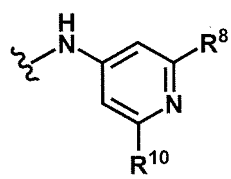
(2f)

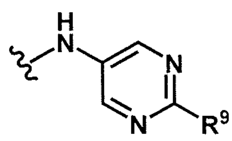
(2g)

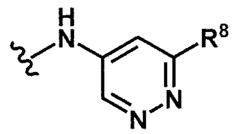
(2h)

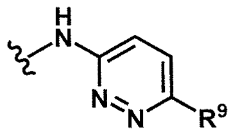
(2i)

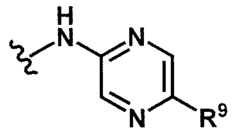
(2j)

wherein

$R^8$, $R^9$, and $R^{10}$ are independently

(a) hydrogen atom,

(b) halogen atom,
(c) cyano,
(d) $C_{1-6}$ alkyl which may be optionally substituted with the same or different 1 to 3 substituents selected from the group consisting of halogen atom; hydroxy; saturated or partially-unsaturated $C_{3-7}$ carbocyclyl; $C_{1-6}$ alkoxy which may be optionally substituted with hydroxy or $C_{1-6}$ alkoxy; 4- to 7-membered saturated or partially-unsaturated heterocyclyl which may be optionally substituted with $C_{1-6}$ alkoxy or $C_{1-6}$ alkyl; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),
(e) $C_{3-6}$ cycloalkyl which may be optionally substituted with the same or different 1 to 3 halogen atoms,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, $C_{1-6}$ alkoxy, and amino which may be optionally substituted with the same or different 1 to 2 $C_{1-6}$ alkyl,
(g) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl,
(h) amino which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(i) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,
(j) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,
(k) 4- to 7-membered saturated or partially-unsaturated heterocyclyloxy which may be optionally substituted with the same or different 1 to 4 $C_{1-6}$ alkyl,
(l) -C(O)NR$^x$R$^y$, wherein R$^x$ and R$^y$ are independently hydrogen atom, $C_{1-6}$ alkyl, or saturated or partially-unsaturated $C_{3-7}$ carbocyclyl; or R$^x$ and R$^y$ may be taken together with the nitrogen atom to which they are attached to form 4- to 7-membered saturated heterocyclic ring,
(m) -C(O)OR$^Z$, wherein R$^Z$ is $C_{1-6}$ alkyl, or
(n) ethenyl which may be substituted with one 6-membered saturated heterocyclyl,

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl, and
the substitutable carbon atom on the ring of formula (2d), (2e), (2f), (2g), (2h), (2i), or (2j) may have one fluorine atom as a substituent,

(2-4) a group of the following formula (2k):

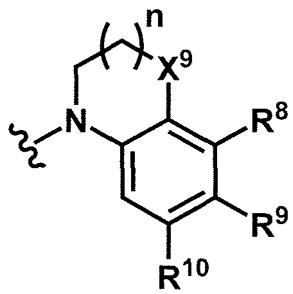

(2k)

wherein

$R^8$, $R^9$, and $R^{10}$ are as defined in the above (2-3),
n is 0, 1, or 2,
$X^9$ is $CH_2$ or O, and
the substitutable carbon atom on the ring of formula (2k) may have one fluorine atom as a substituent,

(2-5) a group of the following formula (21), (2m), or (2n) :

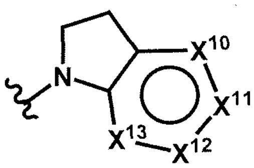

(2l)

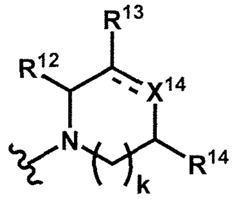

(2m)

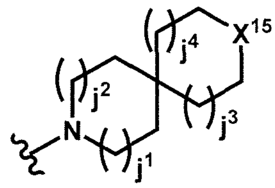

(2n)

wherein

$X^{10}$, $X^{11}$, $X^{12}$, and $X^{13}$ are independently N or $CR^{11}$, wherein $X^{10}$, $X^{11}$, $X^{12}$, and $X^{13}$ are selected so that the 6-membered ring comprising them can be a heteroaromatic ring,
$X^{14}$ is $CR^{15}$, $CHR^{15}$, $NR^{16}$, or O,
provided that when $X^{14}$ is $CR^{15}$, the bond having a broken line in formula (2m) is a double bond, or when $X^{14}$ is $CHR^{15}$, $NR^{16}$, or O, the bond having a broken line in formula (2m) is a single bond,
$X^{15}$ is $NR^{17}$ or O,
$R^{11}$ is independently

(a) hydrogen atom,
(b) halogen atom,
(c) 5- or 6-membered heteroaryl which may be optionally substituted with the same or different 1 to 4 substituents selected from the group consisting of halogen atom, cyano, $C_{1-6}$ alkyl, and $C_{1-6}$ alkoxy,
(d) 5- or 6-membered heteroaryl-methyl which may be optionally substituted with the same or different 1 to 4 substituents selected from the group consisting of halogen atom, cyano, $C_{1-6}$ alkyl, and $C_{1-6}$ alkoxy, or
(e) 4- to 7-membered saturated or partially-unsaturated heterocyclyl which may be optionally substituted with the same or different 1 to 4 substituents selected from the group consisting of $C_{1-6}$ alkyl, $C_{1-6}$ alkoxy, $C_{2-7}$ alkylcarbonyl, and $C_{2-7}$ alkoxycarbonyl,

provided that there are plural $R^{11}$, each $R^{11}$ may be the same or different,
$R^{12}$, $R^{13}$, and $R^{14}$ are independently

(a) hydrogen atom, or

(b) $C_{1-6}$ alkyl,

wherein $R^{12}$ and $R^{14}$, or $R^{13}$ and $R^{14}$ may be taken together with the carbon atoms to which they attach to form a bridged structure,
$R^{15}$ is

(a) phenyl which may be optionally substituted with the same or different 1 to 2 substituents selected from the group consisting of halogen atom, cyano, $C_{1-6}$ alkyl, and $C_{1-6}$ alkoxy,
(b) benzyl which may be optionally substituted with the same or different 1 to 2 substituents selected from the group consisting of halogen atom, cyano, $C_{1-6}$ alkyl, and $C_{1-6}$ alkoxy,
(c) 5- to 10-membered heteroaryl which may be optionally substituted with the same or different 1 to 2 substituents selected from the group consisting of halogen atom, cyano, $C_{1-6}$ alkyl, and $C_{1-6}$ alkoxy,
(d) hydroxy,
(e) phenyloxy which may be optionally substituted with the same or different 1 to 2 substituents selected from the group consisting of halogen atom, cyano, $C_{1-6}$ alkyl, and $C_{1-6}$ alkoxy, or
(f) phenylamino which may be optionally substituted with the same or different 1 to 2 substituents selected from the group consisting of halogen atom, cyano, $C_{1-6}$ alkyl, and $C_{1-6}$ alkoxy,

$R^{16}$ is

(a) phenyl which may be optionally substituted with the same or different 1 to 2 substituents selected from the group consisting of halogen atom, cyano, $C_{1-6}$ alkyl, and $C_{1-6}$ alkoxy,
(b) 5- or 6-membered heteroaryl which may be optionally substituted with the same or different 1 to 2 substituents selected from the group consisting of halogen atom, cyano, $C_{1-6}$ alkyl which may be optionally substituted with 1 to 3 fluorine atoms, and $C_{1-6}$ alkoxy which may be optionally substituted with 1 to 3 fluorine atoms,
(c) 5- or 6-membered heteroarylmethyl which may be optionally substituted with the same or different 1 to 2 substituents selected from the group consisting of halogen atom, cyano, $C_{1-6}$ alkyl, and $C_{1-6}$ alkoxy,
(d) 5- or 6-membered saturated or partially-unsaturated carbocyclyl which may be optionally substituted with the same or different 1 to 3 halogen atoms, or
(e) 5- or 6-membered saturated heterocyclyl which may be optionally substituted with the same or different 1 to 3 halogen atoms,

$R^{17}$ is

(a) pyridyl which may be optionally substituted with the same or different 1 to 2 substituents selected from the group consisting of halogen atom, cyano, $C_{1-6}$ alkyl, and $C_{1-6}$ alkoxy, or
(b) 5- or 6-membered saturated heterocyclyl which may be optionally substituted with the same or different 1 to 3 halogen atoms,

k is 0, 1, or 2, and
$j^1$, $j^2$, $j^3$, and $j^4$ are independently 0 or 1,

(2-6) a group of the following formula (2o):

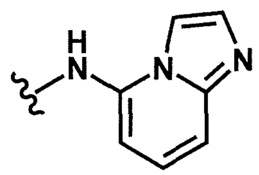

(2o) ,

or
(2-7) a group of the following formula (2p) or (2q):

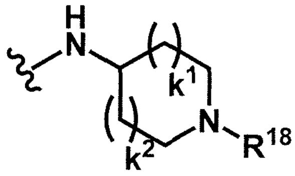 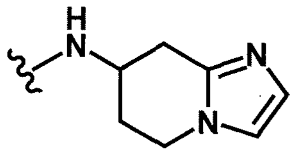

(2p) (2q)

wherein

$R^{18}$ is

(a) phenyl which may be optionally substituted with the same or different 1 to 2 substituents selected from the group consisting of halogen atom, cyano, $C_{1-6}$ alkyl, and $C_{1-6}$ alkoxy, or
(b) benzyl which may be optionally substituted with the same or different 1 to 2 substituents selected from the group consisting of halogen atom, cyano, $C_{1-6}$ alkyl, and $C_{1-6}$ alkoxy,

$k^1$ and $k^2$ are independently 0 or 1,
wherein the nitrogen-containing saturated ring in formula (2p) may be optionally substituted with oxo; and Ring Cy is an optionally-substituted 5- to 10-membered heteroarylene.

**6.** The medicament of claim 5, wherein

Ring Cy is a group of formula (a):

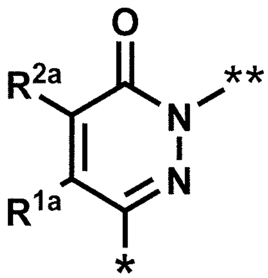

(a)

wherein
* is binding point to $M^1$, and ** is binding point to $CH_2C(=O)M^2$,

$R^{1a}$ and $R^{2a}$ are independently

(3-1) hydrogen atom,
(3-2) halogen atom,
(3-3) cyano,
(3-4) $C_{1-6}$ alkyl which may be optionally substituted with the same or different 1 to 3 substituents selected from the group consisting of

(a) halogen atom,
(b) hydroxy,
(c) saturated or partially-unsaturated $C_{3-7}$ carbocyclyl,
(d) $C_{1-6}$ alkoxy, and
(e) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,

(3-5) saturated or partially-unsaturated $C_{3-7}$ carbocyclyl which may be optionally substituted with the

same or different 1 to 4 substituents selected from the group consisting of halogen atom, hydroxy, $C_{1-6}$ alkyl, and $C_{1-6}$ alkoxy,
(3-6) $C_{2-6}$ alkenyl which may be optionally substituted with the same or different 1 to 4 halogen atoms,
(3-7) $C_{1-6}$ alkoxy which may be optionally substituted with the same or different 1 to 3 substituents selected from the group consisting of halogen atom, hydroxy, saturated or partially-unsaturated $C_{3-7}$ carbocyclyl, and $C_{1-6}$ alkoxy, or
(3-8) amino which may be optionally substituted with the same or different 1 to 2 substituents selected from the group consisting of $C_{1-6}$ alkyl which may be optionally substituted with the same or different 1 to 3 halogen atoms, saturated or partially-unsaturated $C_{3-7}$ carbocyclyl, and $C_{2-7}$ alkylcarbonyl; or

$R^{1a}$ and $R^{2a}$ may be taken together with the carbon atoms to which they are attached to form

(4-1) 5- to 7-membered saturated or partially-unsaturated carbon ring which may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy, or

(4-2) 5 - to 7-membered saturated or partially-unsaturated hetero ring which may be optionally substituted with the same or different 1 to 4 substituents selected from the group consisting of the above (a) - (d) in (4-1), or a group of formula (b):

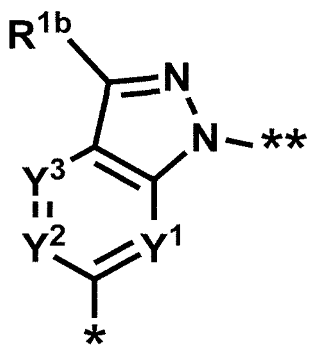

(b)

wherein
* is binding point to $M^1$, and ** is binding point to $CH_2C(=O)M^2$,

$Y^1$, $Y^2$, and $Y^3$ are independently N or $CR^{2b}$;
$R^{1b}$ is hydrogen atom, halogen atom, cyano, $C_{1-6}$ alkyl which may be optionally substituted with the same or different 1 to 3 halogen atoms, $C_{3-6}$ cycloalkyl which may be optionally substituted with the same or different 1 to 3 halogen atoms, $C_{1-6}$ alkoxy which may be optionally substituted with the same or different 1 to 3 halogen atoms, or amino which may be optionally substituted with the same or different 1 to 2 $C_{1-6}$ alkyl;
$R^{2b}$ is, independently if there are plural $R^{2b}$, hydrogen atom, halogen atom, cyano, $C_{1-6}$ alkyl which may be optionally substituted with the same or different 1 to 3 halogen atoms, $C_{3-6}$ cycloalkyl which may be optionally substituted with the same or different 1 to 3 halogen atoms, $C_{1-6}$ alkoxy which may be optionally substituted with the same or different 1 to 3 halogen atoms, or amino which may be optionally substituted with the same or different 1 to 2 $C_{1-6}$ alkyl, provided that when there are plural $R^{2b}$, each $R^{2b}$ may be the same or different.

**7.** The medicament of claim 5 or 6, wherein

$M^2$ is

(1) a group of any one of the following formulae (2a-1) - (2a-23) and (2b-1) - (2b-11):

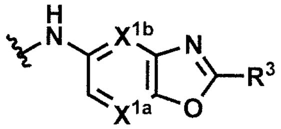
(2a-1)

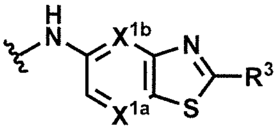
(2a-2)

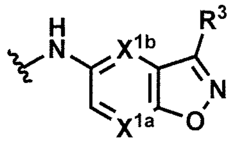
(2a-3)

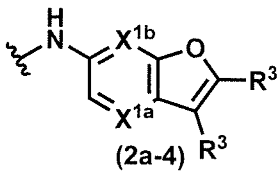
(2a-4)

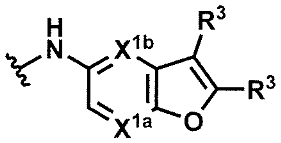
(2a-5)

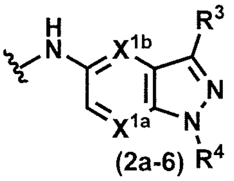
(2a-6)

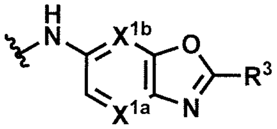
(2a-7)

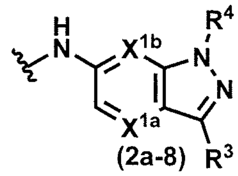
(2a-8)

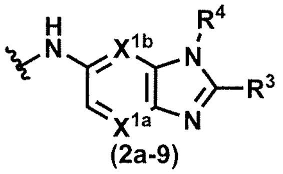
(2a-9)

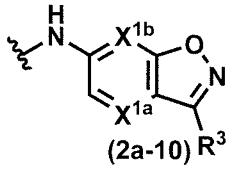
(2a-10)

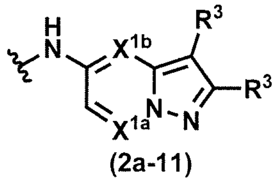
(2a-11)

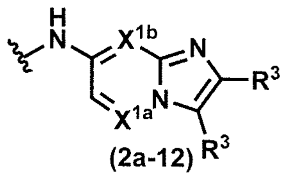
(2a-12)

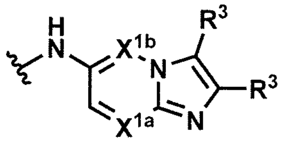
(2a-13)

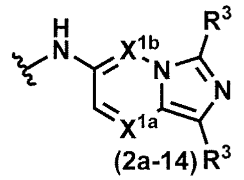
(2a-14)

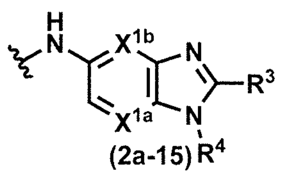
(2a-15)

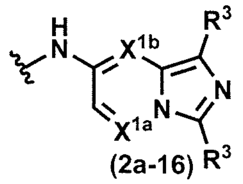
(2a-16)

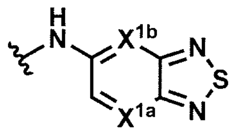
(2a-17)

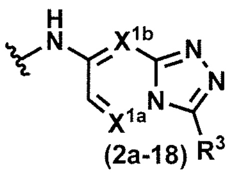
(2a-18)

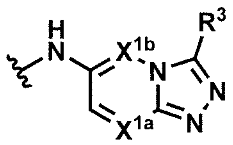
(2a-19)

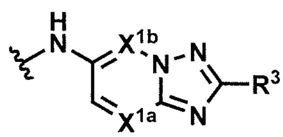
(2a-20)

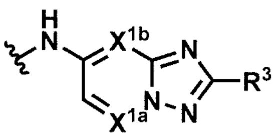
(2a-21)

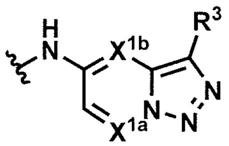
(2a-22)

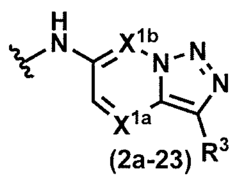
(2a-23)

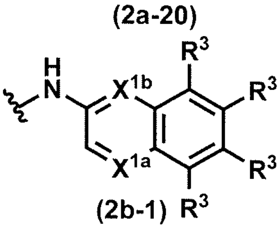
(2b-1)

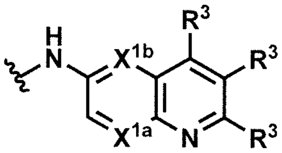
(2b-2)

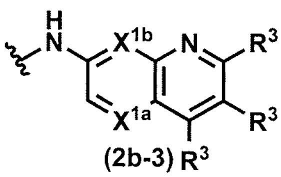
(2b-3)

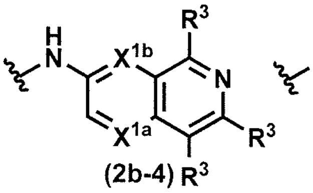
(2b-4)

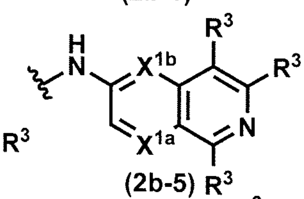
(2b-5)

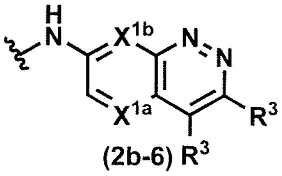
(2b-6)

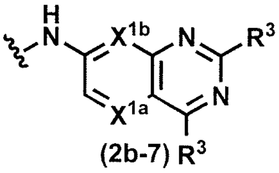
(2b-7)

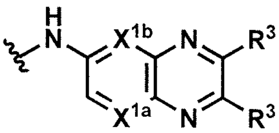
(2b-8)

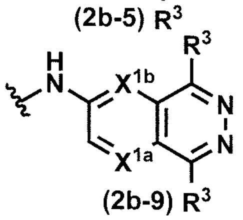
(2b-9)

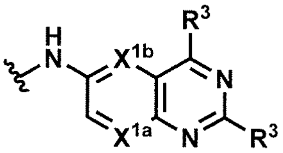
(2b-10)

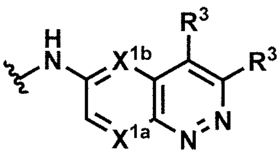
(2b-11)

wherein $X^{1a}$, $X^{1b}$, $R^3$, and $R^4$ are as defined in claim 5,
(2) a group of the following (2c'):

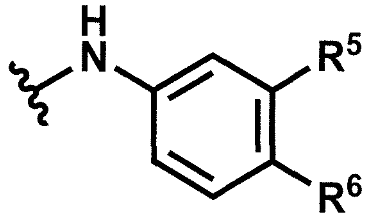

(2c')

wherein

$R^5$ and $R^6$ are independently

(a) hydrogen atom,
(b) halogen atom,
(c) cyano,
(d) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,
(e) 5- or 6-membered heteroaryl which may be optionally substituted with the same or different 1 to 4 substituents selected from the group consisting of halogen atom, cyano, and $C_{1-6}$ alkyl,
(f) 4- to 7-membered saturated or partially-unsaturated heterocyclyl which may be optionally substituted with the same or different 1 to 4 substituents selected from the group consisting of halogen atom, hydroxy, $C_{1-6}$ alkyl, and $C_{1-6}$ alkoxy, or
(g) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring, or

$R^5$ and $R^6$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom, oxo, $C_{1-6}$ alkyl, $C_{1-6}$ alkoxy, and $C_{2-7}$ alkoxycarbonyl,
the substitutable carbon atom on the ring of formula (2c') may have one fluorine atom as a substituent,

(3) a group of the following formula (2d), (2f), (2g) , or (2h):

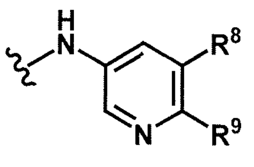

(2d)

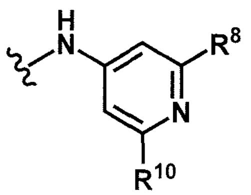

(2f)

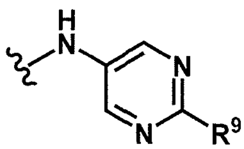

(2g)

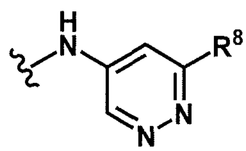

(2h)

wherein

$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected

from the group consisting of halogen atom; hydroxy; $C_{1-6}$ alkoxy which may be optionally substituted with hydroxy or $C_{1-6}$ alkoxy; 4-to 7-membered saturated or partially-unsaturated heterocyclyl which may be optionally substituted with $C_{1-6}$ alkyl or $C_{1-6}$ alkoxy; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),

(e) $C_{1-6}$ alkoxy which may be optionally substituted with the same or different 1 to 3 substituents selected from the group consisting of halogen atom, hydroxy, and $C_{1-6}$ alkoxy,

(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,

(g) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,

(h) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,

(i) 4- to 7-membered saturated or partially-unsaturated heterocyclyloxy which may be optionally substituted with the same or different 1 to 4 $C_{1-6}$ alkyl,

(j) -C(O) $NR^xR^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,

(k) -C(O) $OR^Z$, wherein $R^Z$ is $C_{1-6}$ alkyl, or

(l) ethenyl which may be substituted with one 6-membered saturated heterocyclyl,

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl, and

the substitutable carbon atom on the ring of formula (2d), (2f), (2g), or (2h) may have one fluorine atom as a substituent, or

(4) a group of formula (2k'):

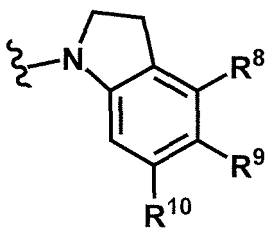

(2k')

wherein $R^8$, $R^9$, and $R^{10}$ are as defined in the above (3) .

**8.** The medicament of claim 5 or 6, wherein $M^2$ is a group of

(1) a group of the following formulae (2a-1), (2a-2), (2a-12), (2a-20), (2a-21), (2b-3), (2b-4), (2b-7), or (2b-10):

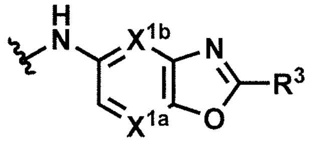
(2a-1)

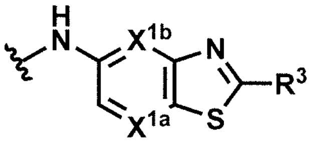
(2a-2)

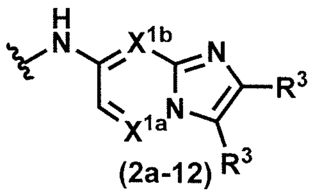
(2a-12)

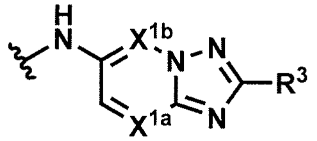
(2a-20)

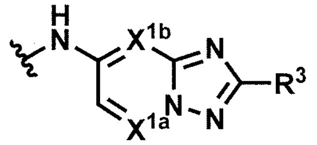
(2a-21)

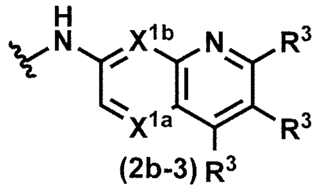
(2b-3)

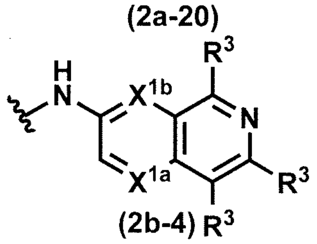
(2b-4)

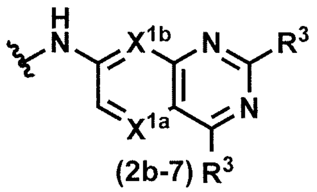
(2b-7)

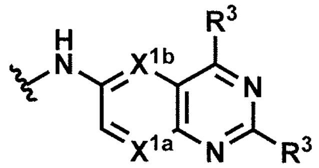
(2b-10)

wherein $X^{1a}$, $X^{1b}$, and $R^3$ are as defined in claim 5,

(2) a group of the following formula (2c'):

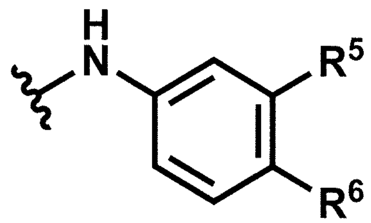
(2c')

wherein

$R^5$ and $R^6$ are independently

(a) hydrogen atom,
(b) halogen atom,
(c) cyano, or
(d) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl,

the substitutable carbon atom on the ring of formula (2c') may have one fluorine atom as a substituent,

(3) a group of the following formula (2d) or (2f):

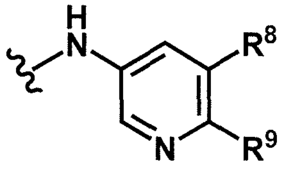

(2d)

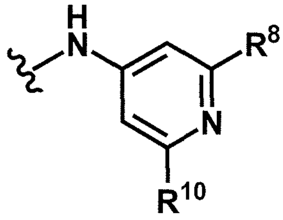

(2f)

wherein $R^8$, $R^9$, and $R^{10}$ are as defined in claim 7,
the substitutable carbon atom on the ring of formula (2d) or (2f) may have one fluorine atom as a substituent, or
(4) a group of the following formula (2k"):

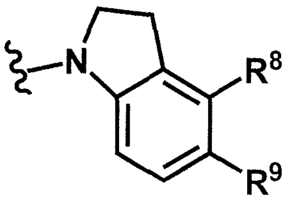

(2k")

wherein $R^8$ and $R^9$ are as defined in claim 7.

**9.** The medicament of any one of claims 5 to 8, wherein
$M^1$ is

(1) saturated or partially-unsaturated $C_{4-12}$ carbocyclyl which may be optionally substituted with the same or different 1 to 4 substituents selected from the group consisting of halogen atom; and $C_{1-6}$ alkyl which may be optionally substituted with the same or different 1 to 3 substituents selected from the group consisting of halogen atom, hydroxy, and $C_{1-6}$ alkoxy,
(2) 4- to 12-membered saturated or partially-unsaturated heterocyclyl which may be optionally substituted with the same or different 1 to 4 substituents selected from the group consisting of halogen atom; and $C_{1-6}$ alkyl which may be optionally substituted with the same or different 1 to 3 substituents selected from the group consisting of halogen atom, hydroxy, and $C_{1-6}$ alkoxy, or
(3) $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(d) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy.

**10.** The medicament of any one of claims 5 to 9, wherein
$M^2$ is a group of the following formula (2a-20), (2a-21), (2b-3), (2b-4), or (2b-7):

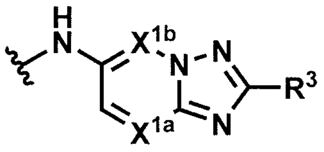

(2a-20)

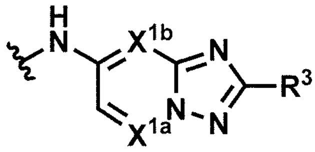

(2a-21)

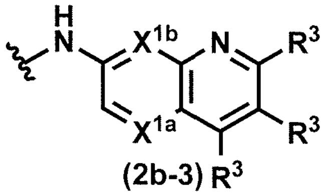

(2b-3)

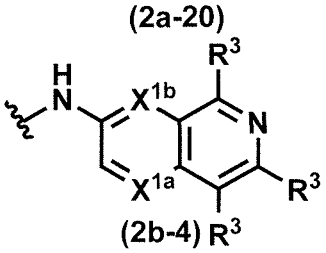

(2b-4)

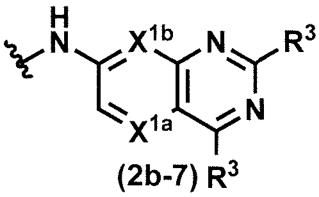

(2b-7)

wherein

$X^{1a}$ and $X^{1b}$ are independently N or $CR^3$;
$R^3$ is independently

(a) hydrogen atom,
(b) halogen atom,
(c) cyano,
(d) hydroxy,
(e) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, $C_{1-6}$ alkoxy, 4- to 7-membered saturated heterocyclyl which may be optionally substituted with $C_{1-6}$ alkoxy, and amino which may be optionally substituted with the same or different 1 to 2 $C_{1-6}$ alkyl,
(f) saturated or partially-unsaturated $C_{3-7}$ carbocyclyl which may be optionally substituted with the same or different 1 to 4 substituents selected from the group consisting of halogen atom, hydroxy, $C_{1-6}$ alkyl, $C_{1-6}$ alkoxy, and amino which may be optionally substituted with the same or different 1 to 2 $C_{1-6}$ alkyl,
(g) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl, or
(h) amino which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,

provided that when there are plural $R^3$, each $R^3$ may be the same or different.

**11.** The medicament of any one of claims 5 to 9, wherein
$M^2$ is a group of the following formula (2a'-20), (2a'-21), (2b'-3), (2b'-4), or (2b'-7):

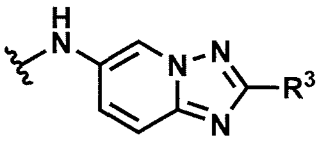

(2a'-20)

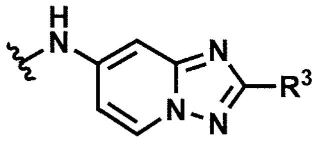

(2a'-21)

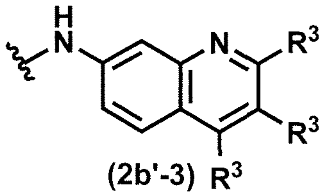

(2b'-3)

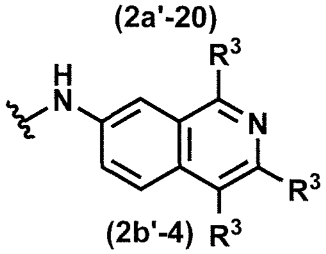

(2b'-4)

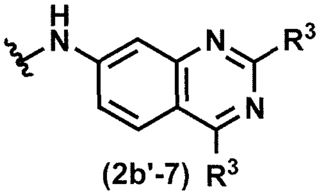

(2b'-7)

wherein

$R^3$ is independently

(a) hydrogen atom,
(b) halogen atom,
(c) cyano,
(d) $C_{1-6}$ alkyl which may be optionally substituted with the same or different 1 to 3 substituents selected from the group consisting of halogen atom, hydroxy, saturated or partially-unsaturated $C_{3-7}$ carbocyclyl, $C_{1-6}$ alkoxy, 4- to 7-membered saturated heterocyclyl which may be optionally substituted with $C_{1-6}$ alkoxy, and amino which may be optionally substituted with the same or different 1 to 2 $C_{1-6}$ alkyl,
(e) $C_{1-6}$ alkoxy which may be optionally substituted with the same or different 1 to 3 substituents selected from the group consisting of halogen atom, hydroxy, $C_{1-6}$ alkoxy, and amino which may be optionally substituted with the same or different 1 to 2 $C_{1-6}$ alkyl, or
(f) amino which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,

provided that when there are plural $R^3$, each $R^3$ may be the same or different.

**12.** The medicament of any one of claims 5 to 9, wherein
$M^2$ is a group of the following formula (2d) or (2f):

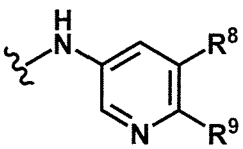

(2d)

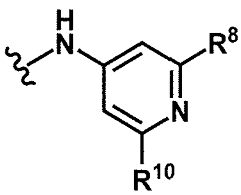

(2f)

wherein
$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy

or $C_{1-6}$ alkoxy; 4-to 7-membered saturated or partially-unsaturated heterocyclyl which may be optionally substituted with $C_{1-6}$ alkyl or $C_{1-6}$ alkoxy; 5- or 6-membered heteroaryl which may be optionally substituted with $C_{1-6}$ alkyl; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; saturated or partially-unsaturated $C_{3-7}$ carbocyclyl; 4- to 7-membered saturated heterocyclyl which may be optionally substituted with $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy),
(e) $C_{1-6}$ alkoxy which may be optionally substituted with the same or different 1 to 3 substituents selected from the group consisting of halogen atom, hydroxy, and $C_{1-6}$ alkoxy,
(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy,
(g) 5- or 6-membered heteroaryl which may be optionally substituted with the same or different 1 to 4 substituents selected from the group consisting of halogen atom, cyano, $C_{1-6}$ alkyl, $C_{1-6}$ alkoxy, and $C_{2-7}$ alkoxycarbonyl,
(h) 4- to 7-membered saturated or partially-unsaturated heterocyclyl which may be optionally substituted with the same or different 1 to 4 substituents selected from the group consisting of $C_{1-6}$ alkyl which may be optionally substituted with the same or different 1 to 3 substituents selected from the group consisting of halogen atom, hydroxy, and $C_{1-6}$ alkoxy; $C_{1-6}$ alkoxy; 4- to 7-membered saturated or partially-unsaturated heterocyclyl; $C_{2-7}$ alkylcarbonyl which may be optionally substituted with the same or different 1 to 3 substituents selected from the group consisting of halogen atom, hydroxy, and $C_{1-6}$ alkoxy; and oxo,
(i) 4- to 7-membered saturated or partially-unsaturated heterocyclyloxy which may be optionally substituted with the same or different 1 to 4 $C_{1-6}$ alkyl,
(j) -C(O)NR$^x$R$^y$, wherein $R^x$ and $R^y$ are independently hydrogen atom, $C_{1-6}$ alkyl, or saturated or partially-unsaturated $C_{3-7}$ carbocyclyl; or $R^x$ and $R^y$ may be taken together with the nitrogen atom to which they are attached to form 4- to 7-membered saturated heterocyclic ring,
(k) -C(O)OR$^Z$, wherein $R^Z$ is $C_{1-6}$ alkyl, or
(l) ethenyl which may be substituted with one 6-membered saturated heterocyclyl,

wherein $R^8$ and $R^9$ may be taken together with the carbon atoms to which they are attached to form 5- to 7-membered saturated or partially-unsaturated carbocyclic ring or heterocyclic ring, wherein the ring may be optionally substituted with the same or different 1 to 4 substituents selected from the group consisting of halogen atom and $C_{1-6}$ alkyl.

**13.** The medicament of claim 12, wherein
$R^8$, $R^9$, and $R^{10}$ are independently

(a) hydrogen atom,
(b) halogen atom,
(c) cyano,
(d) $C_{1-6}$ alkyl which may be optionally substituted with the same or different 1 to 3 substituents selected from the group consisting of halogen atom; hydroxy; $C_{1-6}$ alkoxy which may be optionally substituted with hydroxy or $C_{1-6}$ alkoxy; and amino (which may be optionally substituted with the same or different 1 to 2 substituents selected from the group consisting of $C_{1-6}$ alkyl which may be optionally substituted with the same or different 1 to 3 substituents selected from the group consisting of halogen atom, hydroxy, and $C_{1-6}$ alkoxy; and $C_{2-7}$ alkylcarbonyl which may be optionally substituted with the same or different 1 to 3 substituents selected from the group consisting of halogen atom, hydroxy, and $C_{1-6}$ alkoxy,
(e) $C_{1-6}$ alkoxy which may be optionally substituted with the same or different 1 to 3 substituents selected from the group consisting of halogen atom, hydroxy, and $C_{1-6}$ alkoxy, or
(f) amino which may be optionally substituted with the same or different 1 to 2 substituents selected from the group consisting of $C_{1-6}$ alkyl which may be optionally substituted with the same or different 1 to 3 halogen atoms; saturated or partially-unsaturated $C_{3-7}$ carbocyclyl; and $C_{2-7}$ alkylcarbonyl which may be optionally substituted with the same or different 1 to 3 substituents selected from the group consisting of halogen atom, hydroxy, and $C_{1-6}$ alkoxy.

**14.** The medicament of any one of claims 5 to 13, wherein
$M^1$ is a group of the following formula (3):

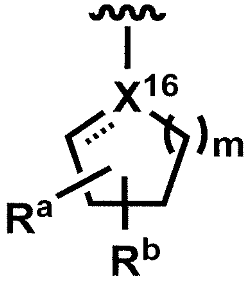

(3)

wherein

$X^{16}$ is N, C, or CH,
the bond having a broken line is a single bond or a double bond,
m is 0, 1, 2, or 3,
$R^a$ and $R^b$ are independently

(1-1) hydrogen atom,
(1-2) halogen atom, or
(1-3) $C_{1-6}$ alkyl which may be optionally substituted with the same or different 1 to 3 substituents selected from the group consisting of halogen atom, hydroxy, and $C_{1-6}$ alkoxy, or

$R^a$ and $R^b$ may be taken together with the carbon atom(s) to which they are attached to form 3- to 6-membered saturated carbocyclic ring, wherein the ring may be optionally substituted with the same or different 1 to 4 substituents selected from the group consisting of

(a) halogen atom,
(b) hydroxy,
(c) $C_{1-6}$ alkyl which may be optionally substituted with the same or different 1 to 3 substituents selected from the group consisting of halogen atom, hydroxy, and $C_{1-6}$ alkoxy, and
(d) $C_{1-6}$ alkoxy which may be optionally substituted with the same or different 1 to 3 substituents selected from the group consisting of halogen atom, hydroxy, and $C_{1-6}$ alkoxy.

**15.** The medicament of claim 14, wherein $X^{16}$ is C, and the bond having a broken line is a double bond.

**16.** The medicament of claim 14, wherein $X^{16}$ is CH, and the bond having a broken line is a single bond.

**17.** The medicament of claim 14, wherein $X^{16}$ is N, and the bond having a broken line is a single bond.

**18.** The medicament of any one of claims 5 to 13, wherein
$M^1$ is $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl,
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl,
(e) $C_{6-10}$ aryl which may be optionally substituted with the same or different 1 to 3 substituents selected from the group consisting of halogen atom, cyano, $C_{1-6}$ alkyl, and $C_{1-6}$ alkoxy, or
(f) 5- to 10-membered heteroaryl which may be optionally substituted with the same or different 1 to 3 substituents selected from the group consisting of halogen atom, cyano, $C_{1-6}$ alkyl, and $C_{1-6}$ alkoxy.

**19.** The medicament of any one of claims 5 to 13, wherein
$M^1$ is $-NR^eR^f$, wherein $R^e$ and $R^f$ are independently

(a) hydrogen atom,
(b) $C_{1-6}$ alkyl which may be optionally substituted with the same or different 1 to 3 halogen atoms,
(c) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or
(d) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl.

**20.** The medicament of any one of claims 5 to 13, wherein
$M^1$ is $-NR^eR^f$, wherein

$R^e$ is methyl, and
$R^f$ is

(a) $C_{1-6}$ alkyl which may be optionally substituted with the same or different 1 to 3 halogen atoms,
(b) $C_{3-10}$ cycloalkyl which may be optionally substituted with the same or different 1 to 3 substituents selected from the group consisting of halogen atom, $C_{1-6}$ alkyl, and $C_{3-6}$ cycloalkyl, or
(c) $C_{3-10}$ cycloalkyl-$C_{1-4}$ alkyl which may be optionally substituted with the same or different 1 to 3 substituents selected from the group consisting of halogen atom, $C_{1-6}$ alkyl, and $C_{3-6}$ cycloalkyl.

**21.** The medicament of any one of claims 5 to 13, wherein
$M^1$ is a group of any one of the following formulae (3a-1) - (3a-4):

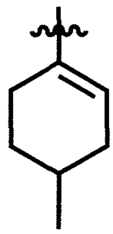
(3a-1)

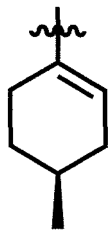
(3a-2)

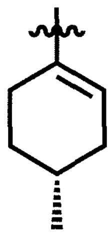
(3a-3)

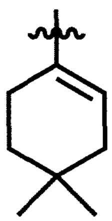
(3a-4) .

**22.** The medicament of any one of claims 5 to 13, wherein
$M^1$ is a group of any one of the following formulae (3c-1) - (3c-6):

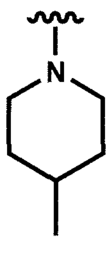
(3c-1)

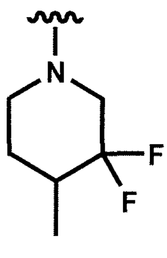
(3c-2)

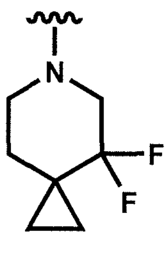
(3c-3)

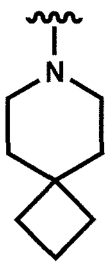
(3c-4)

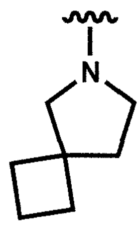
(3c-5)

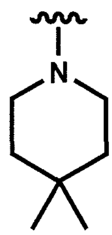
(3c-6) .

**23.** The medicament of any one of claims 5 to 13, wherein
M is a group of any one of the following formulae (3d-1) - (3d-12):

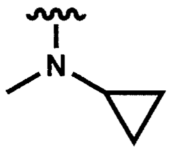
(3d-1)
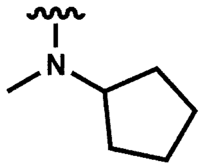
(3d-2)
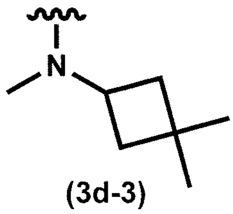
(3d-3)
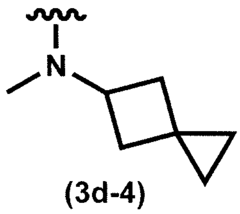
(3d-4)
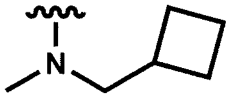
(3d-5)
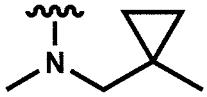
(3d-6)
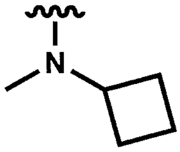
(3d-7)
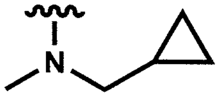
(3d-8)
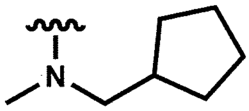
(3d-9)
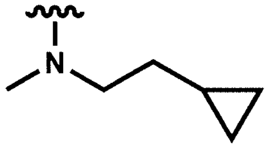
(3d-10)
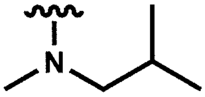
(3d-11)
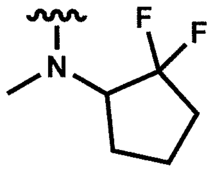
(3d-12) .

**24.** The medicament of any one of claims 6 to 23, wherein Ring Cy is formula (a).

**25.** The medicament of claim 24, wherein $R^{1a}$ and $R^{2a}$ are independently hydrogen atom, halogen atom, cyano, methyl, or methoxy.

**26.** The medicament of claim 24, wherein $R^{1a}$ and $R^{2a}$ are hydrogen atom.

**27.** The medicament of any one of claims 6 to 23, wherein Ring Cy is formula (b).

**28.** The medicament of claim 27, wherein $Y^1$ is N, and $Y^2$ and $Y^3$ are independently $CR^{2b}$.

**29.** The medicament of claim 27, wherein $Y^1$ and $Y^2$ are N, and $Y^3$ is $CR^{2b}$.

**30.** The medicament of claim 27, wherein $Y^1$ and $Y^3$ are N, and $Y^2$ is $CR^{2b}$.

**31.** The medicament of any one of claims 27 to 30, wherein
$R^{1b}$ and $R^{2b}$ are independently hydrogen atom, halogen atom, cyano, $C_{1-6}$ alkyl, $C_{3-6}$ cycloalkyl, or $C_{1-6}$ alkoxy, provided that when there are plural $R^{2b}$, each $R^{2b}$ may be the same or different.

**32.** The medicament of any one of claims 27 to 30, wherein $R^{1b}$ and $R^{2b}$ are hydrogen atom.

**33.** The medicament of claim 5, wherein the Nav1.1 activator is any one compound selected from the group consisting of the following compounds:

N-(4-cyanophenyl)-2-[3-(4-methylpiperidin-1-yl)-6-oxopyridazin-1(6H)-yl]acetamide,
N-(1,3-benzoxazol-5-yl)-2-[3-(4-methylpiperidin-1-yl)-6-oxopyridazin-1(6H)-yl]acetamide,
2-[3-(6-azaspiro[3.4]octan-6-yl)-6-oxopyridazin-1(6H)-yl]-N-(quinazolin-7-yl)acetamide,
N-[2-(dimethylamino)-1,3-benzoxazol-5-yl]-2-[3-(4-methylcyclohex-1-ene)-6-oxopyridazin-1(6H)-yl]acetamide,
N-(2,6-dimethylpyridin-4-yl)-2-[3-(4-methylcyclohex-1-en-1-yl)-6-oxopyridazin-1(6H)-yl]acetamide,
6-(4-methylcyclohex-1-en-1-yl)-2-{2-[4-(2-methylpyridin-3-yl) piperazin-1-yl]-2-oxoethyl}pyridazin-3(2H)-one,
2-[3-(4,4-dimethylcyclohex-1-en-1-yl)-6-oxopyridazin-1(6H)-yl]-N-([1,2,4]triazolo[1,5-a]pyridin-6-yl)acetamide,
2-[3-(4,4-dimethylcyclohex-1-en-1-yl)-6-oxopyridazin-1(6H)-yl]-N-([1,2,4]triazolo[1,5-a]pyridin-7-yl)acetamide,
N-(1,3-benzoxazol-5-yl)-2-[3-(4,4-dimethylcyclohex-1-en-1-yl)-6-oxopyridazin-1(6H)-yl]acetamide,
2-[6-oxo-3-(spiro[2.5]oct-5-en-6-yl)pyridazin-1(6H)-yl]-N-([1,2,4]triazolo[1,5-a]pyridin-7-yl)acetamide,

2-{2-oxo-2-[4-(pyridazin-4-yl)-2.3-dihydro-1H-indol-1-yl]ethyl}-6-(spiro[2.5]oct-5-en-6-yl) pyridazin-3(2H)-one,
N-(1,3-benzoxazol-5-yl) -2-[3-(4-methylcyclohex-1-en-1-yl) -6-oxopyridazin-1(6H)-yl]acetamide,
2-{3-[(4S)-4-methylcyclohex-1-en-1-yl]-6-oxopyridazin-1(6H)-yl}-N-([1,2,4]triazolo[1,5-a]pyridin-7-yl)acetamide,
2-{3-[(4R)-4-methylcyclohex-1-en-1-yl]-6-oxopyridazin-1(6H)-yl}-N-([1,2,4]triazolo[1,5-a]pyridin-7-yl)acetamide,
2-{3-[(4S)-4-methylcyclohex-1-en-1-yl]-6-oxopyridazin-1(6H)-yl}-N-([1,2,4]triazolo[1,5-a]pyridin-6-yl)acetamide,
2-{3-[(4R)-4-methylcyclohex-1-en-1-yl]-6-oxopyridazin-1(6H)-yl}-N-([1,2,4]triazolo[1,5-a]pyridin-6-yl)acetamide,
N-(6-cyanopyridin-3-yl)-2-[3-(4,4-difluoro-6-azaspiro[2.5]octan-6-yl)-6-oxopyridazin-1(6H)-yl]acetamide, and
6-[cyclopentyl(methyl)amino]-2-{2-[4-(morpholin-4-yl)-2,3-dihydro-1H-indol-1-yl]-2-oxoethyl}pyridazin-3(2H)-one.

**34.** The medicament of claim 5, wherein the Nav1.1 activator is any one compound selected from the group consisting of the following compounds:

2-{6-[cyclopentyl(methyl)amino]-1H-pyrazolo[3,4-b]pyrazin-1-yl}-N-([1,2,4]triazolo[1,5-a]pyridin-7-yl)acetamide,
2-[6-(4-methylcyclohex-1-en-1-yl)-1H-pyrazolo[3,4-b]pyrazin-1-yl]-N-([1,2,4]triazolo[1,5-a]pyridin-7-yl)acetamide,
2-[6-(4,4-difluoro-6-azaspiro[2.5]octan-6-yl)-1H-pyrazolo[3,4-b]pyrazin-1-yl]-N-([1,2,4]triazolo[1,5-a]pyridin-7-yl)acetamide,
2-{6-[methyl(spiro[2.3]hexan-5-yl)amino]-1H-pyrazolo[3,4-b]pyrazin-1-yl}-N-([1,2,4]triazolo[1,5-a]pyridin-7-yl)acetamide,
2-{6-[(3,3-dimethylcyclobutyl)(methyl)amino]-1H-pyrazolo[3,4-d]pyrazin-1-yl}-N-([1,2,4]triazolo[1,5-a]pyridin-7-yl)acetamide,
2-{6-[(3,3-dimethylcyclobutyl)(methyl)amino]-1H-pyrazolo[3,4-d]pyrazin-1-yl}-N-([1,2,4]triazolo[1,5-a]pyridin-6-yl)acetamide,
N-(6-cyanopyridin-3-yl)-2-[6-(4,4-difluoro-6-azaspiro[2.5]octan-6-yl)-1H-pyrazolo[3,4-b]pyrazin-1-yl]acetamide,
N-(6-cyanopyridin-3-yl)-2-{6-[methyl(spiro[2.3]hexan-5-yl)amino]-1H-pyrazolo[3,4-b]pyrazin-1-yl}acetamide,
2-{6-[cyclopentyl(methyl)amino]-1H-pyrazolo[3,4-d]pyrimidin-1-yl}-N-([1,2,4]triazolo[1,5-a]pyridin-7-yl)acetamide,
2-{6-[(cyclobutylmethyl)(methyl)amino]-1H-pyrazolo[3,4-d]pyrimidin-1-yl}-N-([1,2,4]triazolo[1,5-a]pyridin-6-yl)acetamide,
2-{6-[(3,3-dimethylcyclobutyl)(methyl)amino]-1H-pyrazolo[3,4-d]pyrimidin-1-yl}-N-([1,2,4]triazolo[1,5-a]pyridin-6-yl)acetamide, and
an optically active form of 2-[6-(4-methylcyclohex-1-en-1-yl)-1H-pyrazolo[3,4-b]pyrazin-1-yl]-N-([1,2,4]triazolo[1,5-a]pyridin-7-yl)acetamide.

**35.** The medicament of any one of claims 1 to 34, wherein
the tauopathy is Alzheimer's disease, Alzheimer-type dementia, diffuse neurofibrillary tangles with calcification, amyotrophic lateral sclerosis/parkinsonism-dementia complex of Guam island, amyotrophic lateral sclerosis/parkinsonism-dementia complex of the Kii peninsula, frontotemporal lobar degeneration (including Pick's disease, progressive supranuclear palsy, corticobasal degeneration, argyrophilic grain dementia, globular glial tauopathy, and frontotemporal dementia and parkinsonism linked to chromosome 17), senile dementia of the neurofibrillary tangle type, Down syndrome, chronic traumatic encephalopathy, myotonic dystrophy, Niemann-Pick disease type C, static encephalopathy of childhood with neurodegeneration in adulthood, PLA2G6-associated neurodegeneration, Gerstmann-Straussler-Scheinker disease, familial British dementia, familial Danish dementia, post-encephalitic Parkinsonism, subacute sclerosing panencephalitis, SLC9A6-related mental retardation, or a combination of any two or more of these diseases.

**36.** The medicament of any one of claims 1 to 34, wherein
the tauopathy is diffuse neurofibrillary tangles with calcification, amyotrophic lateral sclerosis/parkinsonism-dementia complex of Guam island, amyotrophic lateral sclerosis/parkinsonism-dementia complex of the Kii peninsula, frontotemporal lobar degeneration (including Pick's disease, progressive supranuclear palsy, corticobasal degeneration, argyrophilic grain dementia, globular glial tauopathy, and frontotemporal dementia and parkinsonism linked to chromosome 17), senile dementia of the neurofibrillary tangle type, Down syndrome, chronic traumatic encephalopathy, myotonic dystrophy, Niemann-Pick disease type C, static encephalopathy of childhood with neurodegeneration in adulthood, PLA2G6-associated neurodegeneration, Gerstmann-Straussler-Scheinker disease, familial British dementia, familial Danish dementia, post-encephalitic Parkinsonism, subacute sclerosing panencephalitis, SLC9A6-related mental retardation, or a combination of any two or more of these diseases.

**37.** The medicament of any one of claims 1 to 36, which can decrease phosphorylated tau aggregation and/or inhibit cerebral atrophy.

**38.** A method for decreasing phosphorylated tau aggregation and/or inhibiting cerebral atrophy, comprising administering a Nav activator.

**39.** A method for treating and/or preventing tauopathy, comprising administering a therapeutically effective amount of a Nav activator to a patient in need thereof.

**40.** Use of a Nav activator in the manufacture of a medicament for treating and/or preventing tauopathy.

**41.** A Nav activator in use for treating and/or preventing tauopathy.

**42.** A combination of the medicament of any one of claims 1 to 37, and at least one medicament selected from another medicament for treating tauopathy, a medicament for treating Alzheimer-type dementia, antiepileptic agent, antipsychotic agent, antidepressive agent, anti-anxiety agent, Chinese herbal drug, sleep-inducing drug, antiparkinsonian agent, antihypertensive drug, anti-inflammatory agent, antidiabetic drug, antihyperlipidemic drug, anti-obesity agent, antiemetic drug, a medicament for treating dysphagia, a medicament for treating dysuria, and cathartic drug.

**43.** The medicament of any one of claims 1 to 37, which is used in combination with at least one medicament selected from another medicament for treating tauopathy, a medicament for treating Alzheimer-type dementia, antiepileptic agent, antipsychotic agent, antidepressive agent, anti-anxiety agent, Chinese herbal drug, sleep-inducing drug, antiparkinsonian agent, antihypertensive drug, anti-inflammatory agent, antidiabetic drug, antihyperlipidemic drug, anti-obesity agent, antiemetic drug, a medicament for treating dysphagia, a medicament for treating dysuria, and cathartic drug.

**44.** A method for treating and/or preventing tauopathy, comprising administering a therapeutically effective amount of the medicament of any one of claims 1 to 37 in combination with at least one medicament selected from another medicament for treating tauopathy, a medicament for treating Alzheimer-type dementia, antiepileptic agent, antipsychotic agent, antidepressive agent, anti-anxiety agent, Chinese herbal drug, sleep-inducing drug, antiparkinsonian agent, antihypertensive drug, anti-inflammatory agent, antidiabetic drug, antihyperlipidemic drug, anti-obesity agent, antiemetic drug, a medicament for treating dysphagia, a medicament for treating dysuria, and cathartic drug, to a patient in need thereof.

Fig. 1

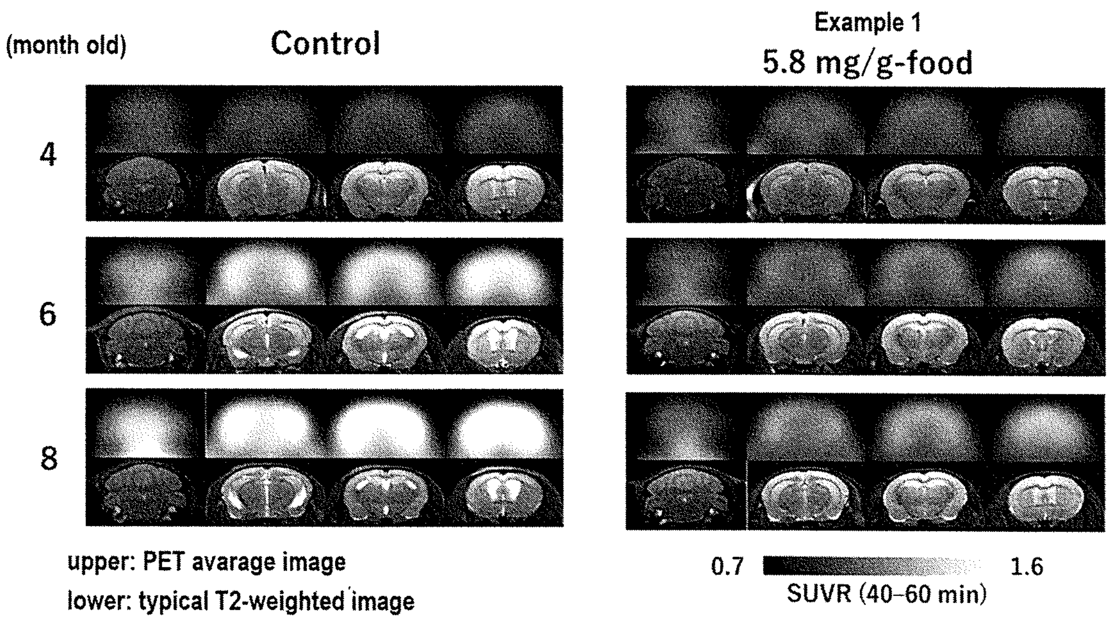


Fig. 2

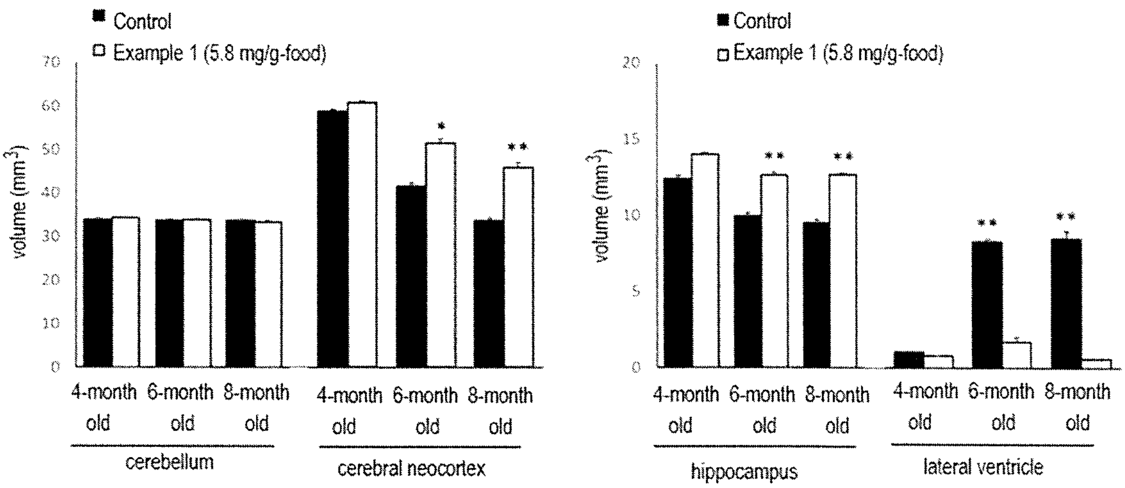


* p<0.05, ** p<0.01 vs. control; Sidak's multiple comparison test (n=6-11)

Fig. 3

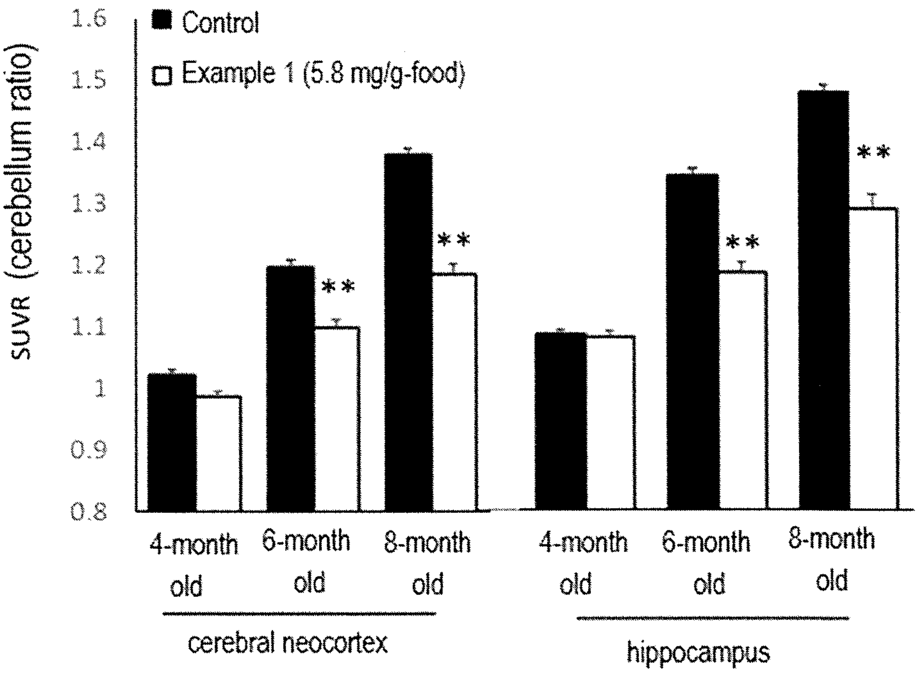


** p<0.01 vs. control; Sidak's multiple comparison test (n=6-11)

Fig. 4

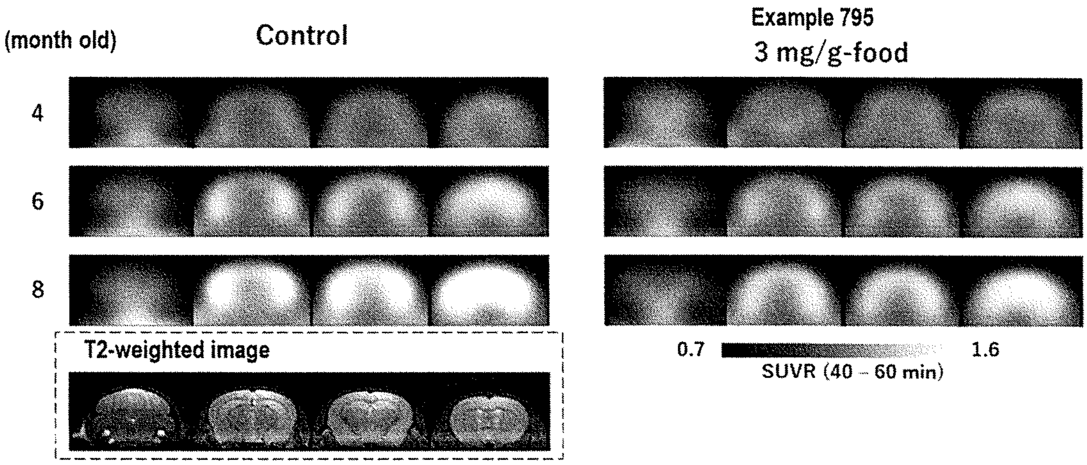

Fig. 5

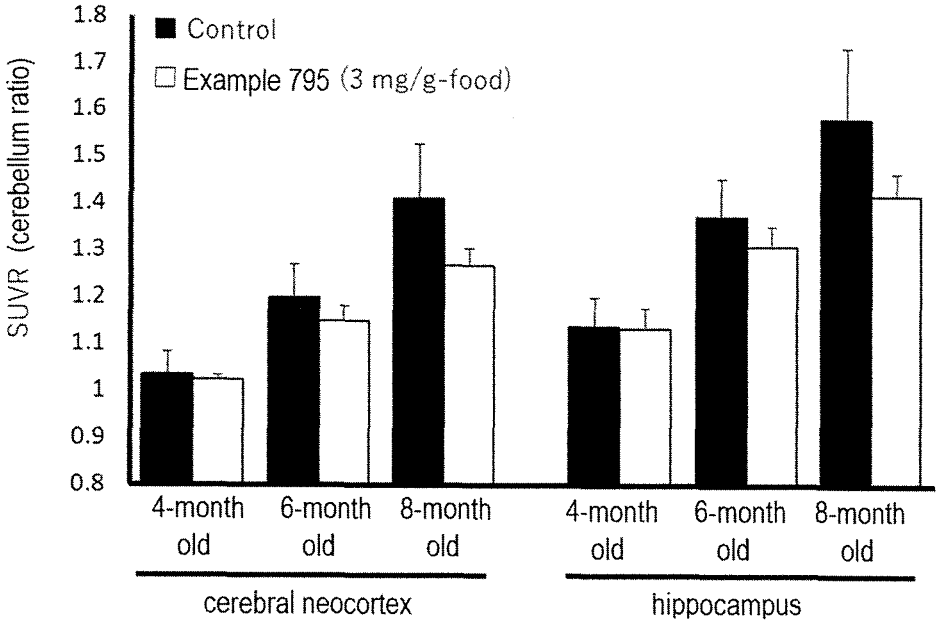
SUVR (cerebellum ratio)
1.8
1.7
1.6
1.5
1.4
1.3
1.2
1.1
1
0.9
0.8
Control
Example 795 (3 mg/g-food)
4-month old
6-month old
8-month old
4-month old
6-month old
8-month old
cerebral neocortex
hippocampus

Fig. 6

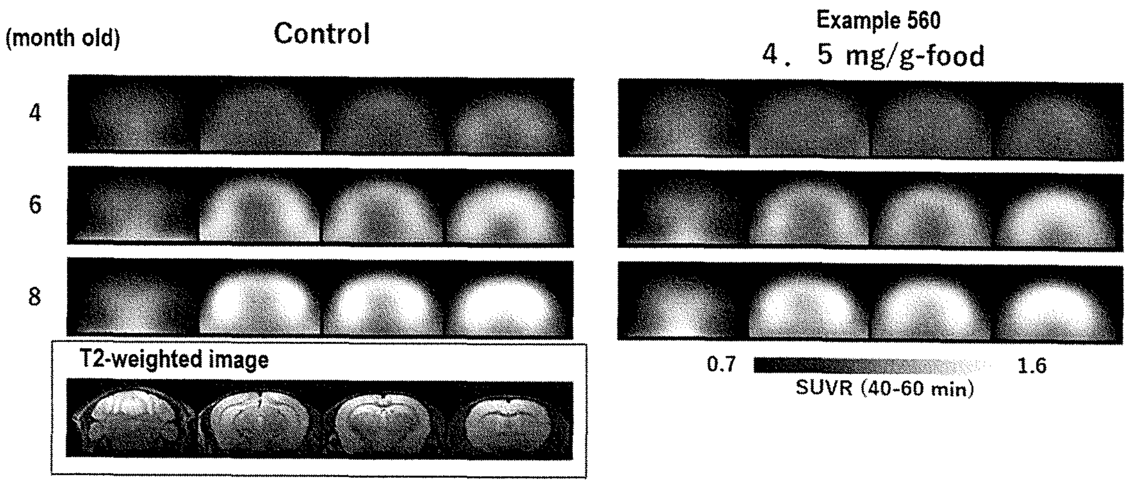
(month old)
Control
Example 560
4. 5 mg/g-food
4
6
8
T2-weighted image
0.7
1.6
SUVR (40-60 min)

Fig. 7

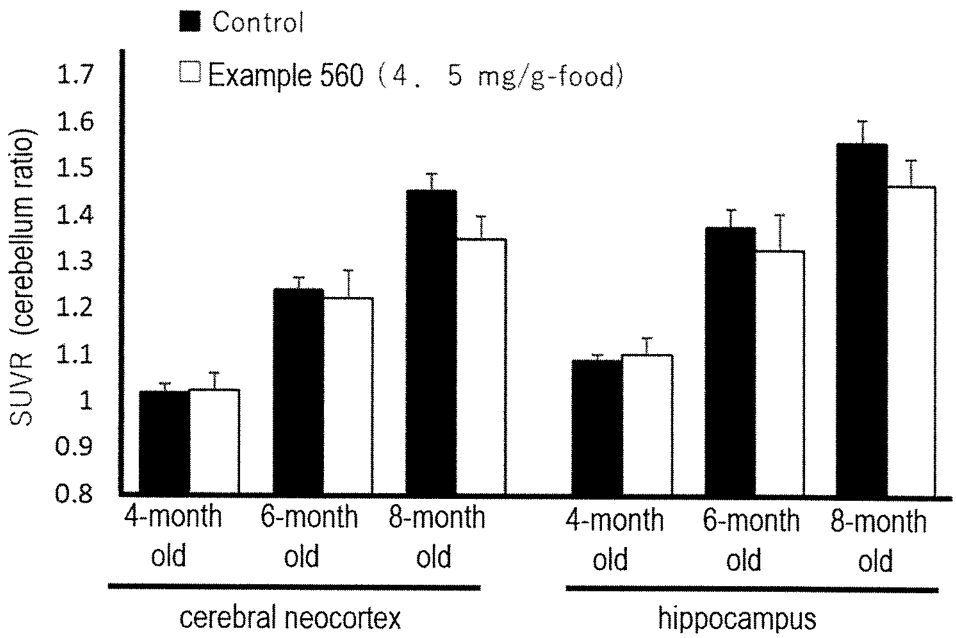
Control
Example 560 (4. 5 mg/g-food)
SUVR (cerebellum ratio)
1.7
1.6
1.5
1.4
1.3
1.2
1.1
1
0.9
0.8
4-month old
6-month old
8-month old
4-month old
6-month old
8-month old
cerebral neocortex
hippocampus

**INTERNATIONAL SEARCH REPORT**

International application No.
PCT/JP2021/000184

A. CLASSIFICATION OF SUBJECT MATTER
See extra sheet

According to International Patent Classification (IPC) or to both national classification and IPC

B. FIELDS SEARCHED

Minimum documentation searched (classification system followed by classification symbols)
A61K45/00; A61K31/437; A61K31/4375; A61K31/496; A61K31/4985; A61K31/50; A61K31/501; A61K31/5025; A61K31/506; A61K31/517; A61K31/519; A61K31/5377; A61K31/538; A61K31/5386; A61K31/55; A61K31/551; A61P21/02; A61P21/04; A61P25/16; A61P25/28; A61P43/00; C07D237/14; C07D237/22; C07D401/04; C07D401/06; C07D401/12; C07D401/14; C07D403/04; C07D403/06; C07D403/12; C07D403/14; C07D405/04; C07D405/06; C07D405/12; C07D405/14; C07D409/14; C07D413/06; C07D413/12; C07D413/14; C07D417/12; C07D417/14; C07D471/04; C07D471/08; C07D487/04; C07D487/10; C07D491/048; C07D491/08; C07D491/107; C07D513/04; C07D519/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS/WPIDS (STN); WPIDS/WPIX (STN); DWPI (Derwent Innovation)

C. DOCUMENTS CONSIDERED TO BE RELEVANT

| Category* | Citation of document, with indication, where appropriate, of the relevant passages | Relevant to claim No. |
|---|---|---|
| P, X<br>P, A | WO 2020/017587 A1 (SUMITOMO DAINIPPON PHARMA CO., LTD.) 23 January 2020 (2020-01-23) claims, examples | 41<br>1-40, 42-44 |
| X<br>A | CRESTEY, F., et al., "Identification and electrophysiological evaluation of 2-methylbenzamide derivatives as NaV1.1 modulators", ACS Chem. Neurosci., 2015, vol. 6, issue 8, pp. 1302-1308 in particular, abstract, scheme 1, table 1 | 41<br>1-40, 42-44 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>08 March 2021 (08.03.2021) | Date of mailing of the international search report<br>16 March 2021 (16.03.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/000184

C (Continuation). DOCUMENTS CONSIDERED TO BE RELEVANT

| Category* | Citation of document, with indication, where appropriate, of the relevant passages | Relevant to claim No. |
|---|---|---|
| X | DEUIS JR., et al., "The pharmacology of voltage-gated sodium channel activators", Neuropharmacology, 2017, vol.127, pp. 87-108 in particular, table 1 | 41 |
| A | in particular, abstract, table 1, page 103; 5.4 Drug leads | 1-40, 42-44 |
| A | JENSEN, HS., et al., "Therapeutic potential of NaV1.1 activators", Trends. Pharmacol. Sci., 2014, vol. 35, no. 3, pp. 113-118 in particular, abstract, page 114, right column, line 53 to page 115 | 1-44 |
| A | CHOW, CY., et al., "Development of NaV-Selective Agonists with Potential for Treatment of Dravet Syndrome Epilepsy", J. Pept. Sci., 2018, vol. 24, suppl. 2, p. S77(MS3) entire text | 1-44 |
| A | JP 2013-535958 A (CURNA, INC.) 19 September 2013 (2013-09-19) claims, paragraphs [0055], [0057], [0106], examples | 1-44 |
| A | WO 2019/109051 A1 (ENCODED THERAPEUTICS, INC.) 06 June 2019 (2019-06-06) claims, paragraph [0312], examples | 1-44 |

Form PCT/ISA/210 (continuation of second sheet) (January 2015)

**INTERNATIONAL SEARCH REPORT**
Information on patent family members

International application No.
PCT/JP2021/000184

| Patent Documents referred in the Report | Publication Date | Patent Family | Publication Date |
|---|---|---|---|
| WO 2020/017587 A1 | 23 Jan. 2020 | (Family: none) | |
| JP 2013-535958 A | 19 Sep. 2013 | WO 2011/163499 A2<br>claims, paragraphs [0075], [0077], [00125], examples<br>US 2013/0096183 A1<br>EP 2585596 A2 | |
| WO 2019/109051 A1 | 06 Jun. 2019 | TW 201925474 A | |

Form PCT/ISA/210 (patent family annex) (January 2015)

INTERNATIONAL SEARCH REPORT

International application No.
PCT/JP2021/000184

<Continuation of Box A>

A61K 45/00(2006.01)i; A61P 21/02(2006.01)i; A61P 21/04(2006.01)i; A61P 25/16(2006.01)i; A61P 25/28(2006.01)i; A61P 43/00(2006.01)i; C07D 237/14(2006.01)n; C07D 237/22(2006.01)n; C07D 401/04(2006.01)n; C07D 401/06(2006.01)n; C07D 401/12(2006.01)n; C07D 401/14(2006.01)n; C07D 403/04(2006.01)n; C07D 403/06(2006.01)n; C07D 403/12(2006.01)n; C07D 403/14(2006.01)n; C07D 405/04(2006.01)n; C07D 405/06(2006.01)n; C07D 405/12(2006.01)n; C07D 405/14(2006.01)n; C07D 409/14(2006.01)n; C07D 413/06(2006.01)n; C07D 413/12(2006.01)n; C07D 413/14(2006.01)n; C07D 417/12(2006.01)n; C07D 417/14(2006.01)n; C07D 471/04(2006.01)n; C07D 471/08(2006.01)n; C07D 491/048(2006.01)n; C07D 491/08(2006.01)n; C07D 491/107(2006.01)n; C07D 513/04(2006.01)n; C07D 487/04(2006.01)n; C07D 487/10(2006.01)n; C07D 519/00(2006.01)n; A61K 31/437(2006.01)i; A61K 31/4375(2006.01)i; A61K 31/496(2006.01)i; A61K 31/4985(2006.01)i; A61K 31/50(2006.01)i; A61K 31/501(2006.01)i; A61K 31/5025(2006.01)i; A61K 31/506(2006.01)i; A61K 31/517(2006.01)i; A61K 31/519(2006.01)i; A61K 31/5377(2006.01)i; A61K 31/538(2006.01)i; A61K 31/5386(2006.01)i; A61K 31/55(2006.01)i; A61K 31/551(2006.01)i

FI: A61K45/00; A61P21/02; A61P25/16; A61P43/00 111; A61P25/28; A61P43/00 121; A61K31/501; A61K31/55; A61K31/506; A61K31/5377; A61K31/517; A61K31/519; A61K31/5025; A61K31/551; A61K31/437; A61K31/4375; A61P21/04; A61K31/50; A61K31/5386; A61K31/538; A61K31/4985; A61K31/496; C07D413/06; C07D403/04; C07D237/22; C07D491/107; C07D405/04; C07D413/14; C07D401/14; C07D471/04 106A; C07D471/04 108E; C07D417/14; C07D403/12; C07D401/12; C07D471/04 104Z; C07D405/14; C07D471/04 101; C07D405/12; C07D403/14; C07D519/00 311; C07D471/08; C07D487/04 144; C07D403/06; C07D491/08; C07D401/06; C07D487/10; C07D513/04 343; C07D487/04 142; C07D491/048; C07D417/12; C07D487/04 133; C07D405/06; C07D413/12; C07D409/14; C07D471/04 113; C07D487/04 141; C07D519/00 301; C07D471/04 106C; C07D401/04 CSP; C07D237/14

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004006922 A
- WO 2012114268 A
- WO 2007020343 A
- US 20050277655 A
- WO 2018081091 A
- WO 2017009798 A

### Non-patent literature cited in the description


- *J. Neurosci.,* 2007, vol. 27, 5903
- *Trends in Pharmacological Sciences,* 2014, vol. 35, 113
- *Curr. Med. Chem.,* 2015, vol. 22, 1850
- *Annu. Rev. Pathol. Mech. Dis.,* 2019, vol. 14, 239
- *Cell,* 2012, vol. 149, 708
- *Journal of Alzheimer's Disease,* 2019, vol. 71 (4), 1163-1174
- Protective Groups in Organic Synthesis. John Wiley & Sons, Inc,
- **R. C. LAROQUE et al.** Comprehensive Organic Transformation. VCH publisher Inc, 1989
- *Tetrahedron,* 2015, vol. 71, 4859
- *Bioorganic & Medicinal Chemistry Letters,* 2015, vol. 25, 1030
- *ACS Medicinal Chemistry Letters,* 2012, vol. 3, 903
- *Bioorganic & Medicinal Chemistry Letters,* 2013, vol. 23, 2007
- *Organic Letters,* 2015, vol. 17, 5517
- *Organic & Biomolecular Chemistry,* 2014, vol. 12, 2049
- *Organic Letters,* 2008, vol. 10, 4815
- *Journal of Organic Chemistry,* 2015, vol. 80, 12182
- *Journal of Medicinal Chemistry,* 1993, vol. 36, 4052
- *European Journal of Organic Chemistry,* 2005, 3761